# EUROPEAN PATENT APPLICATION

(11) **EP 1 167 390 A1**
(43) Date of publication of application: **02.01.2002**
(21) Application number: 00906616.8
(22) Date of filing: 02.03.2000
(51) Int. Cl.: C07K 19/00, C07K 14/535, C07K 14/715, G01N 33/15, G01N 33/68, G06F 17/30, G06F 17/50, C07B 61/00

(54) **CRYSTALS AND STRUCTURAL COORDINATE OF PROTEIN COMPLEX AND UTILISATION OF THE STRUCTURAL COORDINATE**

(30) Priority: 04.03.1999 JP 5690599; 02.08.1999 JP 21869199
(71) Applicant: Biomolecular Engineering Research Institute, Suita-shi, Osaka 565-0874 (JP)
(72) Inventor: Aritomi, Masaharu, Kawanishi-shi, Hyogo 666-0033 (JP); Kunishima, Naoki, Toyonaka-shi, Osaka 560-0055 (JP); Morikawa, Kosuke, Takatsuki-shi, Osaka 569-1022 (JP)
(74) Representative: VOSSIUS & PARTNER
(86) International application number: JP0001217
(87) International publication number: WO0052057

(57) **Abstract**

Crystals of a protein complex composed of granulocyte colony-stimulating factor (G-CSF) and the G-CSF binding region of a G-CSF receptor; and the structural coordinate of each atom determined by the crystallography procedure from these crystals. By using this structural coordinate, it is possible to identify, search, evaluate or design a G-CSF mutant which has a biological activity higher than native G-CSF or an inhibitory activity on G-CSF and is derived by substitution, deletion, insertion or chemical modification of one or more amino acid residues, an agonist which is a compound having a biological activity comparable or superior to the biological activity of G-CSF, and an antagonist which is a compound inhibiting the biological activity of G-CSF.

SEQUENCE LISTING

<110> Biomolecular Engineering Research Institute

<120> Crystal of Protein Complex, Structural Coordinate Thereof and Use
Thereof

<130> 661699

<150> JP 56905/1999 and 218691/1999
<151> 1999-03-04 and 1999-08-02

<160> 3
<210> 1
<211> 175
<212> PRT
<213> homo sapience

<400> 1
Met Thr Pro Leu Gly Pro Ala Ser Ser Leu Pro Gln Ser Phe Leu Leu
1               5                   10                  15
Lys Cys Leu Glu Gln Val Arg Lys Ile Gln Gly Asp Gly Ala Ala Leu
        20                  25                  30
Gln Glu Lys Leu Cys Ala Thr Tyr Lys Leu Cys His Pro Glu Glu Leu
    35                  40                  45
Val Leu Leu Gly His Ser Leu Gly Ile Pro Trp Ala Pro Leu Ser Ser
    50                  55                  60
Cys Pro Ser Gln Ala Leu Gln Leu Ala Gly Cys Leu Ser Gln Leu His
65                  70                  75                  80

Ser Gly Leu Phe Leu Tyr Gln Gly Leu Leu Gln Ala Leu Glu Gly Ile
                85                  90                  95
Ser Pro Glu Leu Gly Pro Thr Leu Asp Thr Leu Gln Leu Asp Val Ala
            100                 105                 110
Asp Phe Ala Thr Thr Ile Trp Gln Gln Met Glu Glu Leu Gly Met Ala
        115                 120                 125
Pro Ala Leu Gln Pro Thr Gln Gly Ala Met Pro Ala Phe Ala Ser Ala
    130                 135                 140
Phe Gln Arg Arg Ala Gly Gly Val Leu Val Ala Ser His Leu Gln Ser
145                 150                 155                 160
Phe Leu Glu Val Ser Tyr Arg Val Leu Arg His Leu Ala Gln Pro
                165                 170                 175

<210> 2
<211> 215
<212> PRT
<213> mouse

<400> 2
Ala Gly Tyr Pro Pro Ala Ser Pro Ser Asn Leu Ser Cys Leu Met His
1               5                   10                  15
Leu Thr Thr Asn Ser Leu Val Cys Gln Trp Glu Pro Gly Pro Glu Thr
            20                  25                  30
His Leu Pro Thr Ser Phe Ile Leu Lys Ser Phe Arg Ser Arg Ala Asp
        35                  40                  45
Cys Gln Tyr Gln Gly Asp Thr Ile Pro Asp Cys Val Ala Lys Lys Arg
    50                  55                  60
Gln Asn Asn Cys Ser Ile Pro Arg Lys Asn Leu Leu Leu Tyr Gln Tyr
65                  70                  75                  80

Met Ala Ile Trp Val Gln Ala Glu Asn Met Leu Gly Ser Ser Glu Ser
                85                  90                  95
Pro Lys Leu Cys Leu Asp Pro Met Asp Val Val Lys Leu Glu Pro Pro
            100                 105                 110
Met Leu Gln Ala Leu Asp Ile Gly Pro Asp Val Val Ser His Gln Pro
        115                 120                 125
Gly Cys Leu Trp Leu Ser Trp Lys Pro Trp Lys Pro Ser Glu Tyr Met
    130                 135                 140
Glu Gln Glu Cys Glu Leu Arg Tyr Gln Pro Gln Leu Lys Gly Ala Asn
145                 150                 155                 160
Trp Thr Leu Val Phe His Leu Pro Ser Ser Lys Asp Gln Phe Glu Leu
                165                 170                 175
Cys Gly Leu His Gln Ala Pro Val Tyr Thr Leu Gln Met Arg Cys Ile
            180                 185                 190
Arg Ser Ser Leu Pro Gly Phe Trp Ser Pro Trp Ser Pro Gly Leu Gln
        195                 200                 205
Leu Arg Pro Thr Met Lys Ala
    210                 215

<210> 3
<211> 215
<212> PRT
<213> homo sapience
<400> 3
Ala Gly Tyr Pro Pro Ala Ile Pro His Asn Leu Ser Cys Leu Met Asn
1               5                   10                  15
Leu Thr Thr Ser Ser Leu Ile Cys Gln Trp Glu Pro Gly Pro Glu Thr
            20                  25                  30
His Leu Pro Thr Ser Phe Thr Leu Lys Ser Phe Lys Ser Arg Gly Asn

        35                  40                  45
Cys Gln Thr Gln Gly Asp Ser Ile Leu Asp Cys Val Pro Lys Asp Gly
    50                  55                  60
Gln Ser His Cys Cys Ile Pro Arg Lys His Leu Leu Leu Tyr Gln Asn
65                  70                  75                  80
Met Gly Ile Trp Val Gln Ala Glu Asn Ala Leu Gly Thr Ser Met Ser
                85                  90                  95
Pro Gln Leu Cys Leu Asp Pro Met Asp Val Val Lys Leu Glu Pro Pro
            100                 105                 110
Met Leu Arg Thr Met Asp Pro Ser Pro Glu Ala Ala Pro Pro Gln Ala
        115                 120                 125
Gly Cys Leu Gln Leu Cys Trp Glu Pro Trp Gln Pro Gly Leu His Ile
    130                 135                 140
Asn Gln Lys Cys Glu Leu Arg His Lys Pro Gln Arg Gly Glu Ala Ser
145                 150                 155                 160
Trp Ala Leu Val Gly Pro Leu Pro Leu Glu Ala Leu Gln Tyr Glu Leu
                165                 170                 175
Cys Gly Leu Leu Pro Ala Thr Ala Tyr Thr Leu Gln Ile Arg Cys Ile
            180                 185                 190
Arg Trp Pro Leu Pro Gly His Trp Ser Asp Trp Ser Pro Ser Leu Glu
        195                 200                 205
Leu Arg Thr Thr Glu Arg Ala
    210                 215

## Description

### TECHNICAL FIELD

The present invention relates to crystals of a complex between granulocyte colony-stimulating factor (hereinafter abbreviated as G-CSF) and the G-CSF binding region (hereinafter abbreviated as CRH-G-CSF-R) of the granulocyte colony-stimulating factor receptor (hereinafter abbreviated as G-CSF-R) and also relates to three-dimensional structure coordinates of the complex between G-CSF and CRH-G-CSF-R obtained by crystallography techniques using a X-ray diffraction method with said crystals of the complex.

The present invention also relates to designing, selecting, and searching for G-CSF variants which bind to G-CSF-R and in which one or more amino acid residues in native G-CSF have been substituted, deleted, inserted, or chemically modified, by means of the three-dimensional structure coordinates of the complex between G-CSF and CRH-G-CSF-R.

The present invention further relates to identifying, searching for, evaluating, or designing compounds which bind to G-CSF-R and which are agonists of G-CSF having biological activities equal or superior to those of G-CSF, by means of the three-dimensional structure coordinates of the complex between G-CSF and CRH-G-CSF-R.

In addition, the present invention relates to identifying, searching for, evaluating, or designing compounds which bind to G-CSF and/or G-CSF-R and which are antagonists inhibiting normal binding of G-CSF to G-CSF-R and thereby reducing the effects of G-CSF, by means of the three-dimensional structure coordinates of the complex between G-CSF and CRH-G-CSF-R.

### BACKGROUND ART

"Cytokine" is the general term for proteinaceous factors which exhibit biological activities at a very small amount by binding to the specific receptors expressed on the cell surface. G-CSF is one of such cytokines and regulates the differentiation and proliferation of a blood cell group primarily classified as granulocytes existing in blood (Metcalf, *D., Nature,* **339**:27-30 (1989)). Genetic analyses of G-CSF revealed that it is a protein consisting of about 180 amino acid residues (Nagata, S. *et al., Nature,* **319**:415-318 (1989); Souza, L. M. *et al. Science,* **232:**61-65 (1986); and Japanese Patent Kohyo Publication No. S63-500636 (1988)).

This factor is generally produced in *Escherichia coli* or animal cells using gene manipulation and it is also commercially available as a reagent for use in scientific experiments. Furthermore, G-CSF has come into practical use as a medicine for patients having a decreased number of leukocytes such as granulocytes, for example, due to chemotherapy or radiotherapy of cancer to restore the number of leukocytes.

Since the medicine of GSF is a protein preparation, however, it is expensive. Therefore, G-CSF variant having sufficiently high biological activities to exert its efficacy at a less amount is being required. Furthermore, the G-CSF preparation in practical use cannot be orally administered because it is proteinaceous. In fact, the preparation is administered via intravenous or subcutaneous injection. Such a preparation cannot be administered by the patients themselves, and it has to be administered by a health professional such as a physician. In addition, such administration routes cause the patient's pain. Thus, an agonist having G-CSF biological activities which can be administered by a easier route of administration, for example, by oral administration, is being desired.

Likewise, an antagonist which inhibits the activities of G-CSF is also being desired as a medicine which can be administered, for example, when leukocytes such as granulocytes abnormally proliferate.

On the other hand, G-CSF-R, which has a function of transmitting the activities of G-CSF to cells, exists on the surfaces of G-CSF-responsive cells, and is a protein consisting of about 800 amino acid residues. G-CSF-R has an ability to bind with G-CSF at a particular region that exists extracellularly. This region has an amino acid sequence similar to those of other cytokine receptors, and is generally known as a cytokine-receptor homology (CRH) region. cDNAs encoding G-CSF-Rs derived from mouse and human have been cloned and their nucleotide sequences have been determined (Fukunaga, R. *et al., Cell,* **61**:341-350 (1990); Fukunaga, R. *et al., Proc. Natl. Acad. Sci. USA,* **87:**8702-8706 (1990); and WO 91/14776). The CRH region is a homology region consisting of about 200 amino acid residues which is found in extracellular regions of cytokine receptors such as those for interleukins 2 to 7, erythropoietin, growth hormone, GM-CSF, and interferons α, β, and γ, and represents ligand binding sites of these receptors. These receptors are collectively called the cytokine receptor family (Bazan, J. F., *Proc*. *Natl*. *Acad*. *Sci. USA*, **87**:6934-6938 (1990)).

The CRH regions are considered as the most critical domains for ligand binding and signal transduction of this family. Elucidation of binding between the CRH regions of receptors and their ligands are, therefore, essential to elucidate the interactions between the ligands and the whole receptors.

The signals resulted from ligand binding of these receptors are transmitted to the intracellular moiety of the receptors to activate phosphorylating enzymes in the cells. The signals are further transduced through a pathway in which specific proteins in the cells are phosphorylated by those phosphorylating enzymes. It is described that biological activities of G-CSF also result from the activation of an intracellular phosphorylating enzyme stimulated by G-CSF binding to G-CSF-R.

Three-dimensional structure coordinates of G-CSF are publicly known (Hill, C. P., *et al*., *Proc*. *Natl. Acad*. *Sci. USA*, **90**:5167-5171 (1993); Protein Data Bank Entry Number: lhrg). Three-dimensional structure coordinates of a part of G-CSF-R have also been revealed (Yamasaki, K. *et al., Nat*. *Struct. Biol.,* **4**:498-404 (1997); Protein Data Bank Entry Number: lgcf). Furthermore, for some cytokines other than G-CSF, a crystal structure of a complex between, for example, growth hormone and the extracellular portion of its receptor has also been solved (de Vos, A. M. *et al., Science,* **255**:306-312 (1992); Protein Data Bank Entry Number: 3hhr).

In spite of such information, three-dimensional structure coordinates of the complex between G-CSF and G-CSF-R itself were not known, and therefore, details of chemical interactions between G-CSF and G-CSF-R in three-dimensional space could not be logically understood. Specifically, although structure coordinates of G-CSF have been solved and a method for producing variants based on the structure coordinates has also been disclosed as described in Japanese Patent Kohyo Publication No. H8-50618 (1996), the prior invention described in the patent publication does not give a precise picture of the chemical interactions with the receptor side, and therefore, could not enable us to produce variants taking into account the receptor side in three-dimensional space. Similarly, other approaches such as those described in Japanese Patent Kokai Publication Nos. H6-309385 (1994) and H7-133233 (1995) could not enable us to design agonists or antagonists of G-CSF, because three-dimensional structure coordinates of the complex between G-CSF and G-CSF-R were not known.

### SUMMARY OF THE INVENTION

In order to solve the above problems, the present inventors prepared crystals of the complex between G-CSF and CRH-G-CSF-R and revealed three-dimensional structure coordinates of the complex between G-CSF and CRH-G-CSF-R for the first time. Since CRH-G-CSF-R can be considered as an equivalent of G-CSF-R in respect to interactions with G-CSF, the three-dimensional structure coordinates can reveal for the first time details of chemical interactions between G-CSF and G-CSF-R in three-dimensional space.

Thus, according to the present invention, a detailed mechanism for transduction of stimulus by G-CSF can be revealed, and one can design a variant G-CSF having higher activities or inhibiting G-CSF activities by altering one or more amino acid residues in the G-CSF protein on the basis of the three-dimensional structure coordinates. Furthermore, according to the present invention, it becomes possible to identify, search for, evaluate, or design an agonist having G-CSF biological activities or an antagonist inhibiting G-CSF biological activities on the basis of the three-dimensional structure coordinates.

Accordingly, the present invention relates to crystals of the protein complex between G-CSF and CRH-G-CSF-R.

The present invention also relates to three-dimensional structure coordinates of the complex formed by G-CSF and CRH-G-CSF-R for use in identifying, searching for, evaluating, or designing a variant, agonist, or antagonist of G-CSF.

The present invention further relates to a computer storage medium storing all or part of the above three-dimensional structure coordinates for use in identifying, searching for, evaluating, or designing a variant, agonist, or antagonist of G-CSF.

The present invention further relates to use of all or part of the above three-dimensional structure coordinates or the above computer storage medium for identifying, searching for, evaluating, or designing a variant, agonist, or antagonist of G-CSF.

The present invention further relates to a method of identifying, searching for, evaluating, or designing a G-CSF variant which has biological activities equal or superior to those of native G-CSF and in which one or more amino acid residues have been substituted, deleted, inserted, or chemically modified, the method being characterized in that it uses all or part of the above three-dimensional structure coordinates or the above computer storage medium.

The present invention further relates to a method of identifying, searching for, evaluating, or designing a G-CSF variant which has activities as an antagonist of G-CSF and in which one or more amino acid residues have been substituted, deleted, inserted, or chemically modified, the method being characterized in that it uses all or part of the above three-dimensional structure coordinates or the above computer storage medium.

The present invention further relates to a method of identifying, searching for, evaluating, or designing an agonist of G-CSF, the method being characterized in that it uses all or part of the above three-dimensional structure coordinates or the above computer storage medium.

The present invention further relates to a method of identifying, searching for, evaluating, or designing an antagonist of G-CSF, the method being characterized in that it uses all or part of the above three-dimensional structure coordinates or the above computer storage medium.

### BRIEF DESCRIPTION OF DRAWING

Fig. 1 is a ribbon drawing of the crystal structure of G-CSF and CRH-G-CSF-R complex showing their backbones viewed from a direction approximately orthogonal to the pseudo-twofold axis of the molecules.

Fig. 2 is a ribbon drawing of the crystal structure of G-CSF and CRH-G-CSF-R complex showing their backbones viewed from a direction approximately parallel to the non-crystallographic pseudo-twofold axis of the molecules.

In Fig. 3, one of the two molecules existing in an asymmetric unit (that is, one molecule of the complex between Molecules A and B, and one molecule of the complex between Molecules C and D) has been displaced onto the other in such a way that the G-CSF portions of respective molecules (Molecule A and Molecule C) are best superposed. This figure schematically represents the backbones of such structures.

### BEST MODE FOR CARRYING OUT THE INVENTION

In the present specification, amino acids, peptides, and proteins are described using the following abbreviations adopted by IUPAC-IUB Commission on Biochemical Nomenclature (CBN). Unless otherwise specified, sequence of amino acid residues in a peptide or protein is indicated from its N-terminus to its C-terminus in the left-to-right direction, with the N-terminus being numbered 1.
- A or Ala:: alanine residue
- D or Asp:: aspartic acid residue
- E or Glu:: glutamic acid residue
- F or Phe:: phenylalanine residue
- G or Gly:: glycine residue
- H or His:: histidine residue
- I or Ile:: isoleucine residue
- K or Lys:: lysine residue
- L or Leu:: leucine residue
- M or Met:: methionine residue
- N or Asn:: asparagine residue
- P or Pro:: proline residue
- Q or Gln:: glutamine residue
- R or Arg:: arginine residue
- S or Ser:: serine residue
- T or Thr:: threonine residue
- V or Val:: valine residue
- W or Trp:: tryptophan residue
- Y or Tyr:: tyrosine residue
- C or Cys:: cysteine residue

### 1. Crystals of the complex between G-CSF and CRH-G-CSF-R

G-CSF and CRH-G-CSF-R used in the present invention are mammalian types, preferably a mouse or a human type, and particularly preferably a human type.

Although "CRH-G-CSF-R" refers to a region from tyrosine residue at position 97 to alanine residue at position 309 in the amino acid sequence of mouse-type G-CSF-R or to corresponding regions of other mammalian type G-CSF-Rs, the beginning and ending positions of CRH-G-CSF-R are not necessarily strict and do not have any great impact on the stereostructure of CRH-G-CSF-R as a whole. The term "CRH-G-CSF-R" includes those regions in which the N-terminus and/or the C-terminus is shifted in either direction by several residues, or those regions in which several amino acid residues are added to the N-terminus and/or the C-terminus, provided that they retain the functions. Usually, such differences in the primary sequence will not have any great effect on the stereostructure of CRH-G-CSF-R as a whole, and the function is therefore expected to be retained. In the examples described below, the region from alanine residue at position 95 to alanine residue at position 309 in the above amino acid sequence was used for mouse-type CRH-G-CSF-R.

The most popular technique for elucidating three-dimensional structure of a protein is X-ray crystallography. In this technique, a crystallized protein is applied to monochromated X-ray to obtain X-ray diffraction patterns based on which three-dimensional structure of the protein is elucidated (Blundell, T. L. and Johnson, L. N., PROTEIN CRYSTALLOGRAPHY, pp. 1-565, 1976, Academic Press, New York).

Crystallization exploits a protein's property of precipitating as crystals under certain conditions when a protein in solution becomes undissolved, for example, by addition of a precipitant to a protein solution or by reducing the volume of solvent through evaporation or the like. A highly purified protein is required for crystallization. In addition, physical and chemical factors such as protein concentration, salt concentration, hydrogen ion concentration (pH), type of precipitant added, temperature, and the like are involved in conditions under which crystallization occurs. Furthermore, there are many crystallization techniques such as batch, dialysis, and vapor-diffusion procedures depending on the method of adding a precipitant or of regulating the volume of solvent (Blundell, T. L. and Johnson, L. N., PROTEIN CRYSTALLOGRAPHY, pp. 59-82, 1976, Academic Press, New York). Thus, in order to obtain a protein crystal suitable for X-ray crystallography, it is necessary for each protein to obtain a highly purified protein and to make a study for optimizing various factors and the crystallization techniques.

The crystals of the present invention between human-type G-CSF and mouse-type CRH-G-CSF-R are prepared as follows.

First, human-type G-CSF and mouse-type CRH-G-CSF-R are highly purified. Purified proteins are then combined with each other and allowed to form a complex. The complex is further purified to a high purity so that it becomes suitable for crystallization. Purification methods which may be used alone or in combination are those methods commonly used in the art for purifying proteins such as column chromatography (affinity, hydrophobic, ion exchange, gel filtration, and the like), salting out, centrifugation, and electrophoresis. The purification steps after the formation of the complex have to be conducted while retaining the complex. It is, therefore, preferred to use purification methods which allow the salt and hydrogen ion concentrations to be adjusted to values more similar to the physiological conditions, for example, gel filtration chromatography.

Secondly, crystals of the complex between G-CSF and CRH-G-CSF-R are prepared. This step may be conducted using a crystallization technique such as a batch, dialysis, or vapor-diffusion procedure. It is also necessary to determine physical and chemical factors such as protein concentration, salt concentration, hydrogen ion concentration (pH), type of precipitant added, temperature, and the like.

For crystallization of the complex between G-CSF and CRH-G-CSF-R, it is essential to conduct the crystallization under conditions which retain the complex. For example, crystals of the complex between G-CSF and CRH-G-CSF-R are obtained by a vapor-diffusion method using a 1.0 to 1.2 M concentration of ammonium sulfate as a precipitant under conditions of pH=7 to 8, a protein concentration of 0.5 to 2 mg/ml, and a temperature of 20°C. Furthermore, under the same conditions containing 2 to 10 % 1,4-dioxane added thereto, larger crystals suitable for X-ray crystallography are obtained.

However, since it is well known to those skilled in the art that the same protein may crystallize under different conditions, the present invention is not limited to such conditions, and crystals of the complex between G-CSF and CRH-G-CSF which provide crystallographic constants substantially identical to those of the present invention are within the scope of the present invention.

### 2. Structure coordinates of the complex between G-CSF and CRH-G-CSF-R

Structure of the crystal of the complex between G-CSF and CRH-G-CSF-R thus obtained is analyzed using X-ray crystal structure analysis techniques known to those skilled in the art.

The crystal of the complex between human-type G-CSF shown in SEQ ID NO: 1 and mouse-type CRH-G-CSF-R shown in SEQ ID NO: 2 belongs to the tetragonal space group of I4₁22, with unit cell parameters of 125 ± 10 Å in the a- and b-axis directions and 373 ± 10 Å in the c-axis direction. The crystal of the complex is further subjected to crystal structure analysis by X-ray diffraction to obtain the three-dimensional structure coordinates of the present invention (values indicating relative spatial positions of each atoms) of the complex between G-CSF and CRH-G-CSF-R. The structure coordinates obtained are shown in Table 1 according to a notation system for three-dimensional structure coordinates of a protein commonly used in the art.

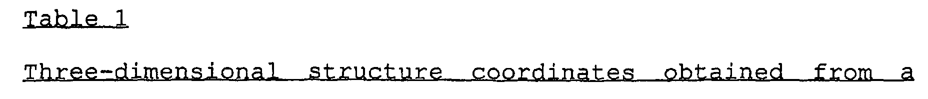

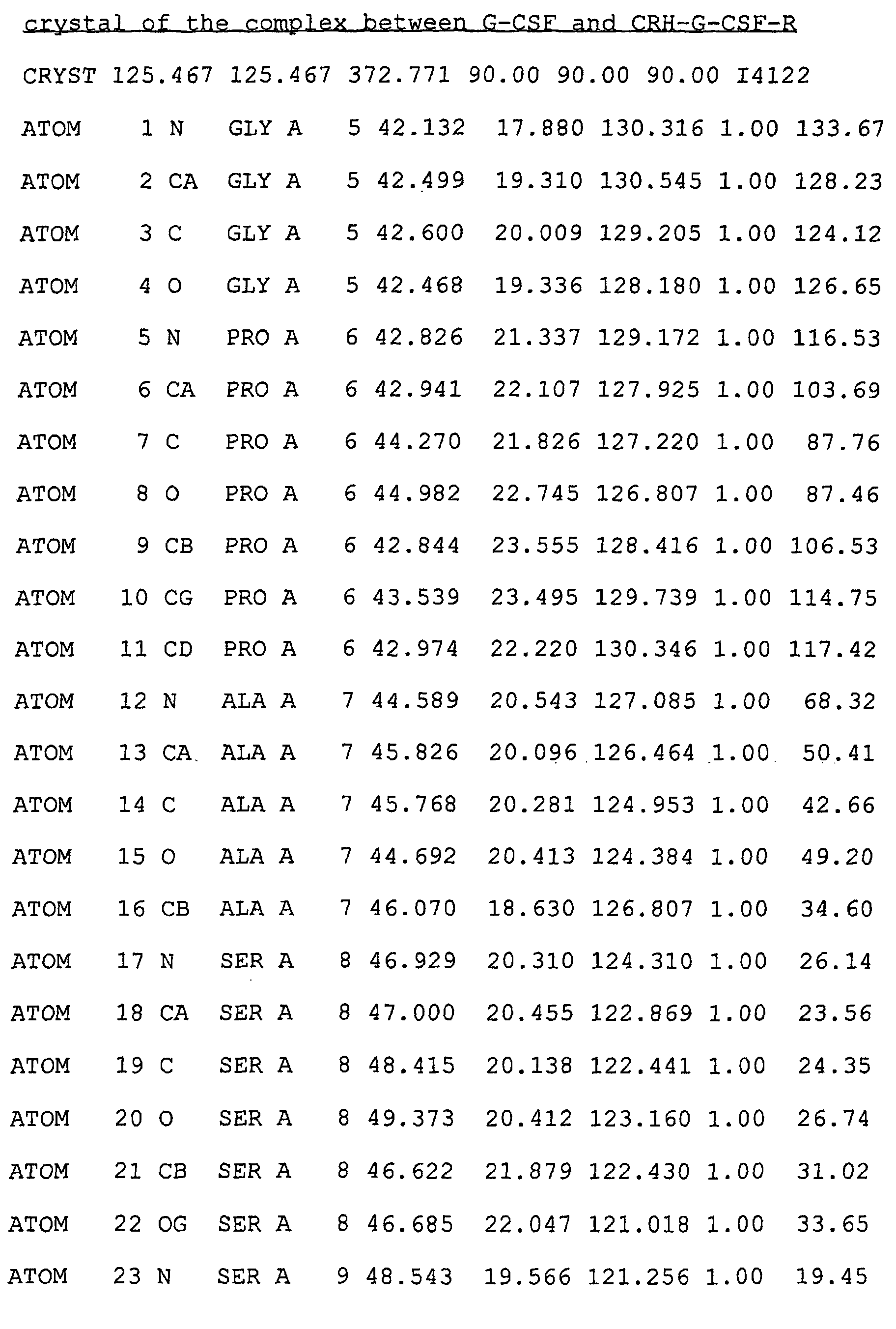

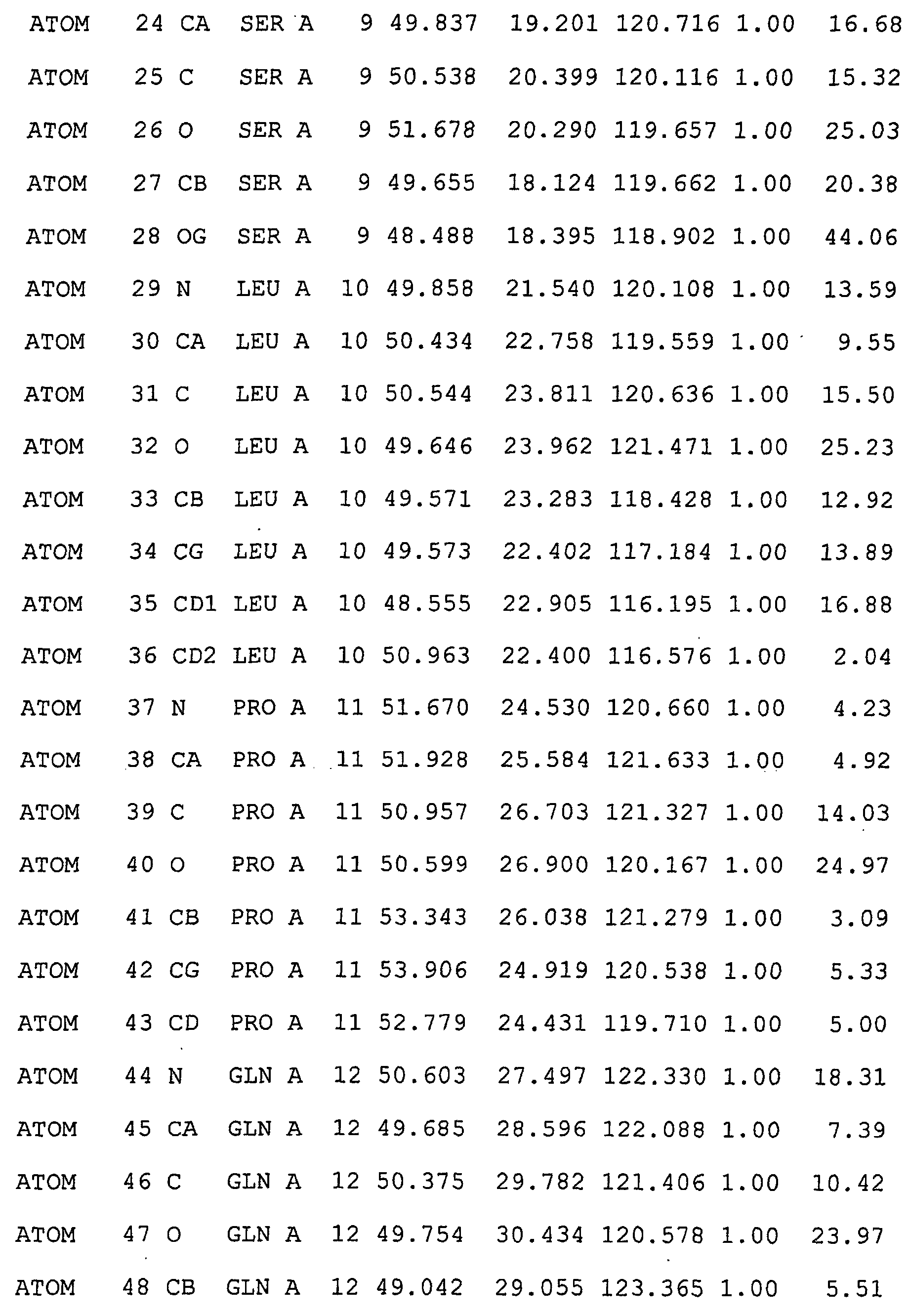

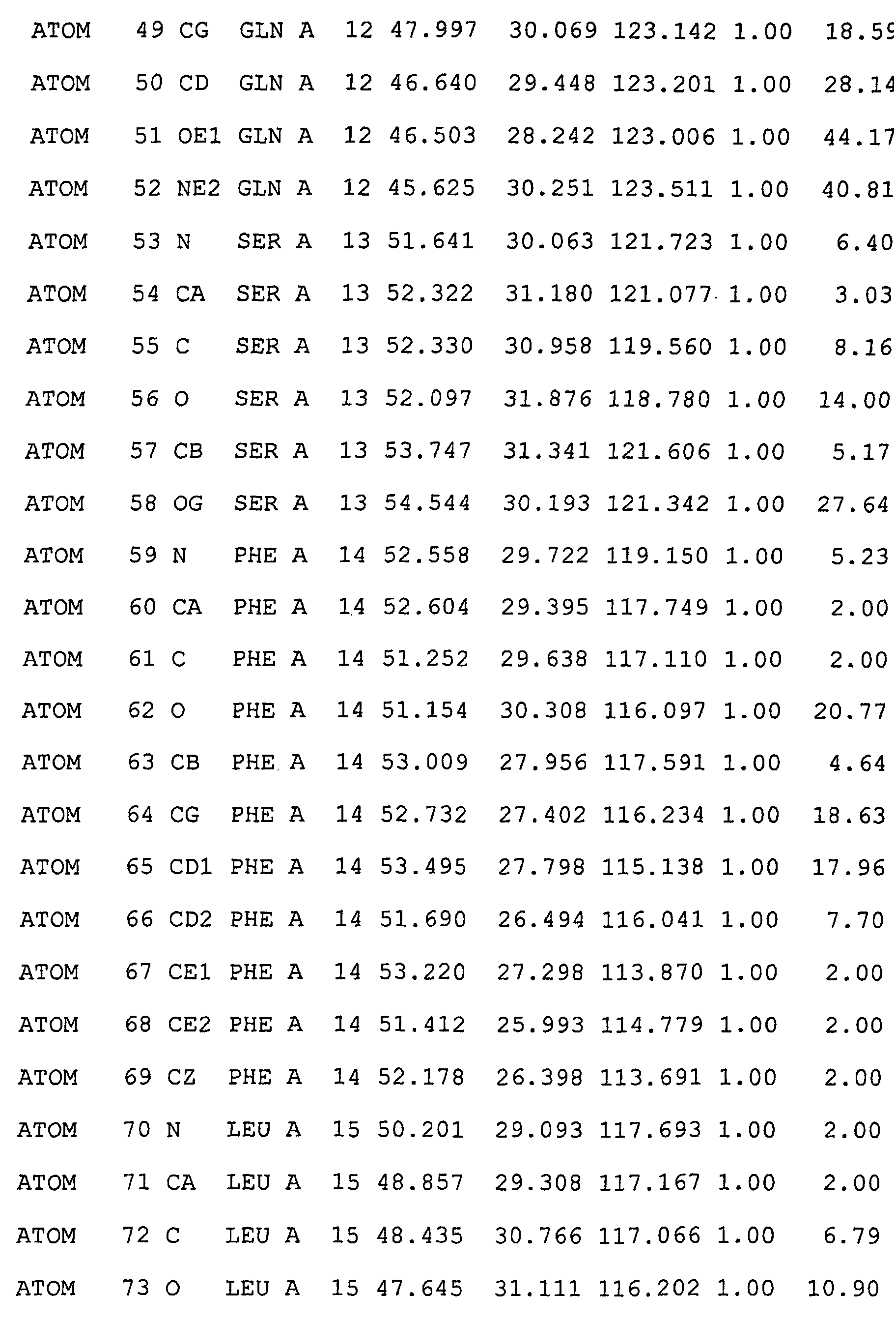

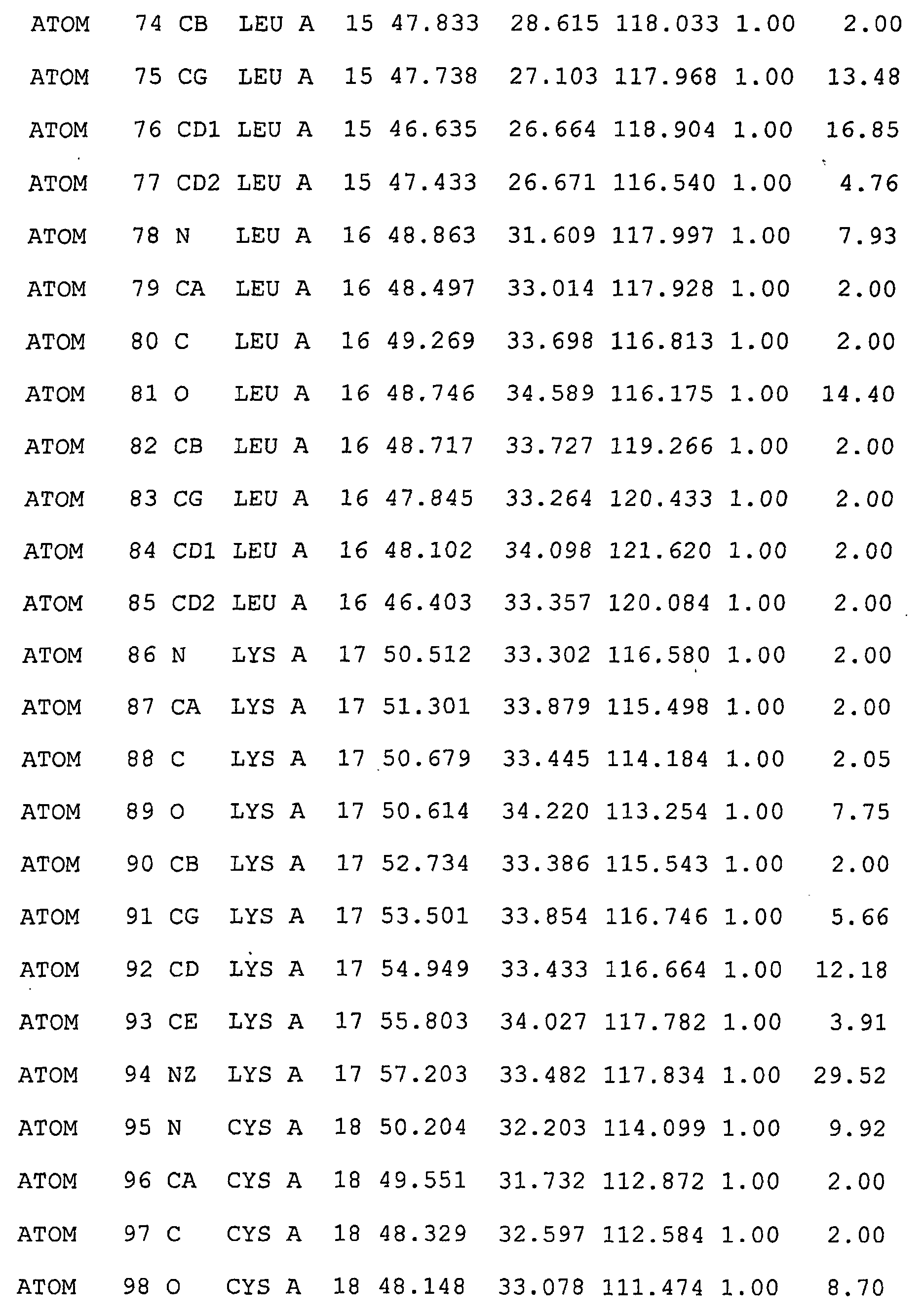

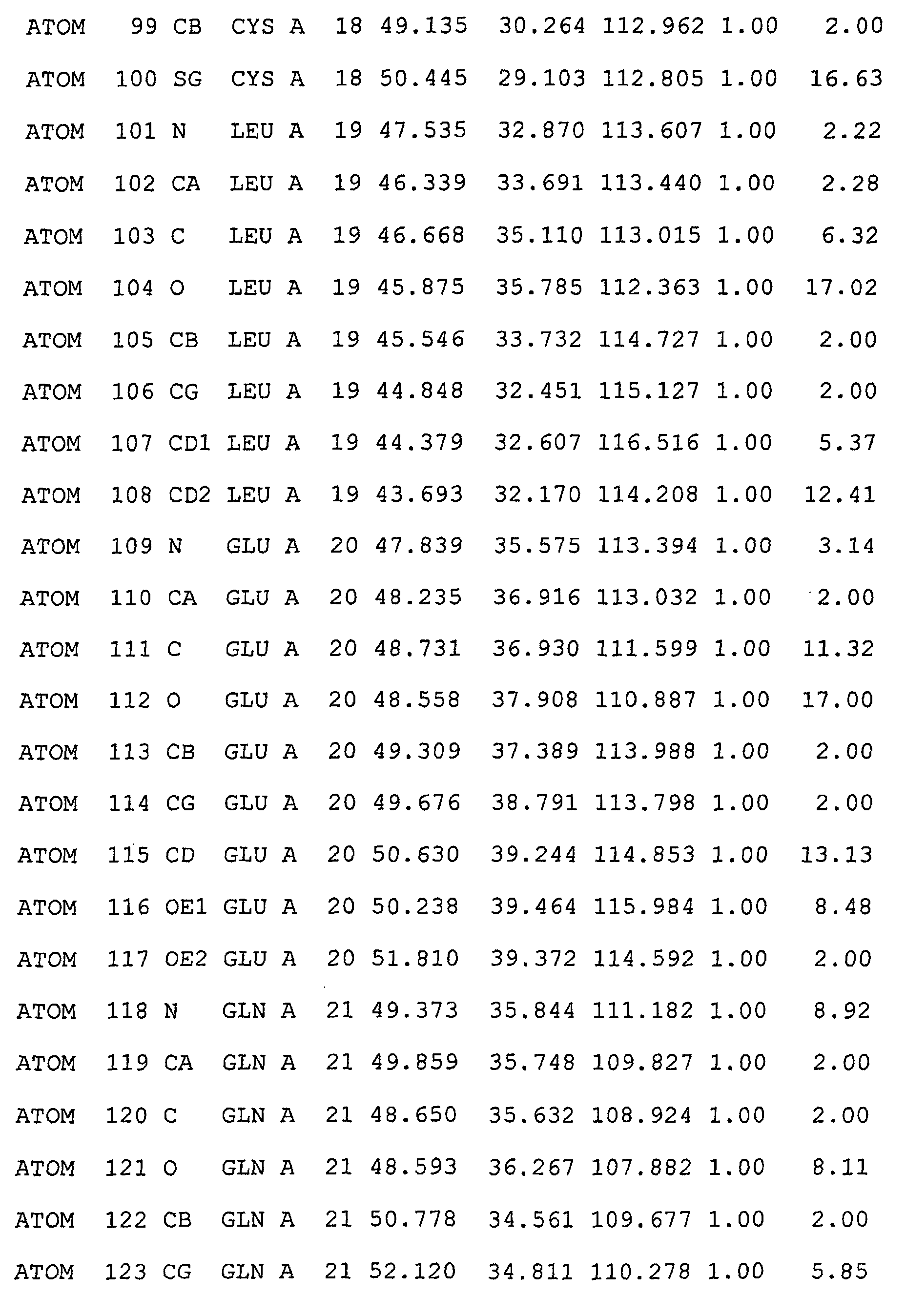

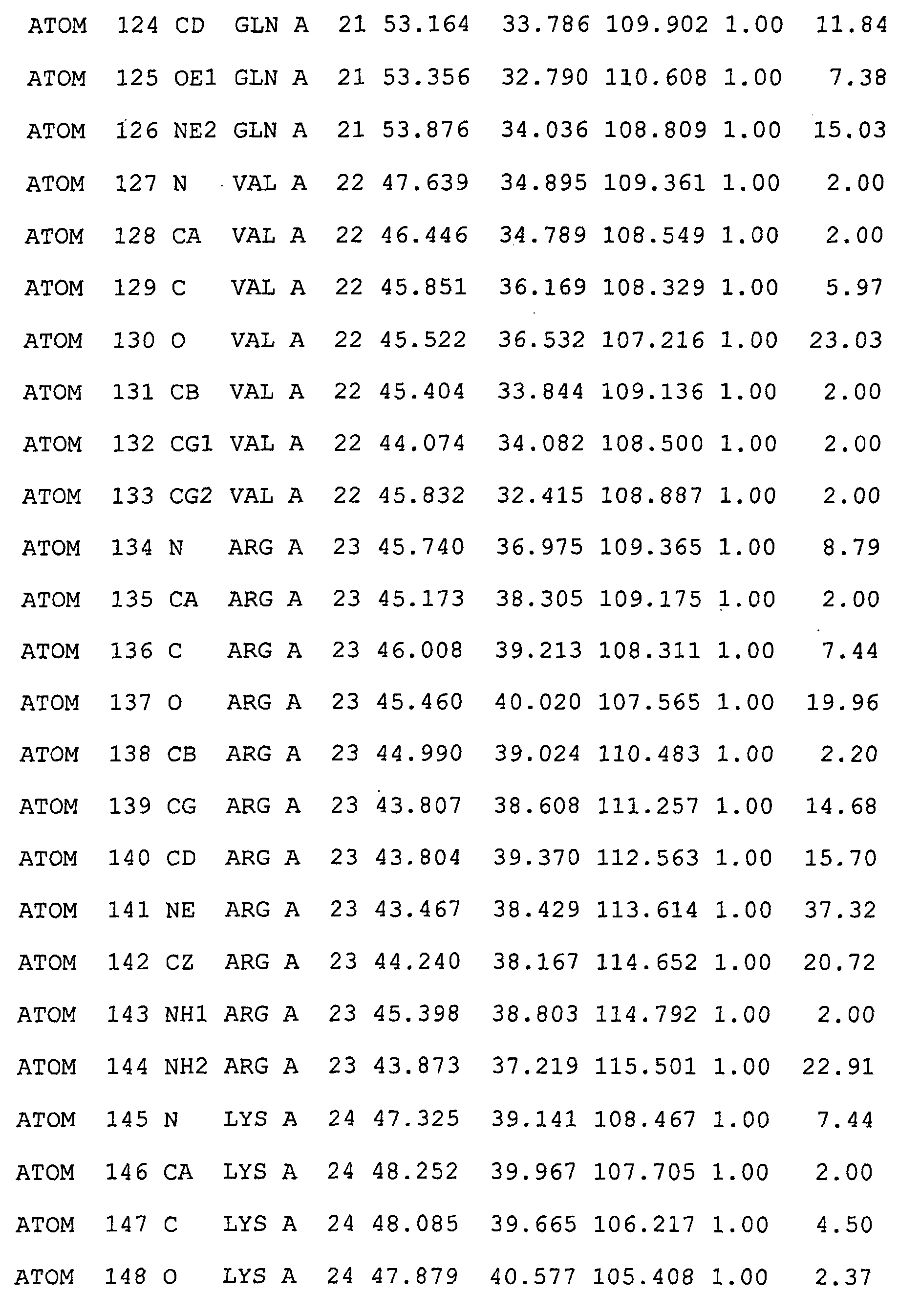

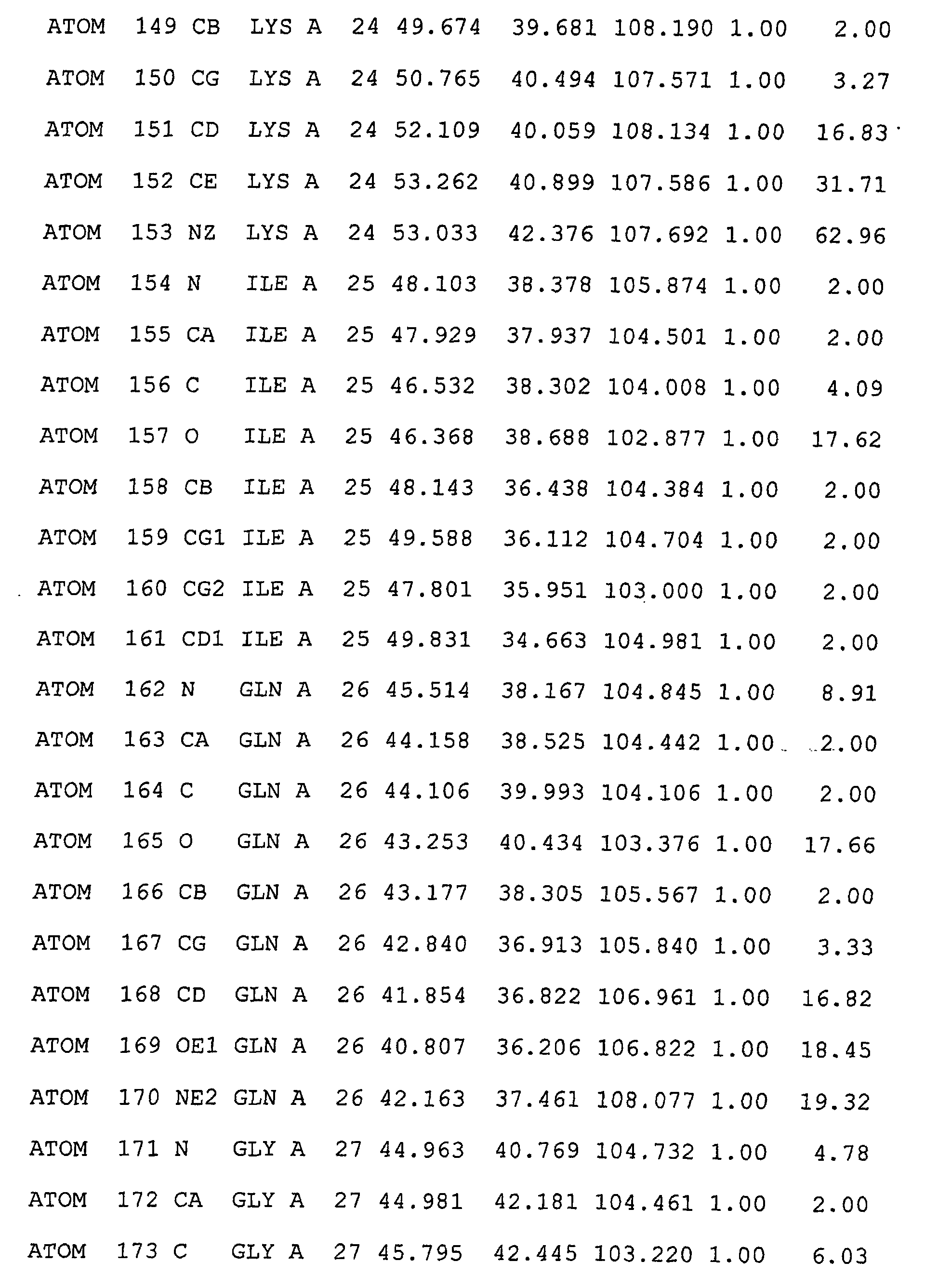

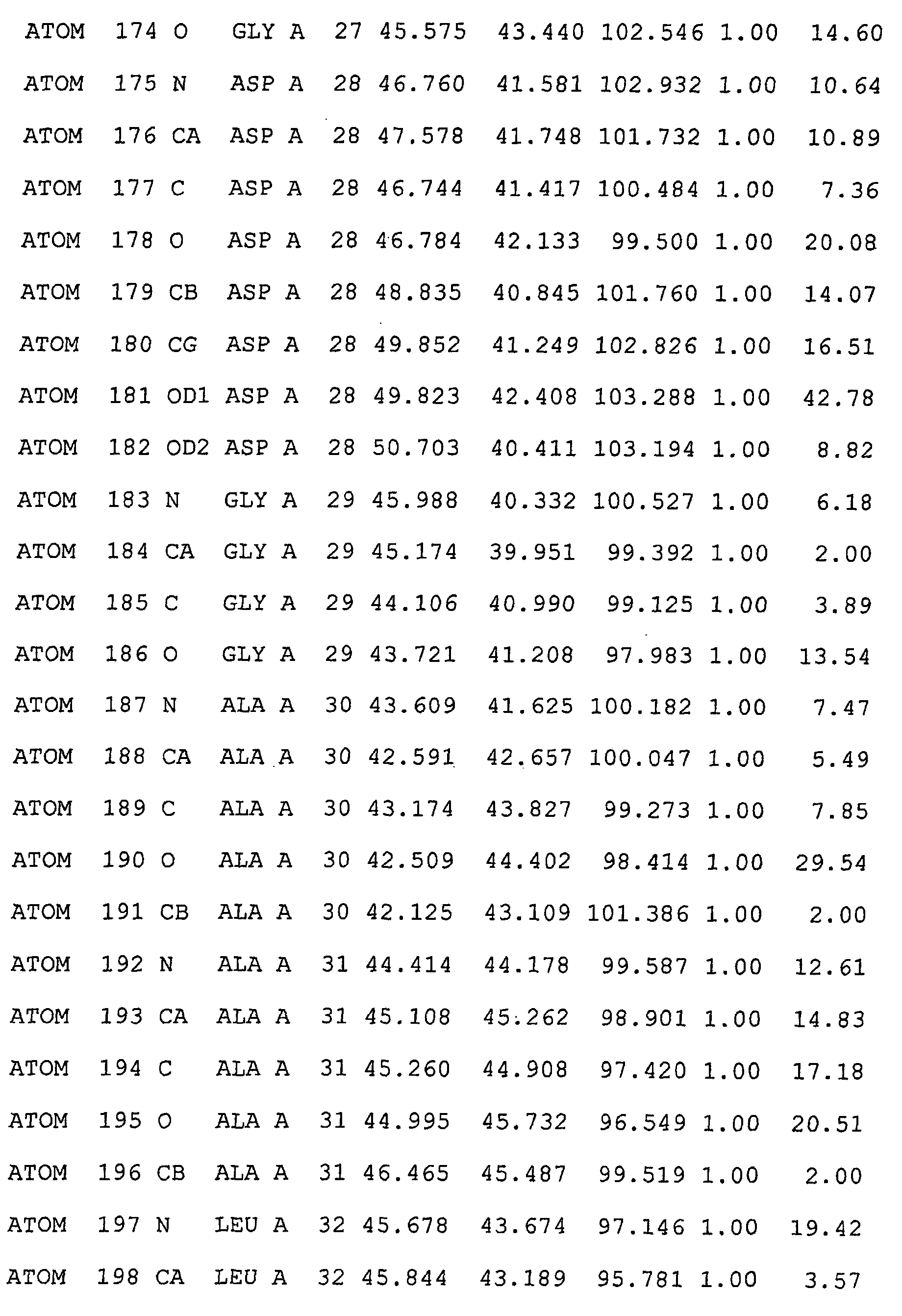

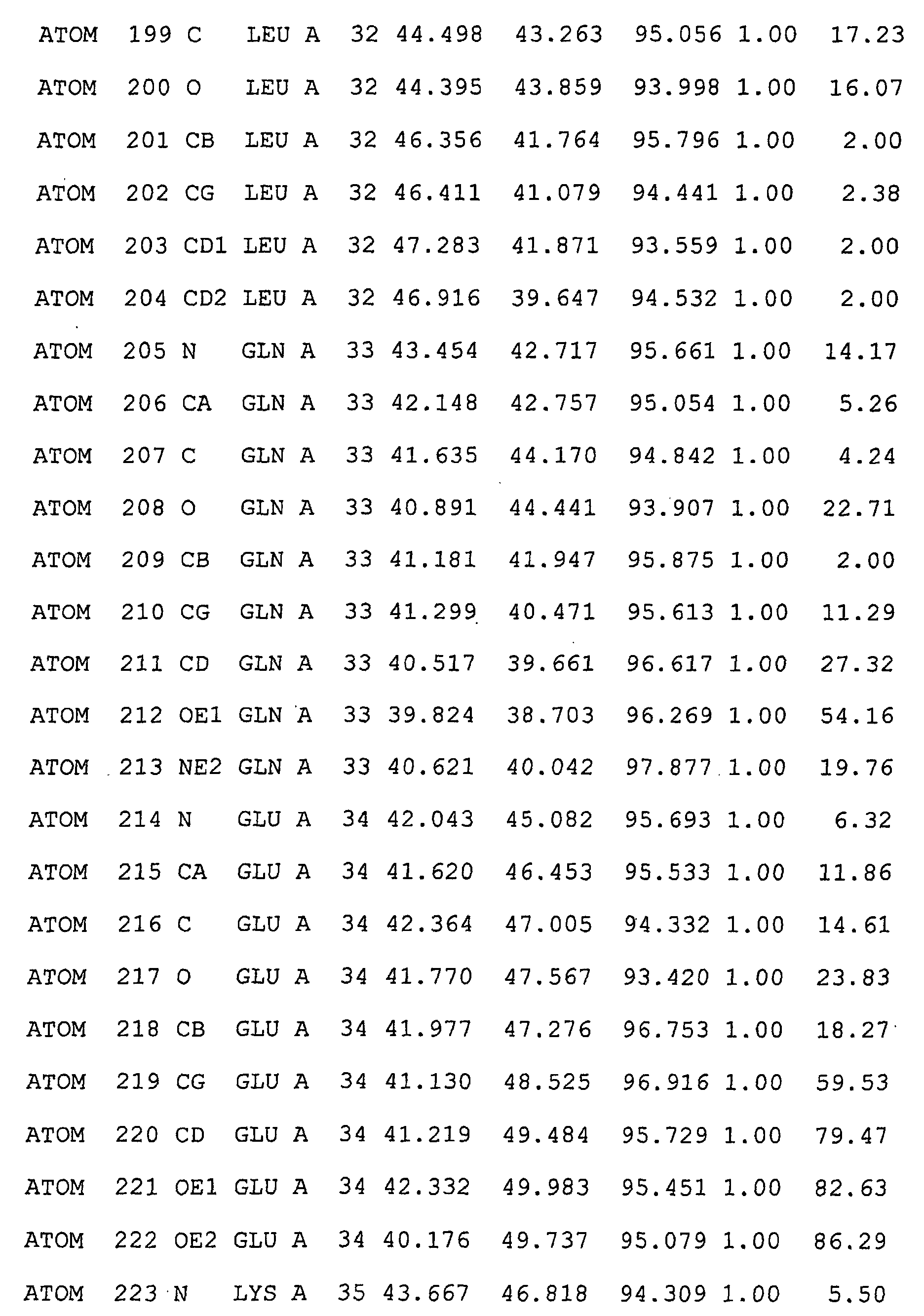

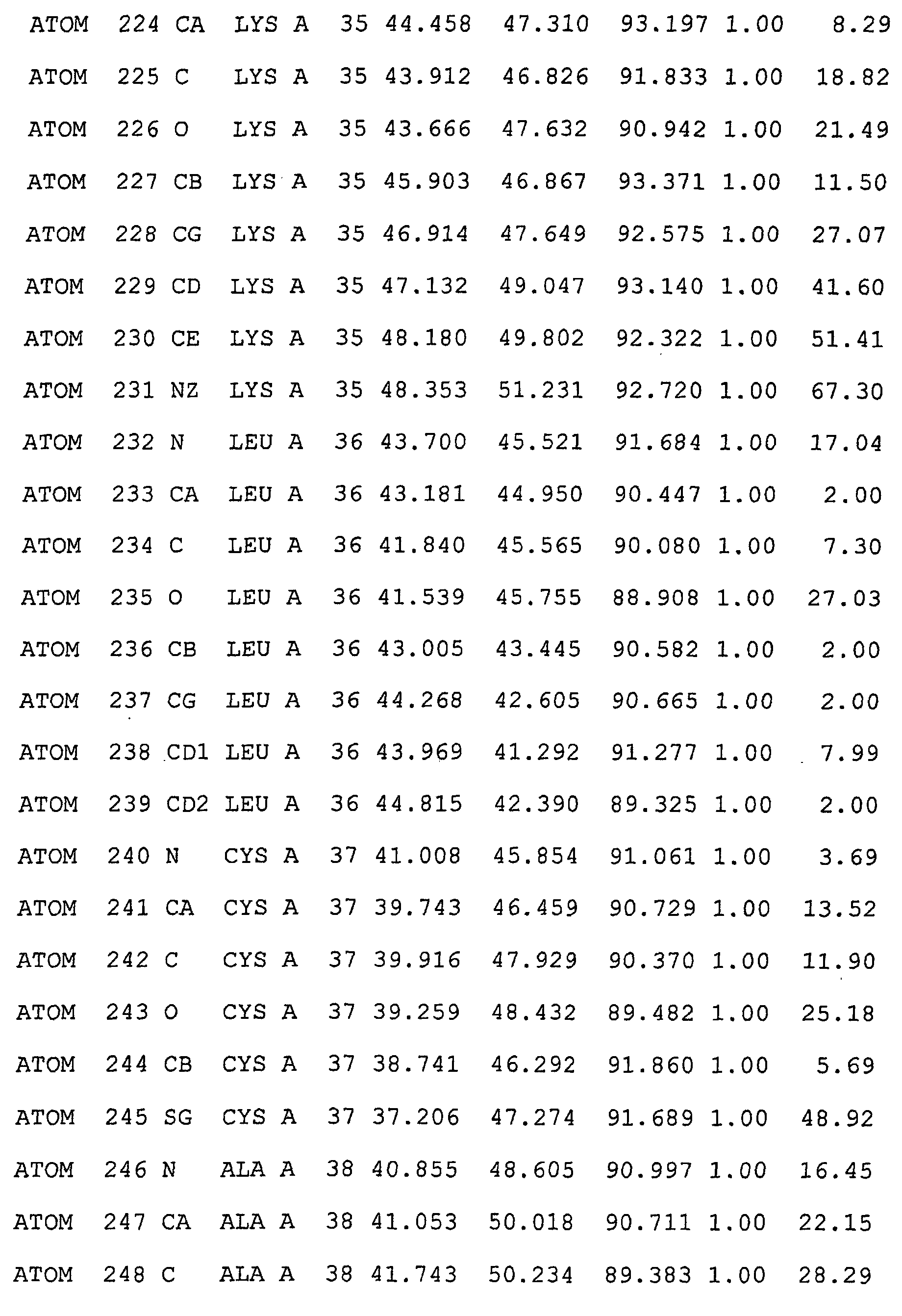

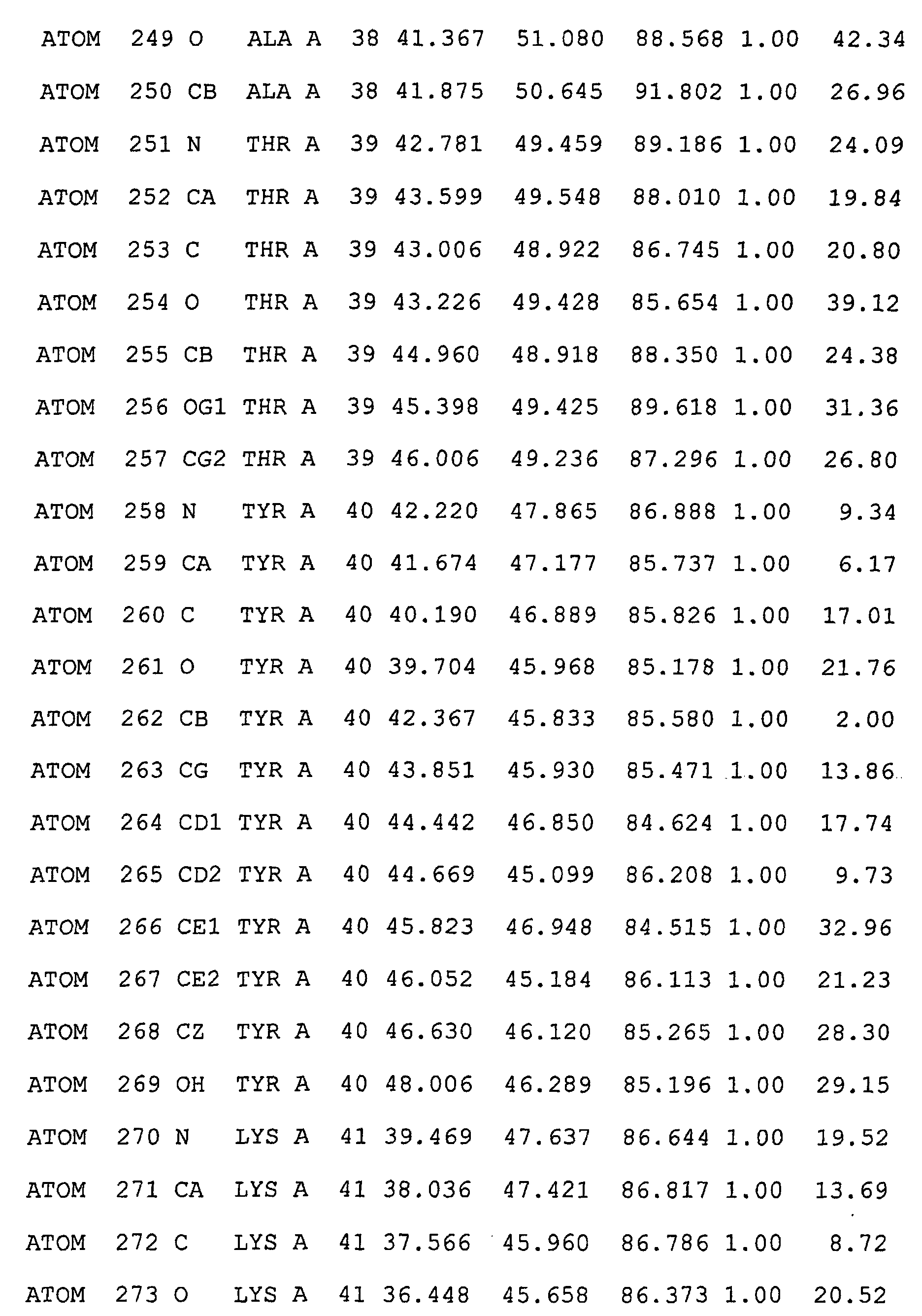

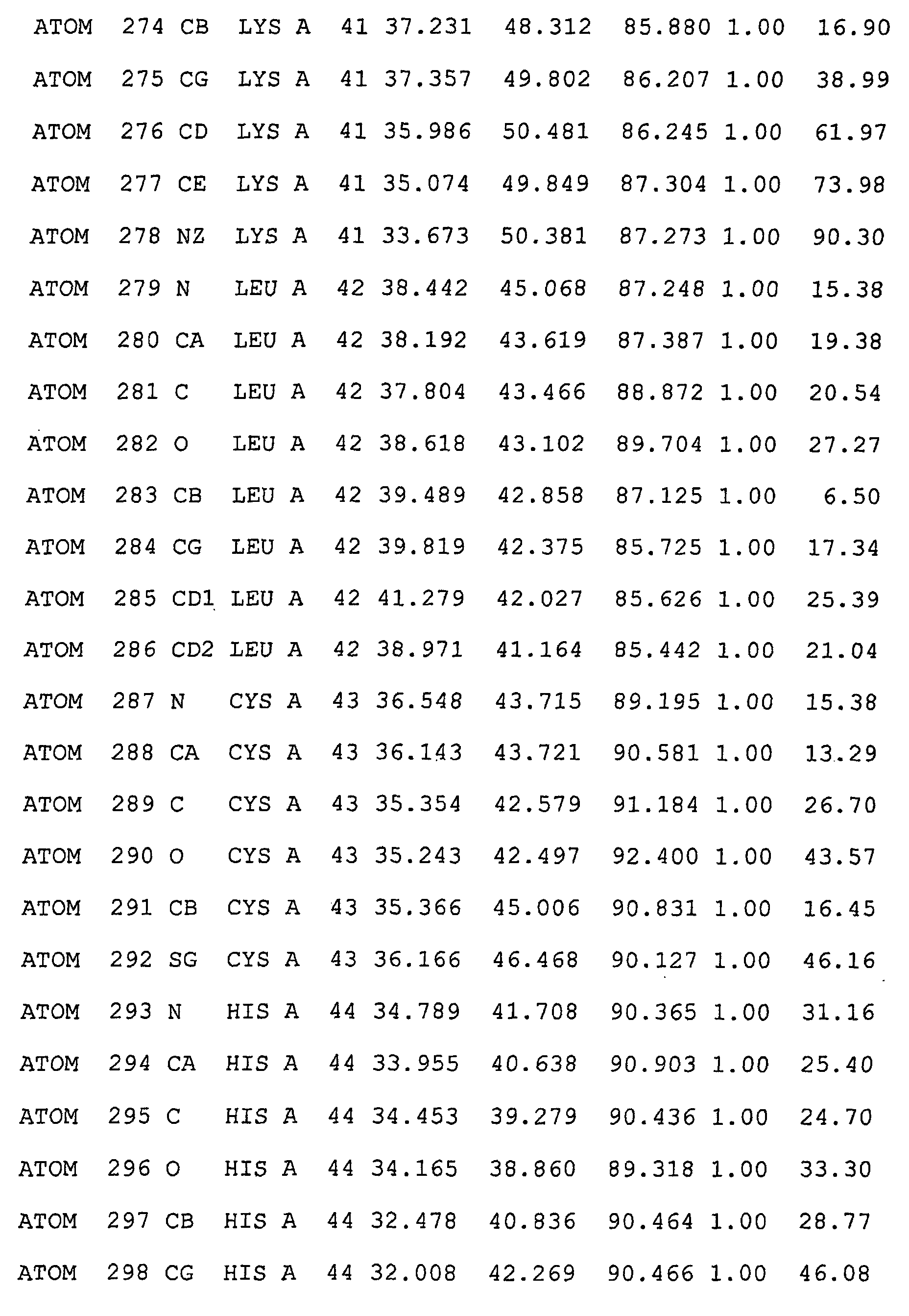

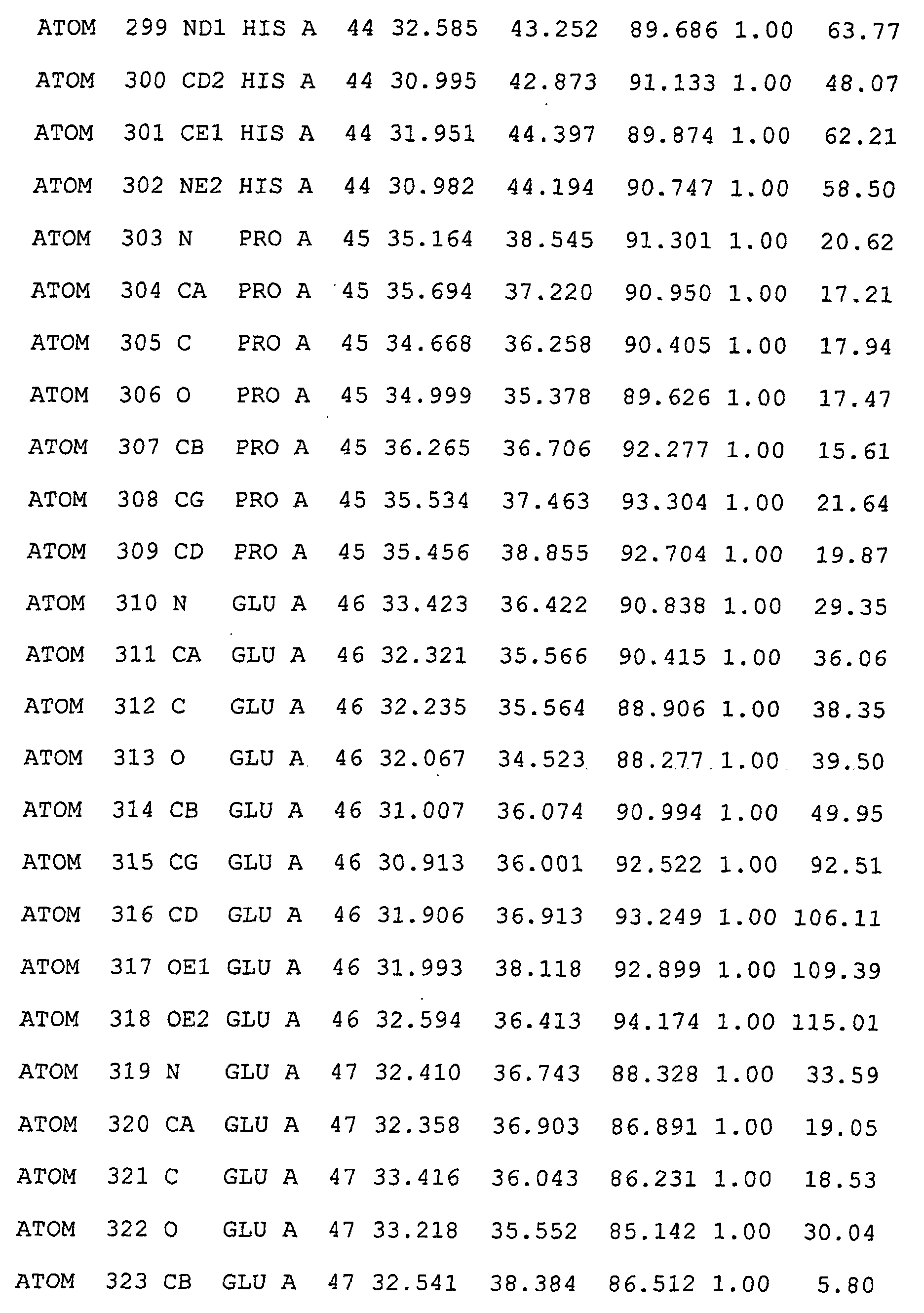

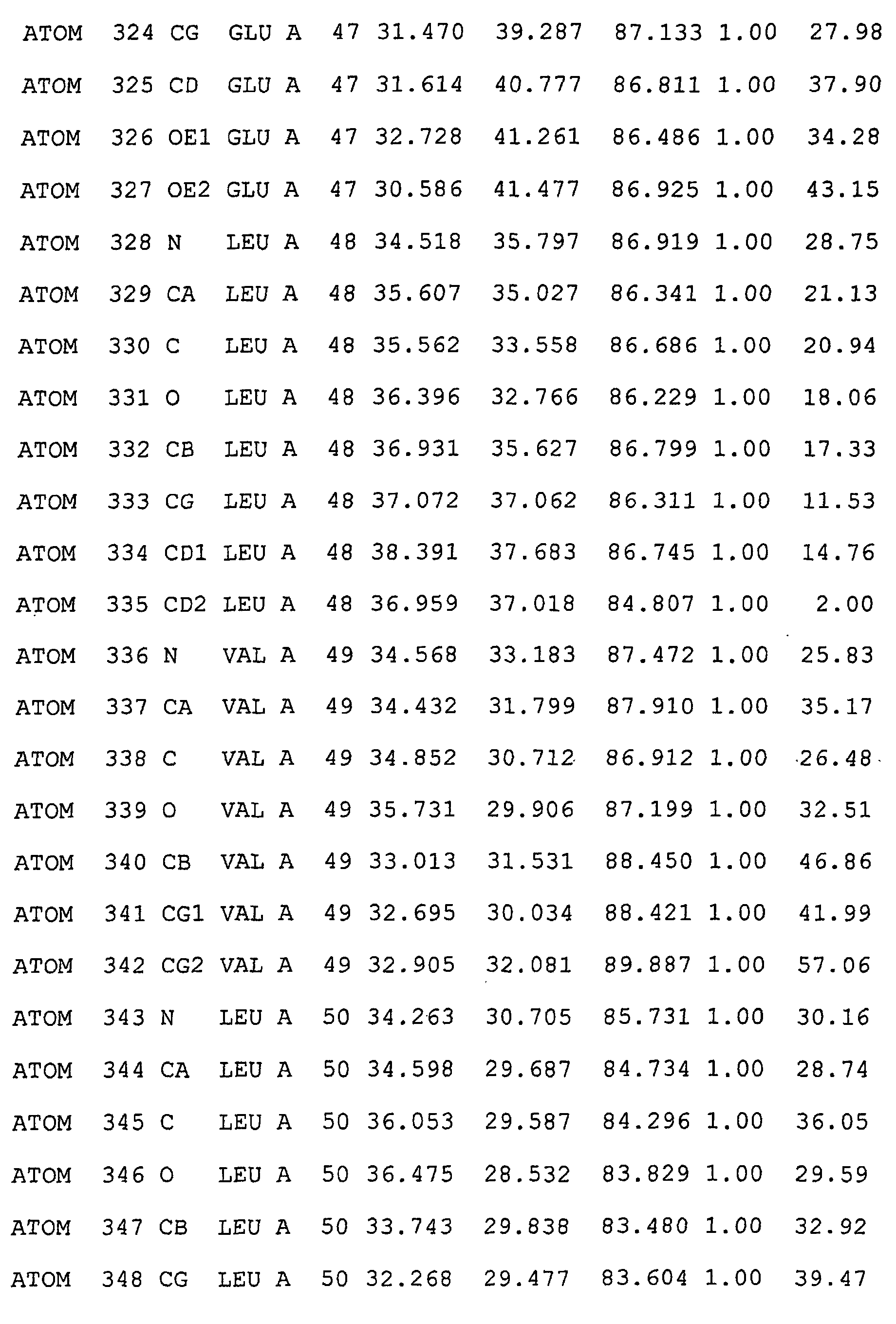

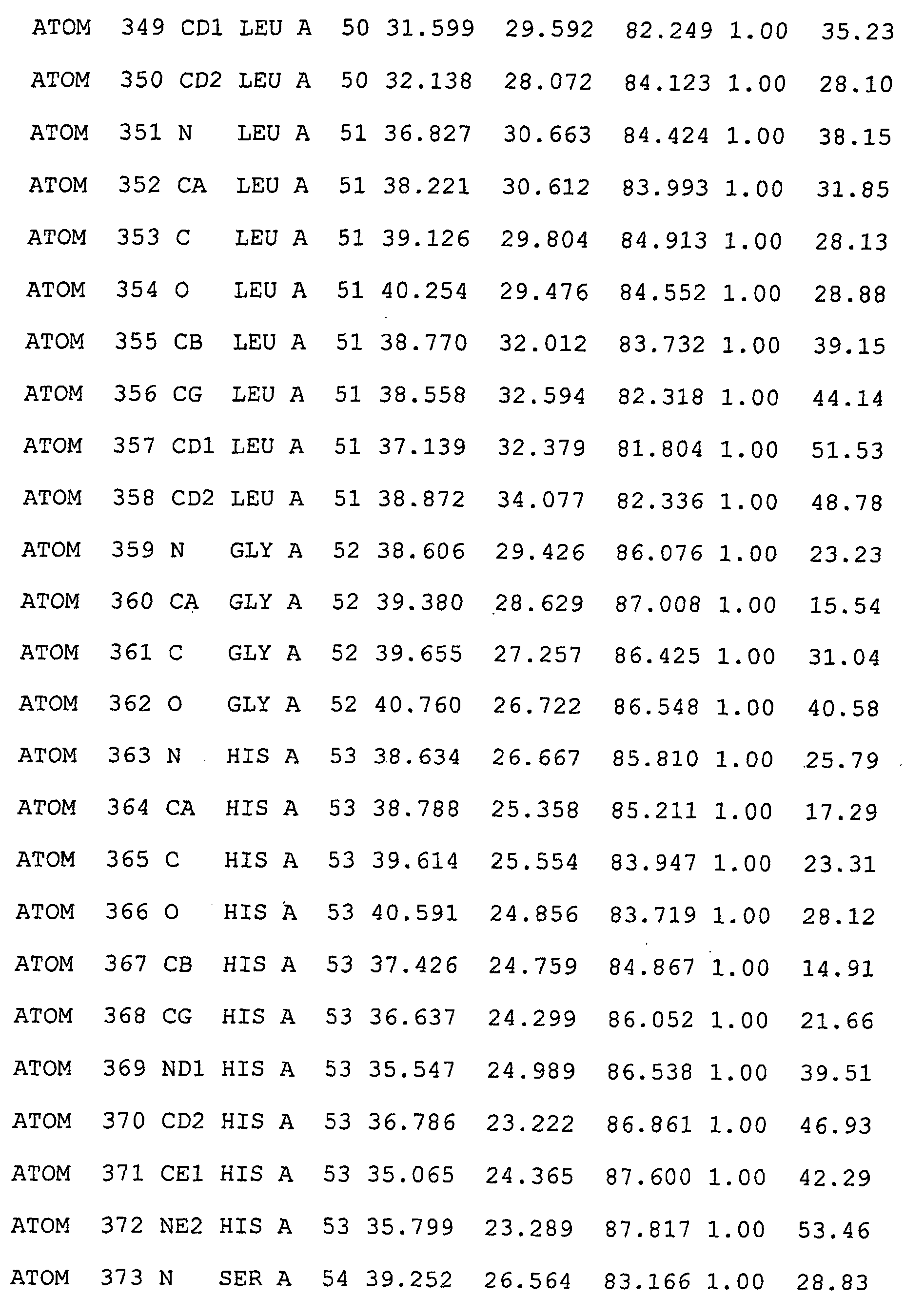

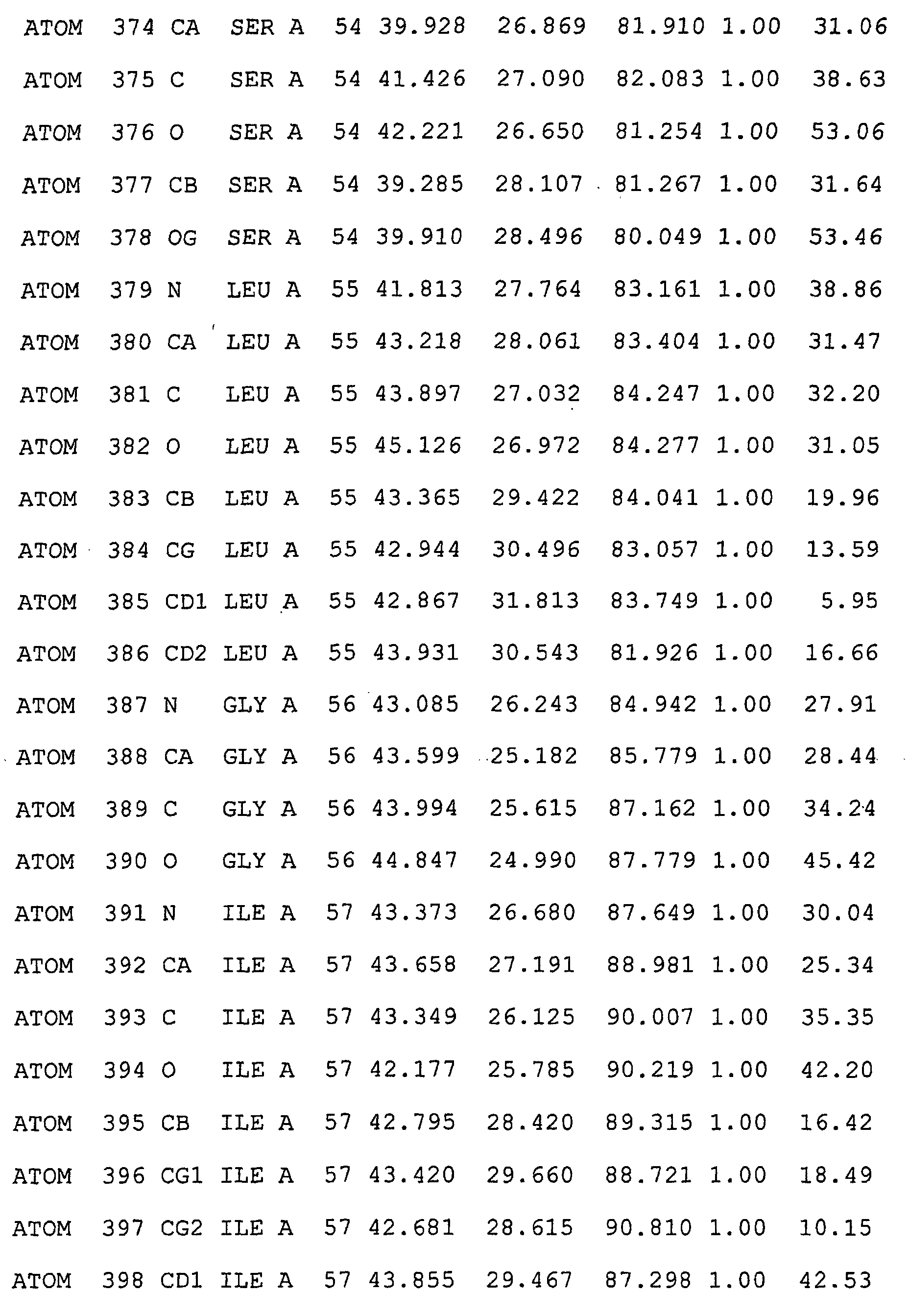

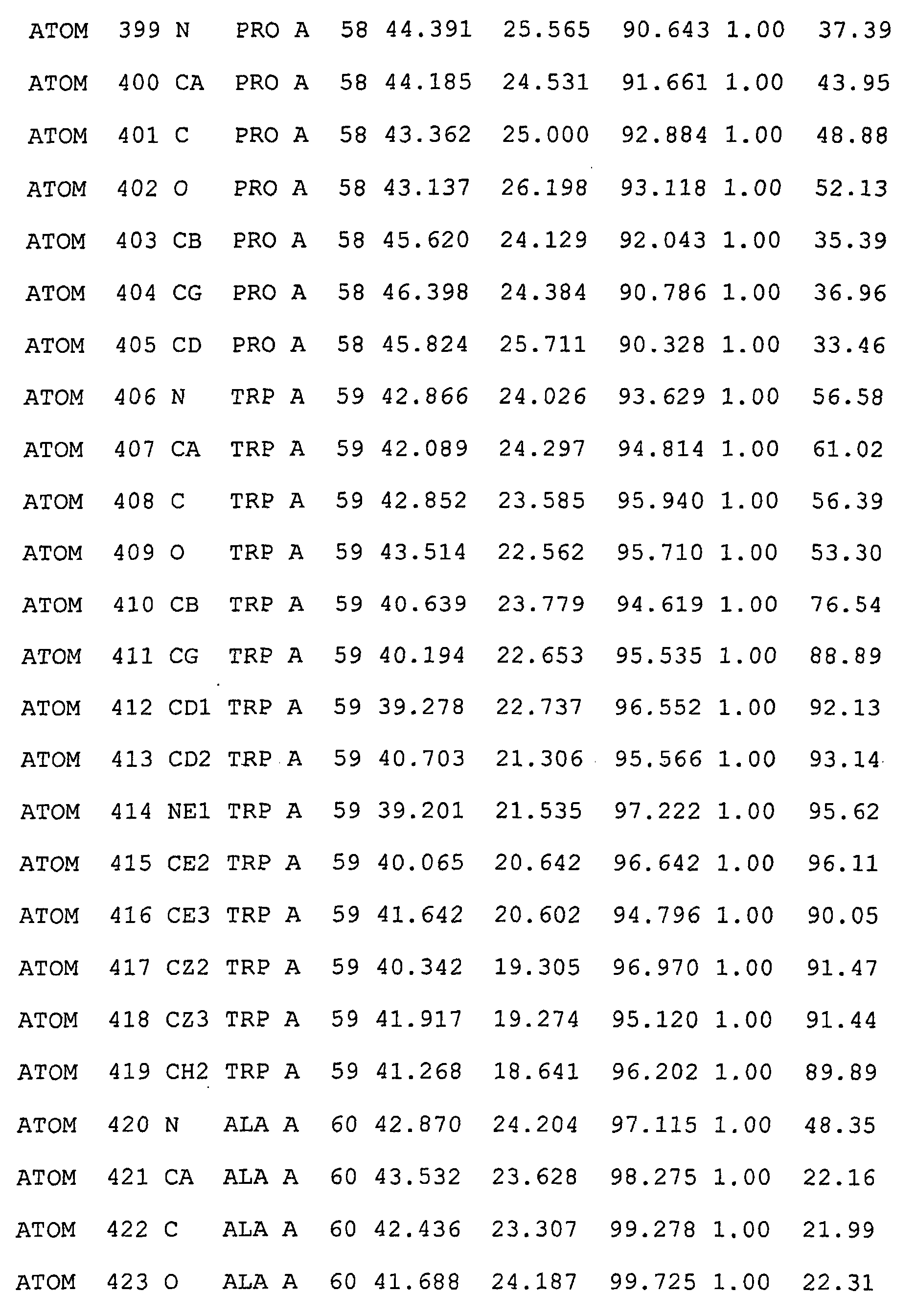

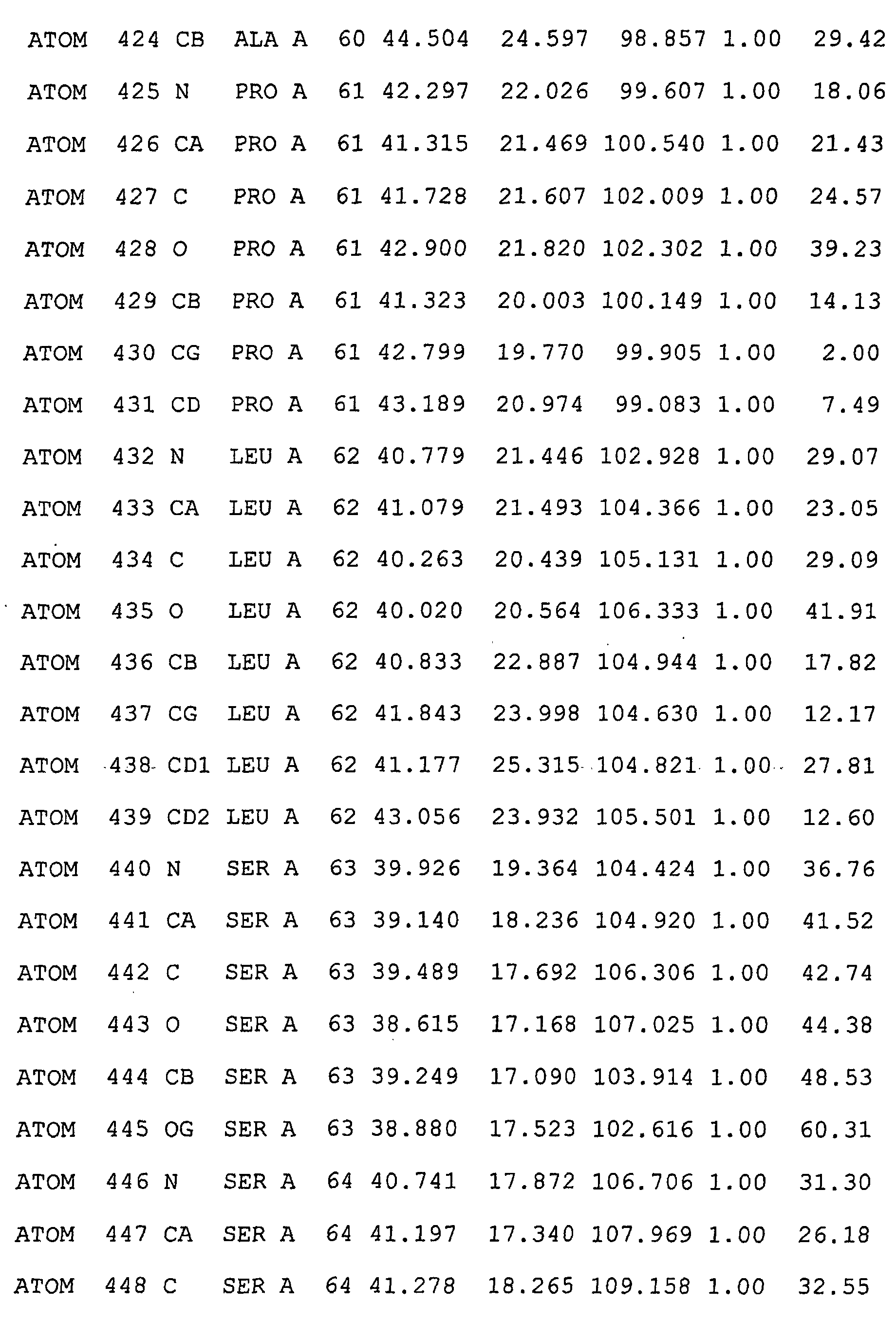

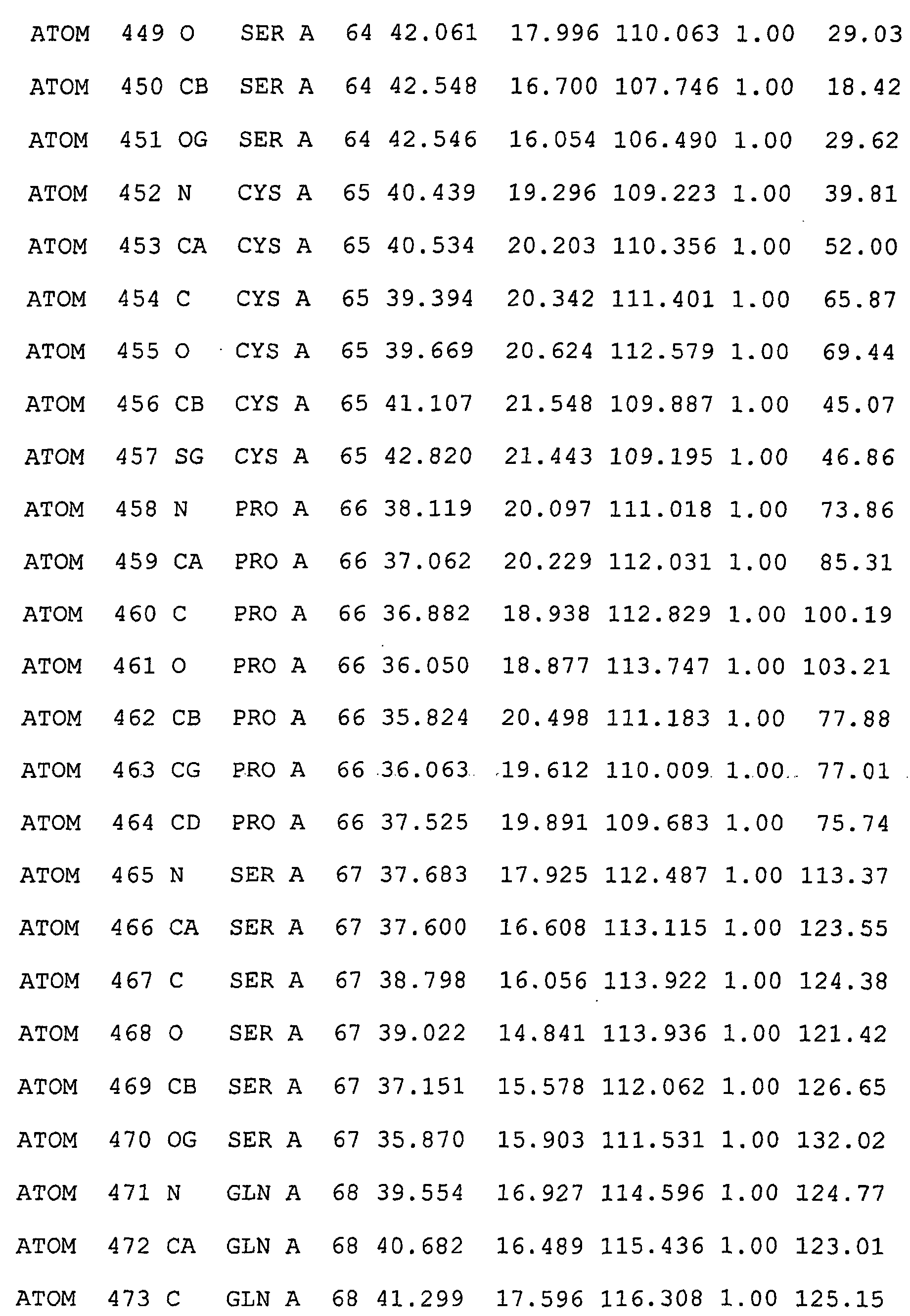

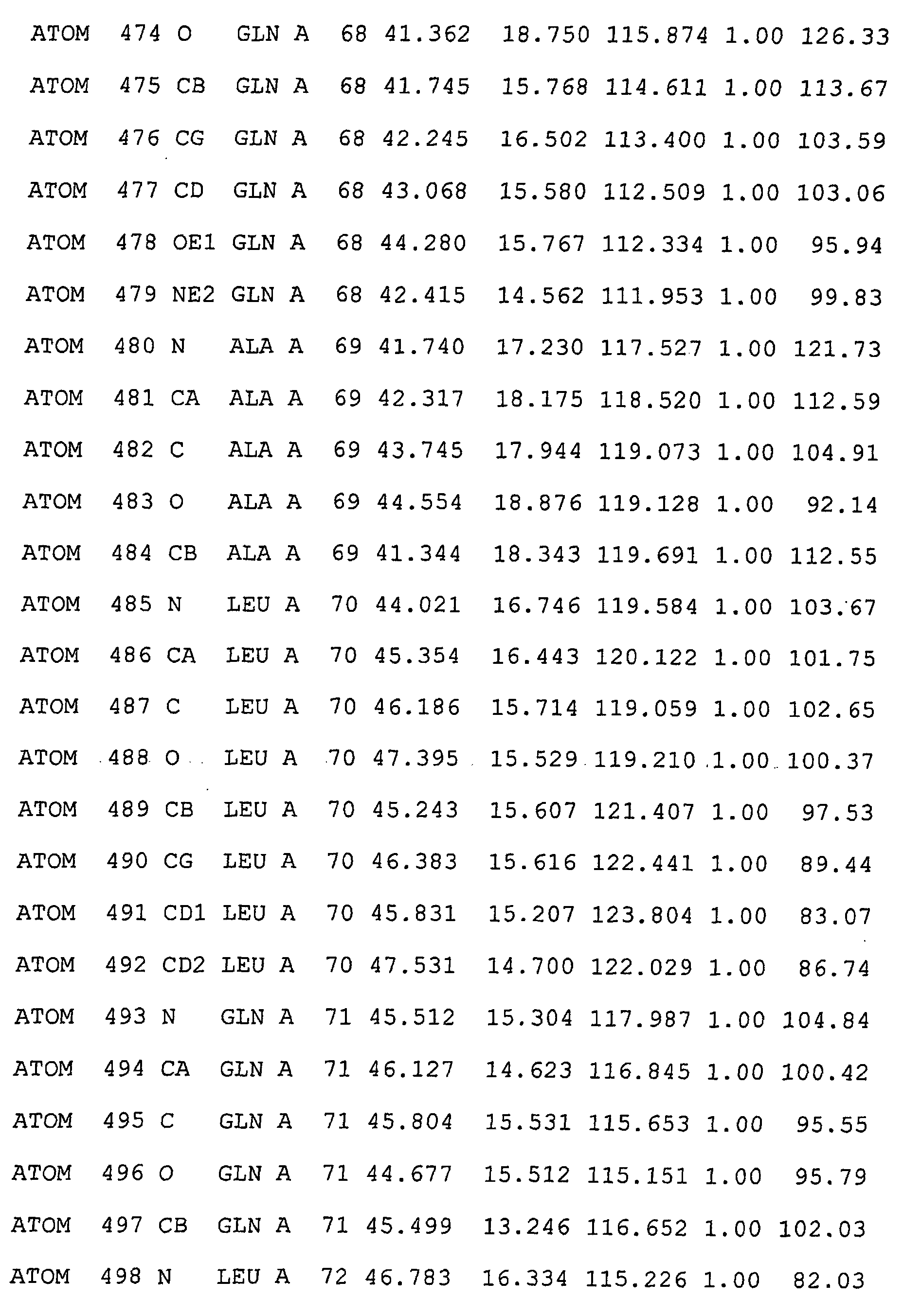

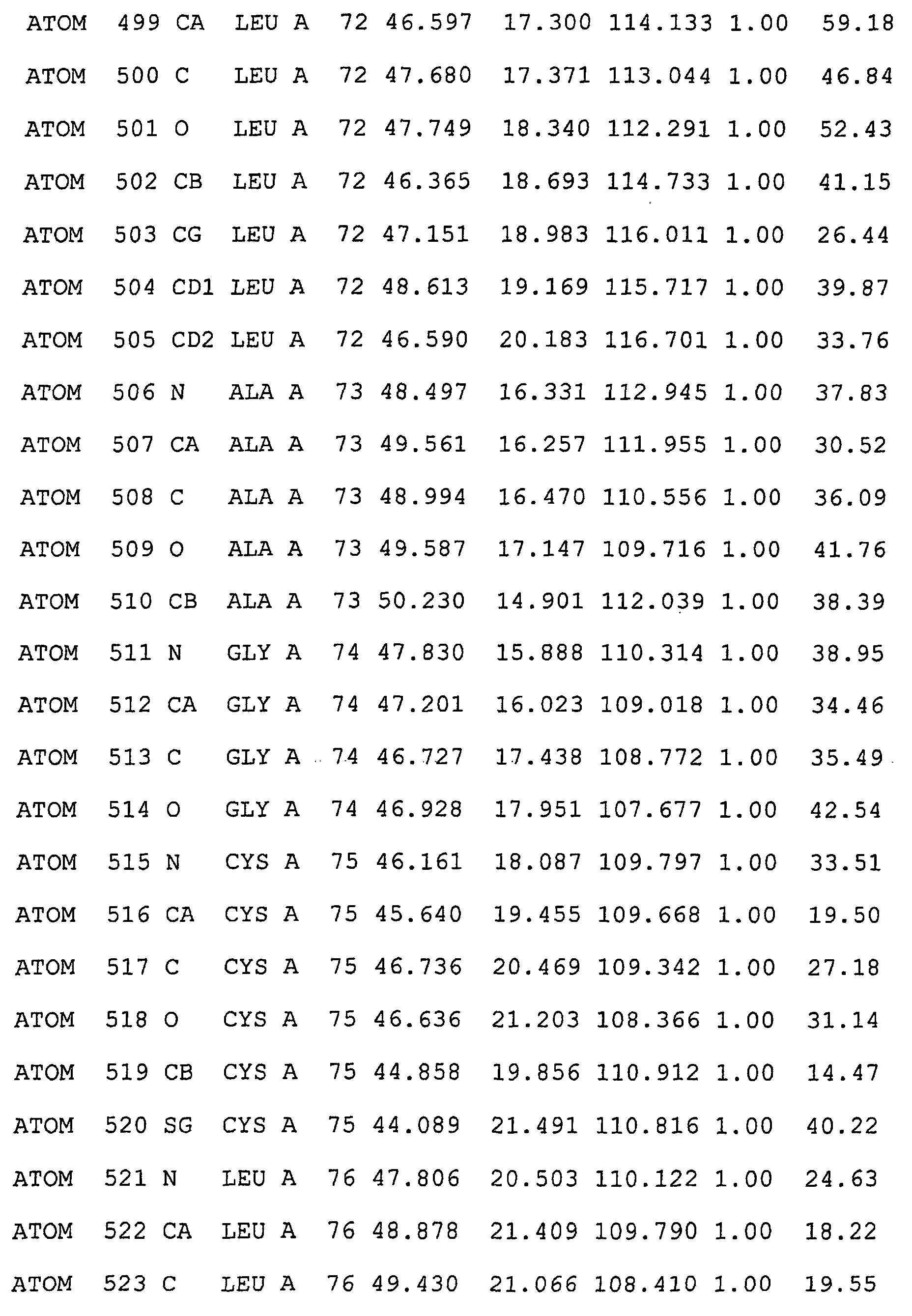

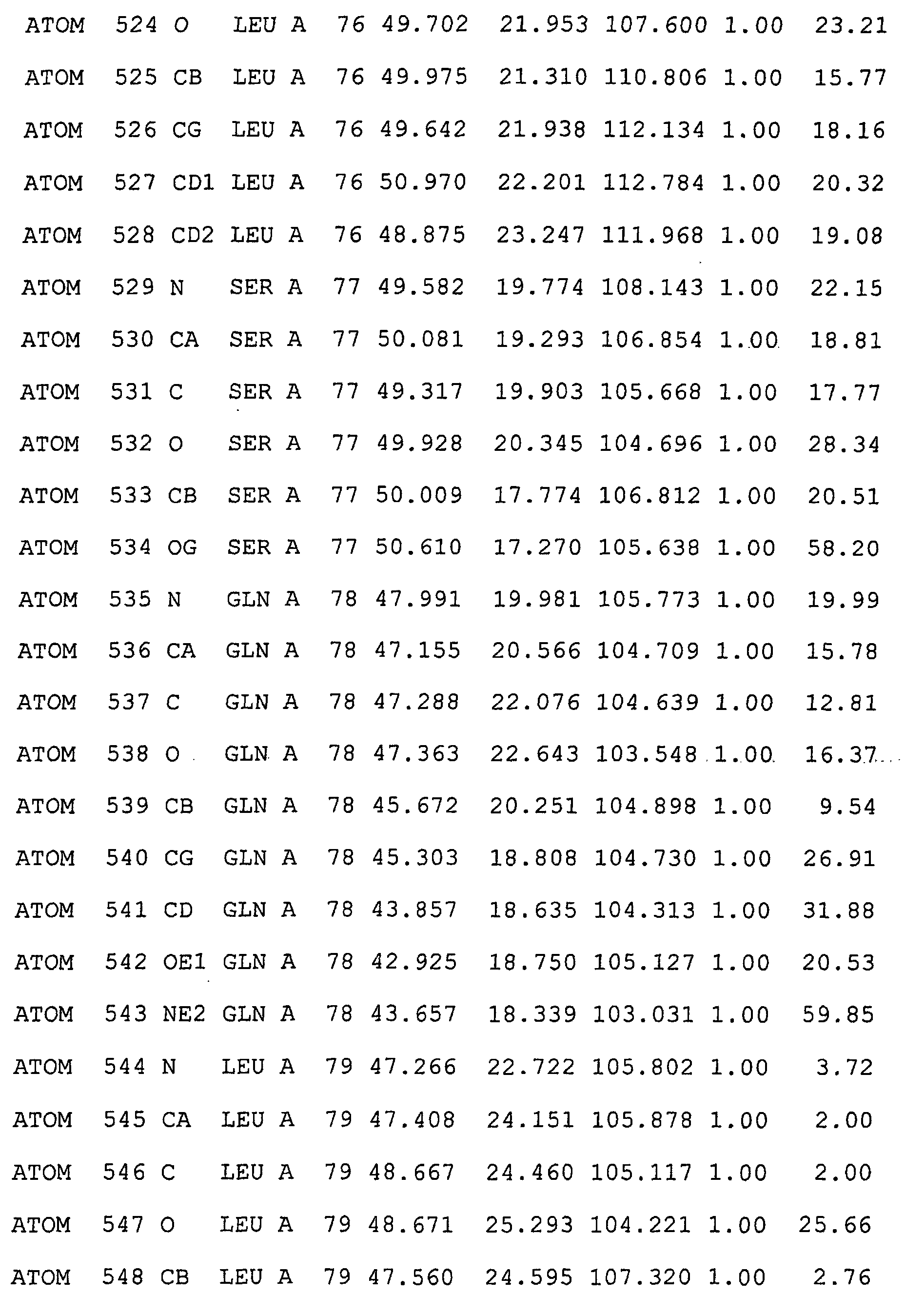

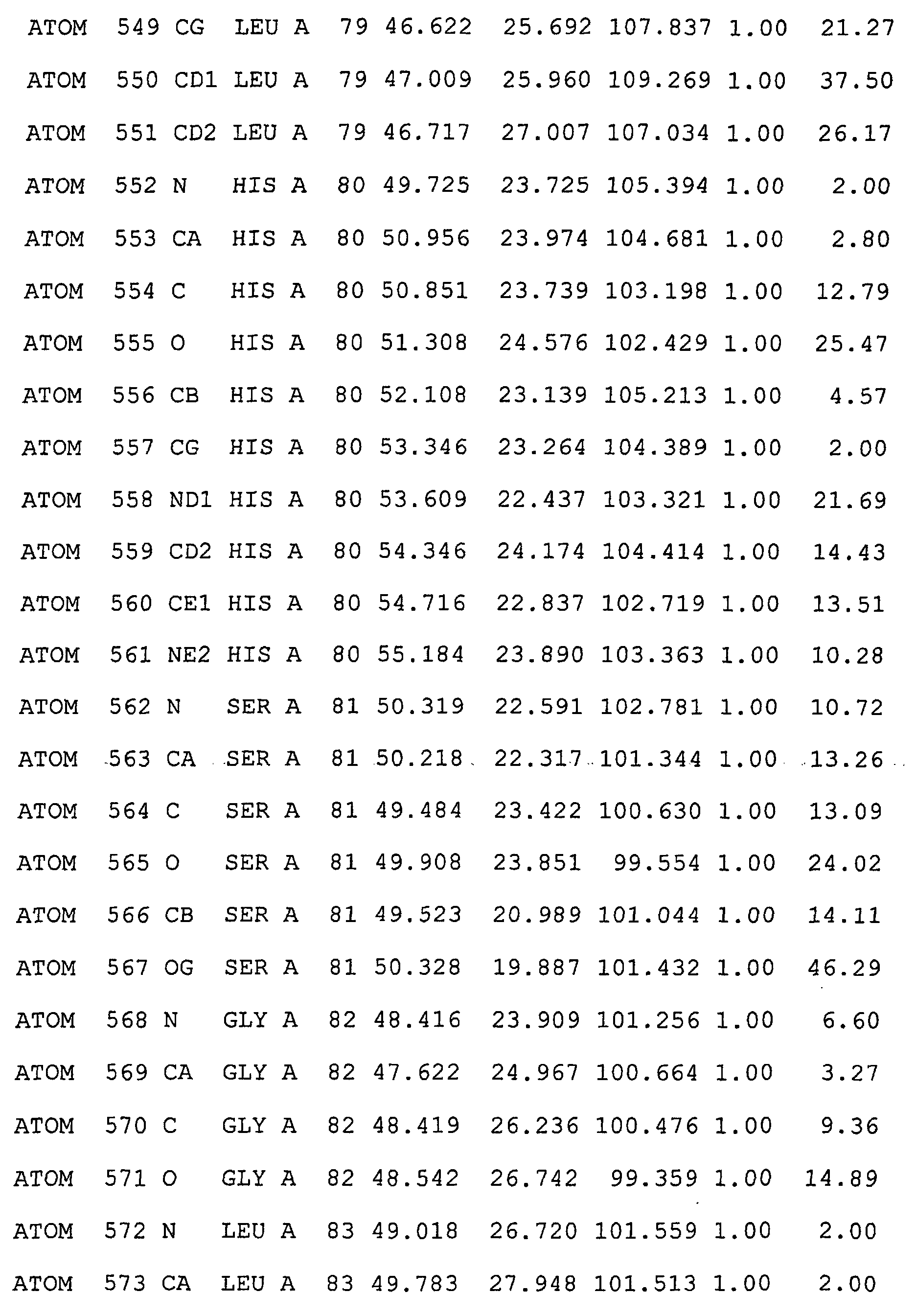

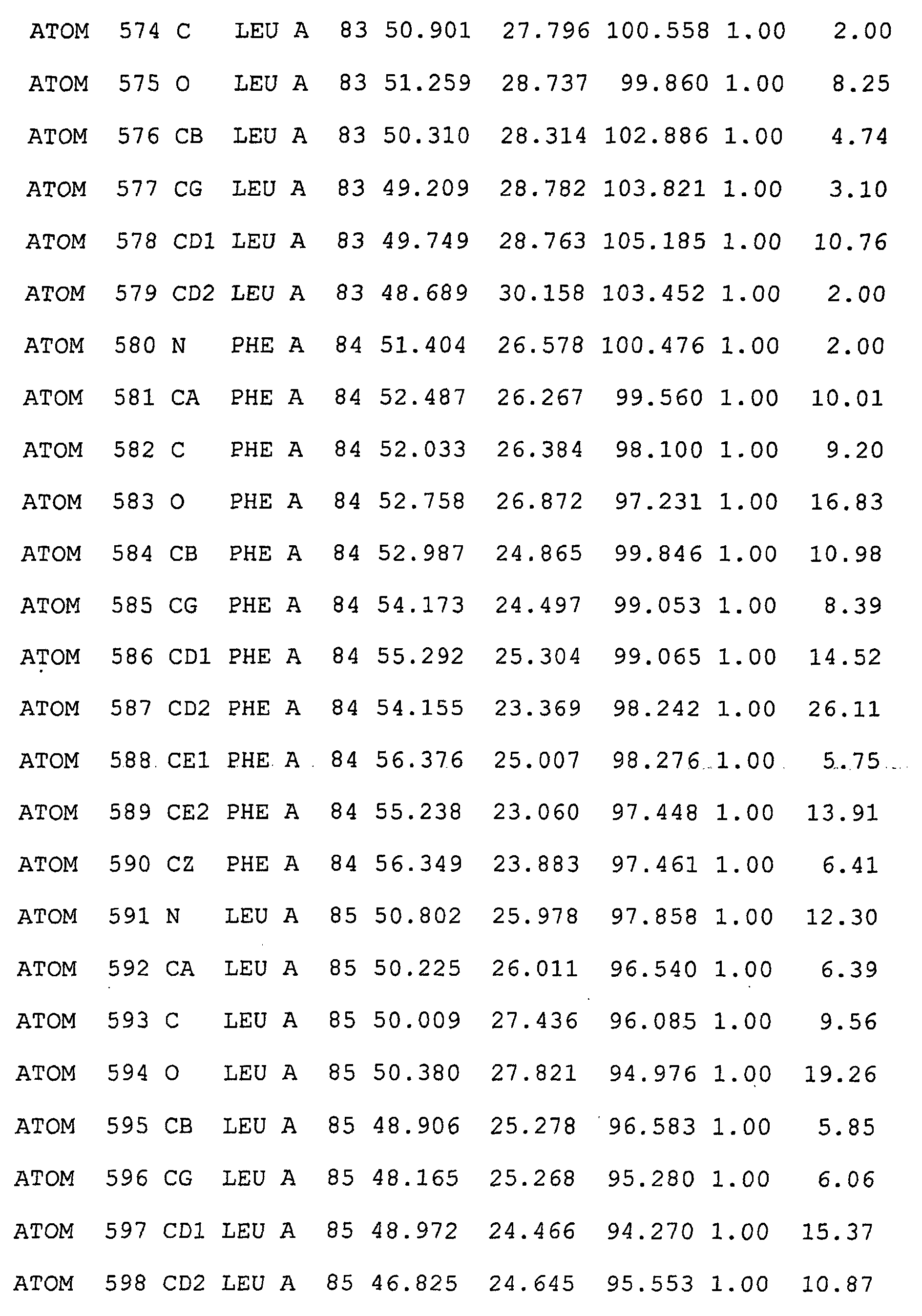

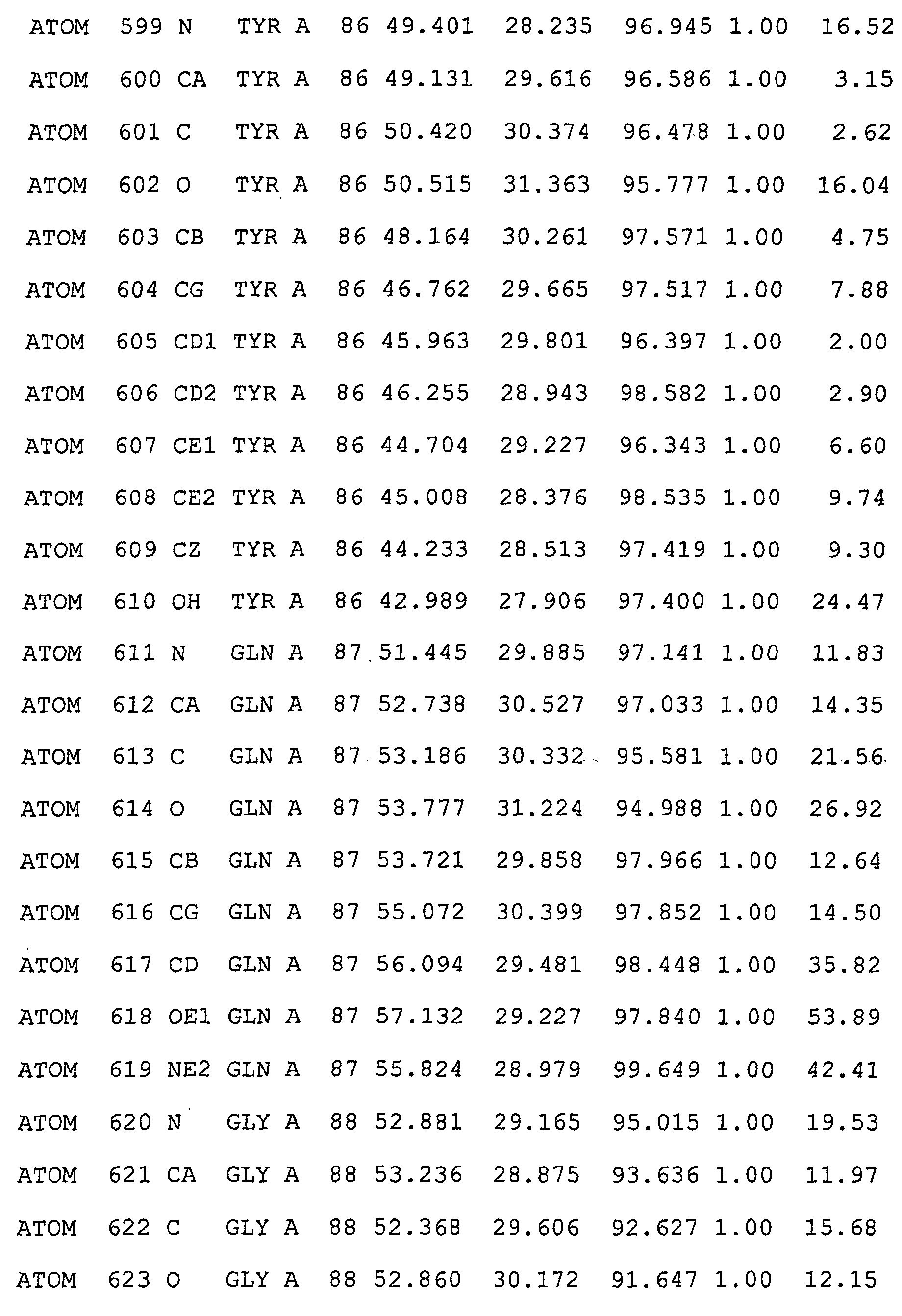

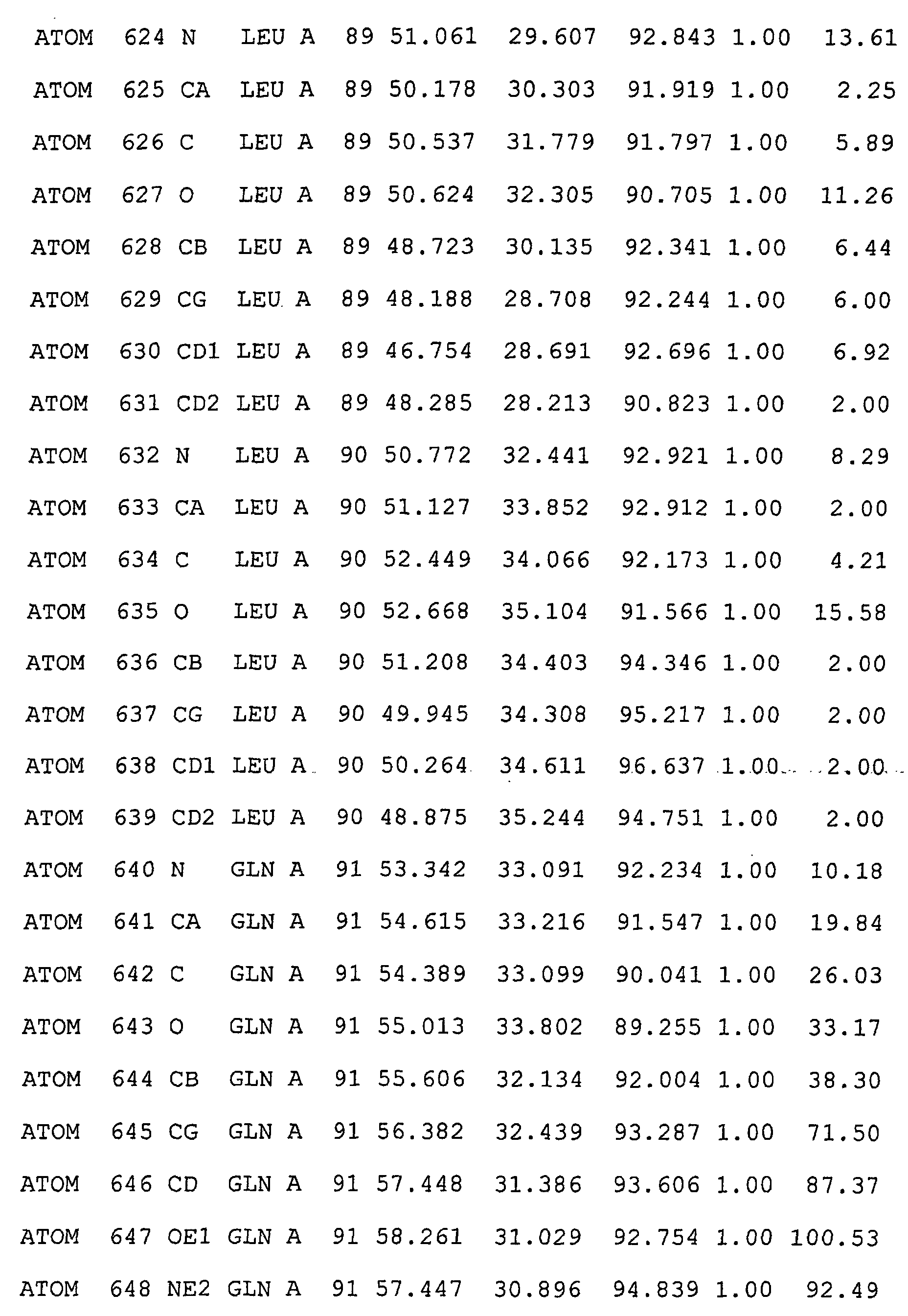

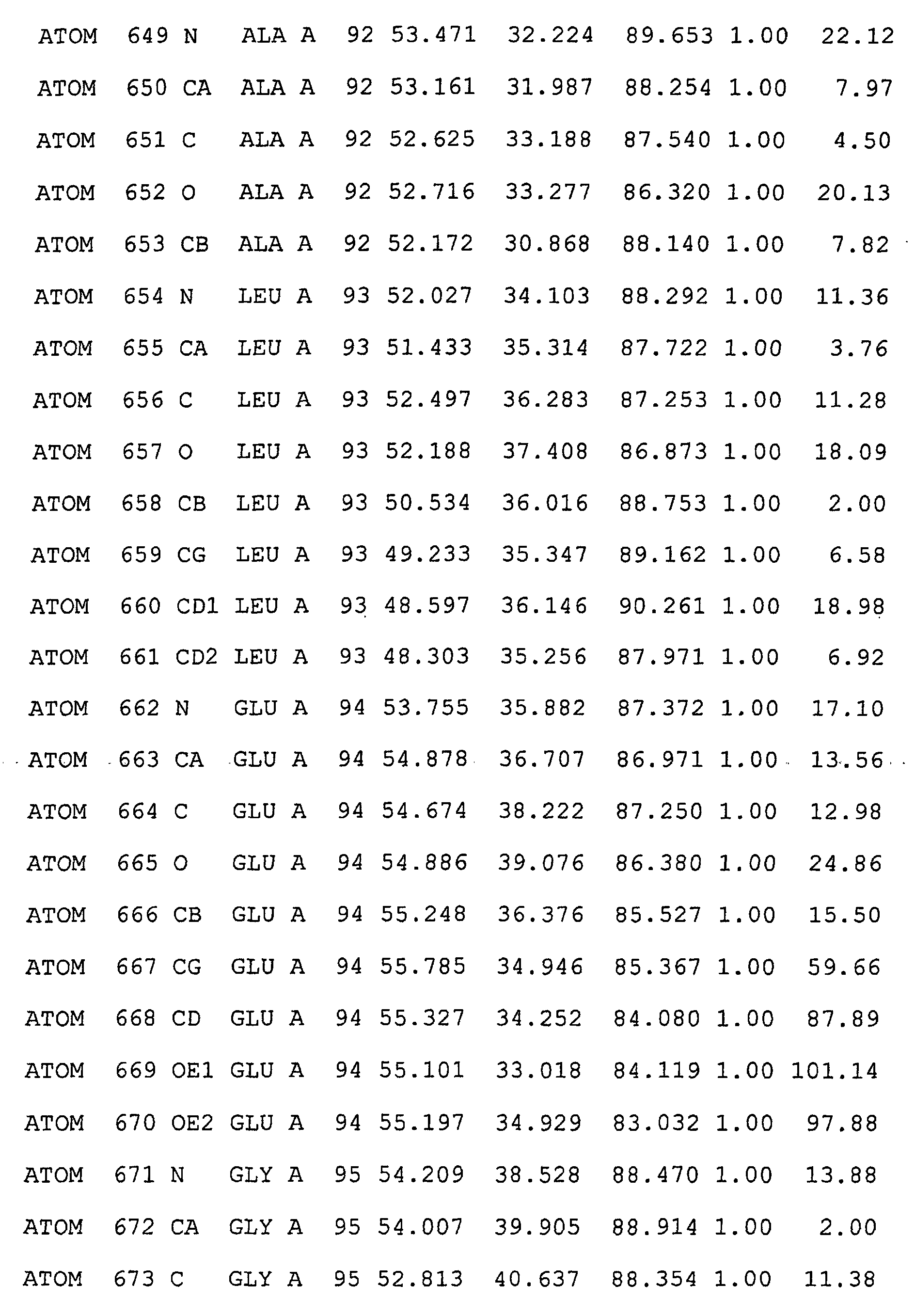

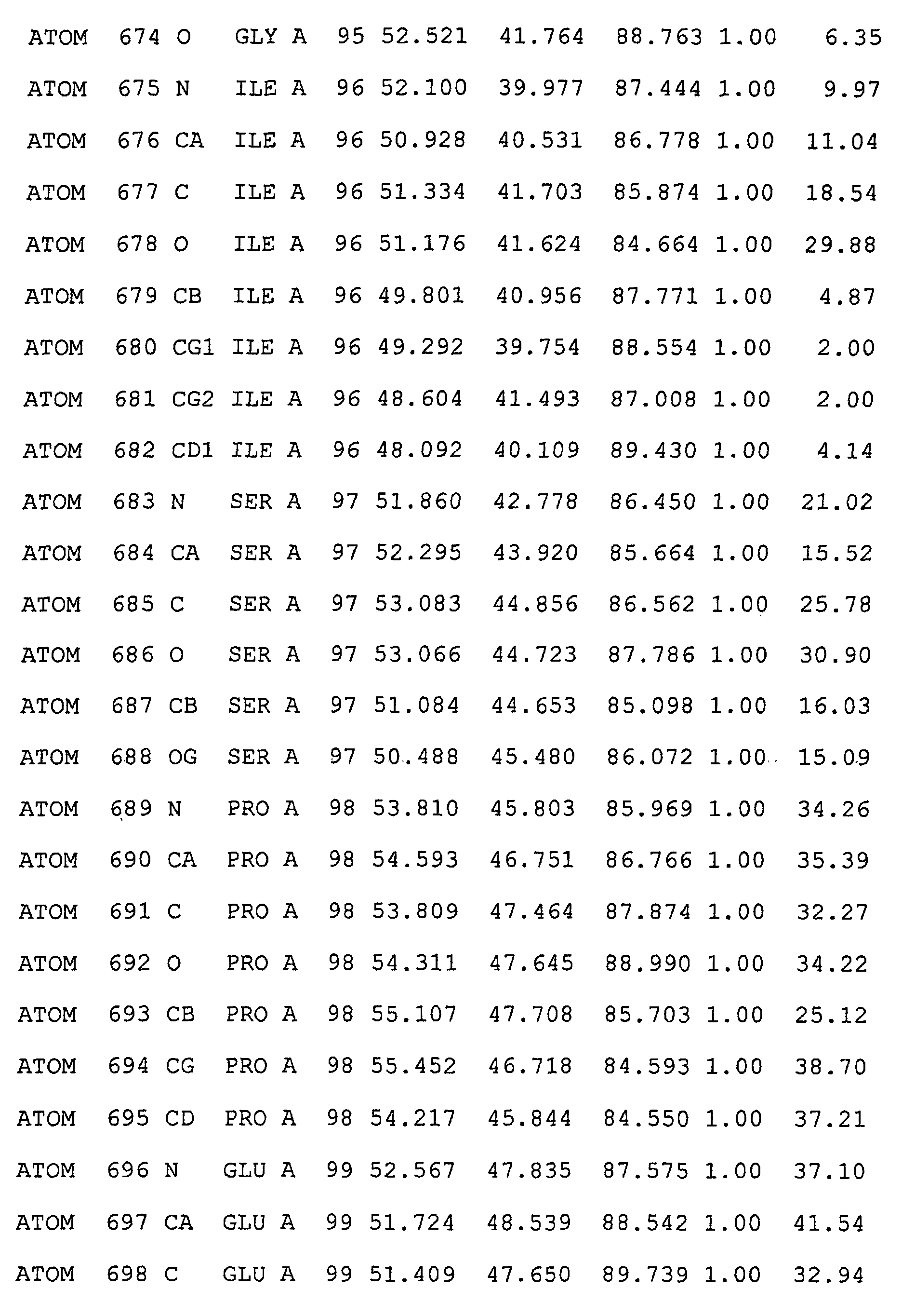

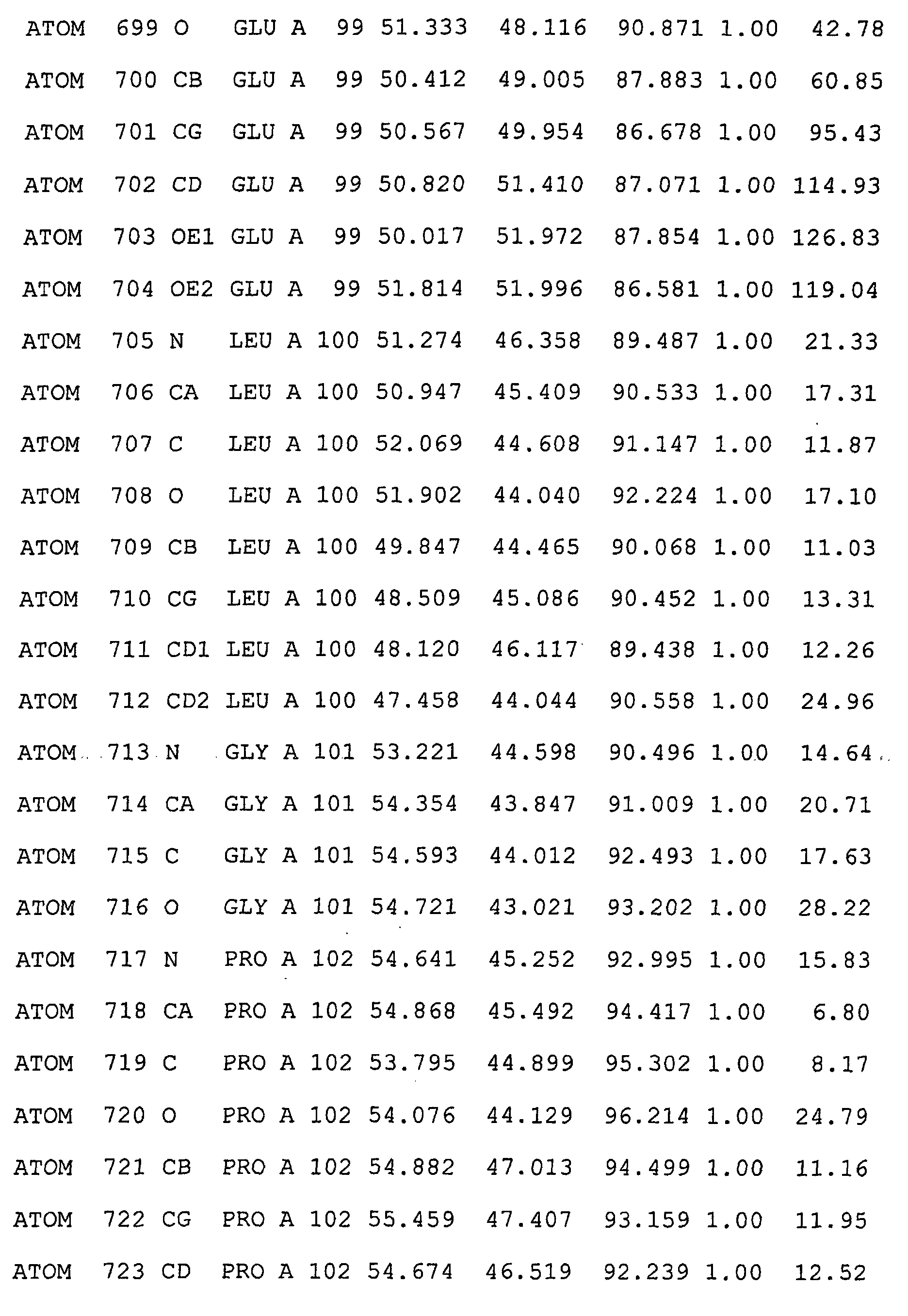

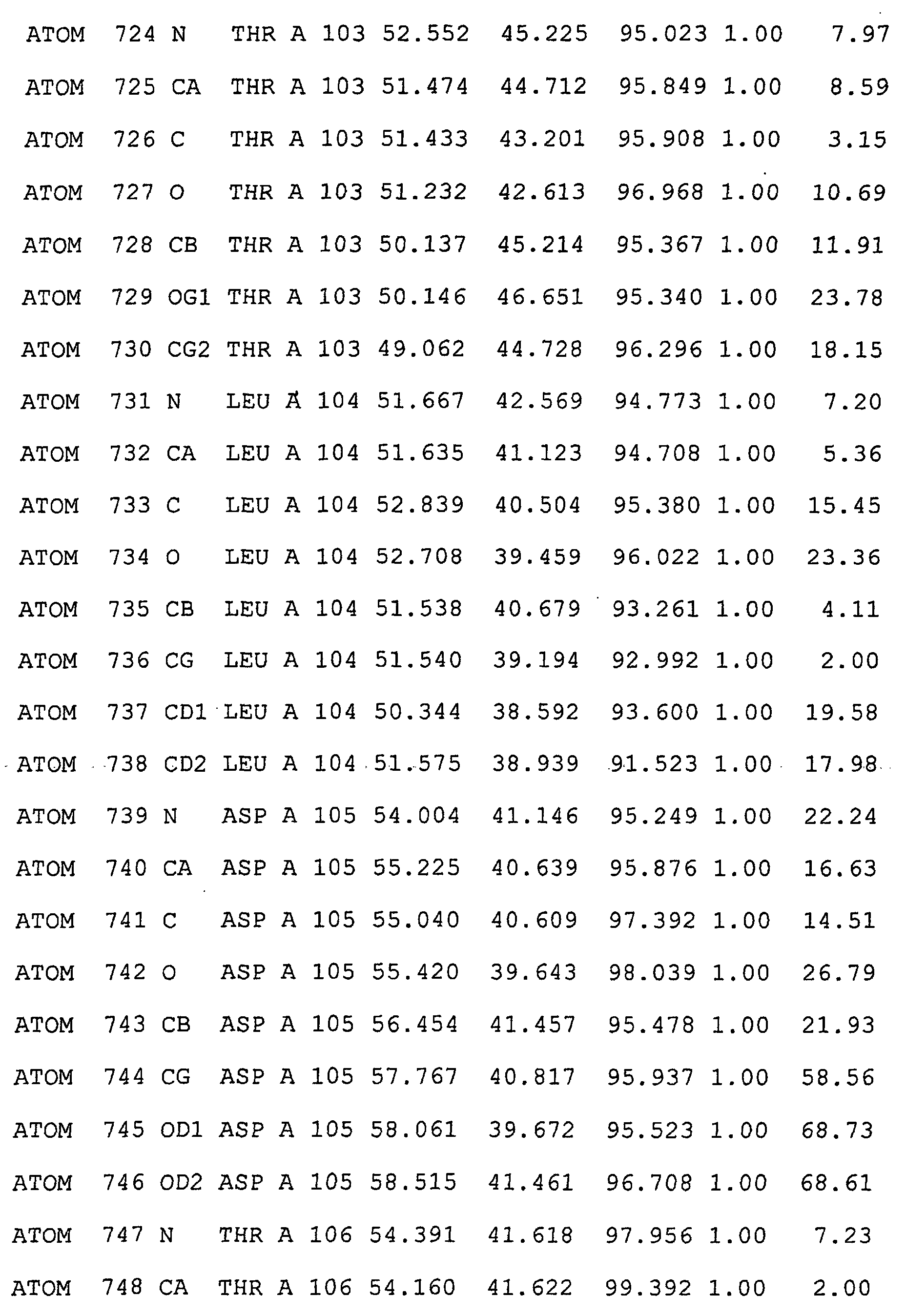

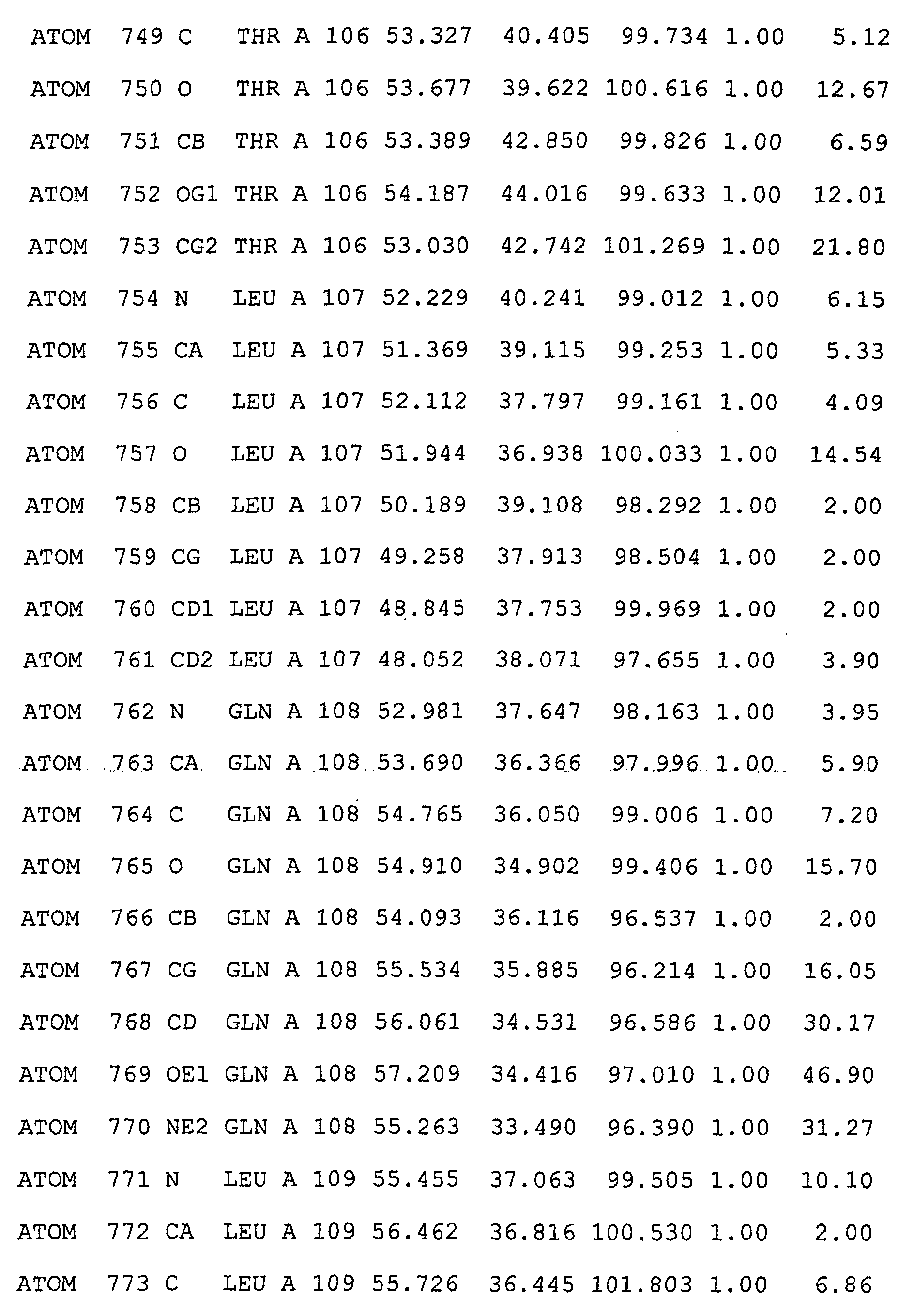

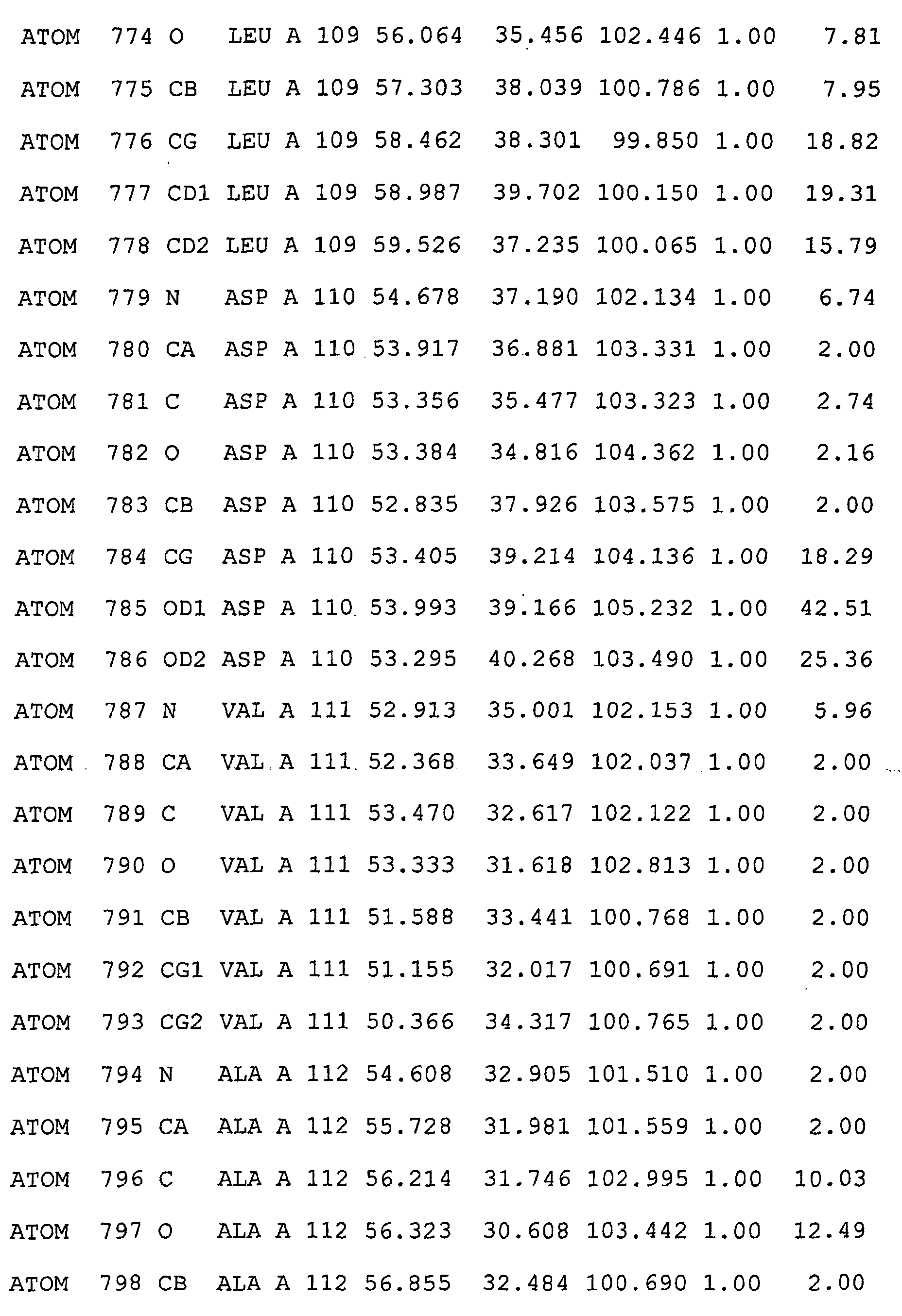

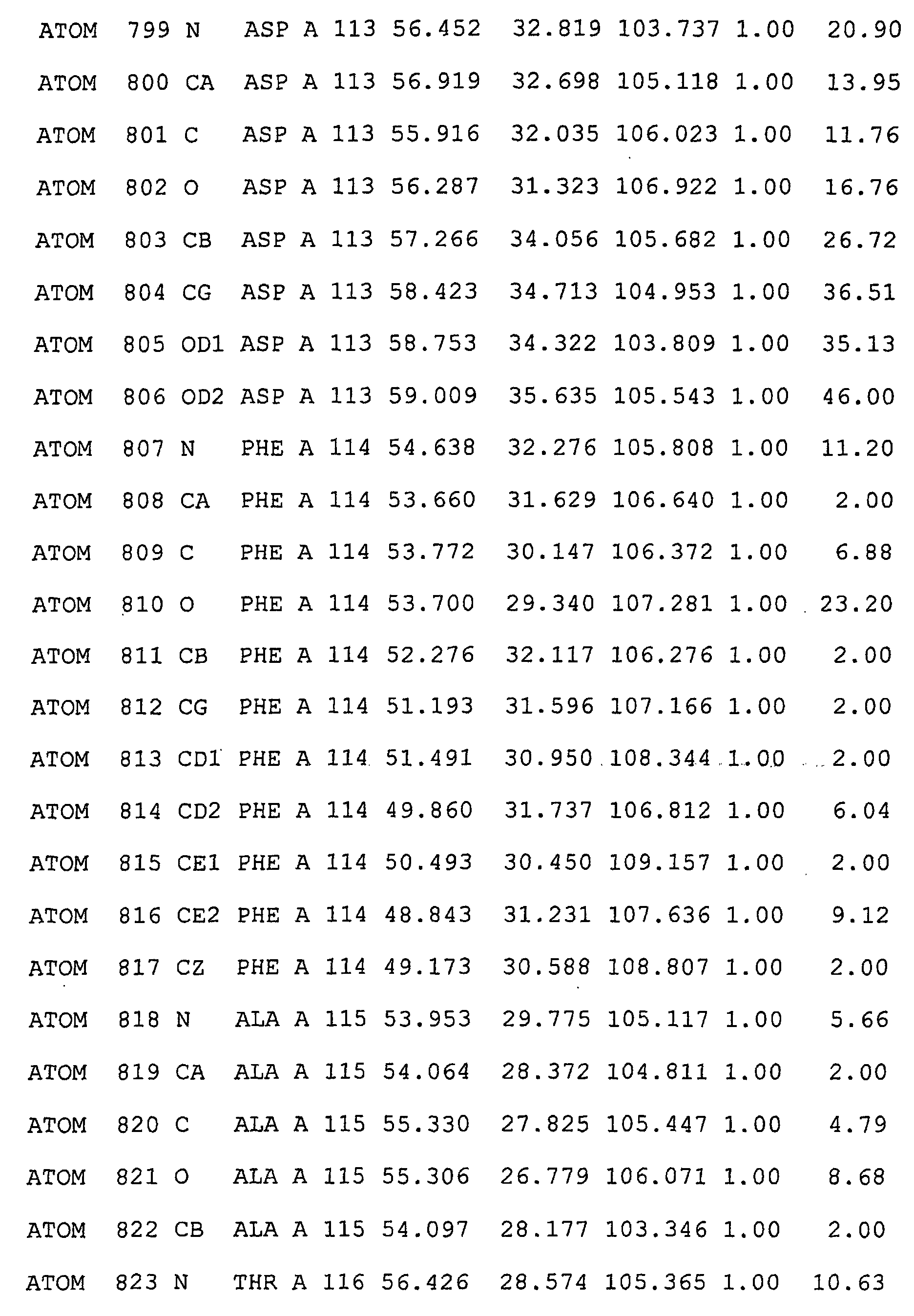

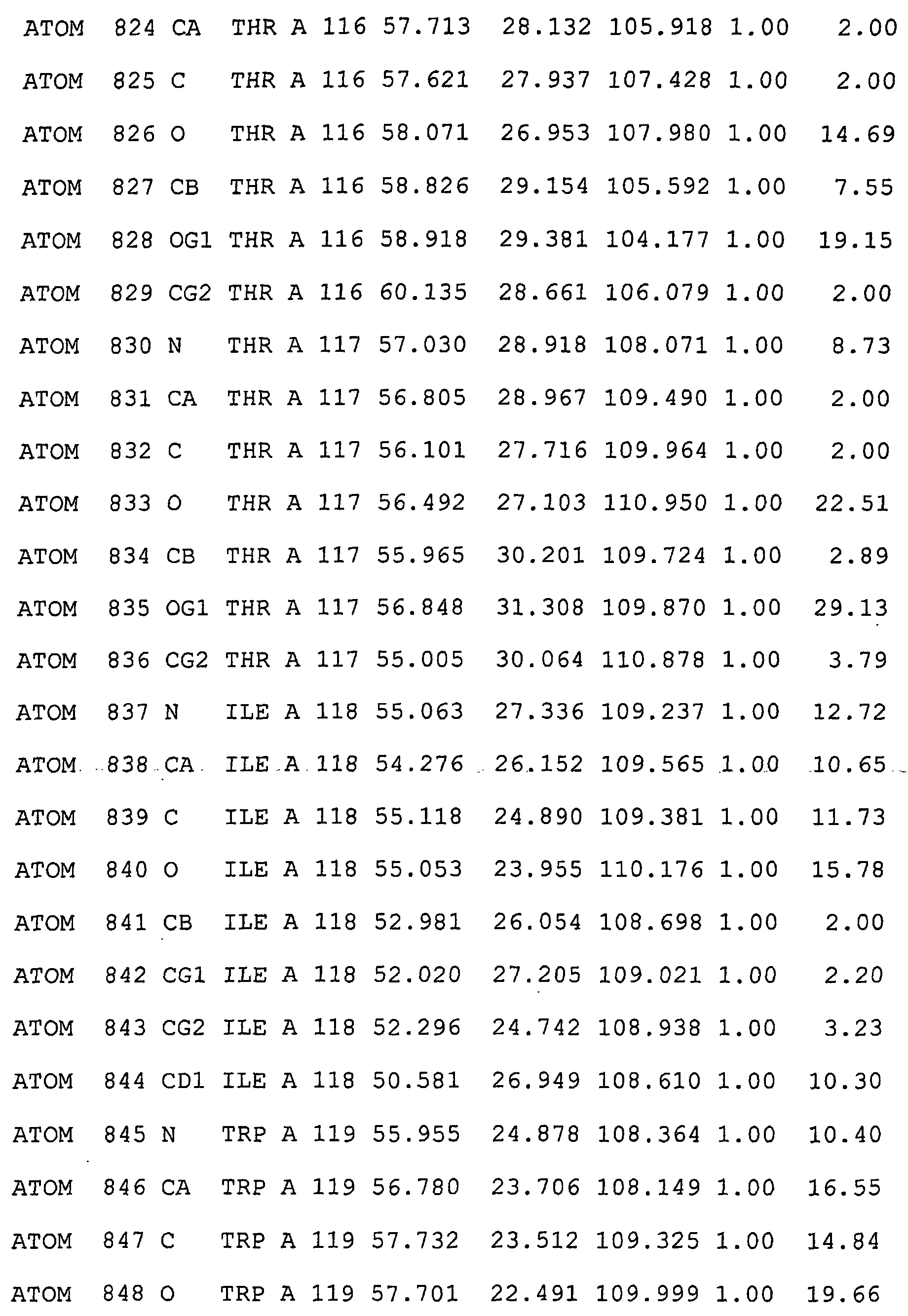

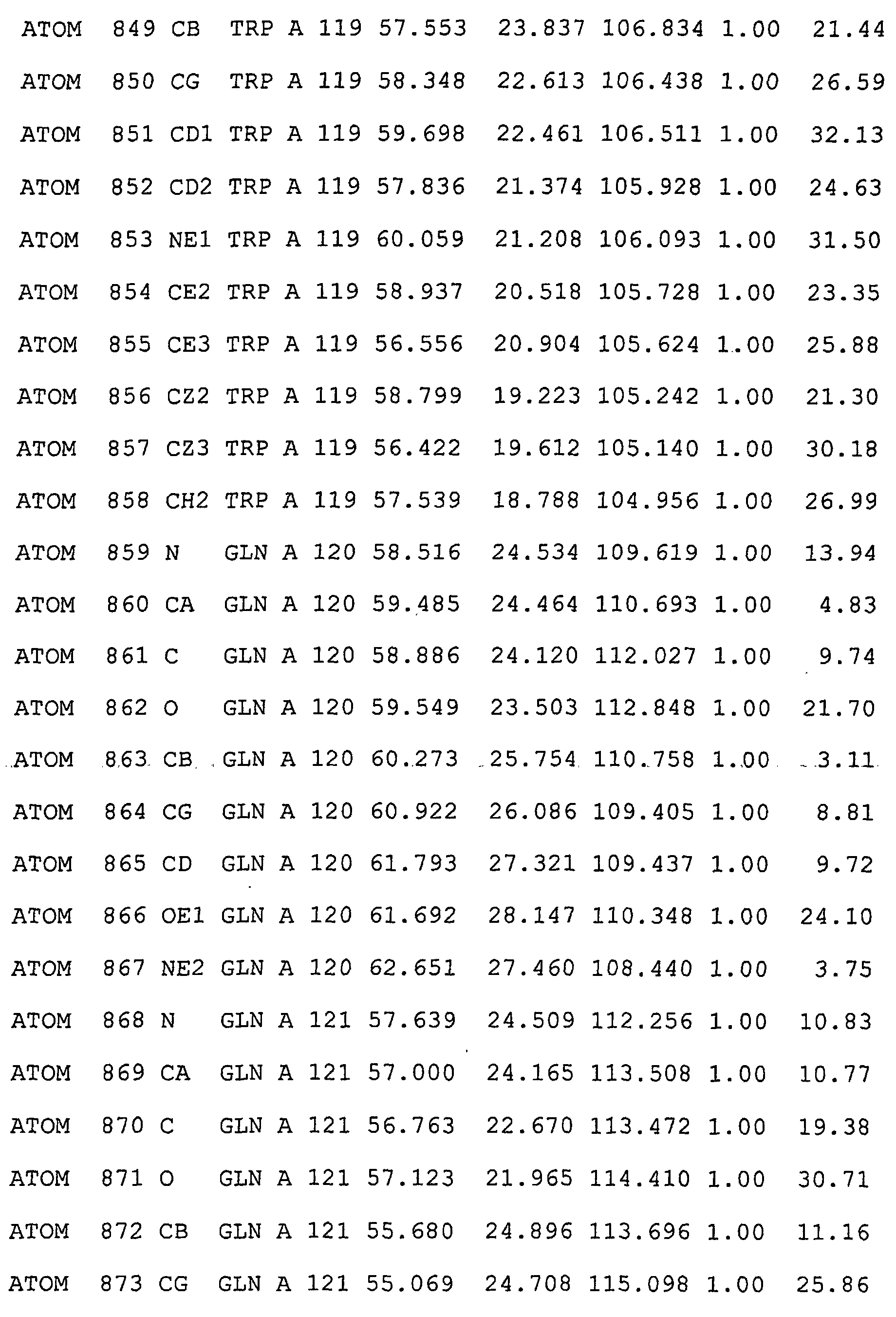

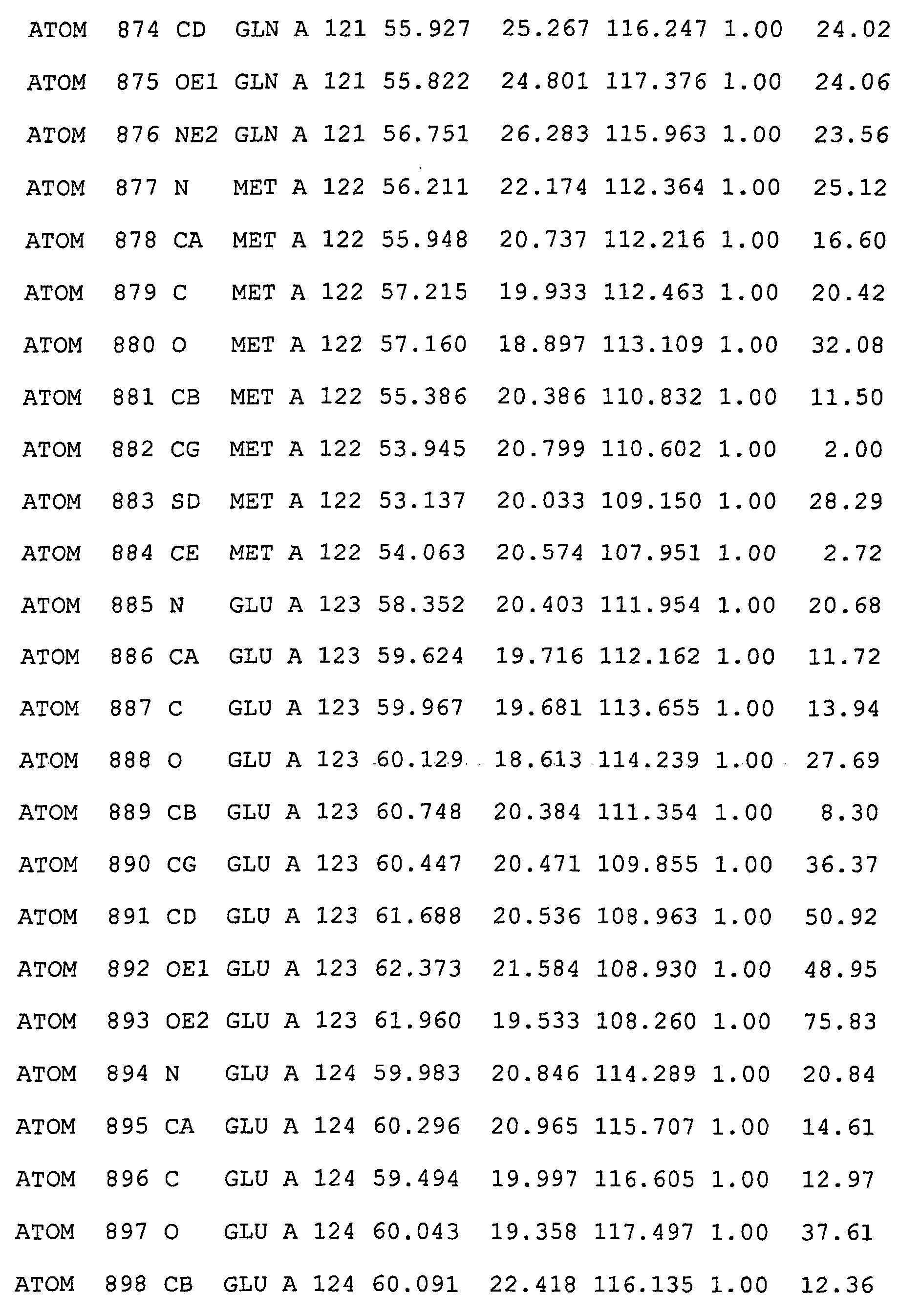

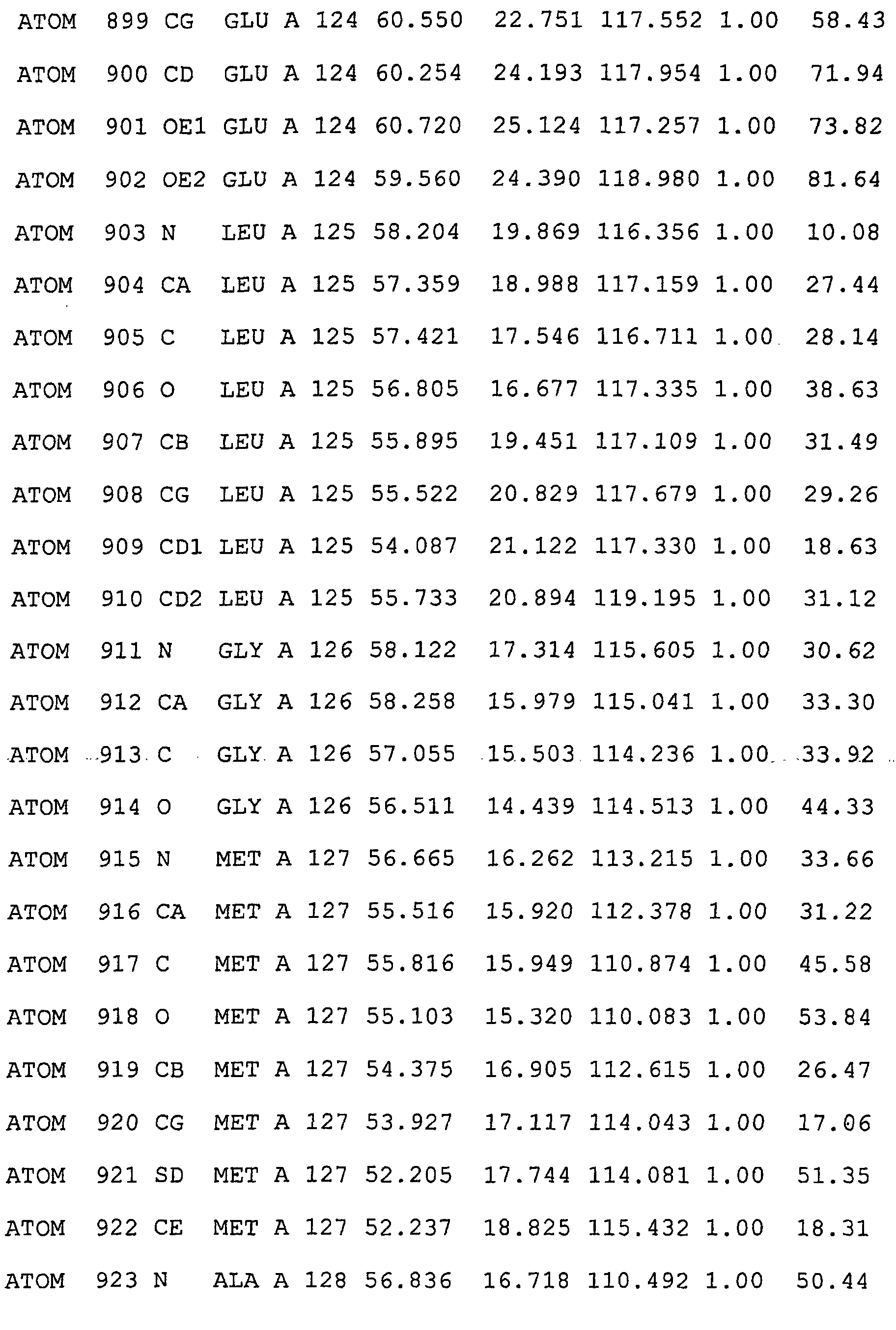

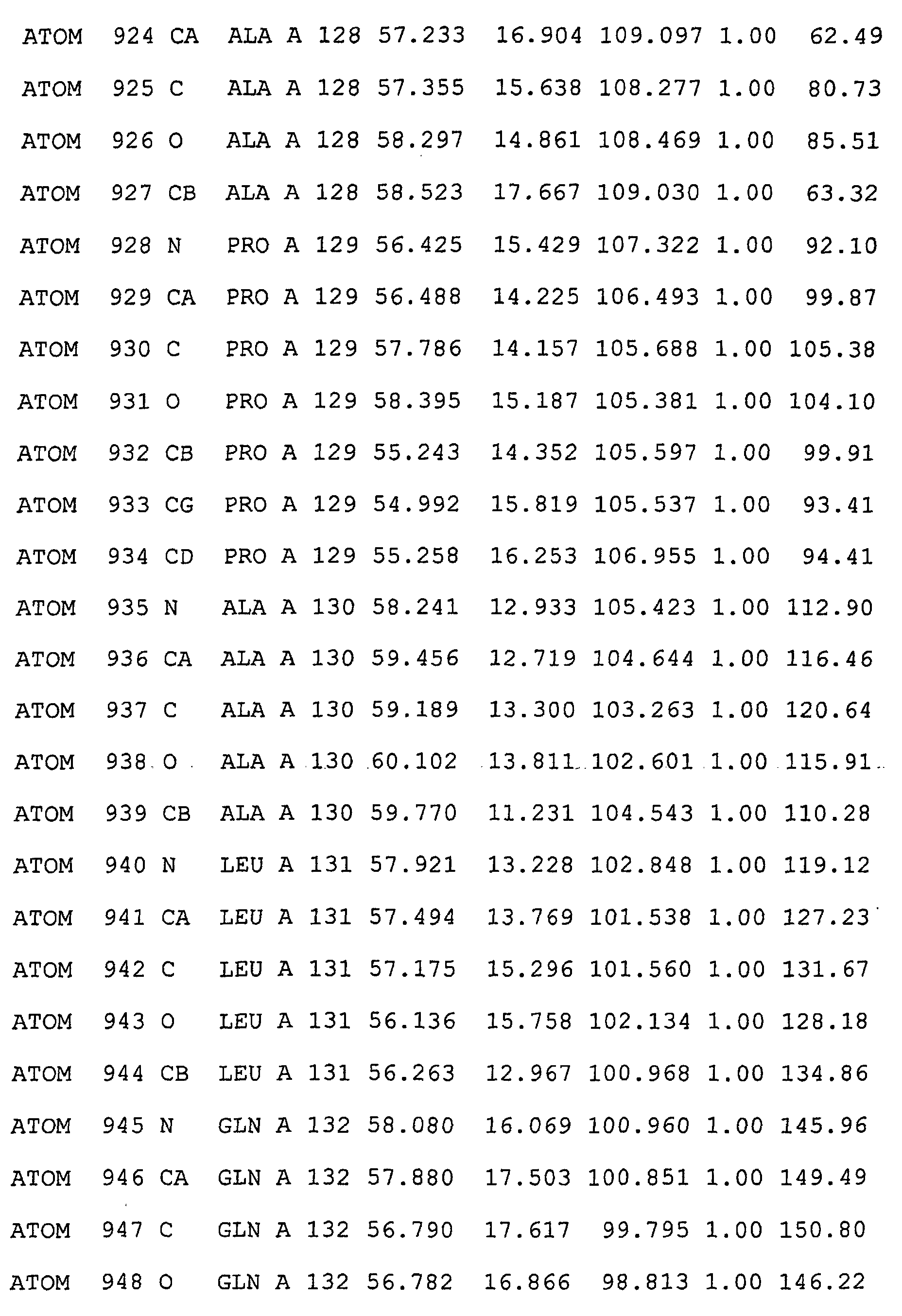

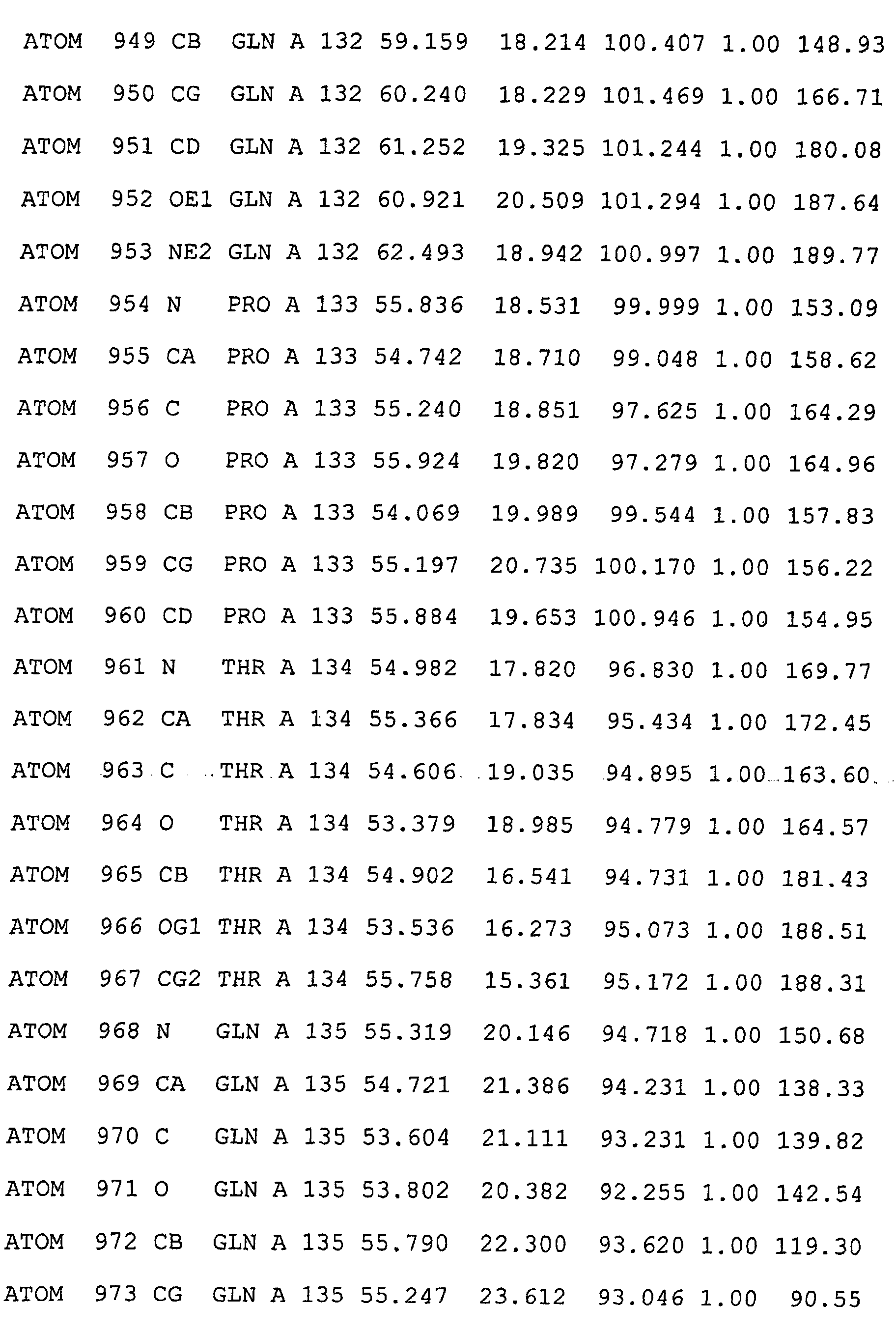

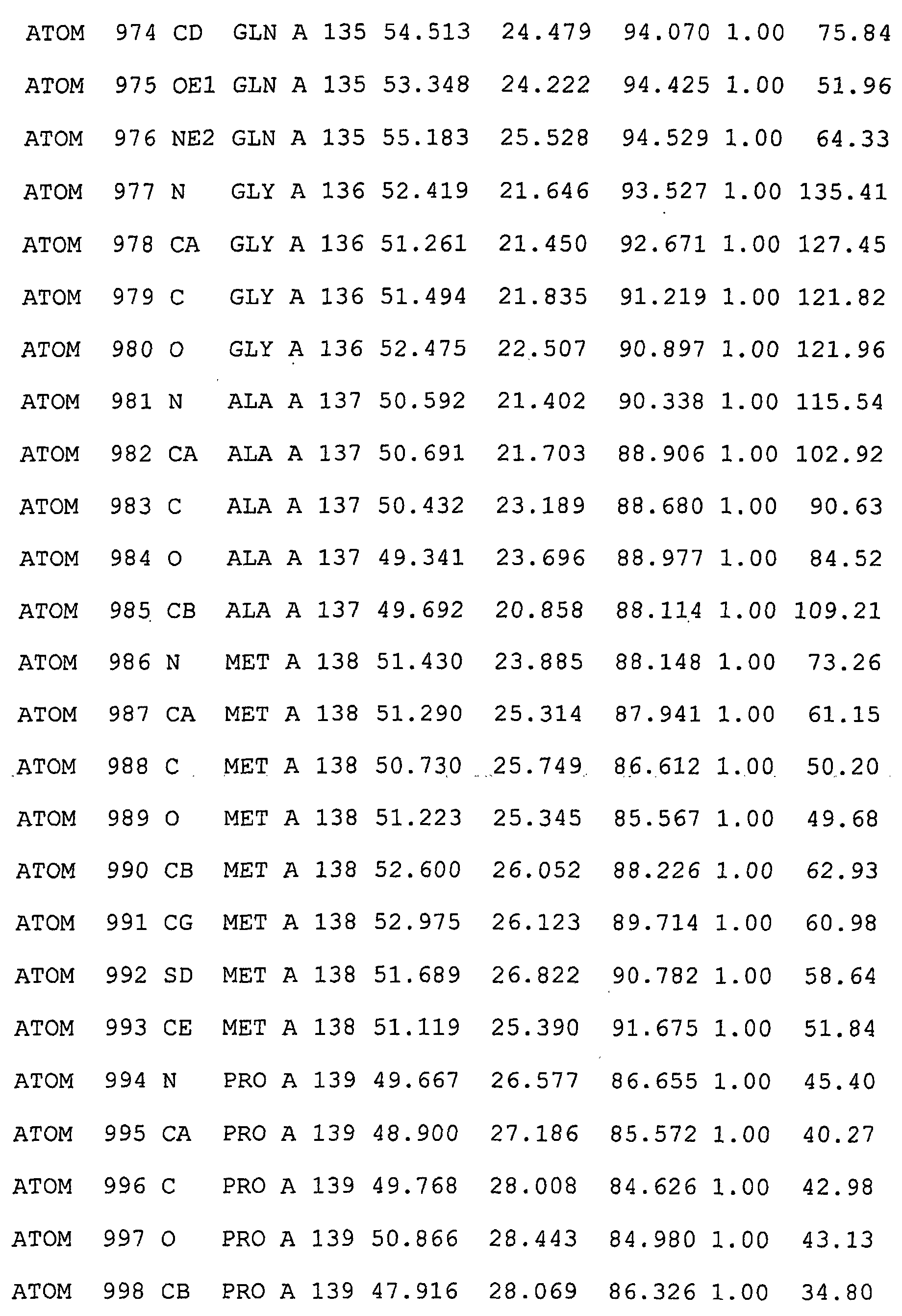

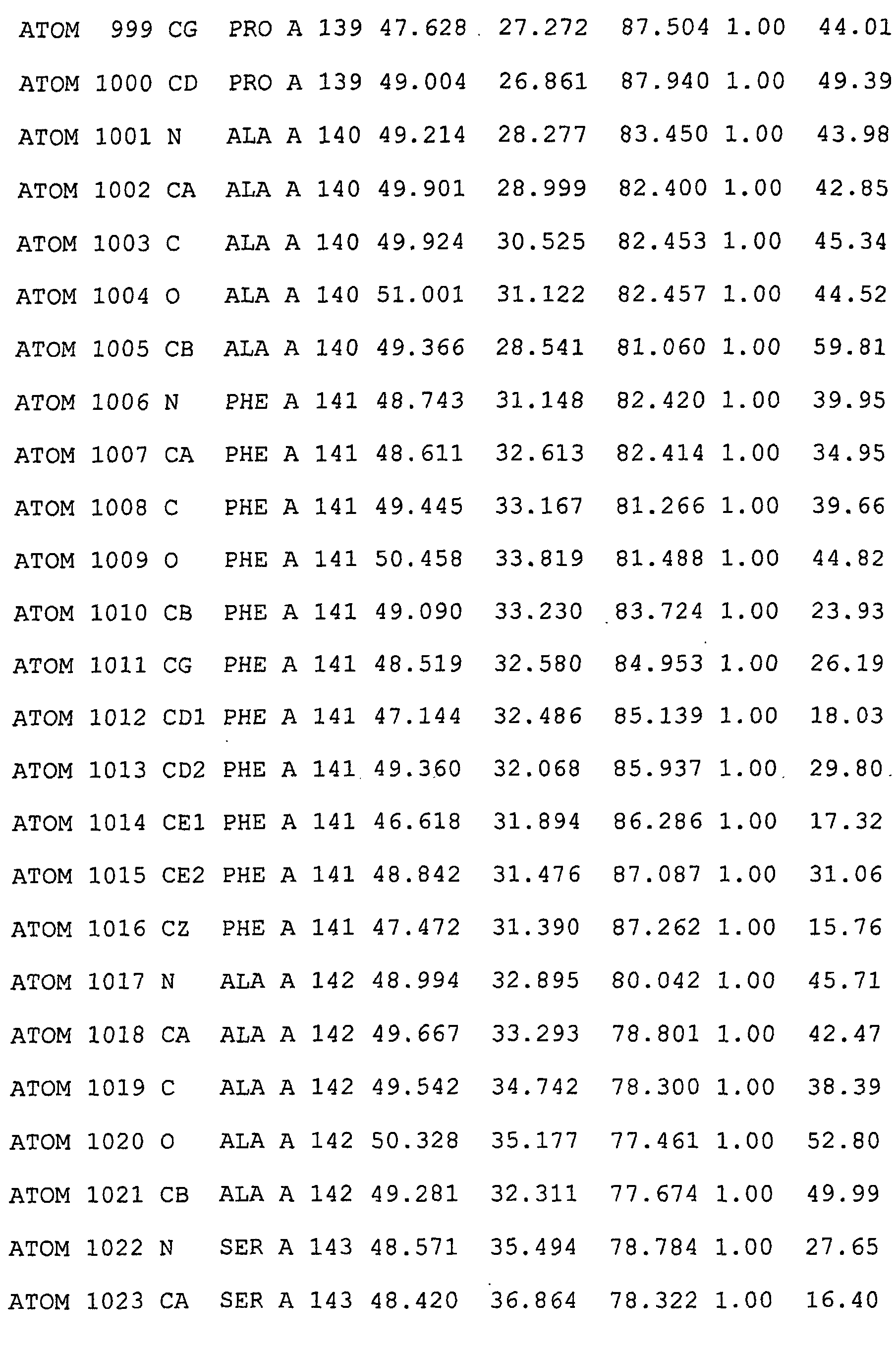

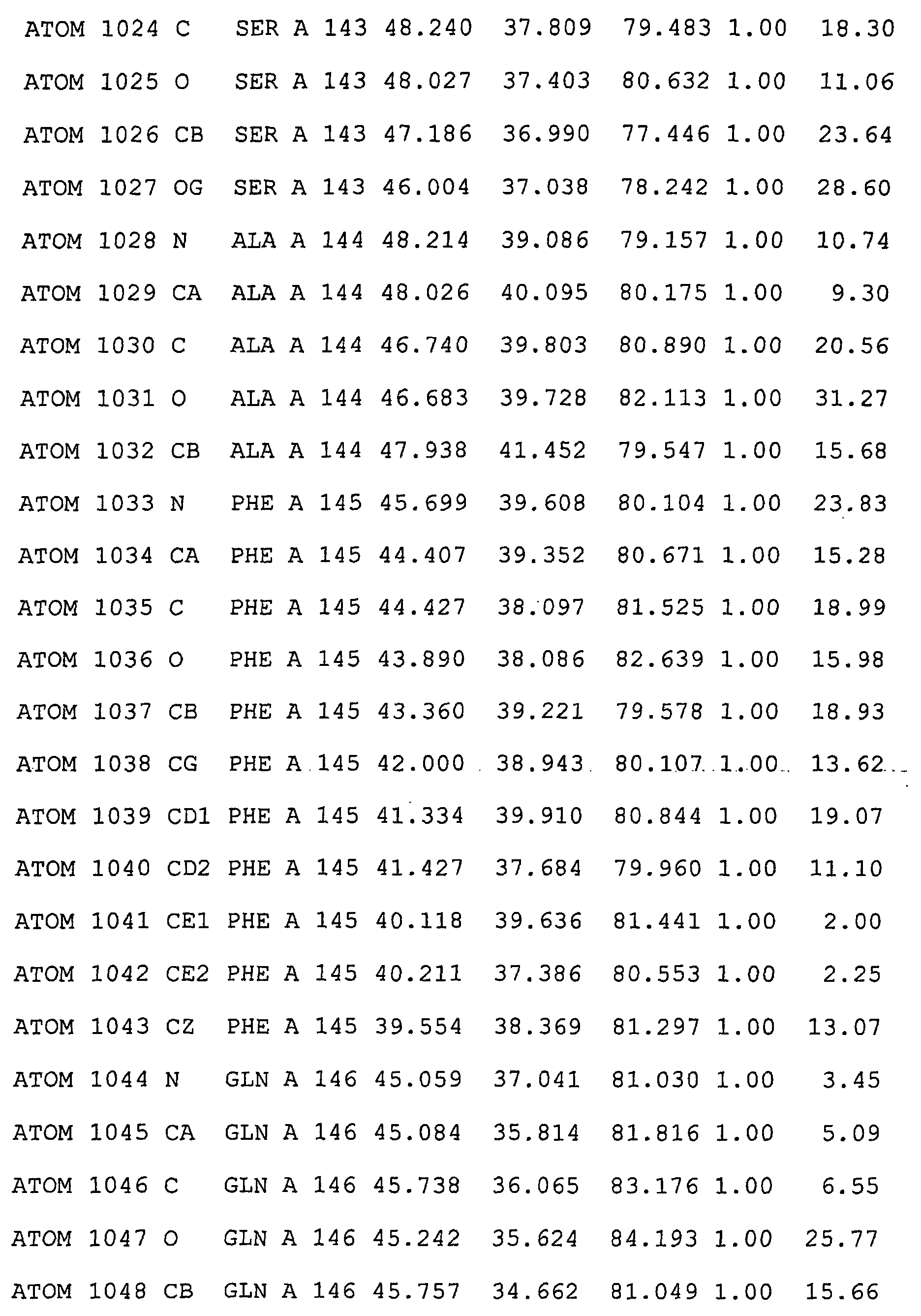

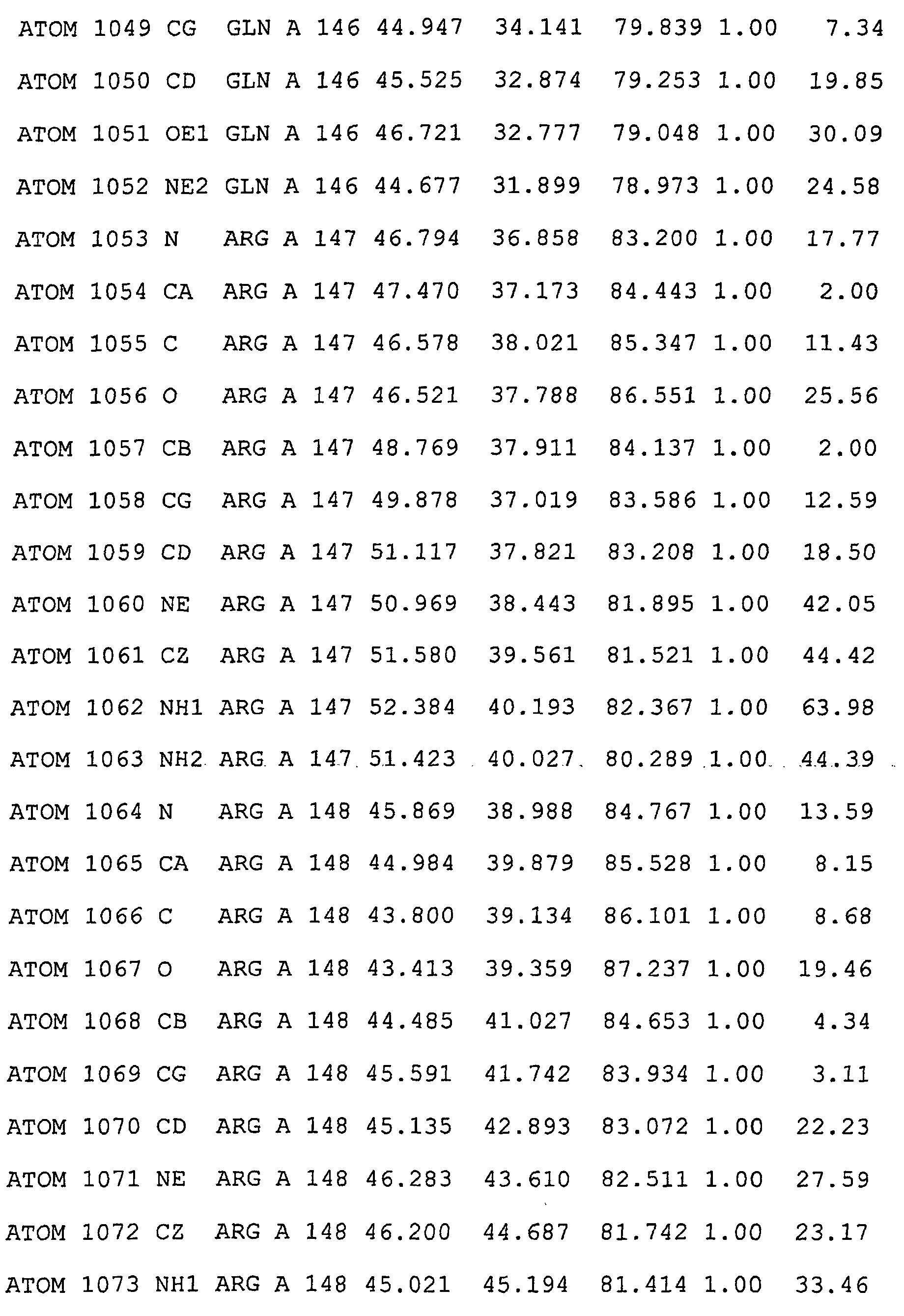

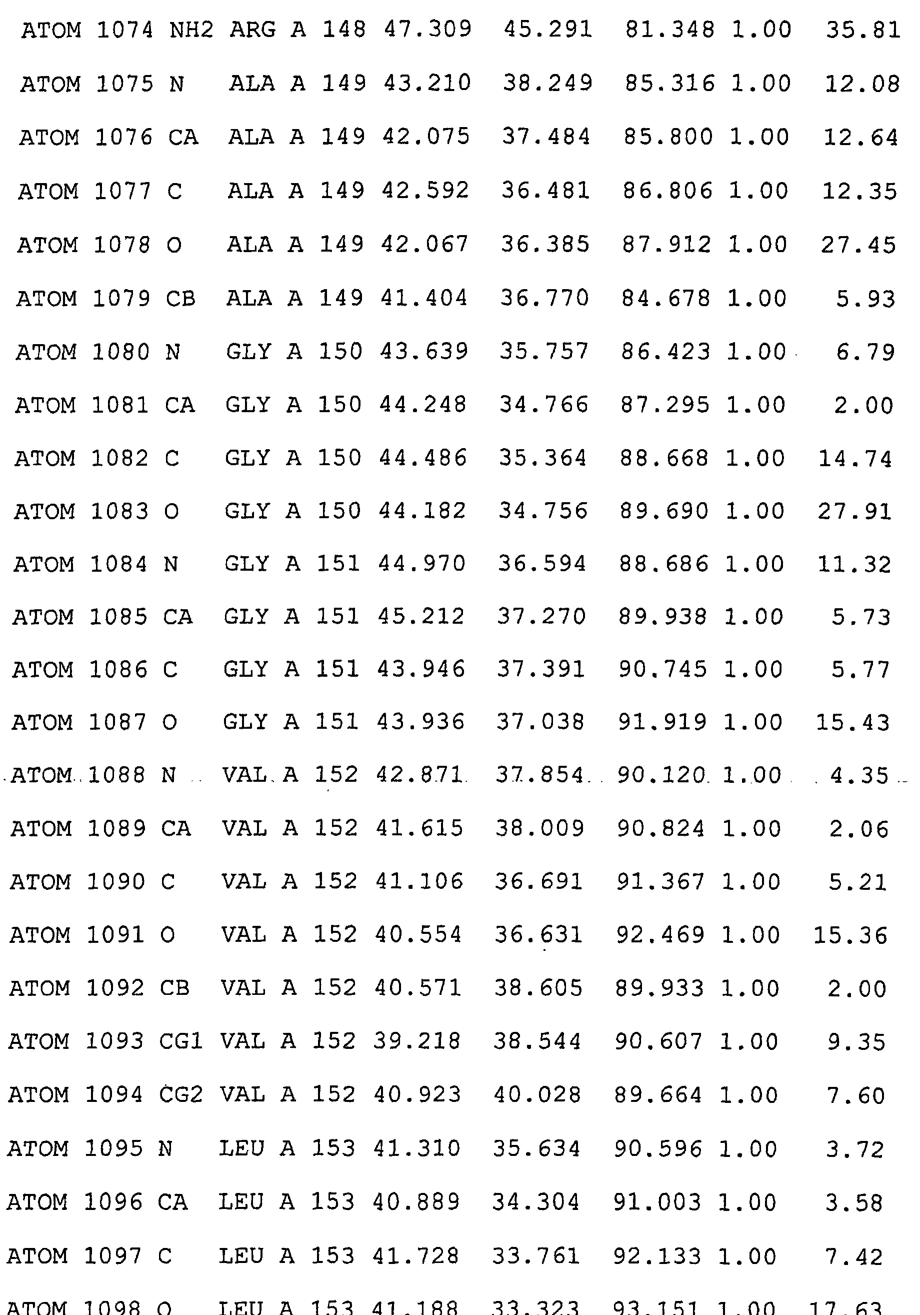

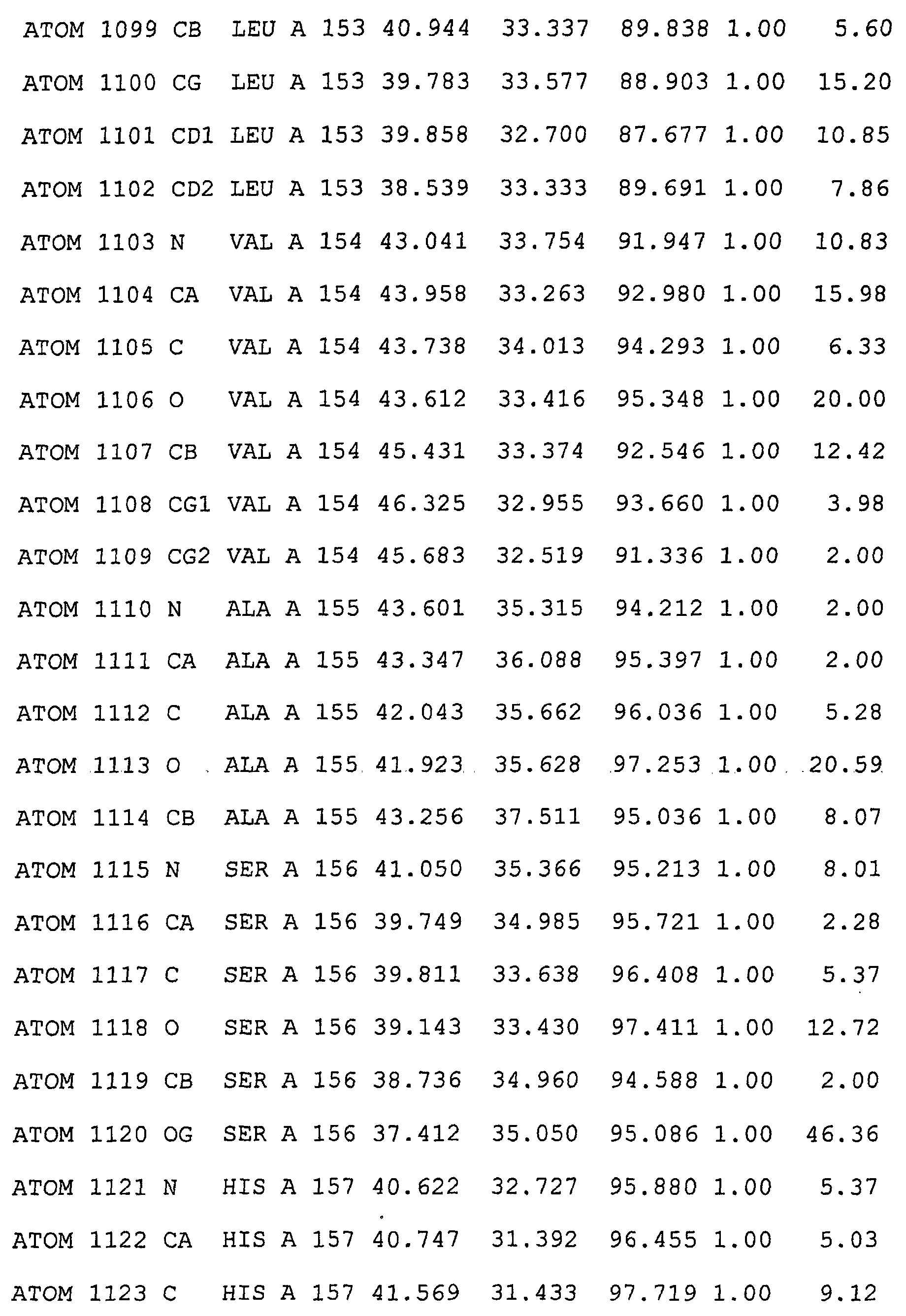

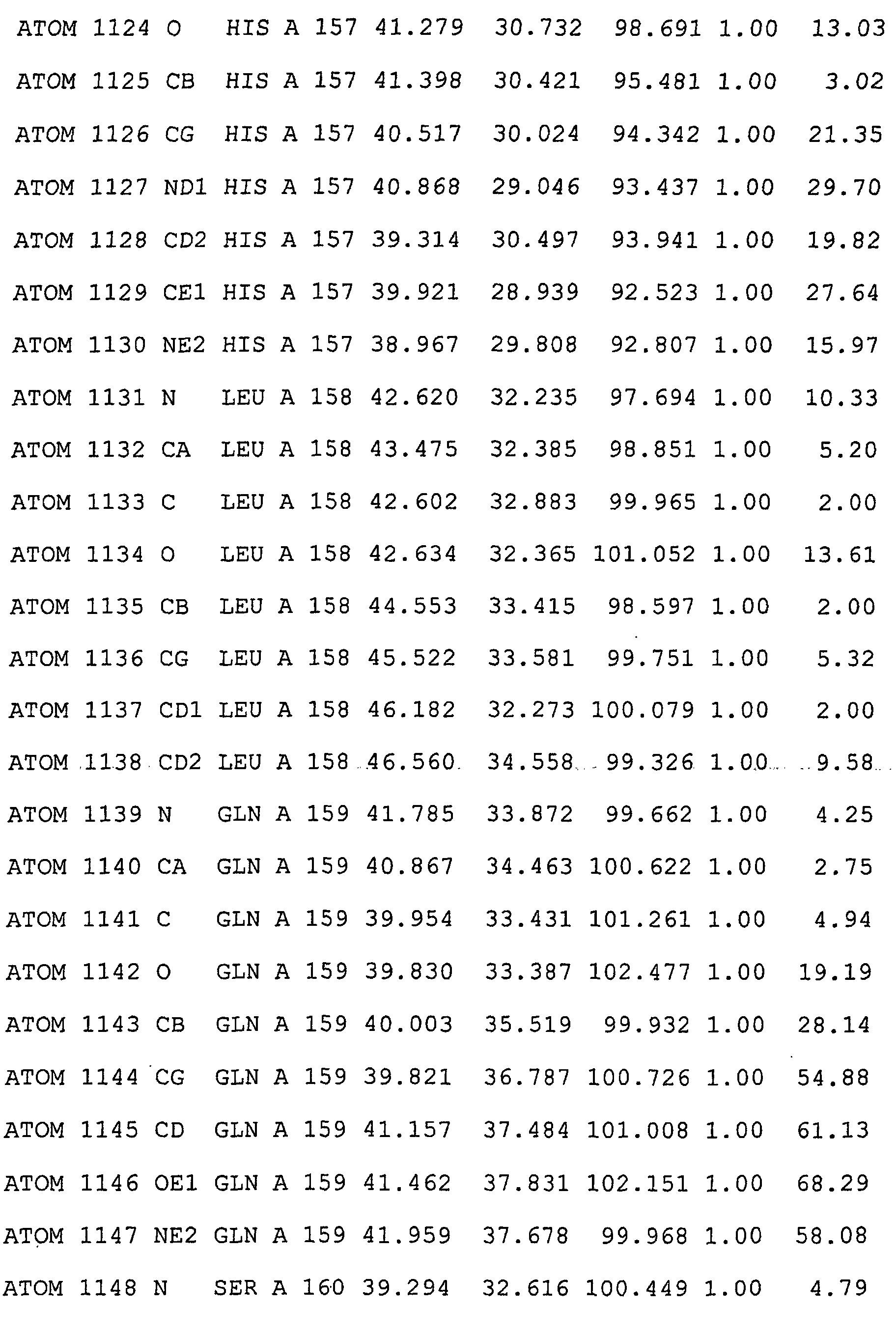

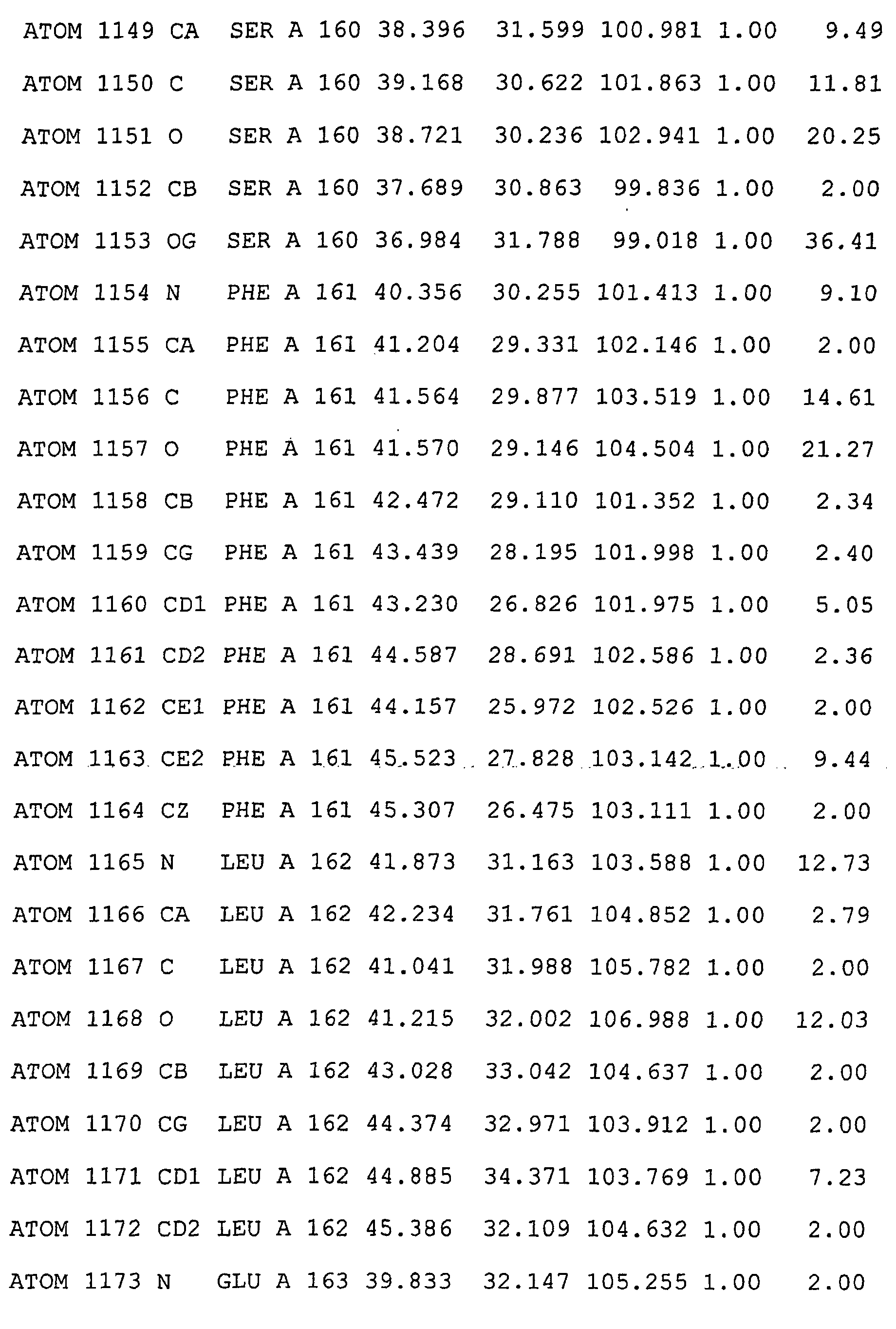

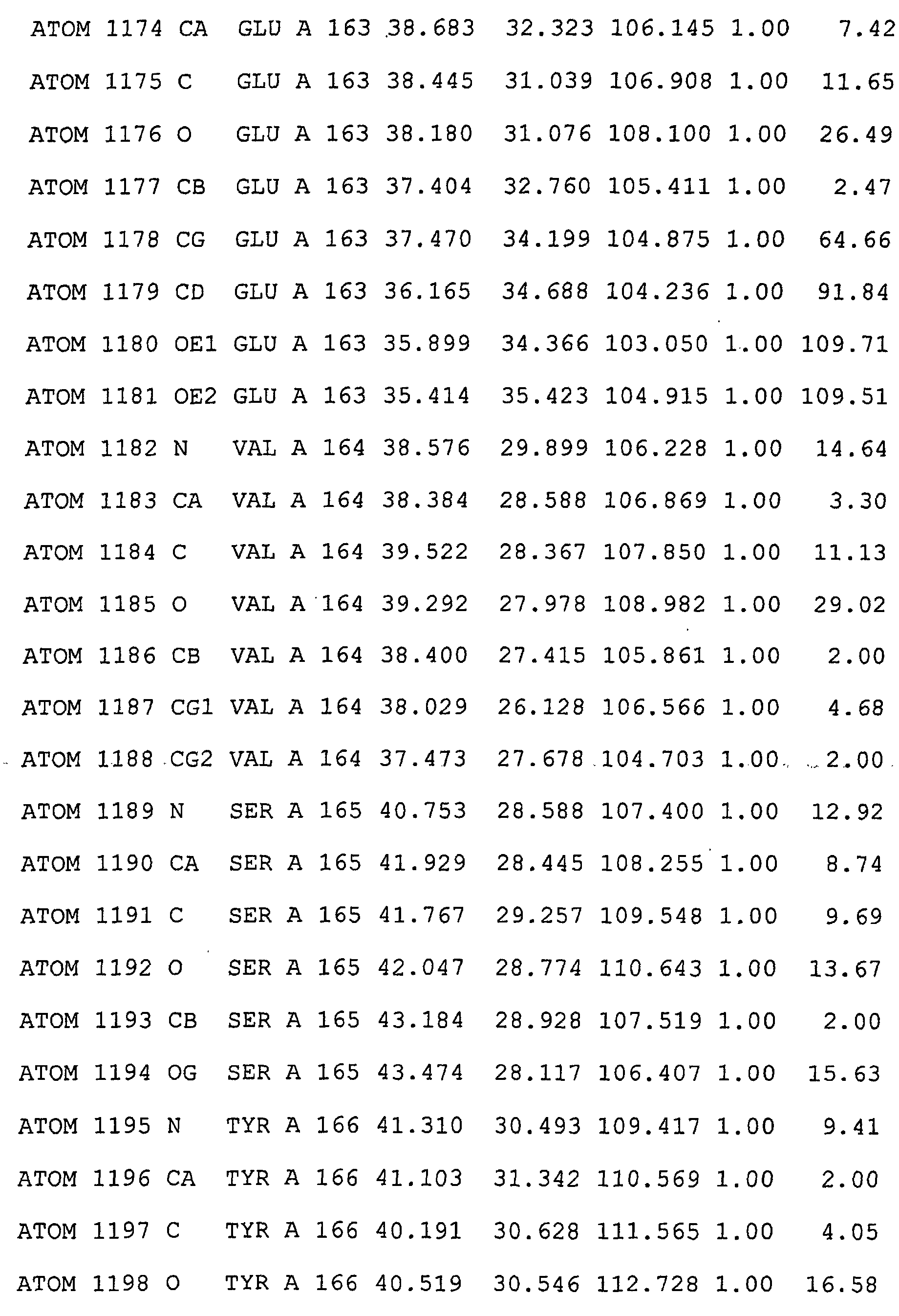

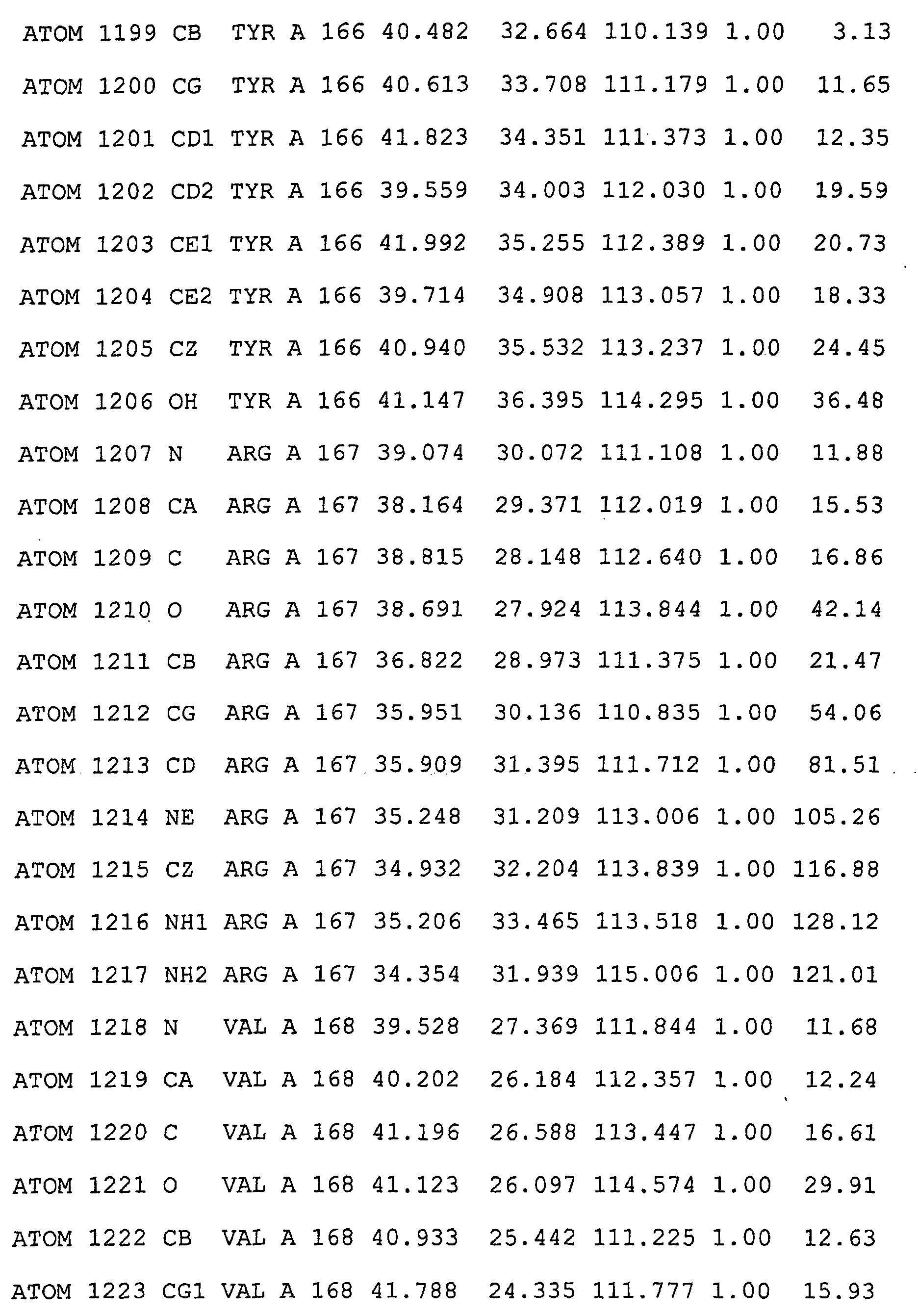

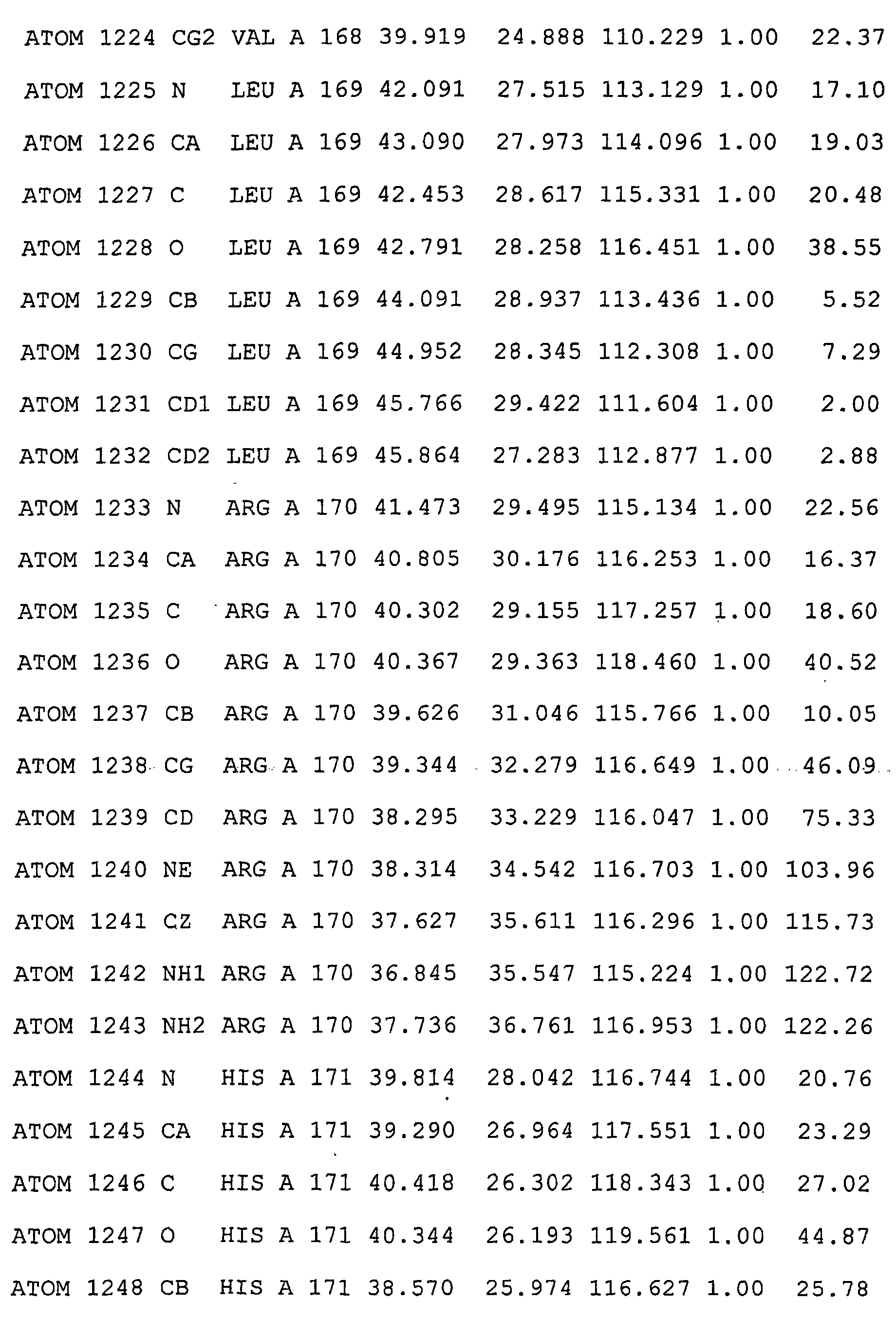

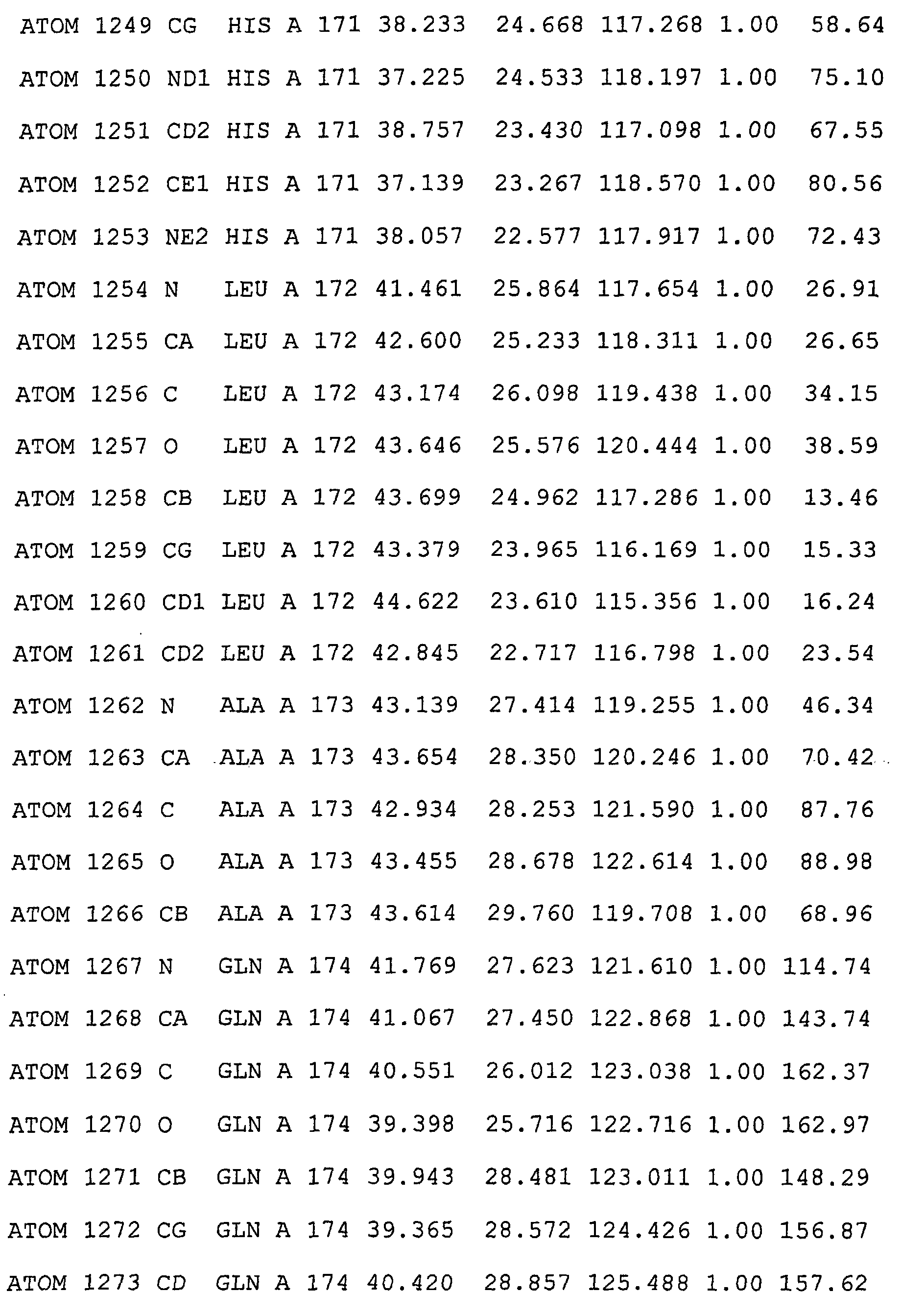

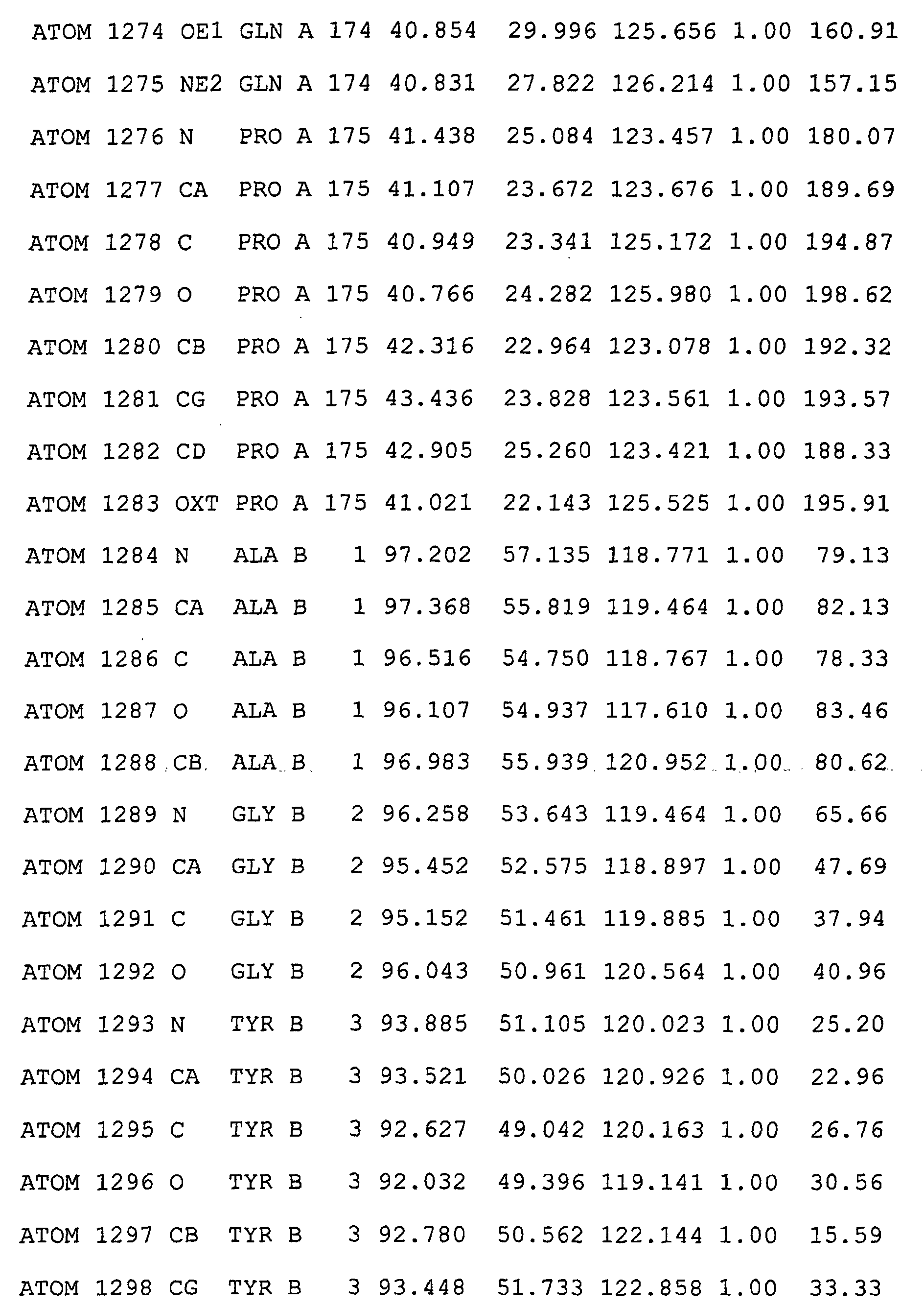

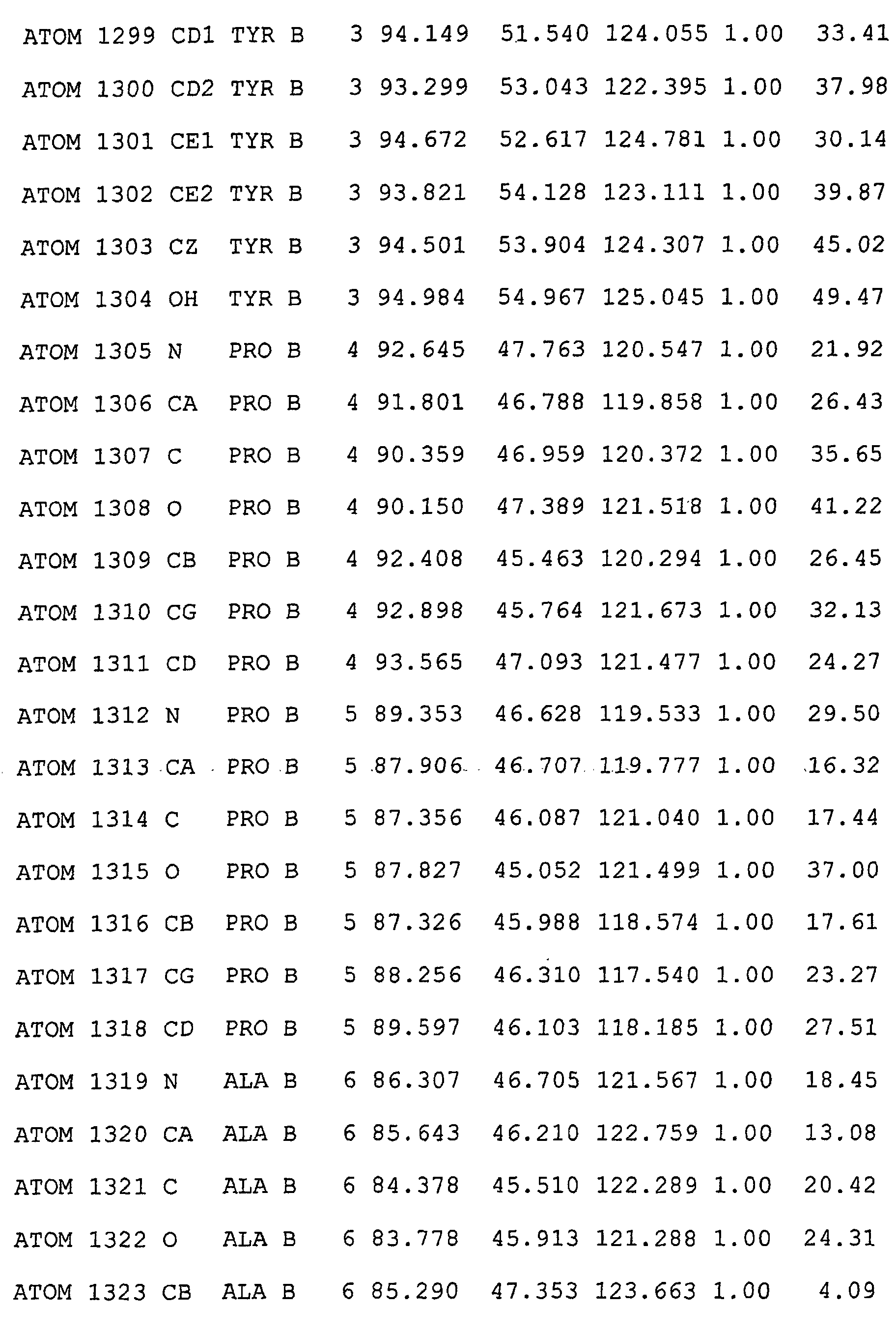

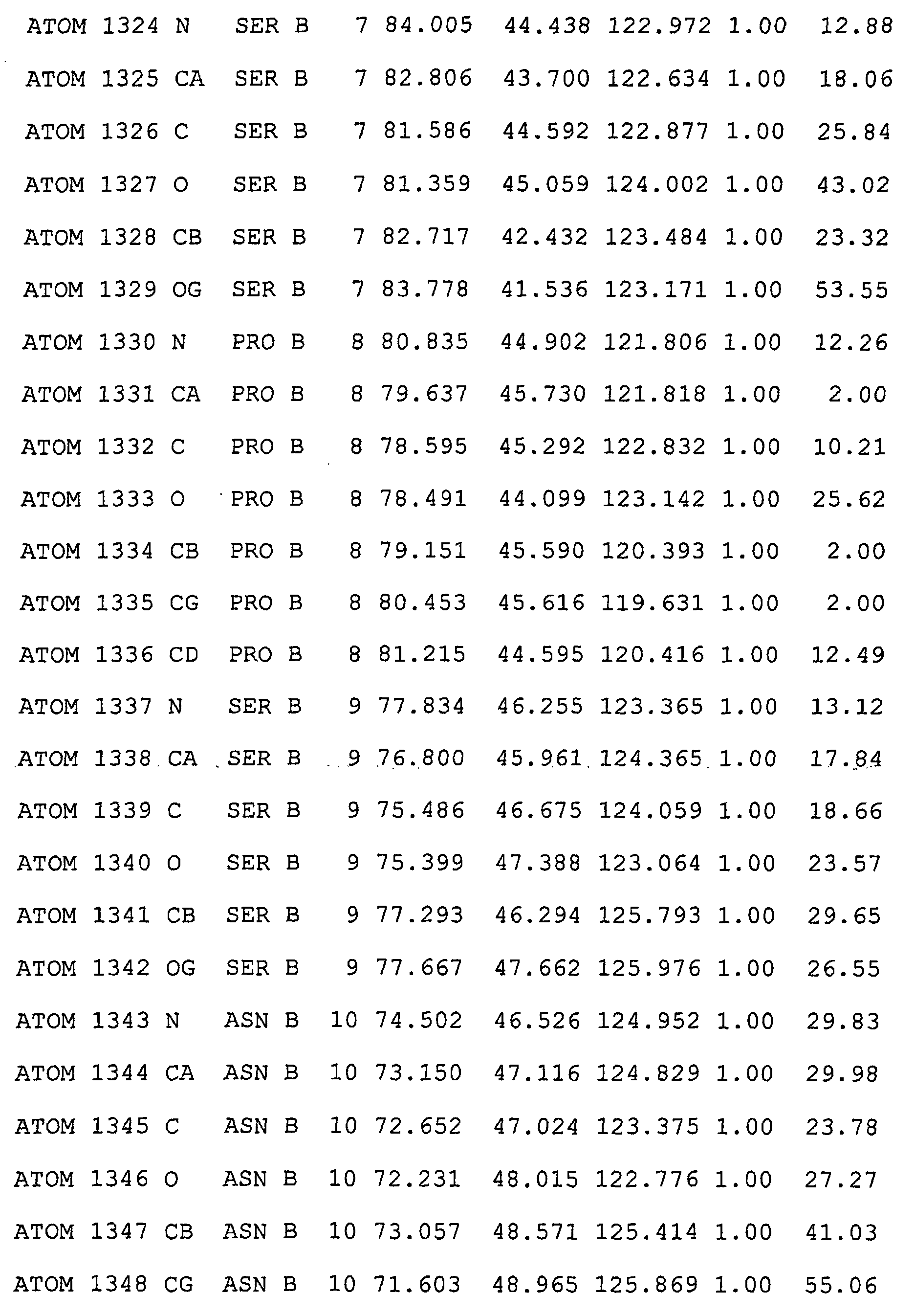

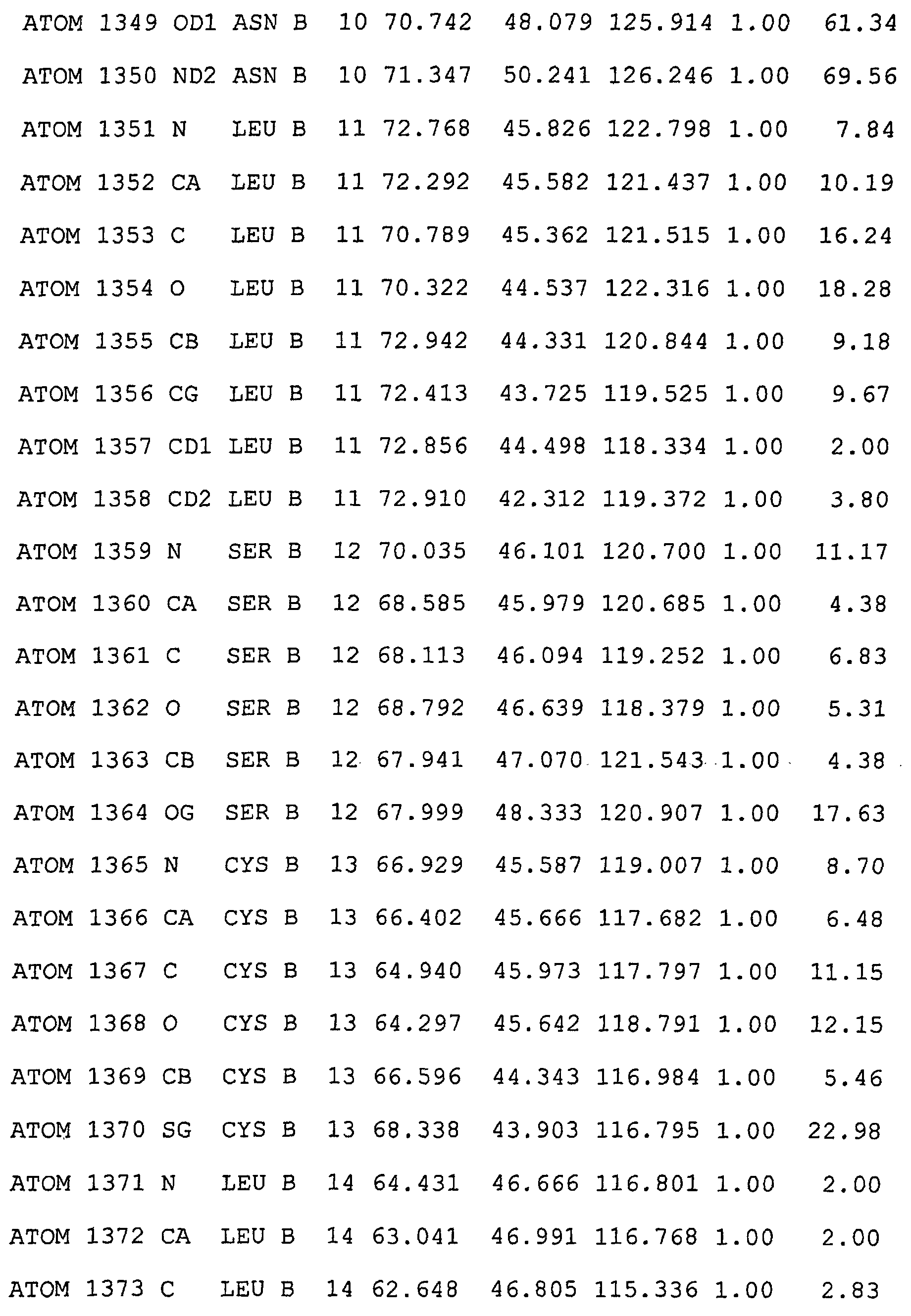

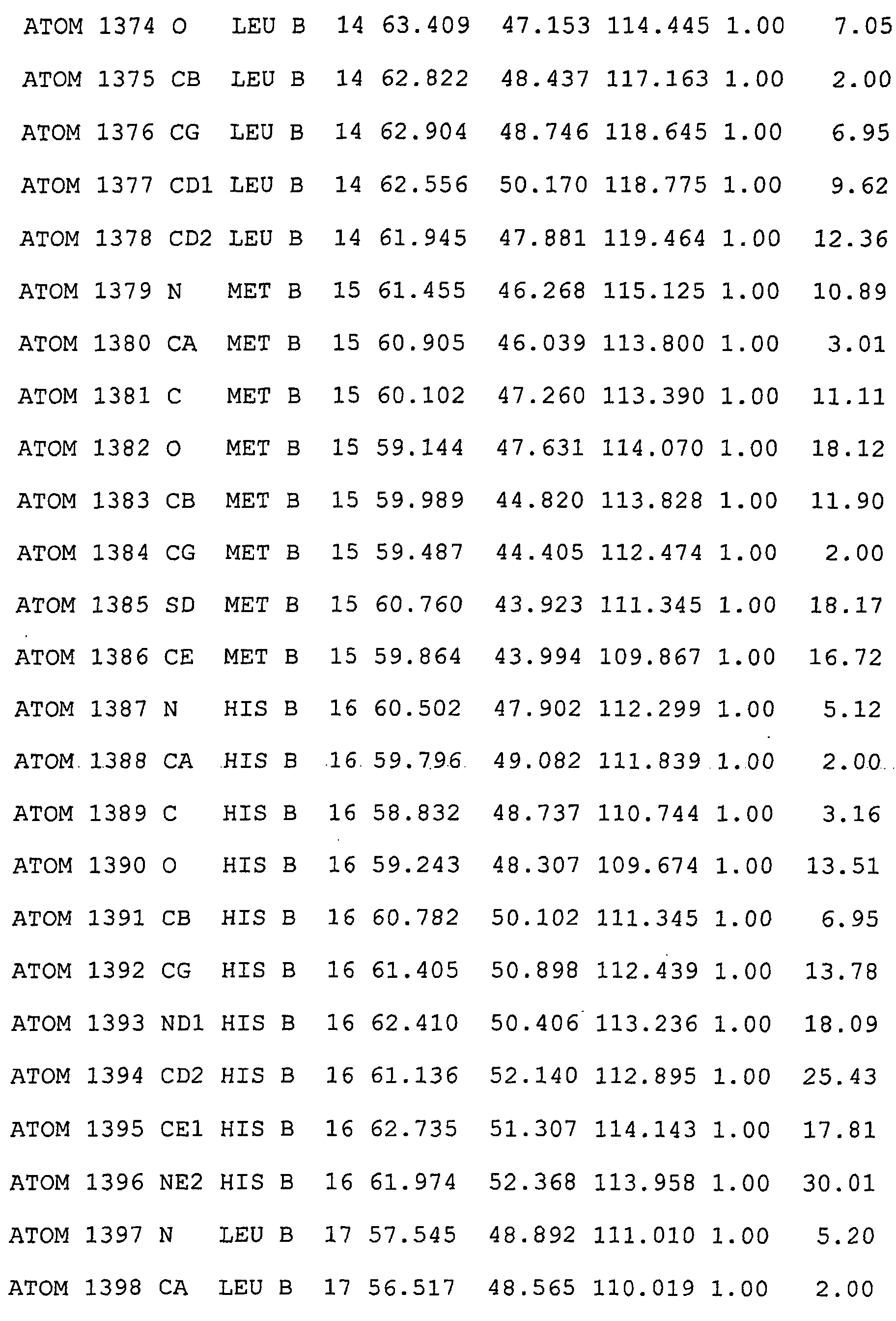

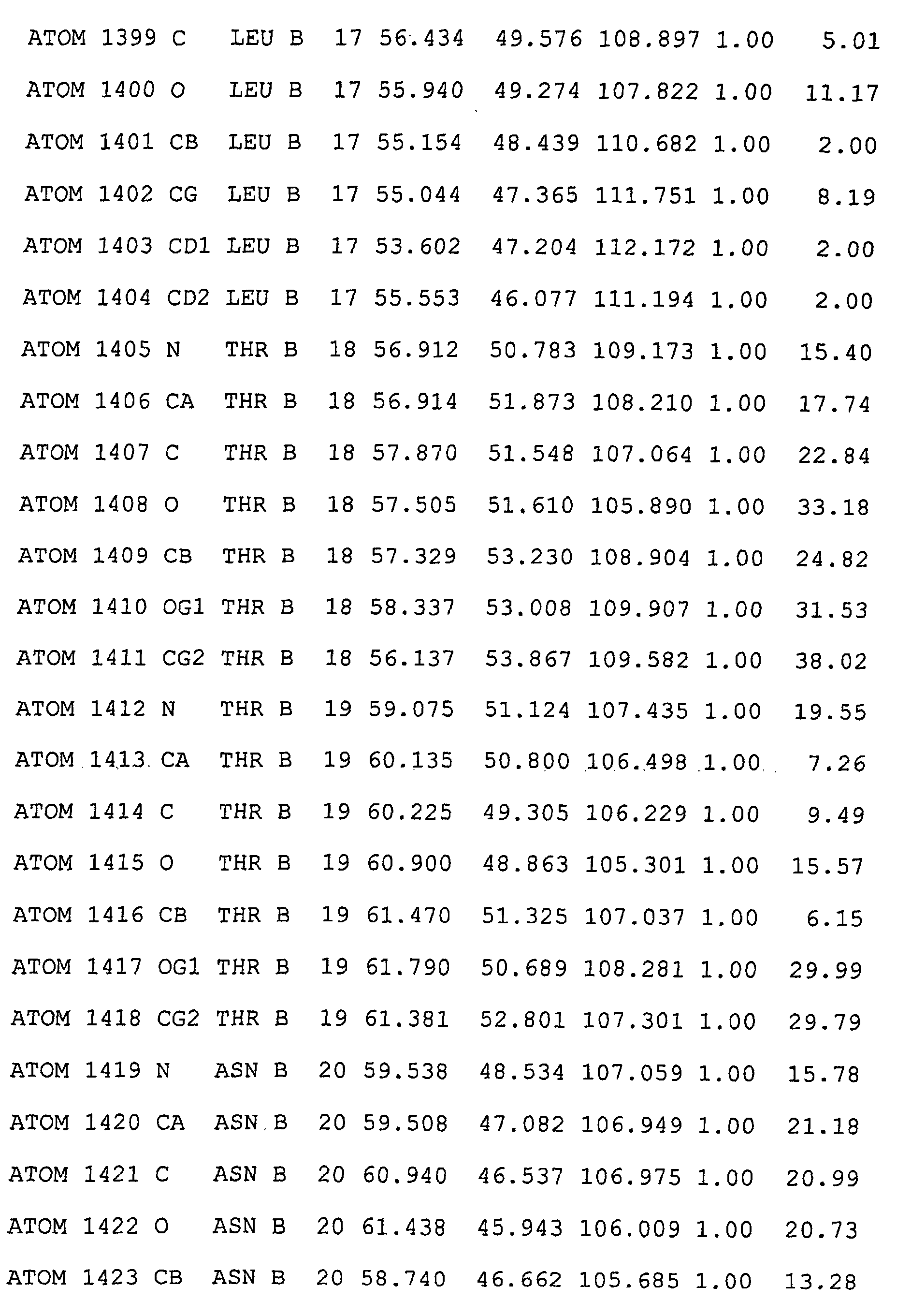

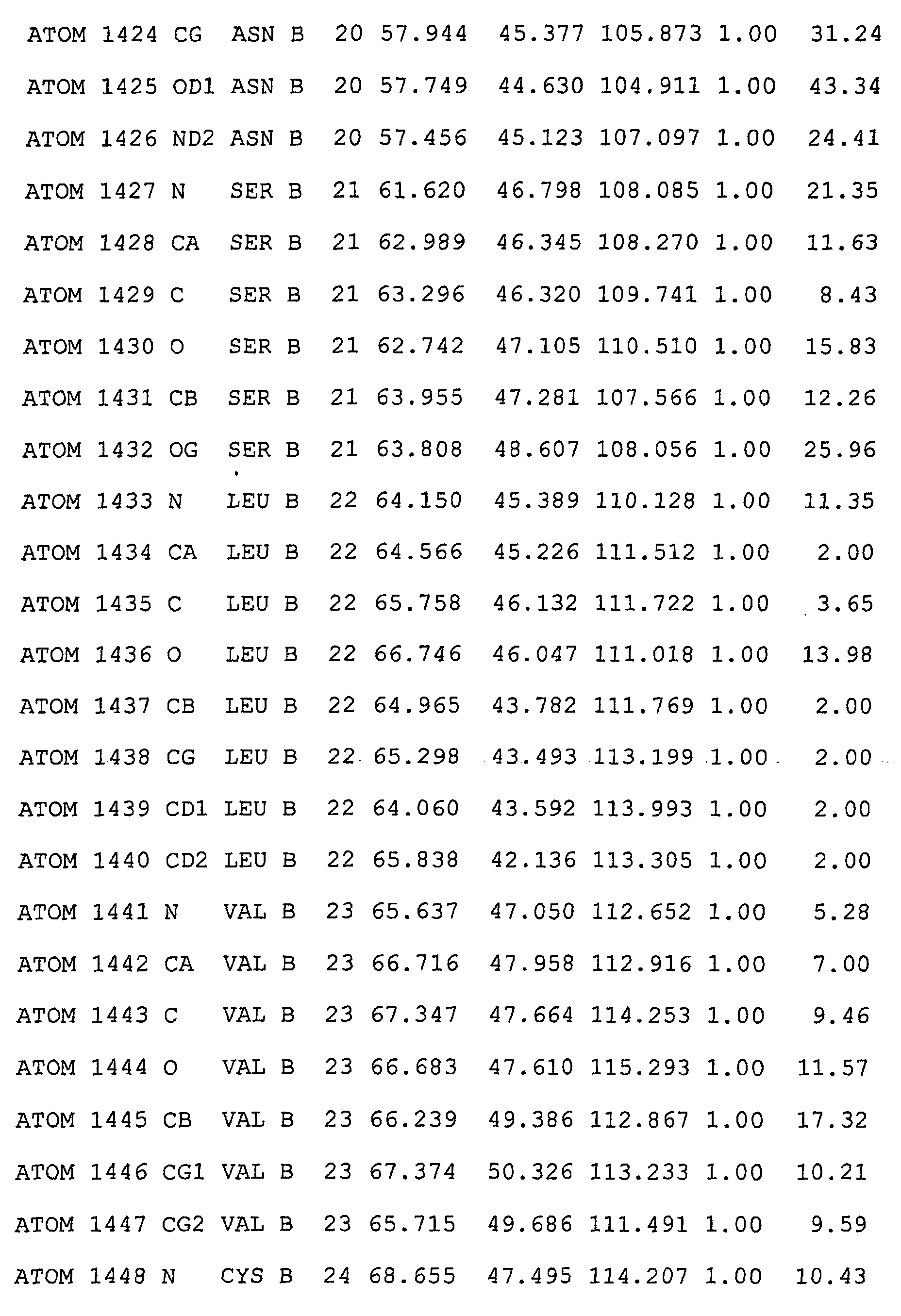

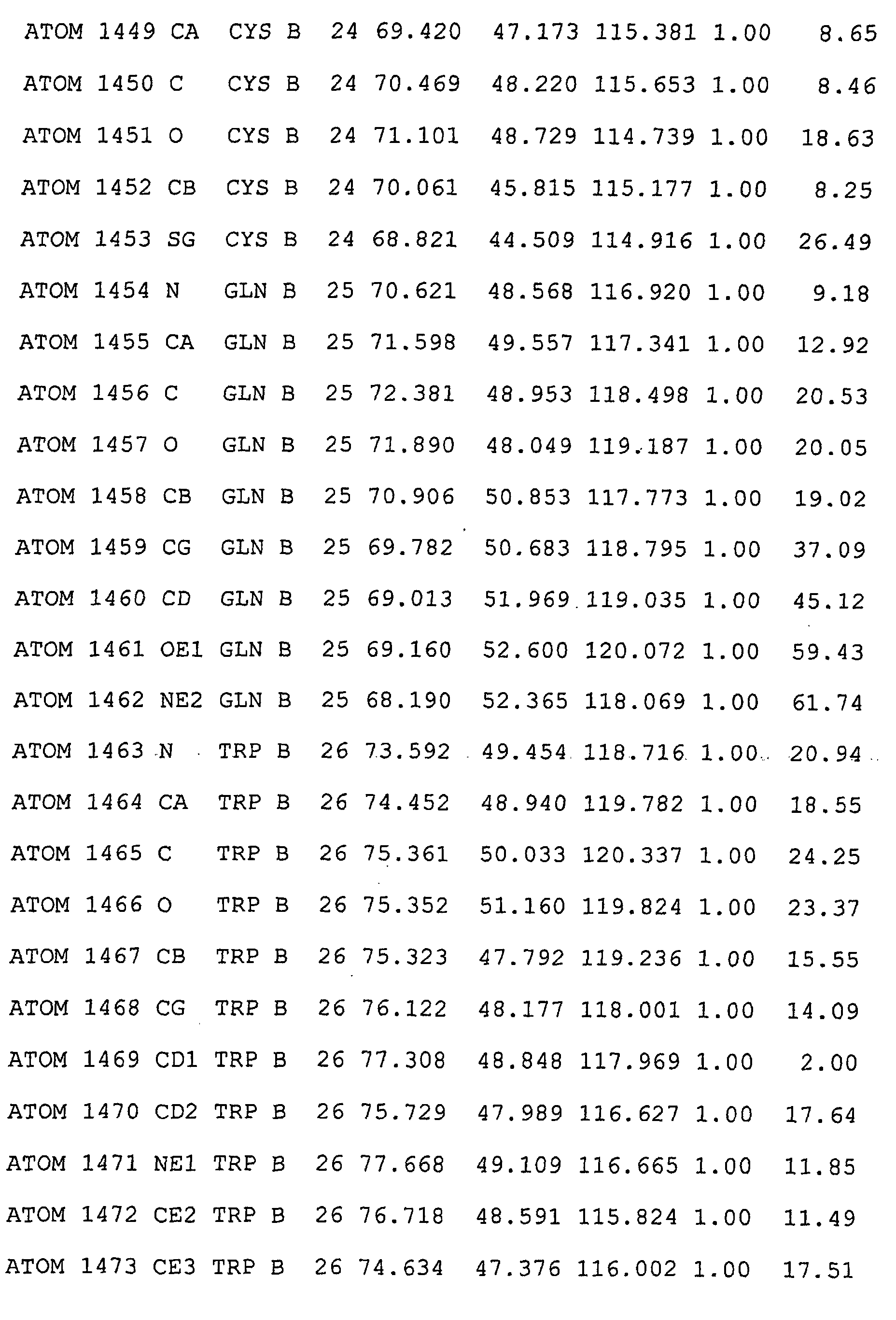

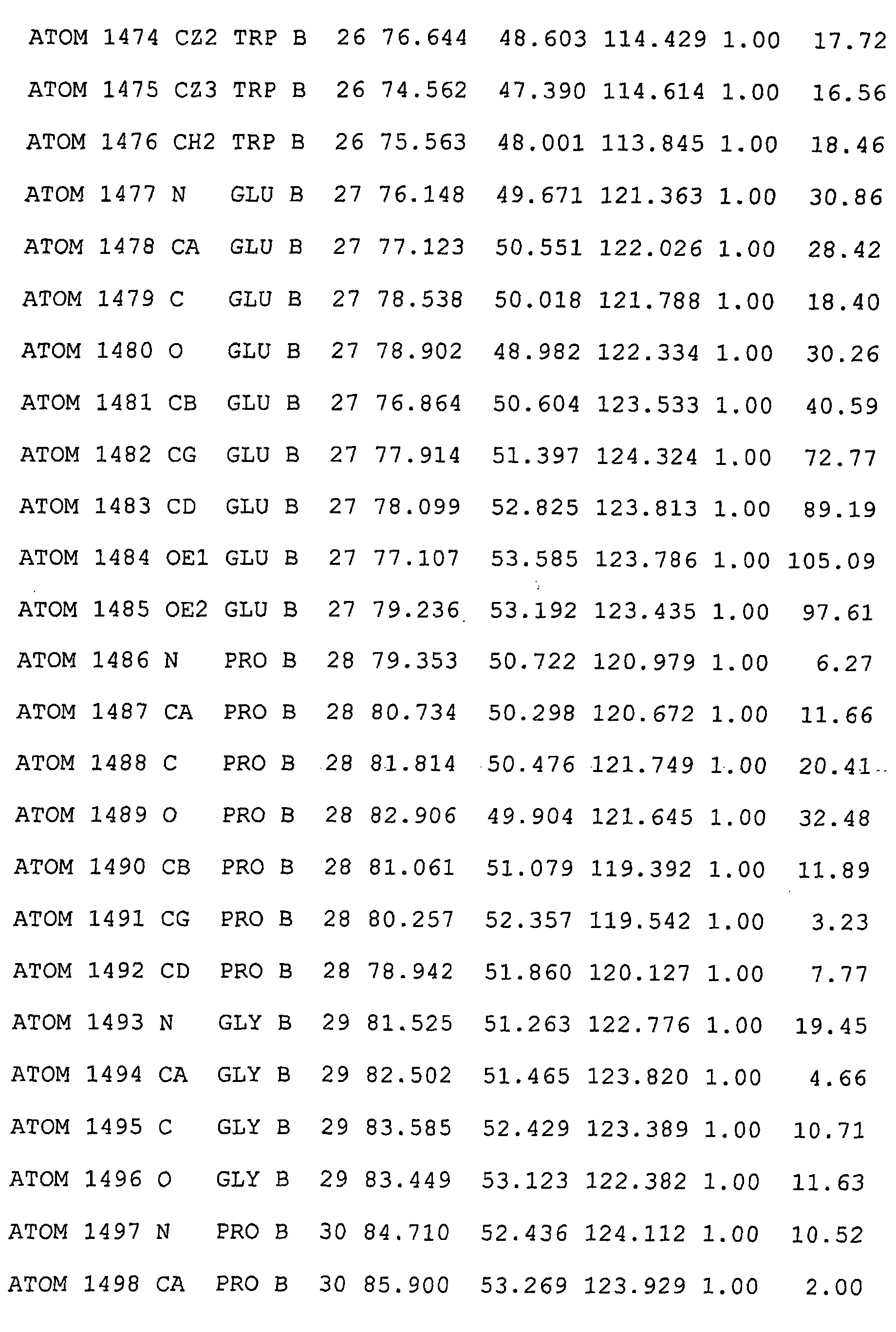

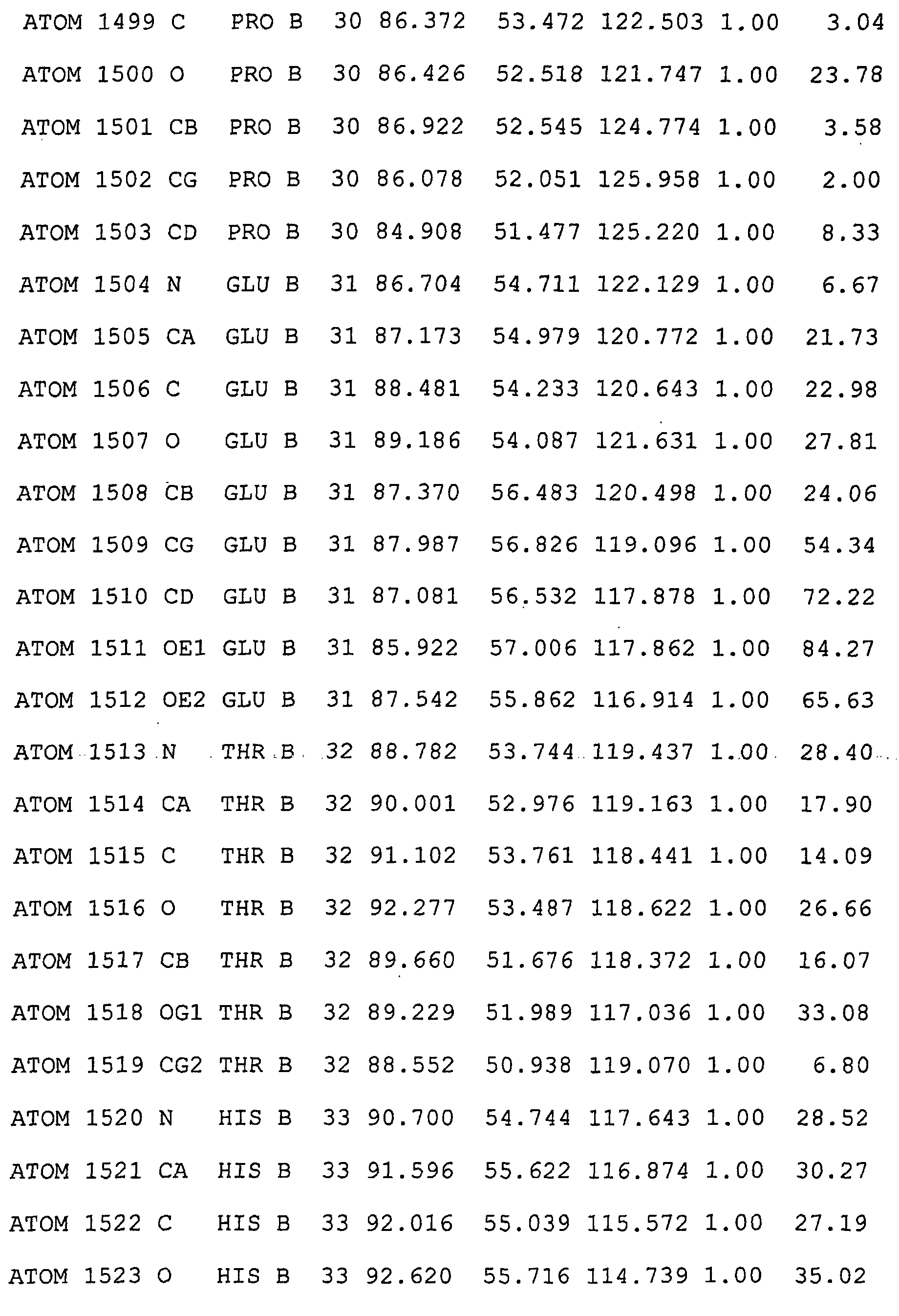

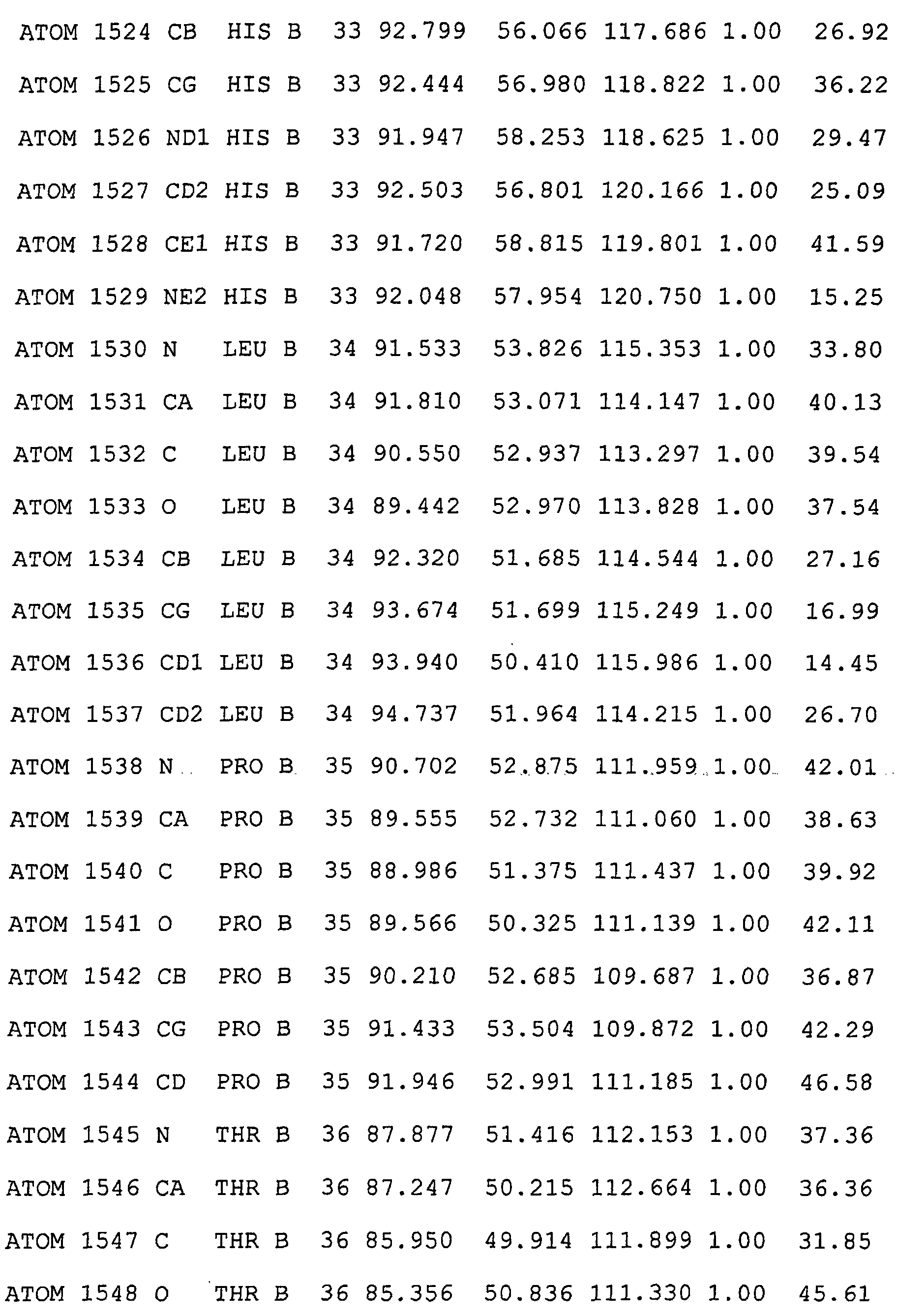

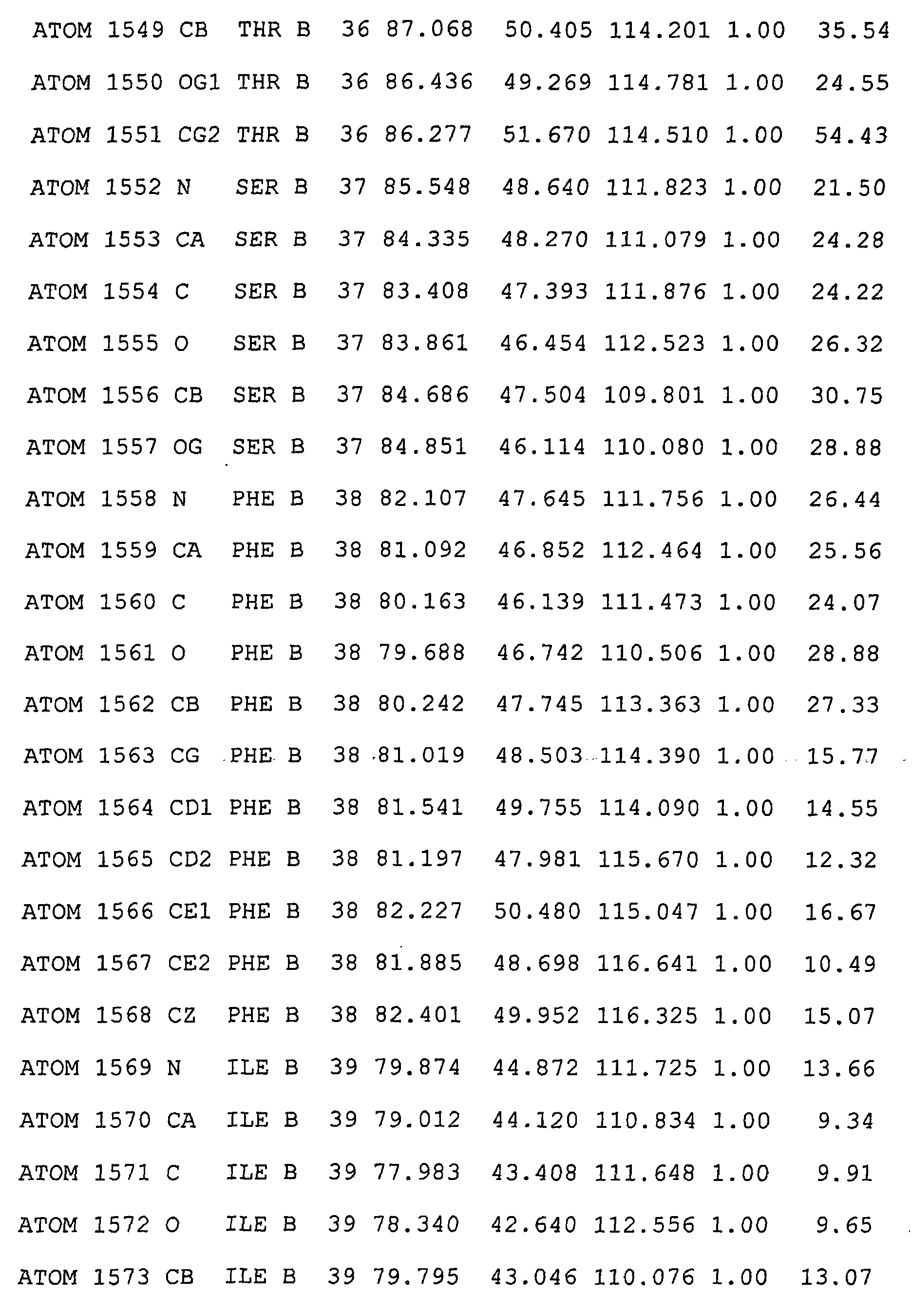

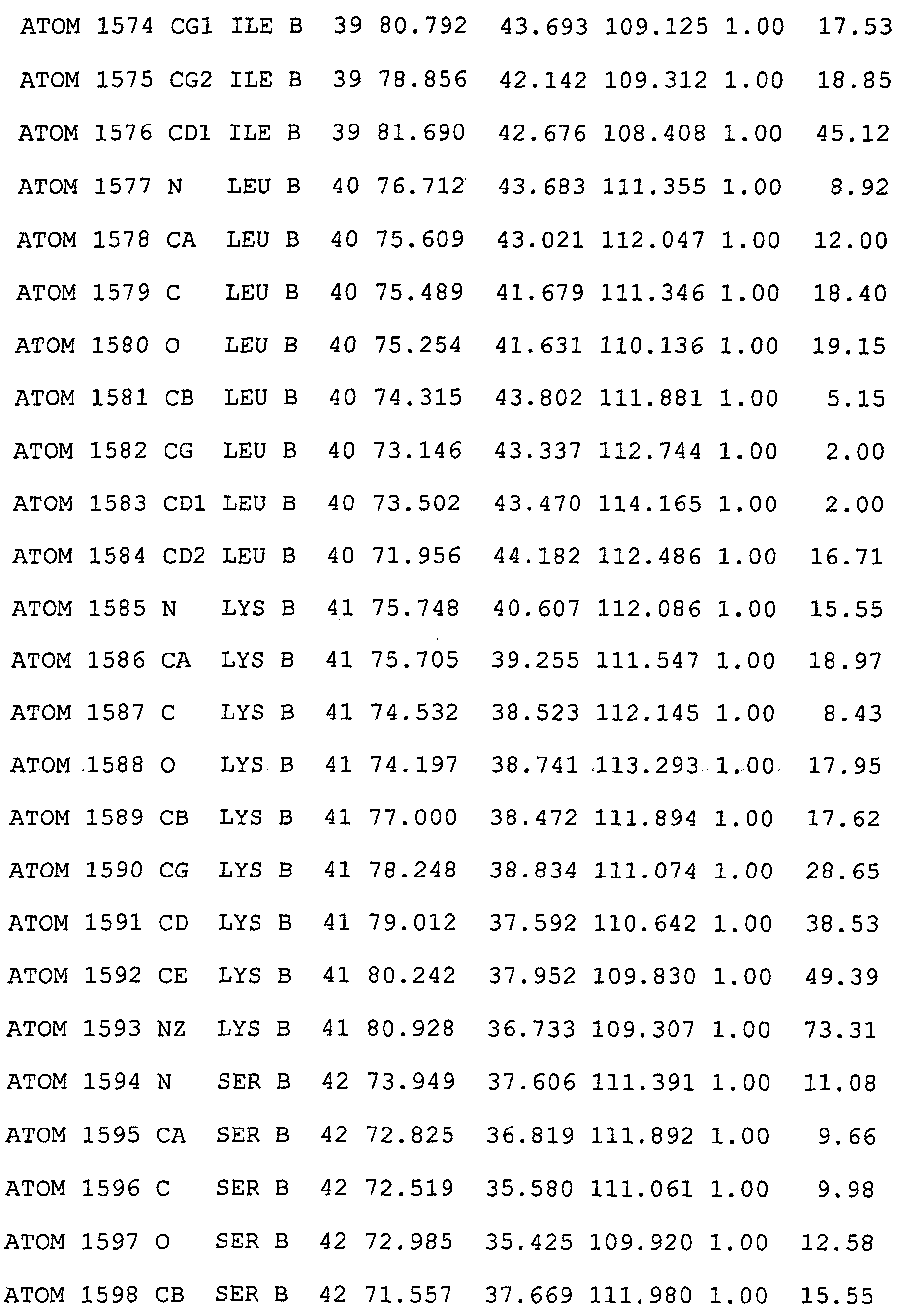

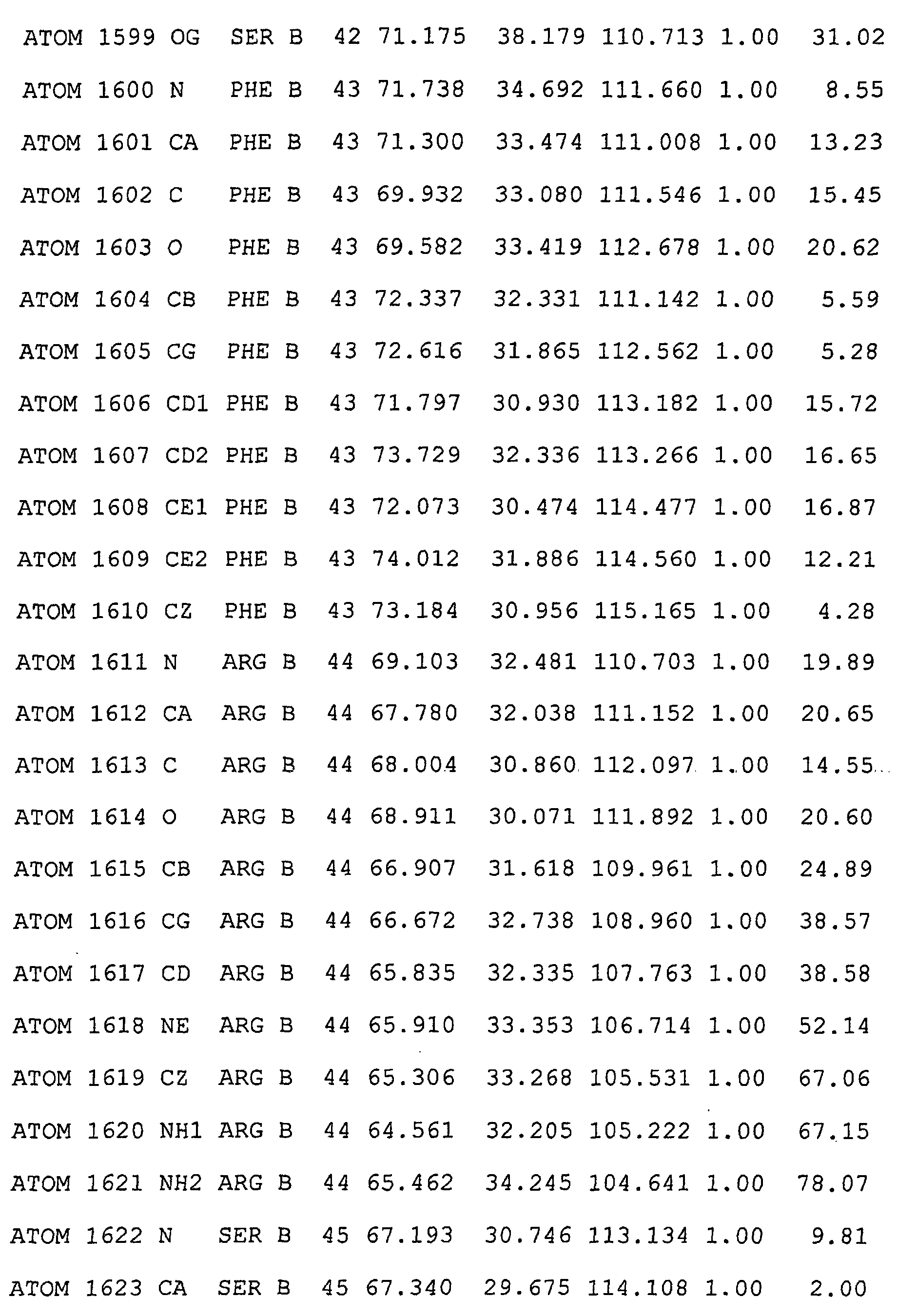

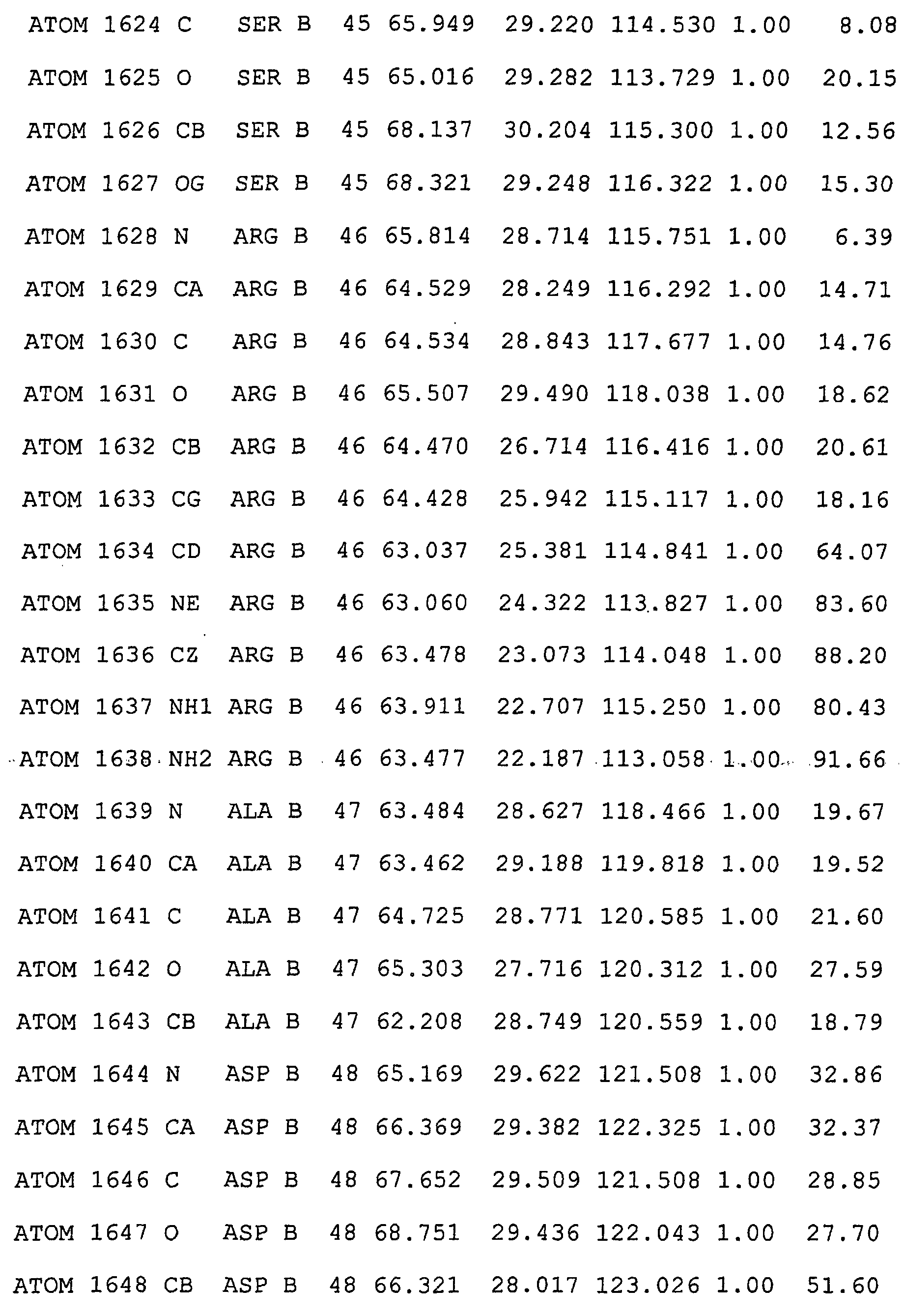

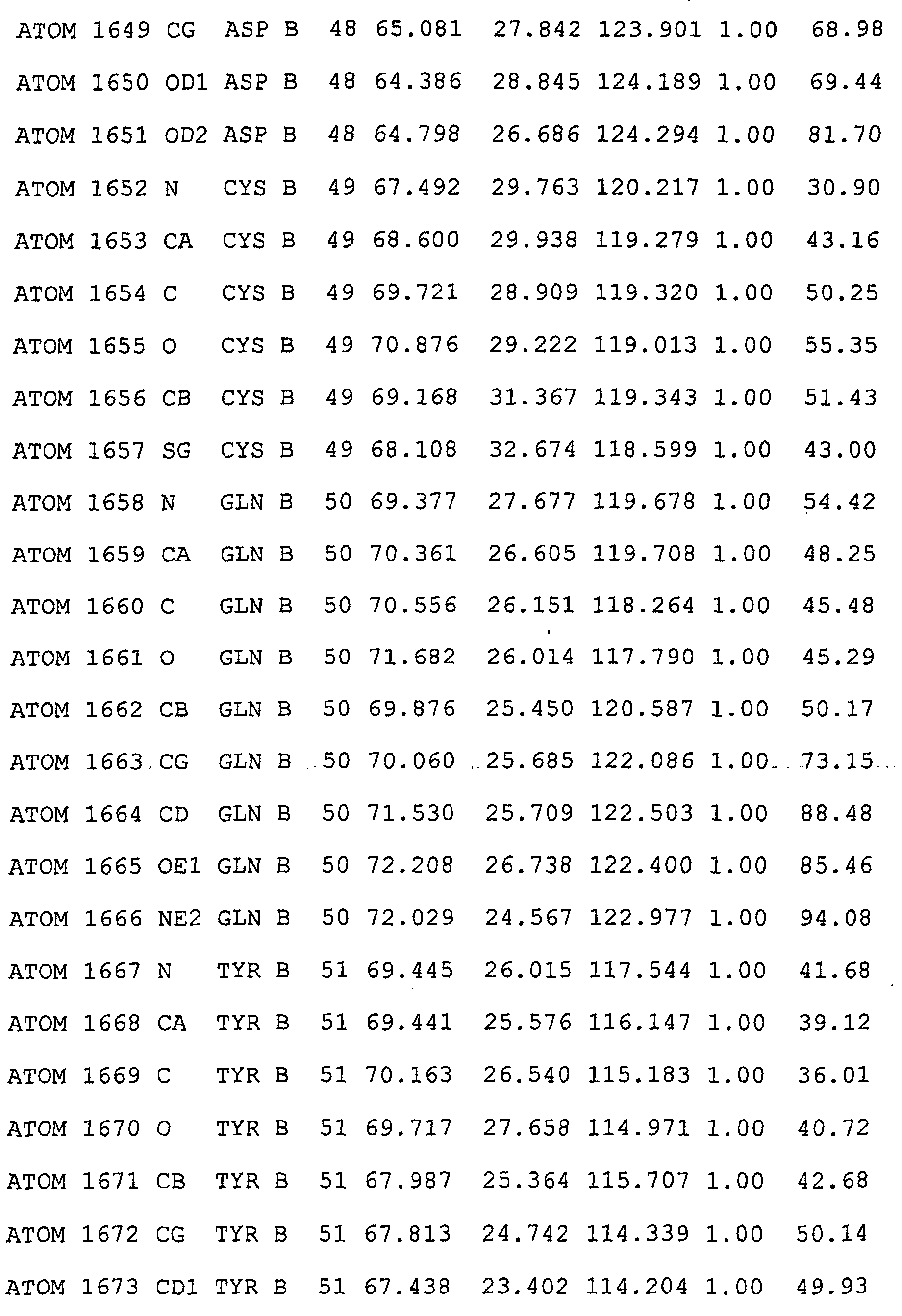

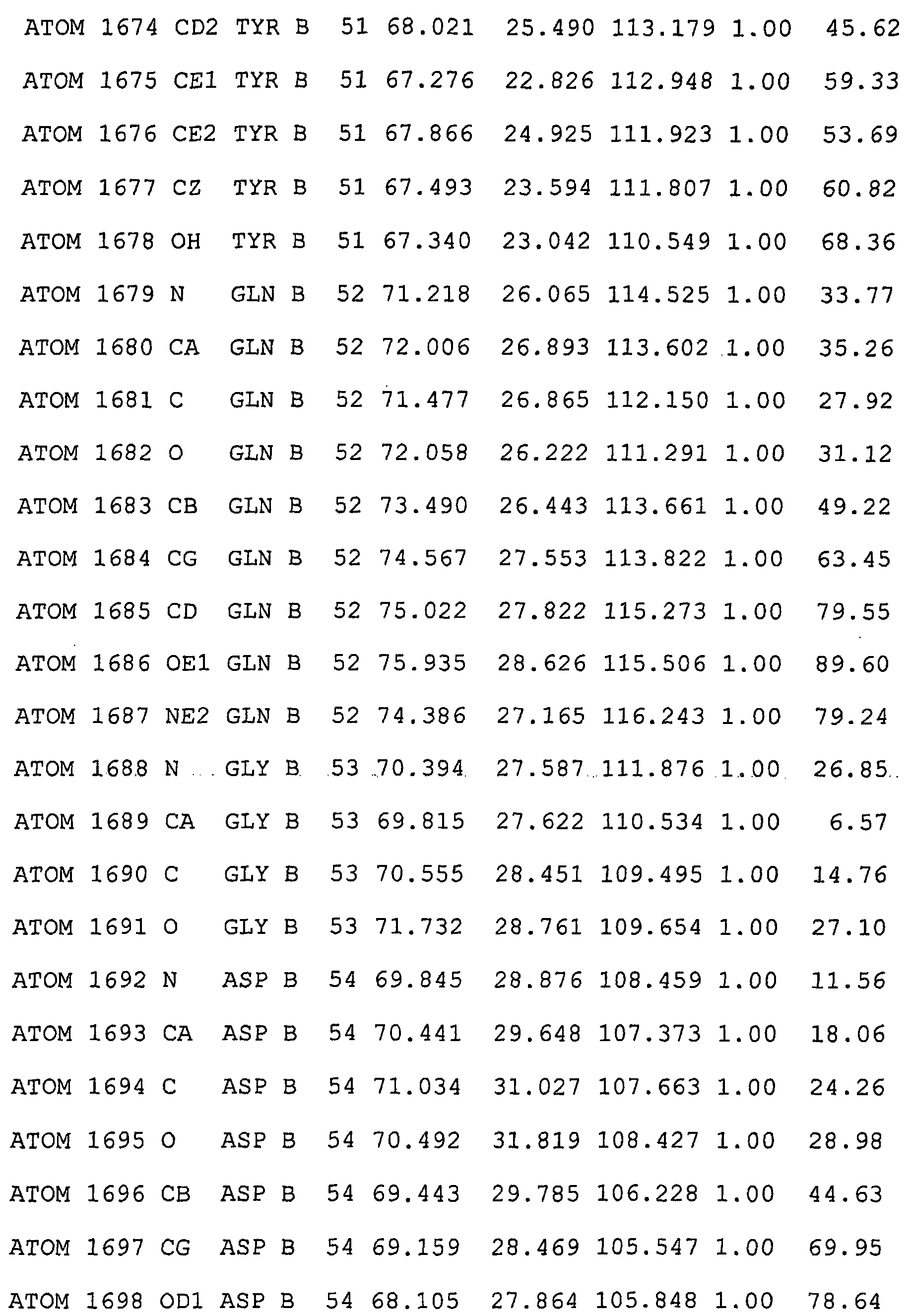

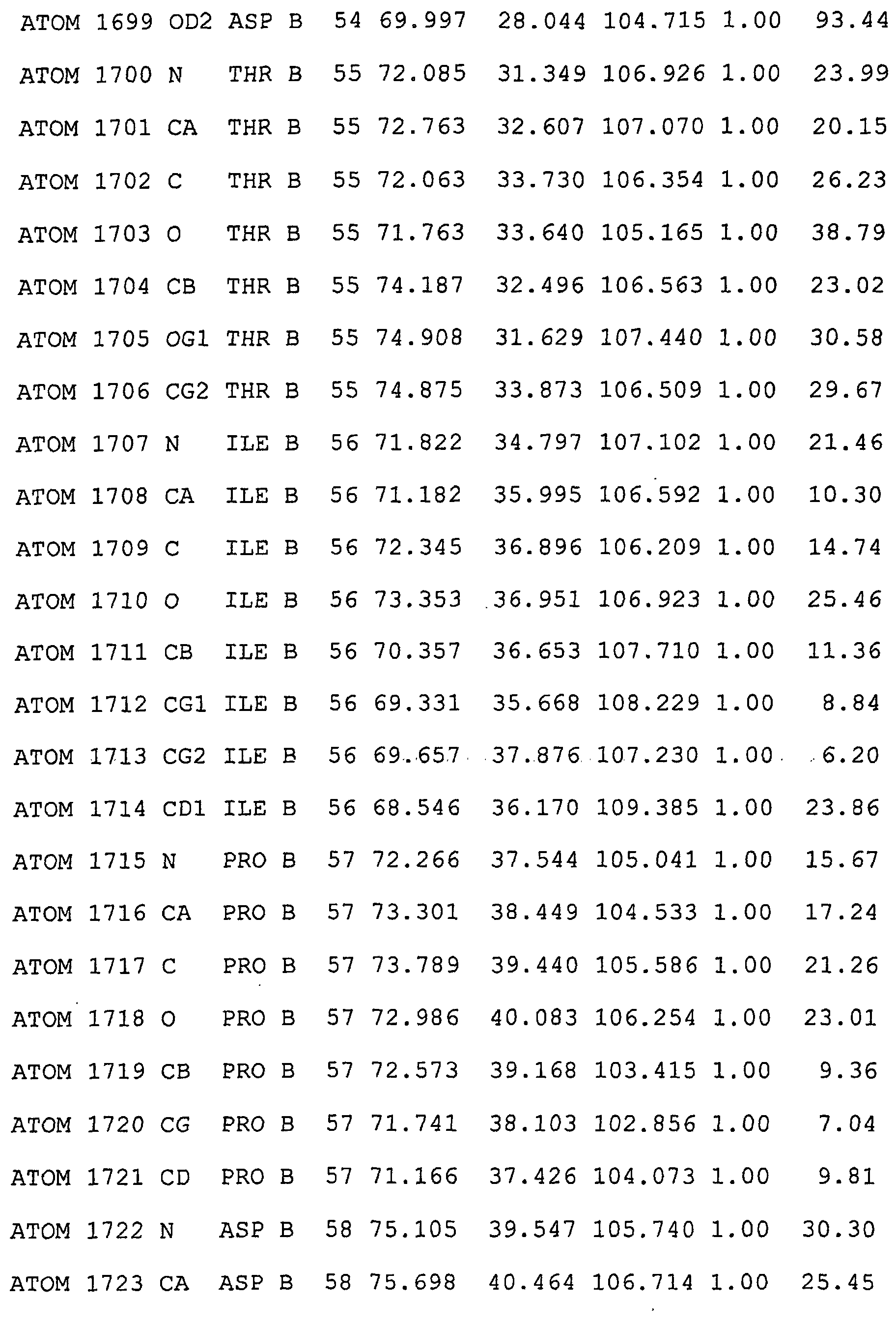

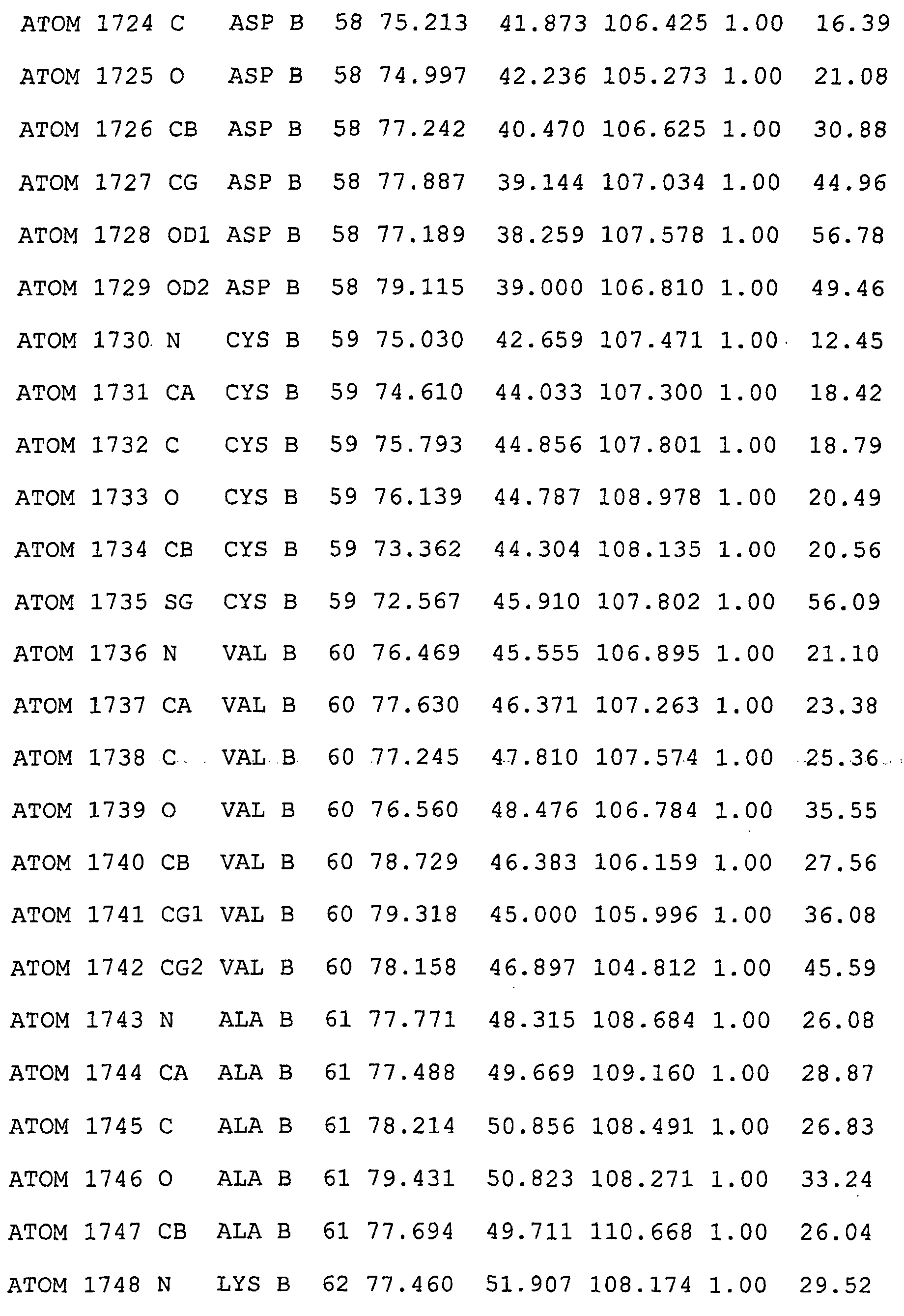

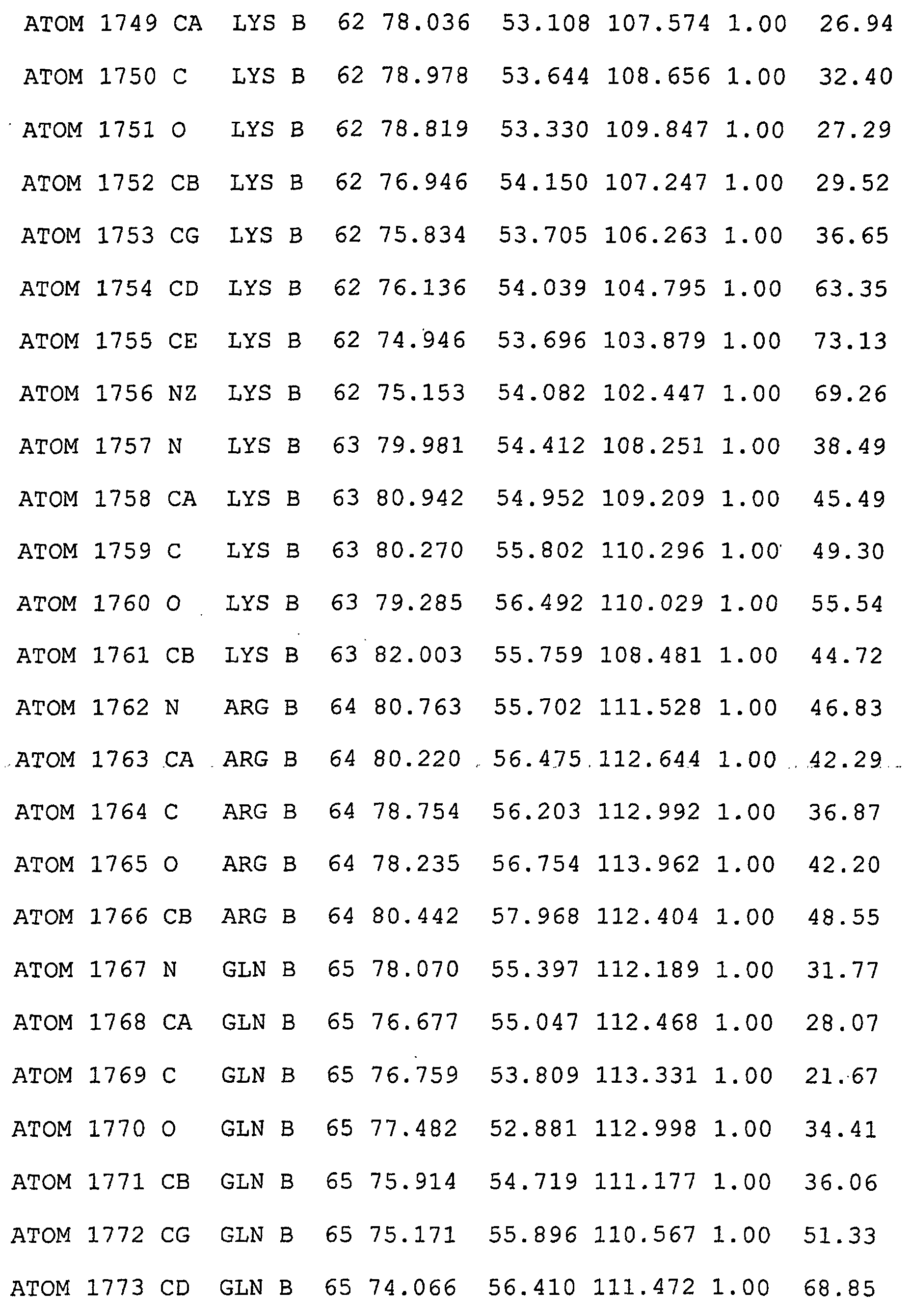

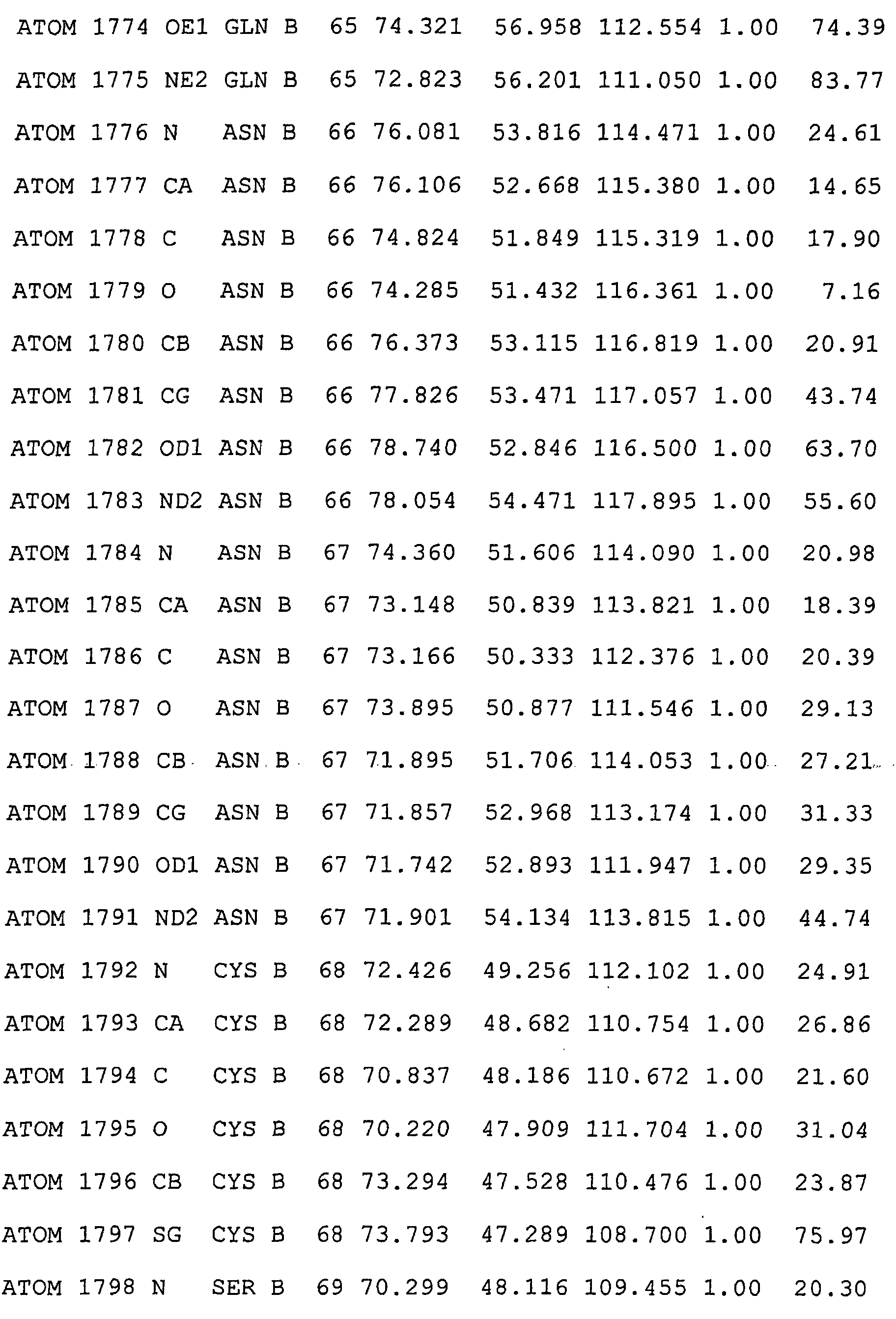

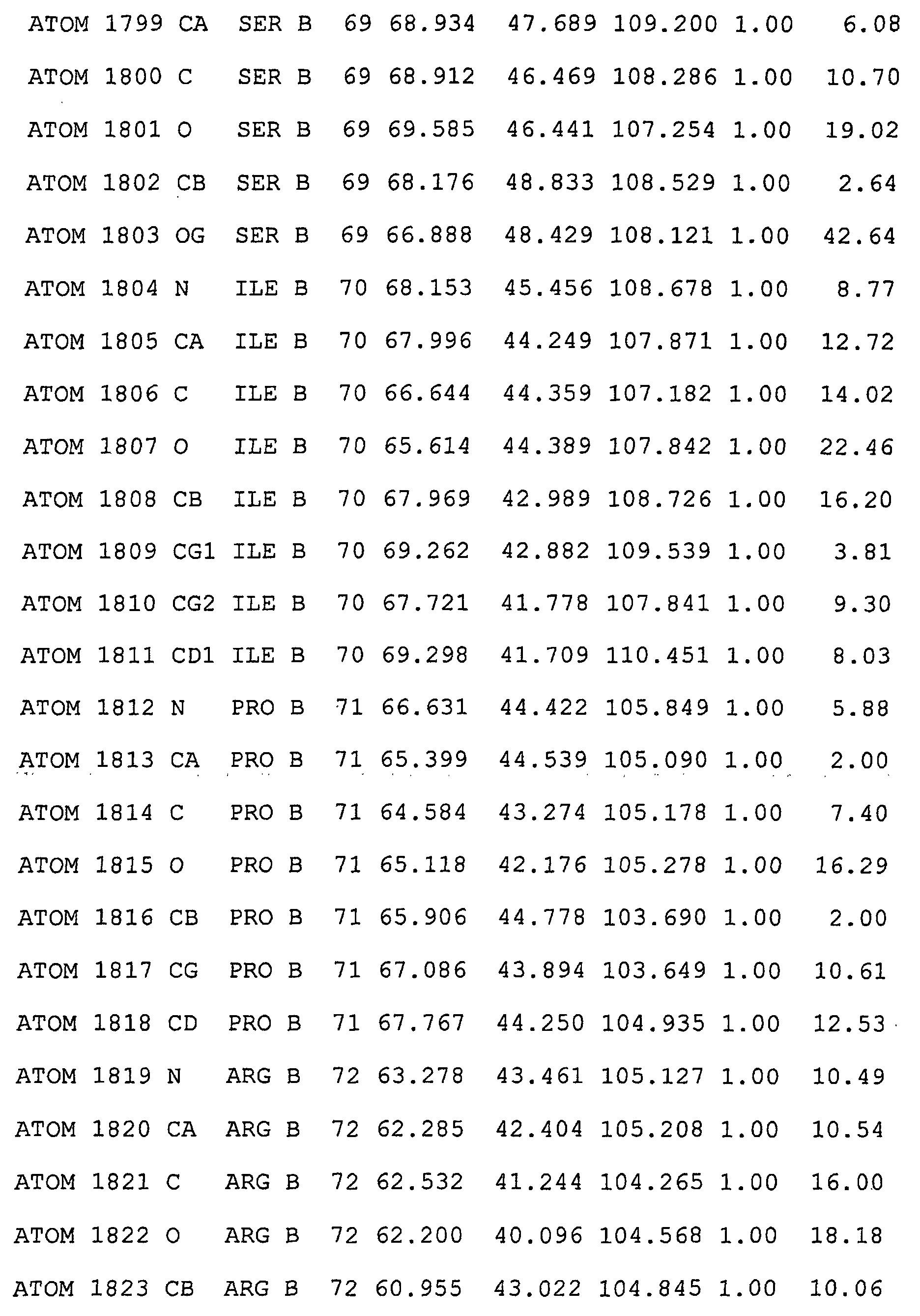

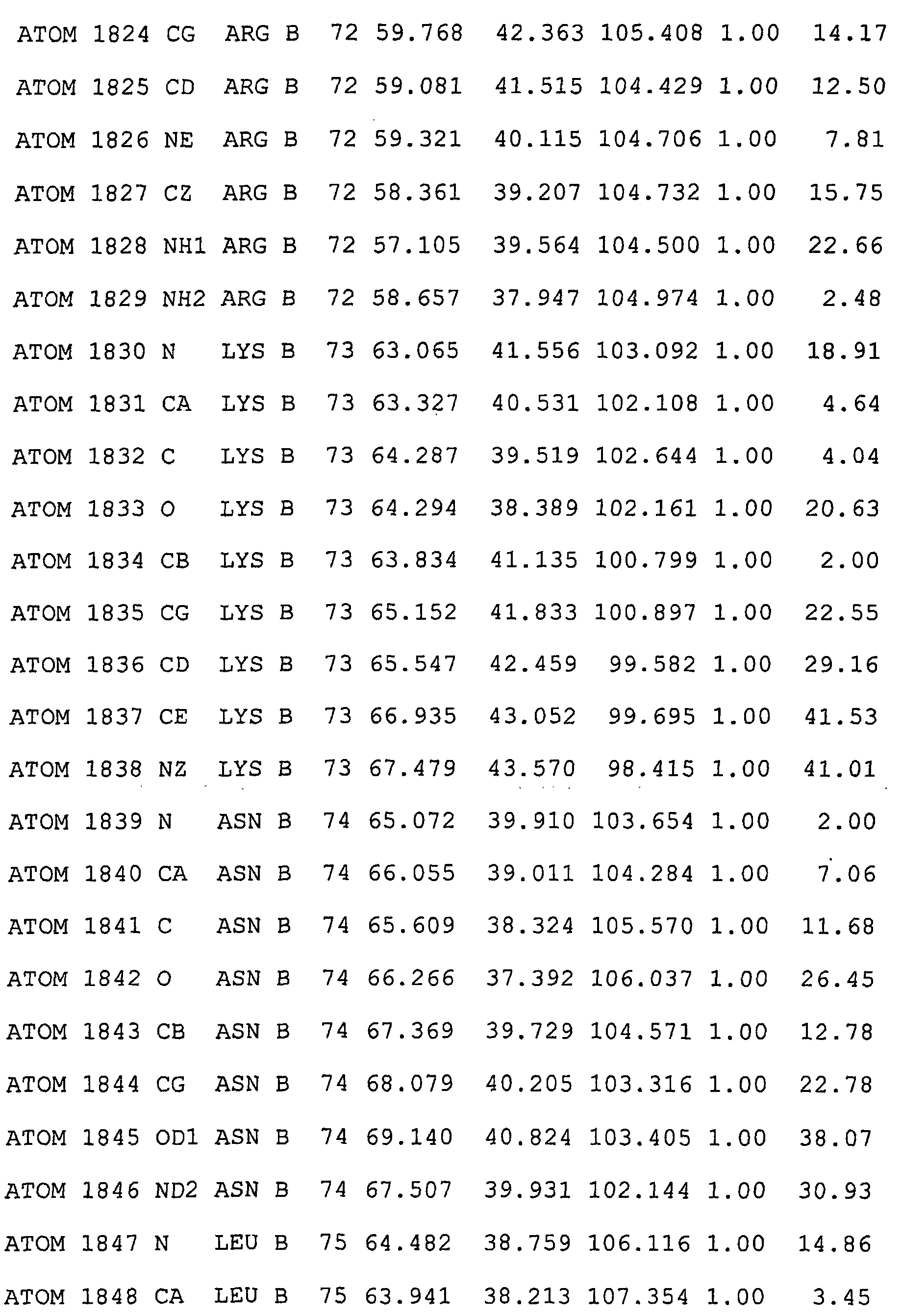

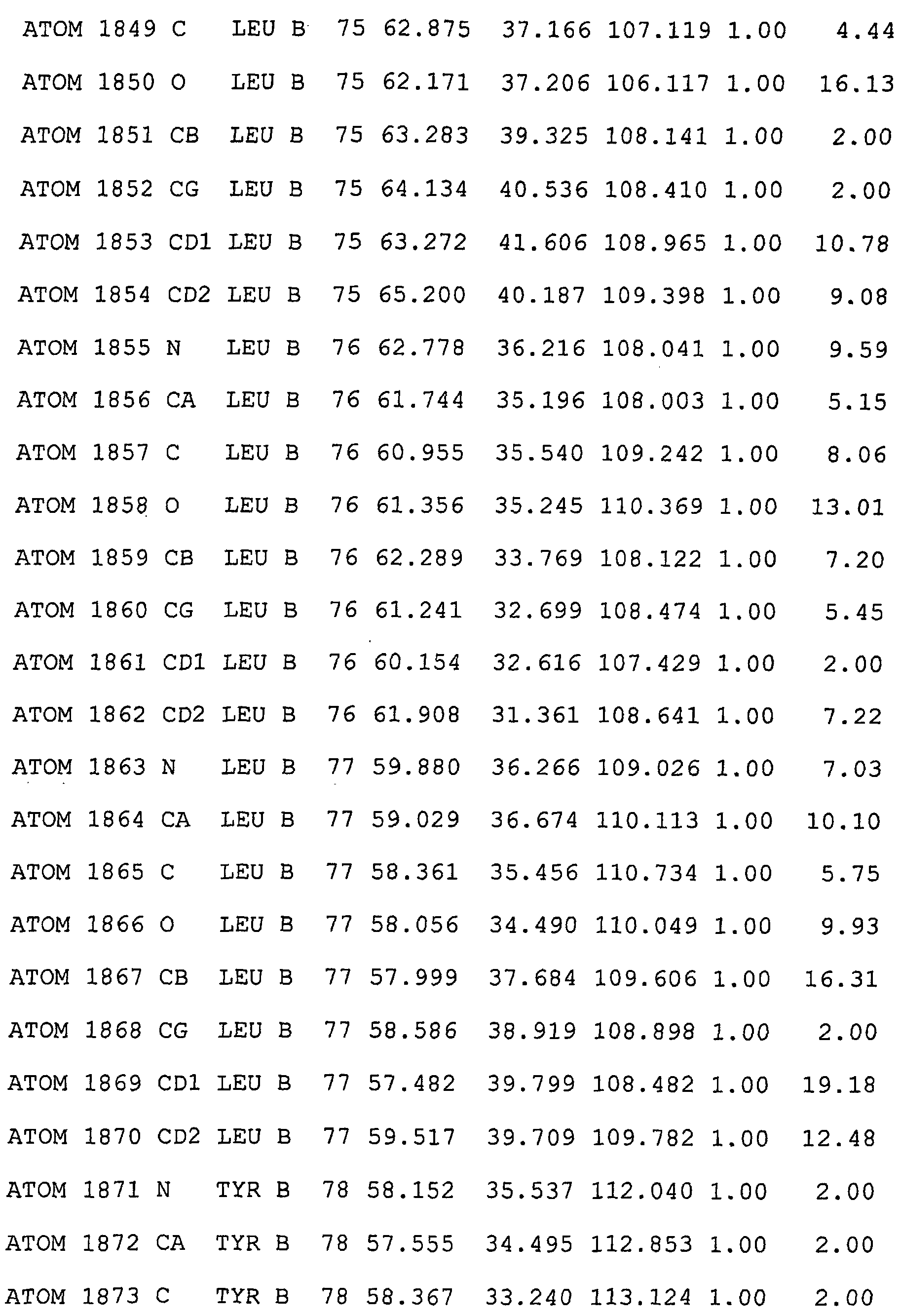

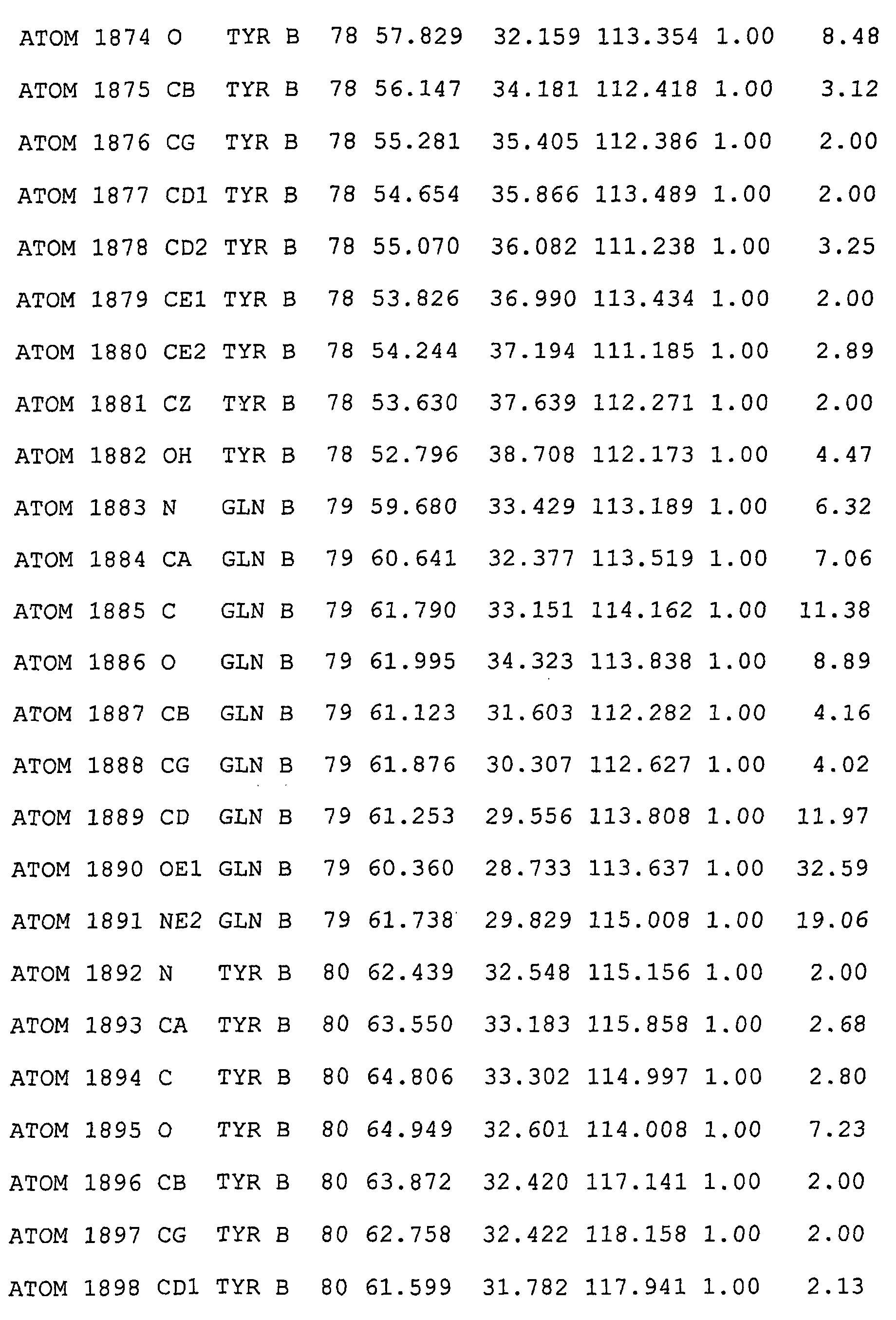

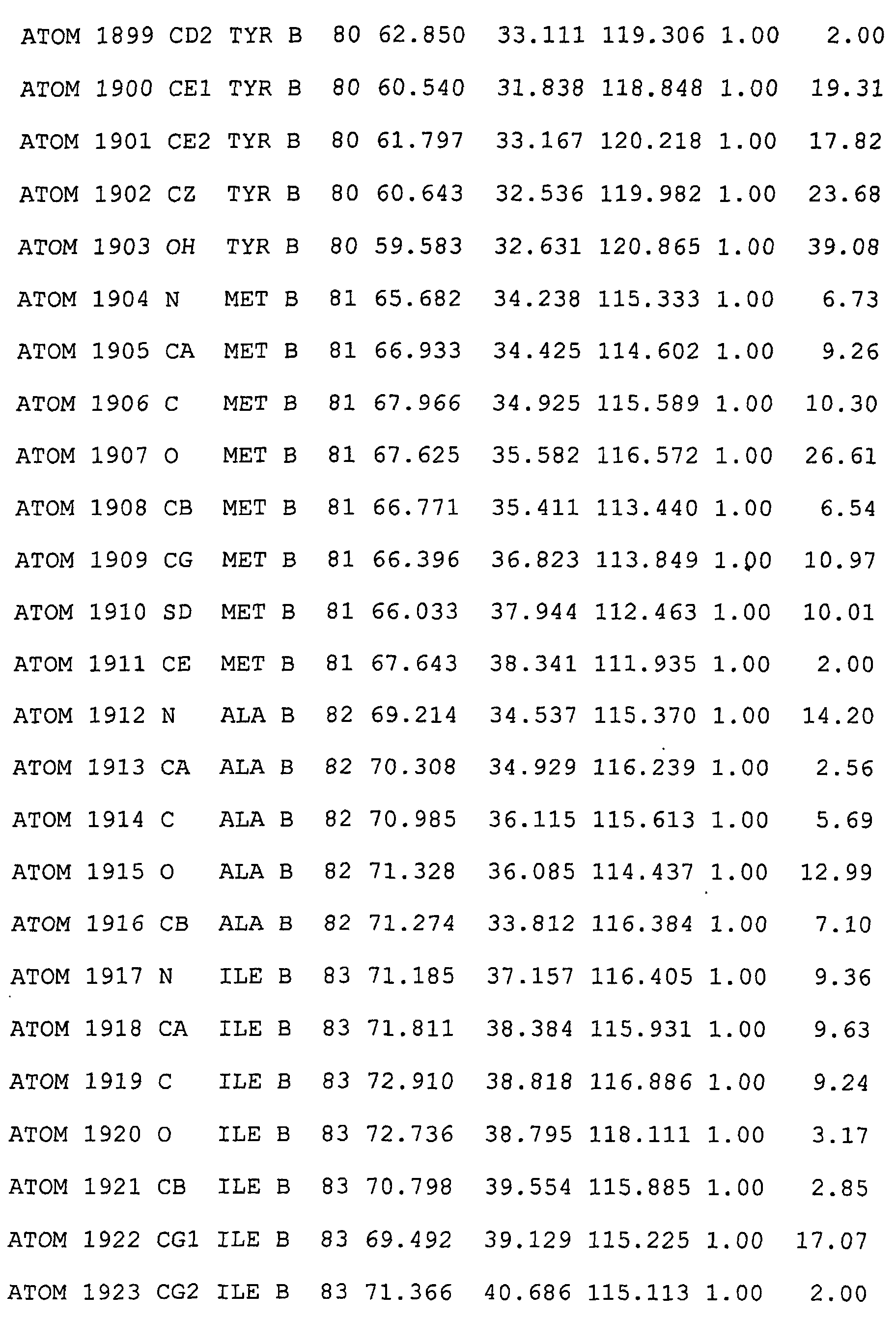

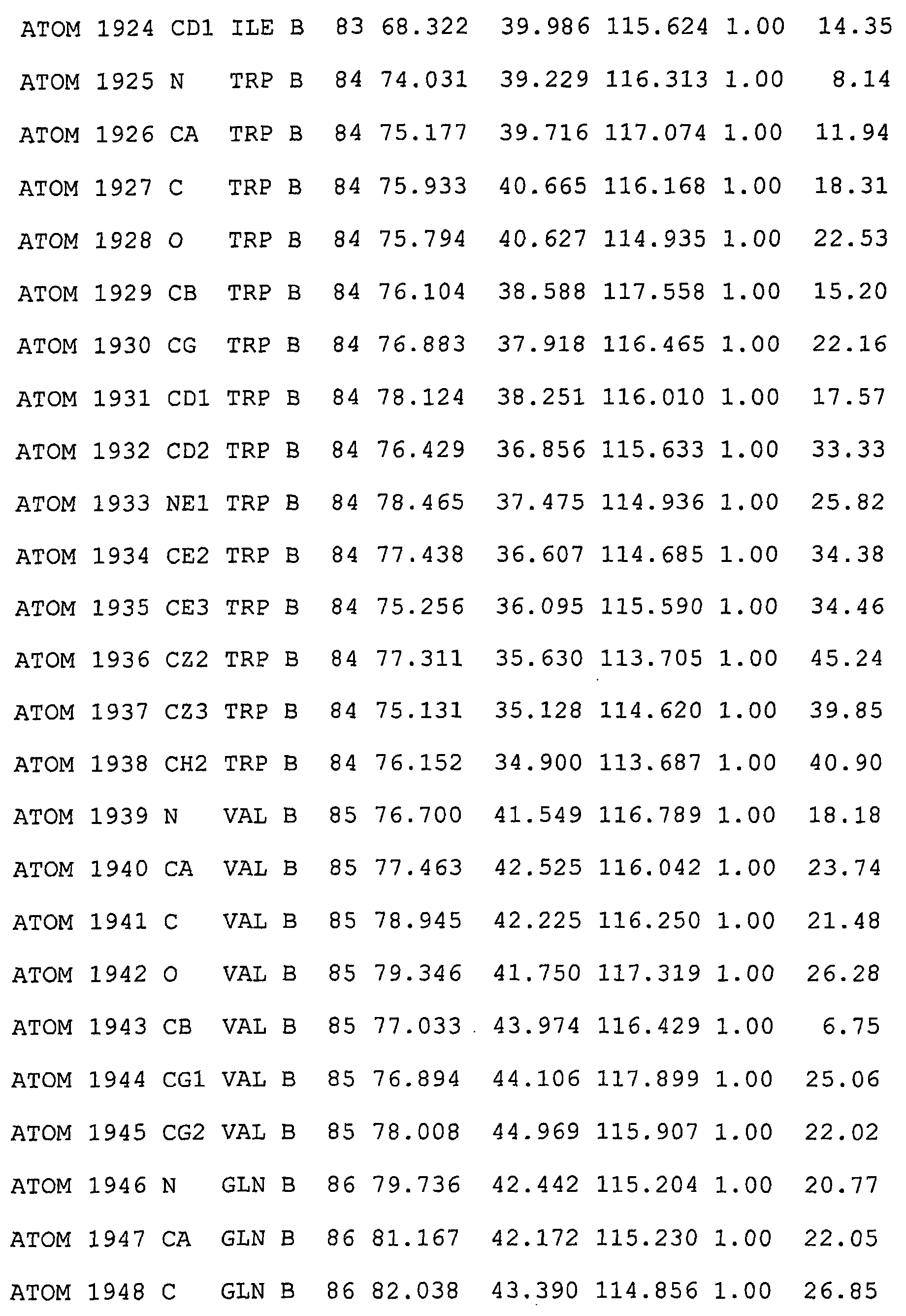

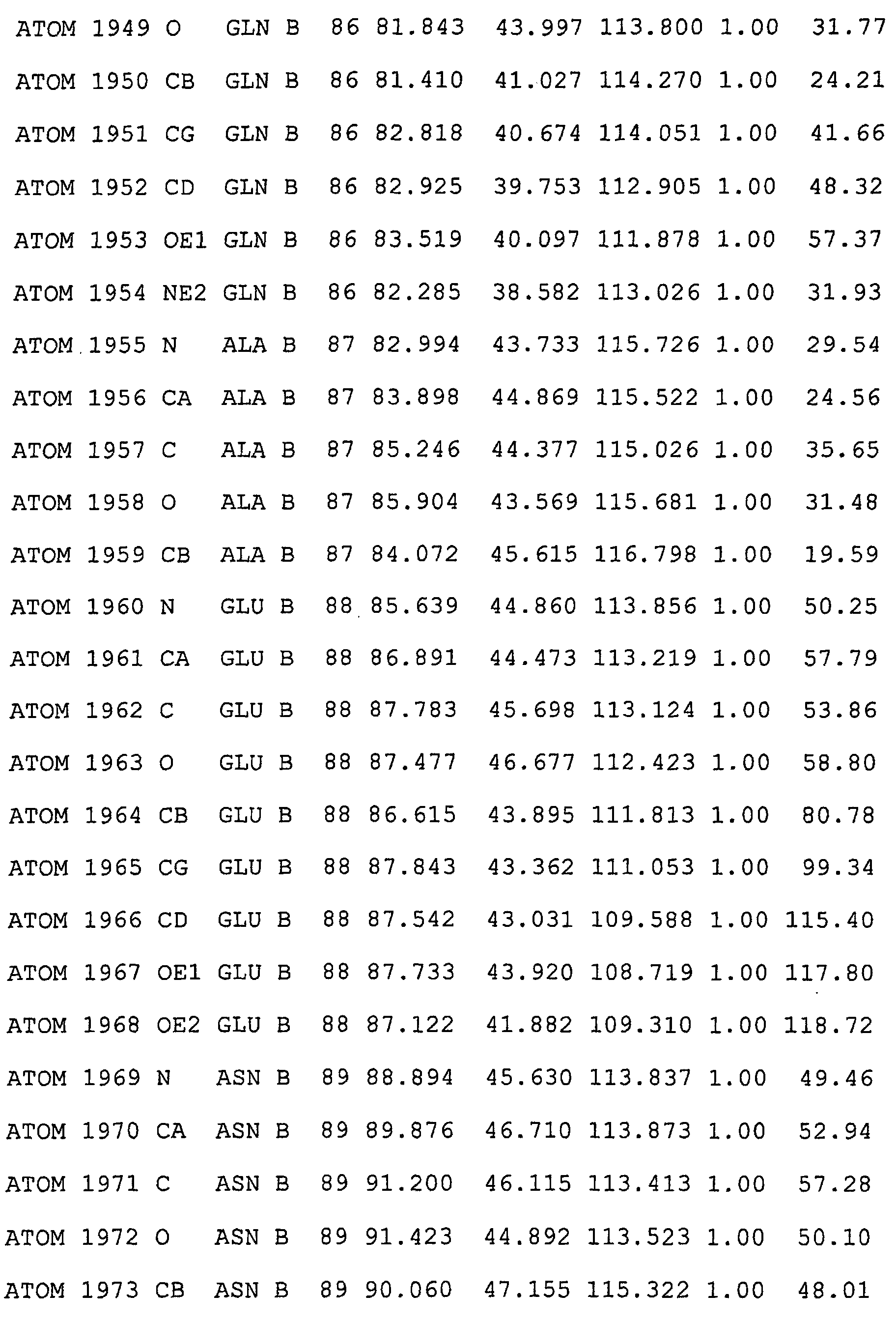

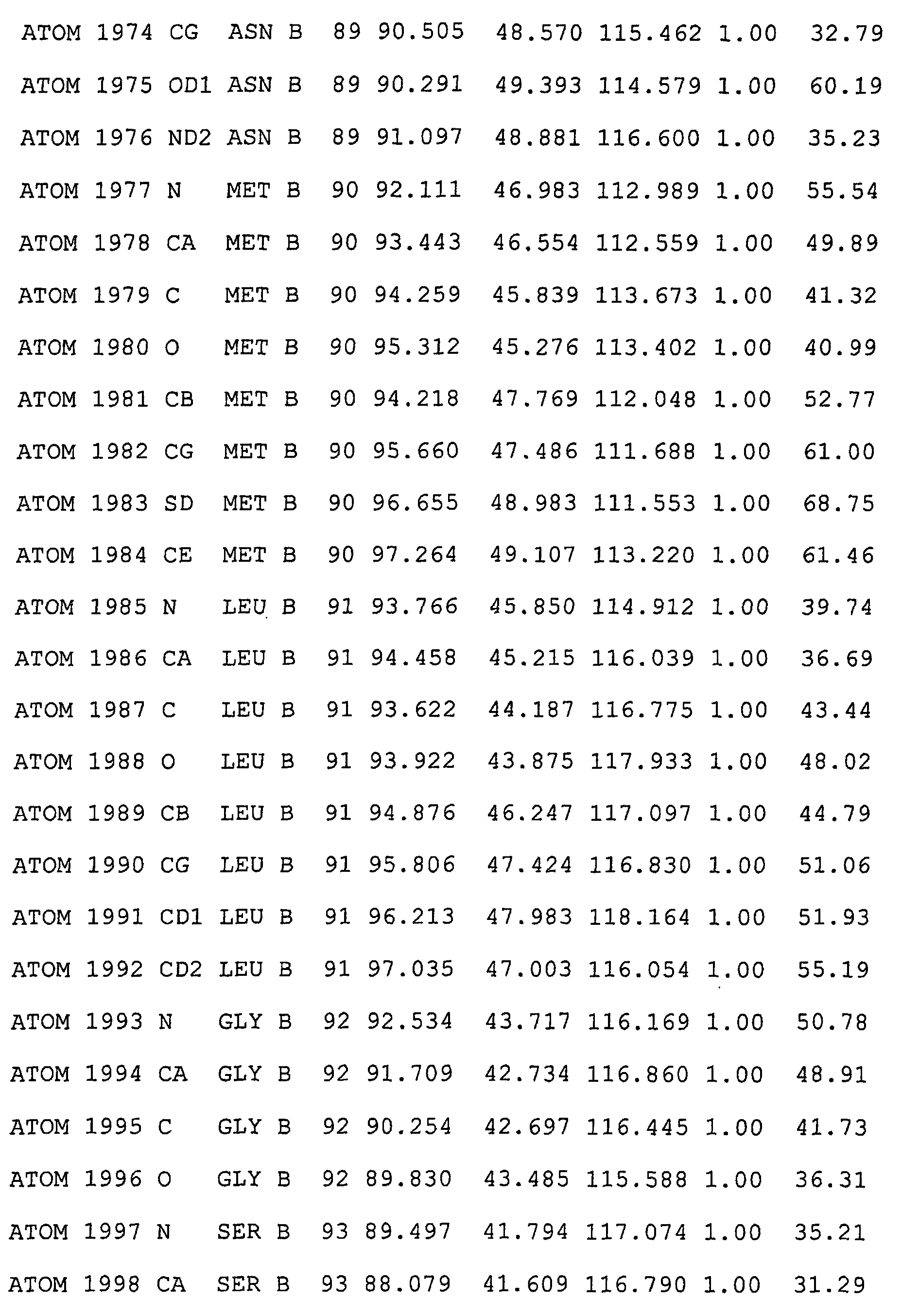

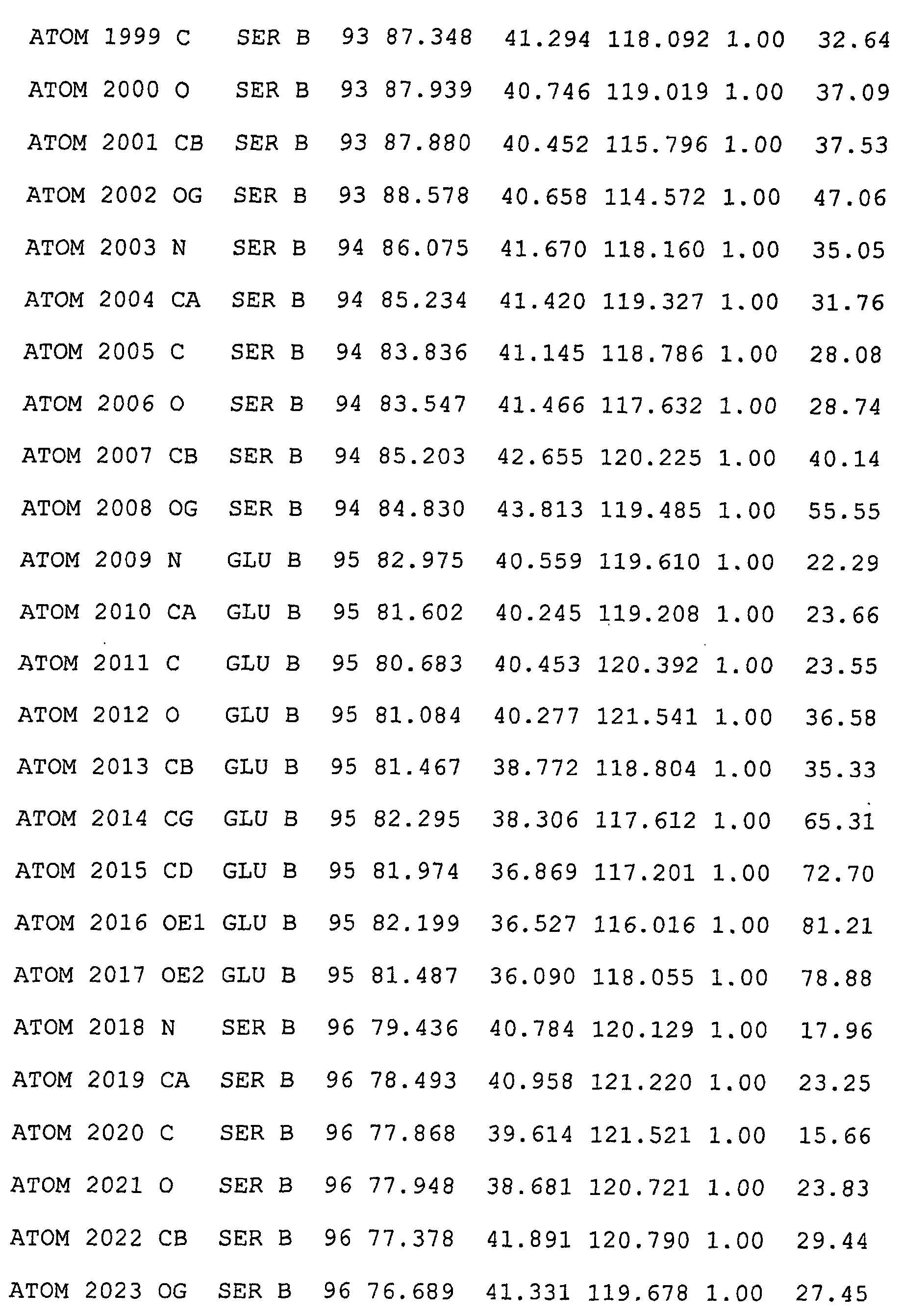

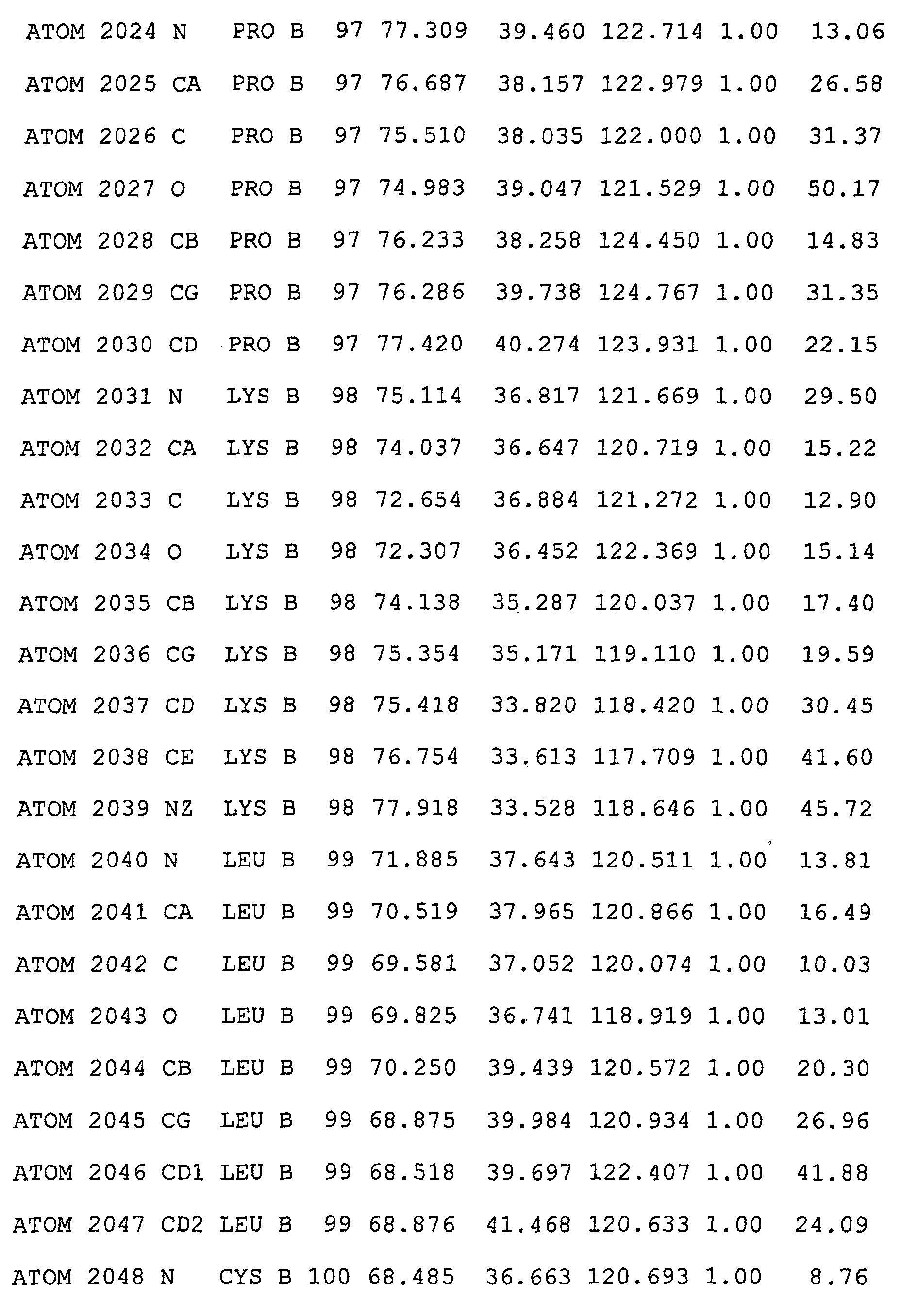

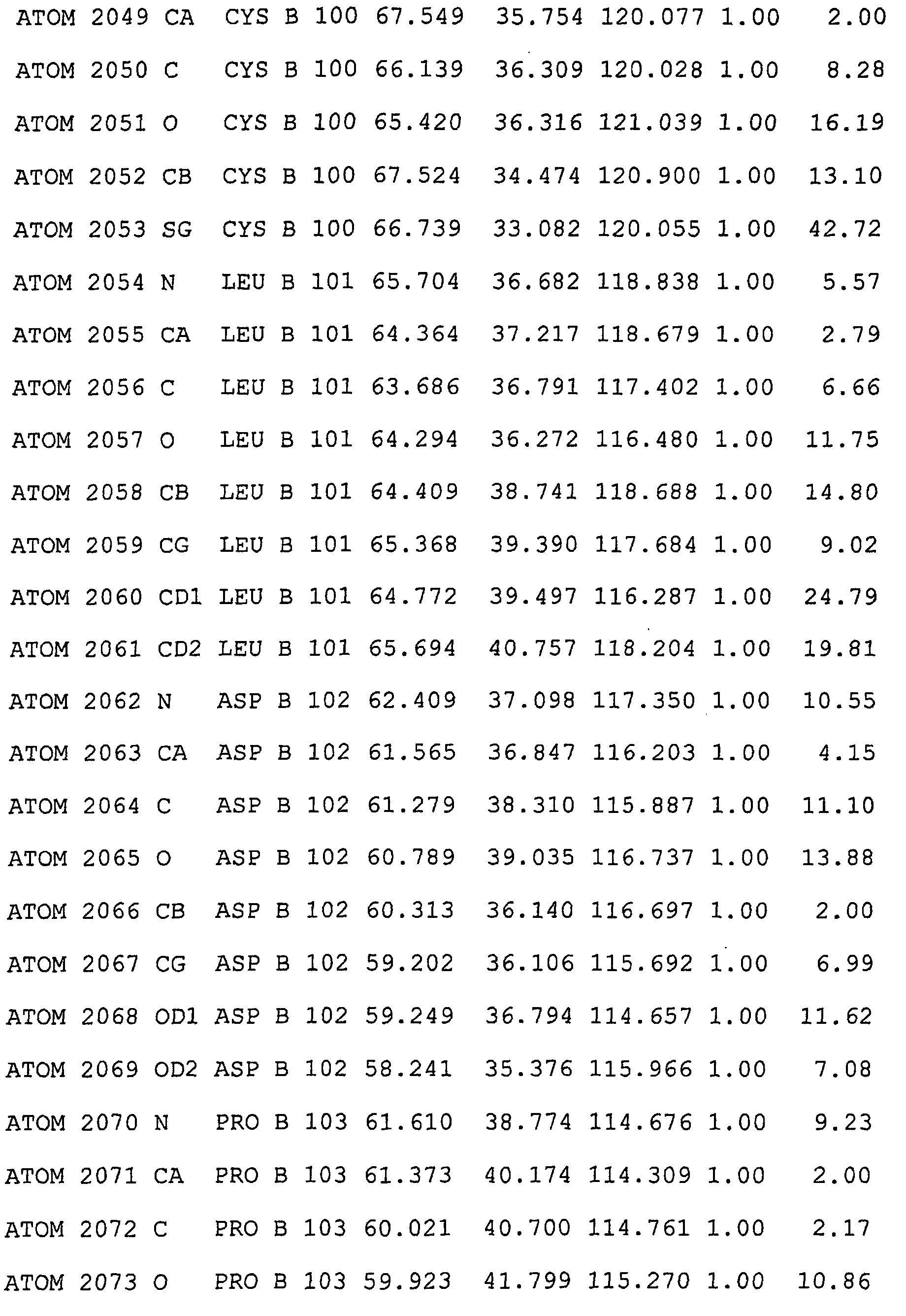

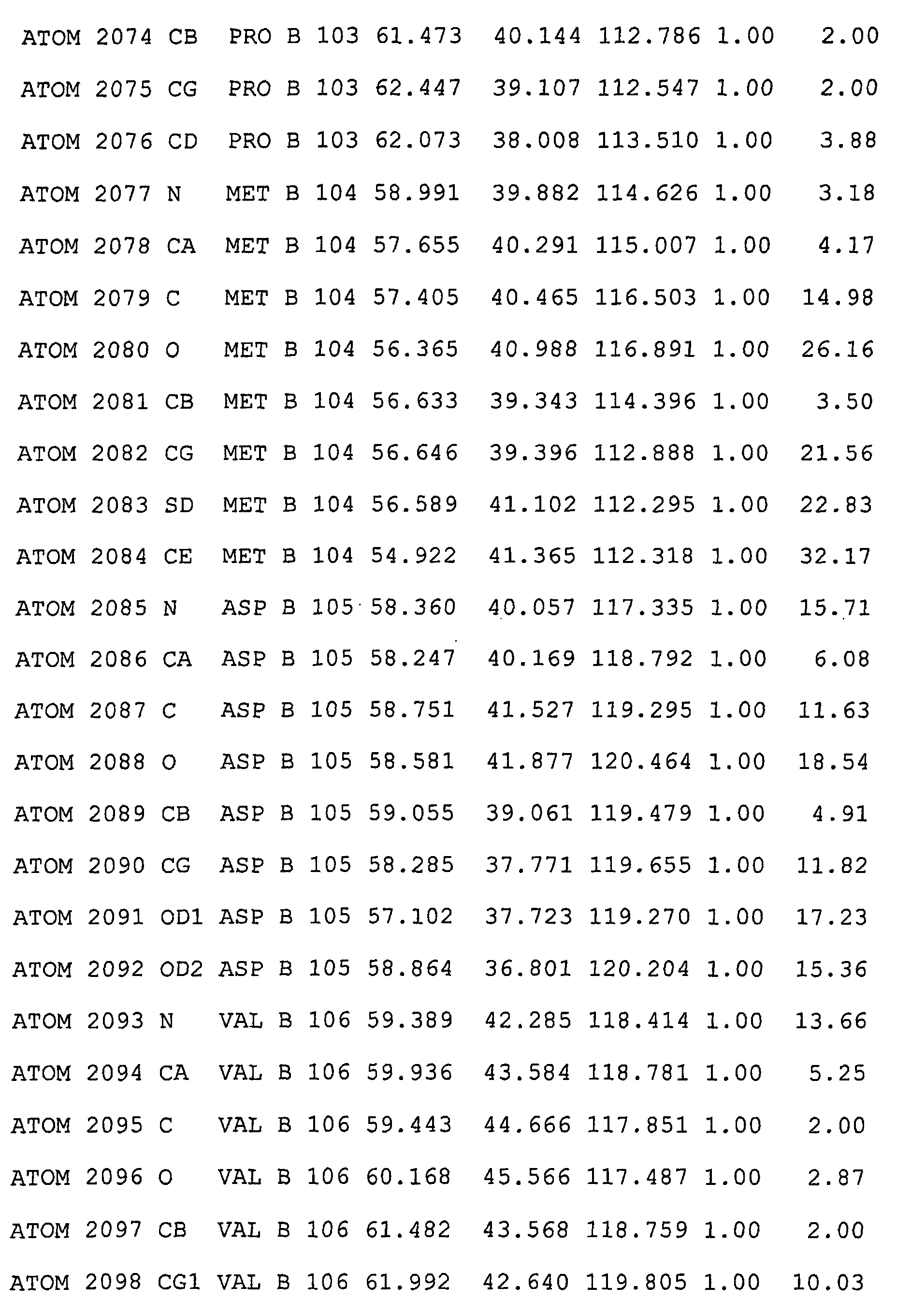

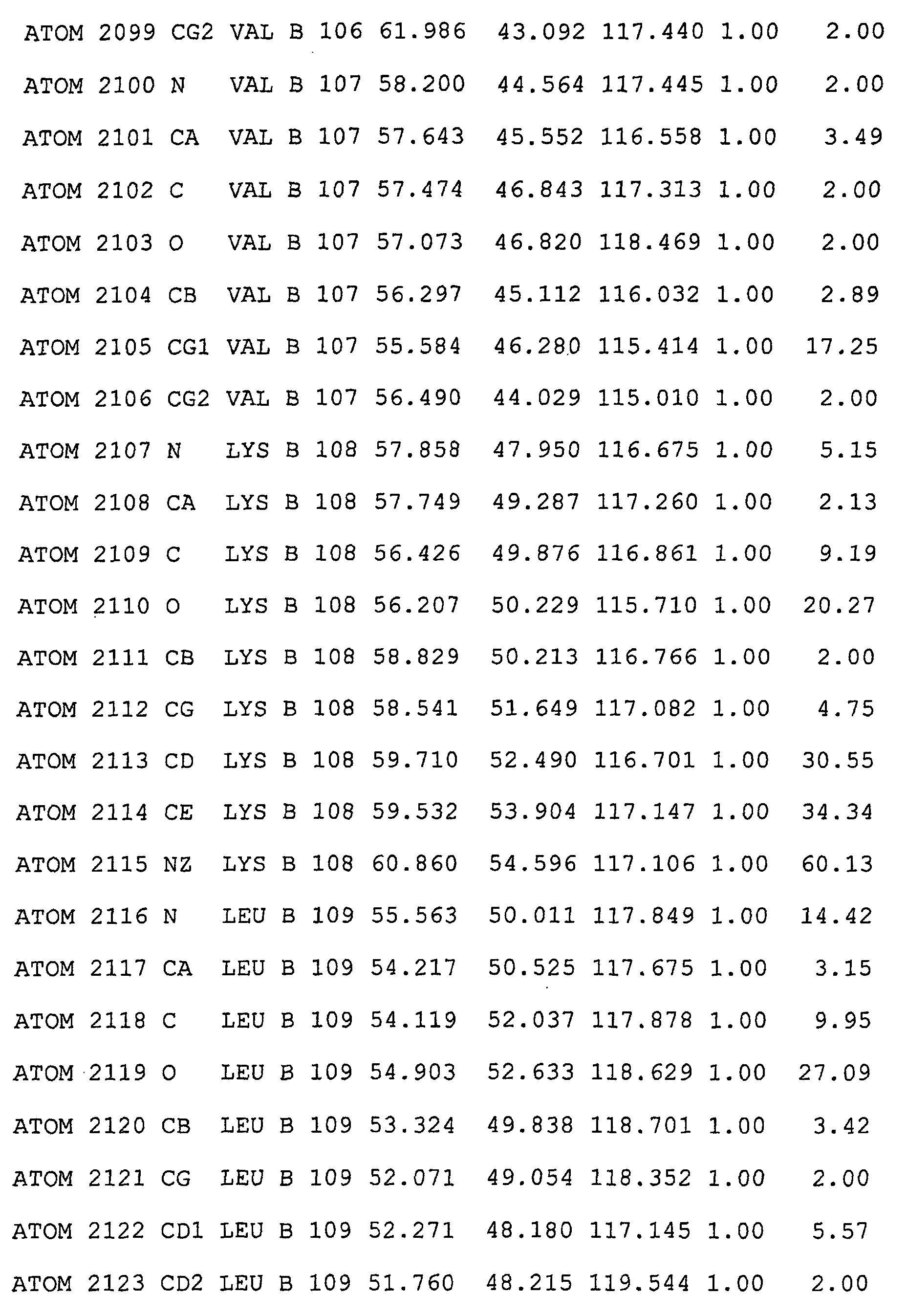

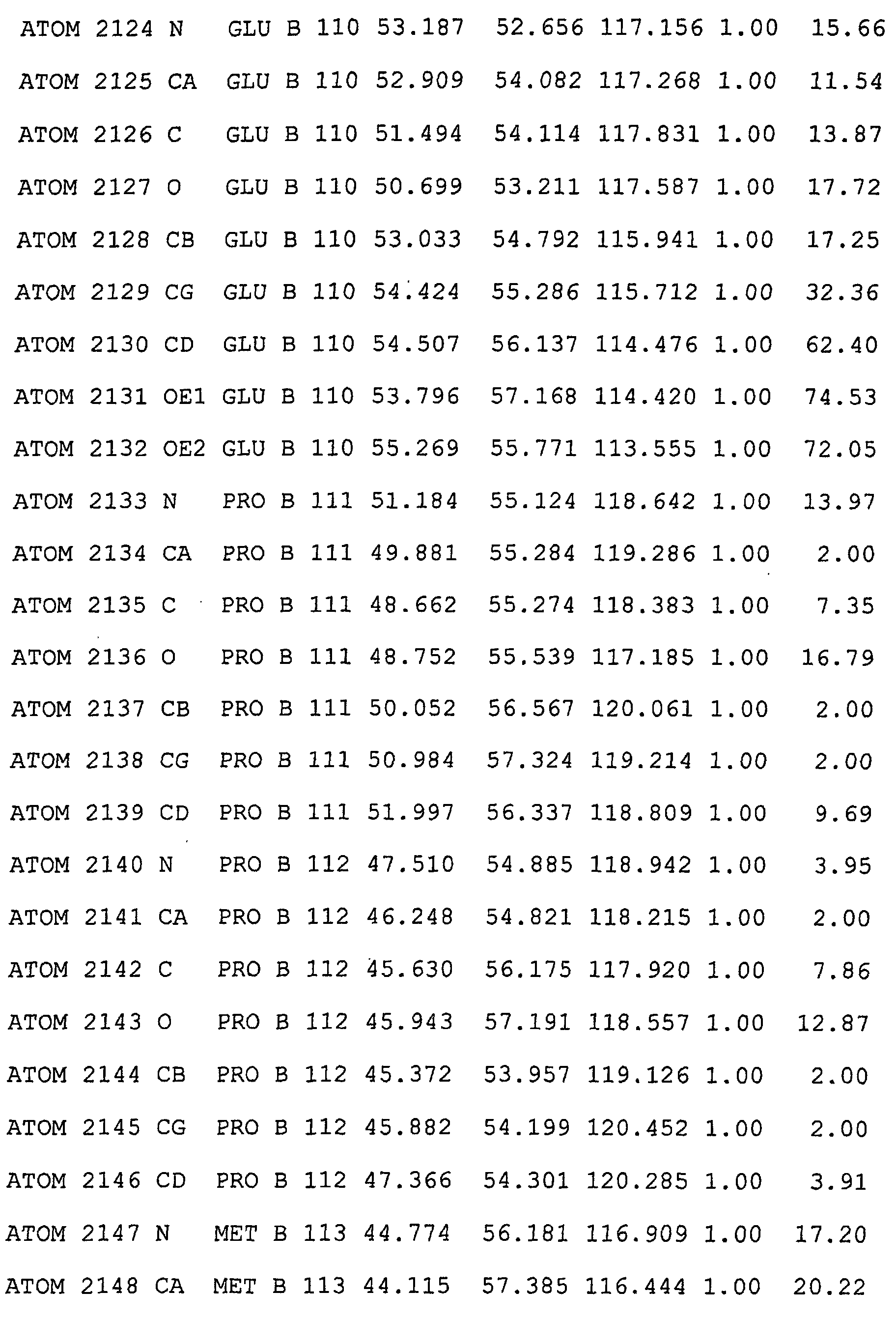

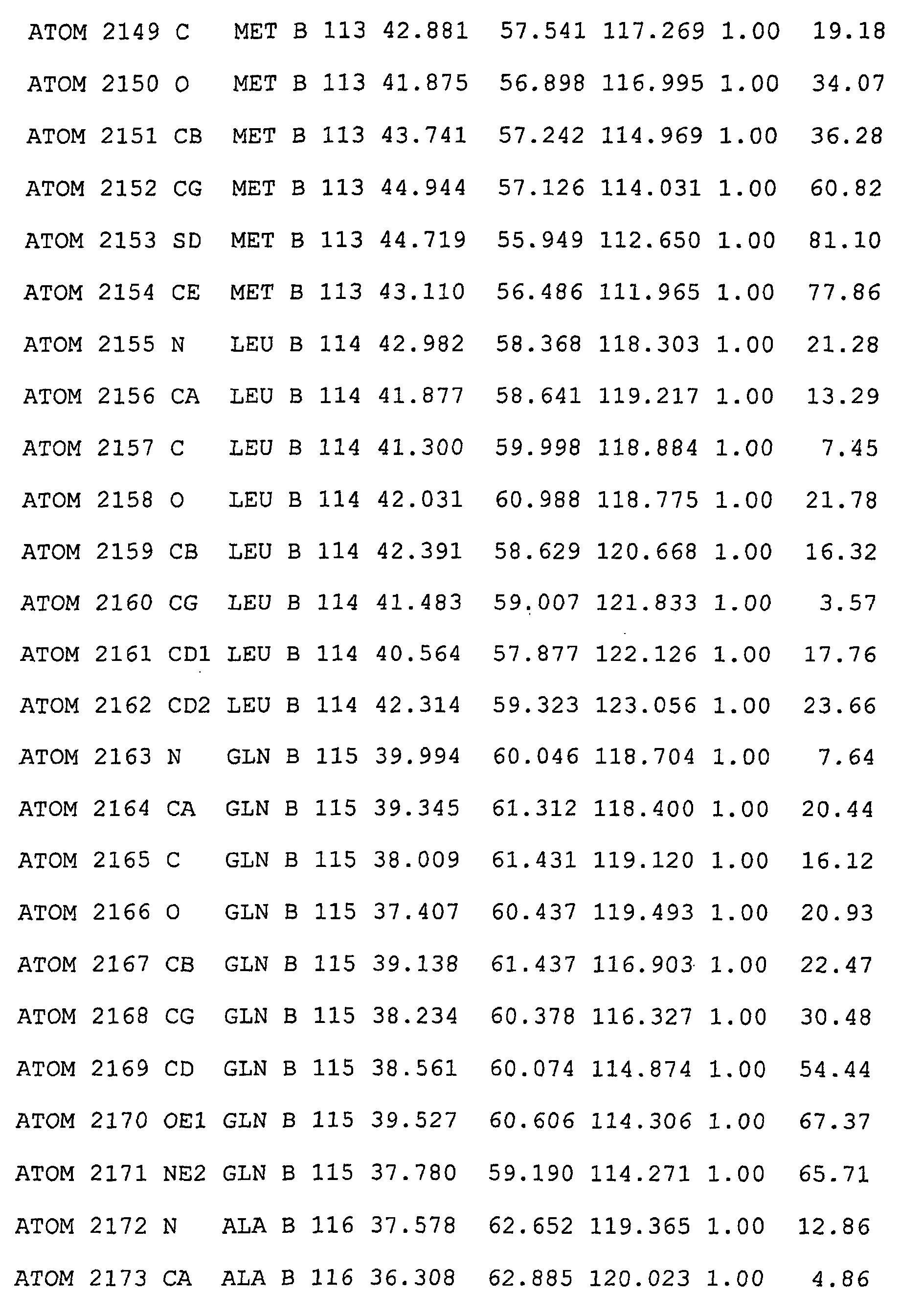

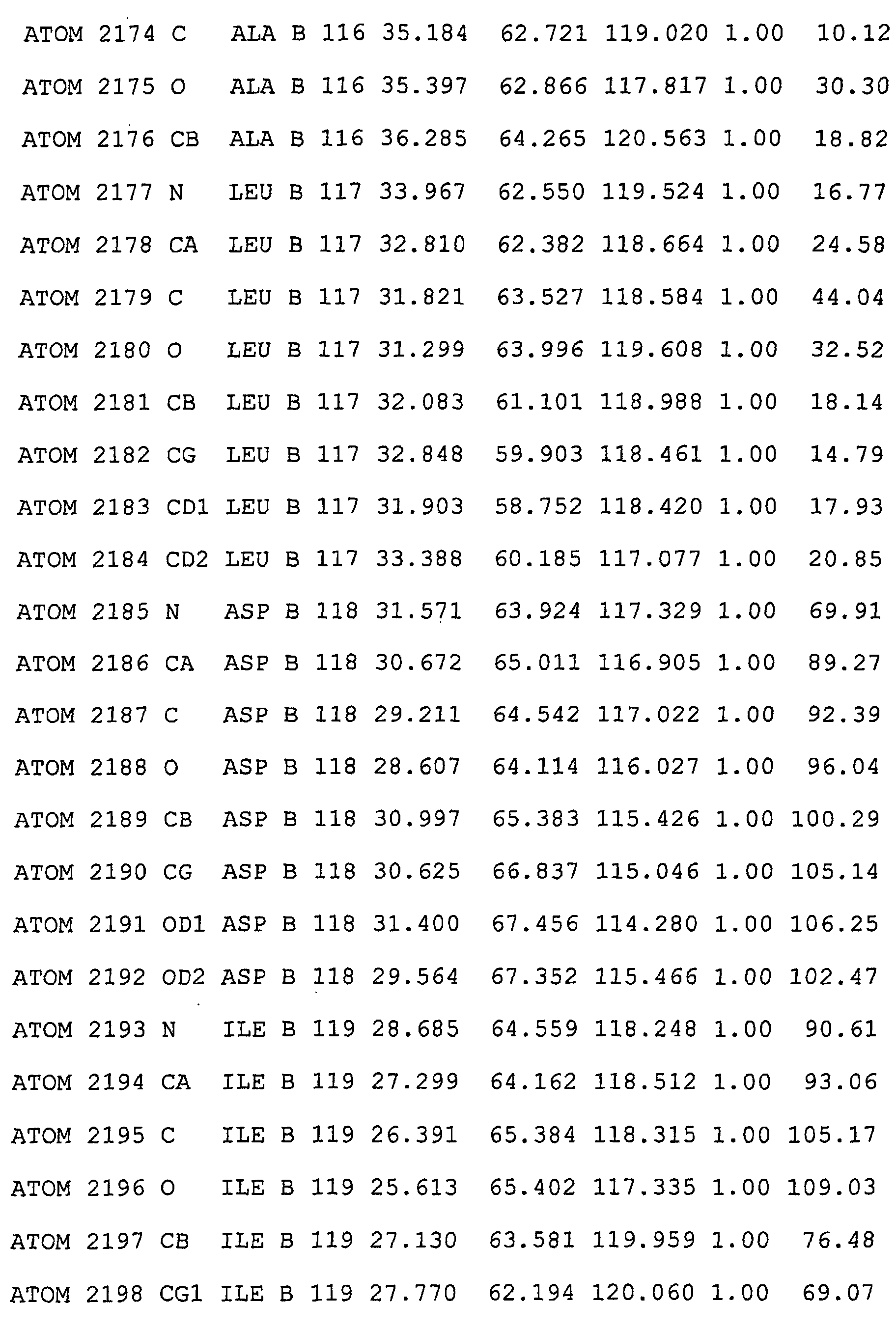

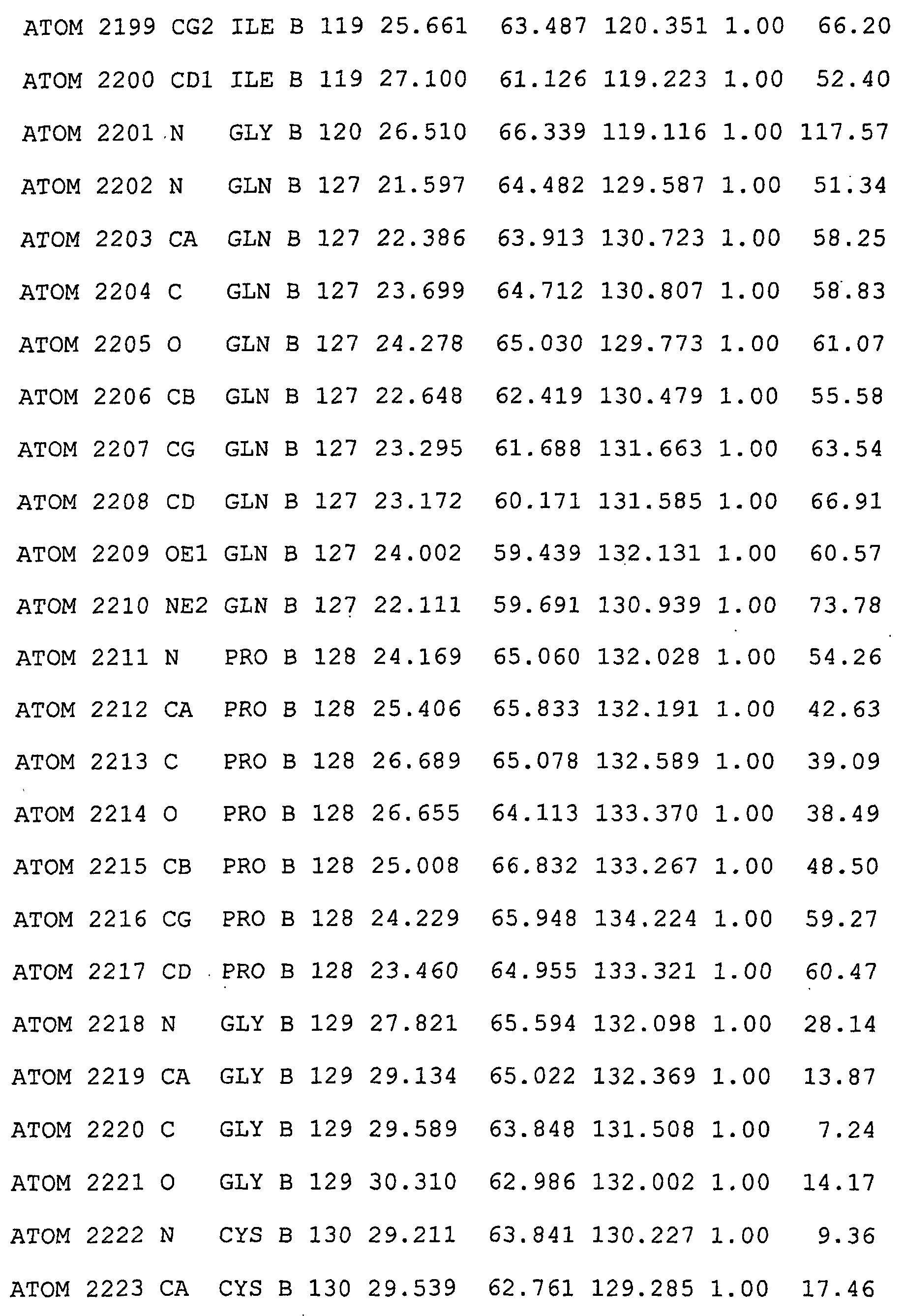

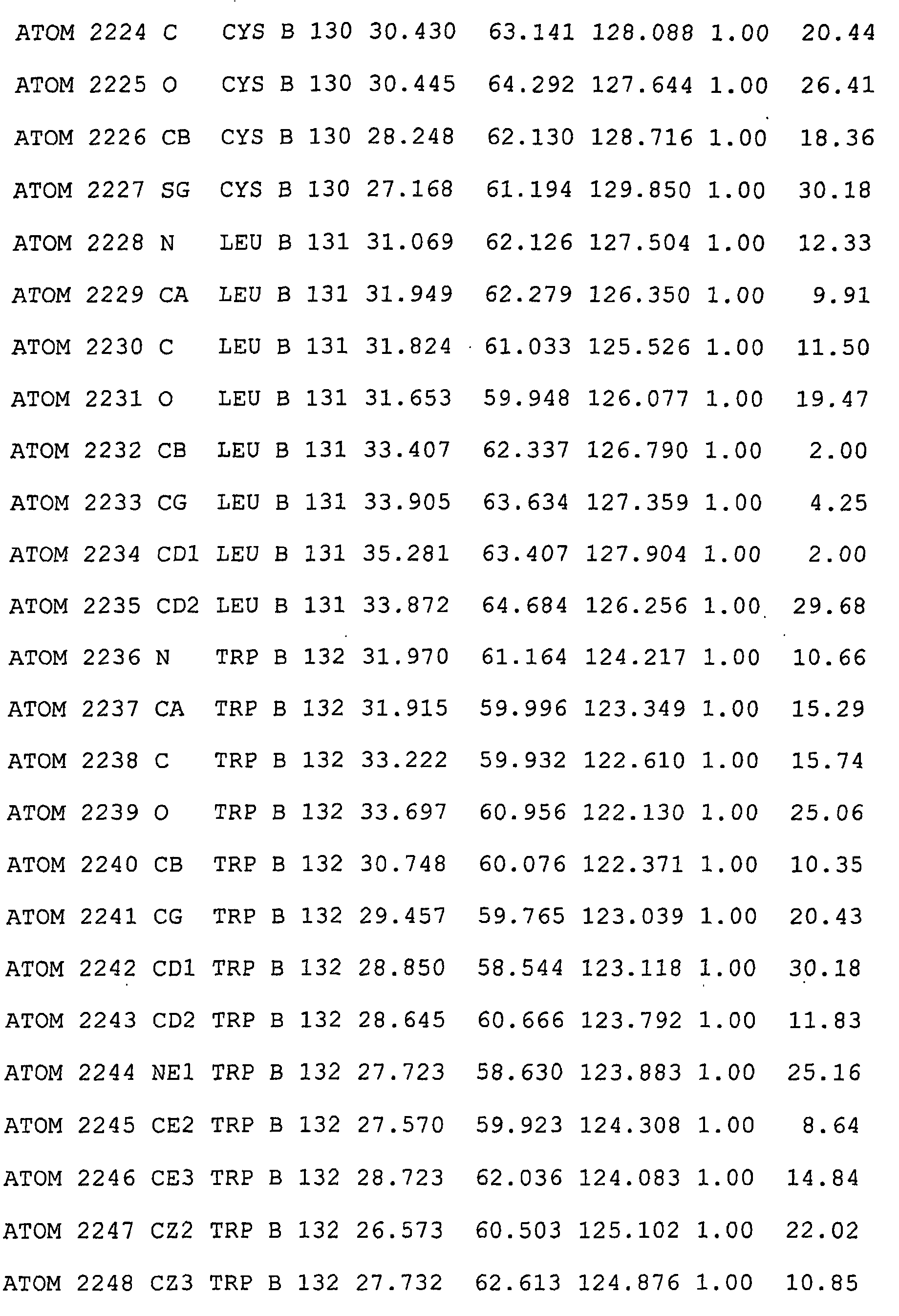

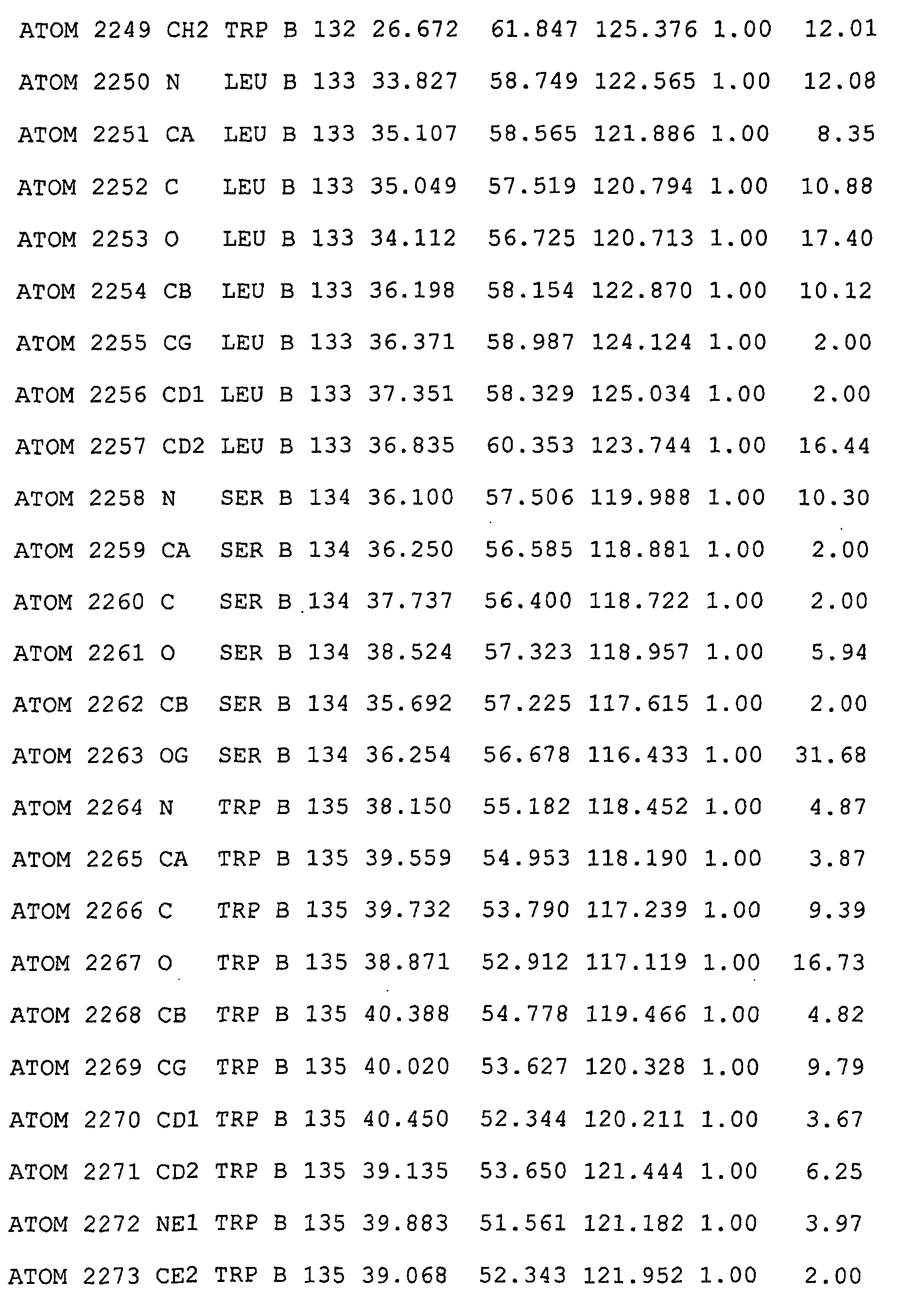

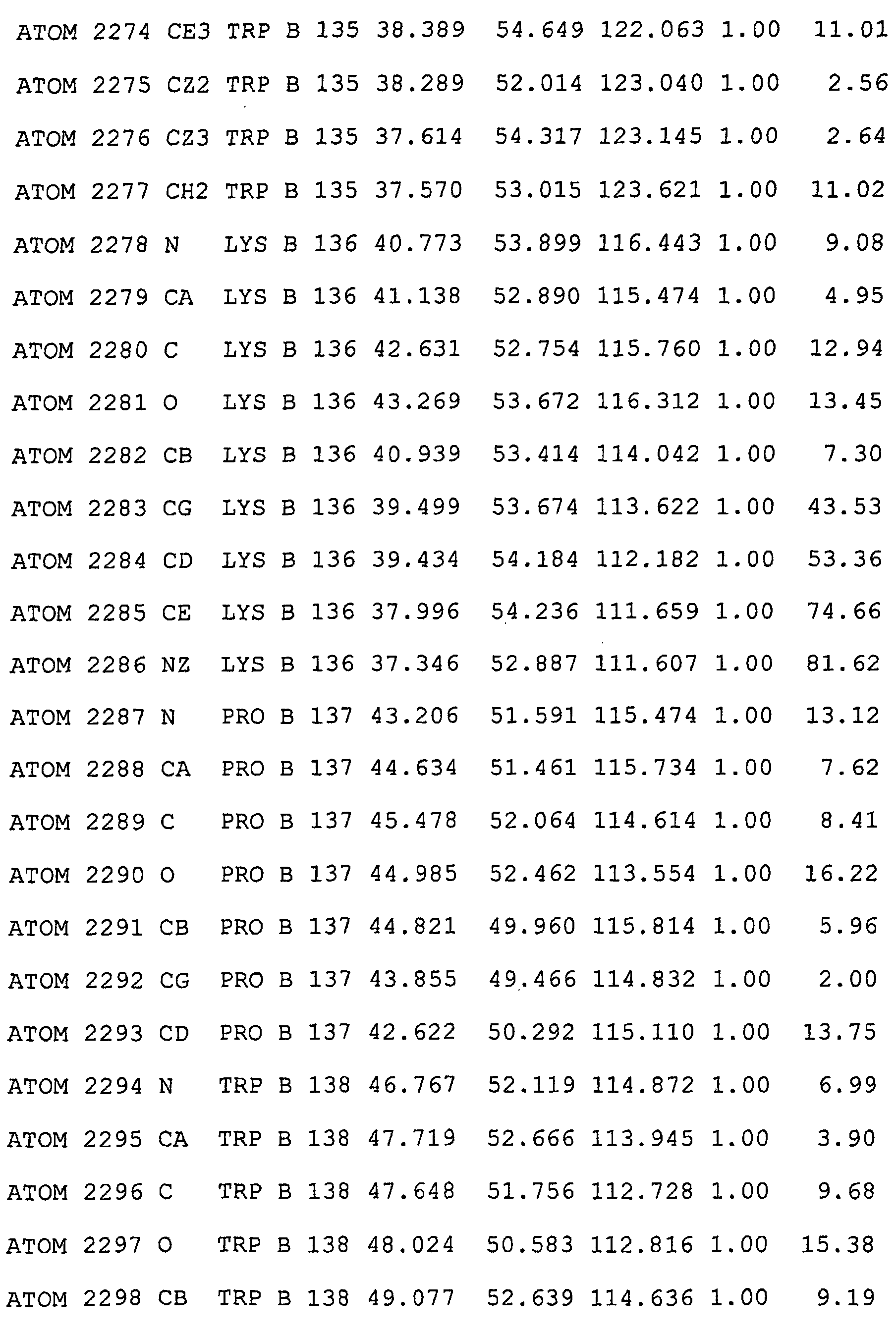

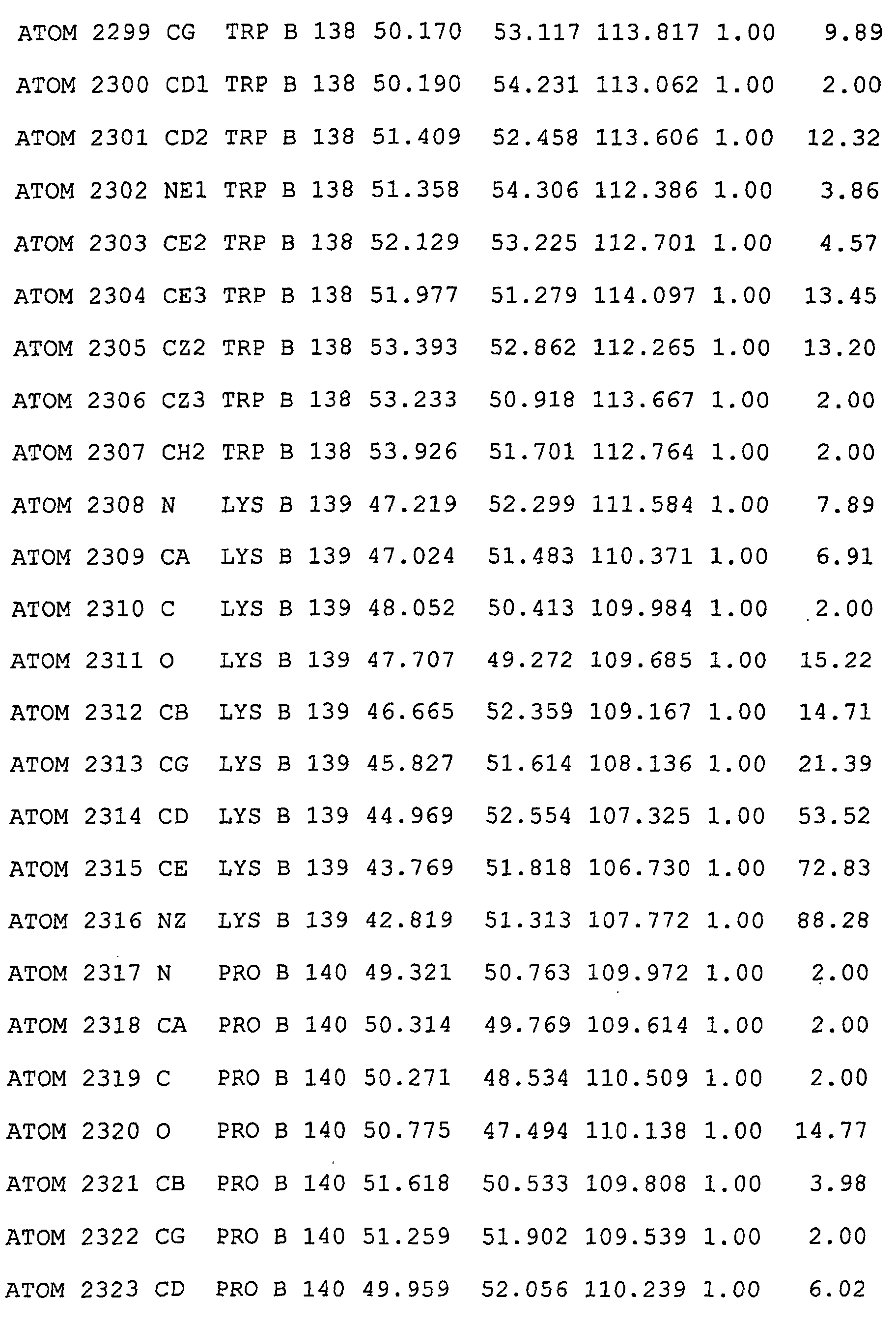

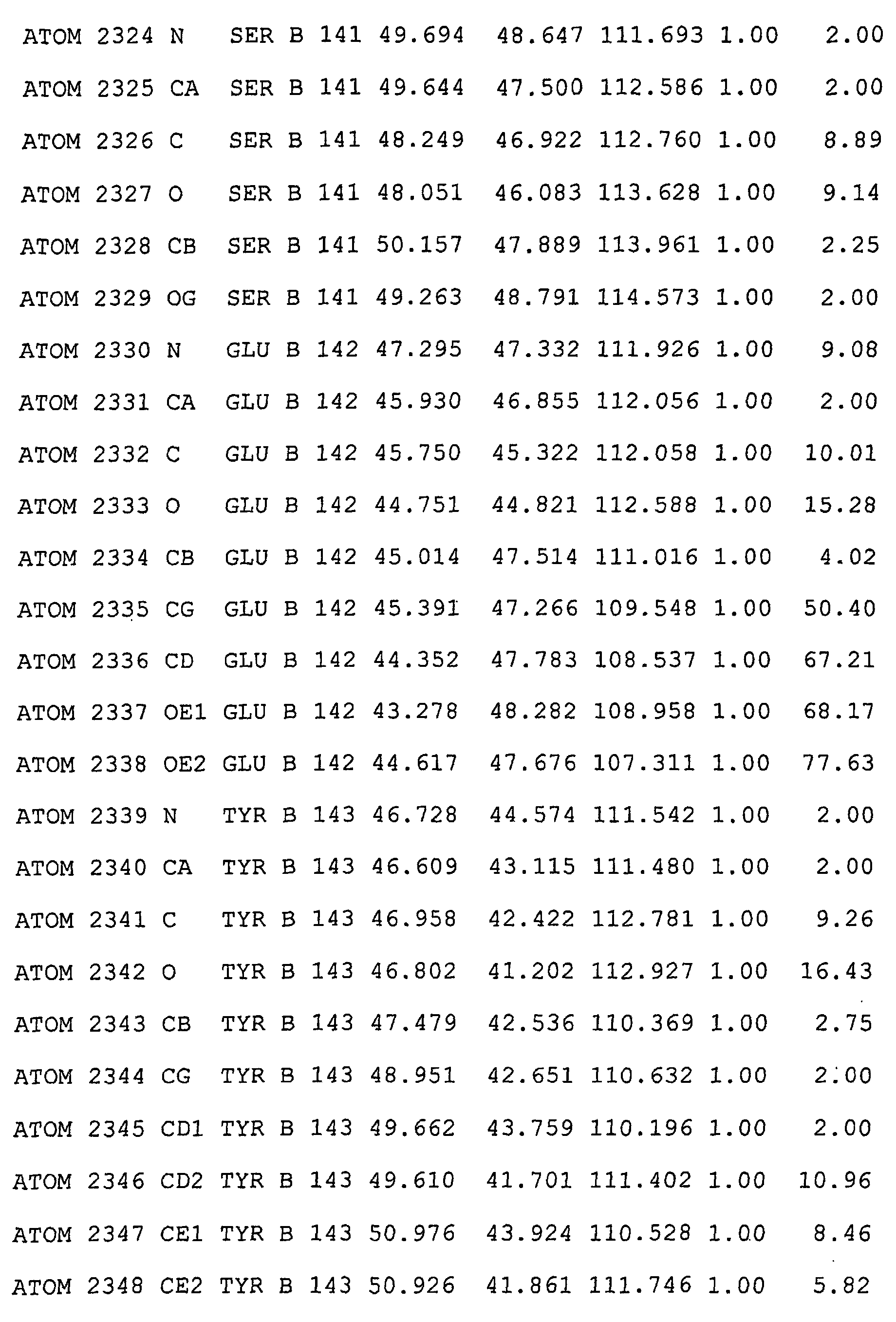

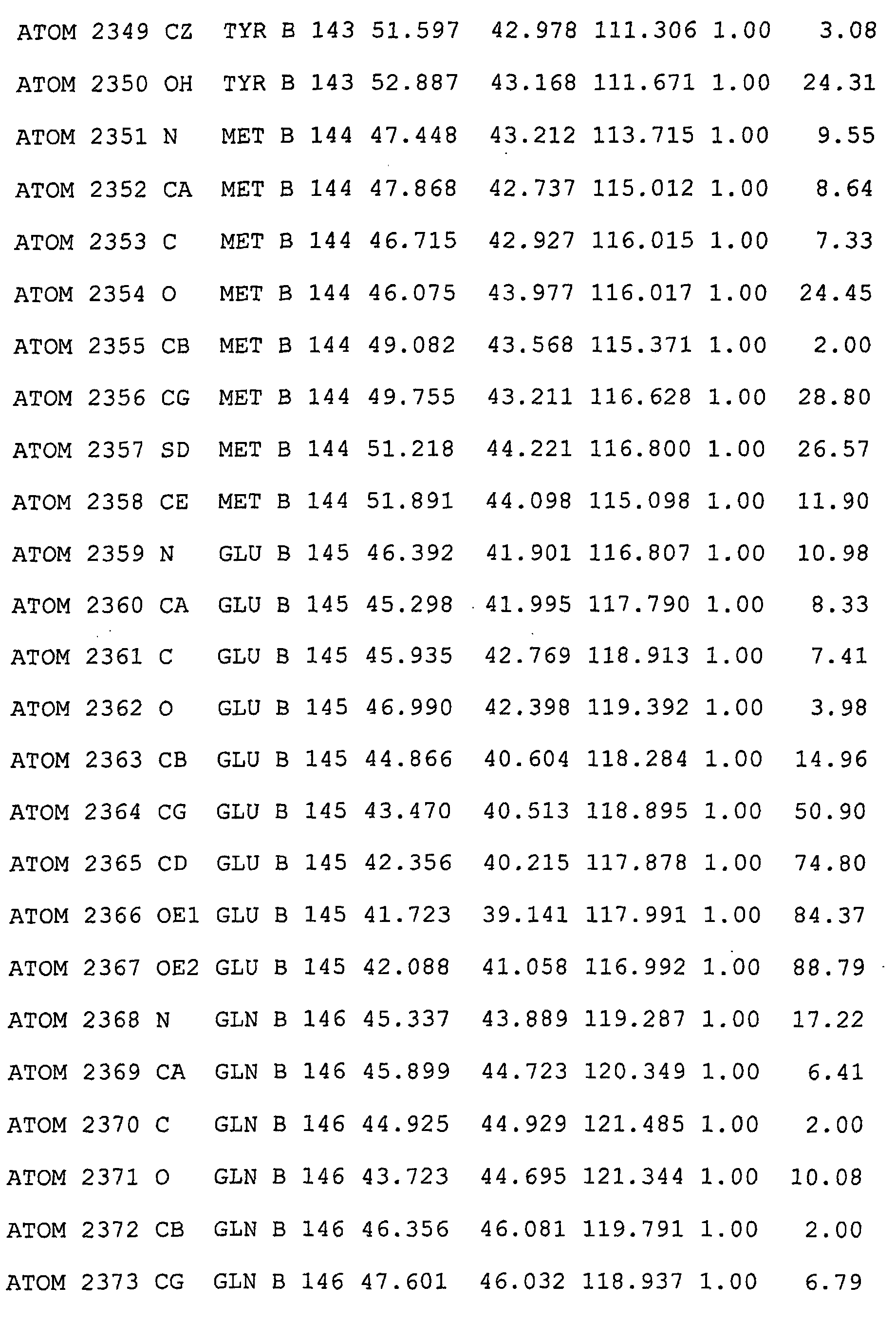

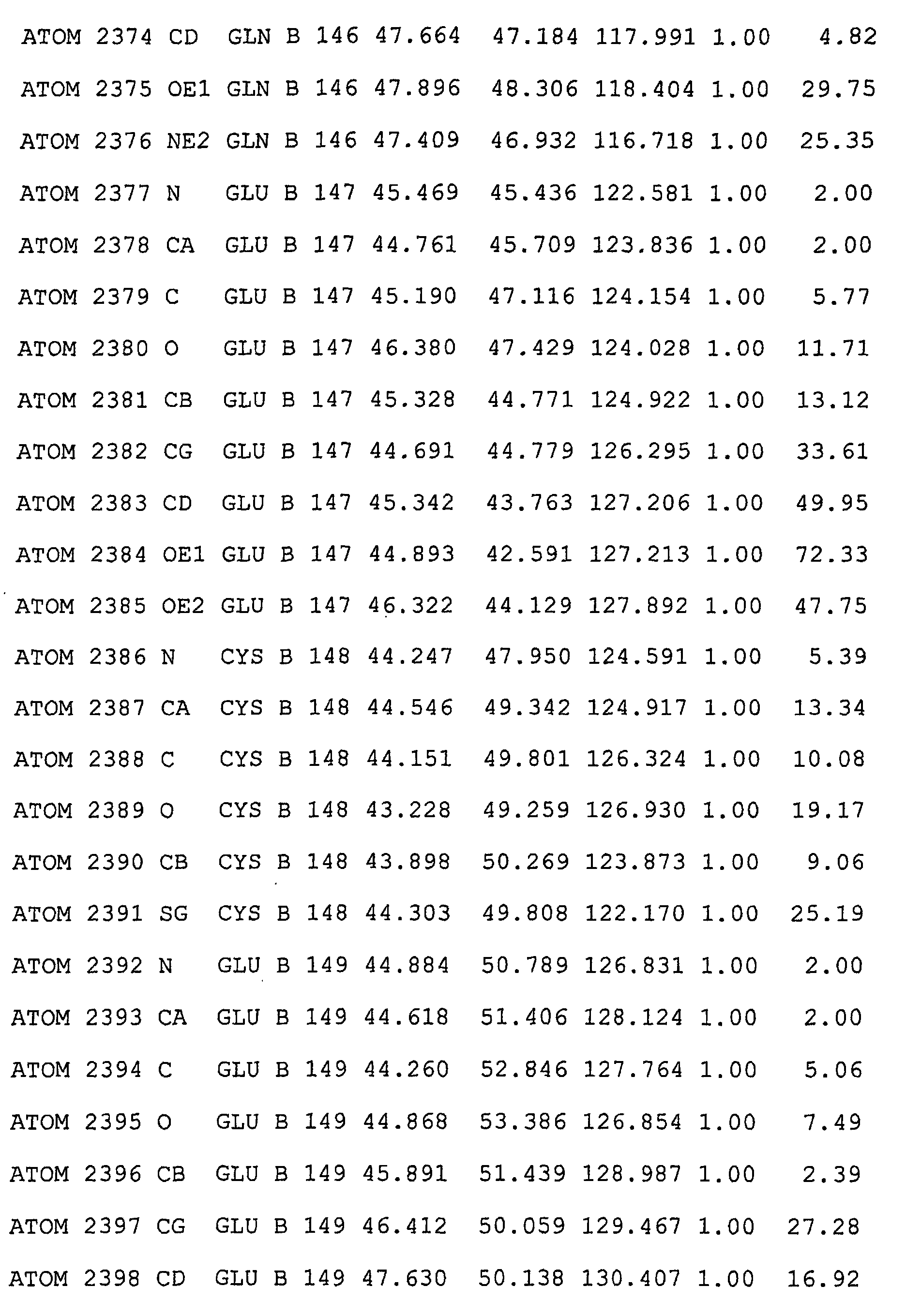

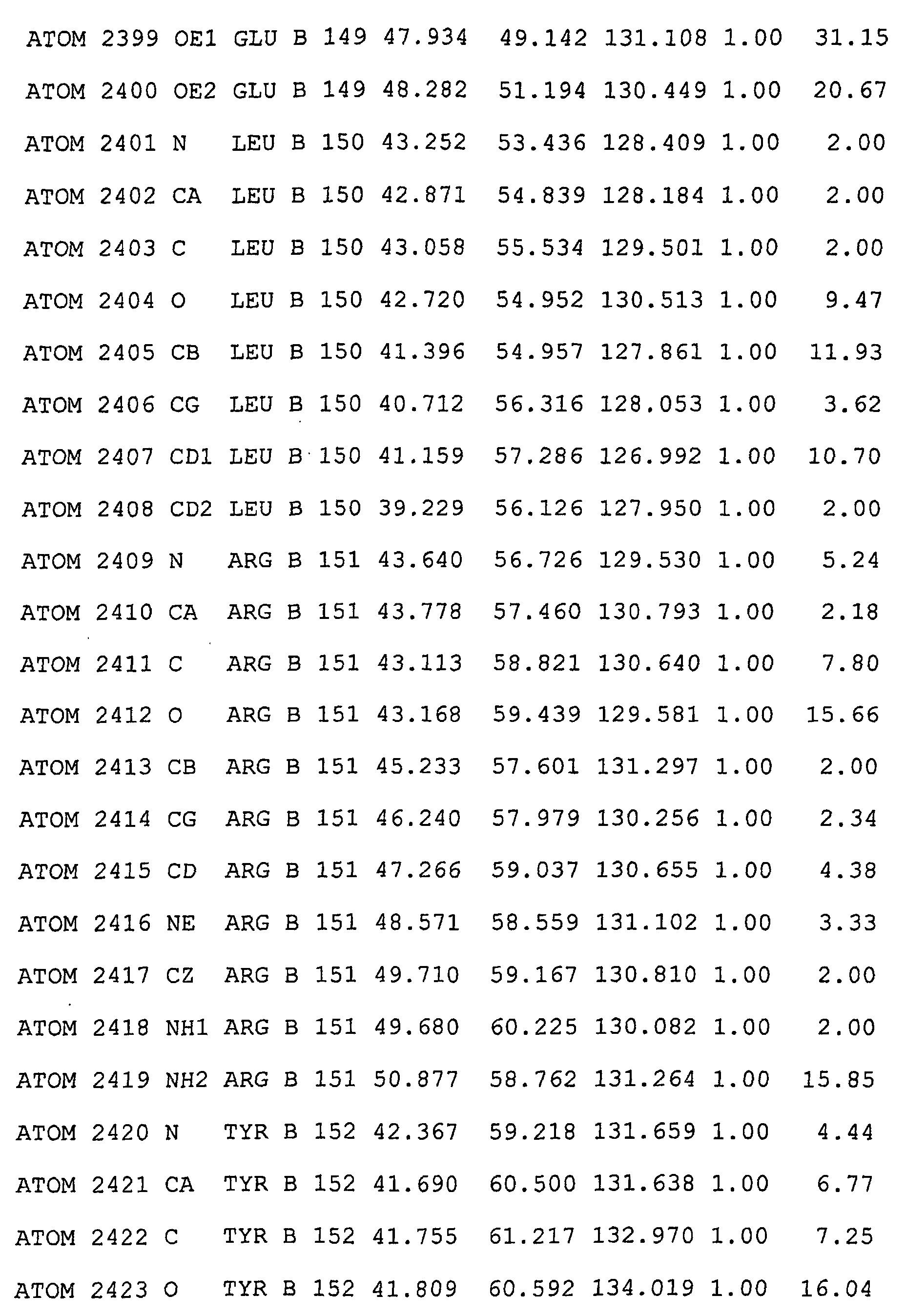

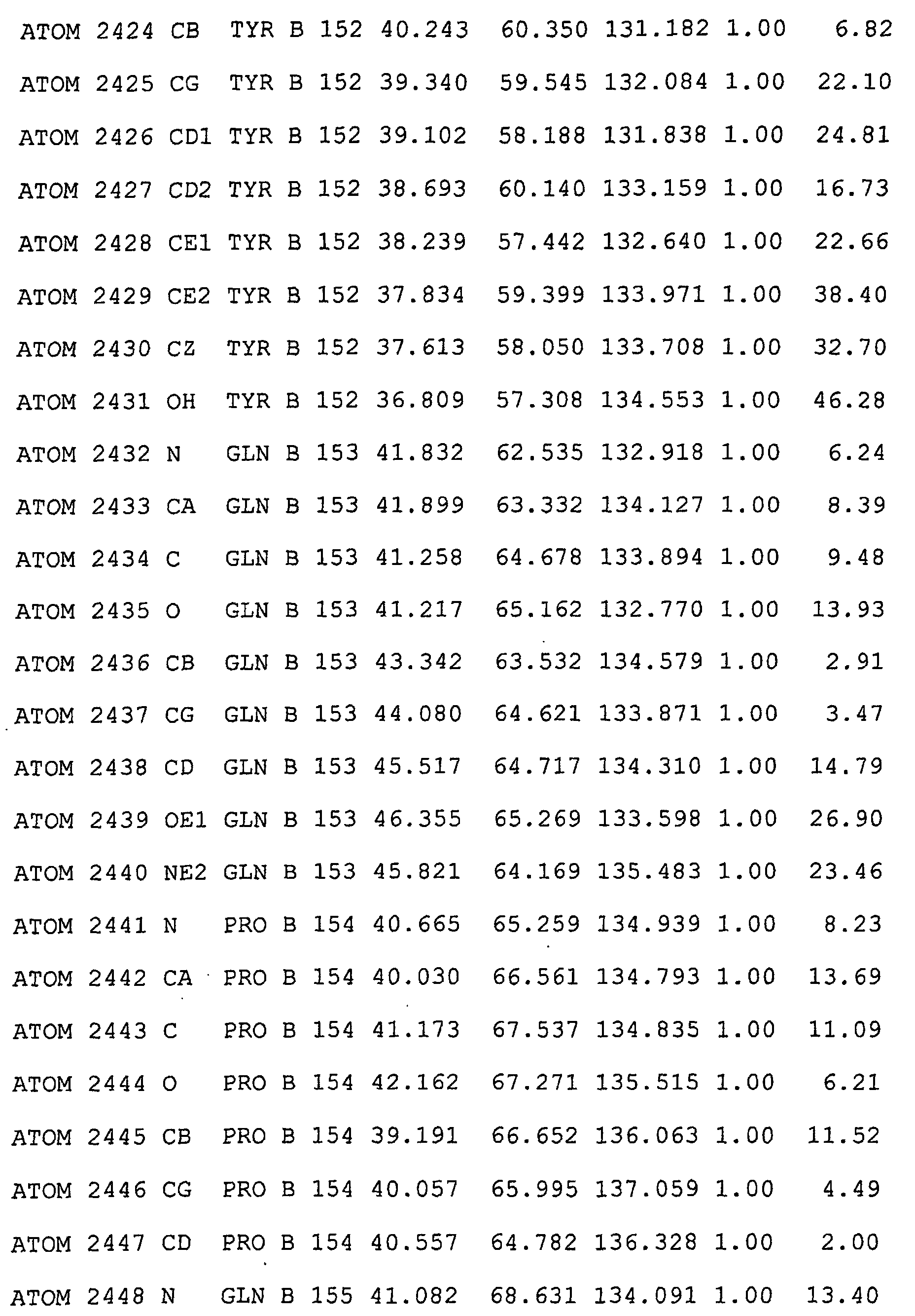

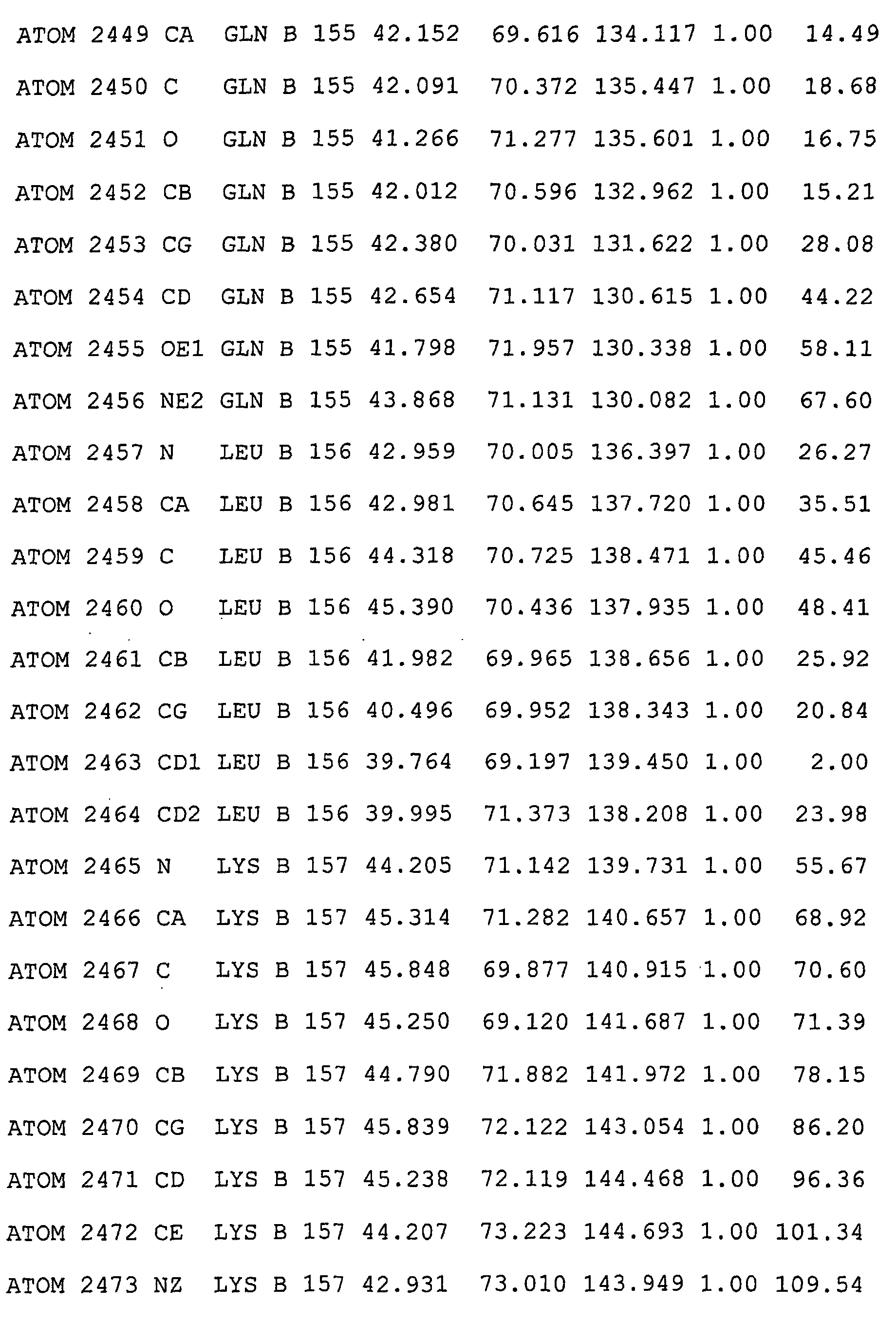

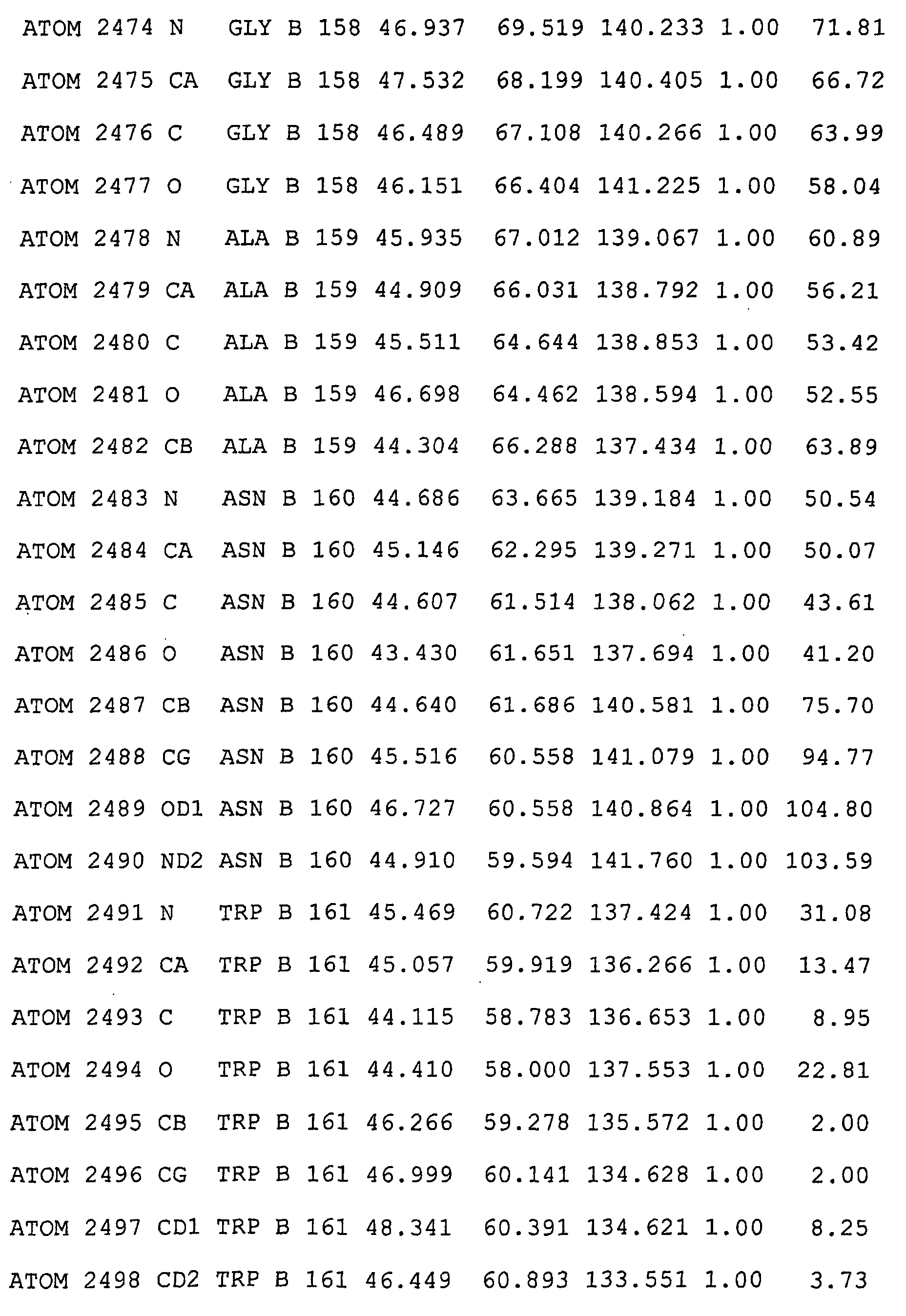

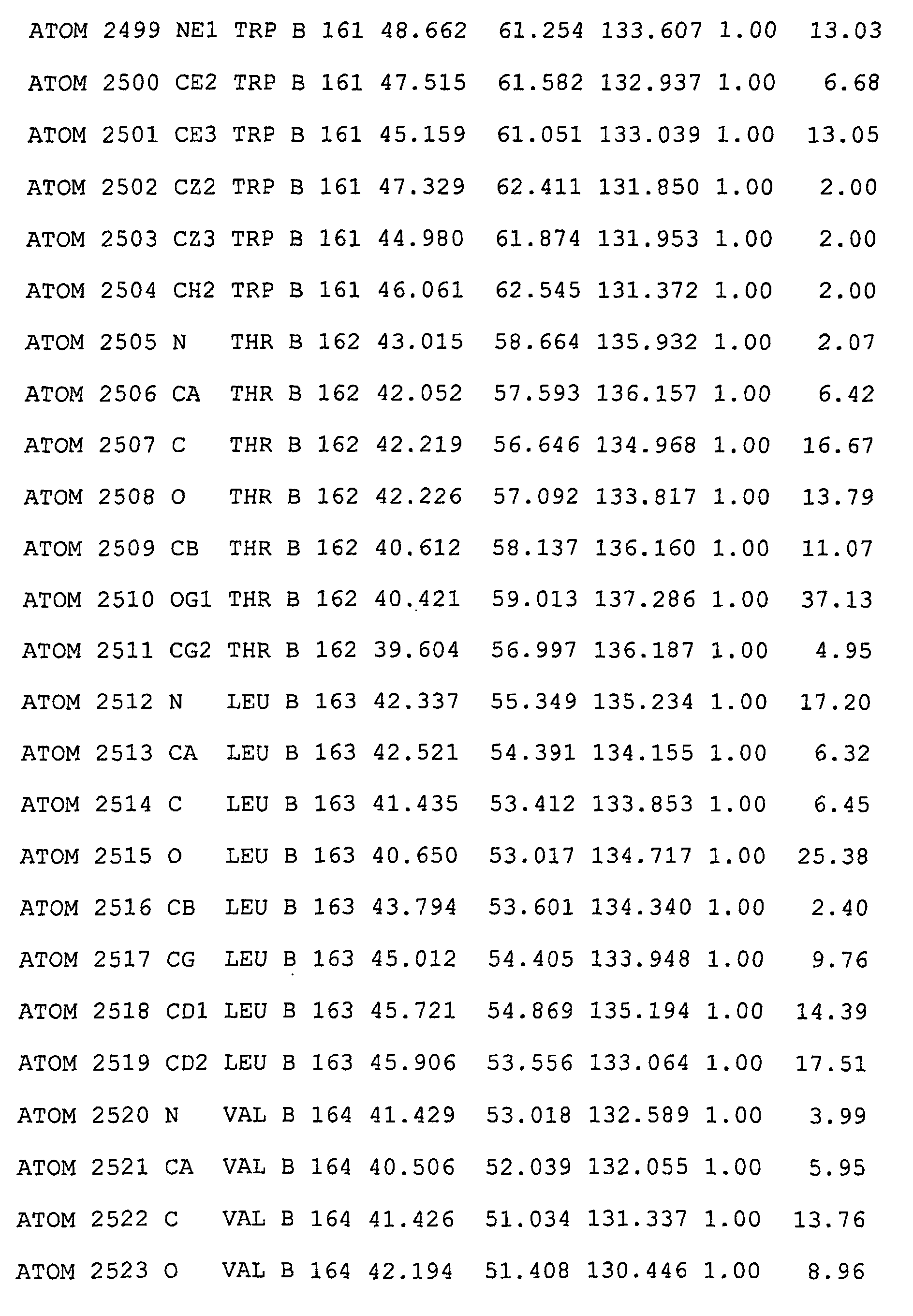

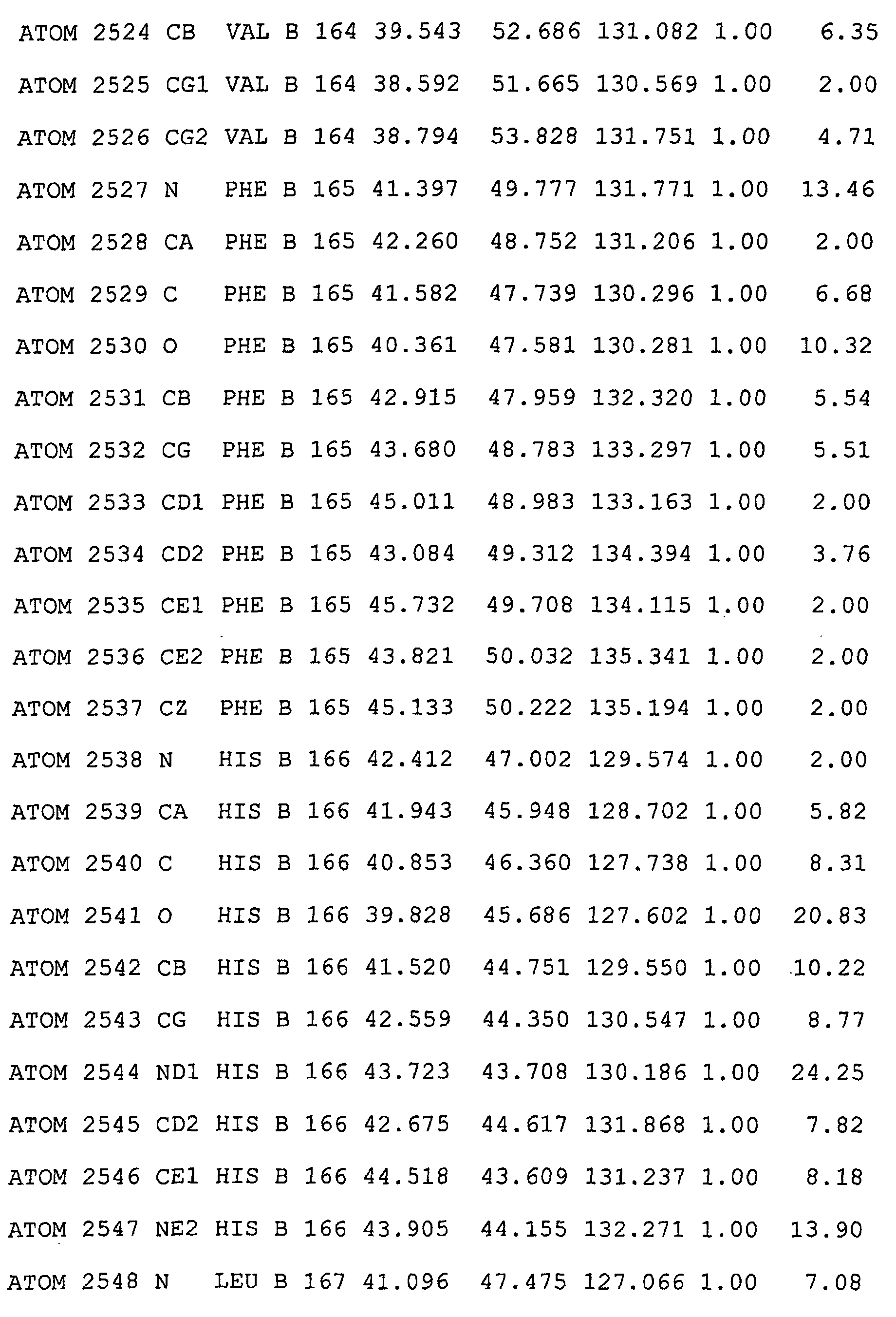

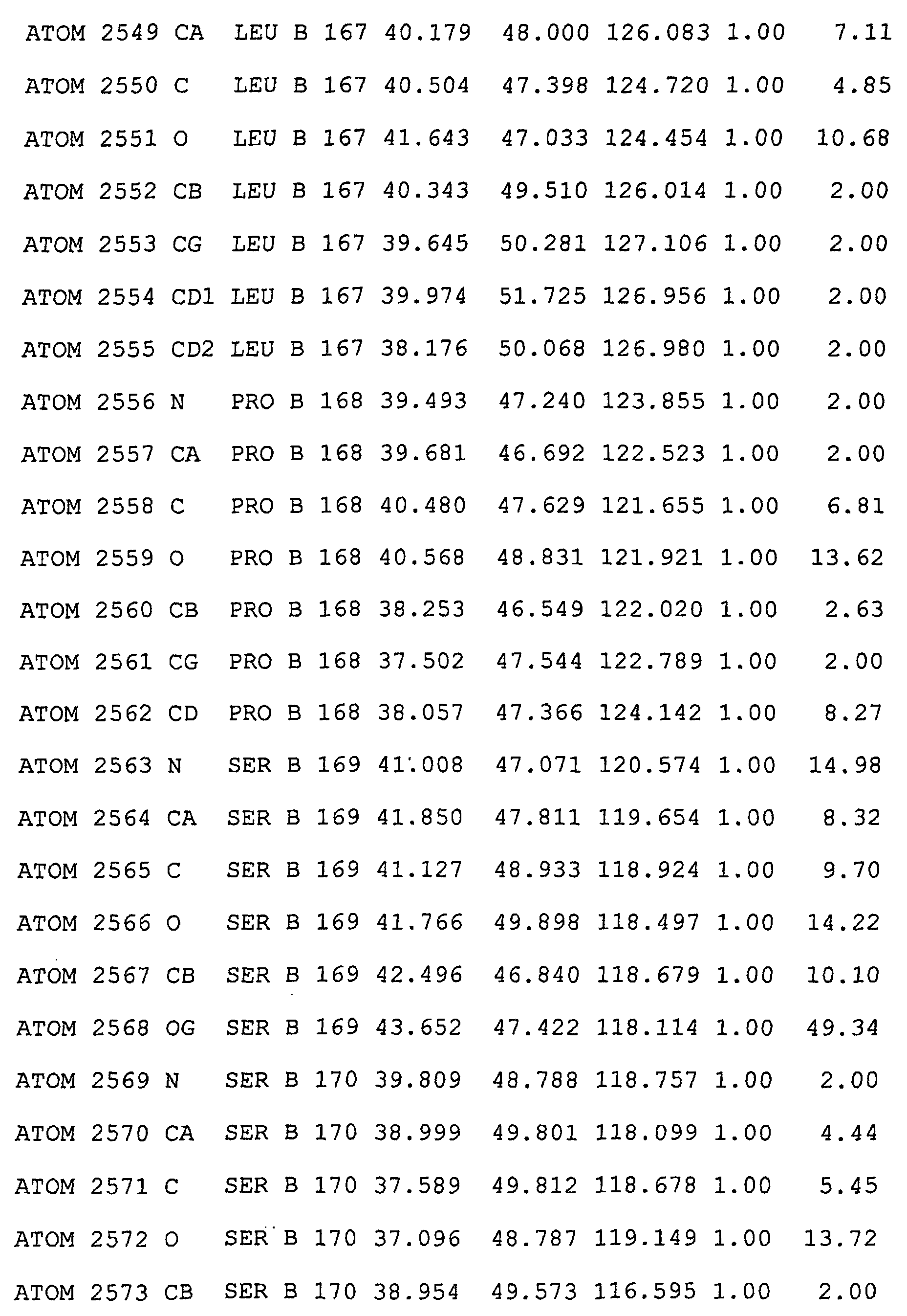

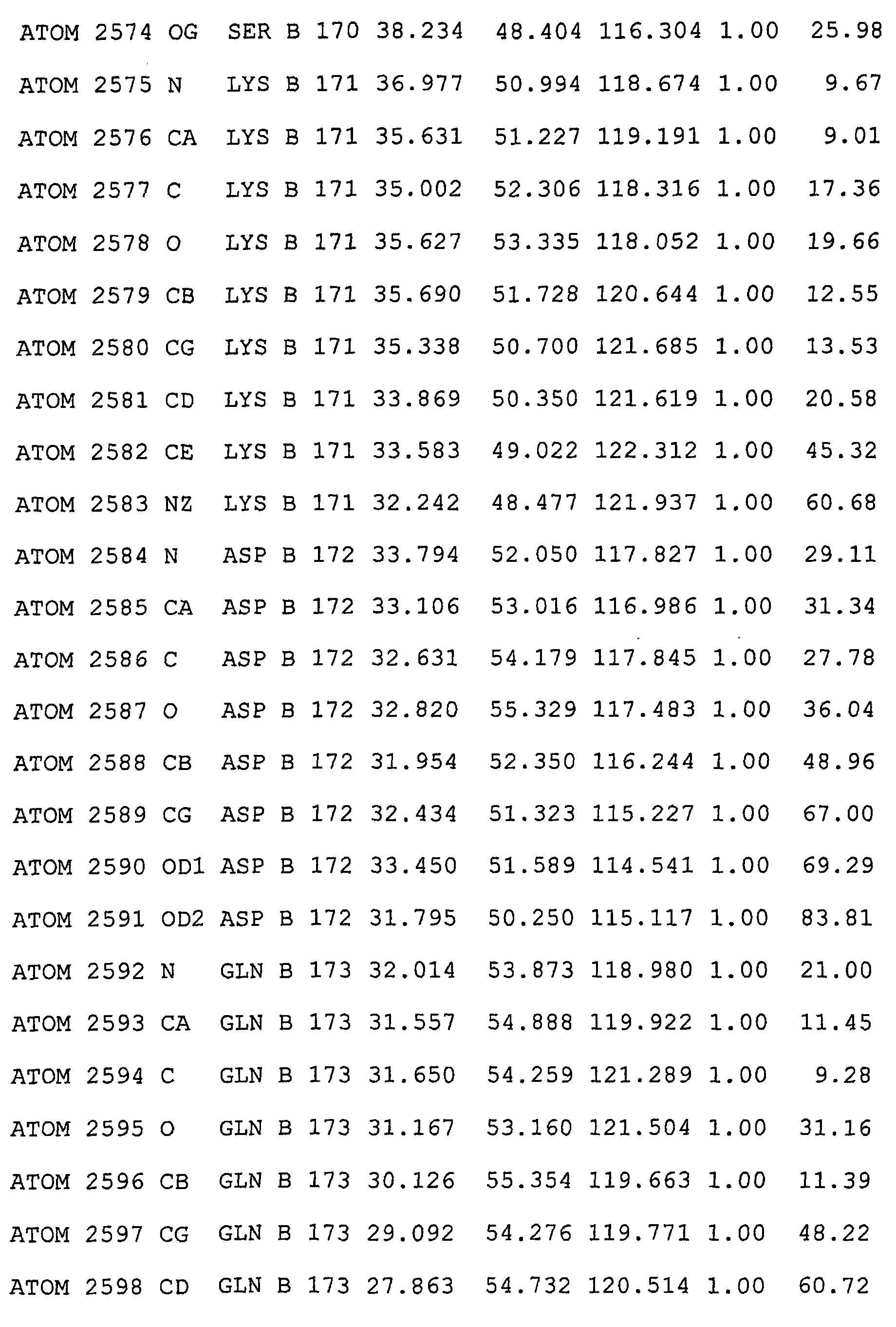

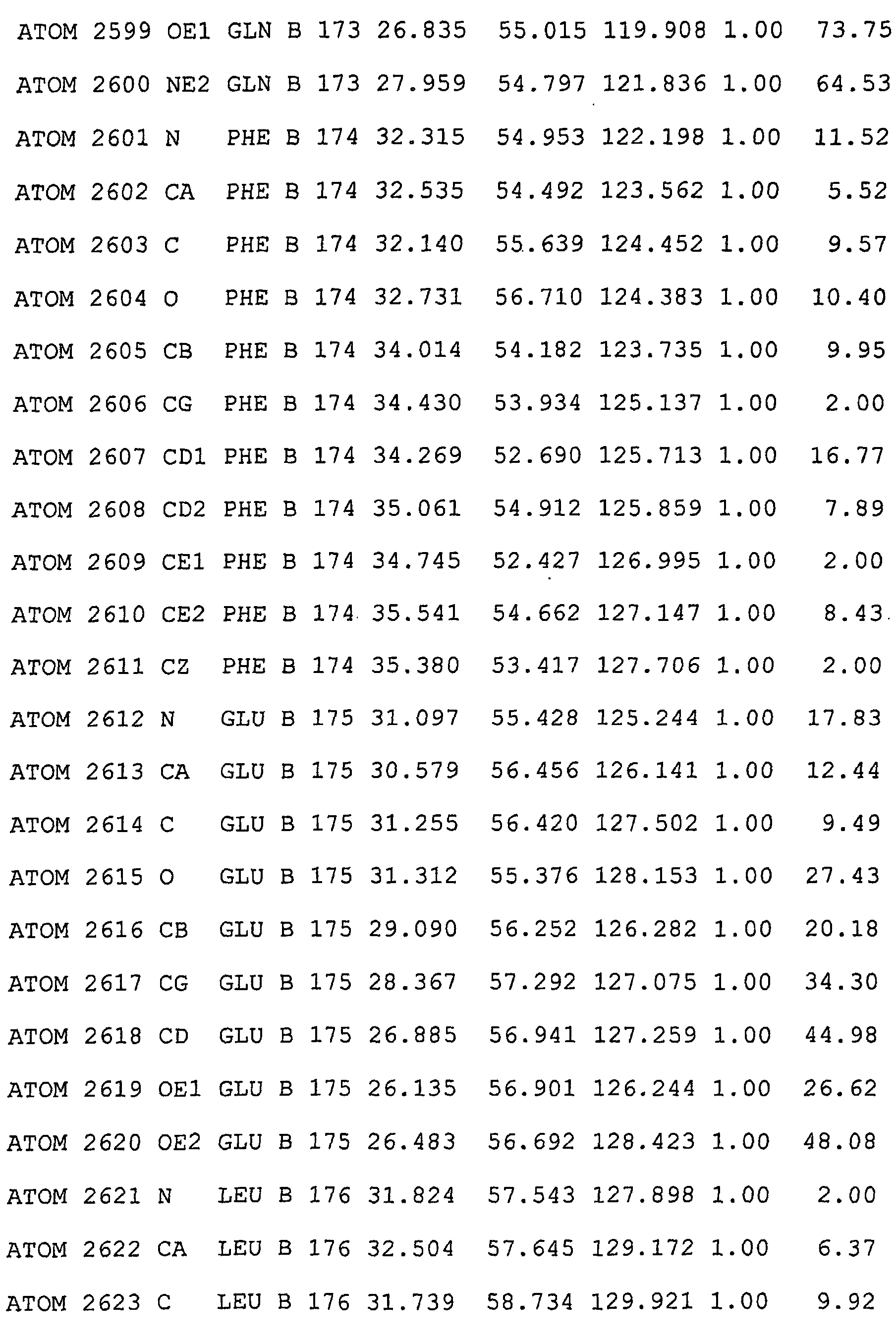

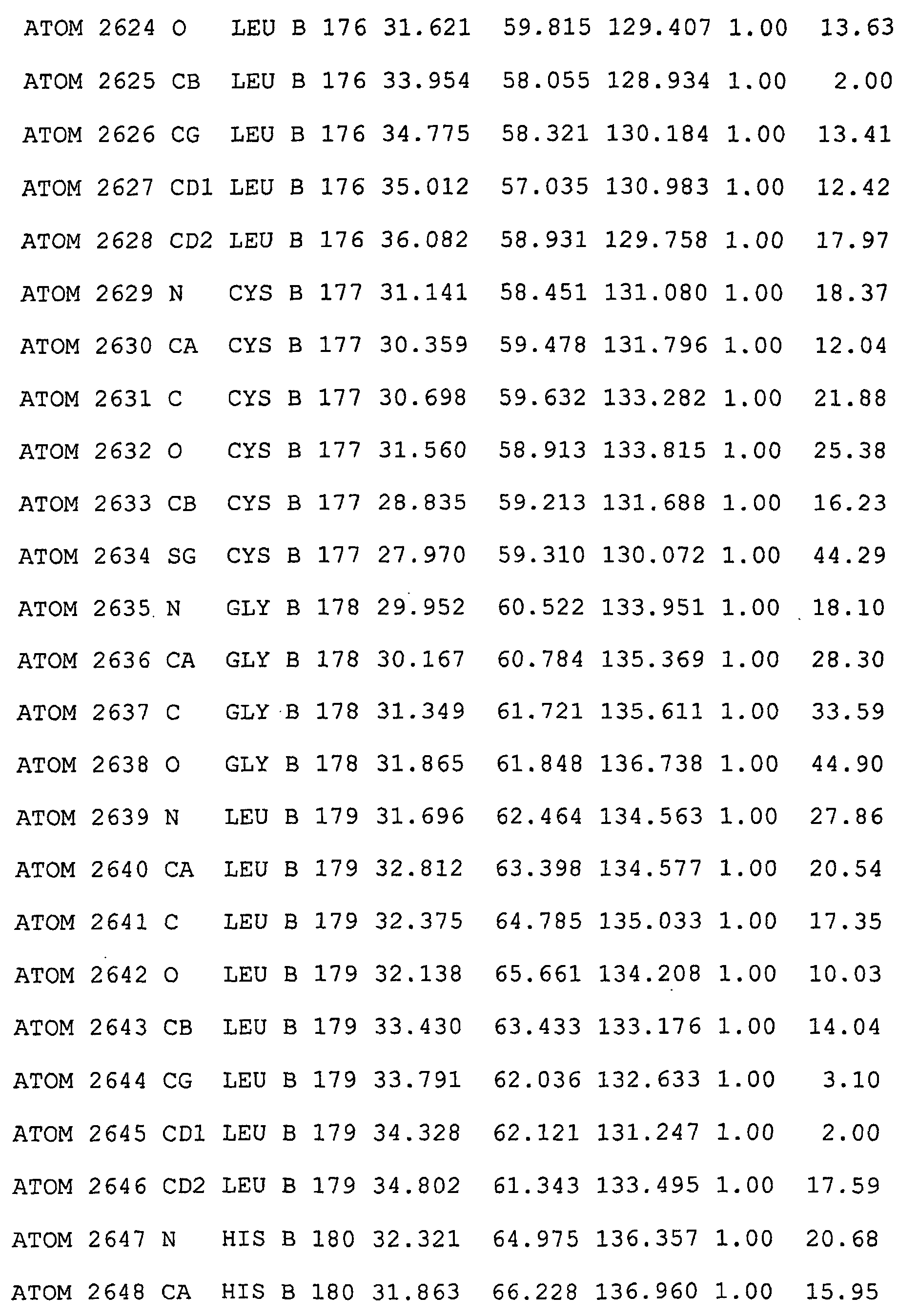

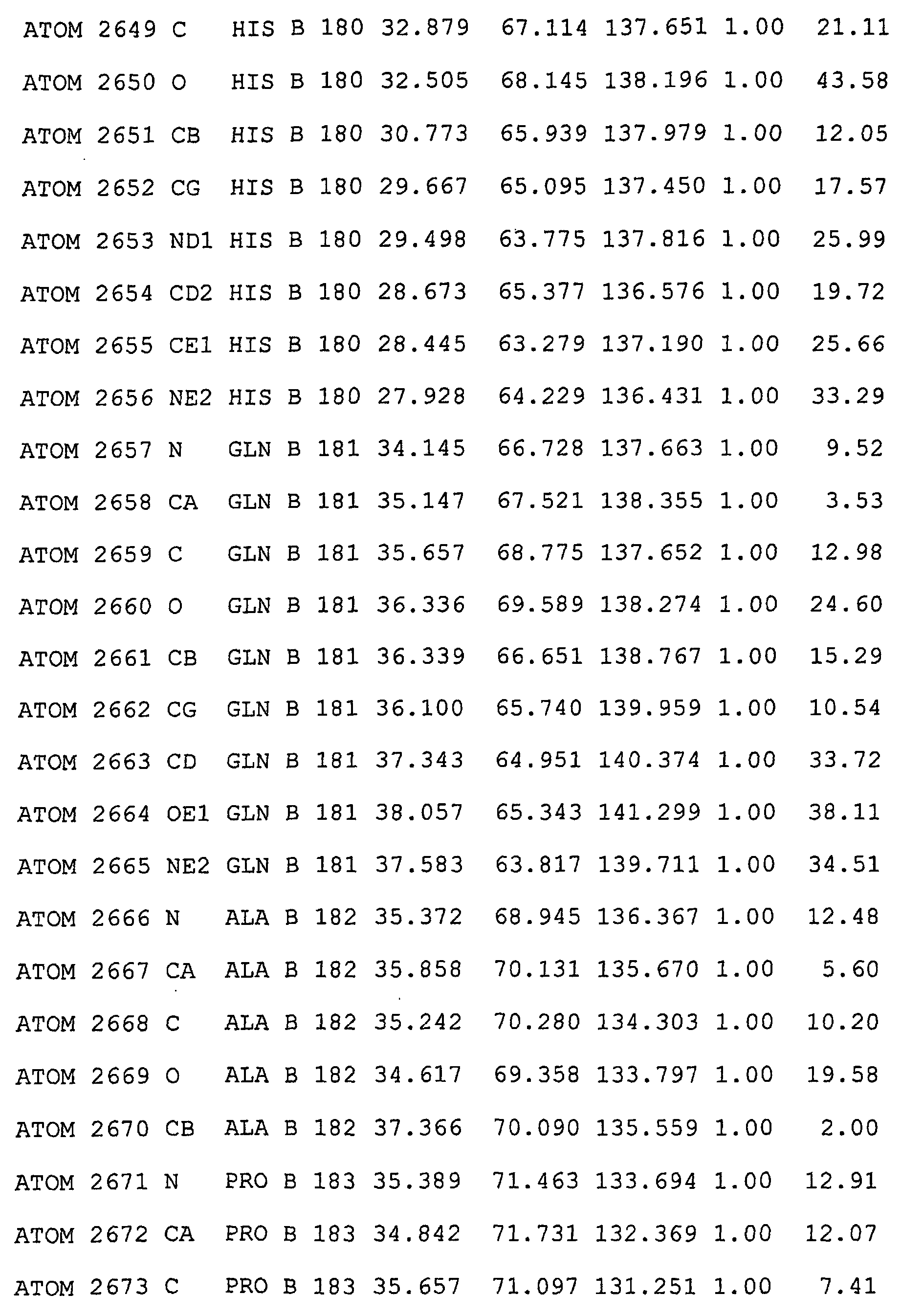

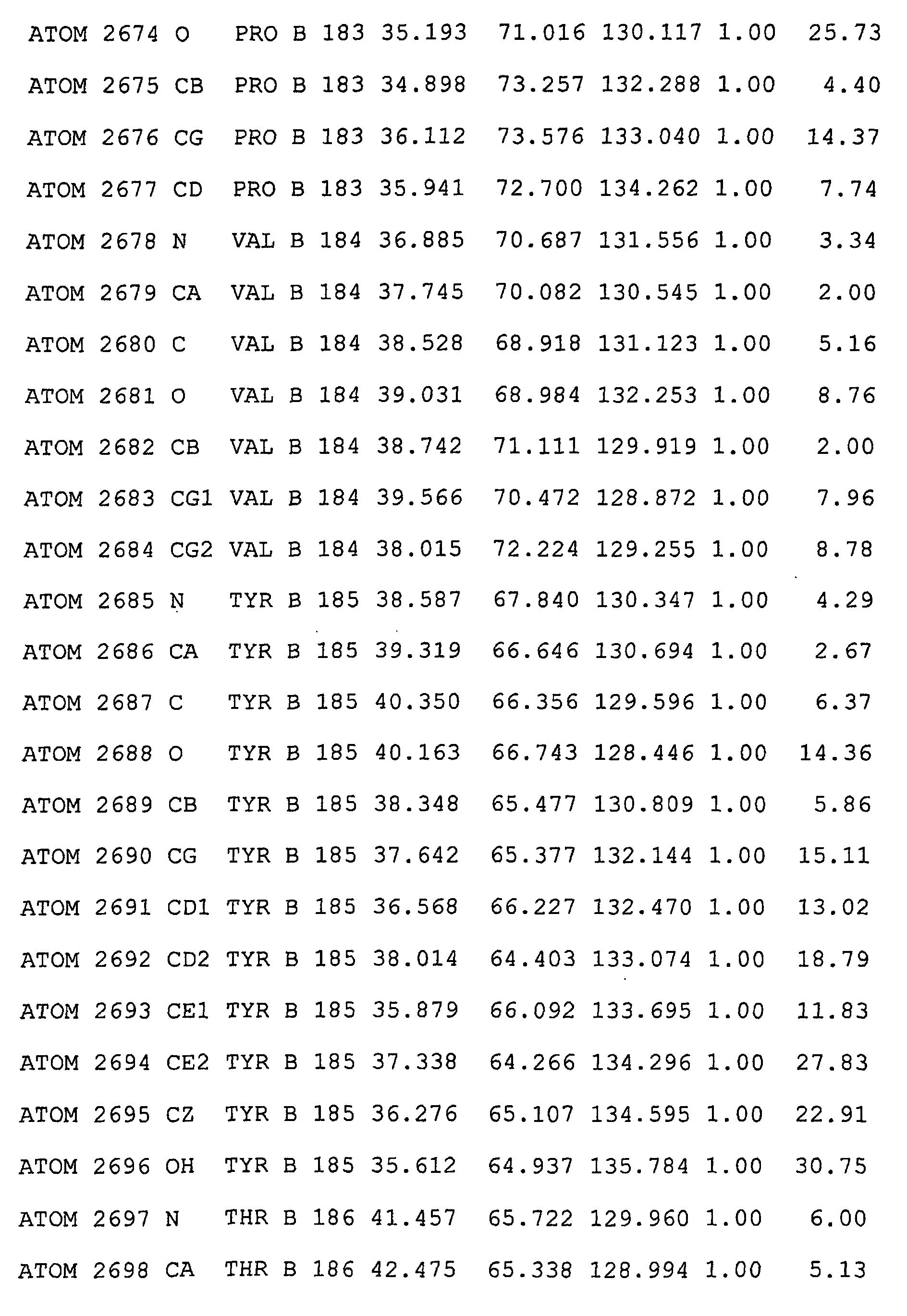

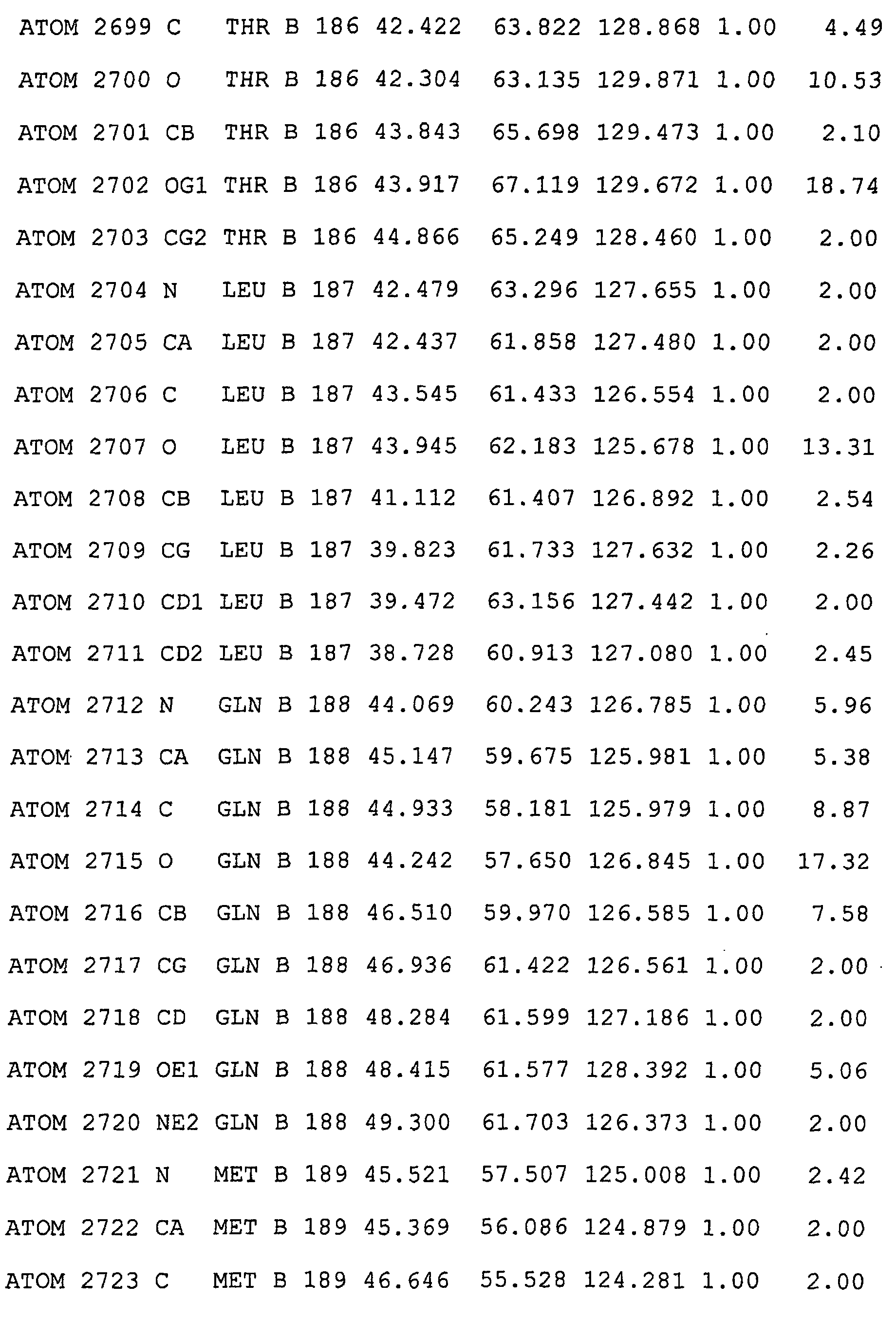

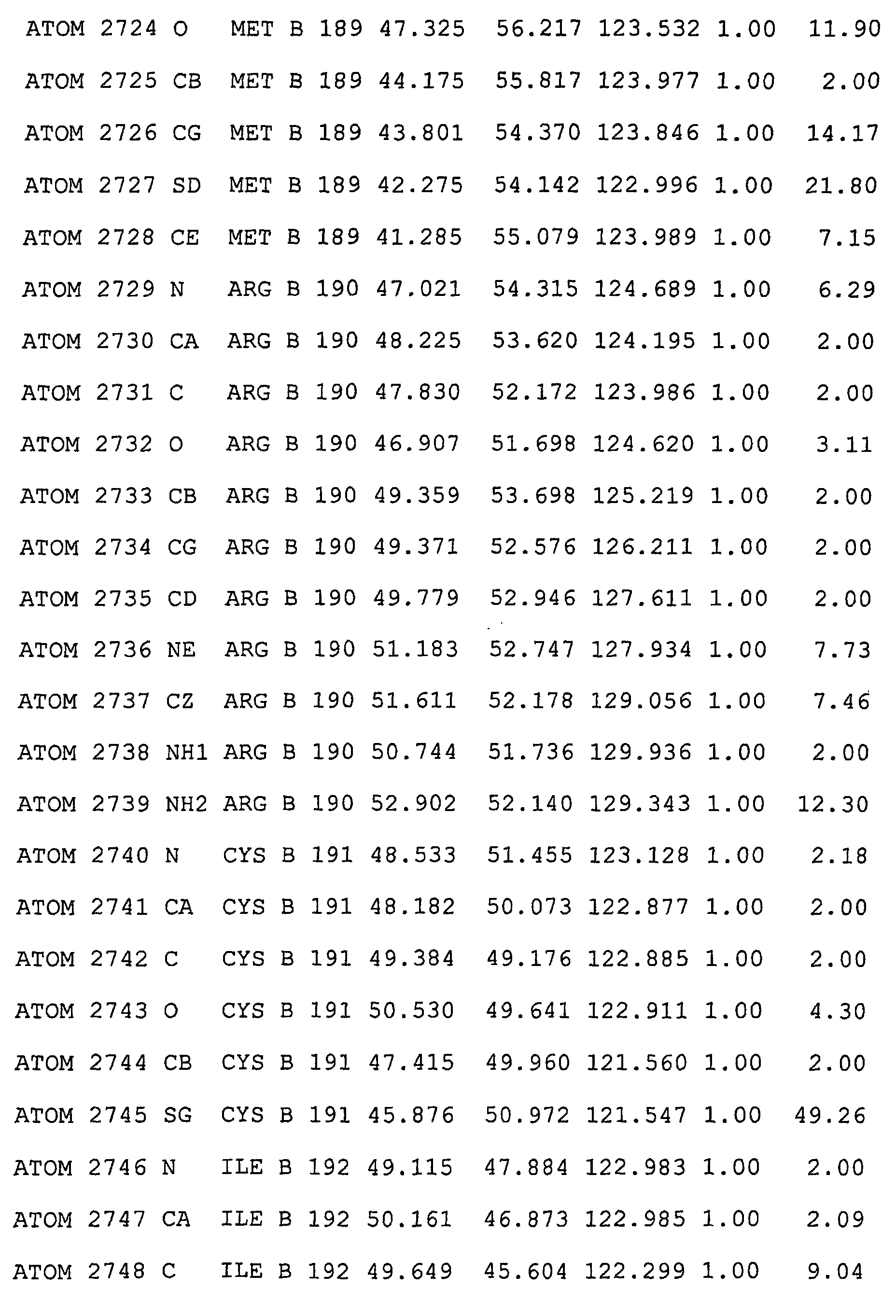

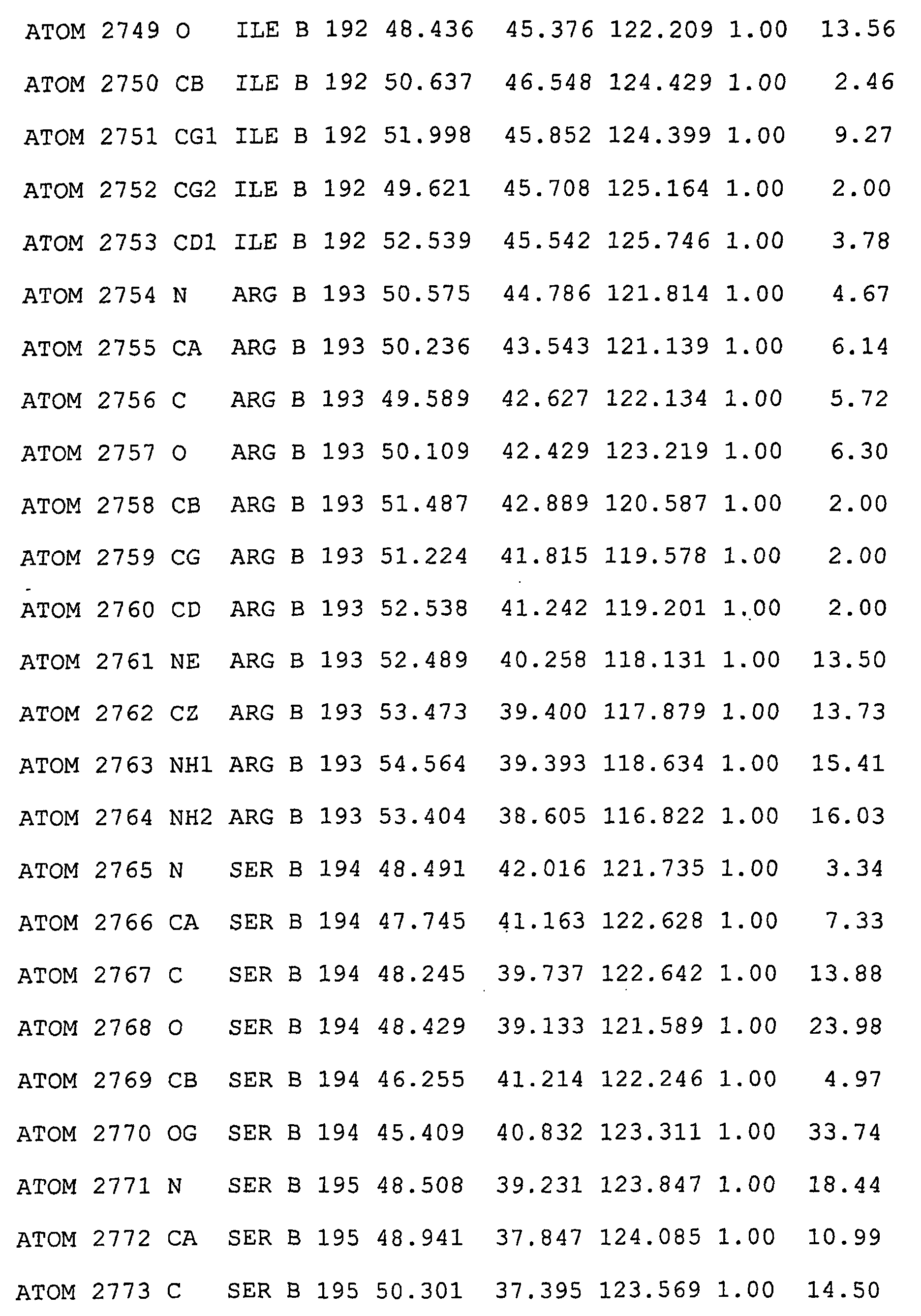

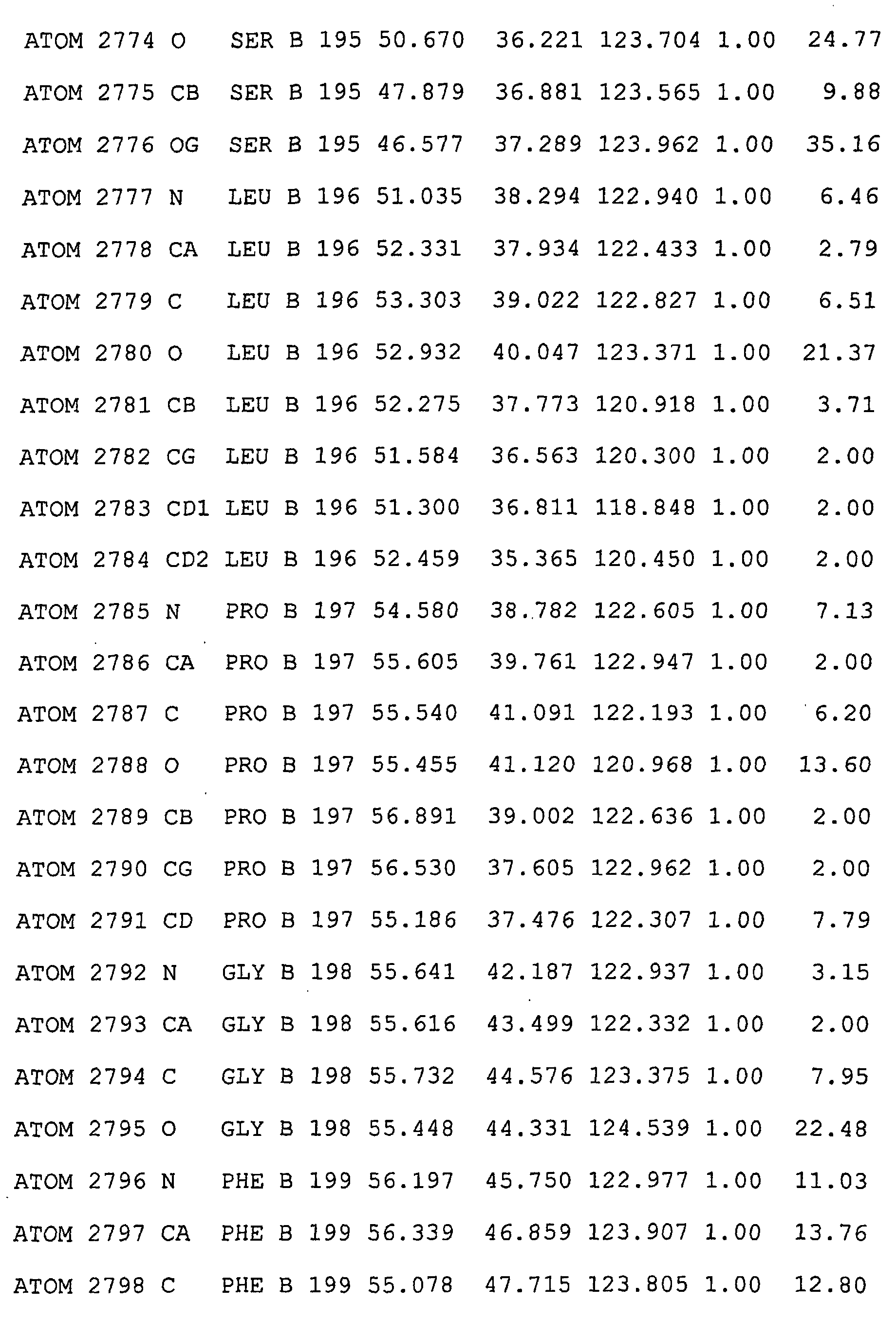

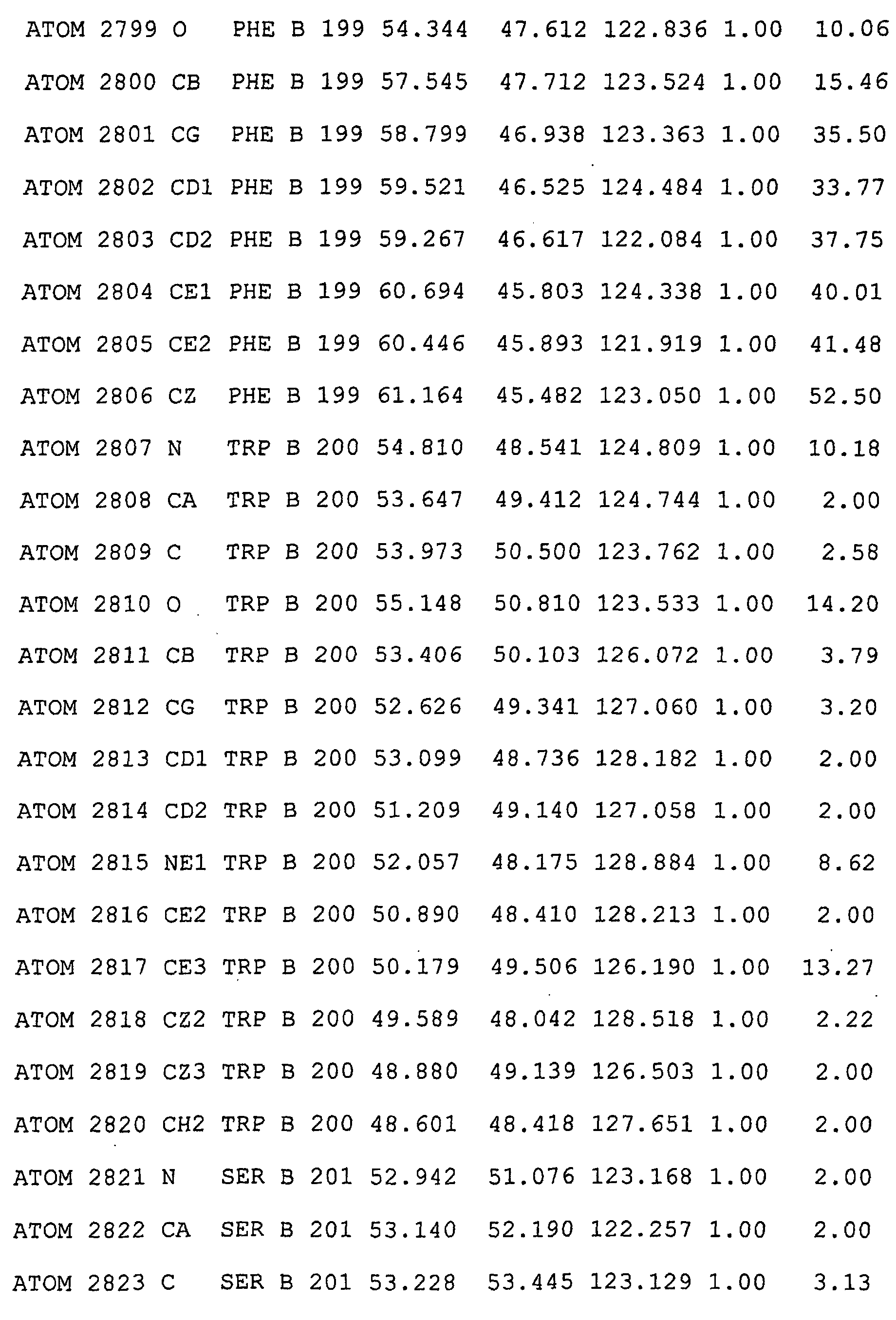

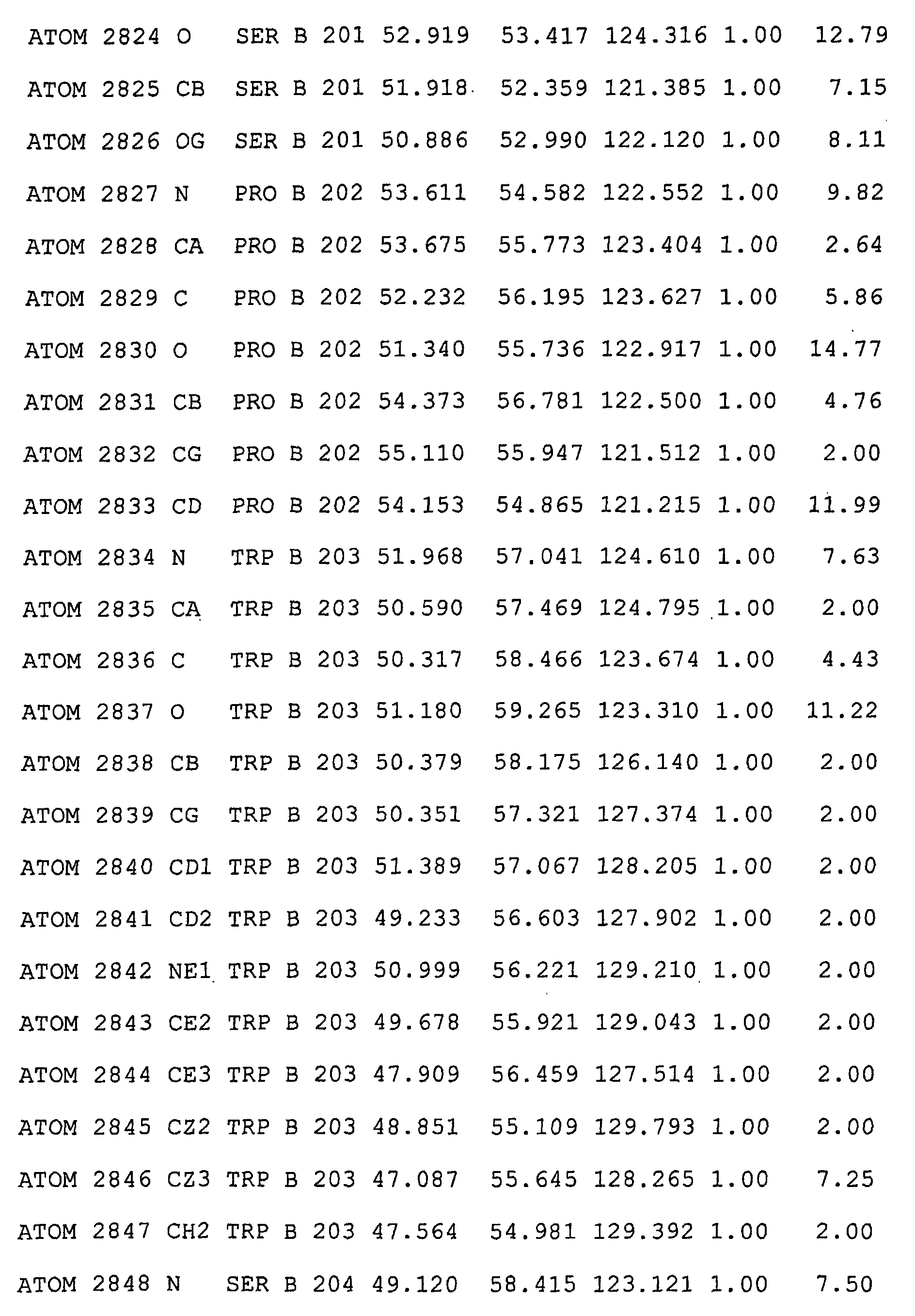

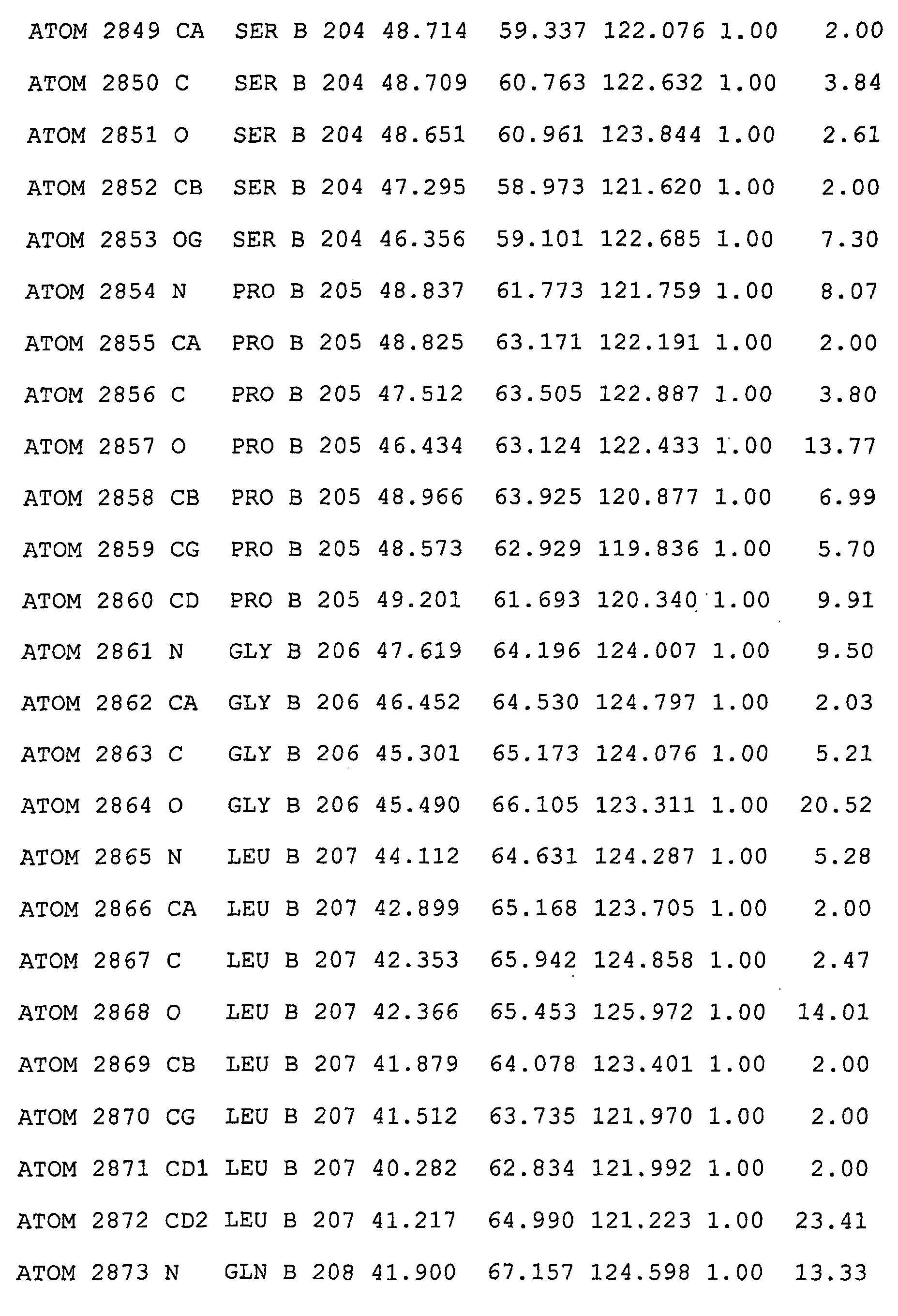

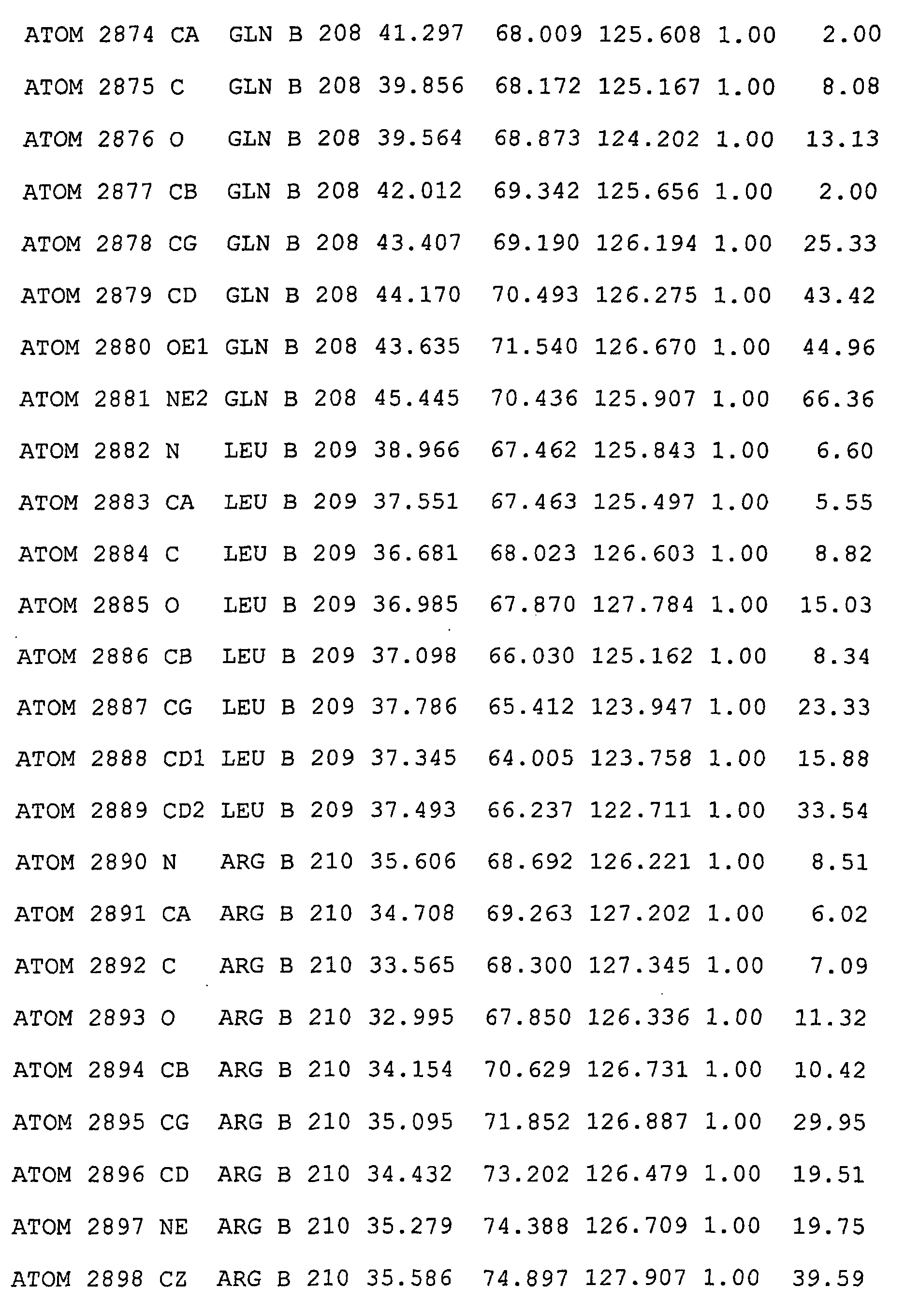

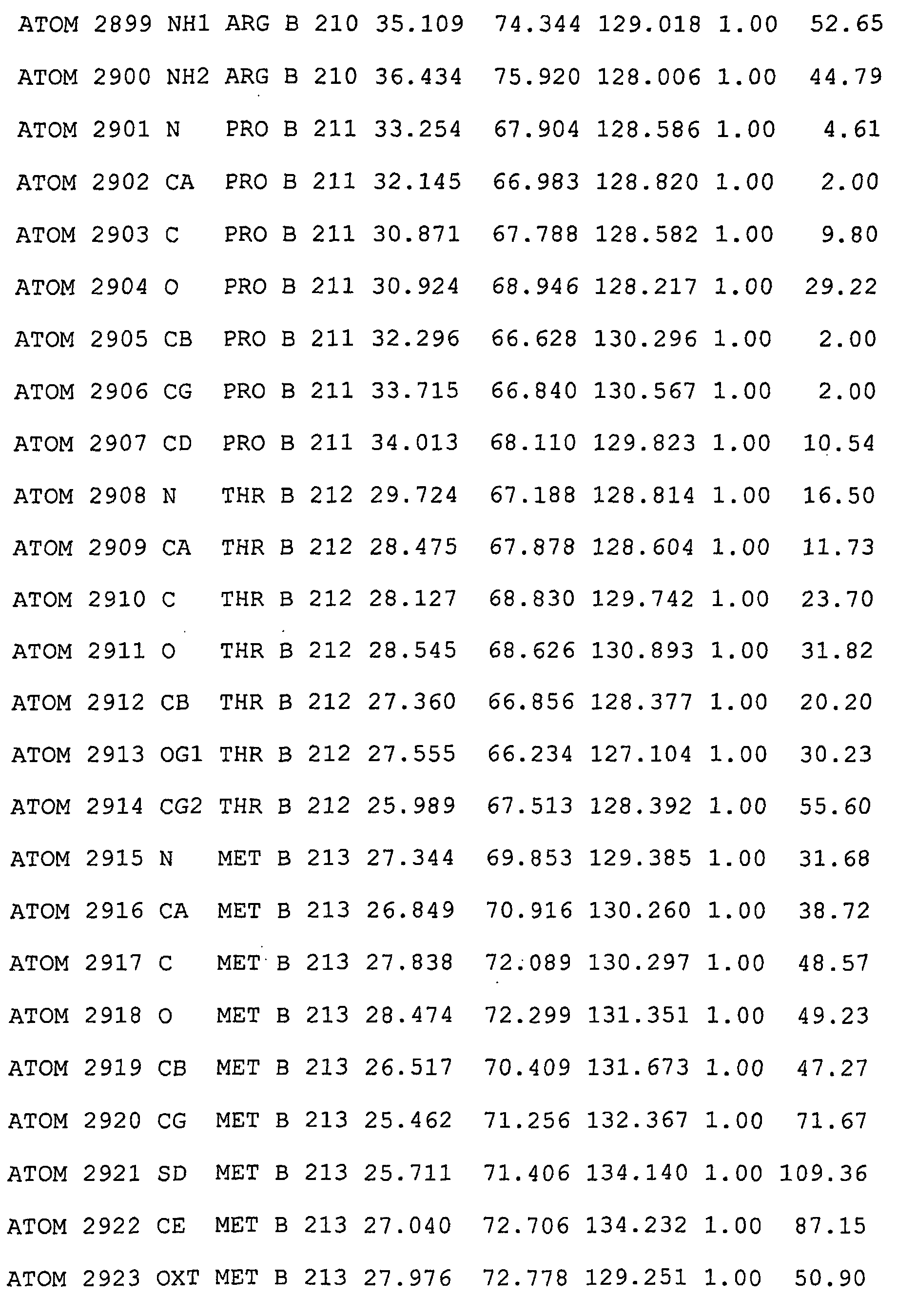

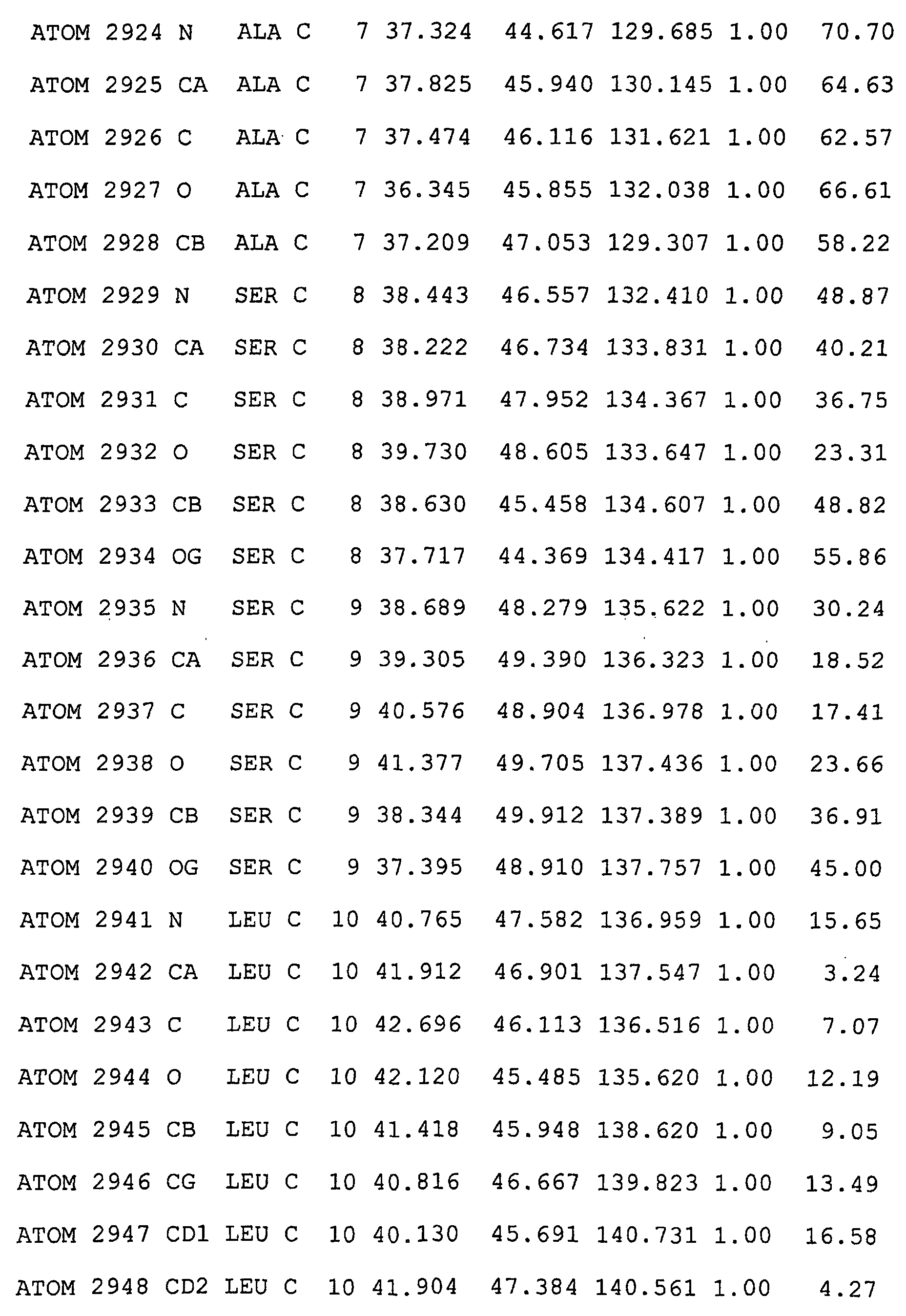

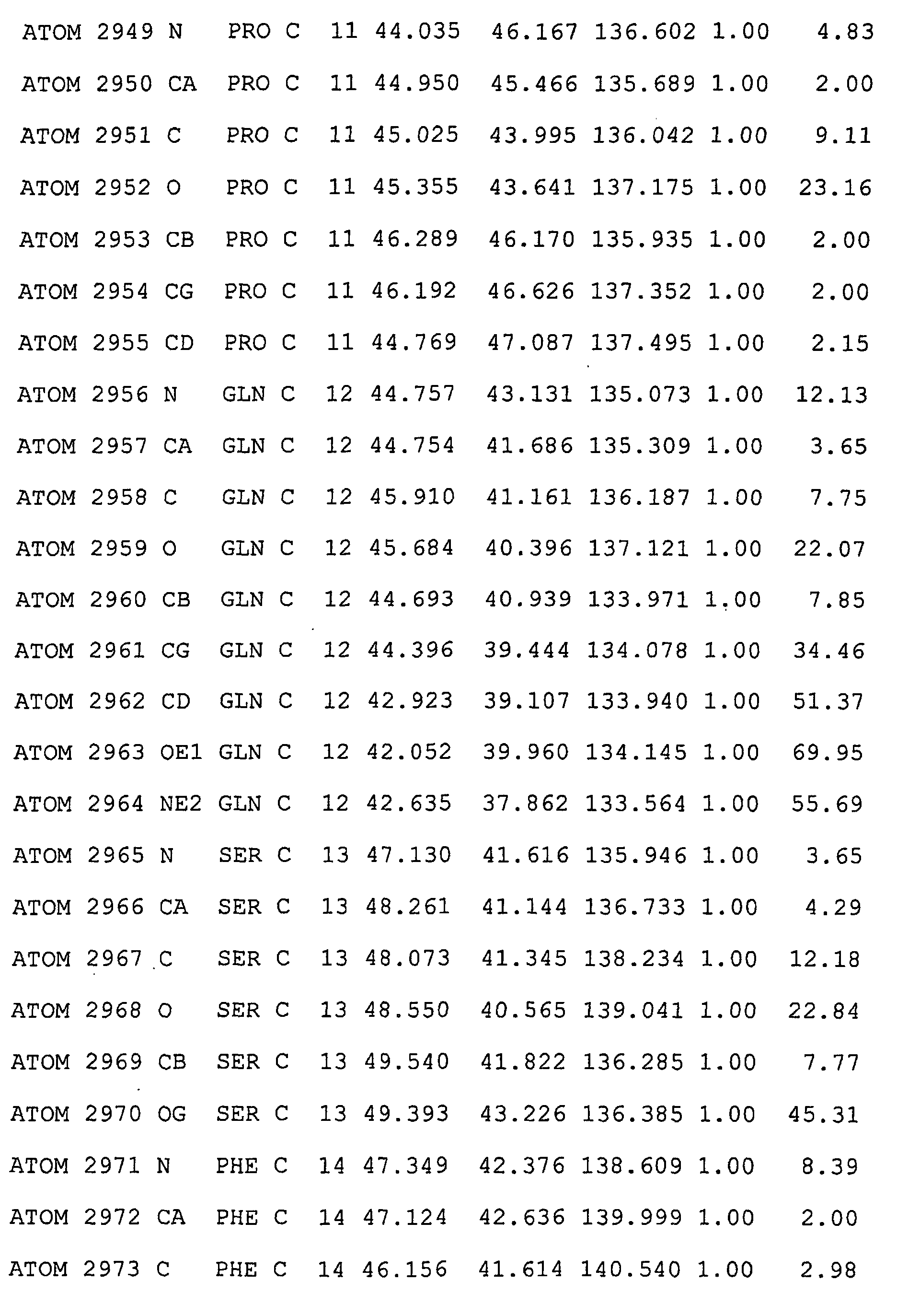

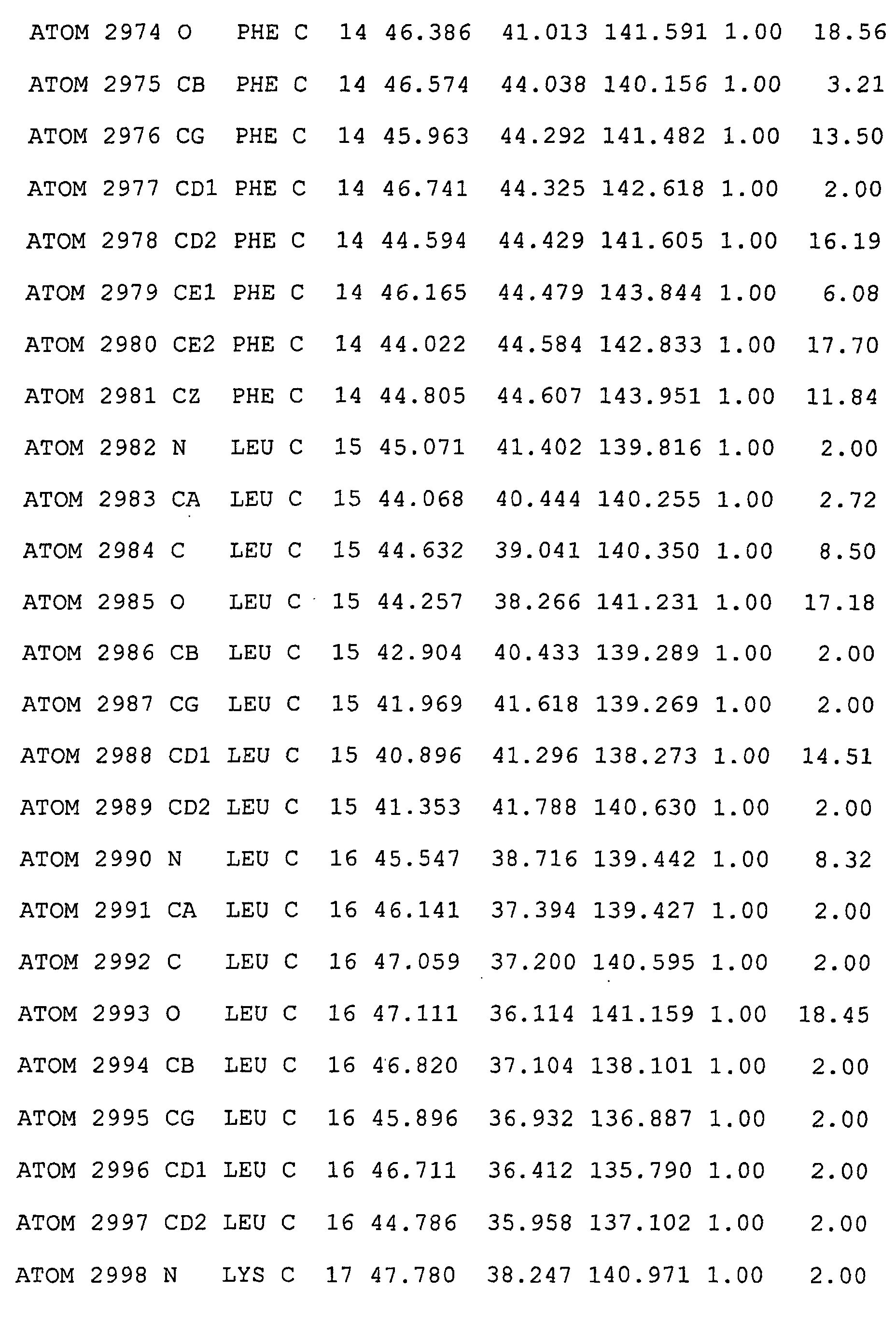

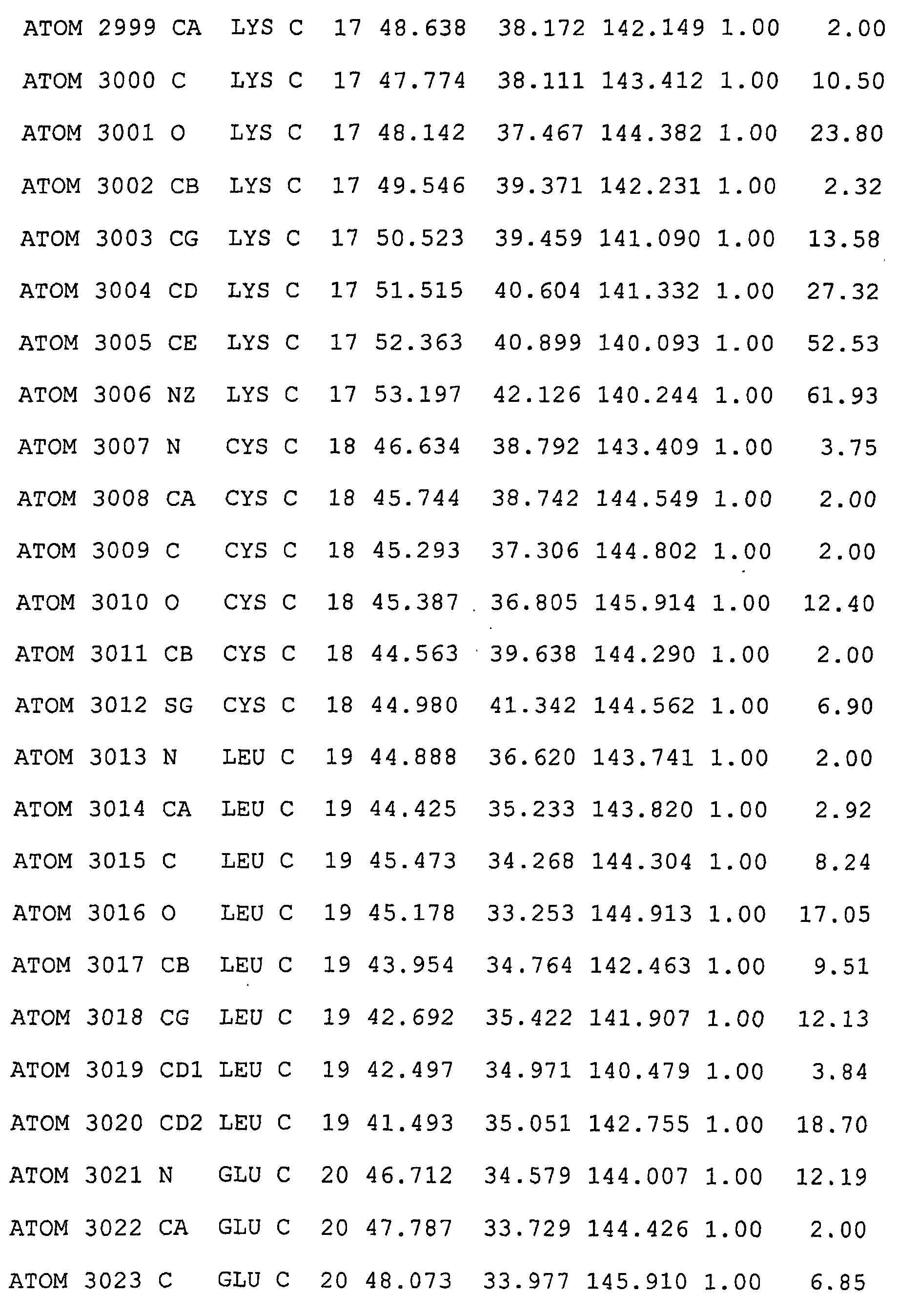

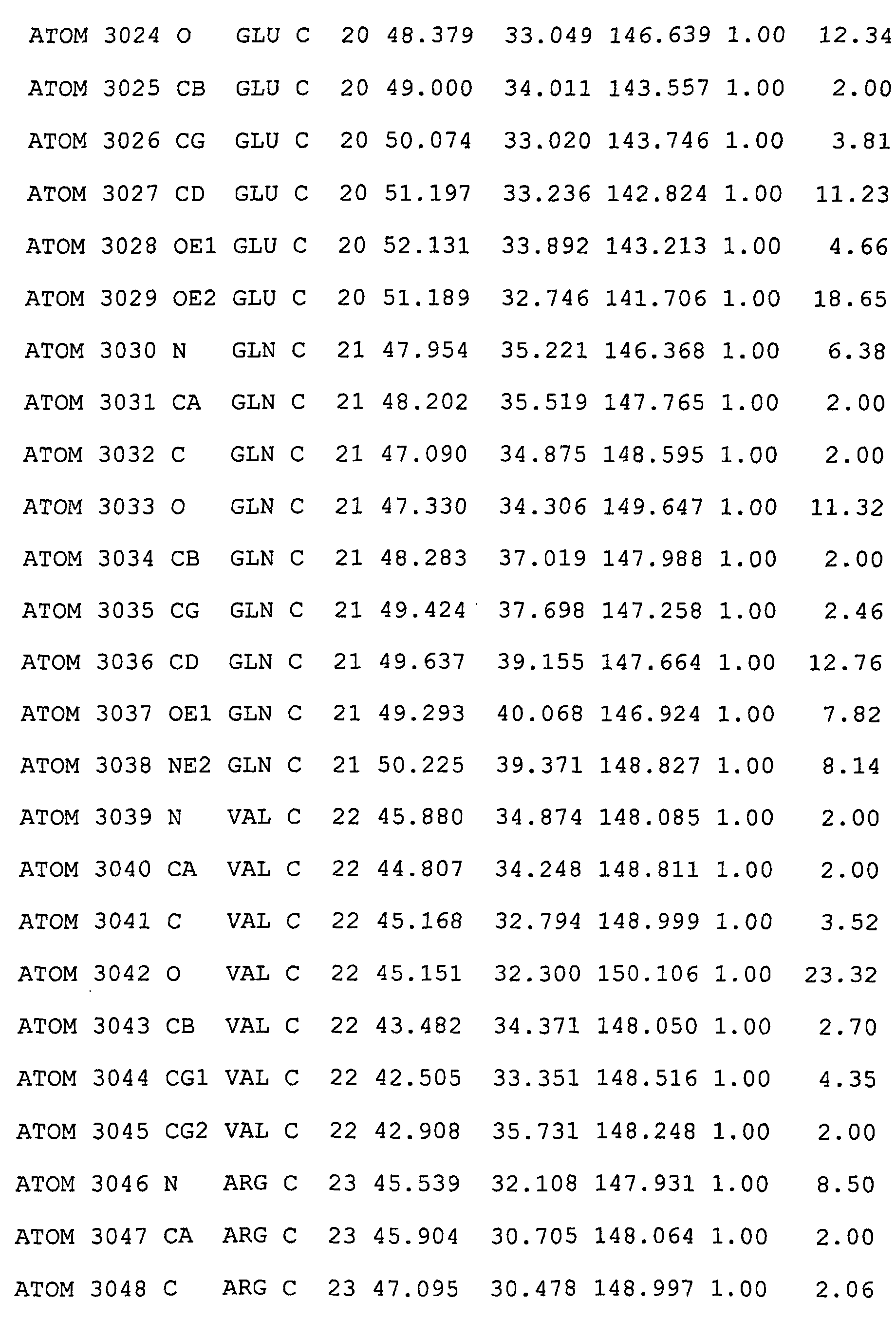

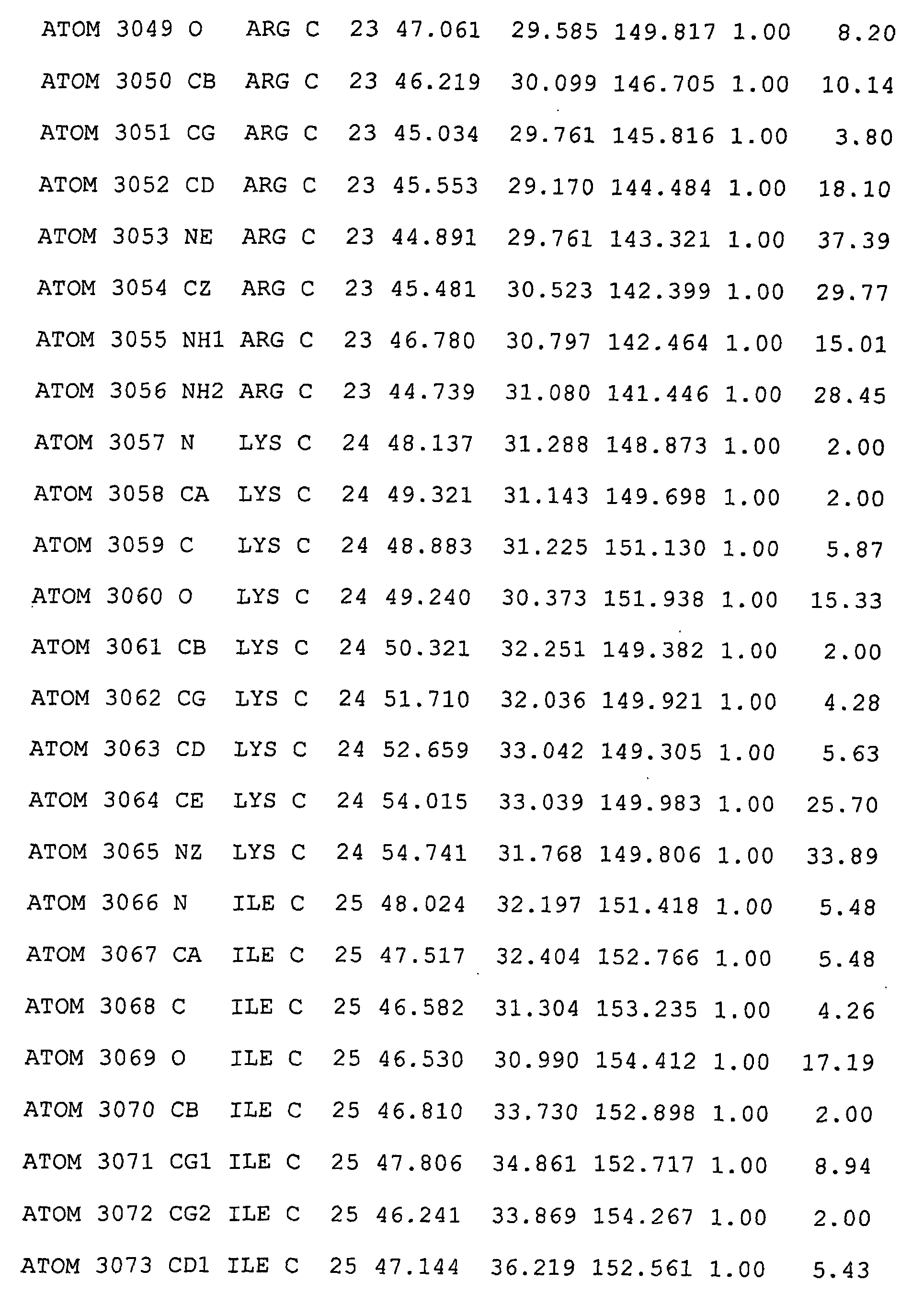

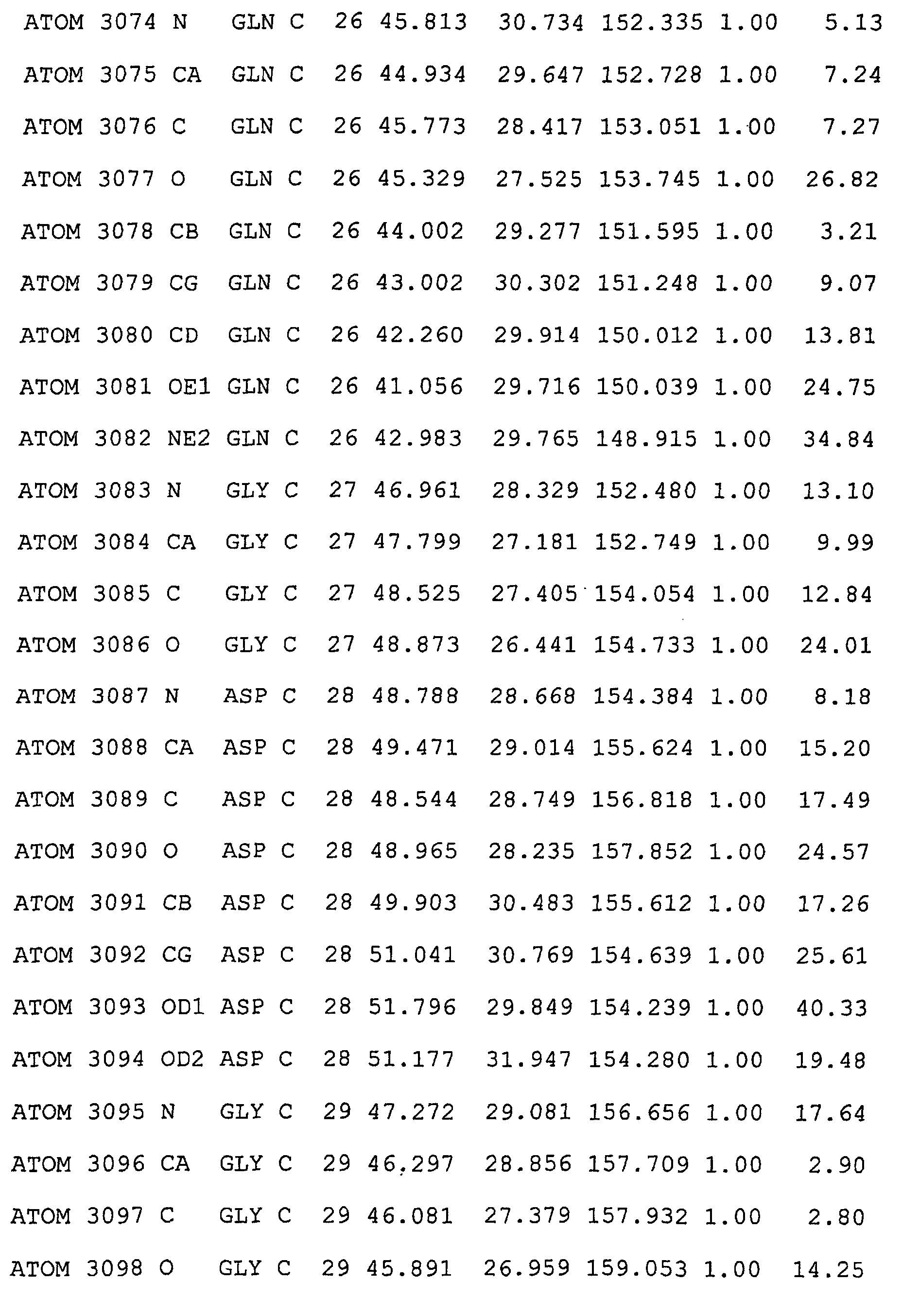

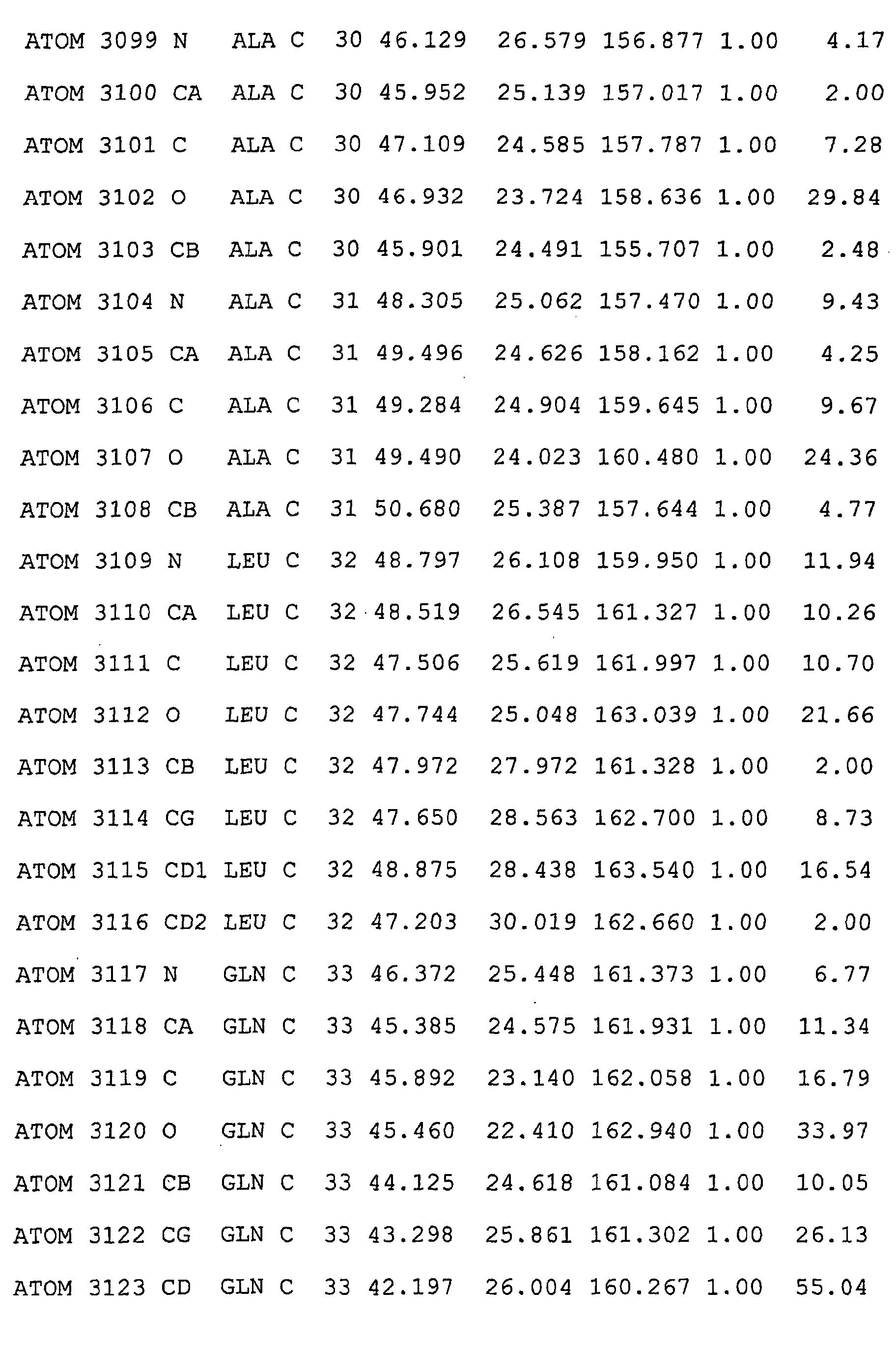

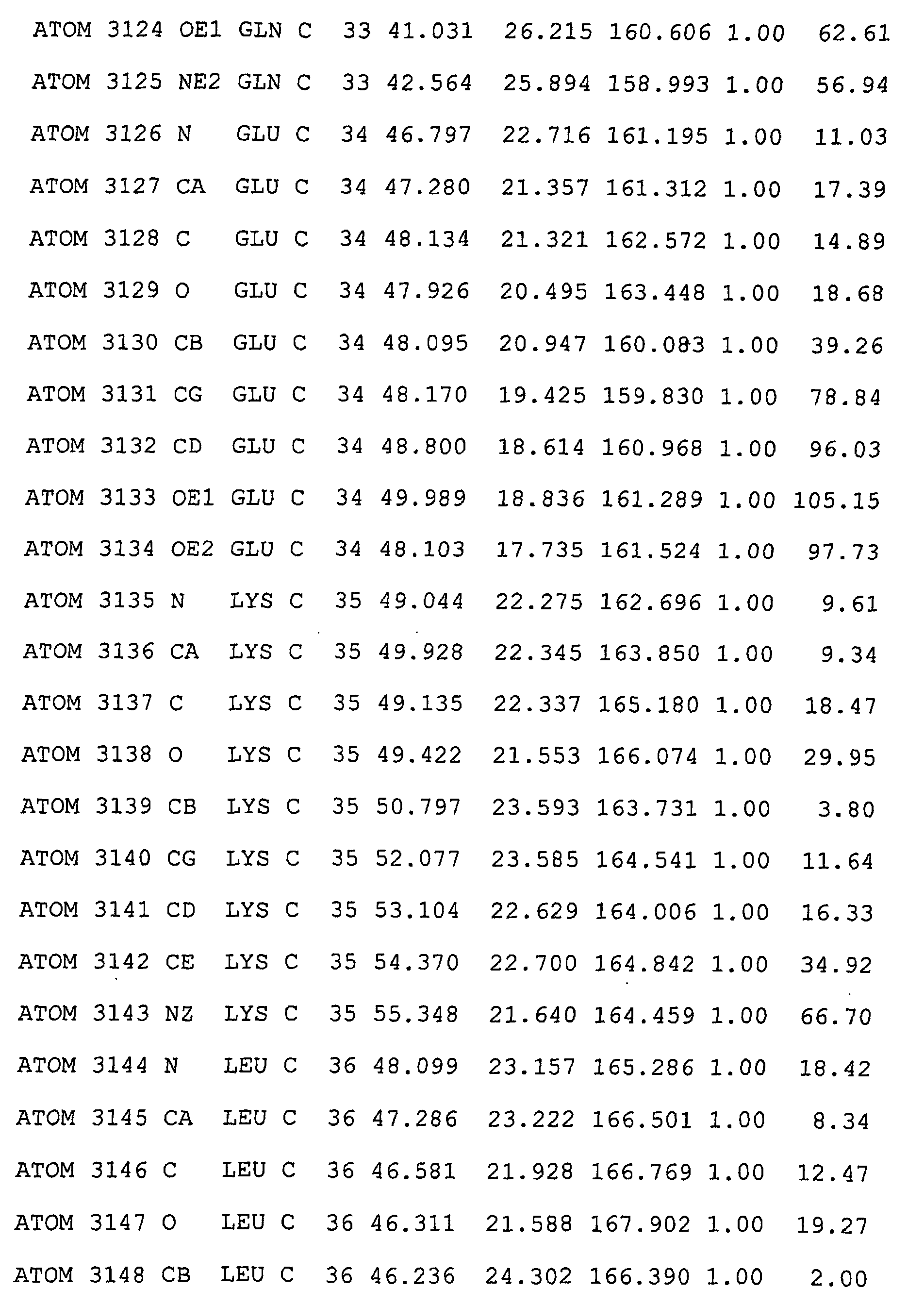

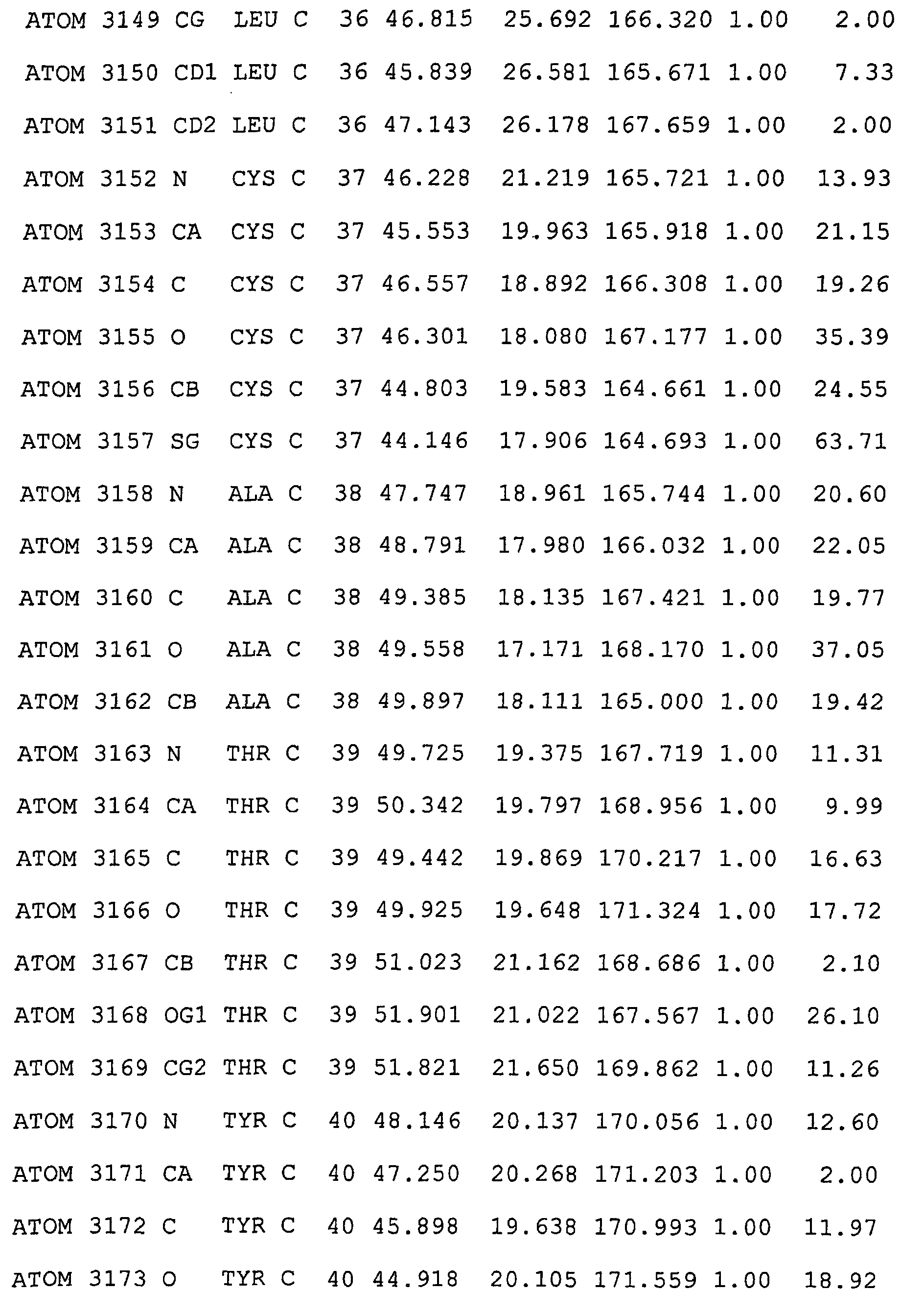

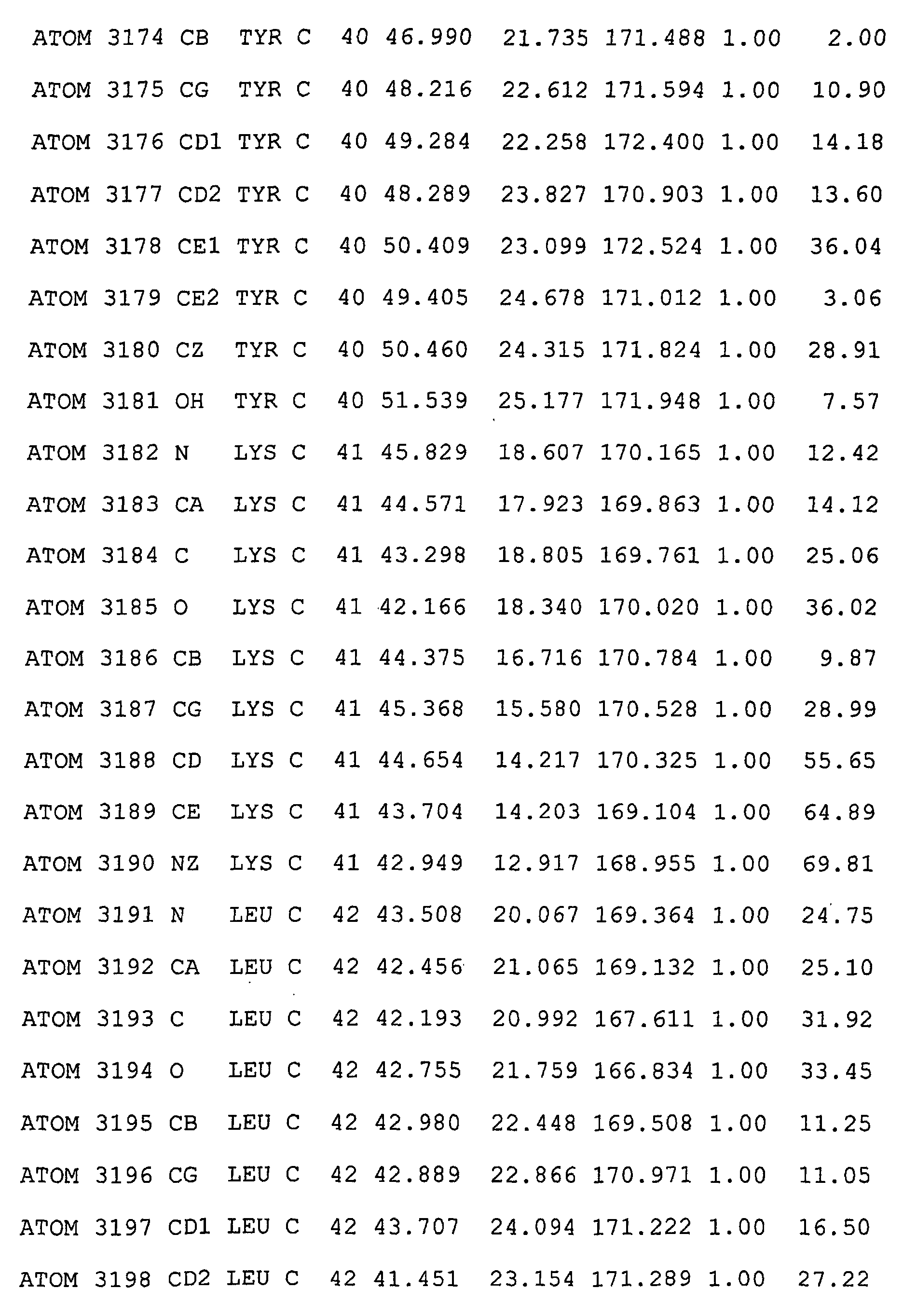

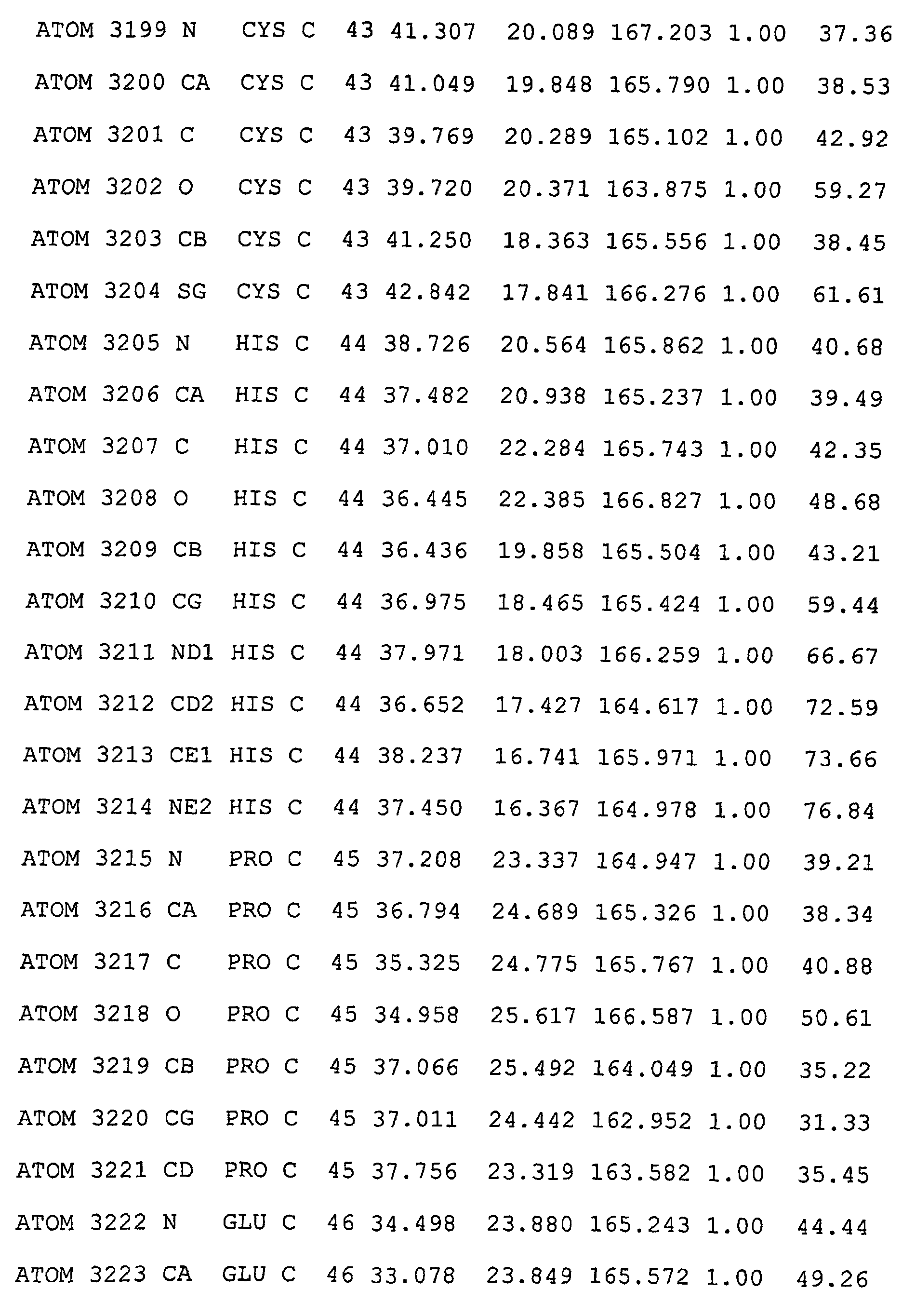

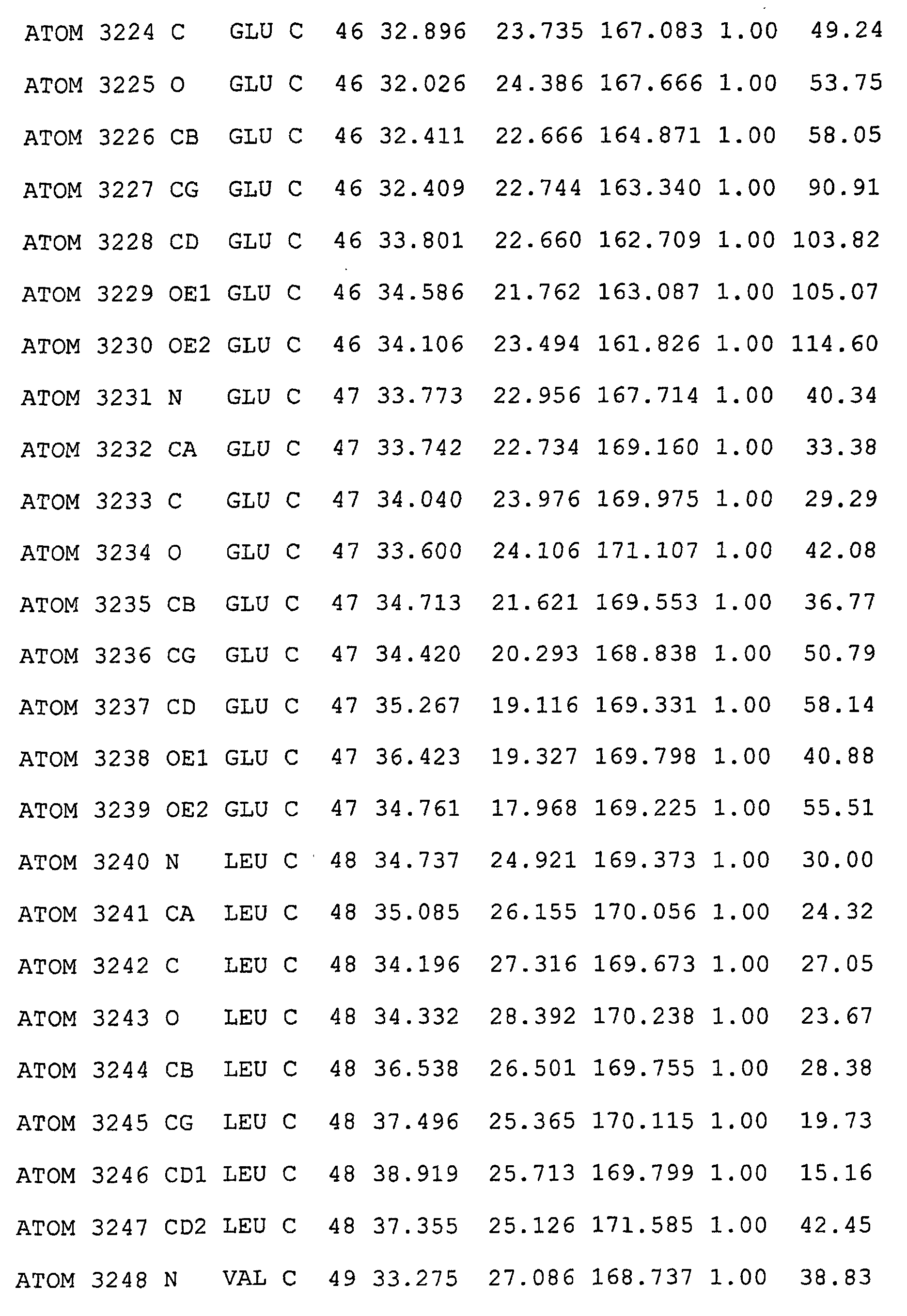

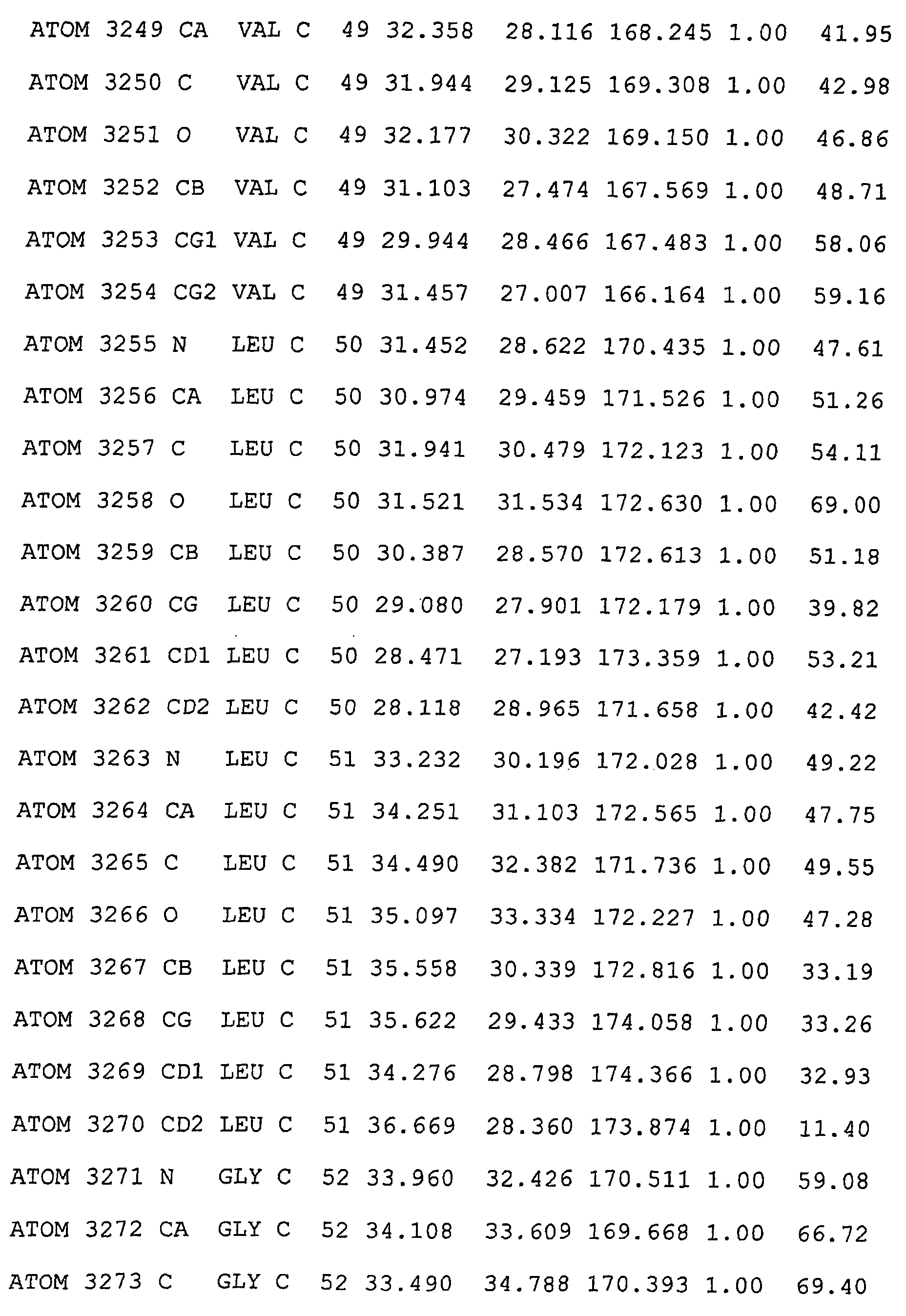

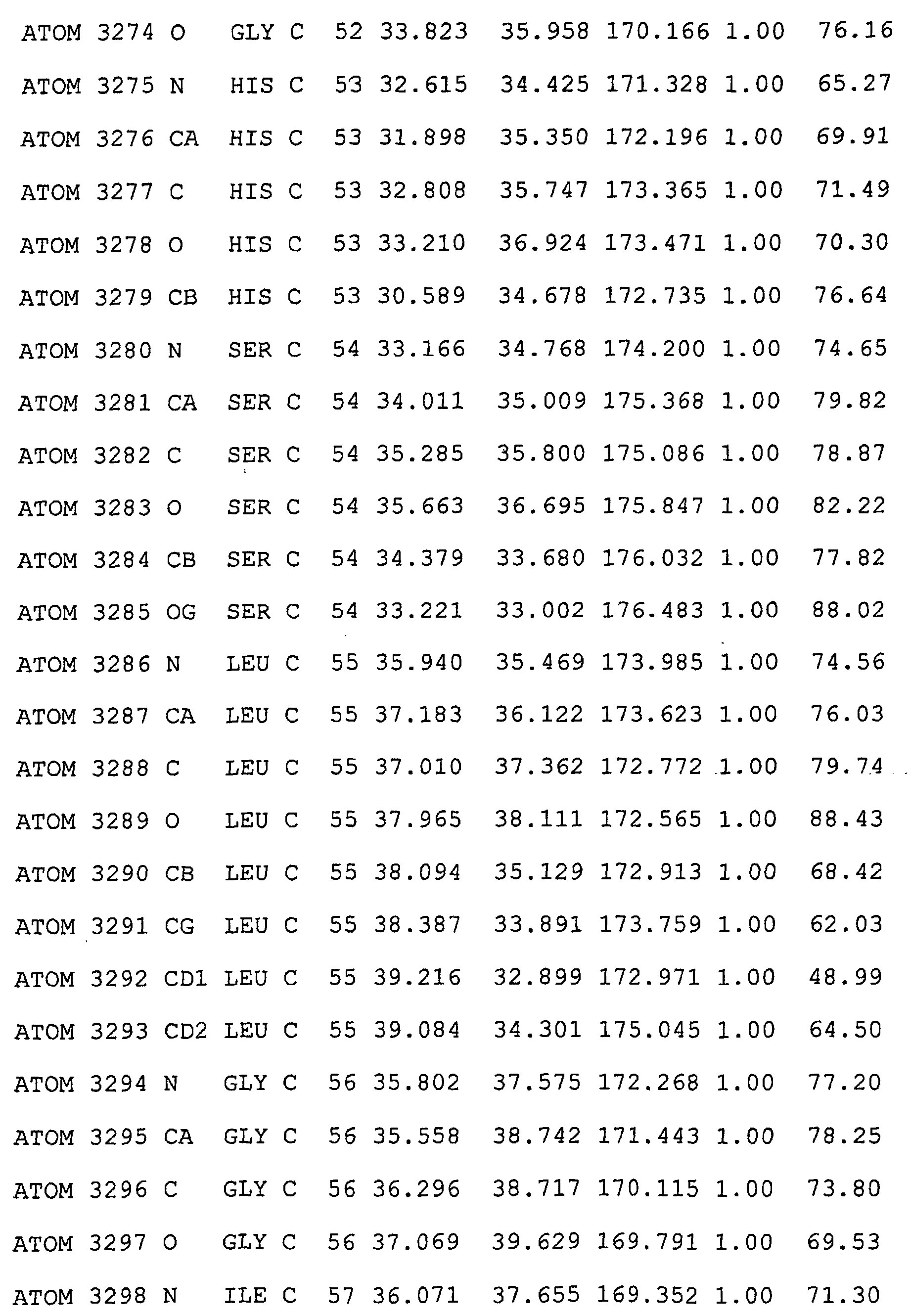

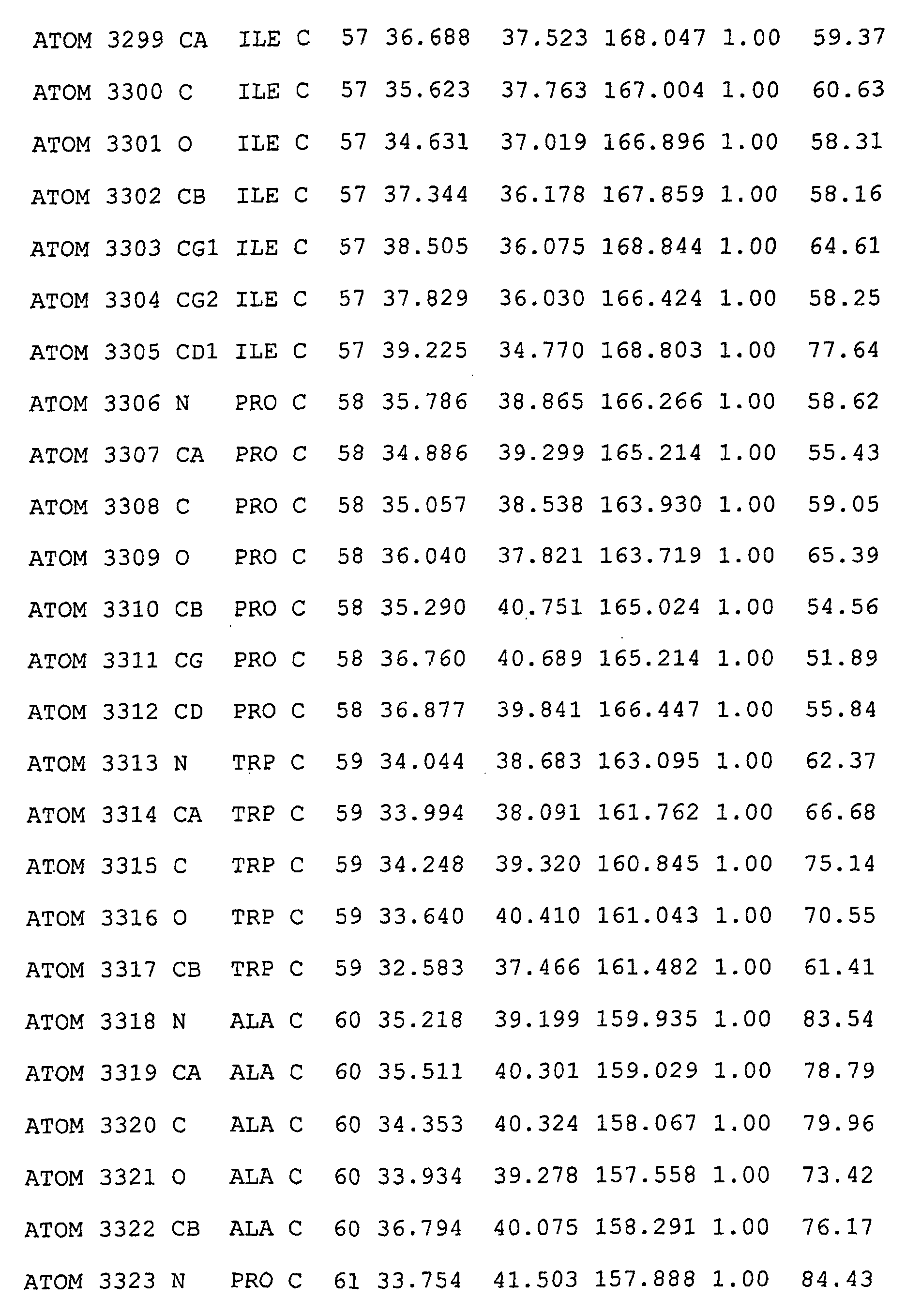

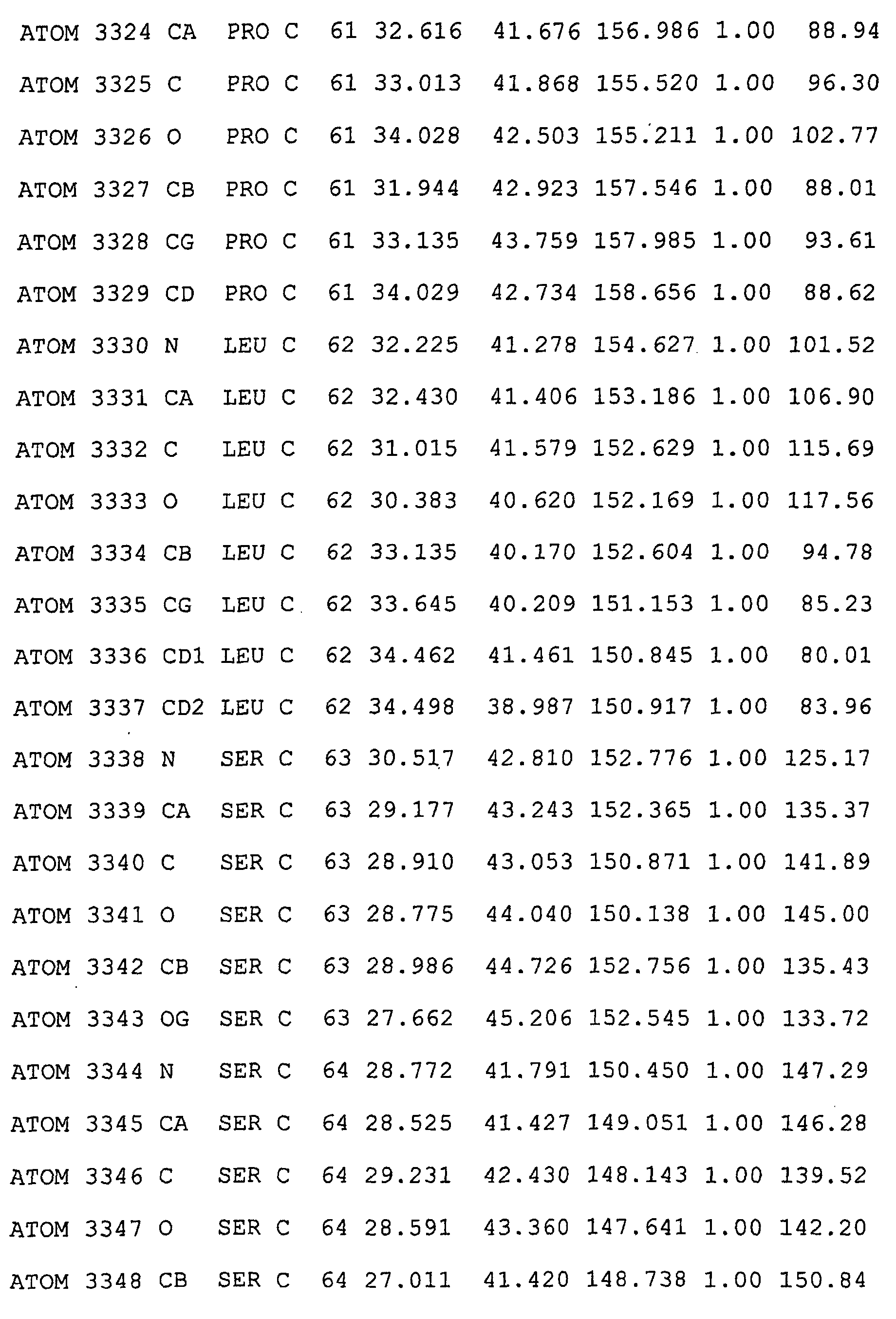

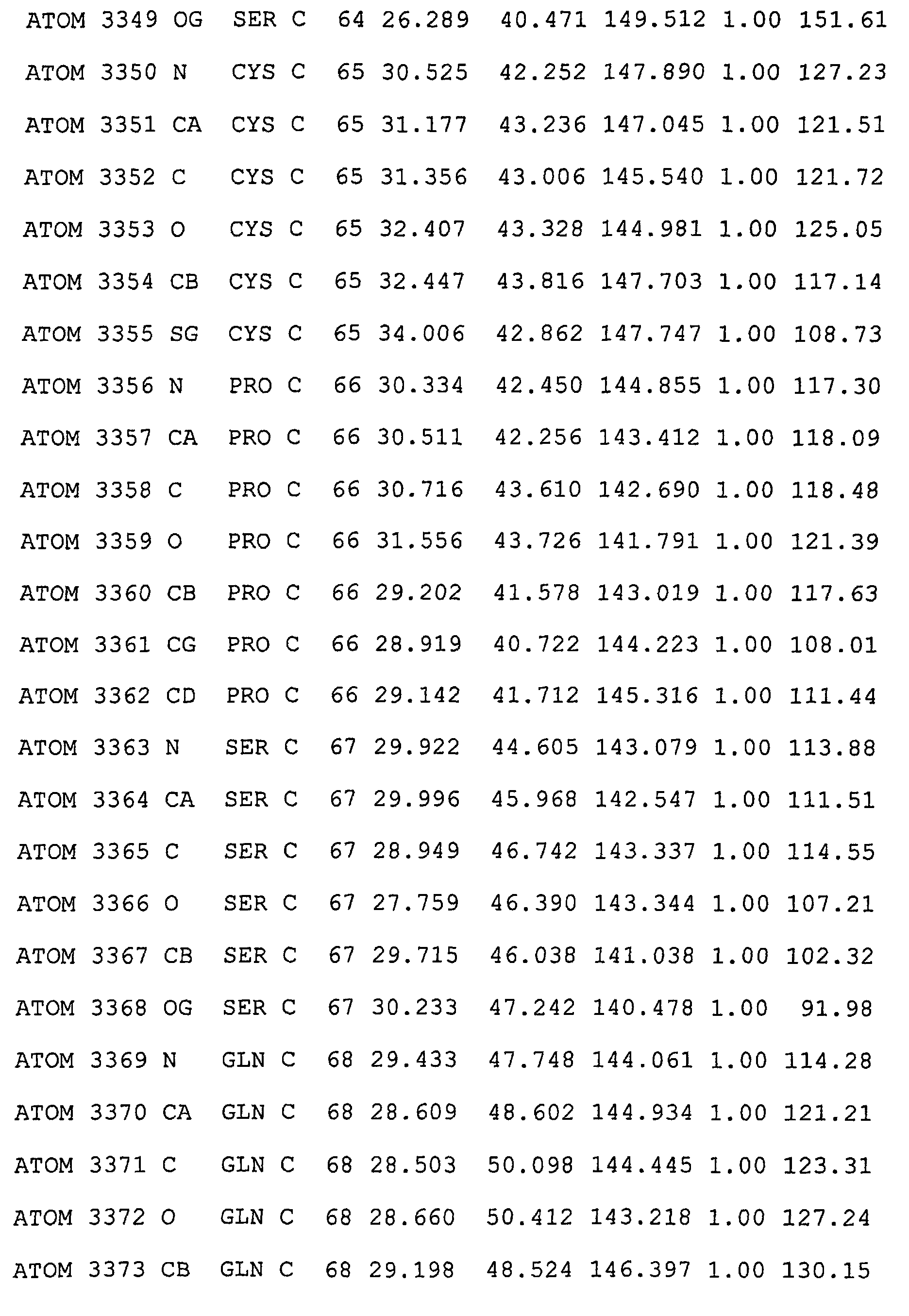

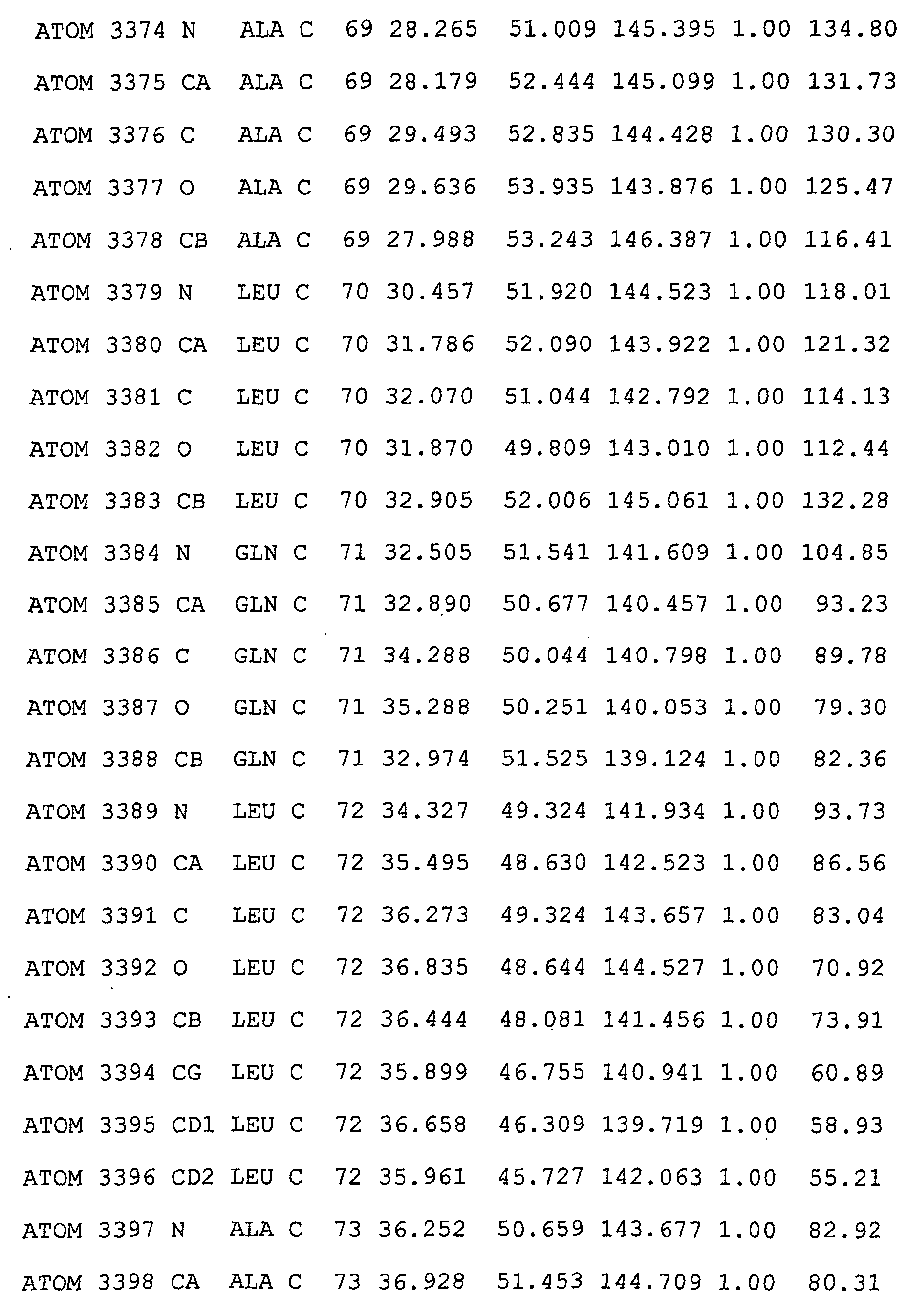

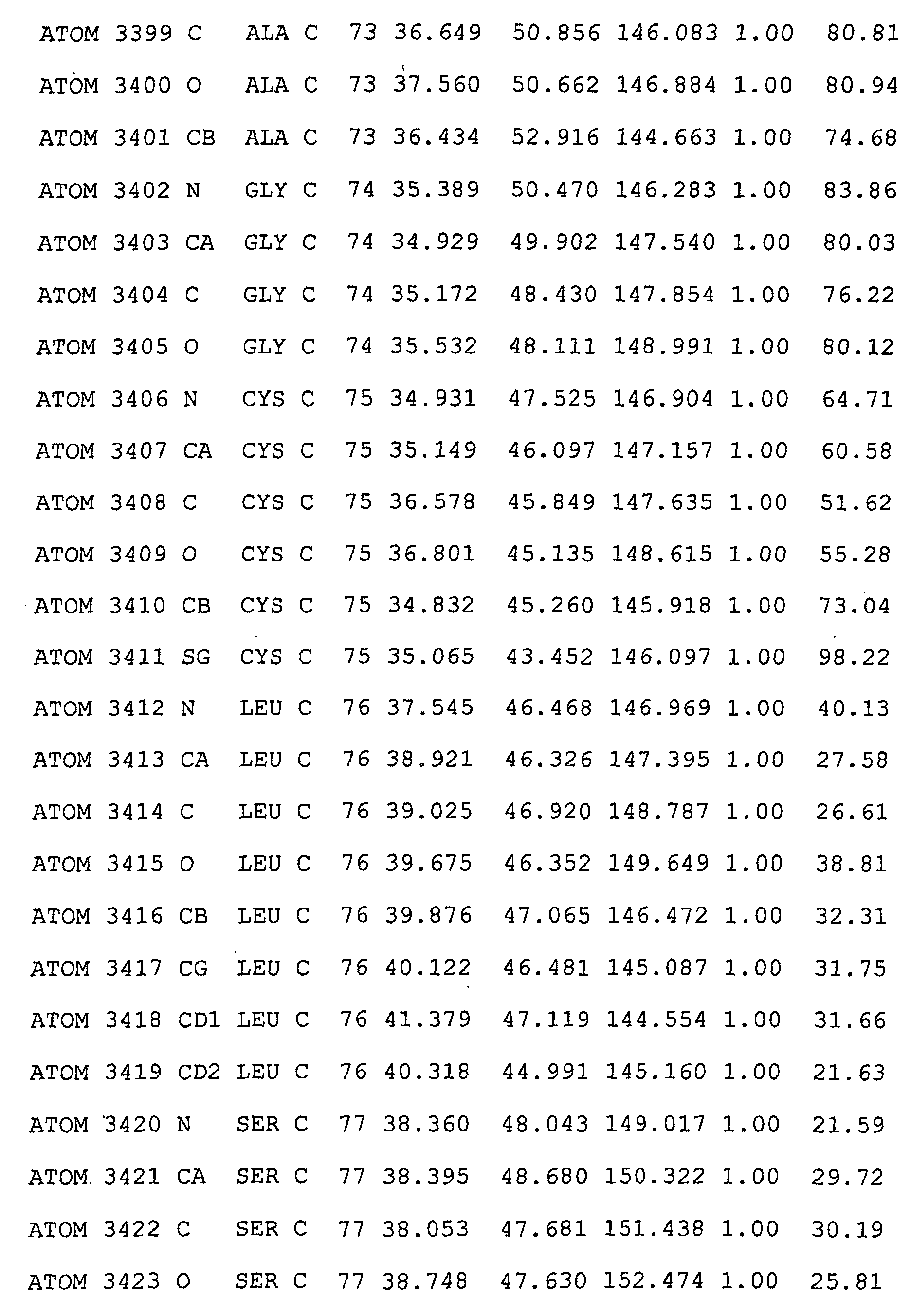

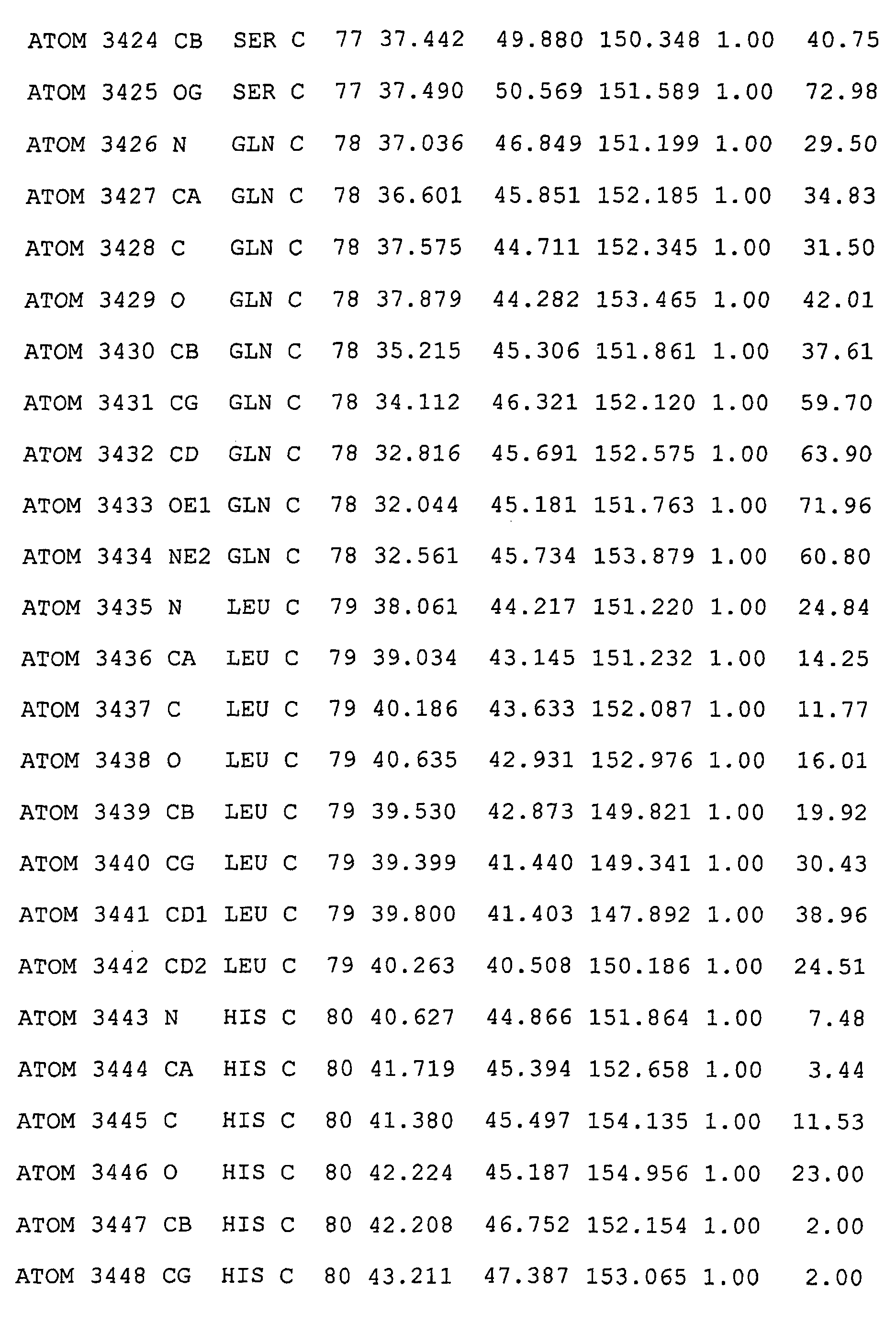

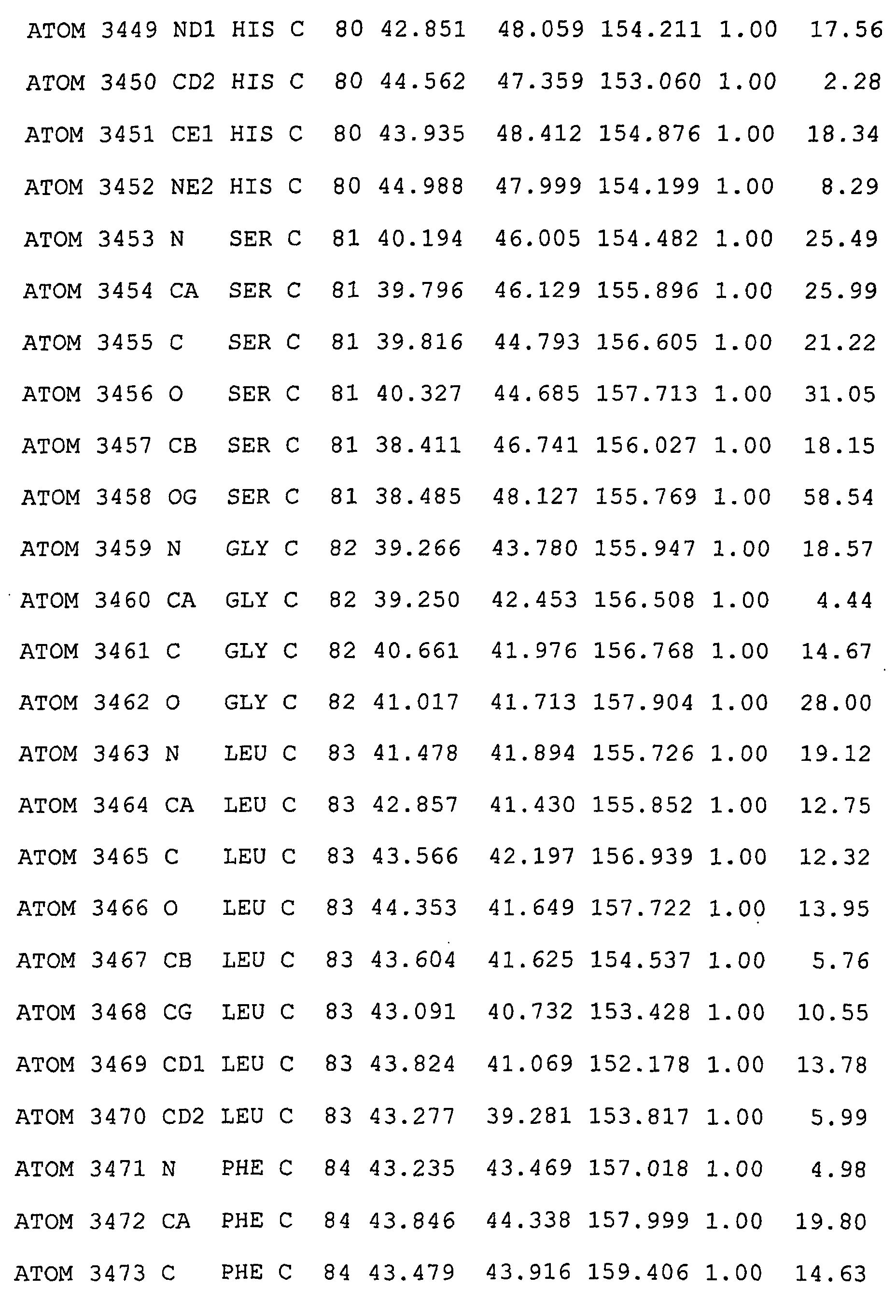

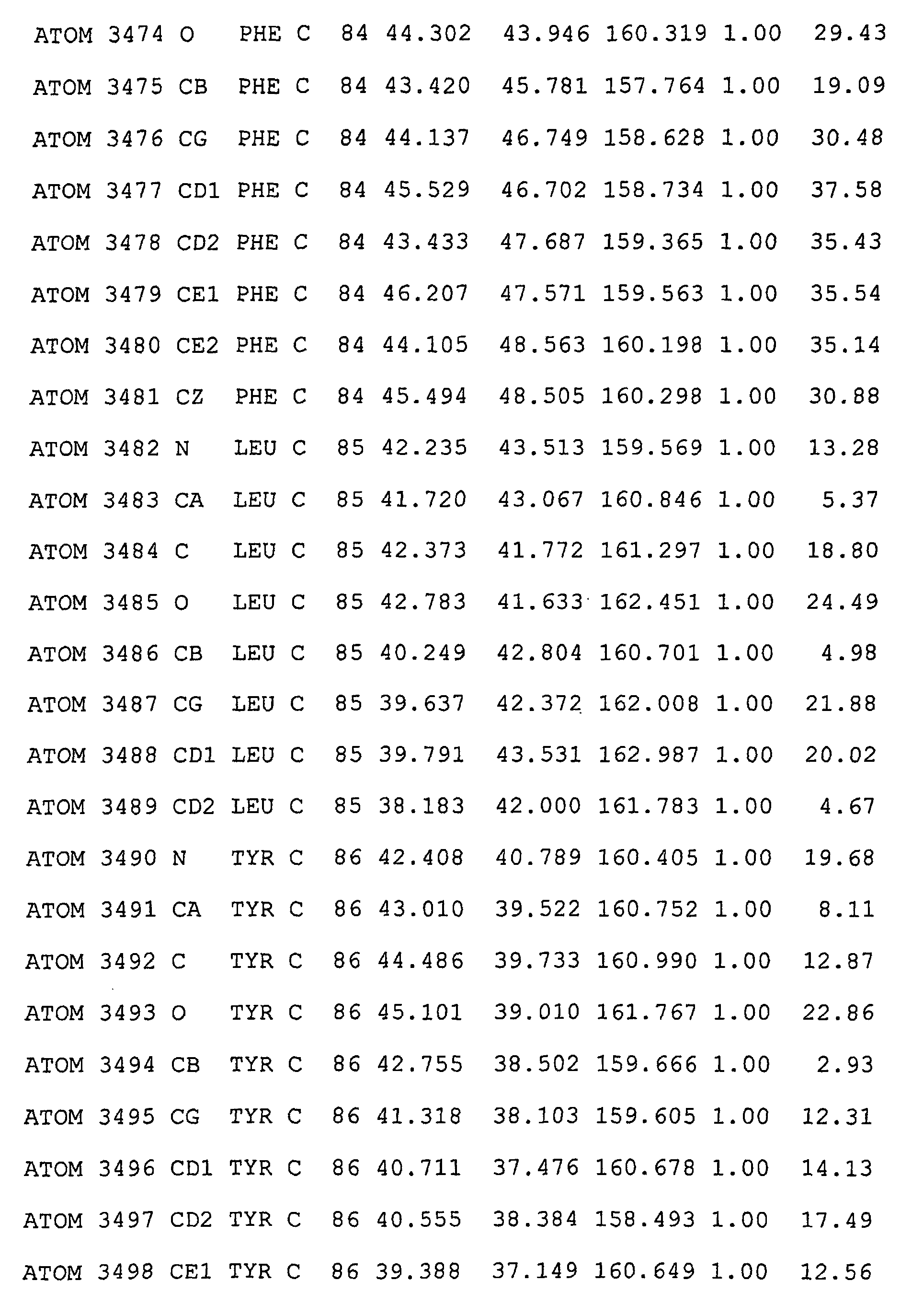

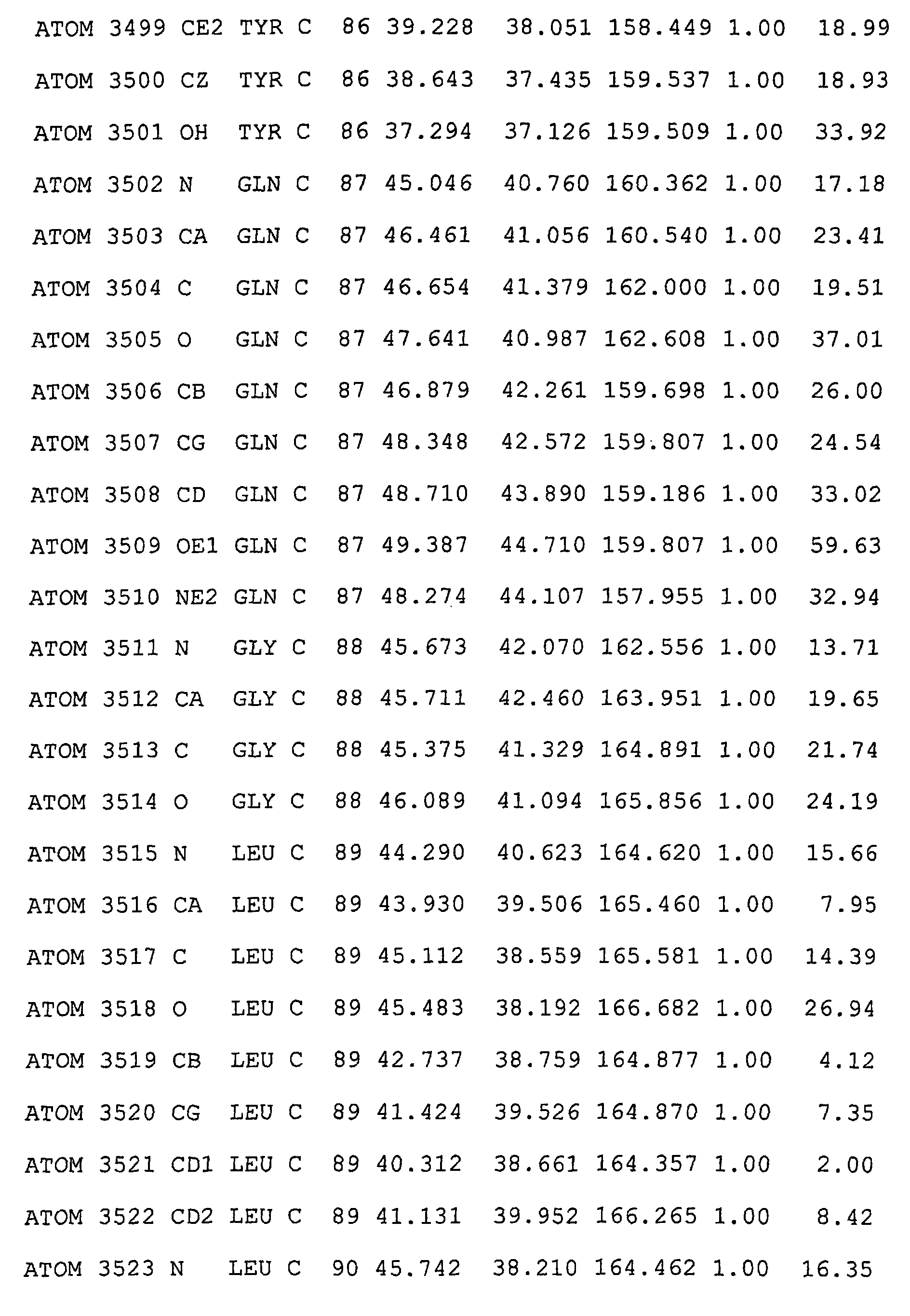

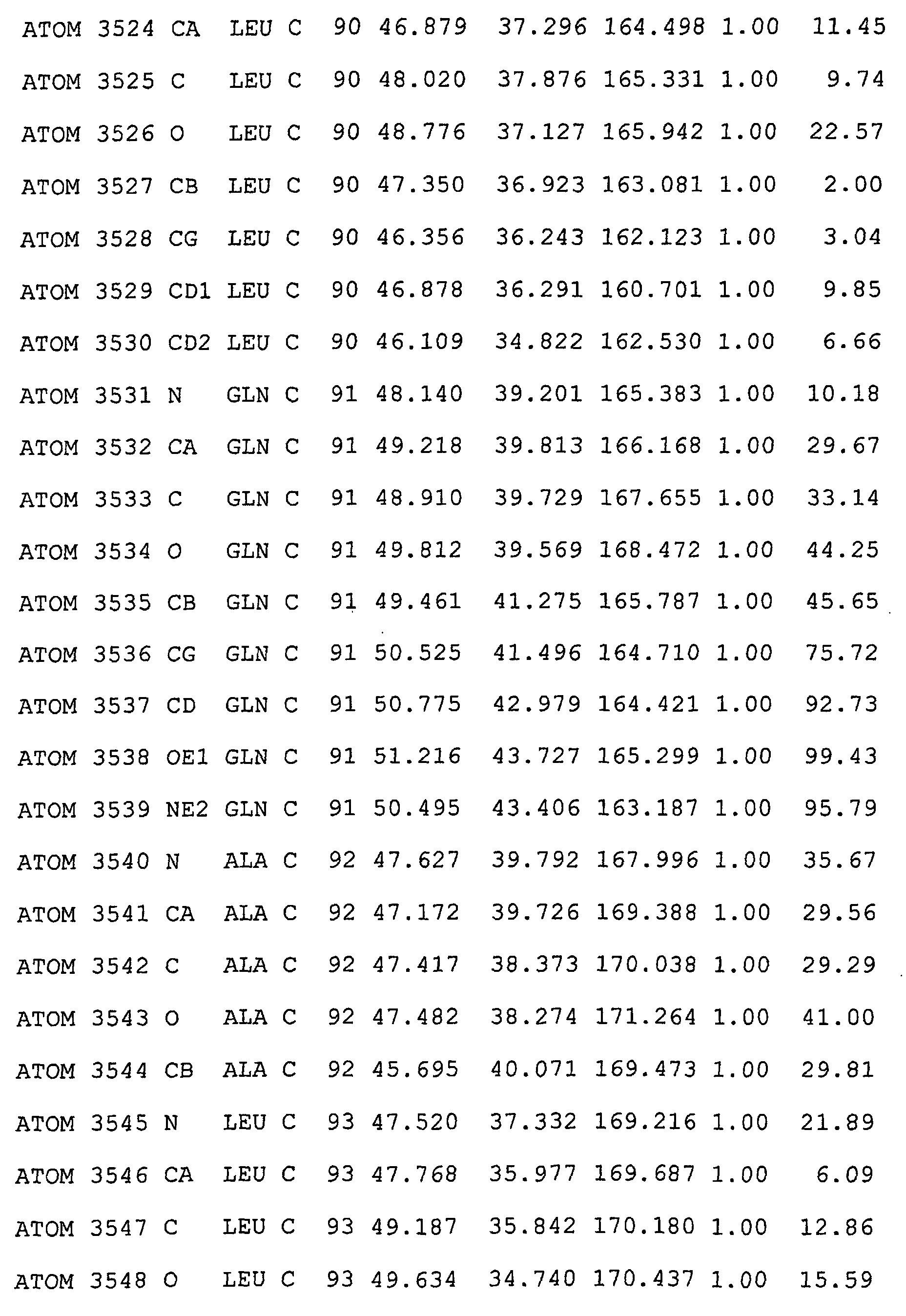

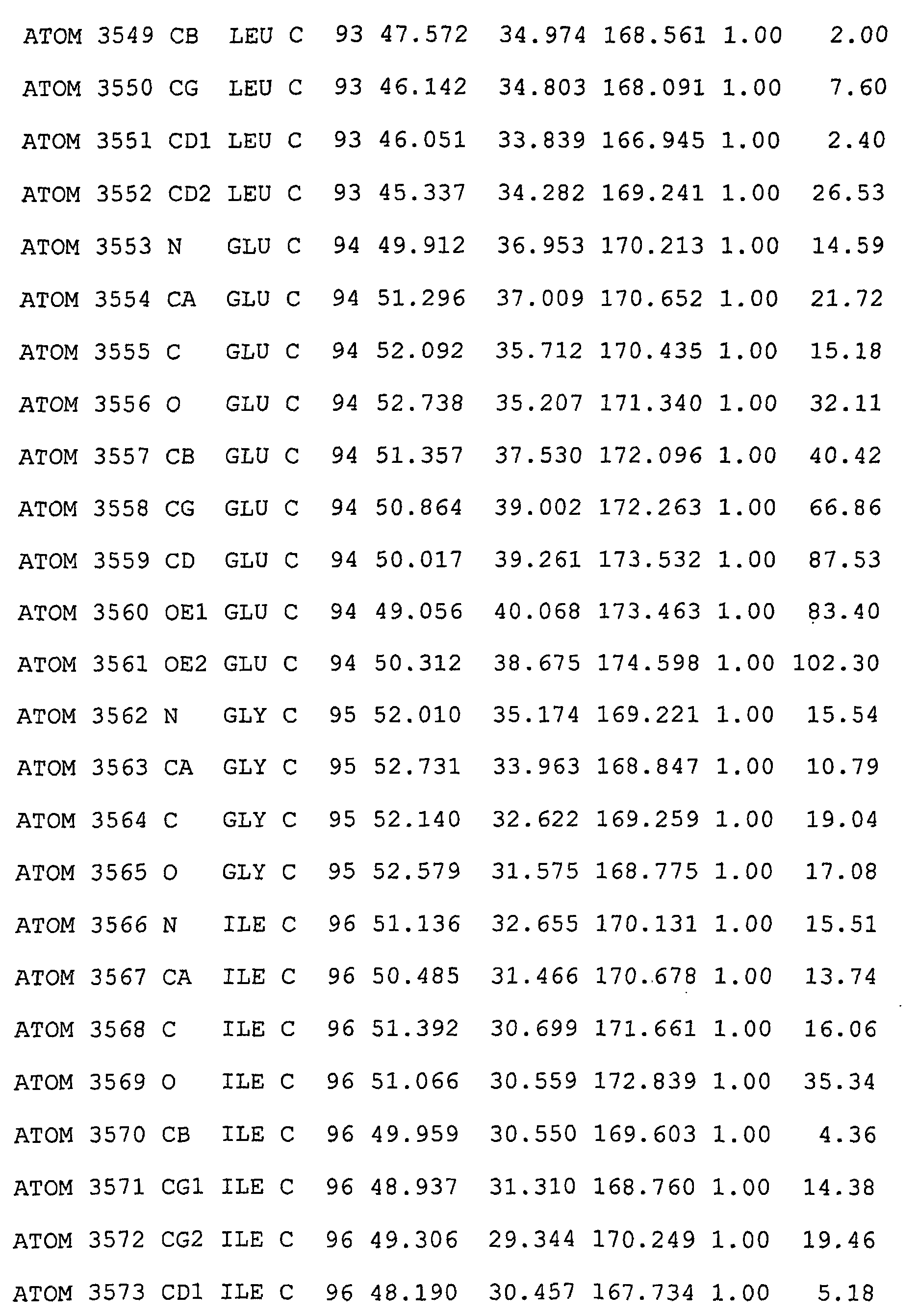

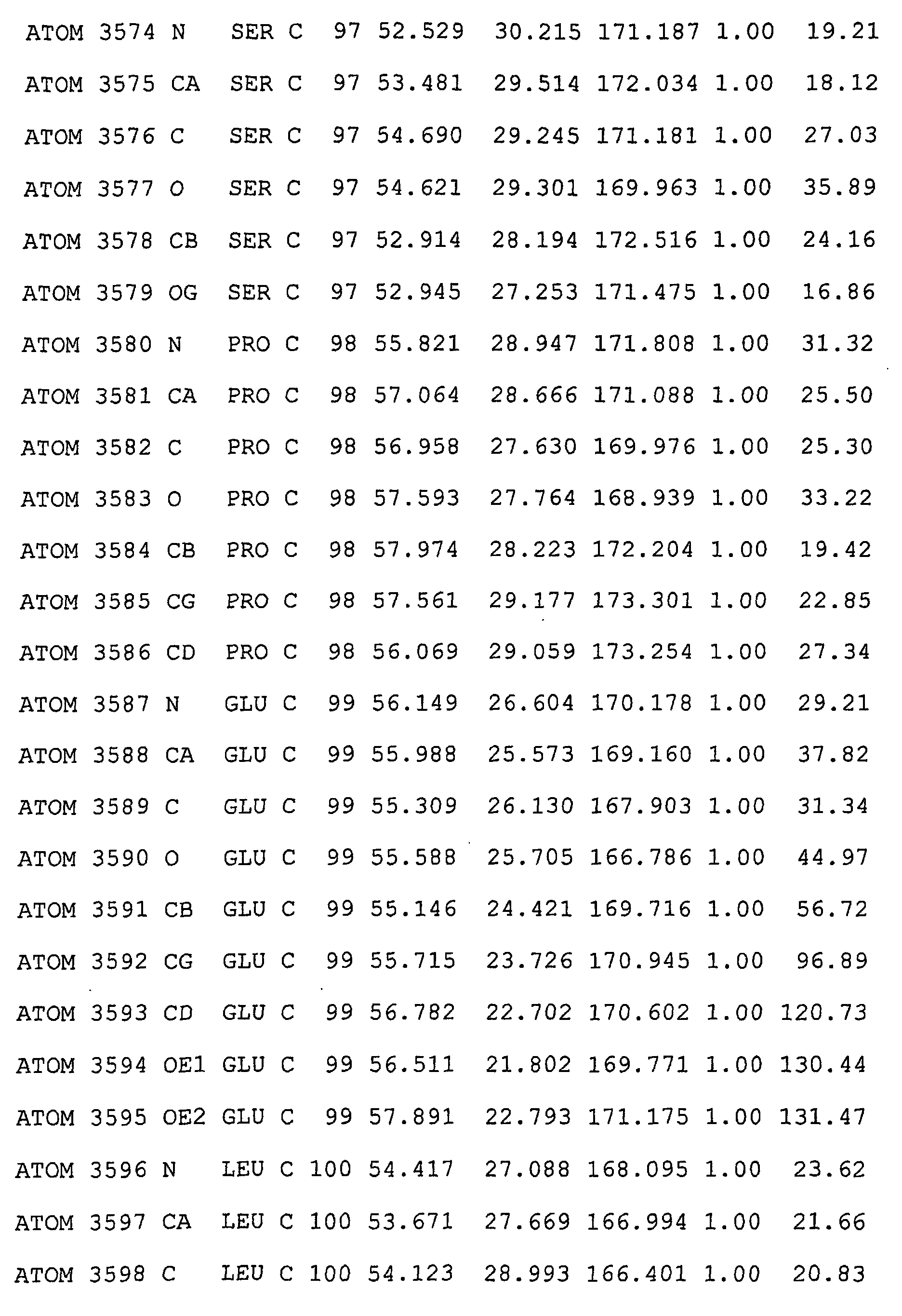

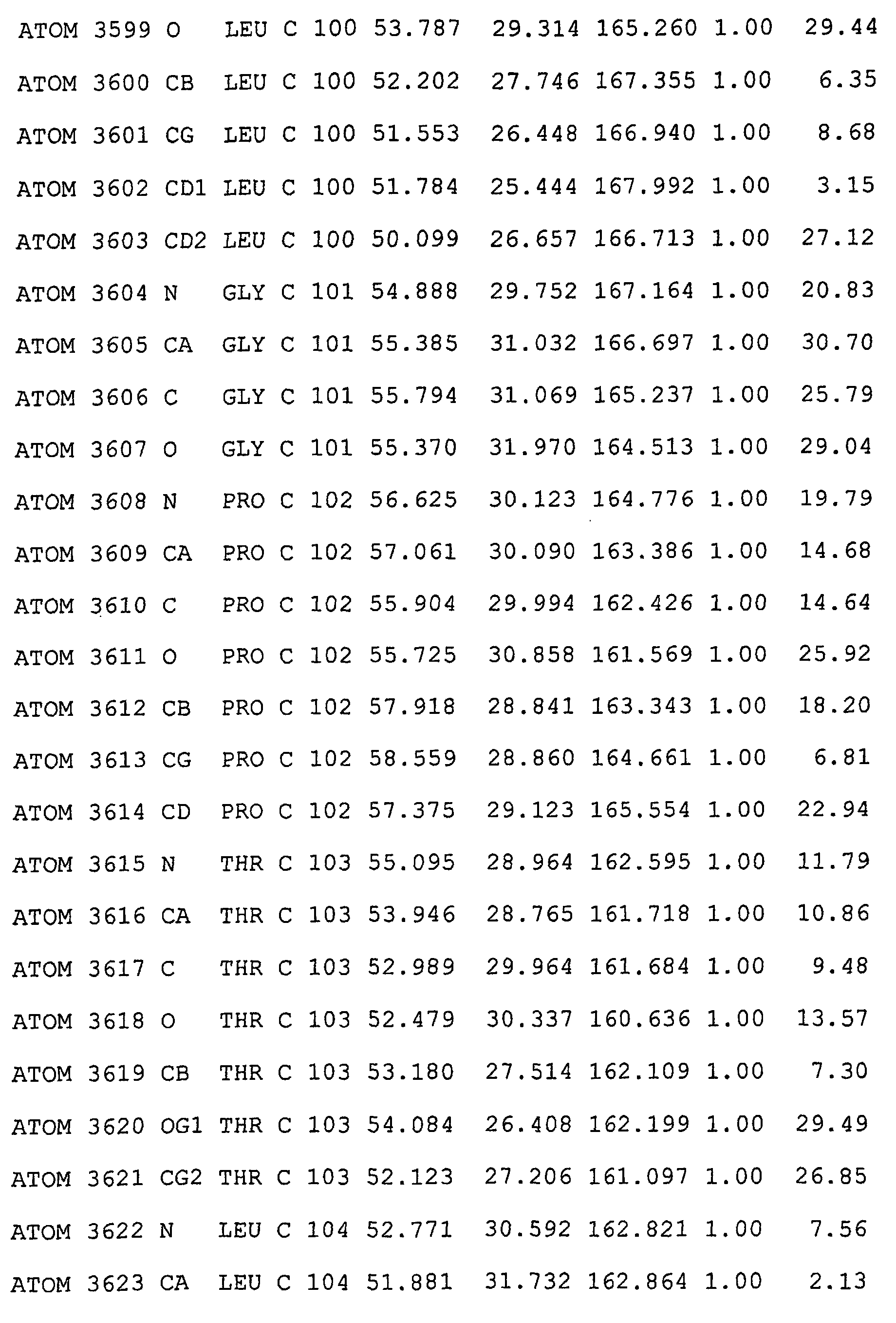

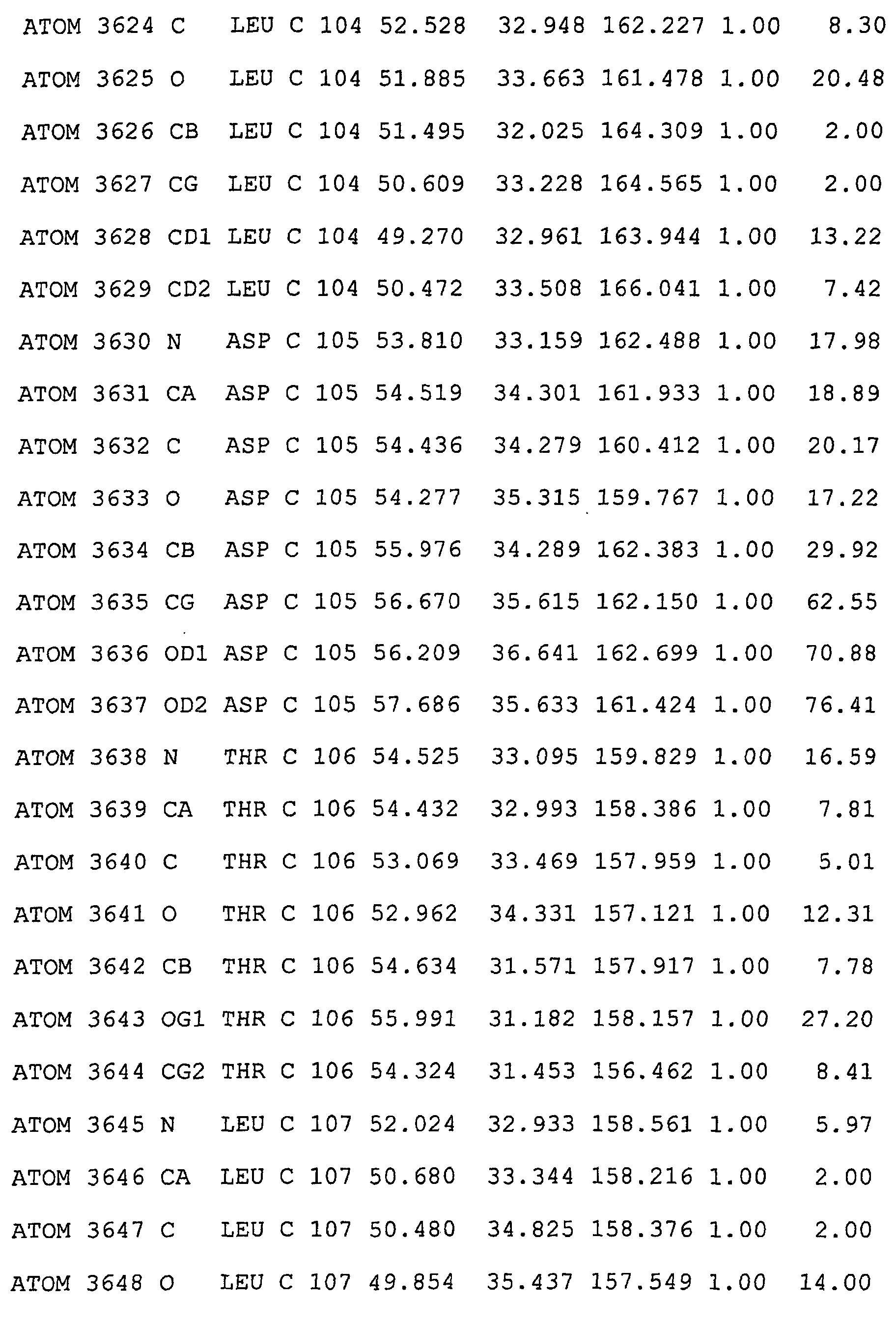

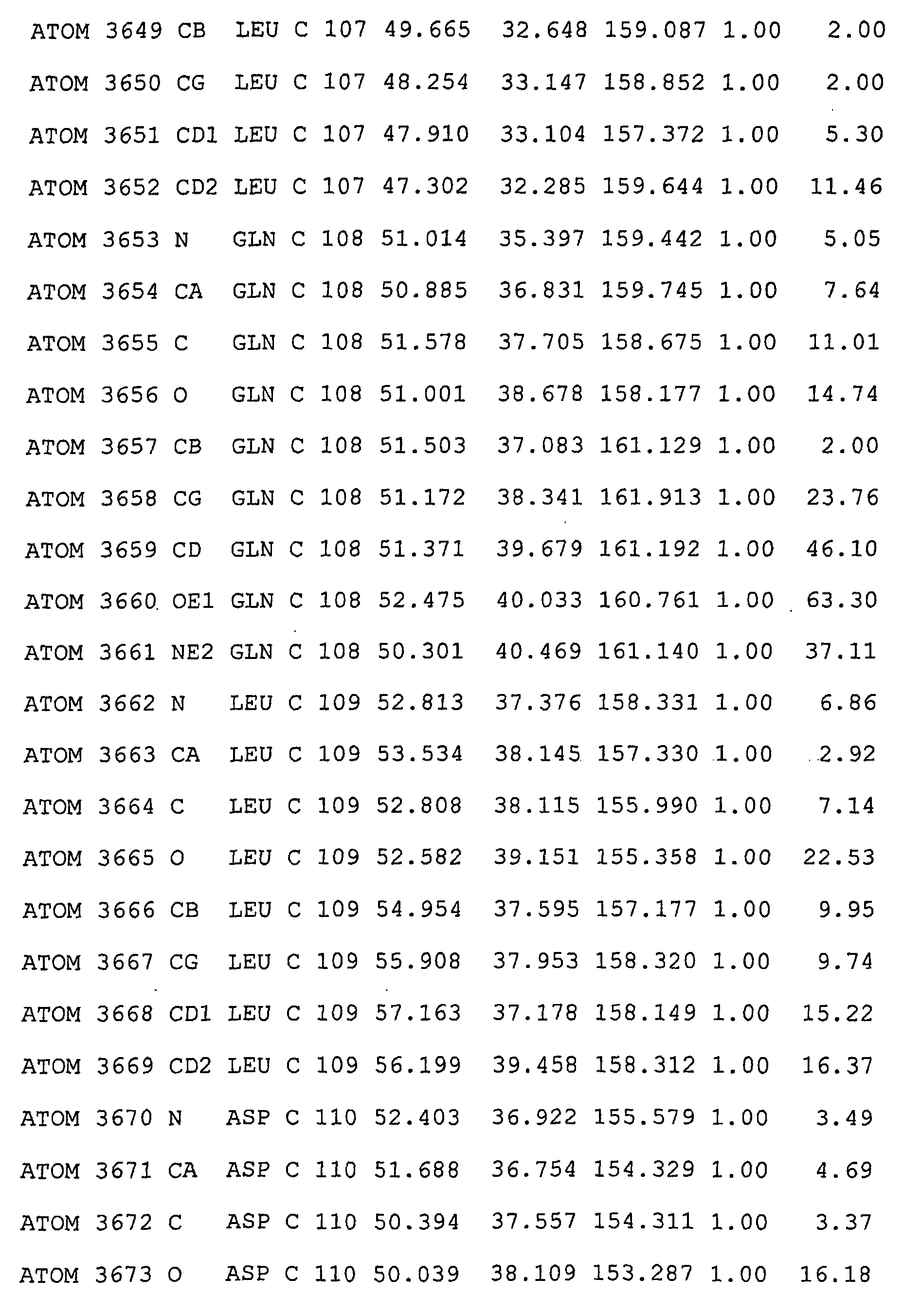

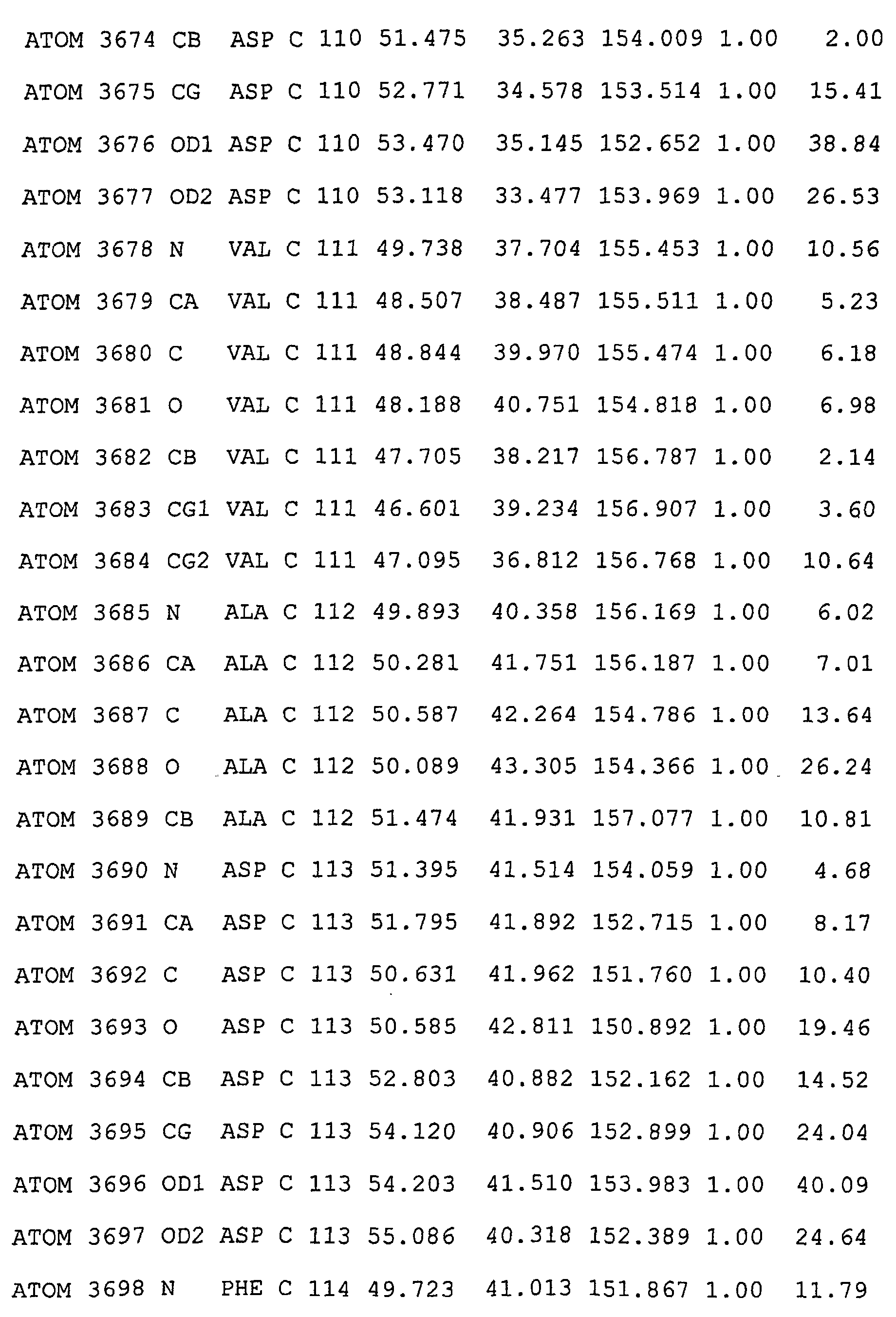

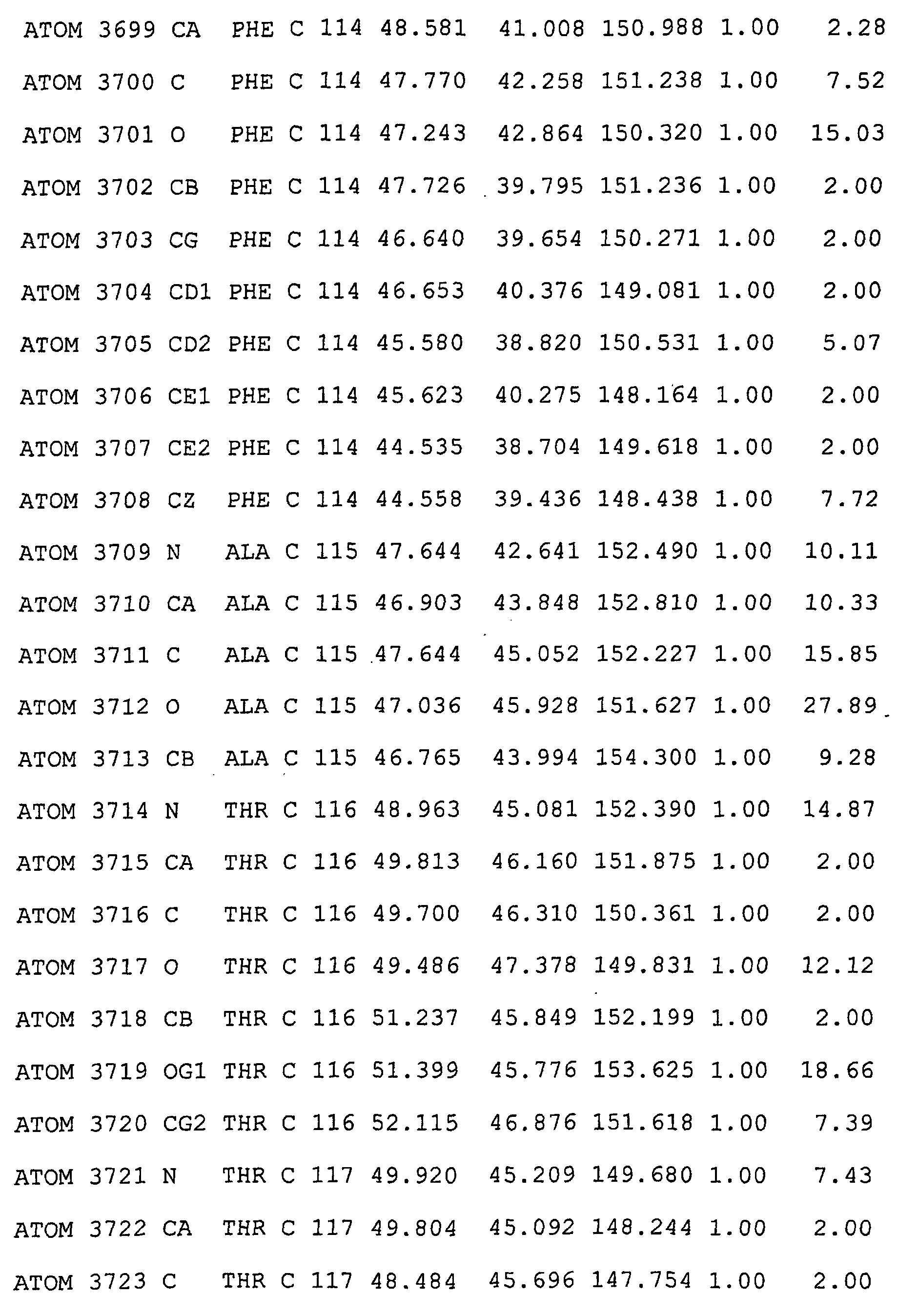

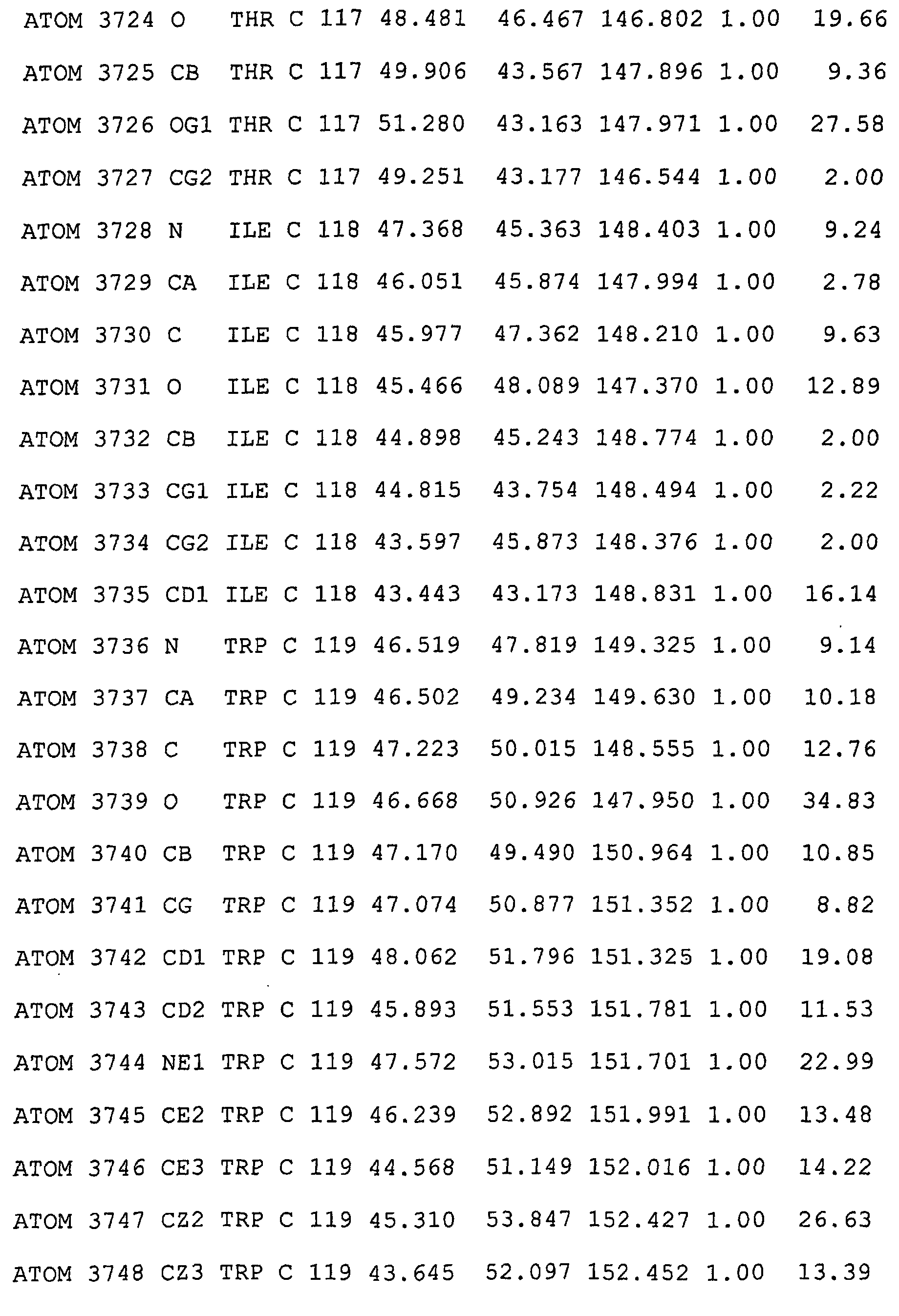

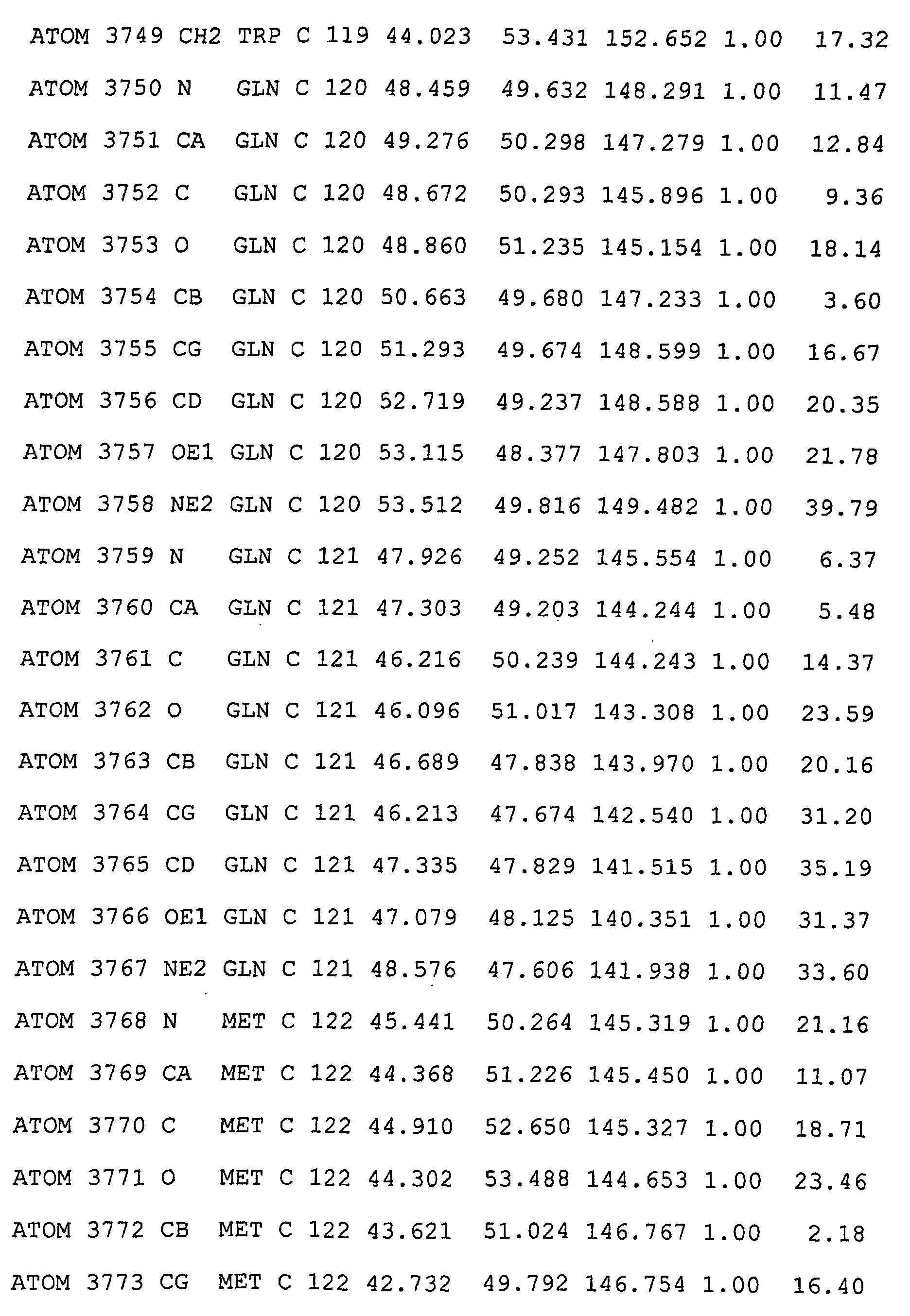

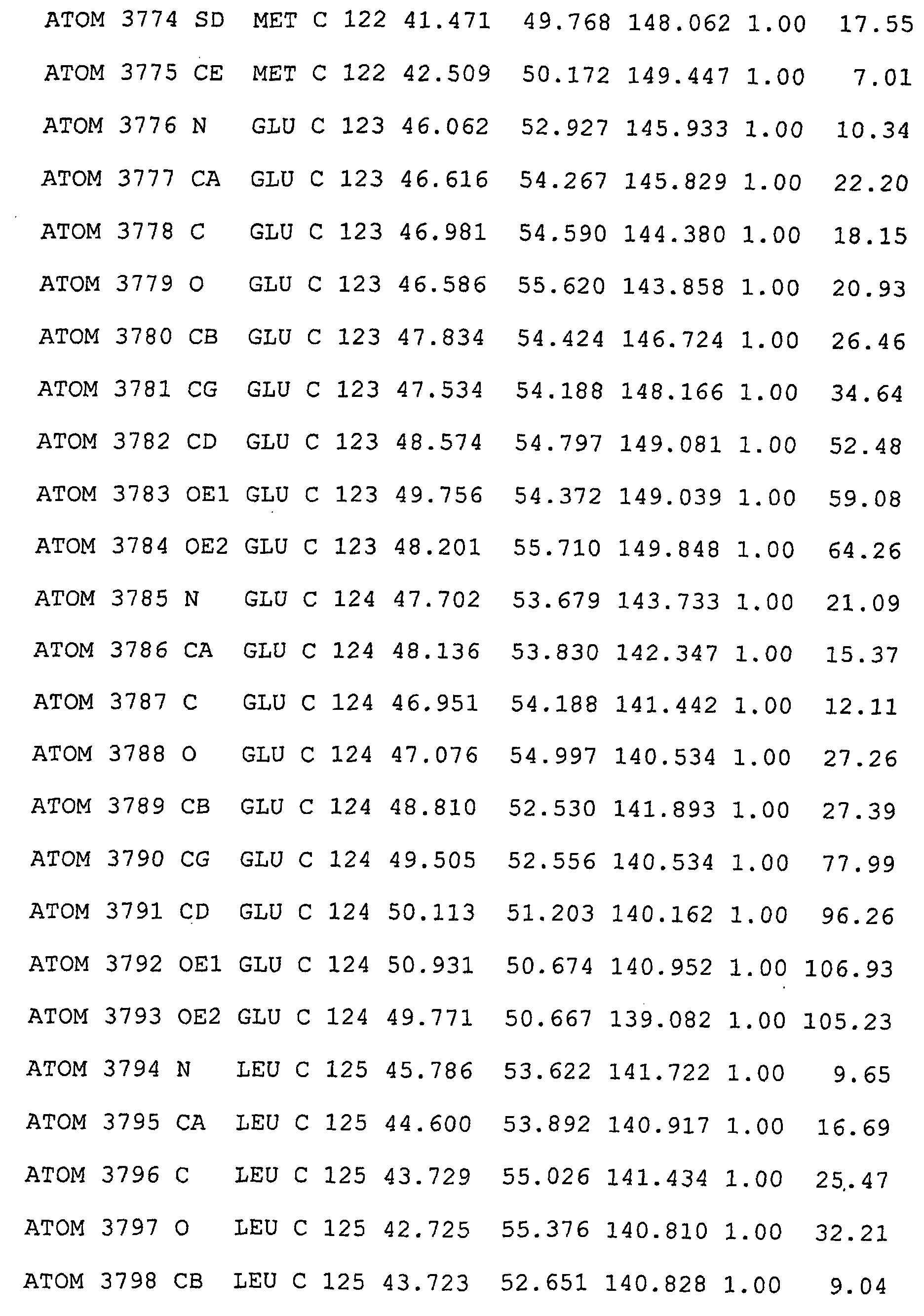

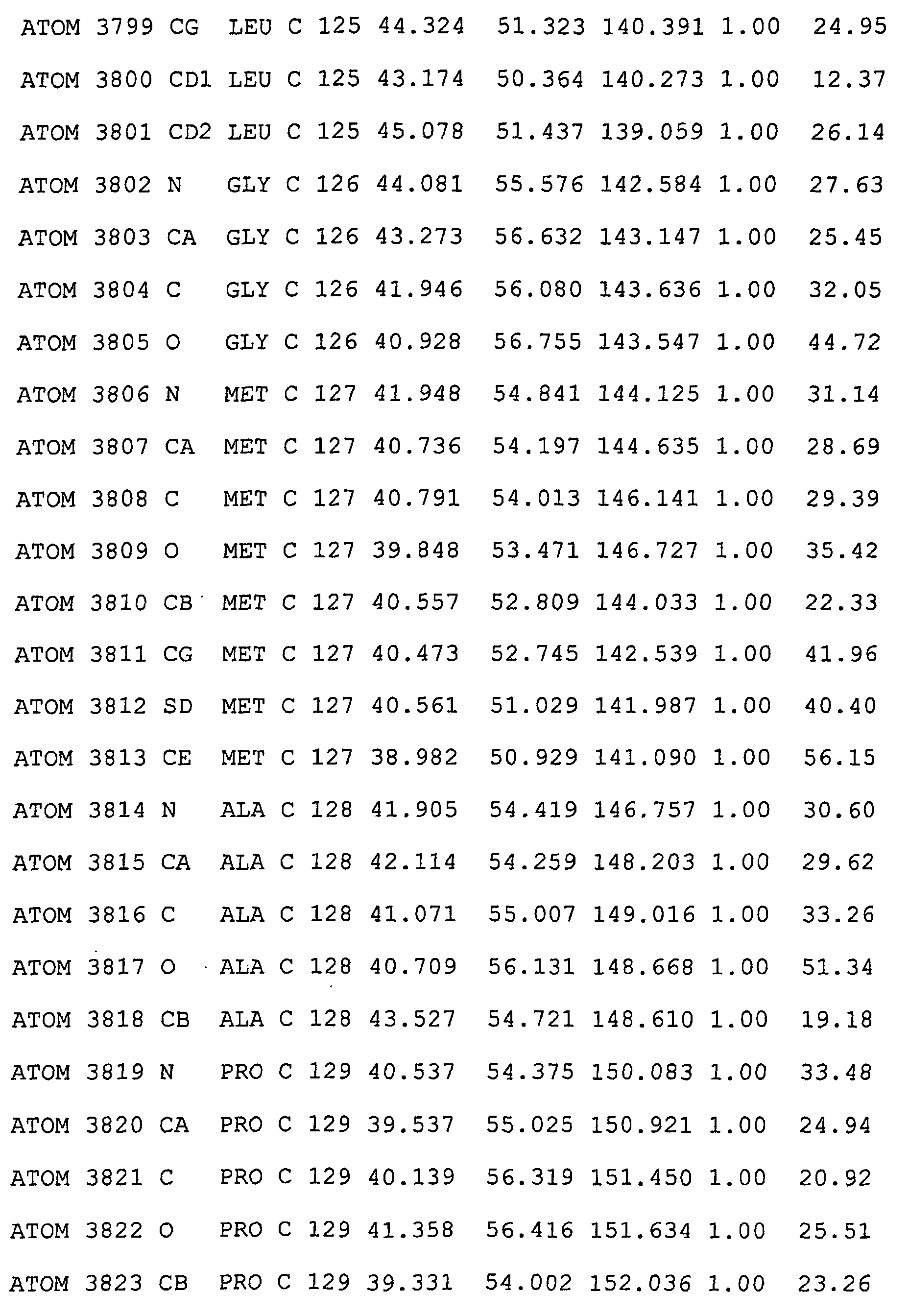

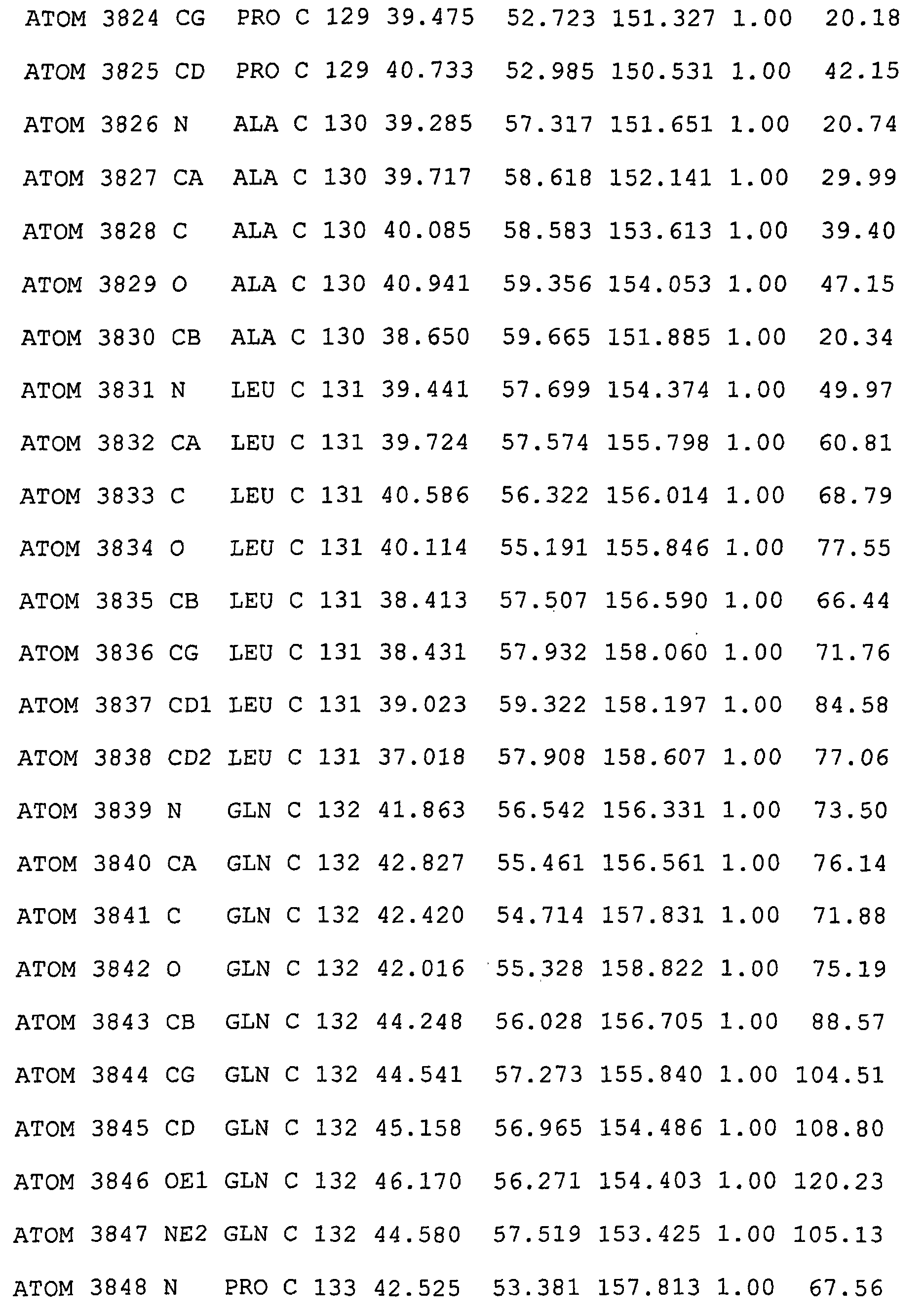

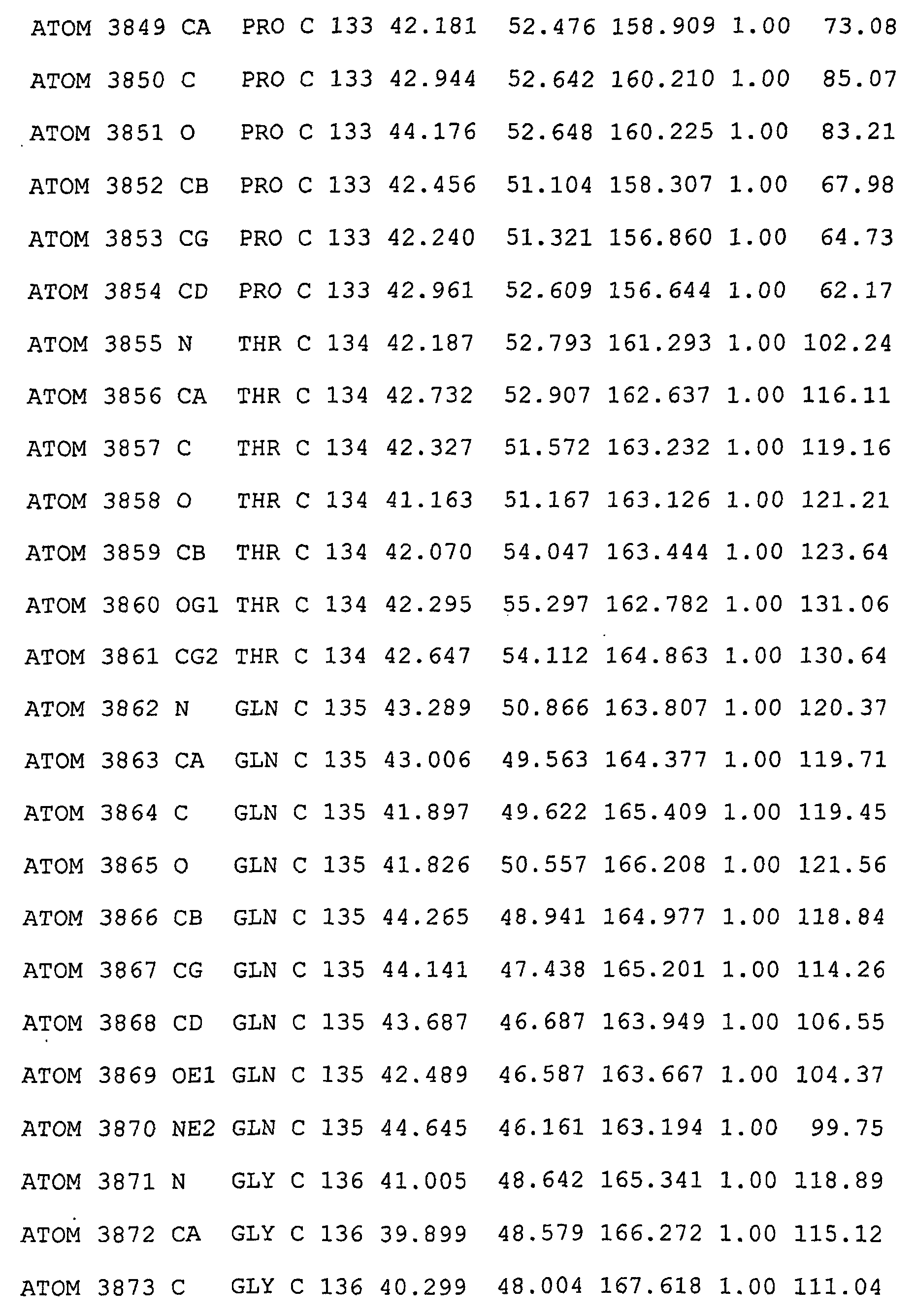

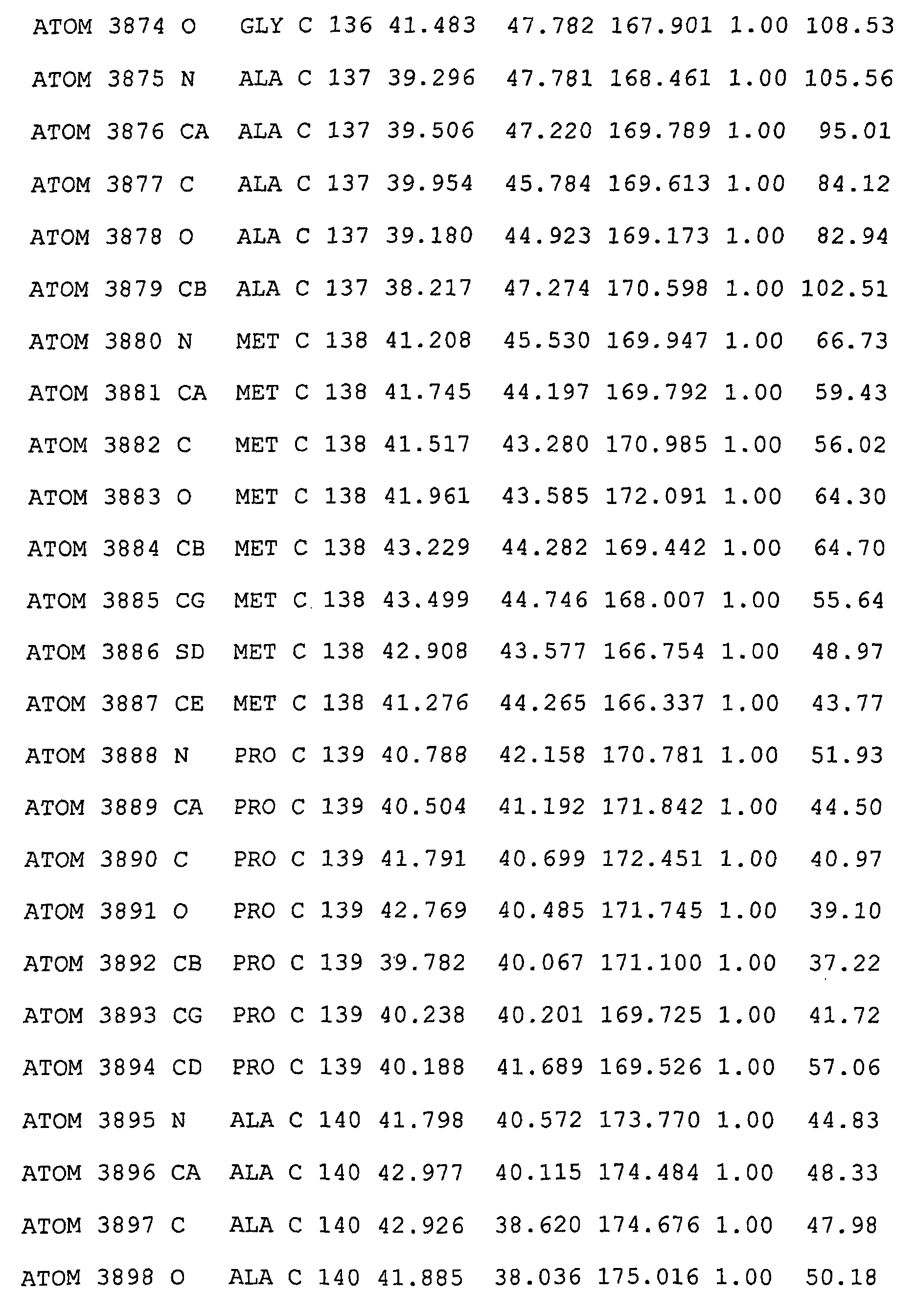

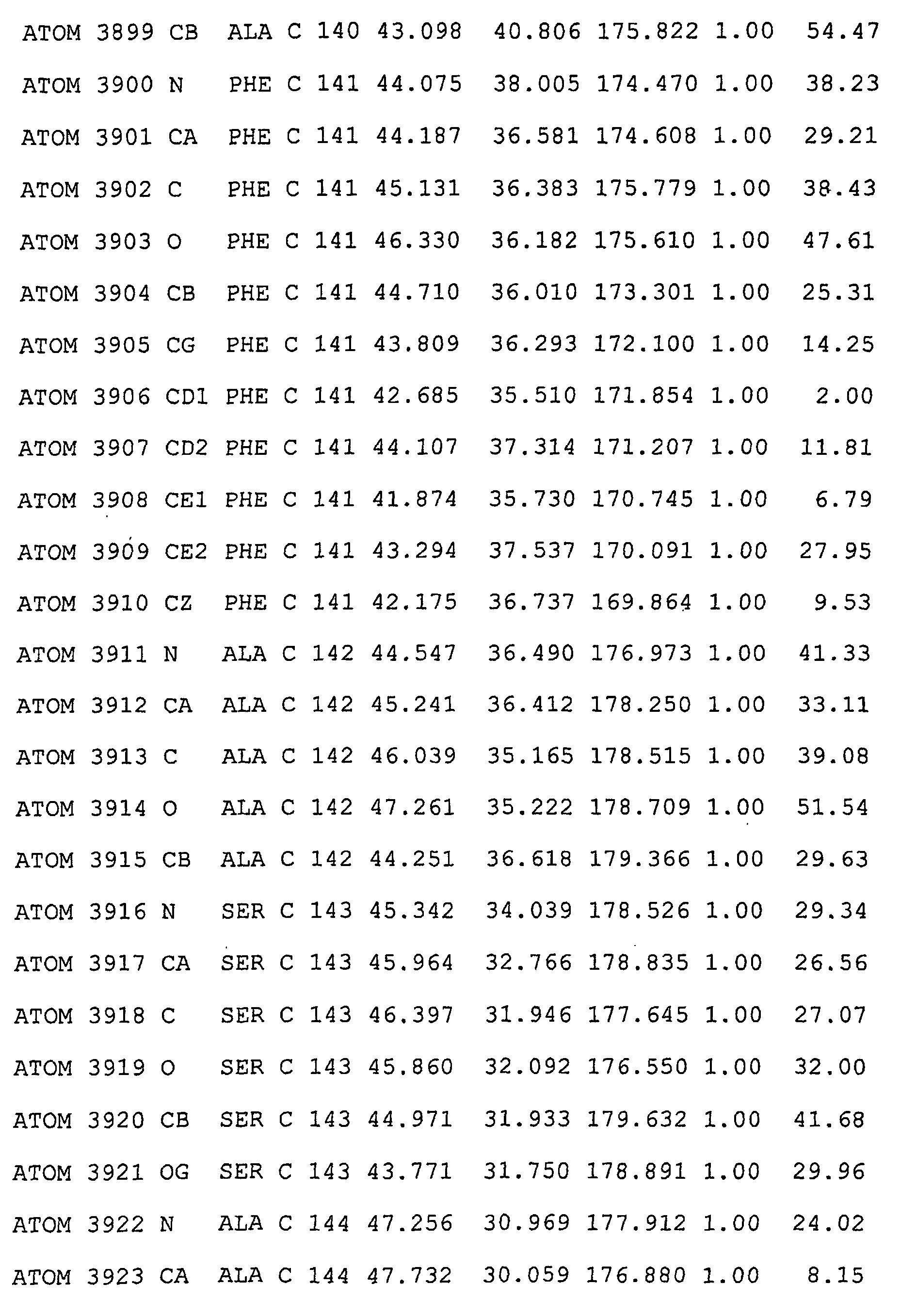

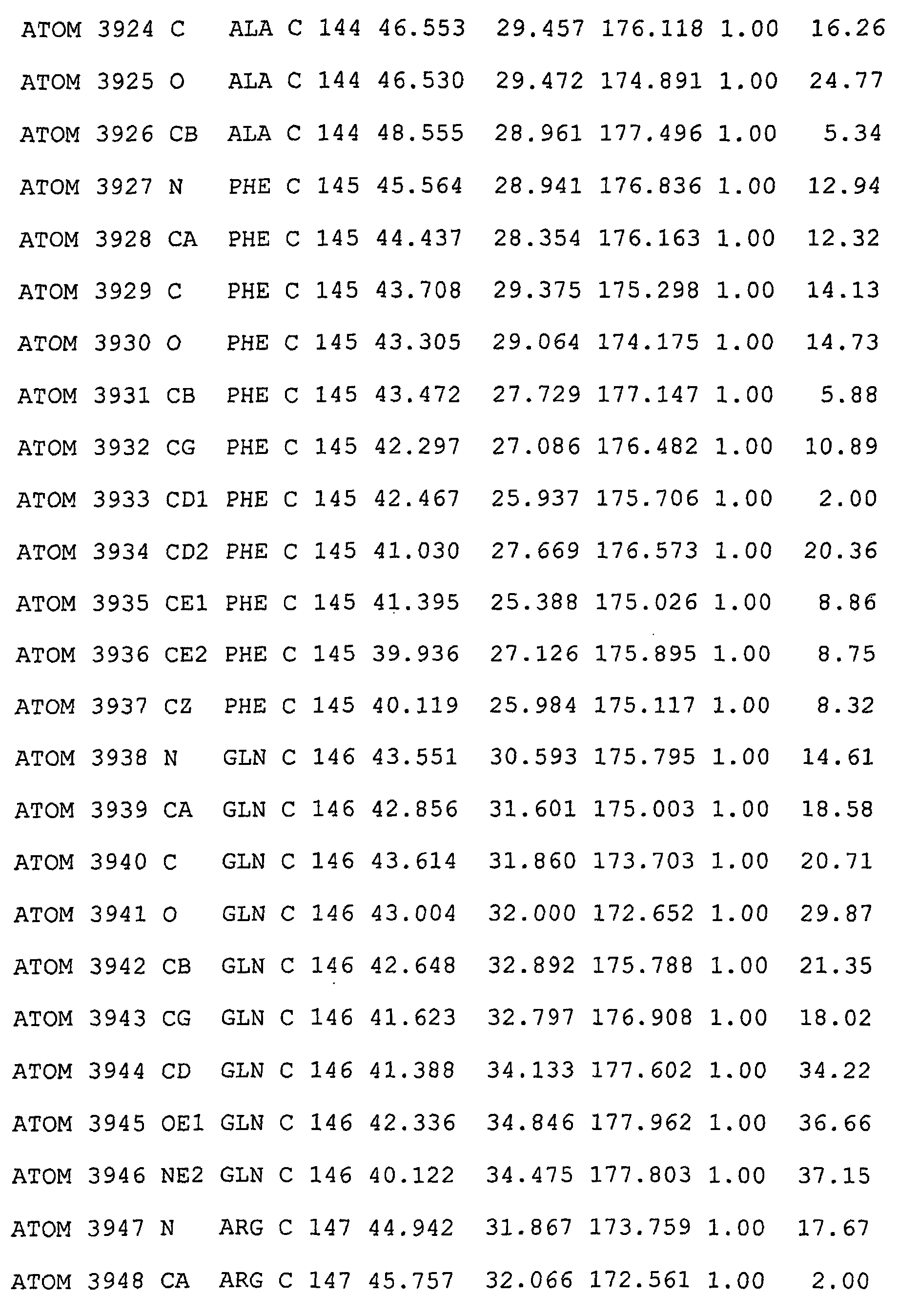

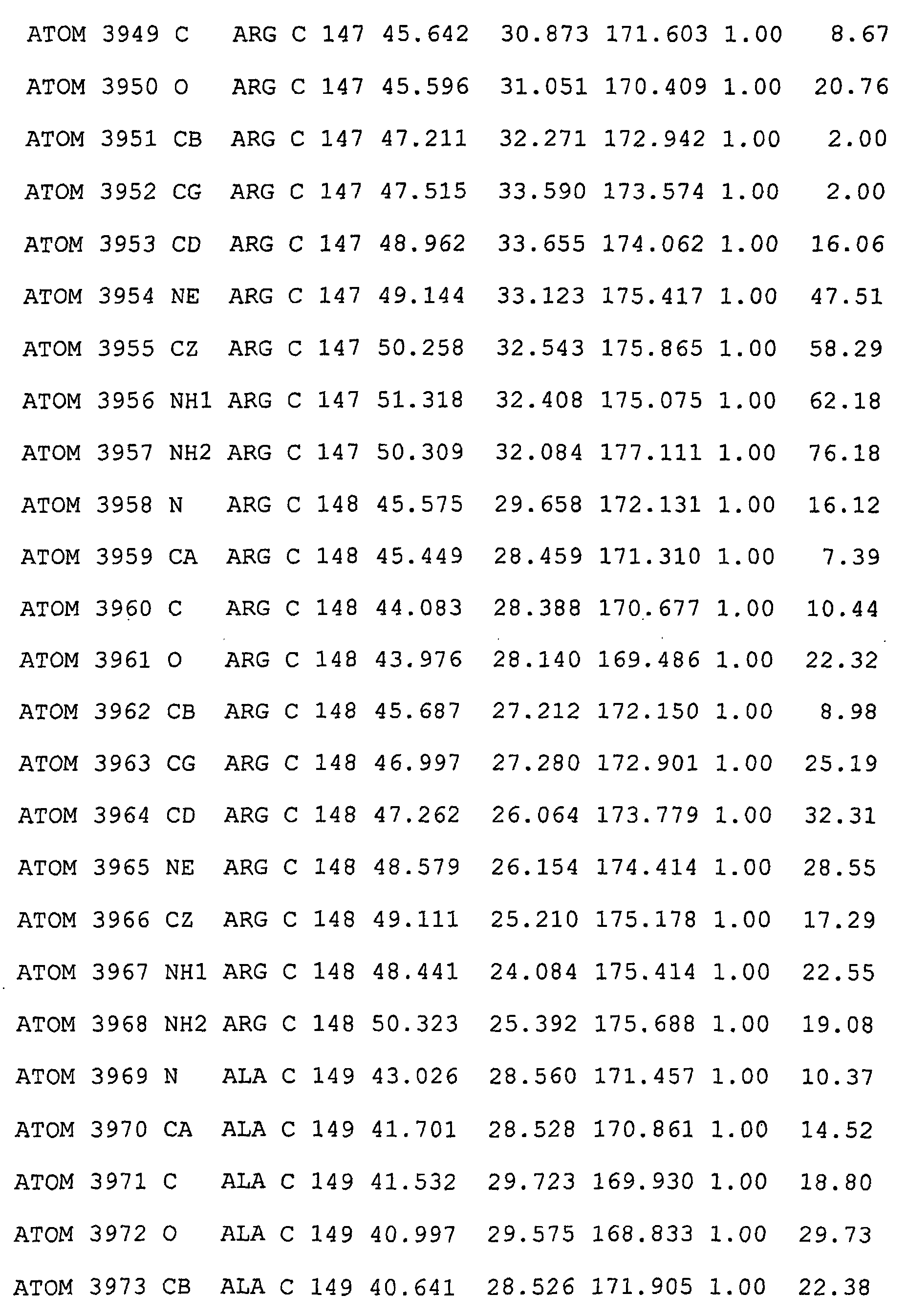

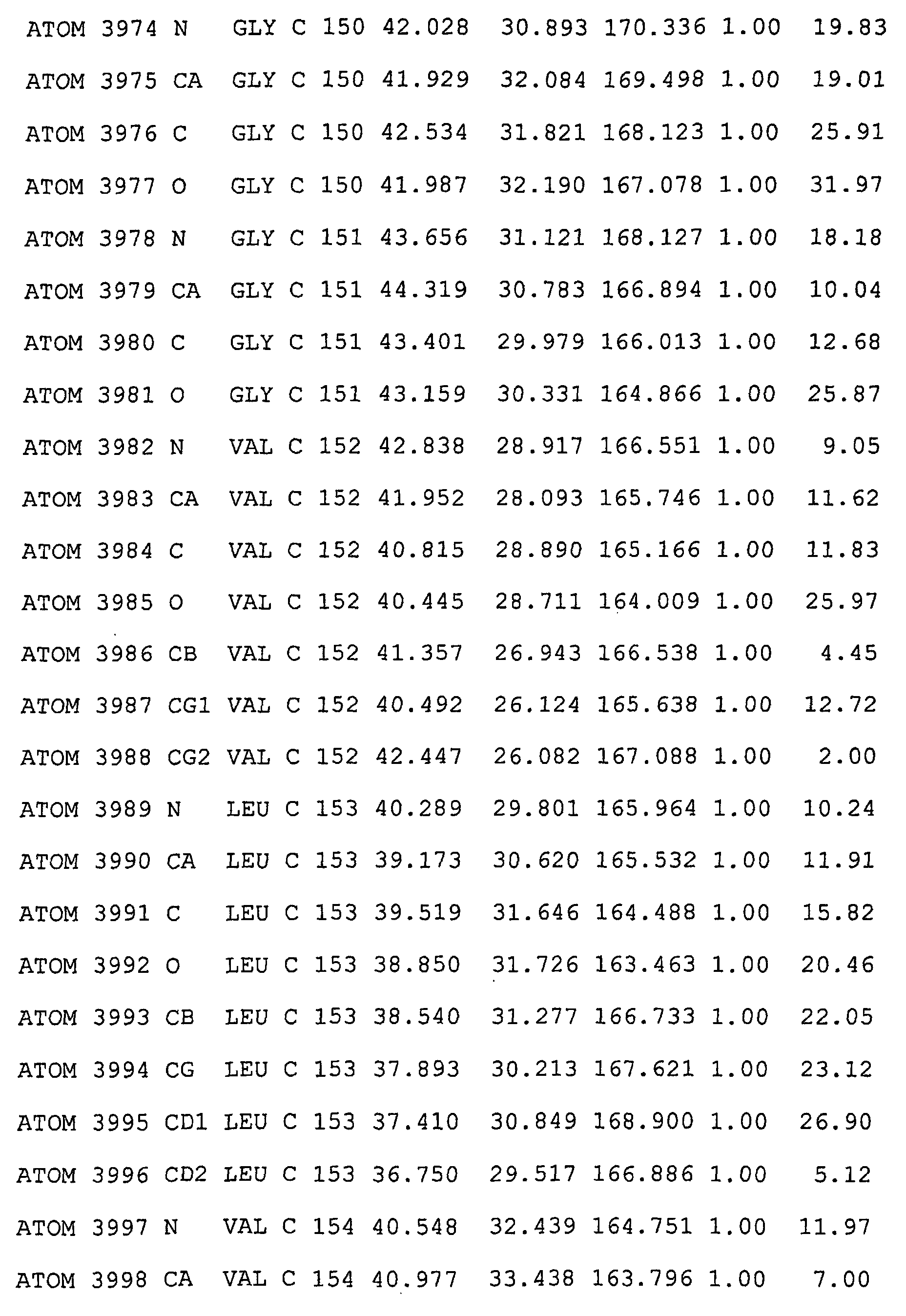

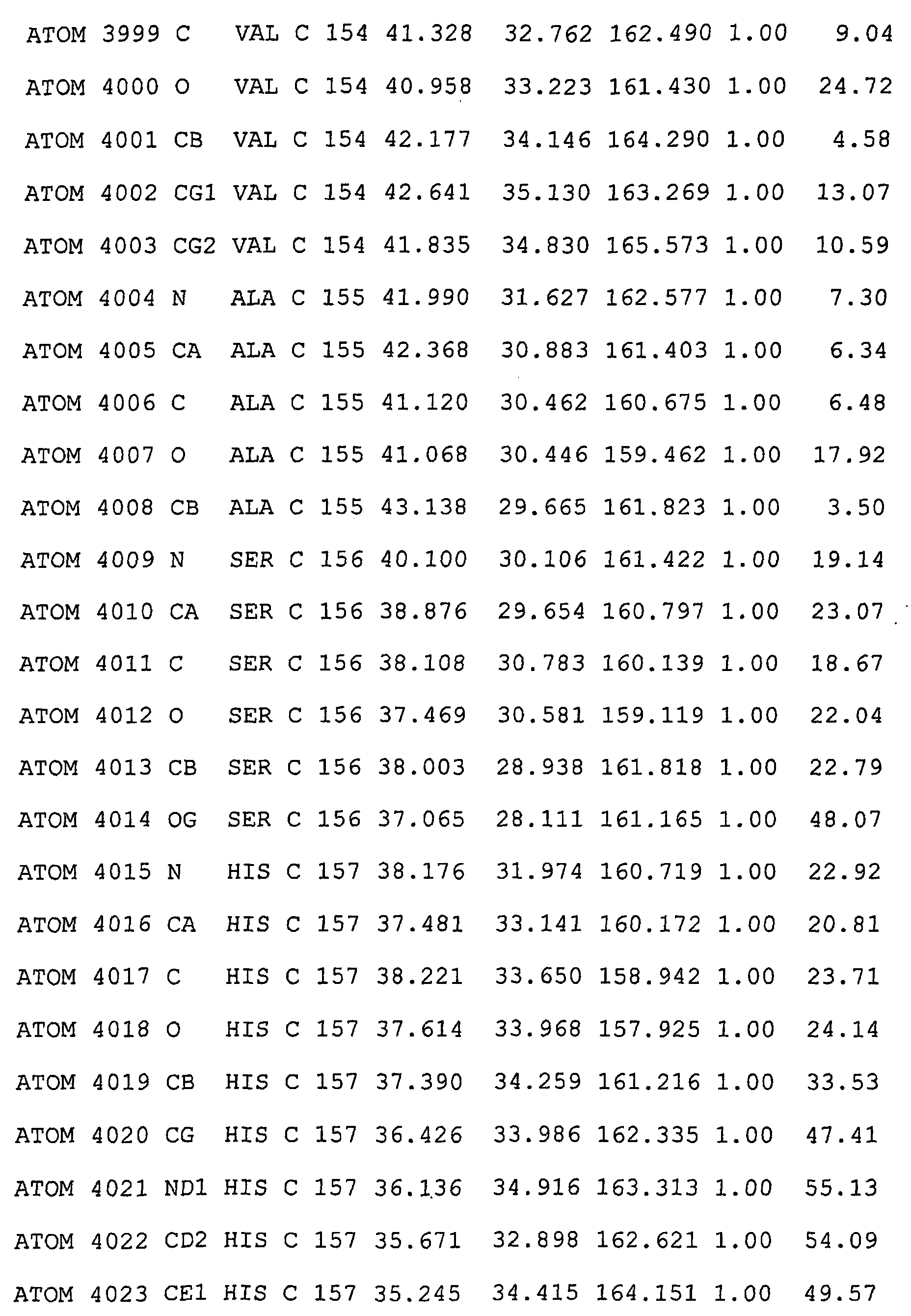

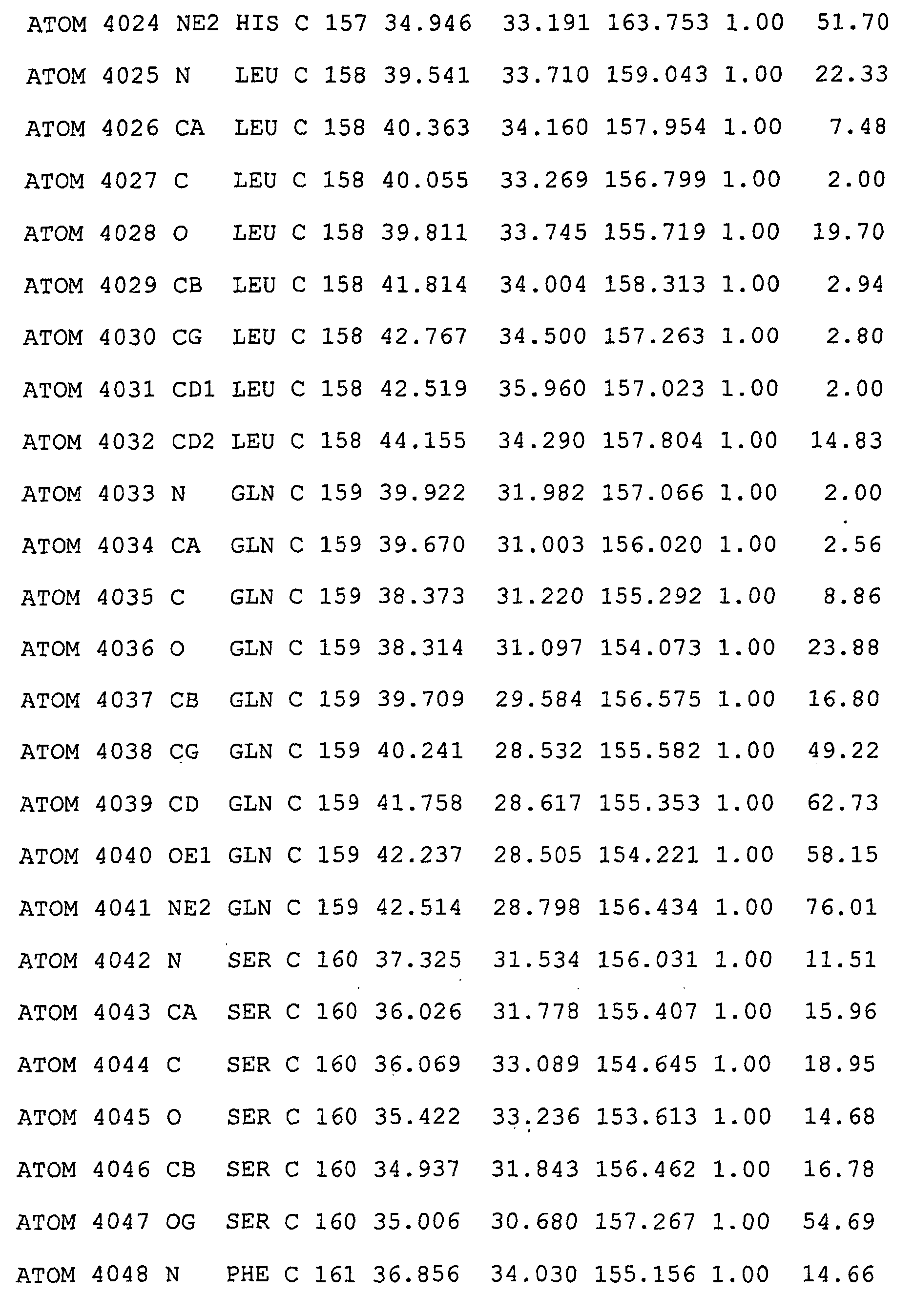

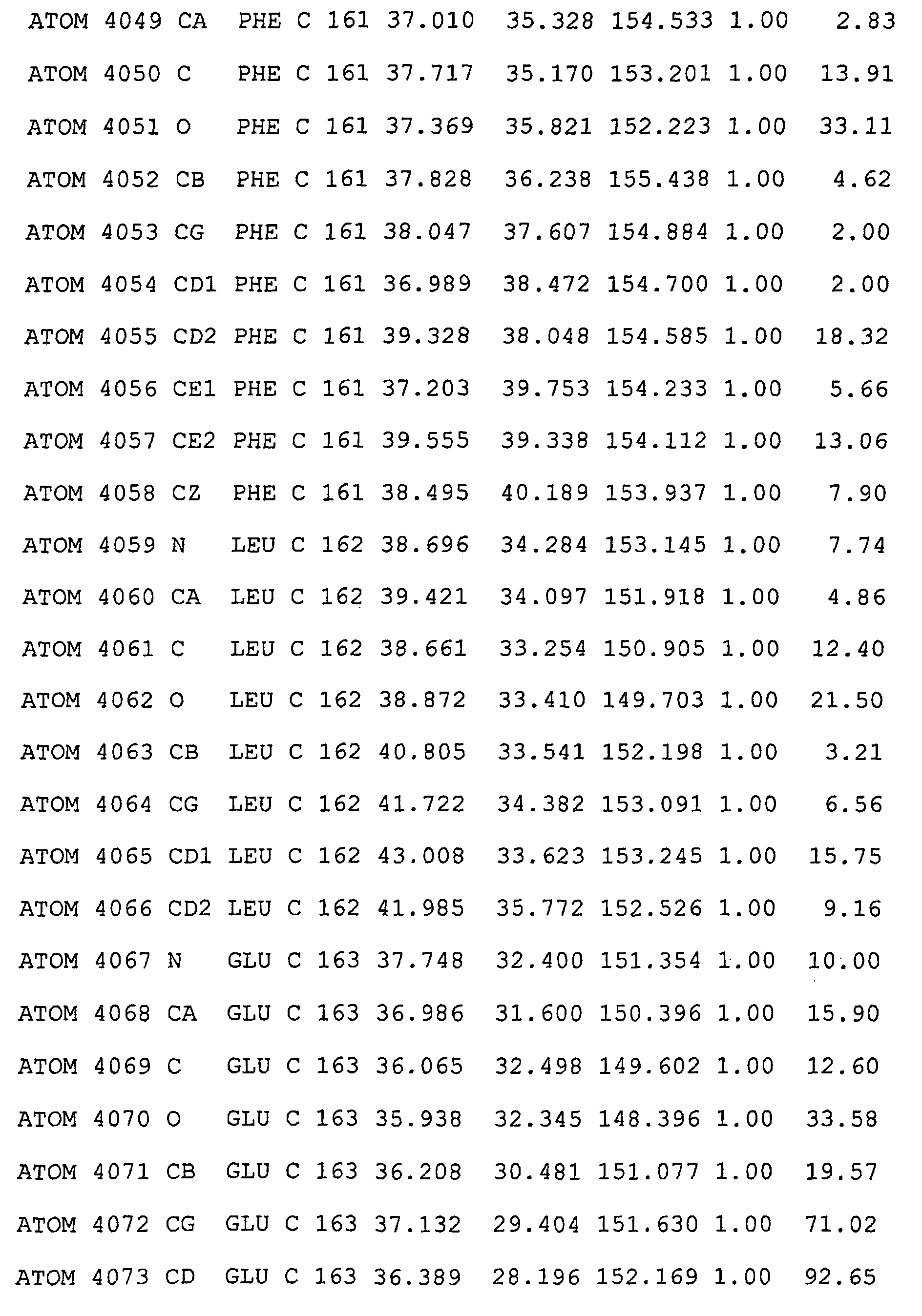

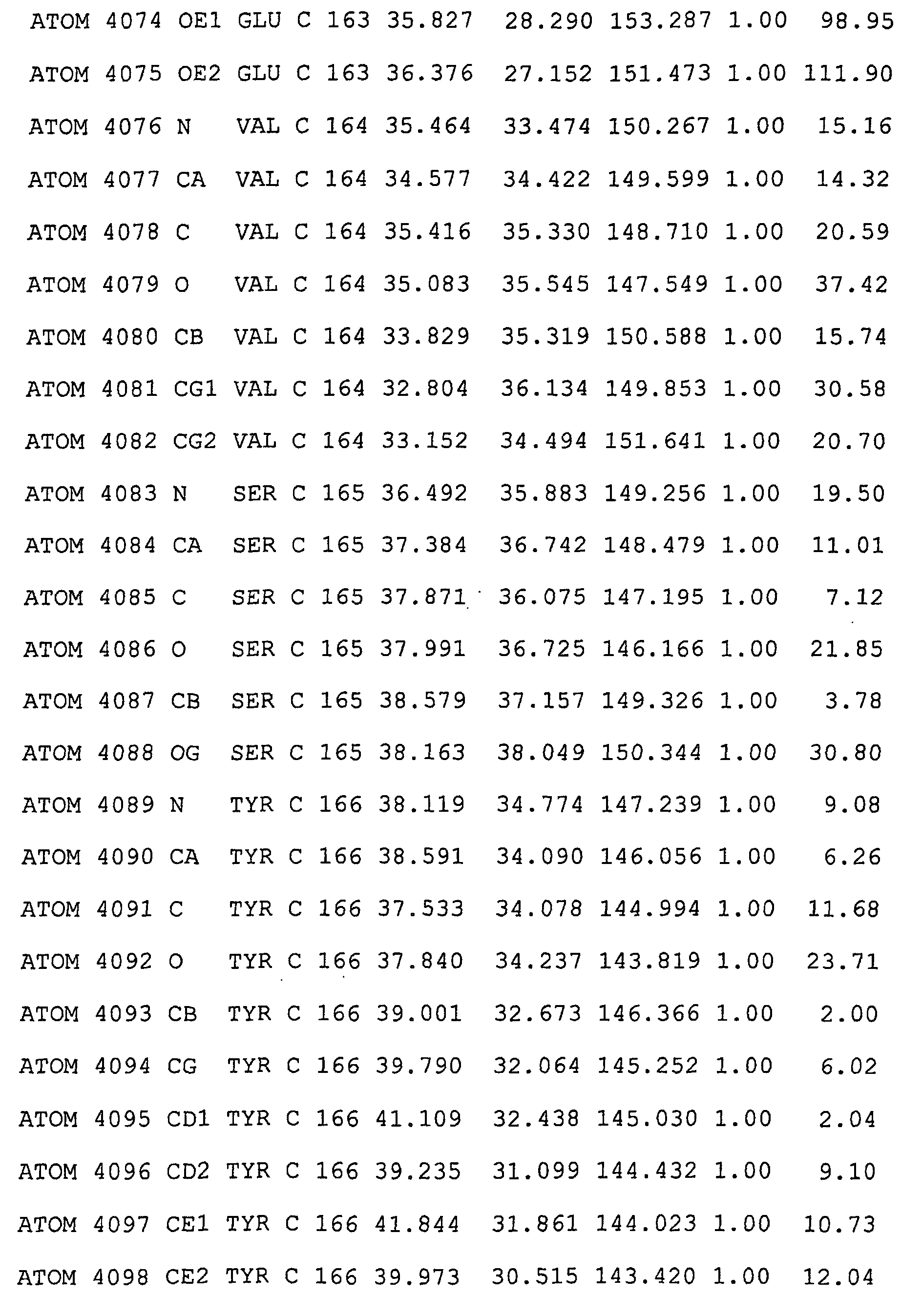

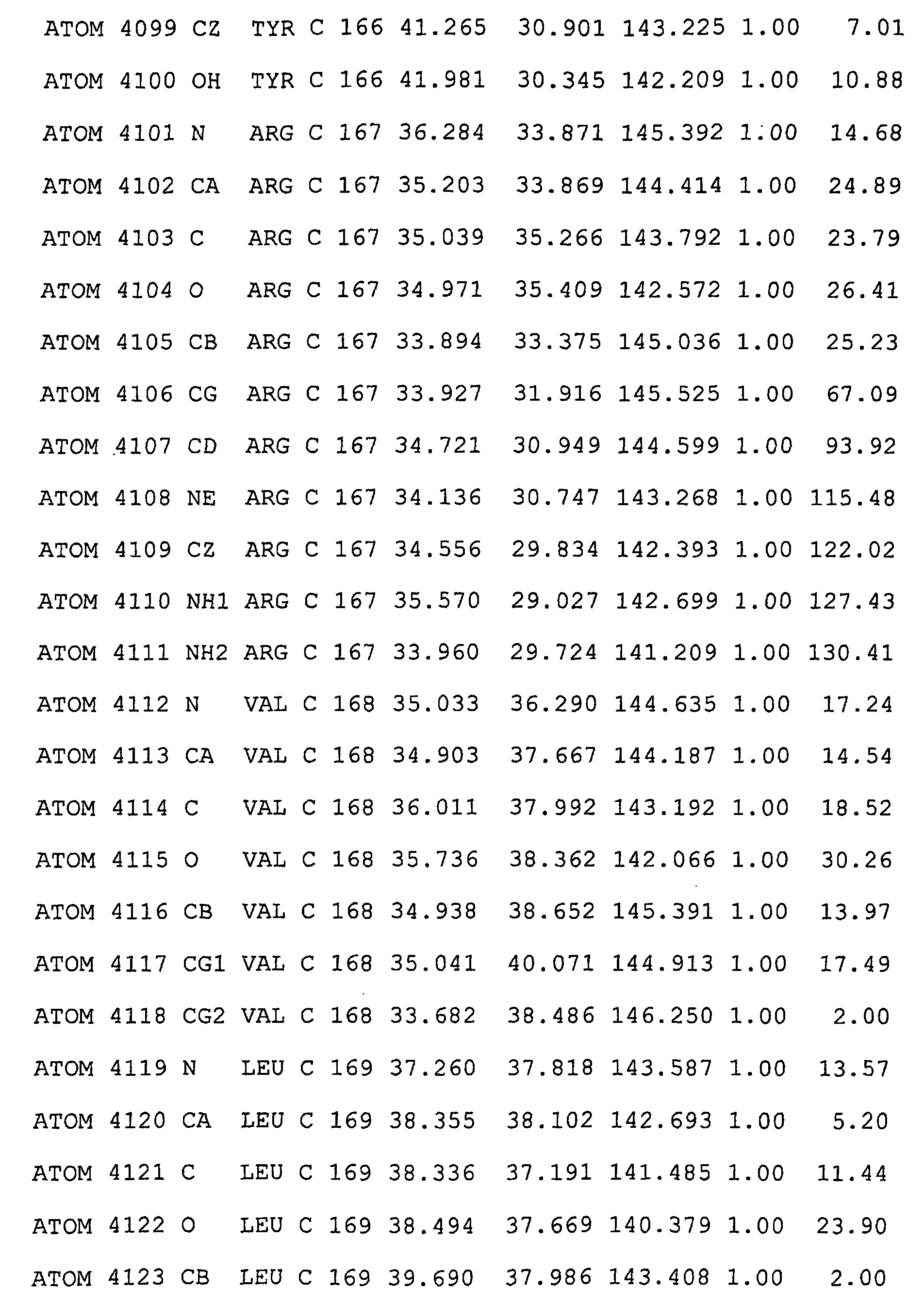

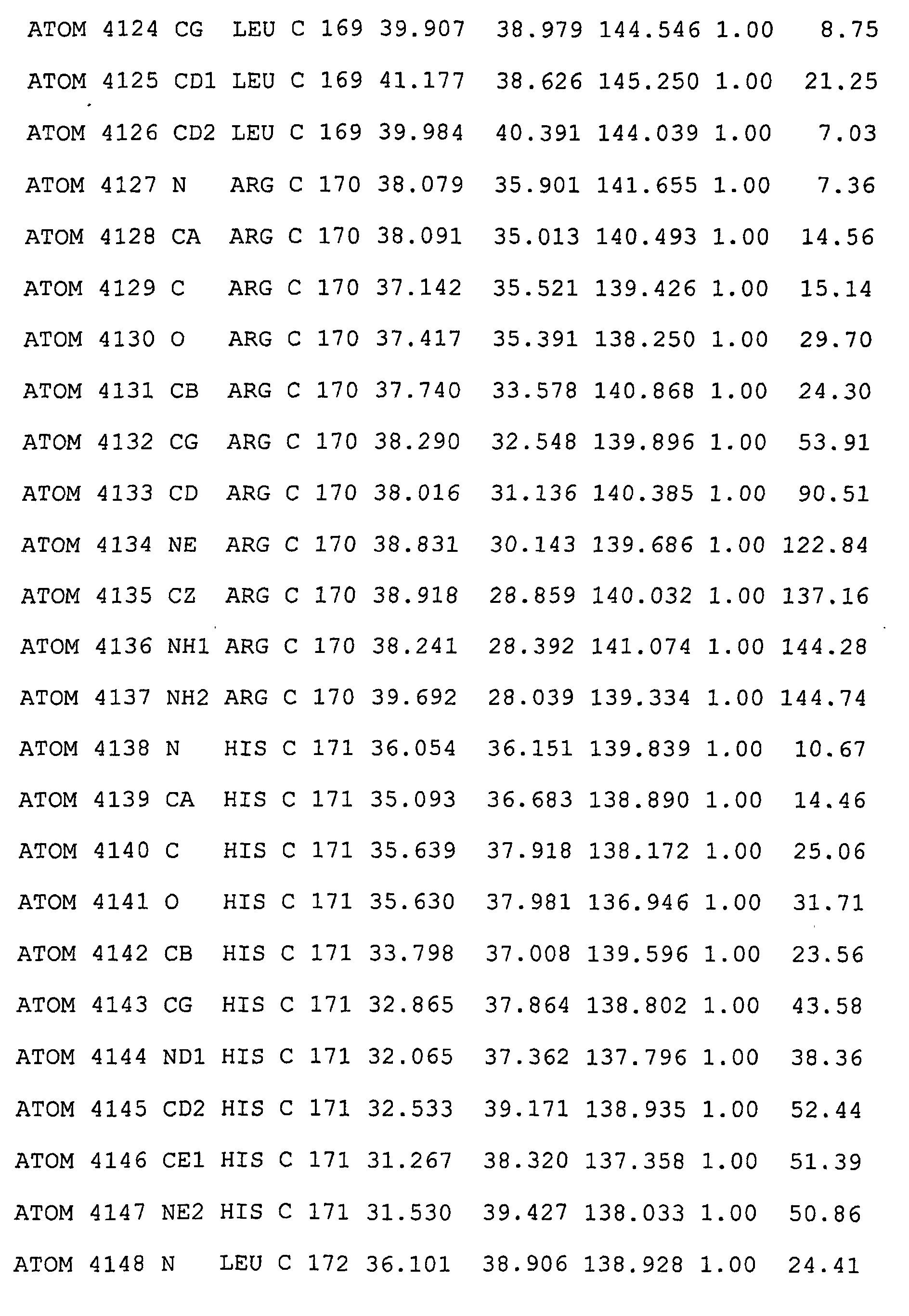

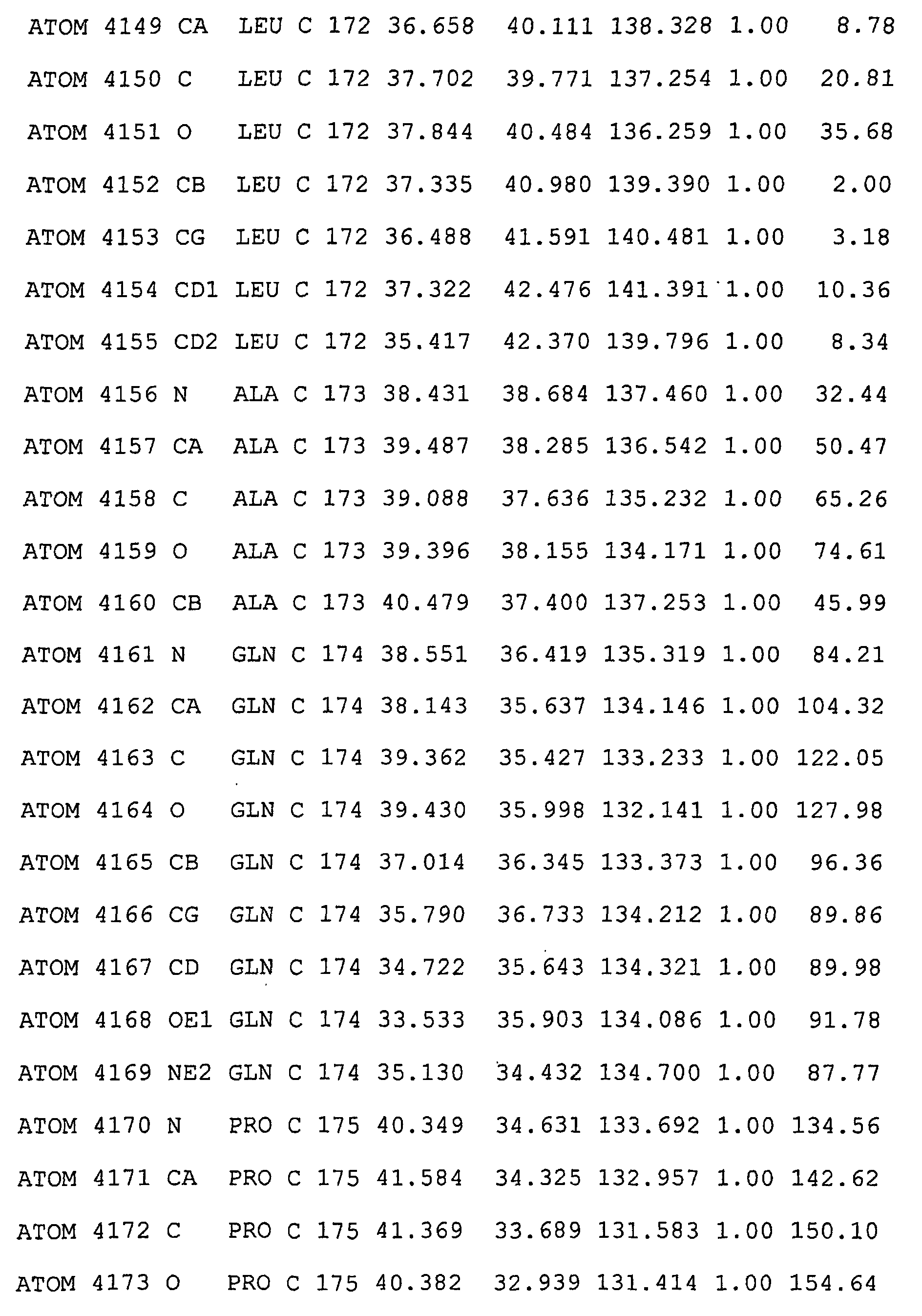

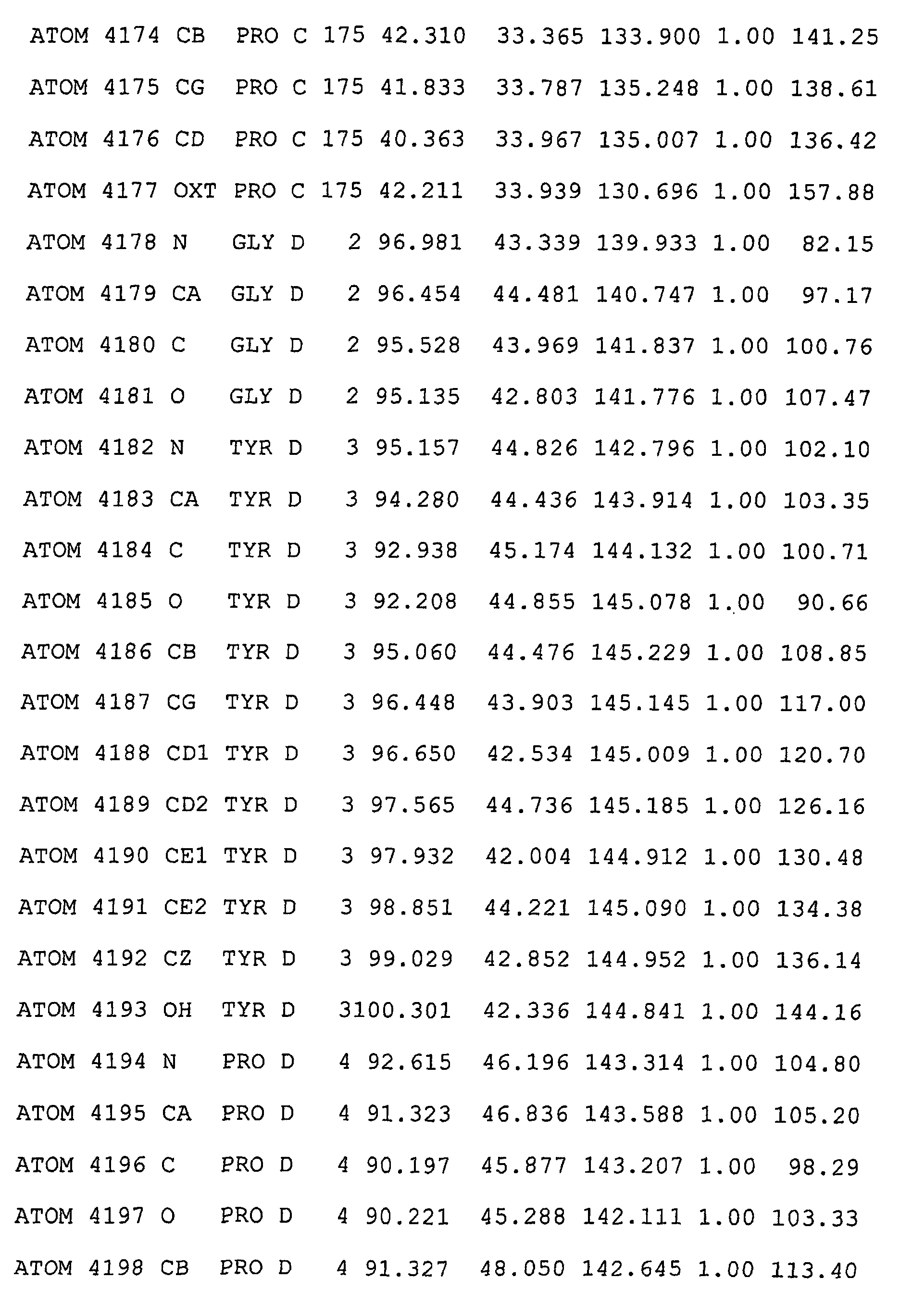

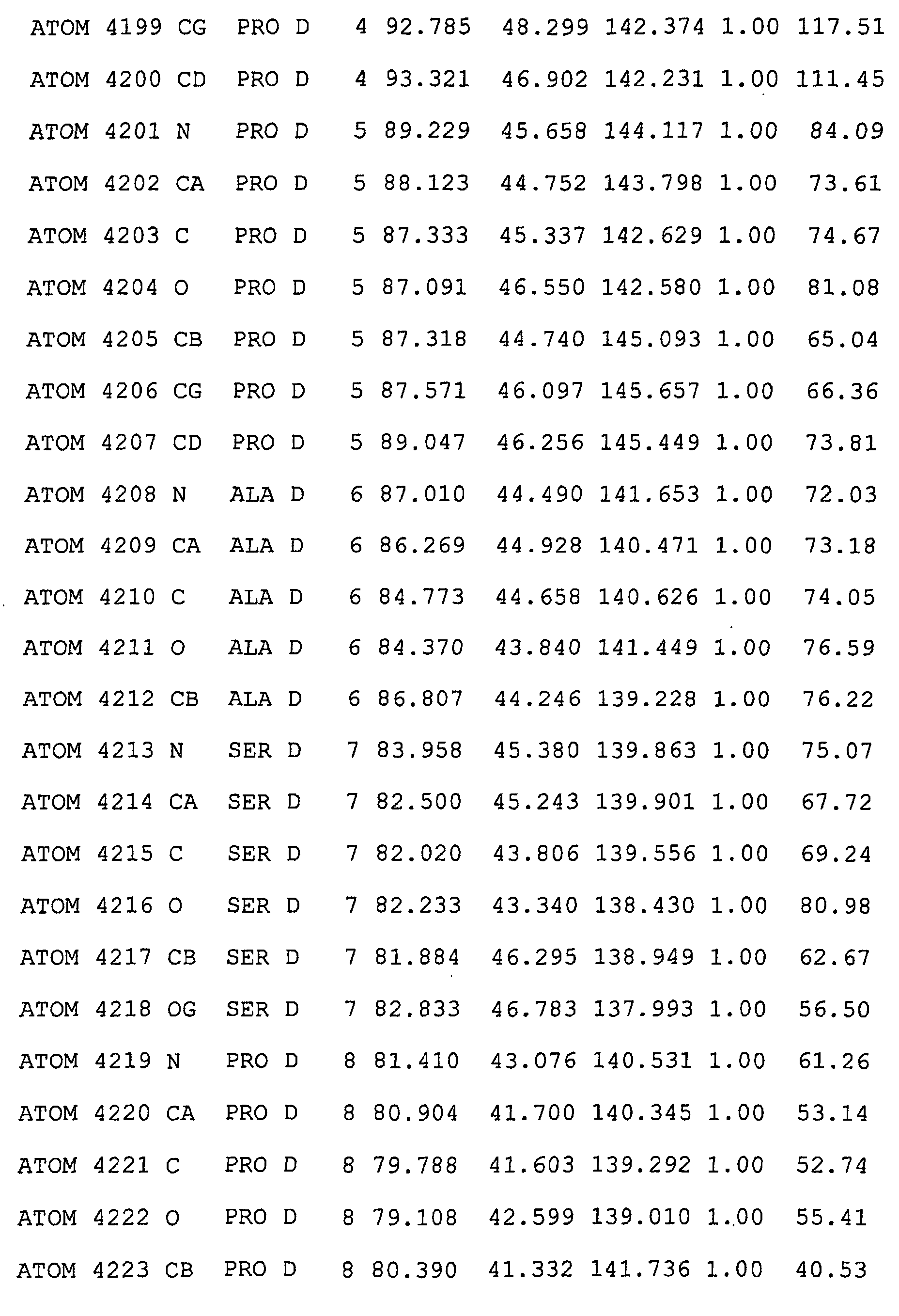

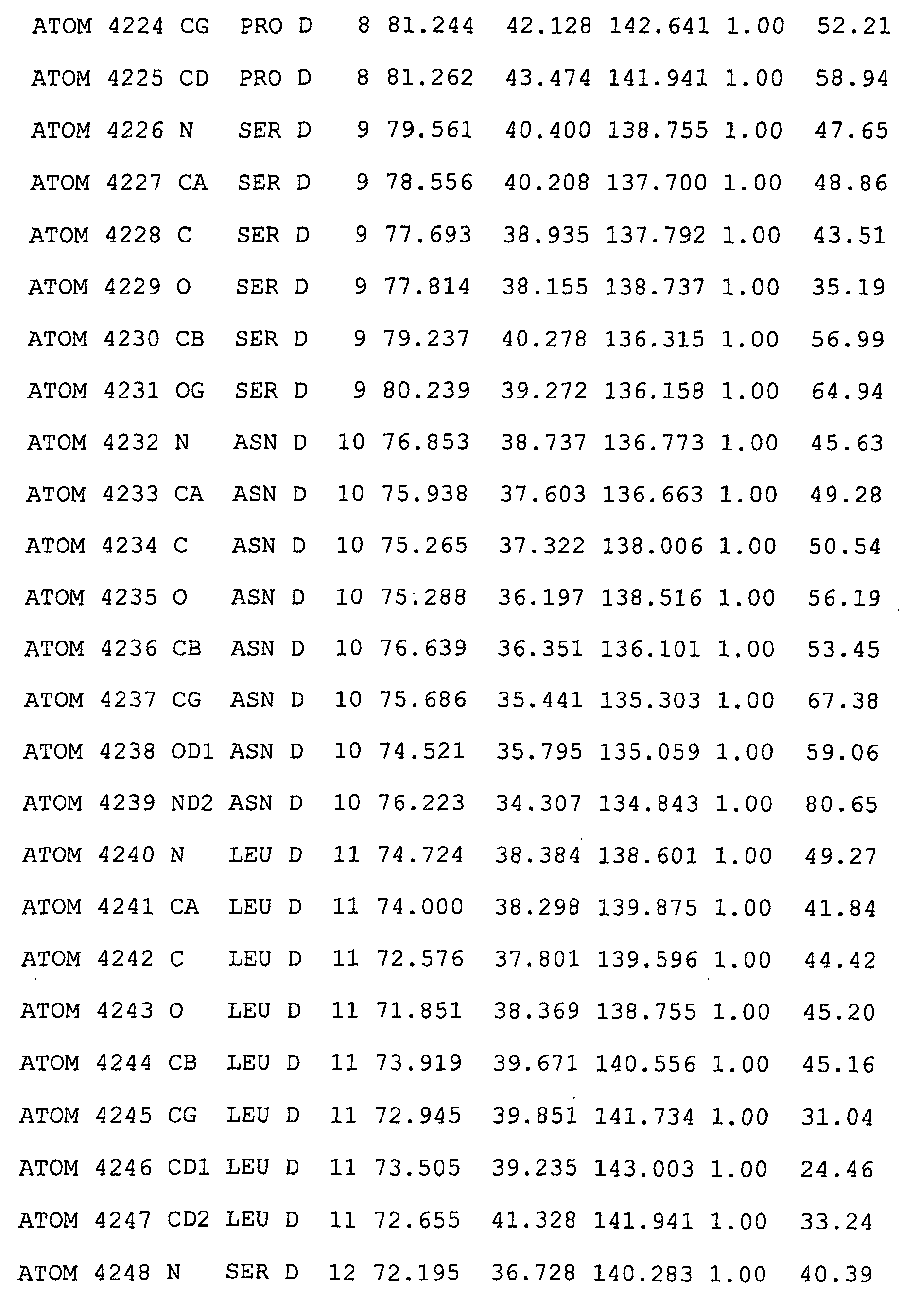

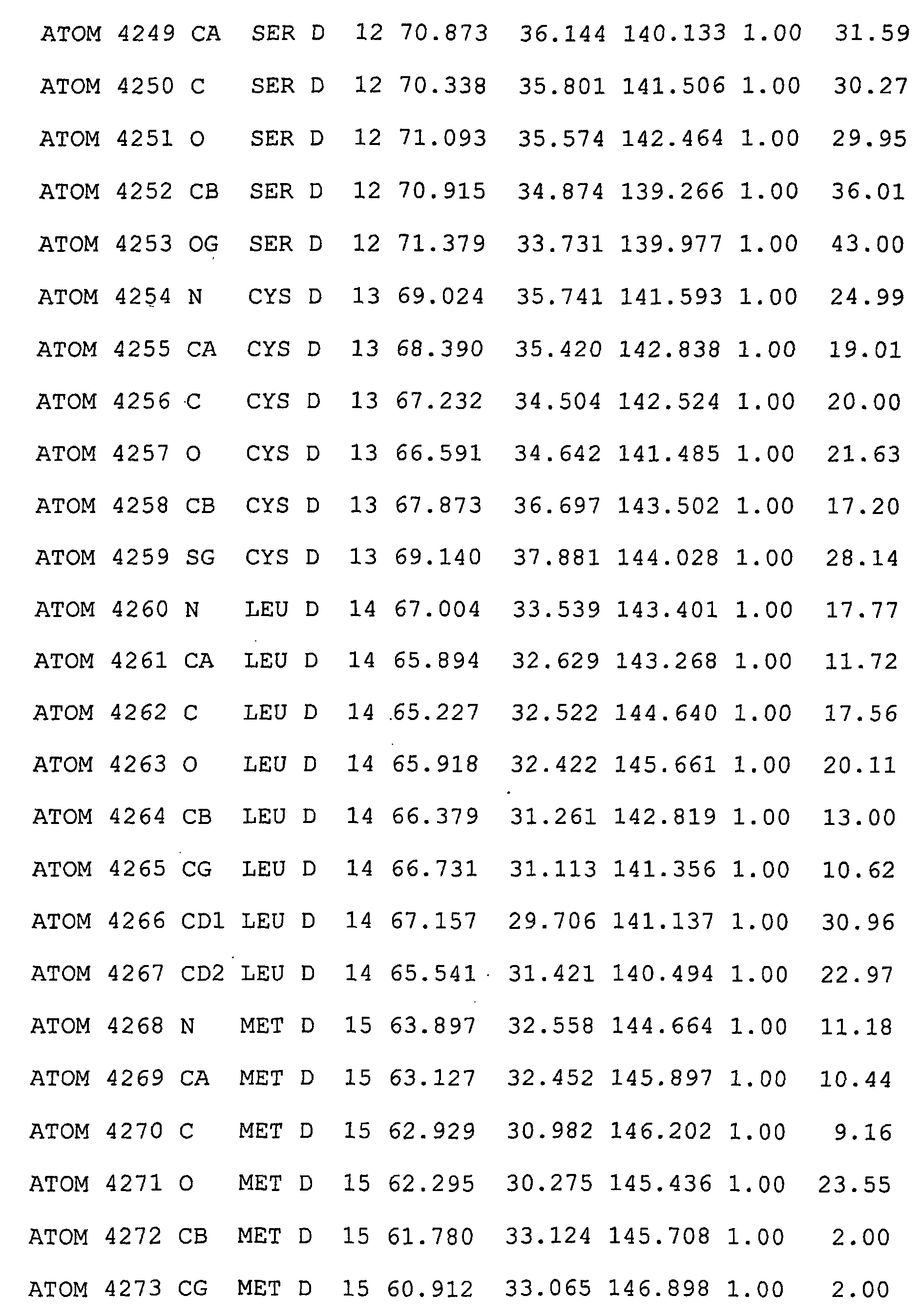

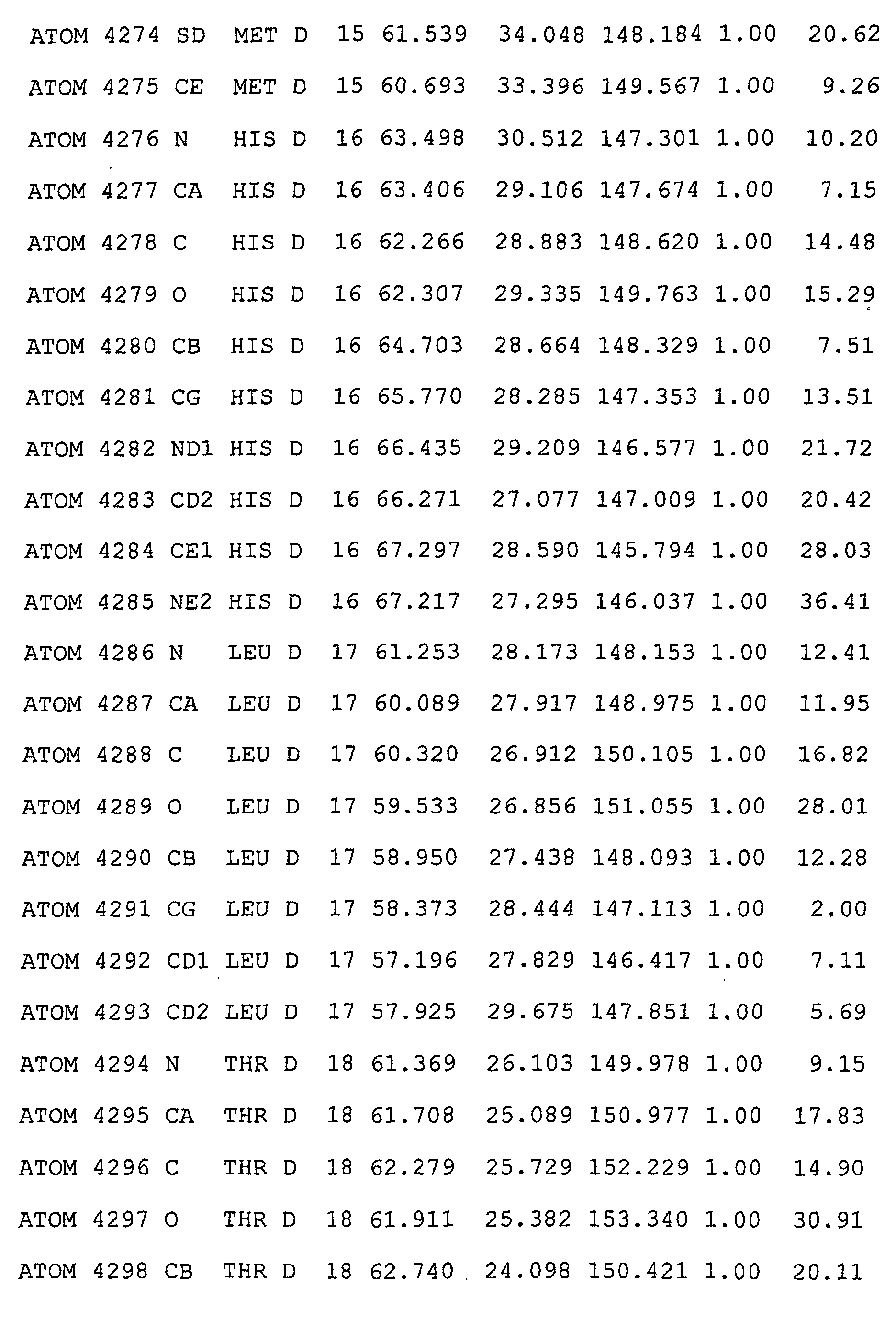

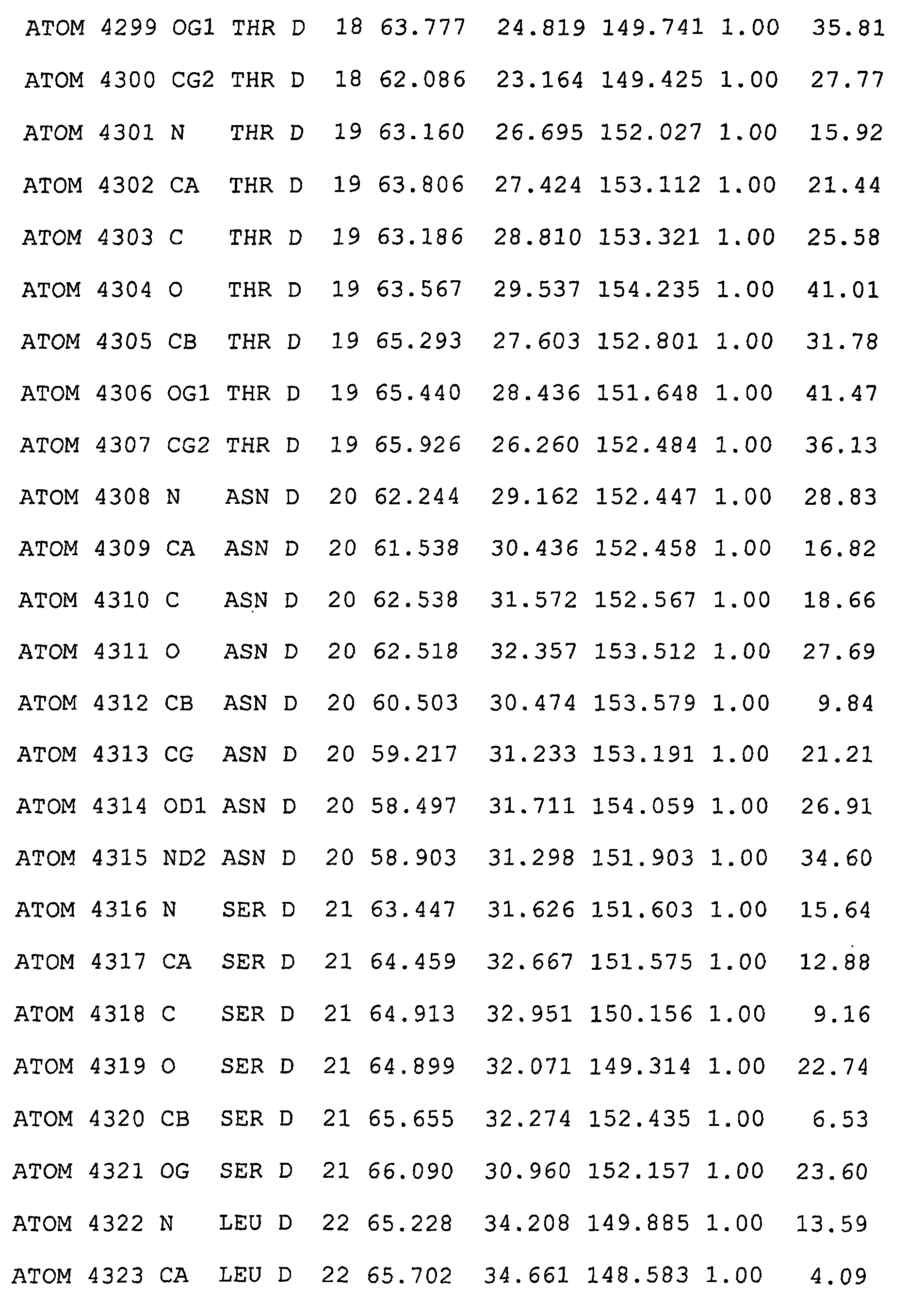

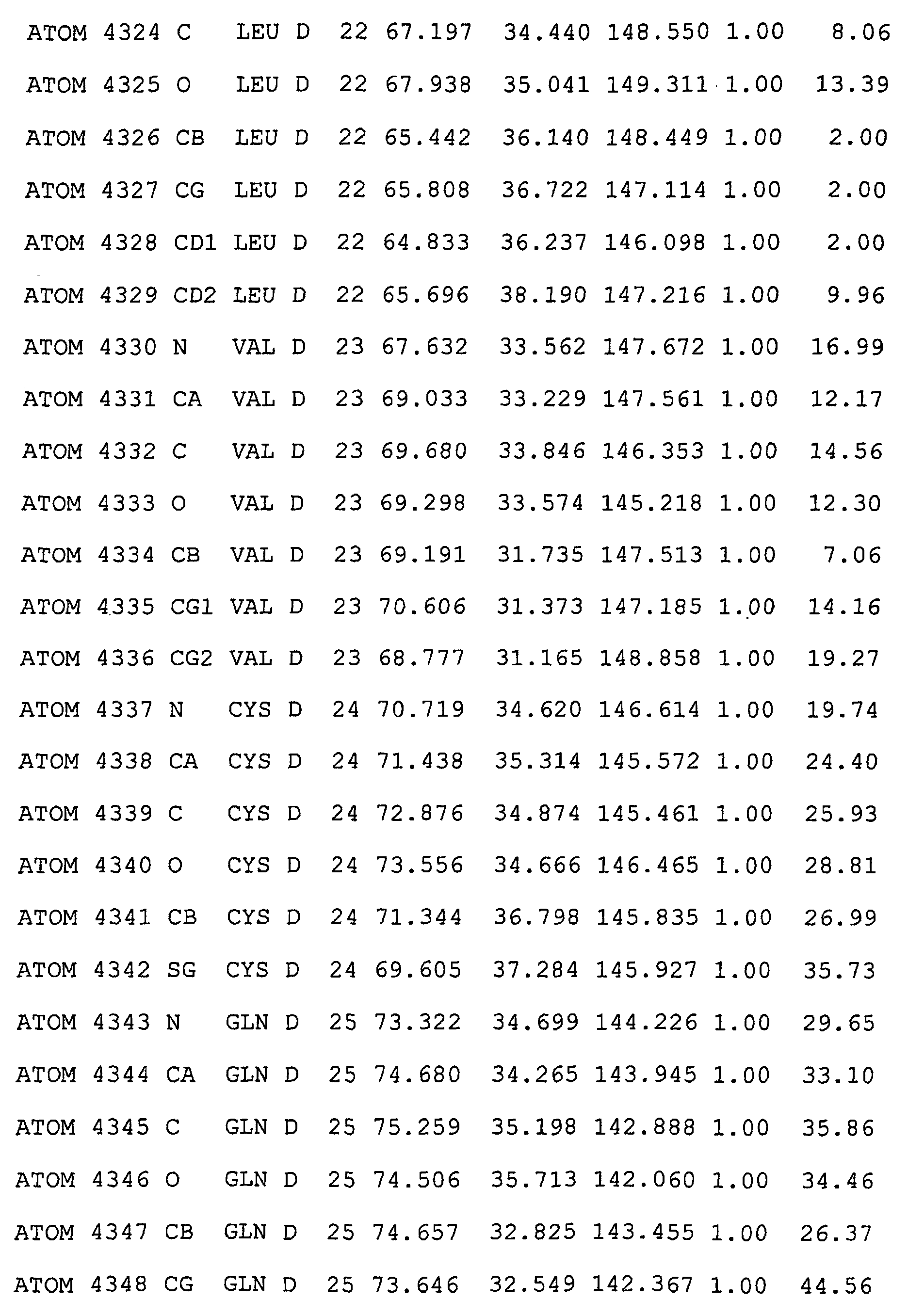

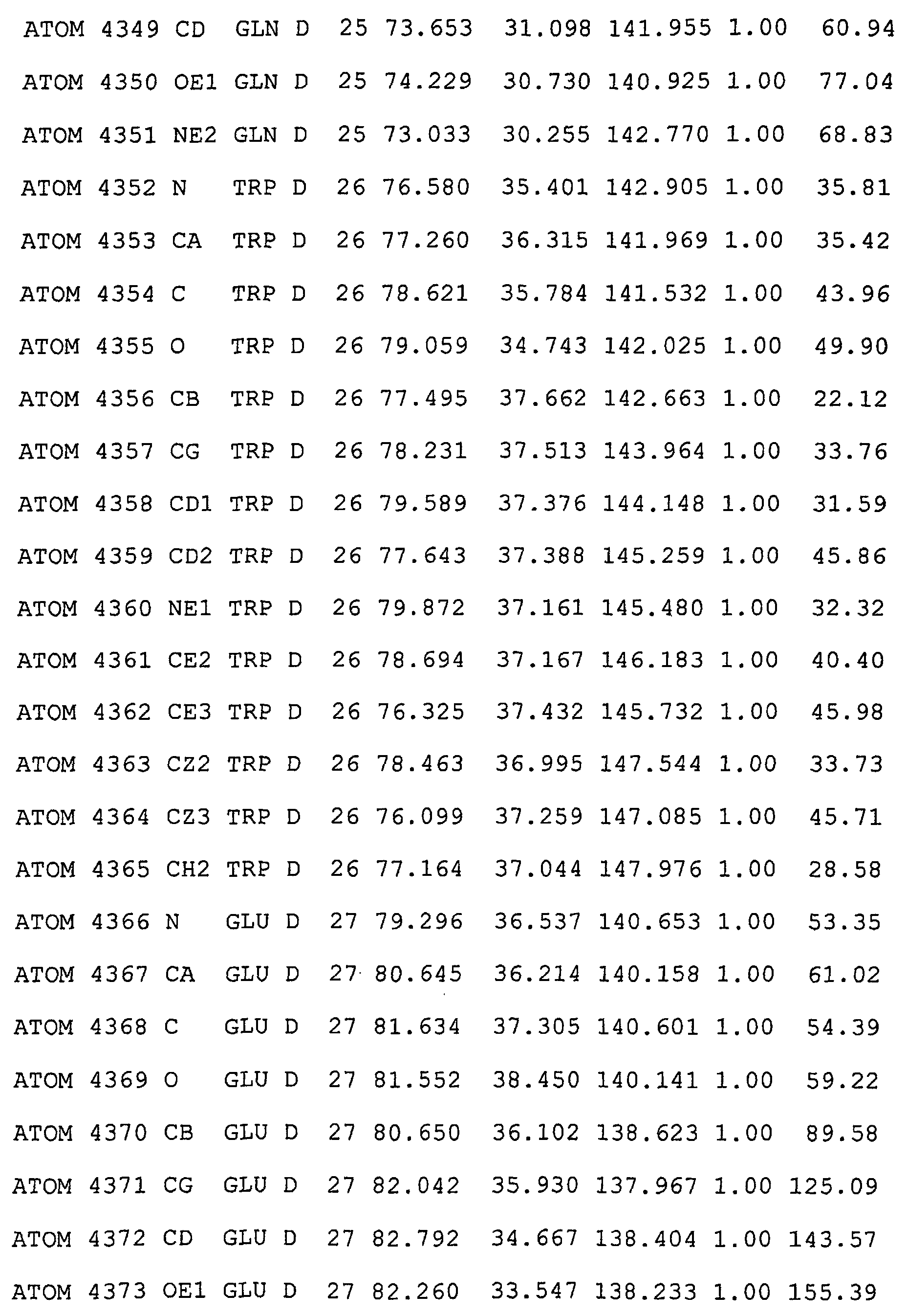

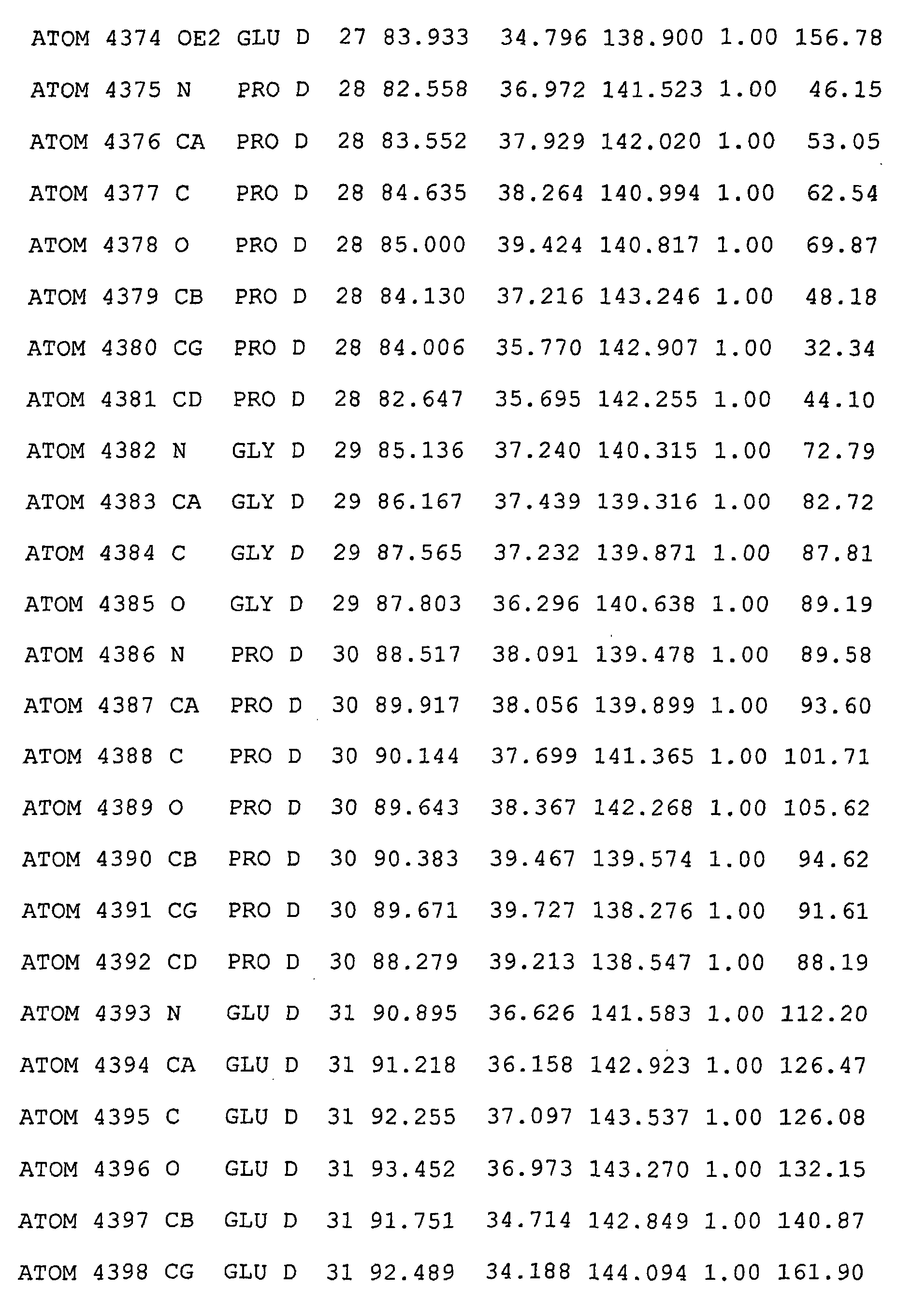

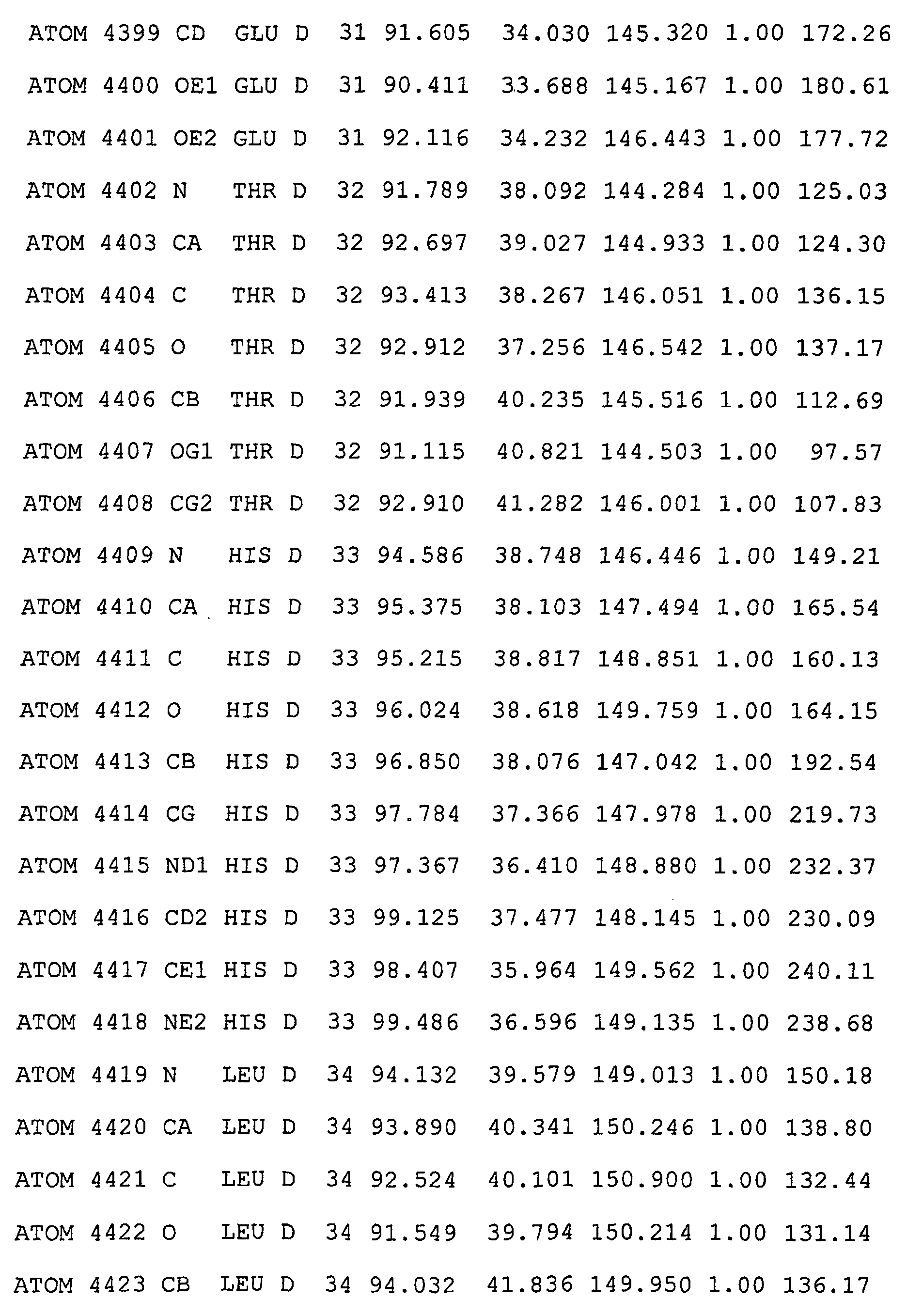

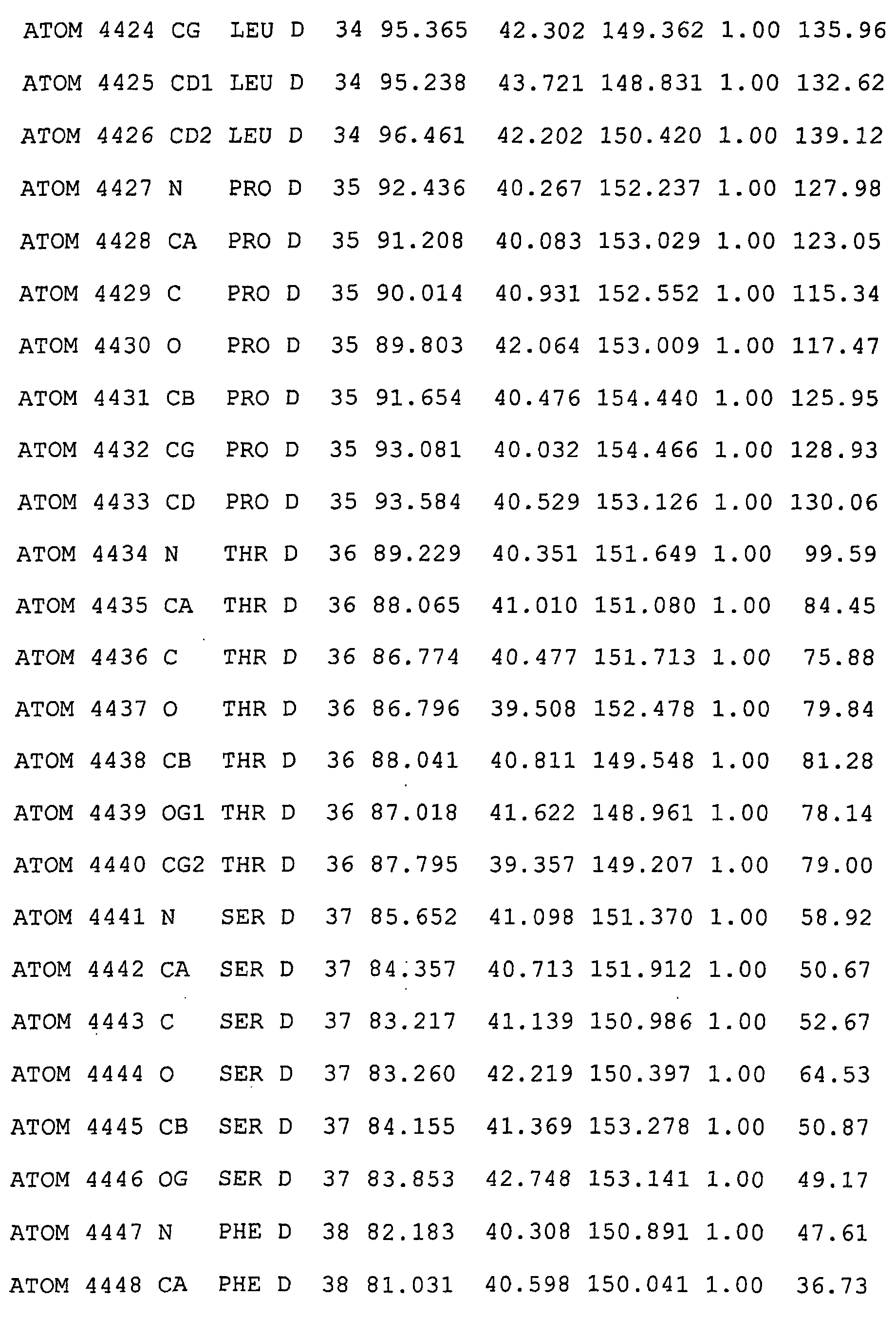

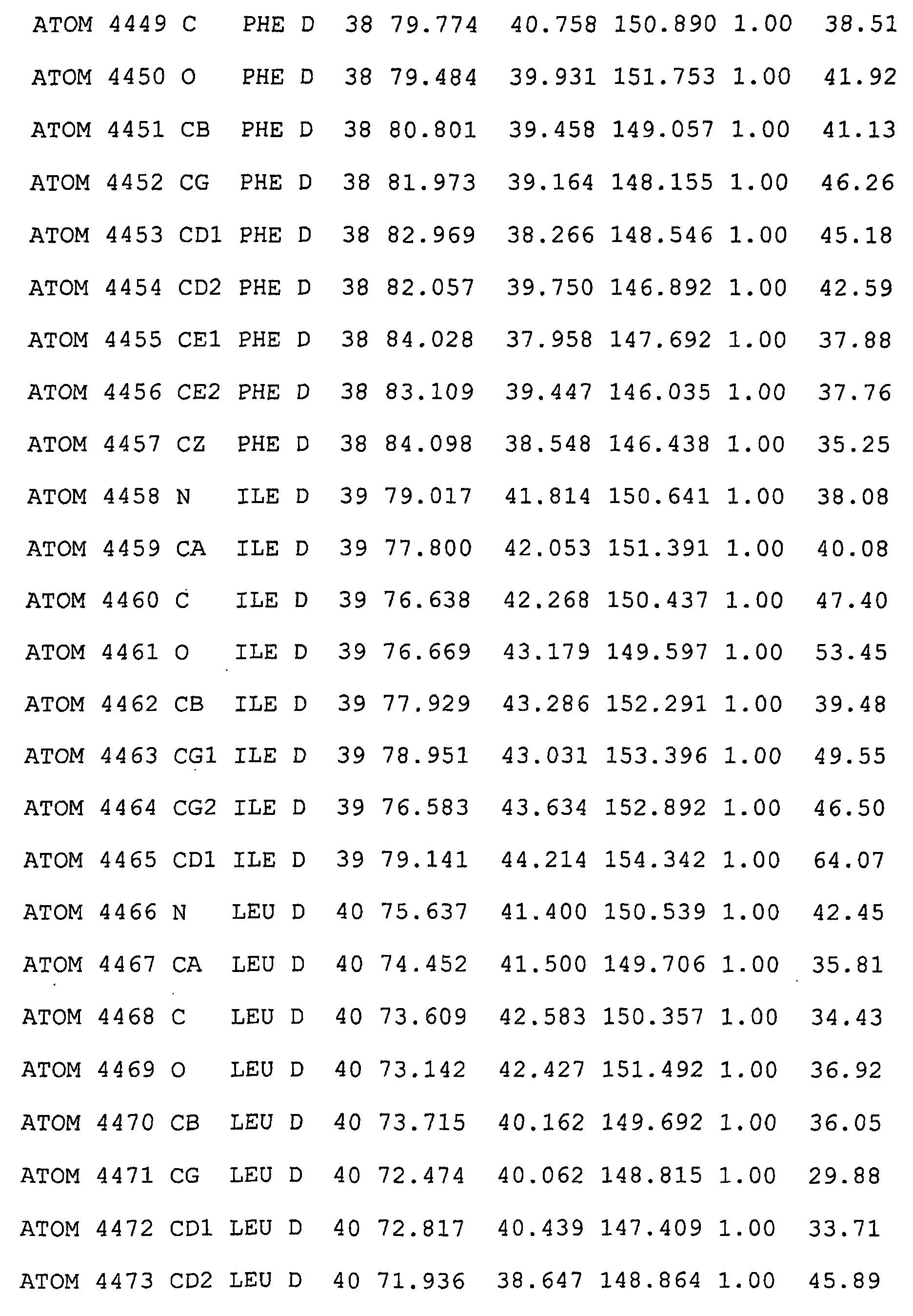

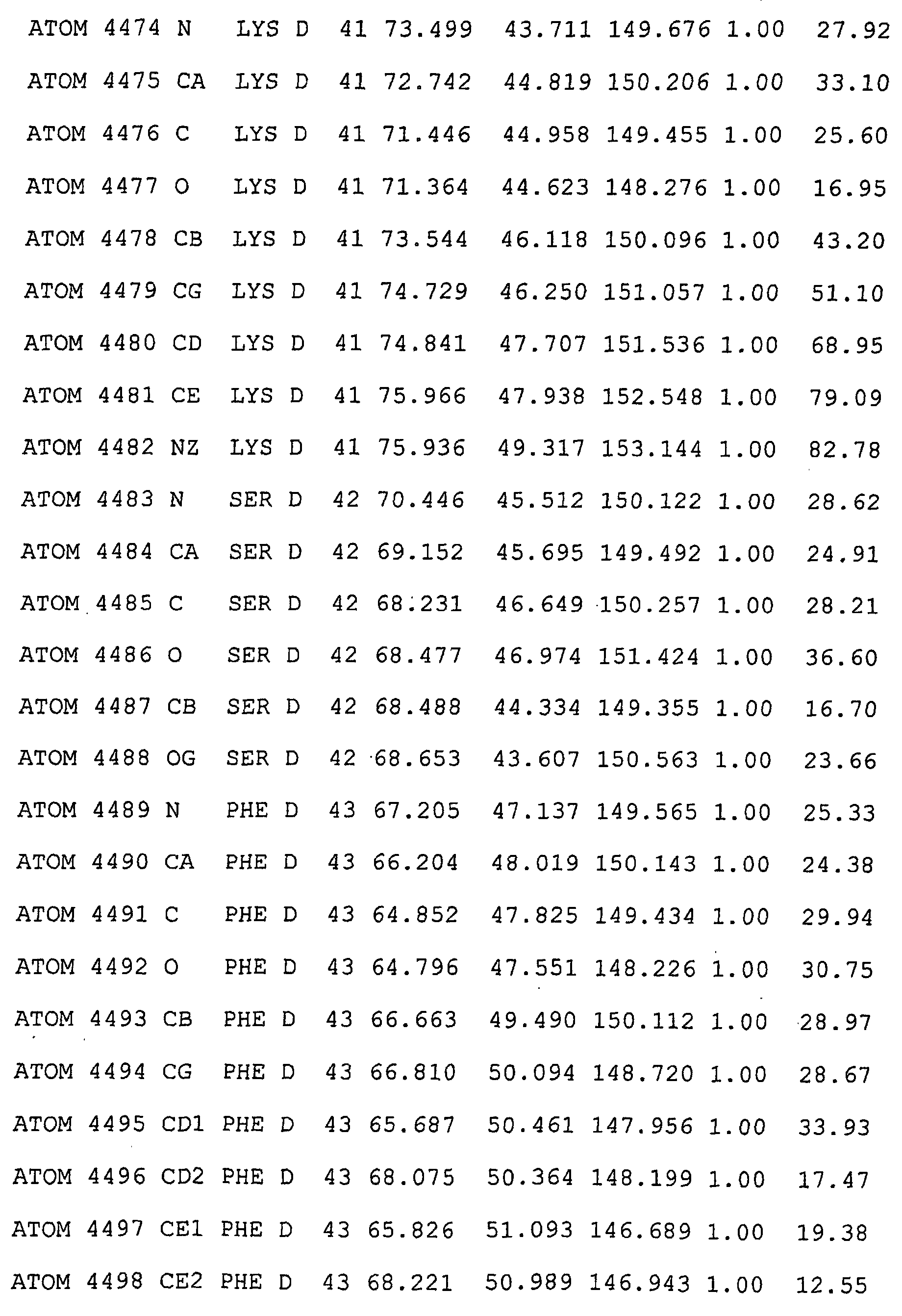

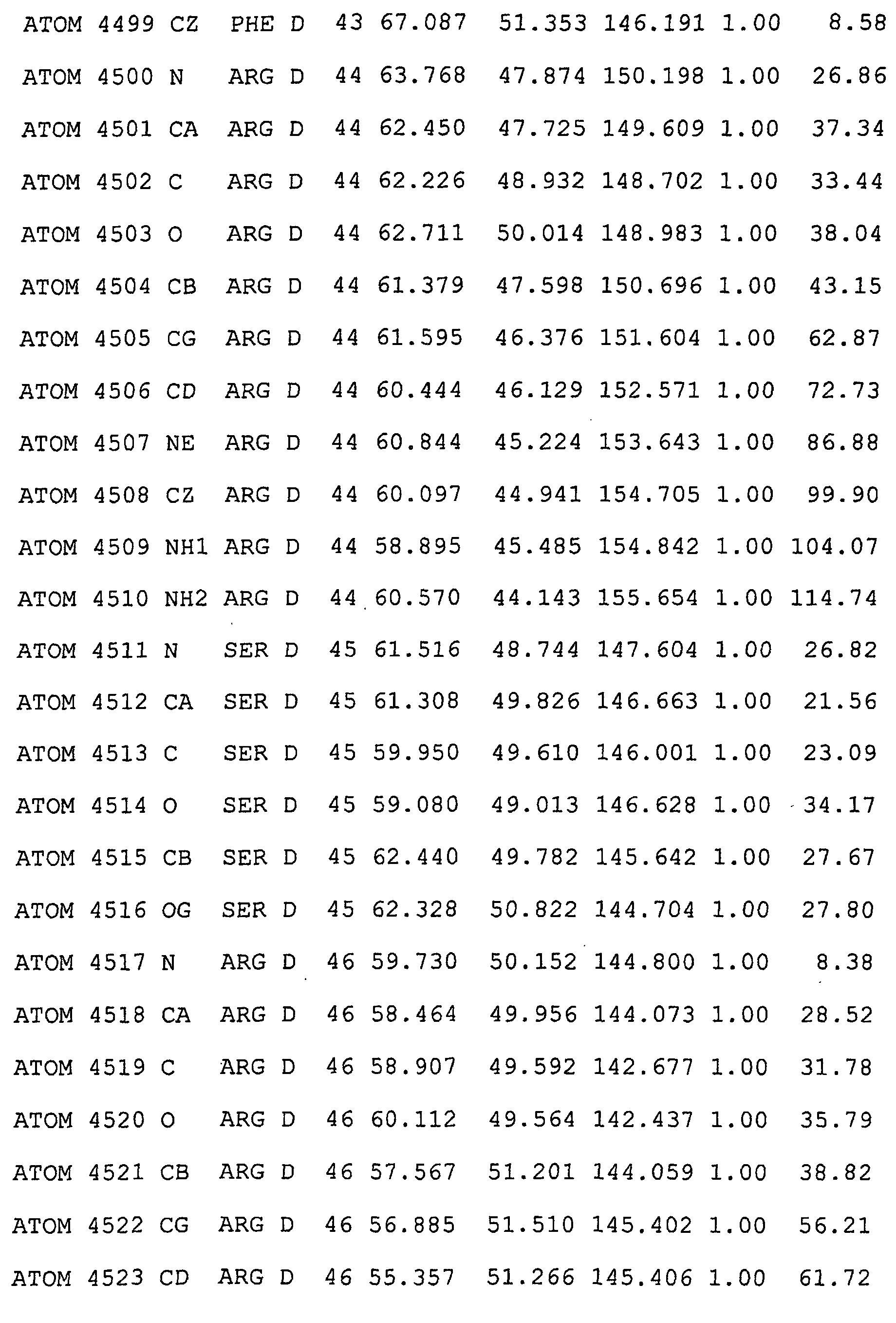

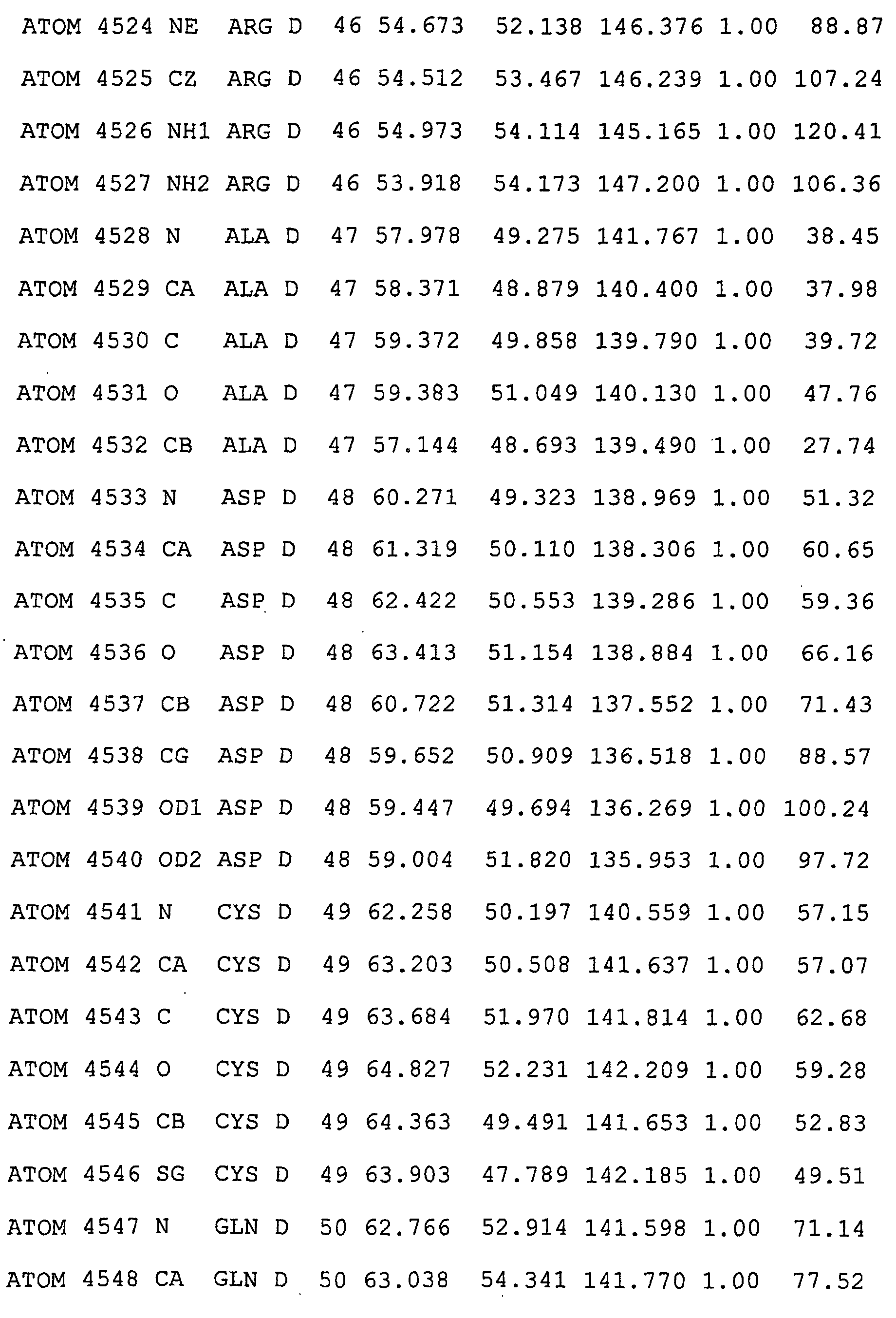

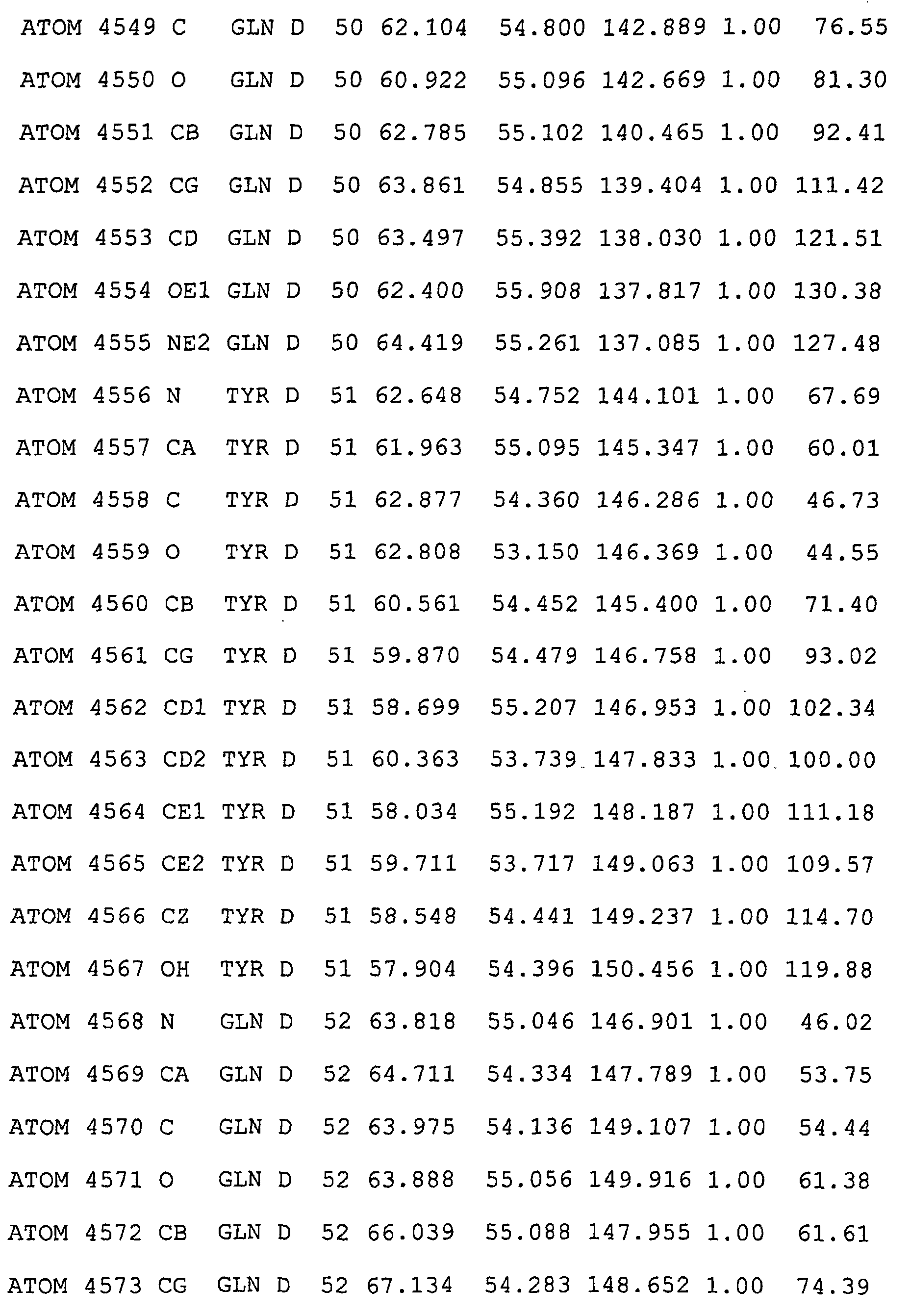

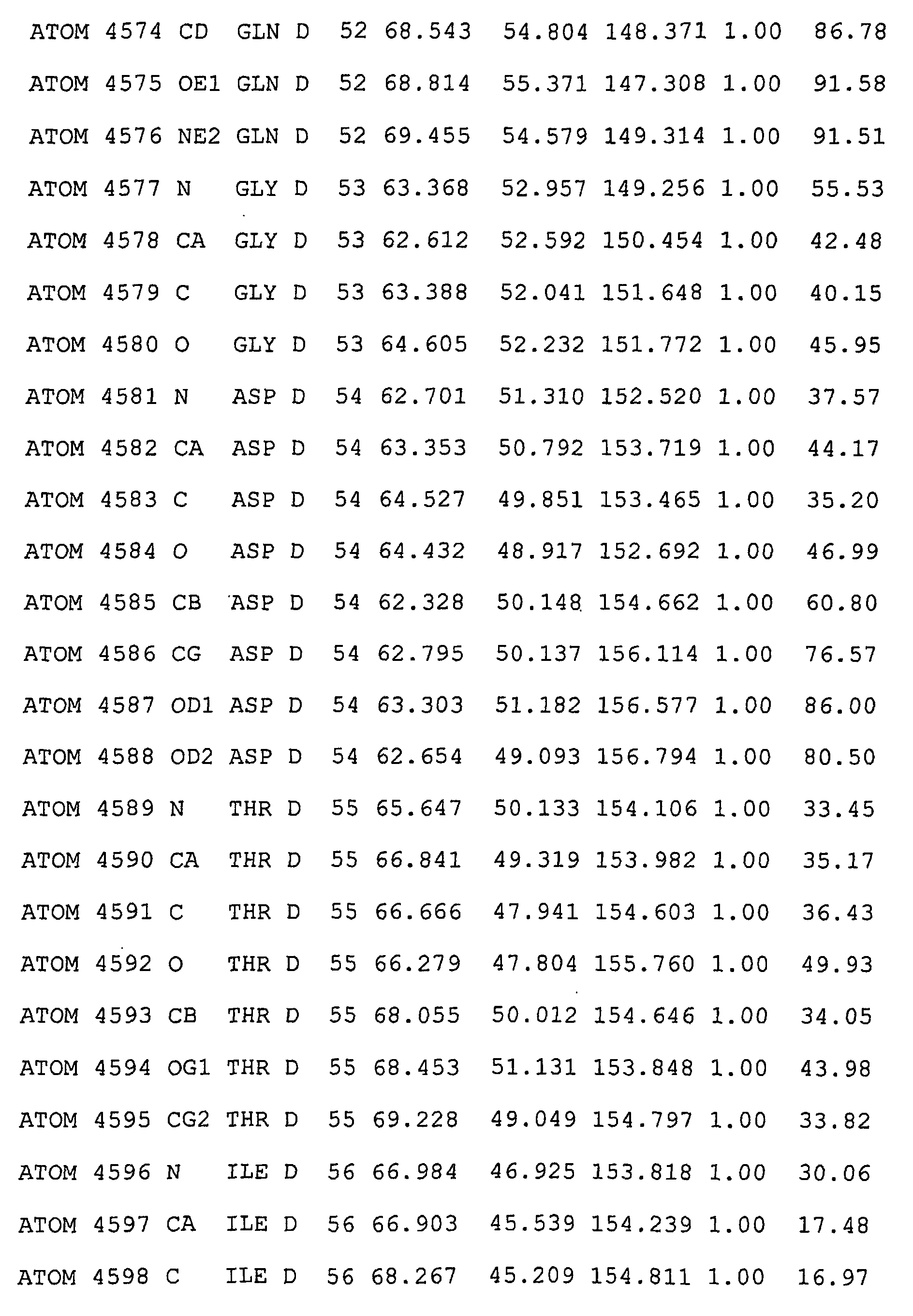

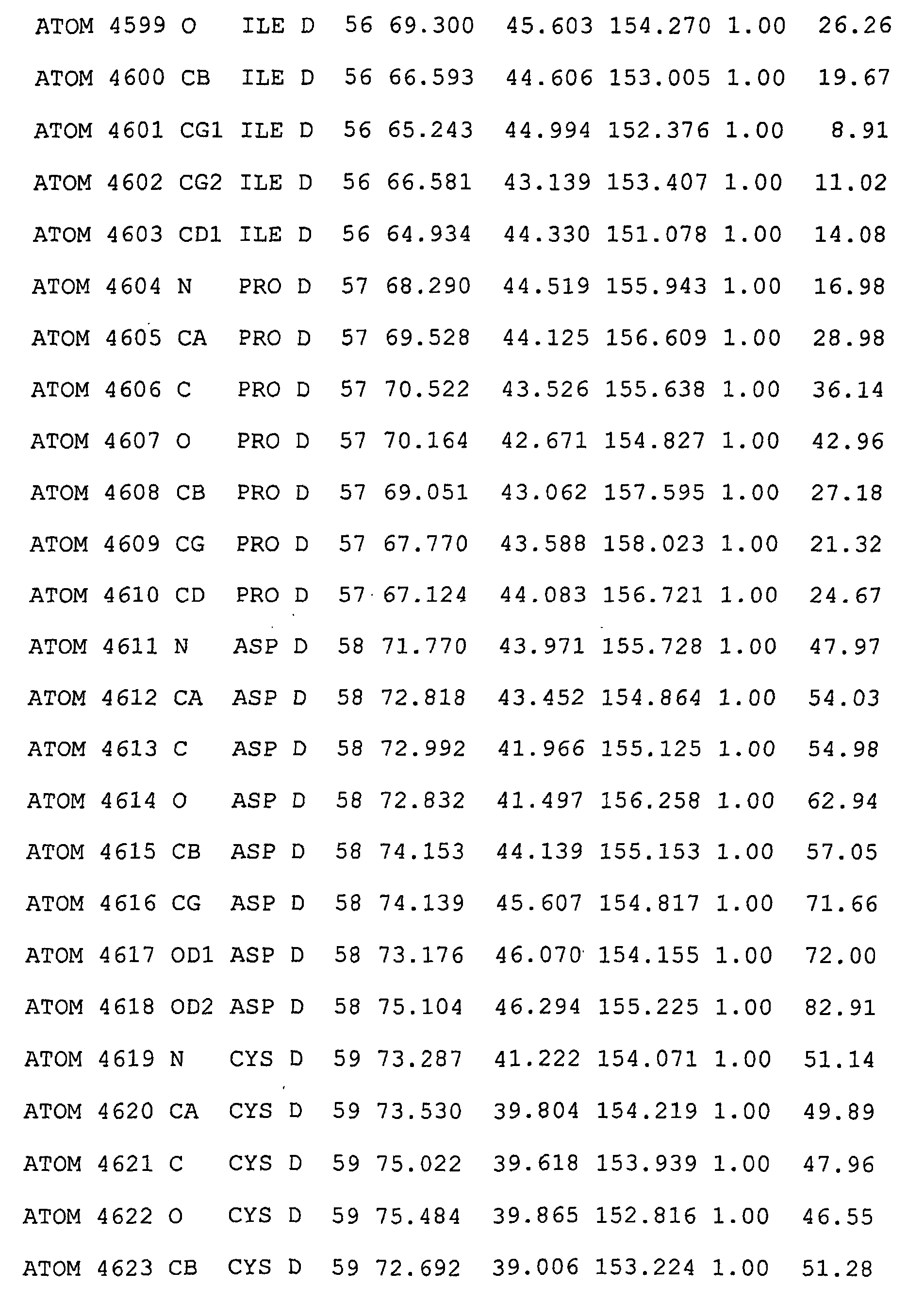

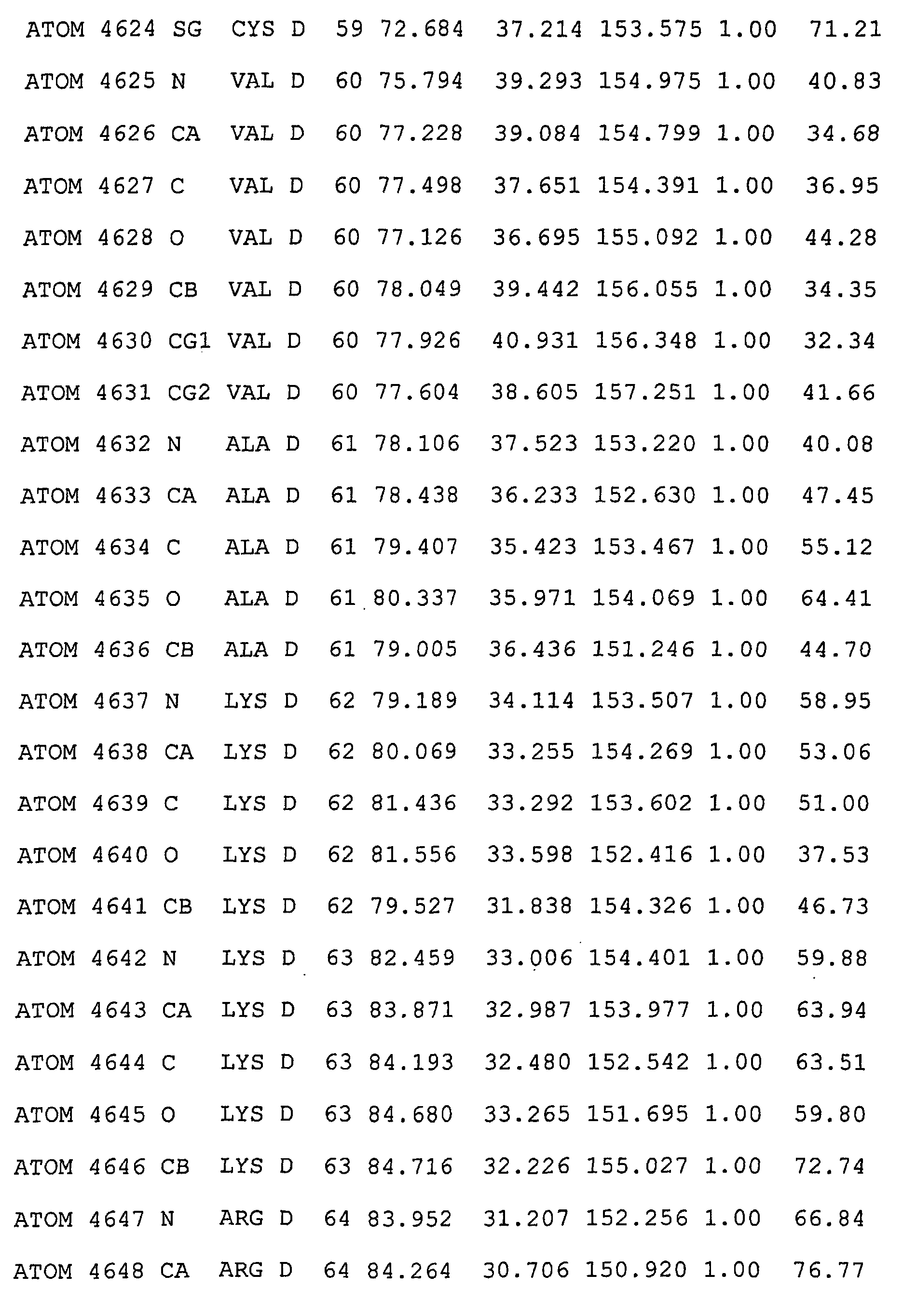

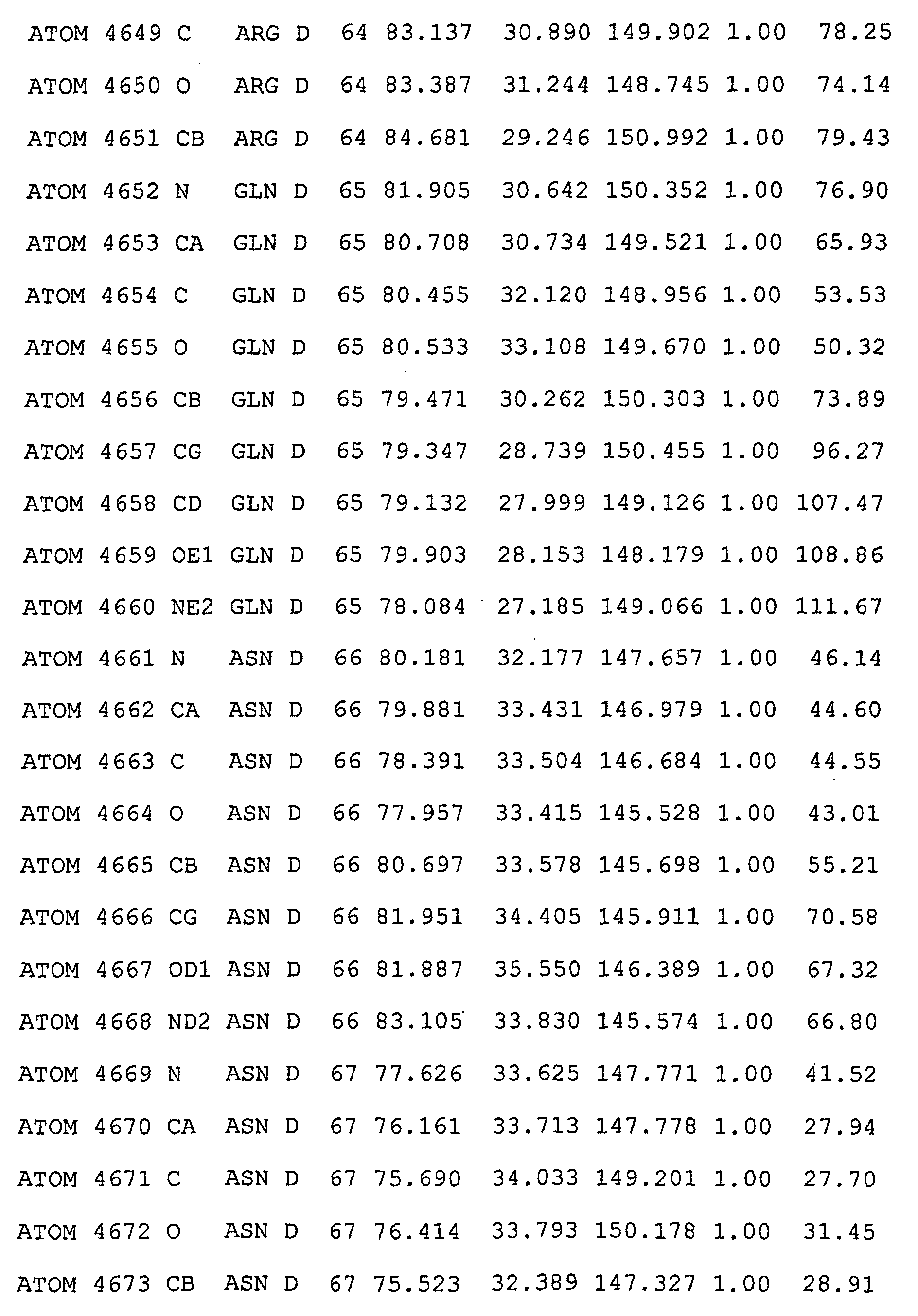

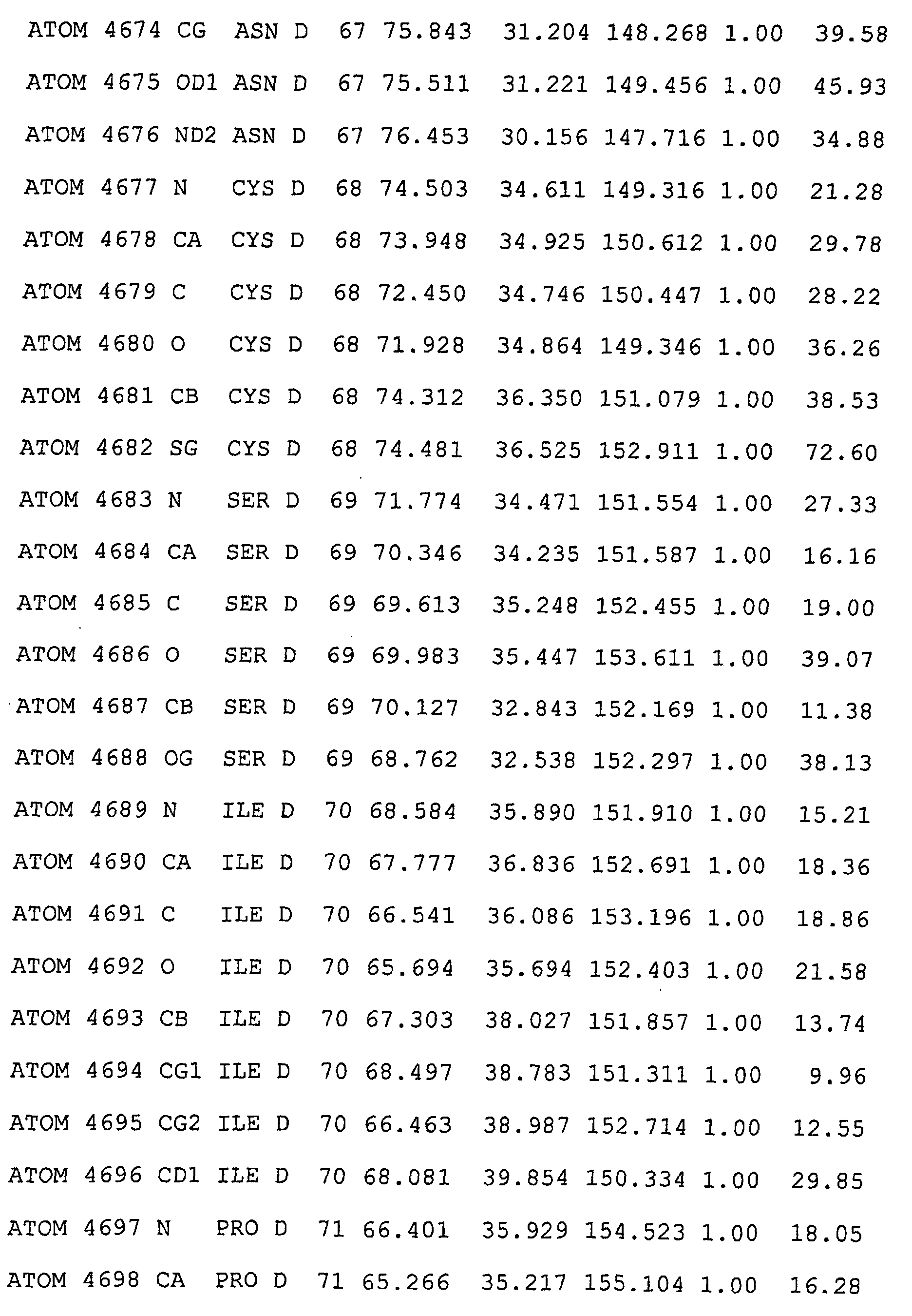

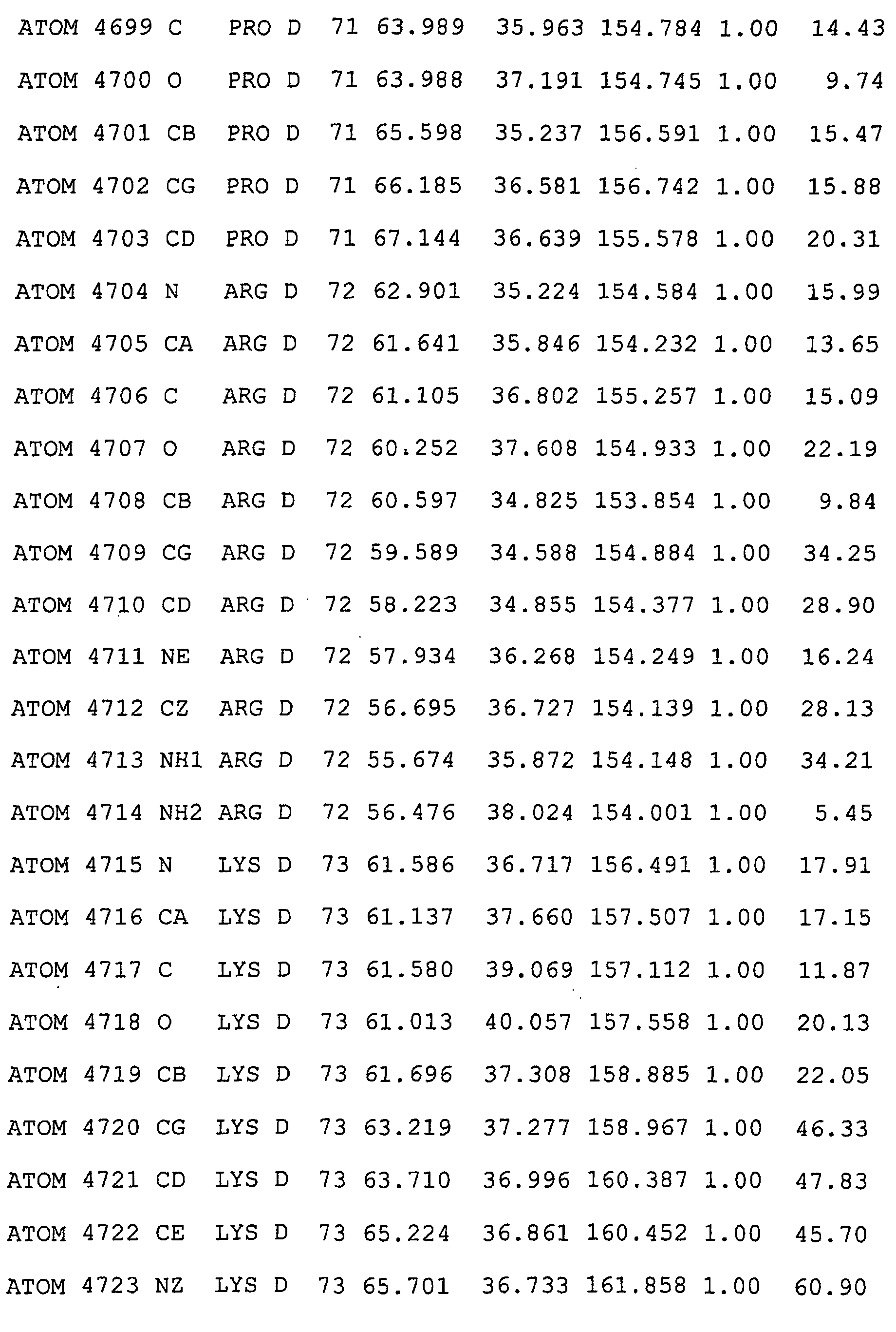

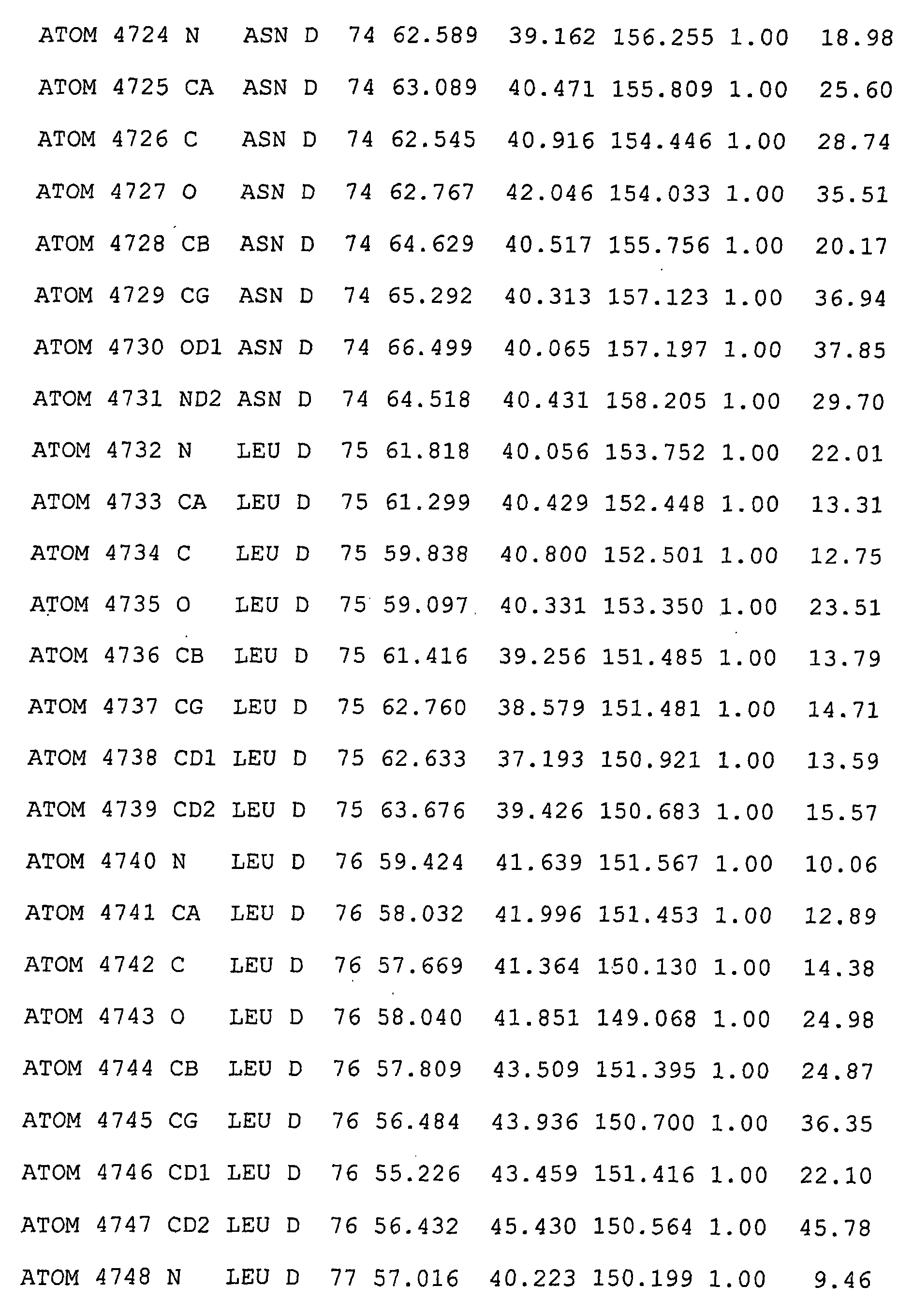

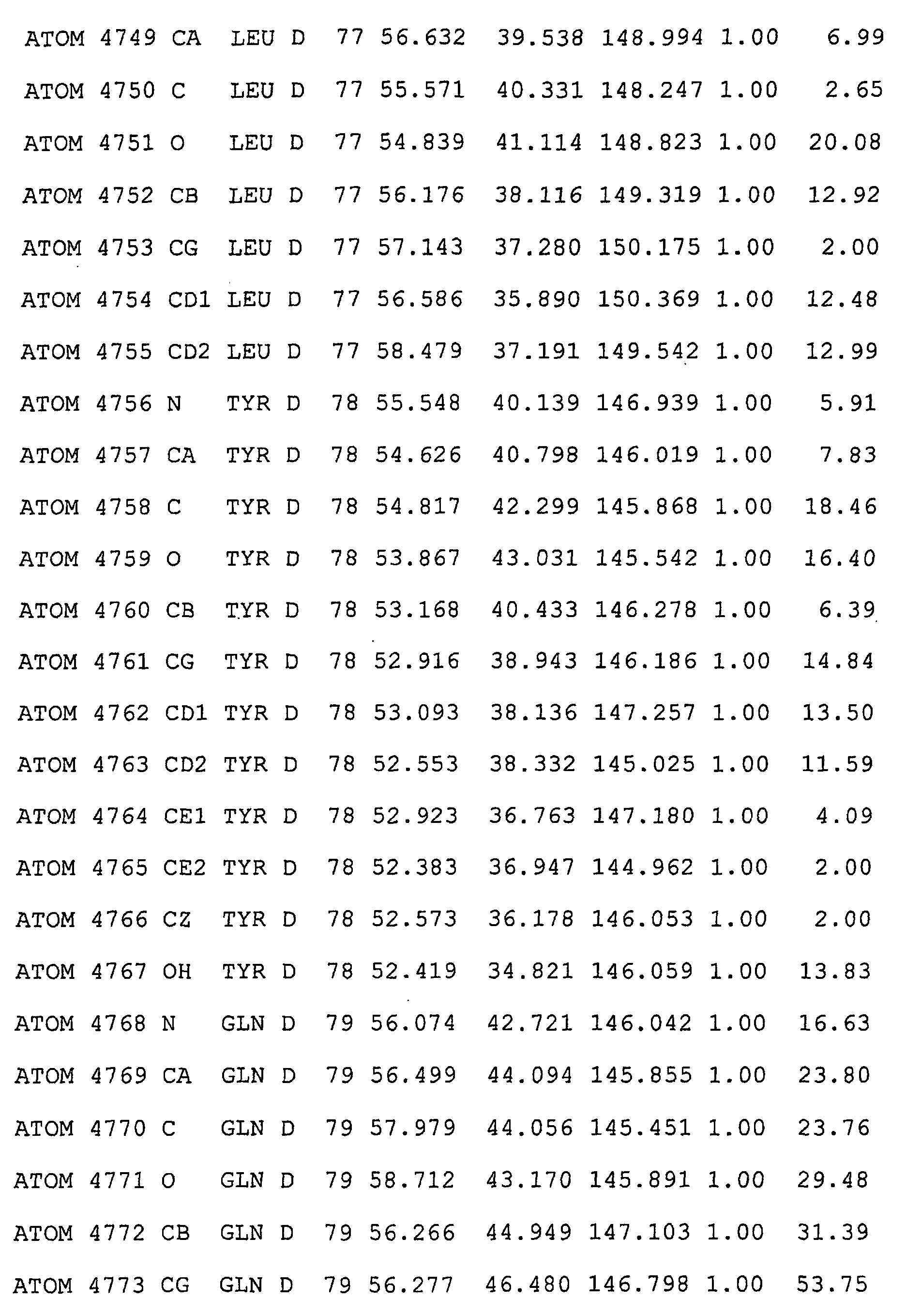

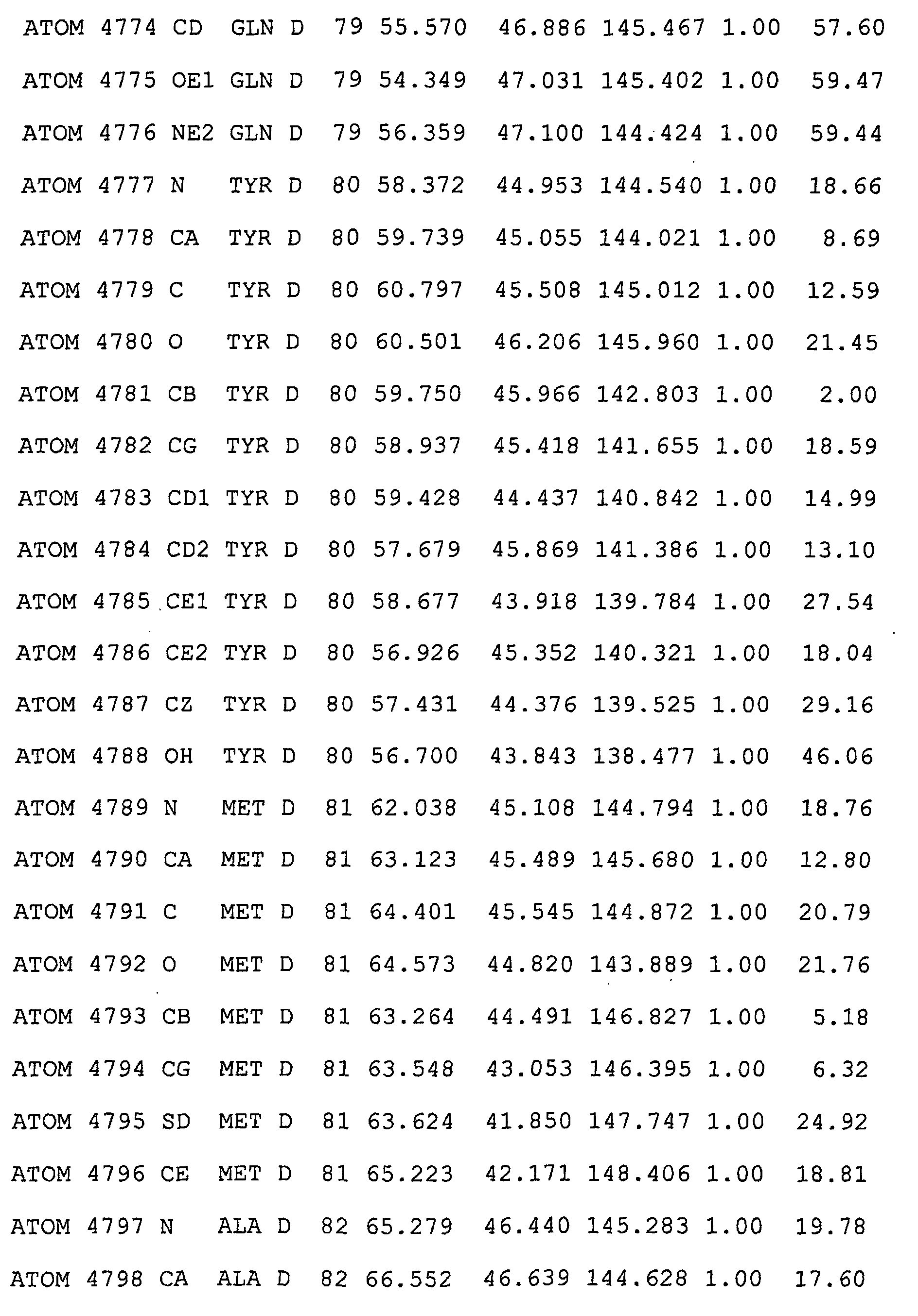

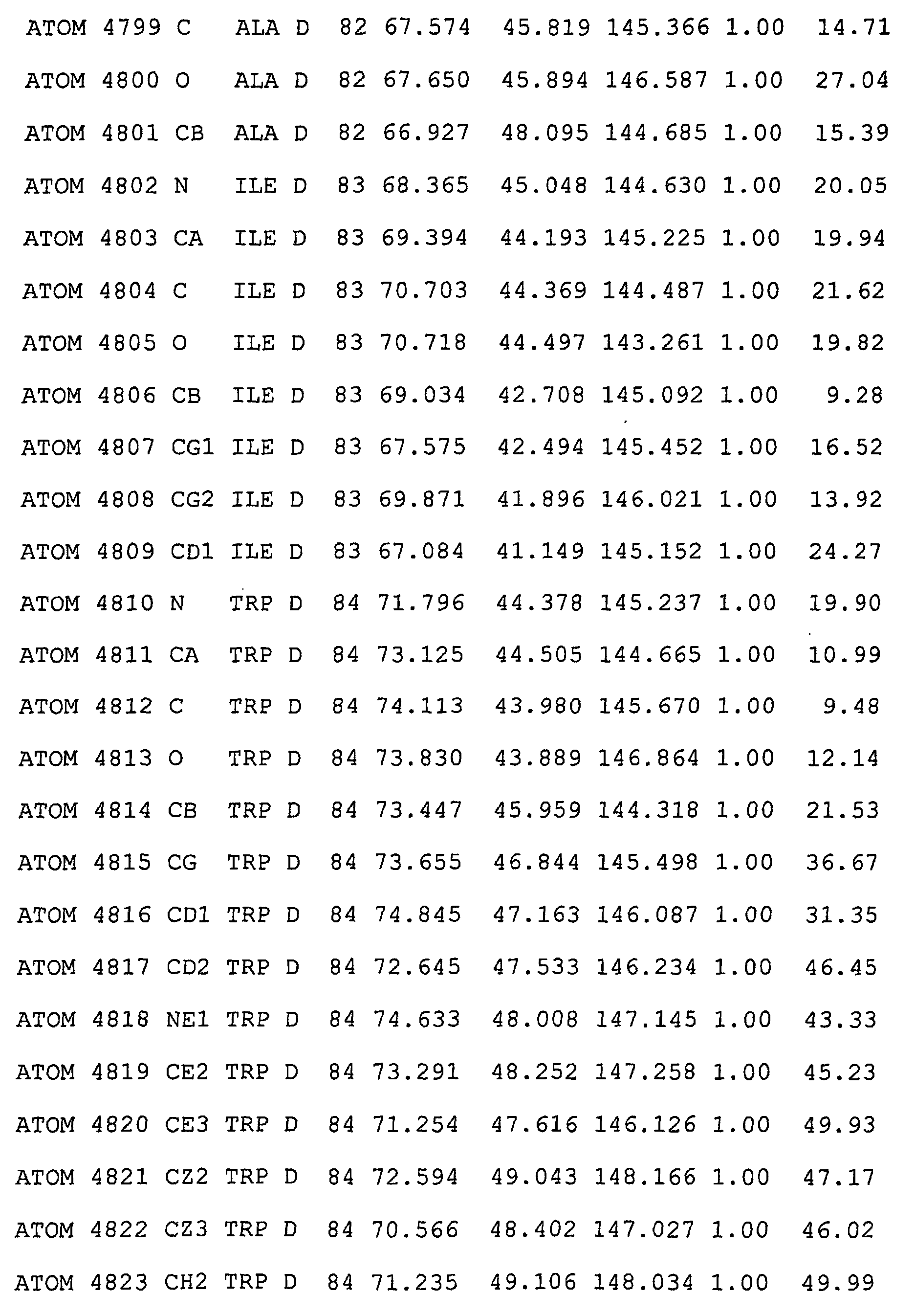

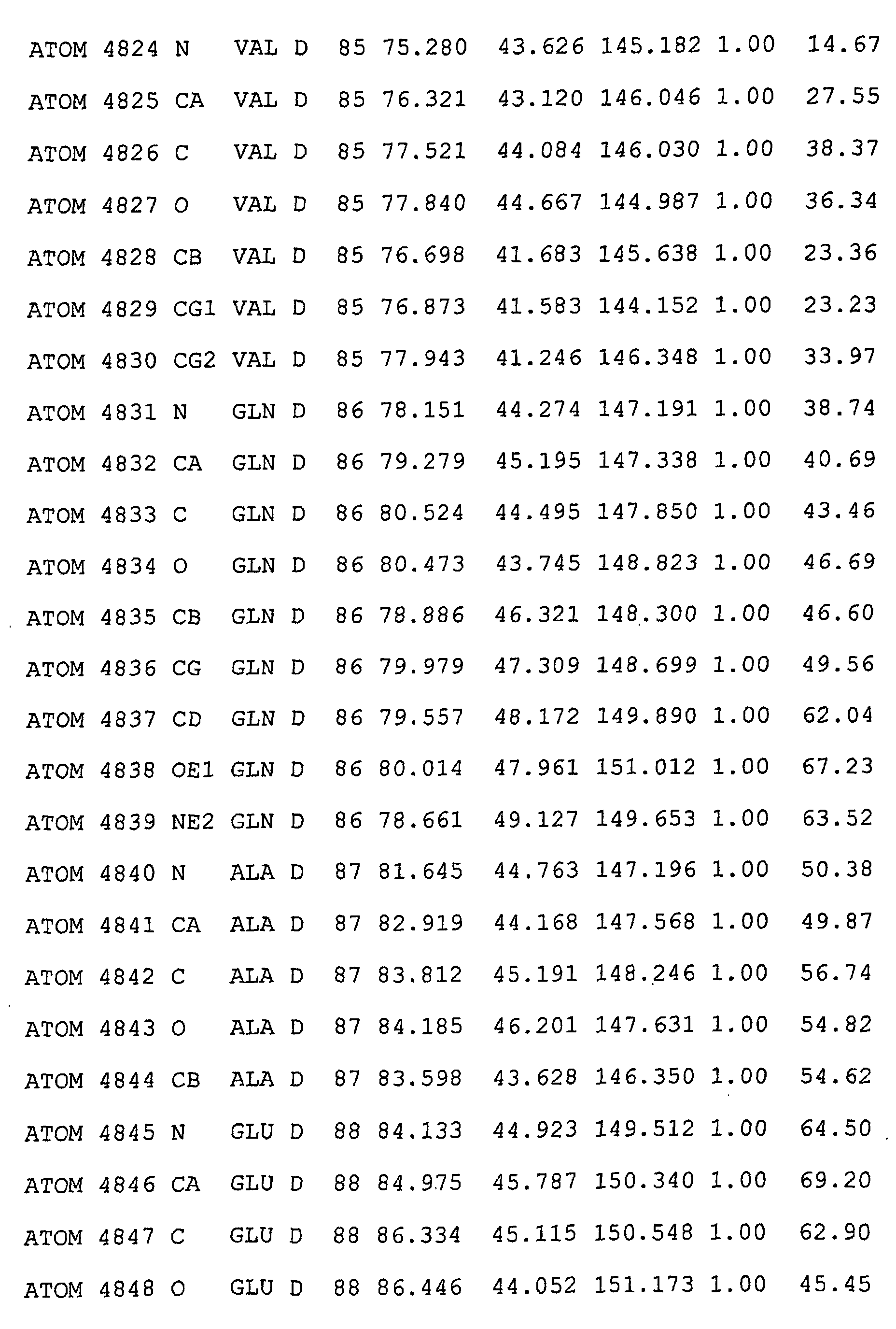

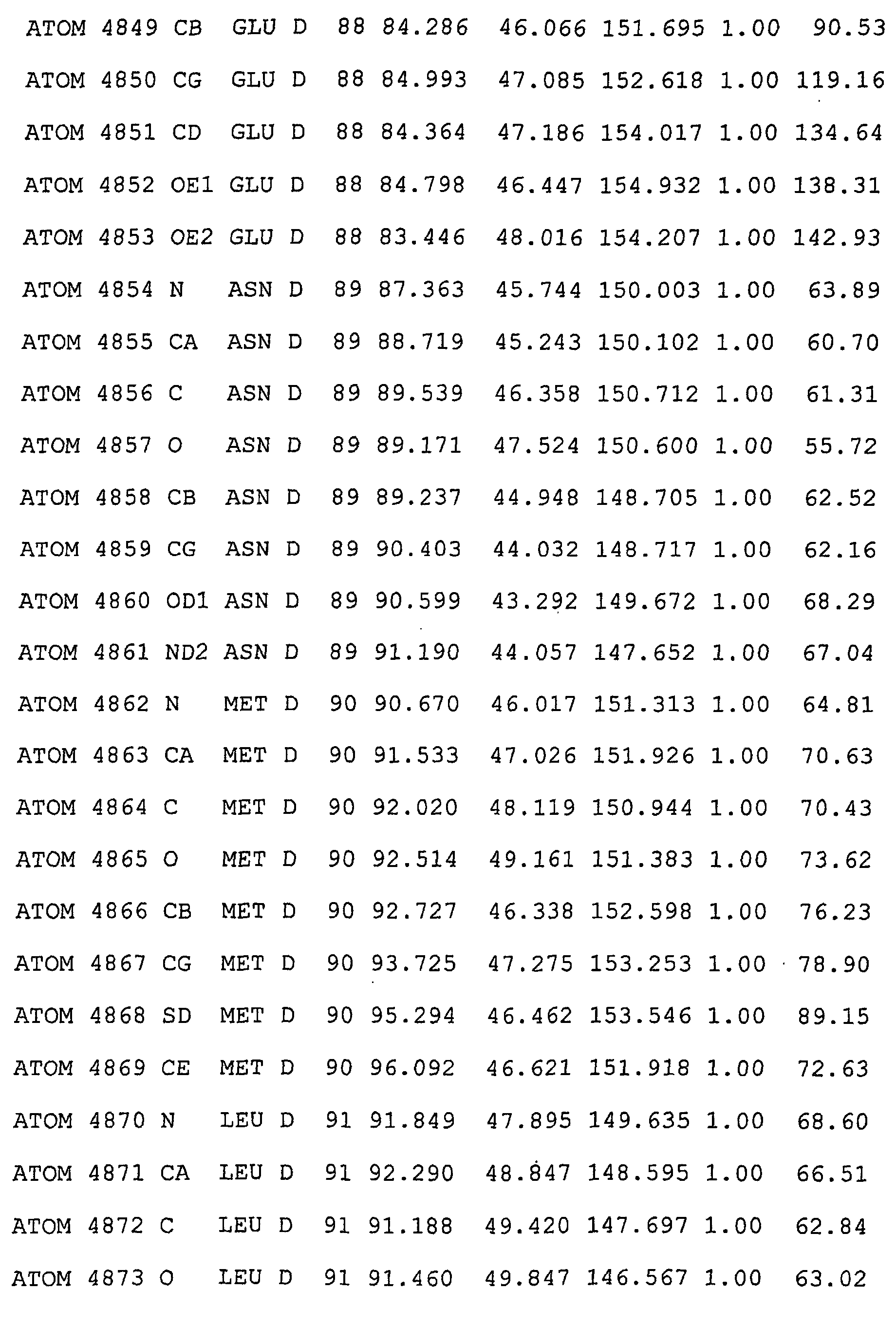

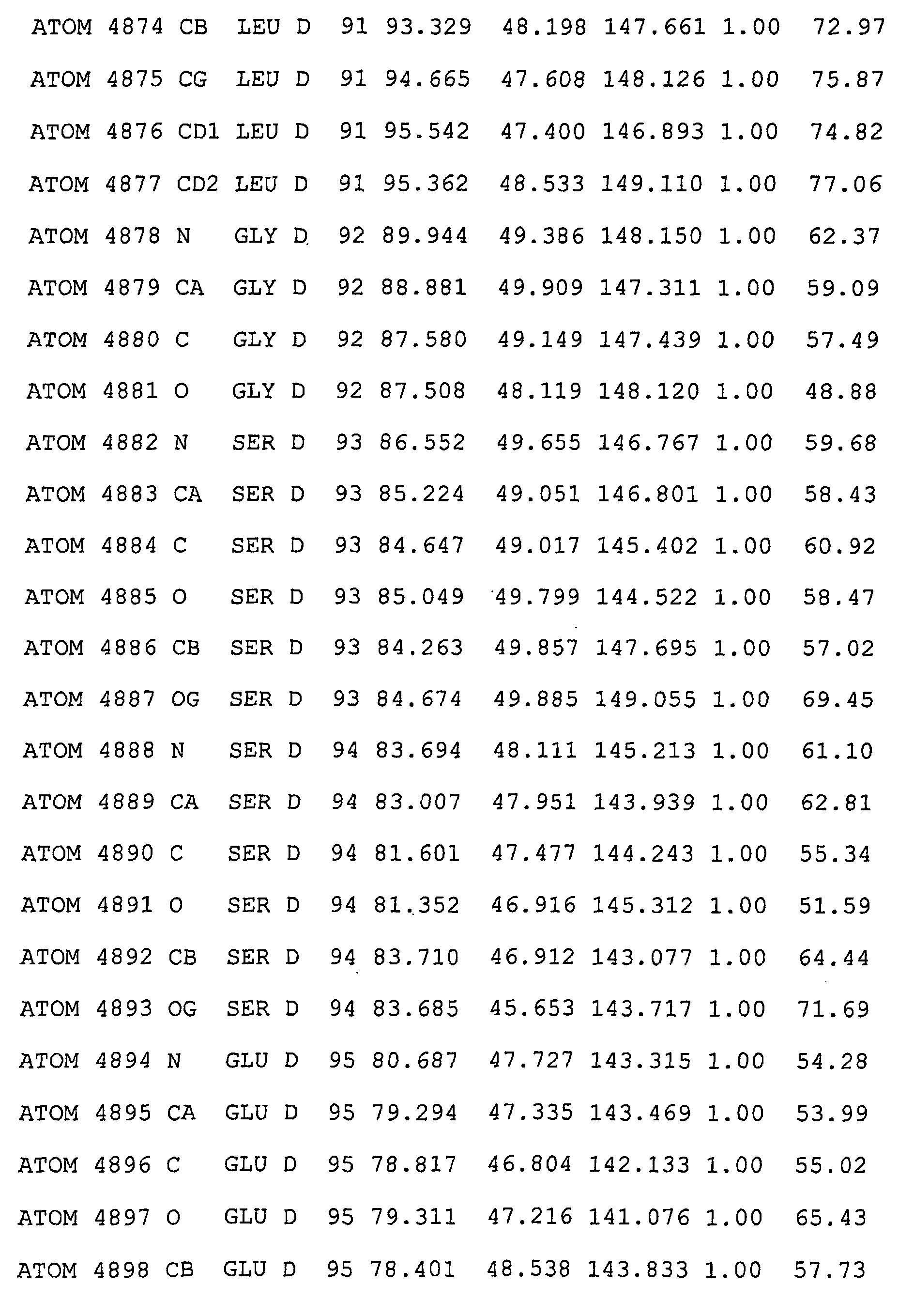

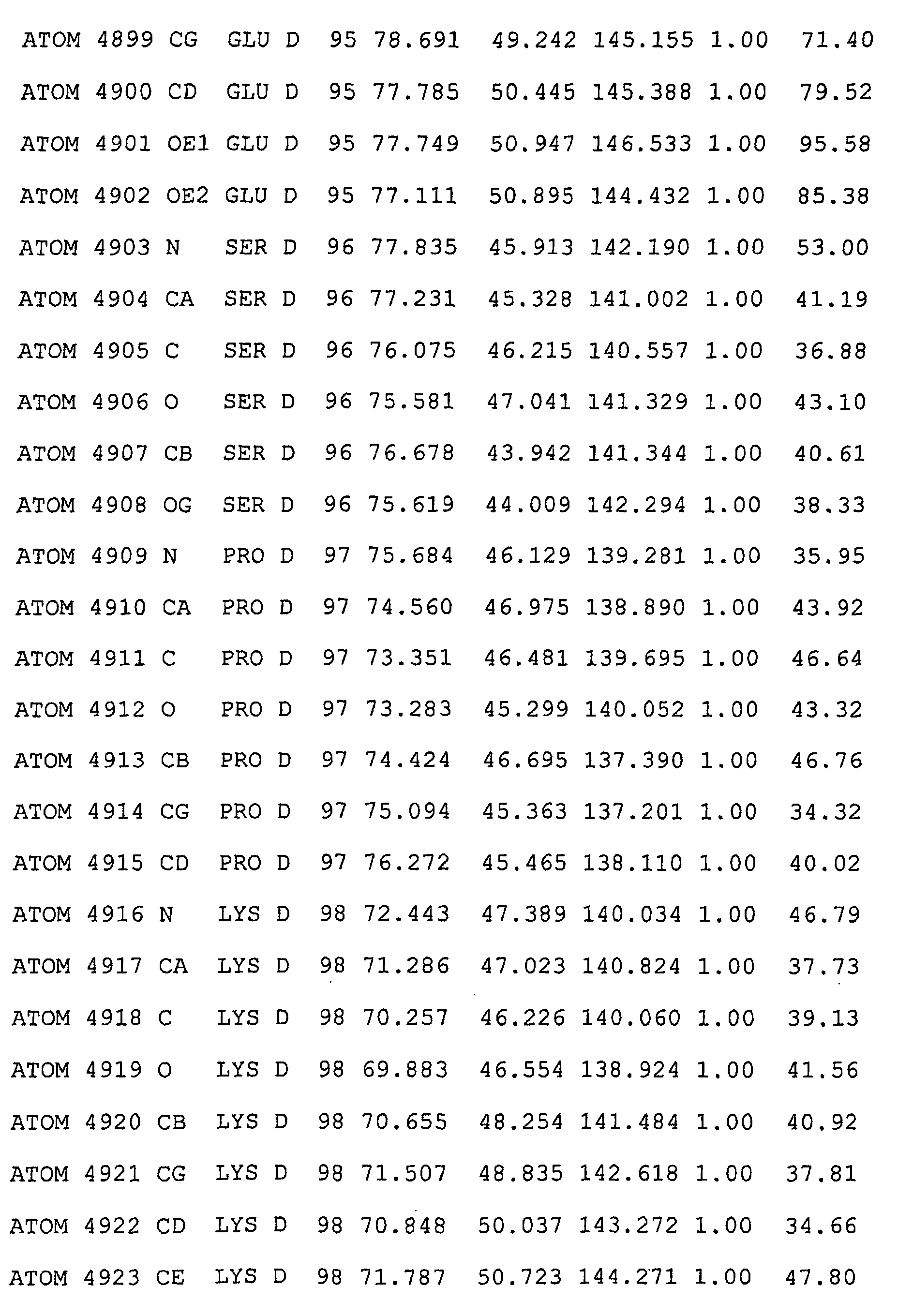

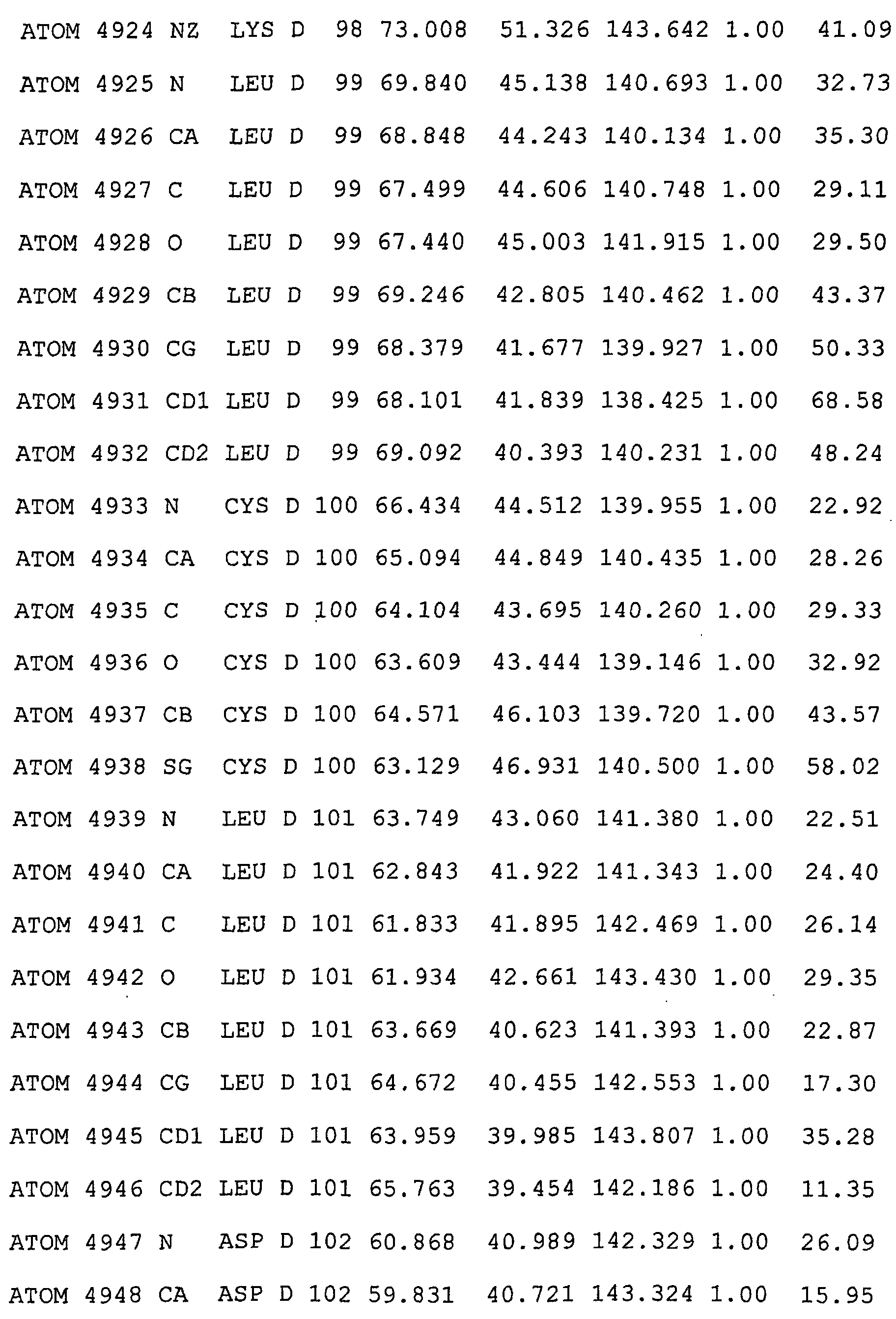

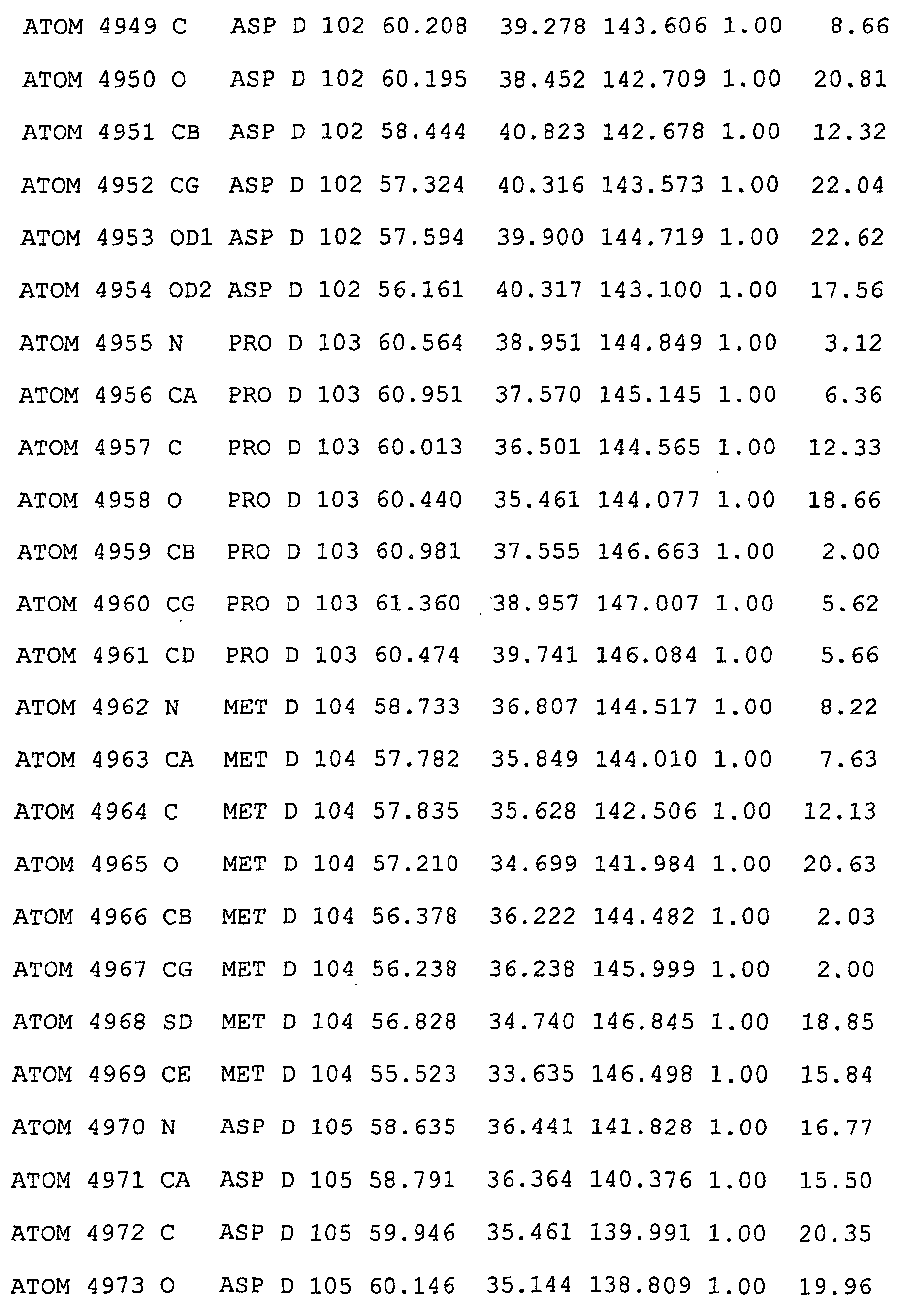

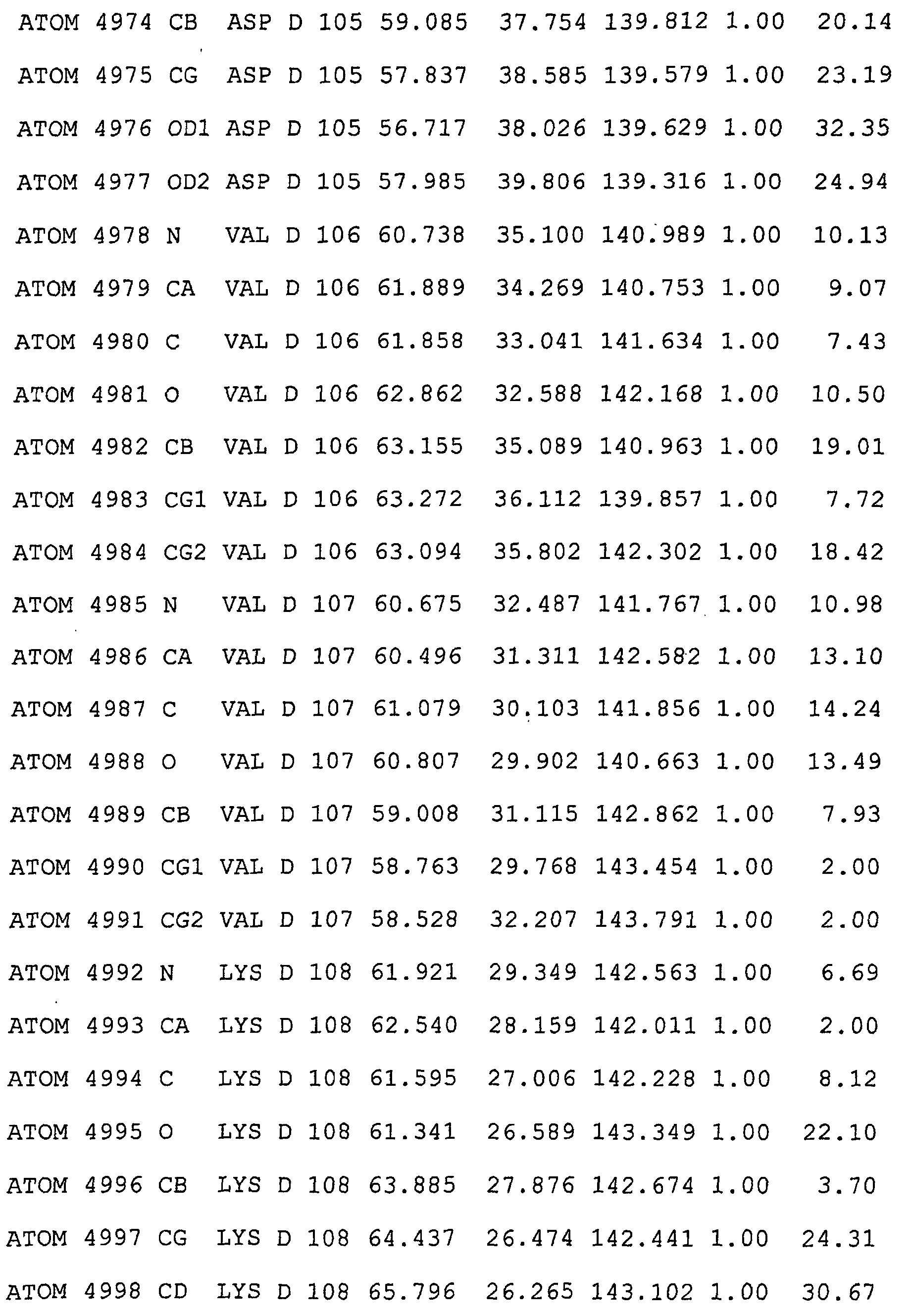

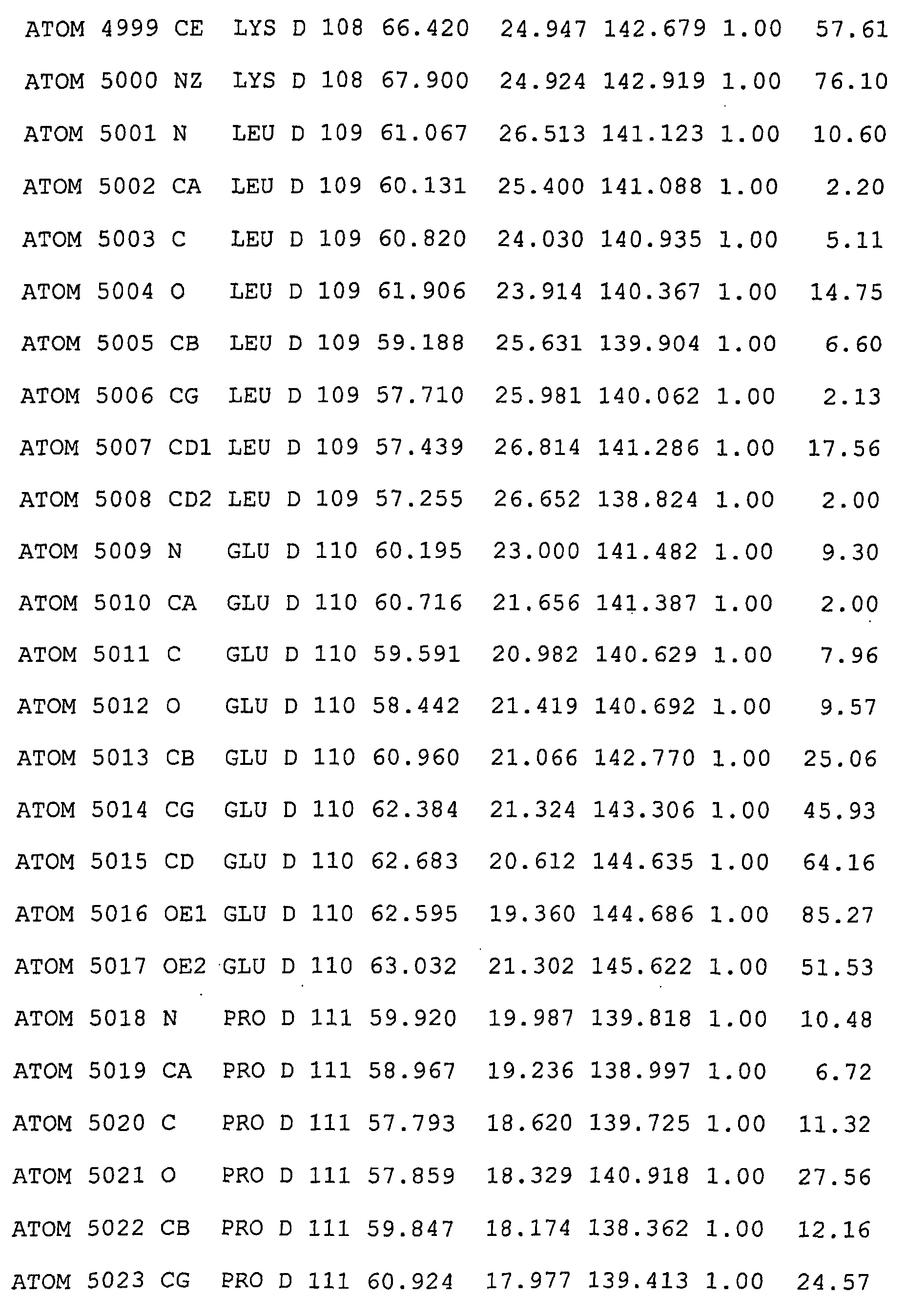

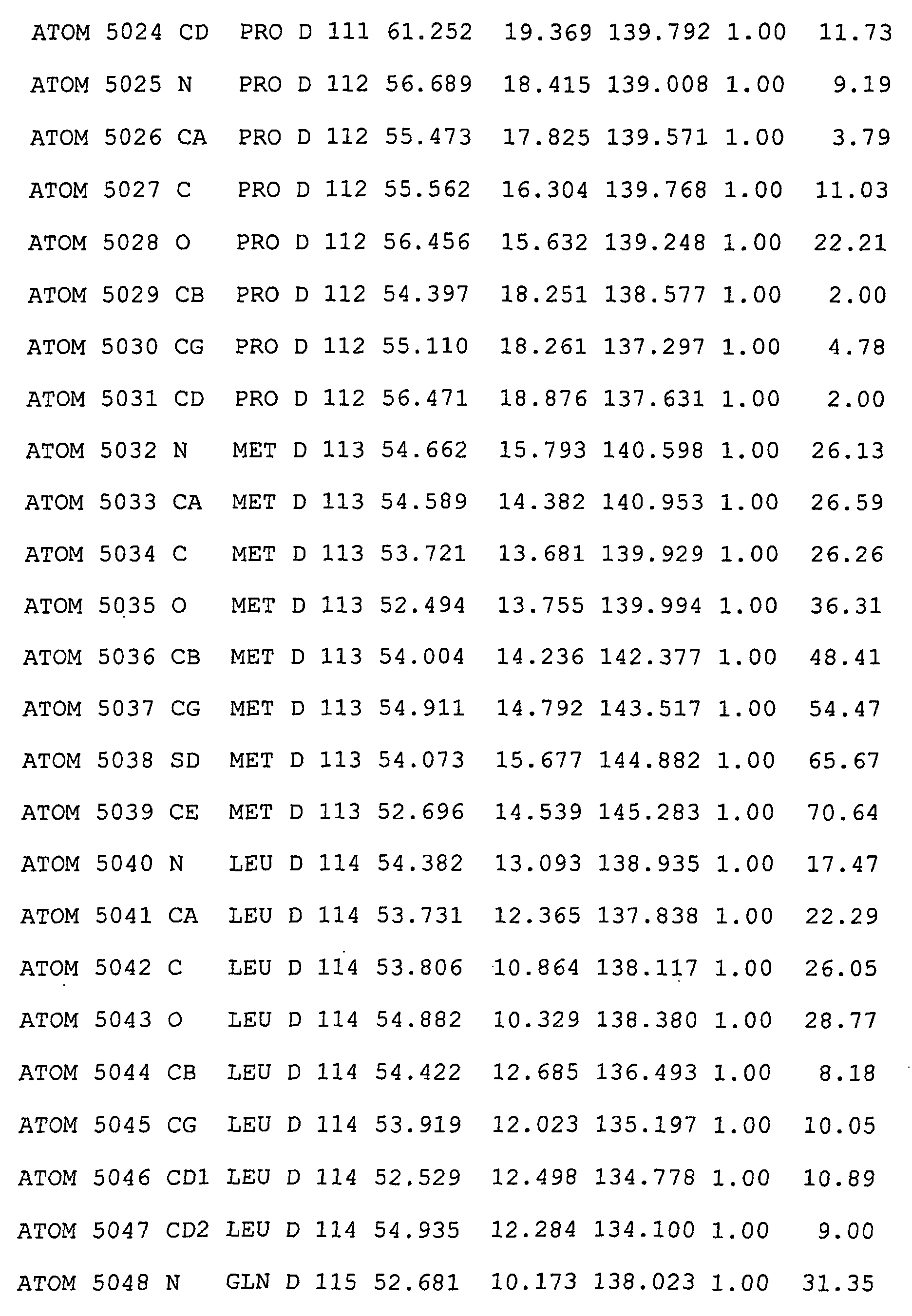

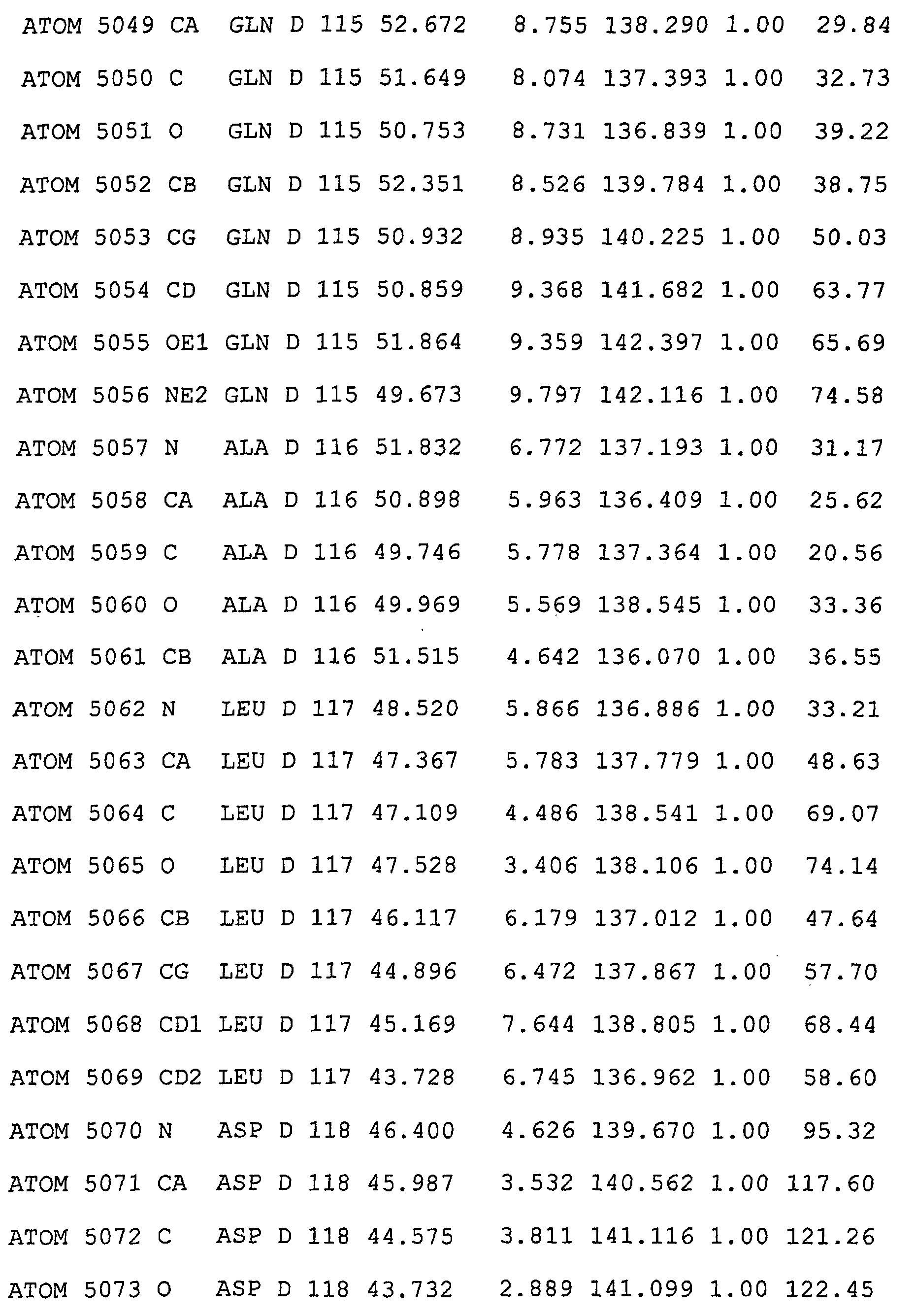

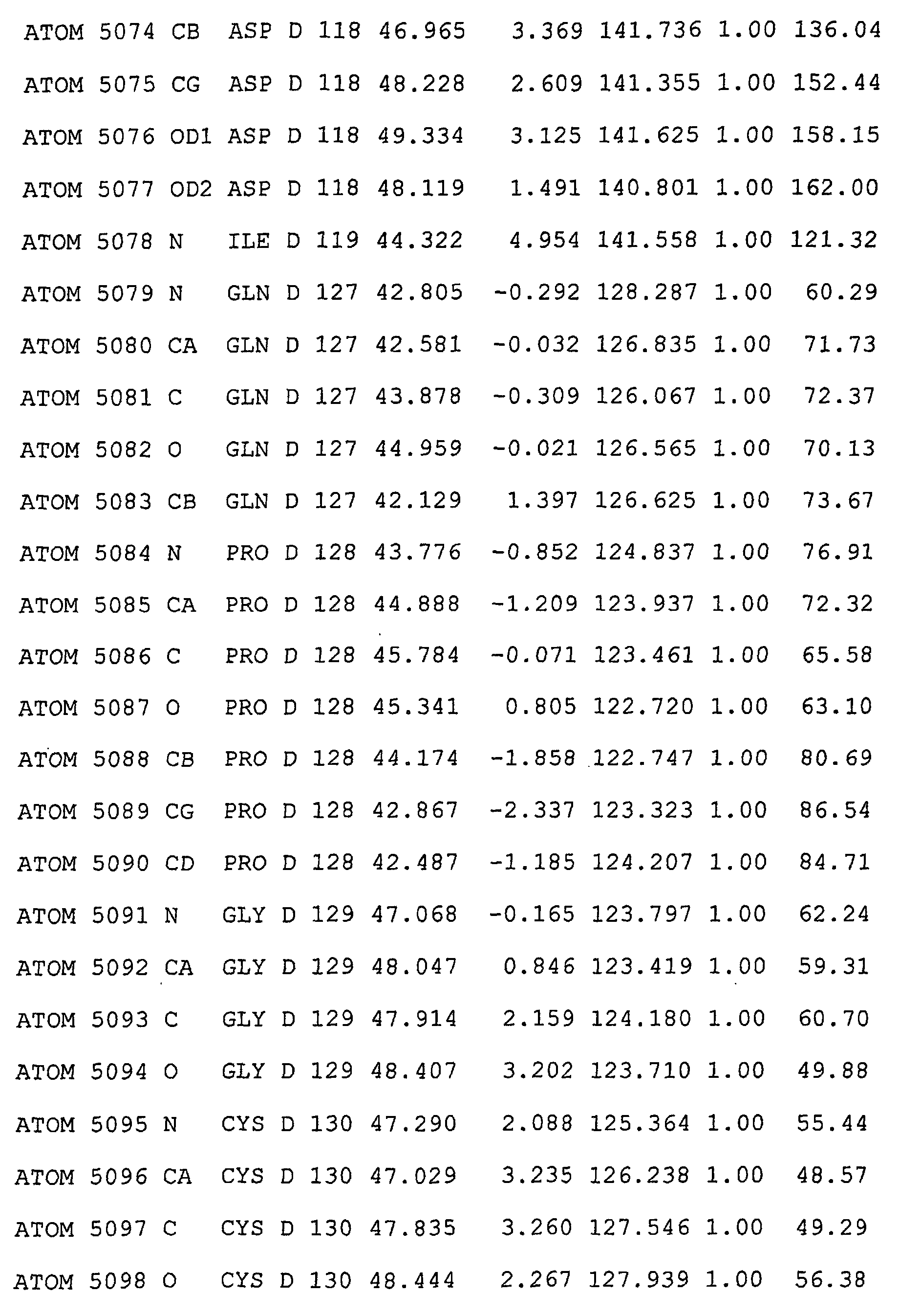

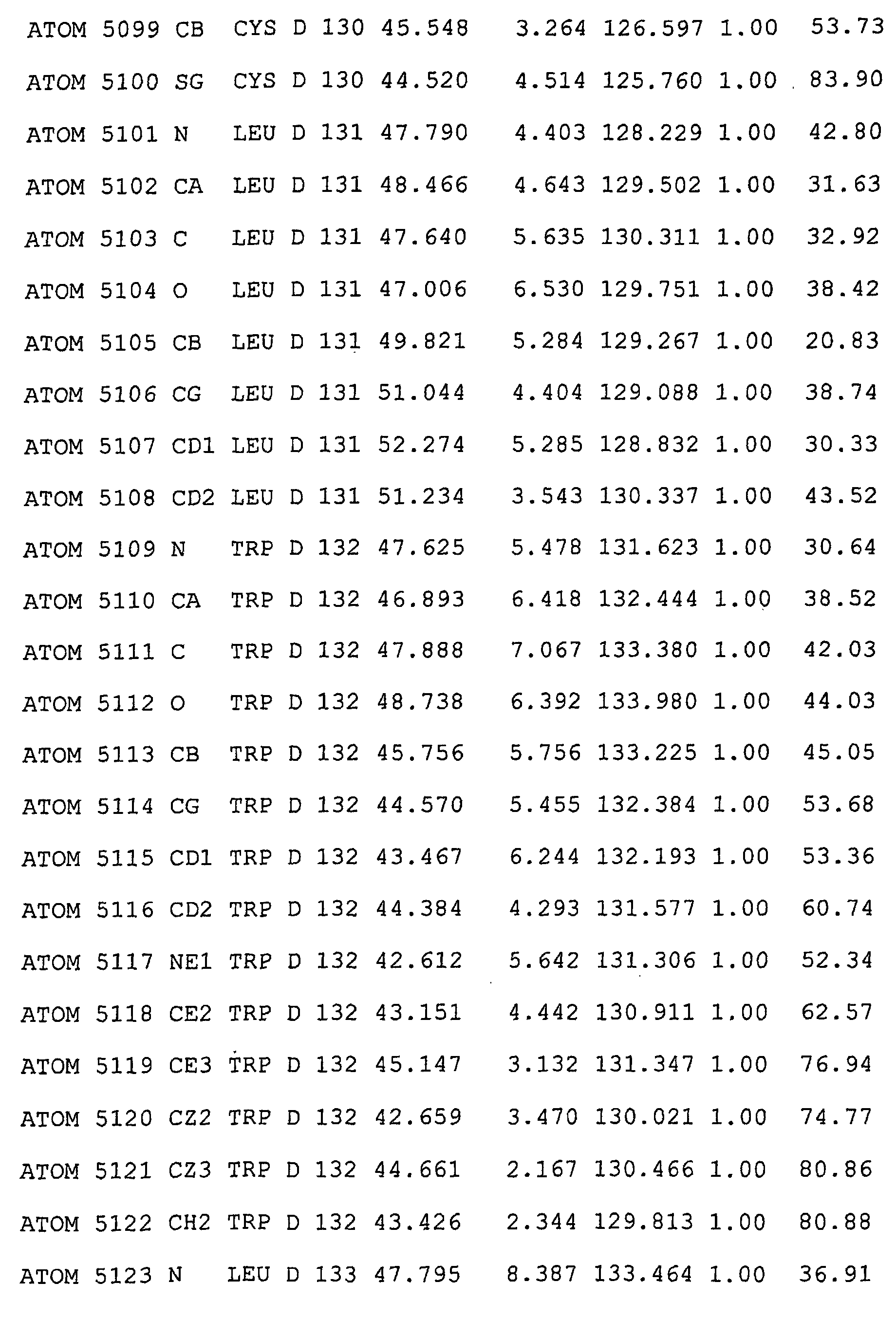

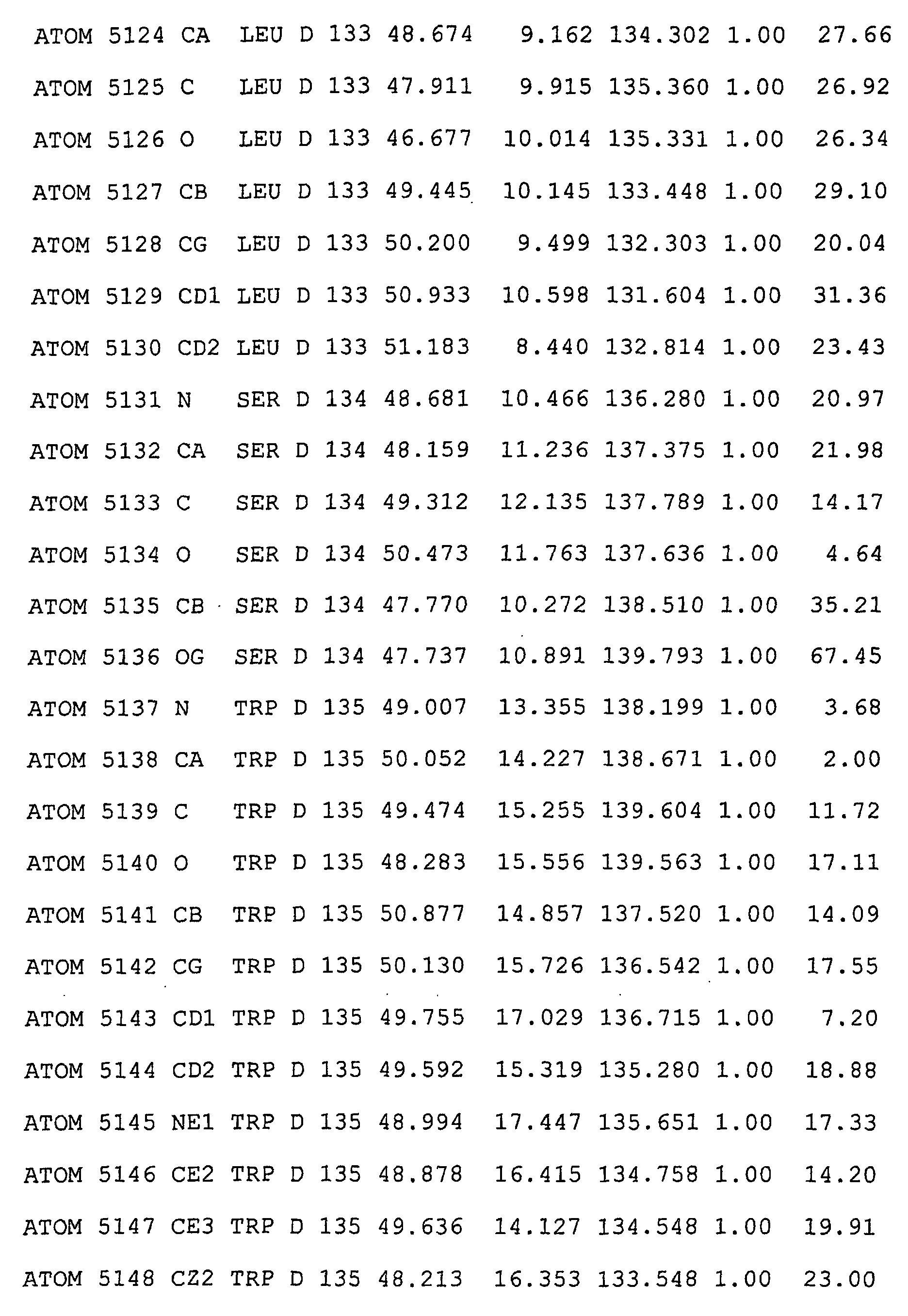

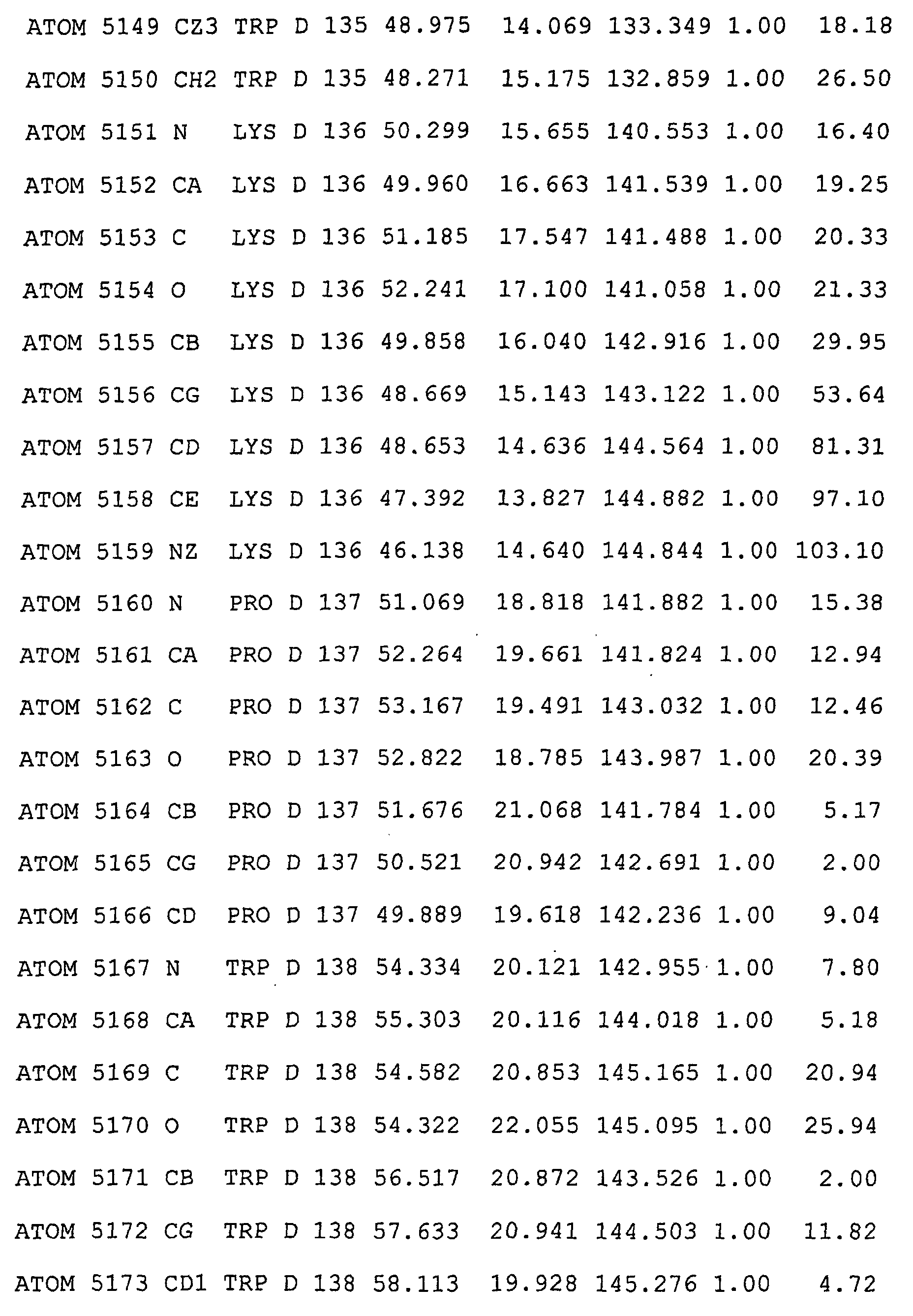

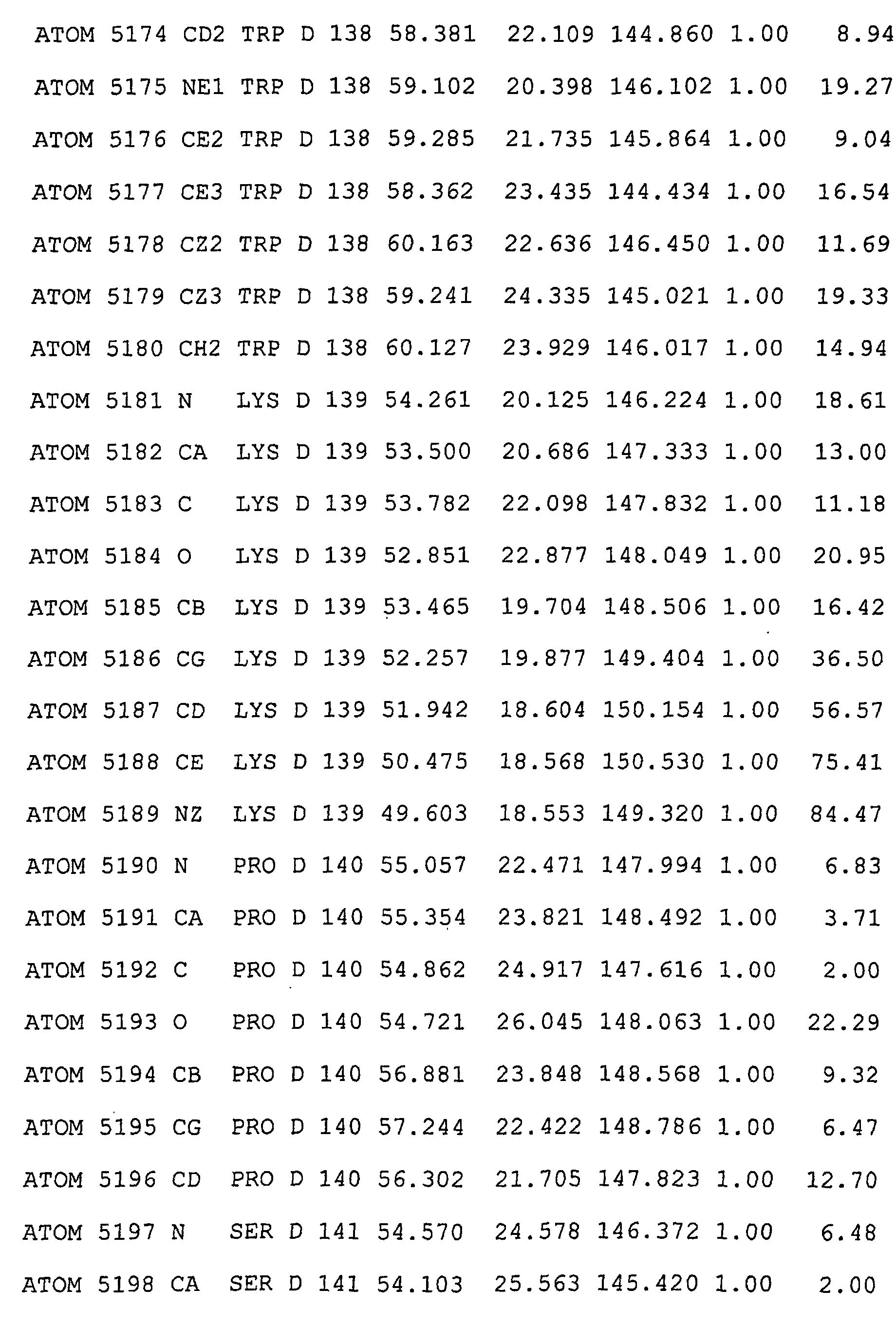

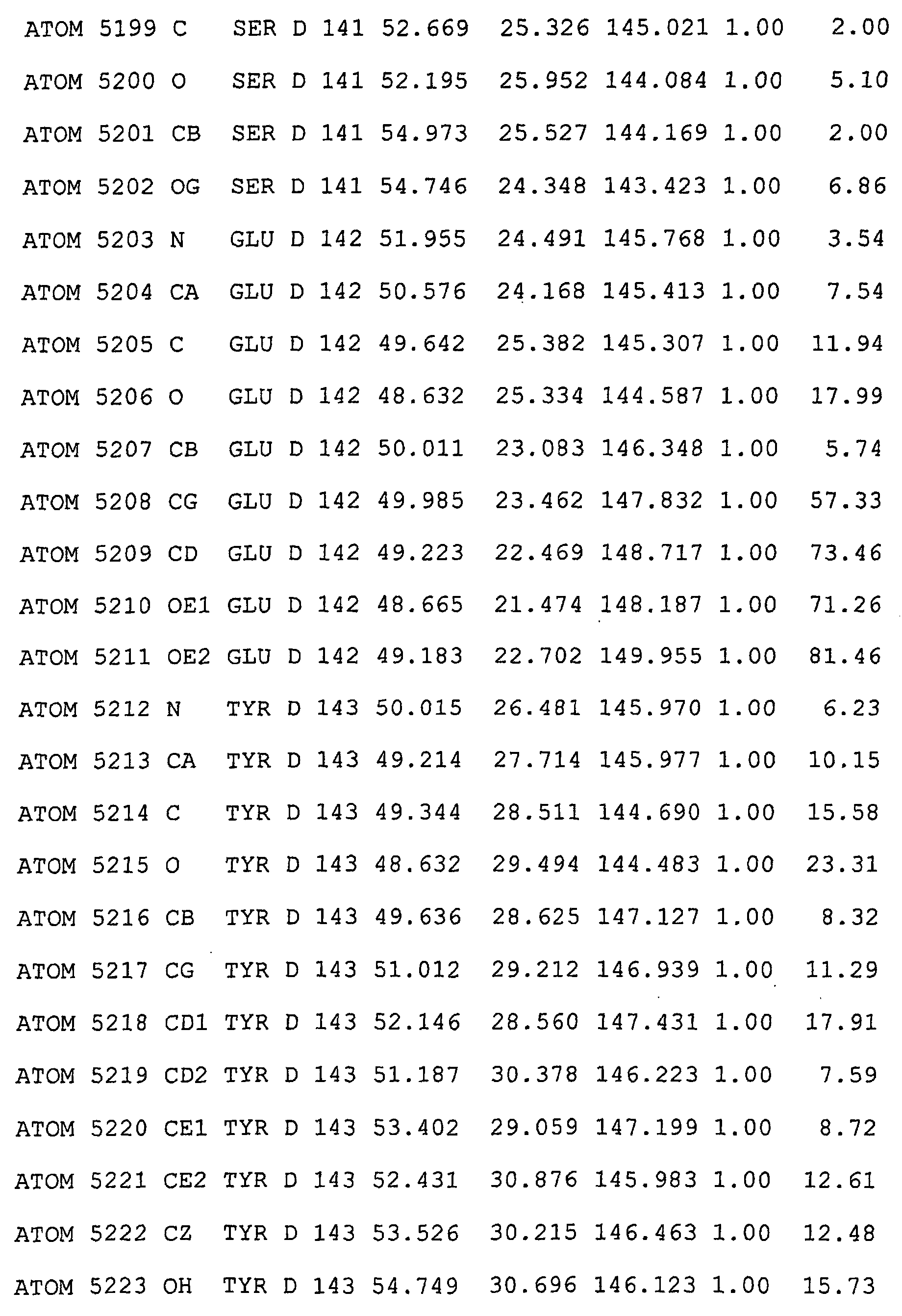

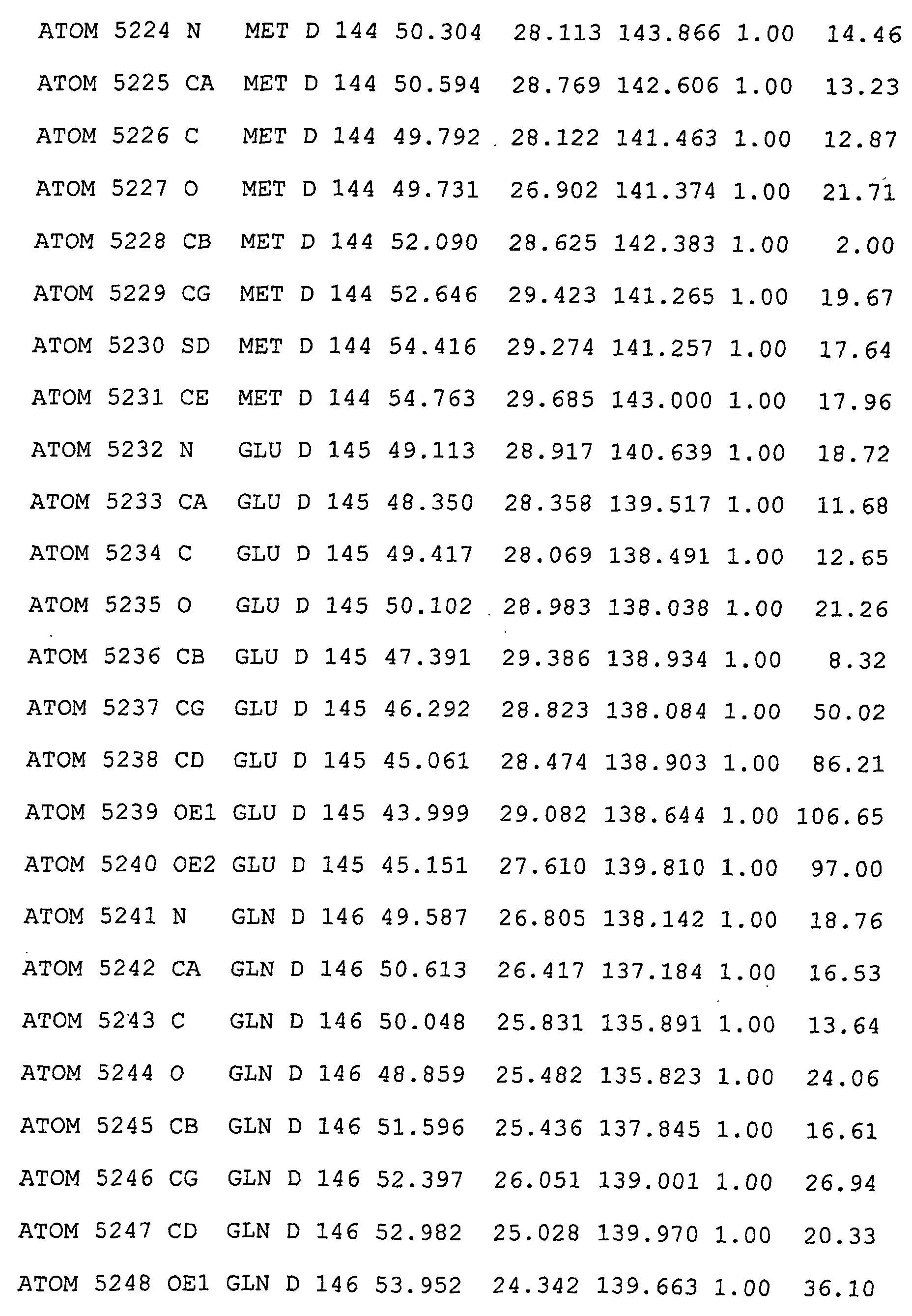

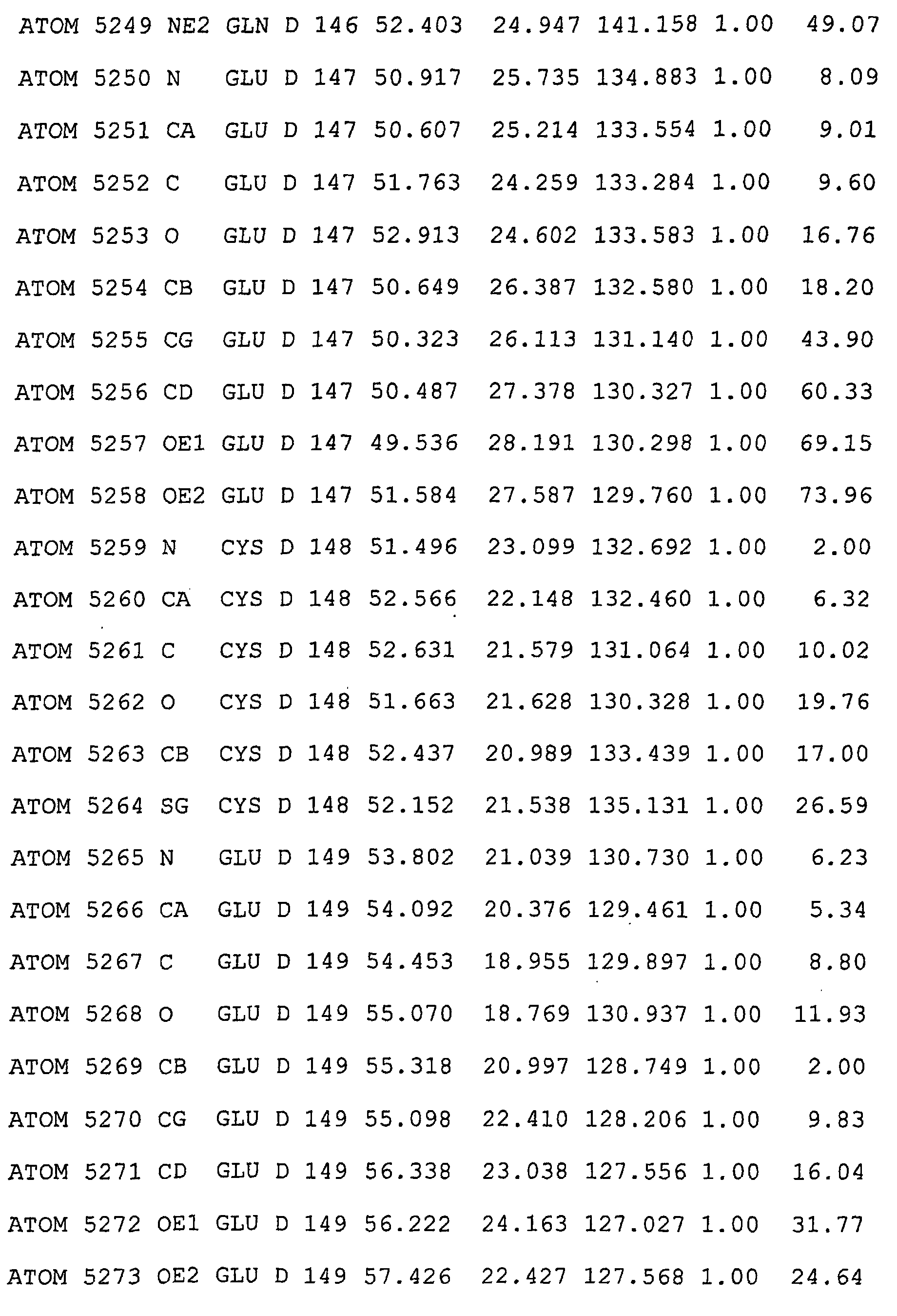

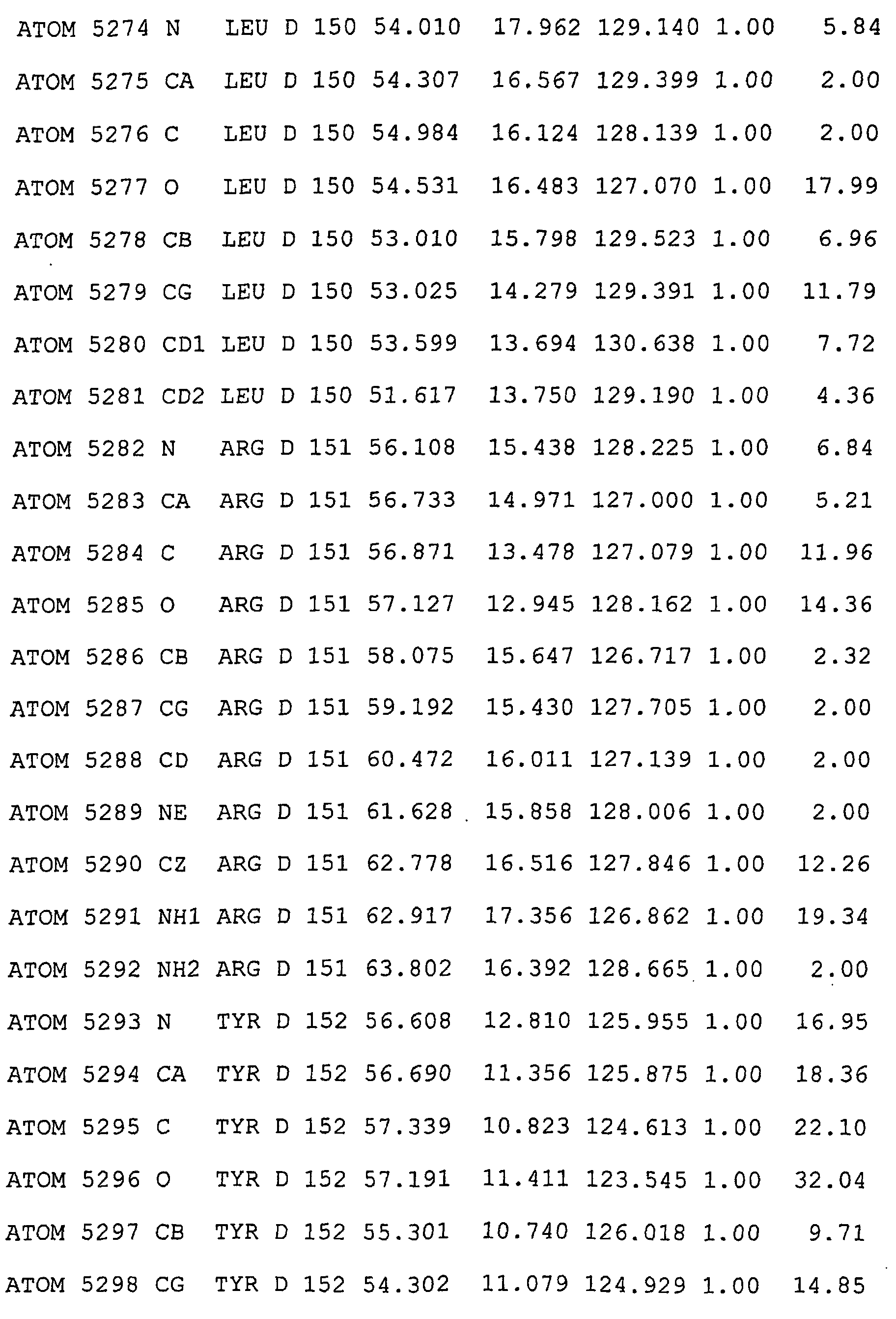

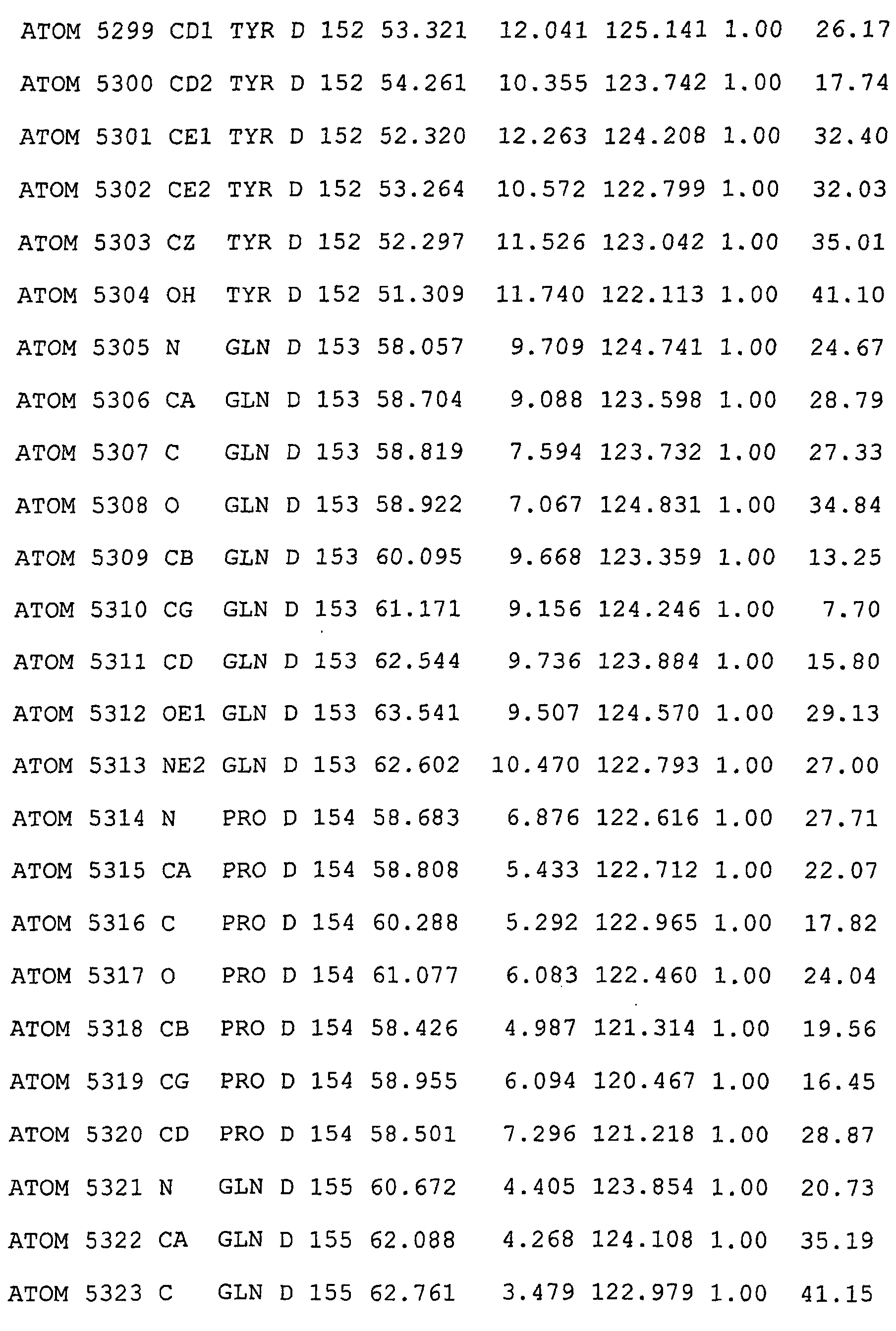

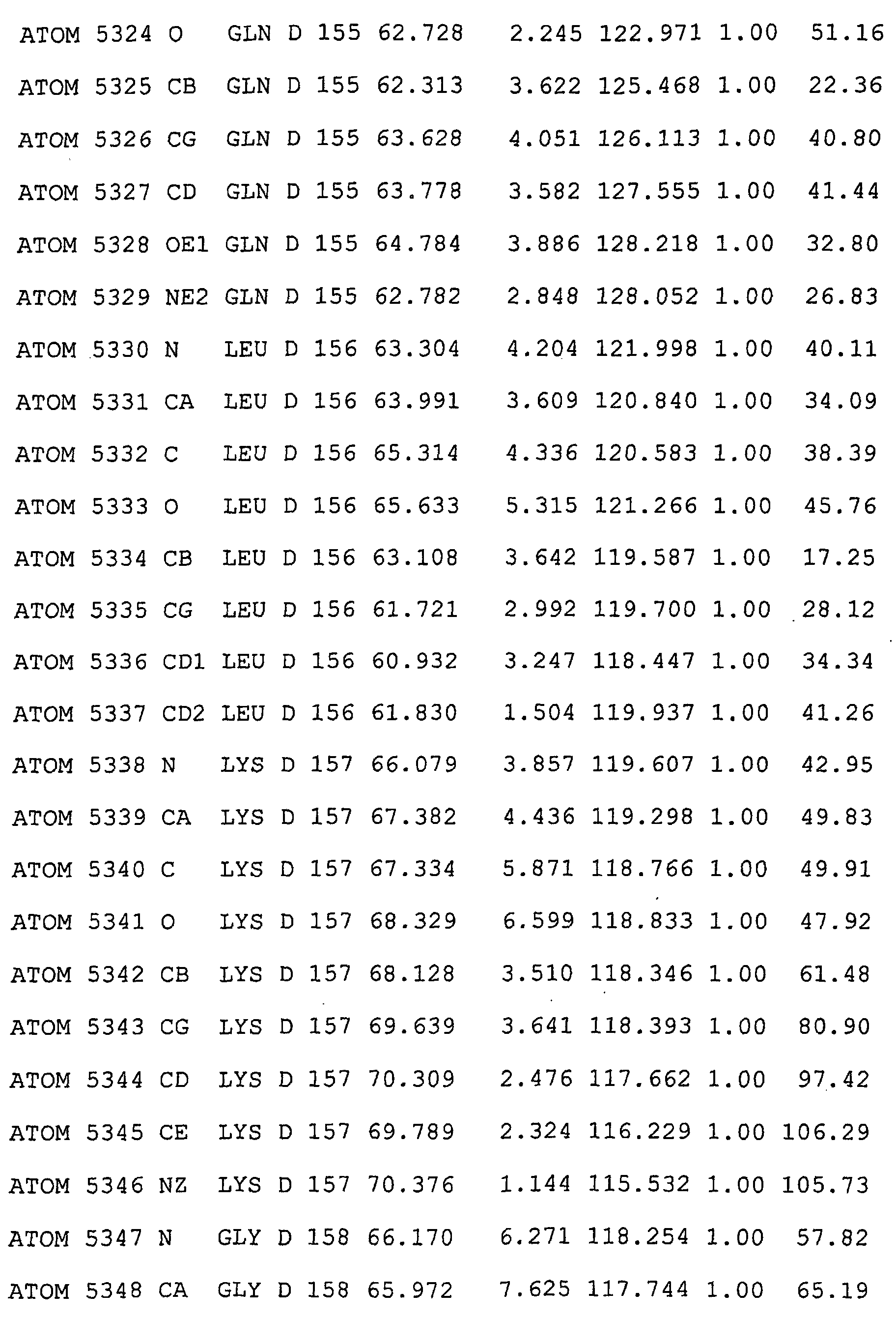

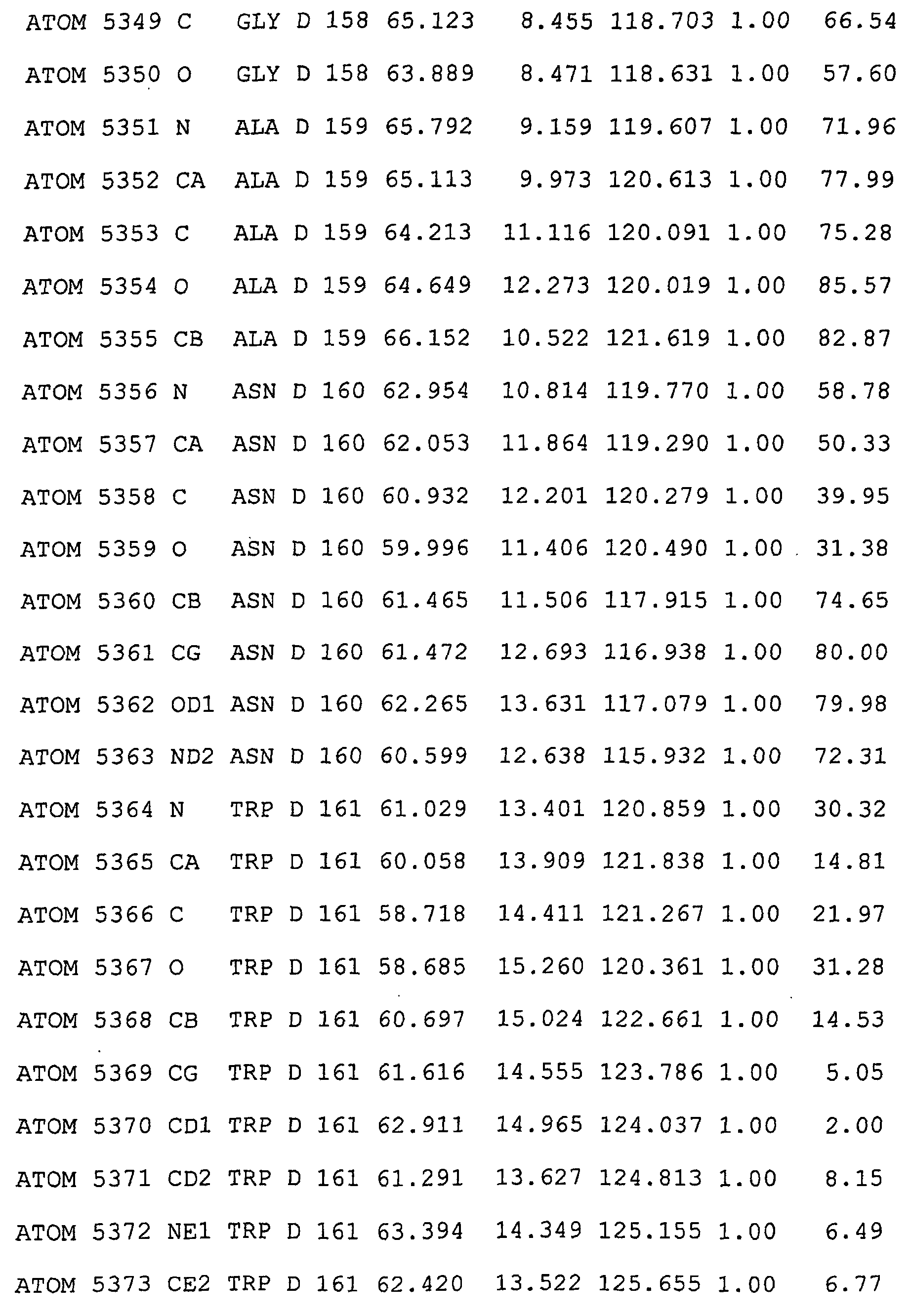

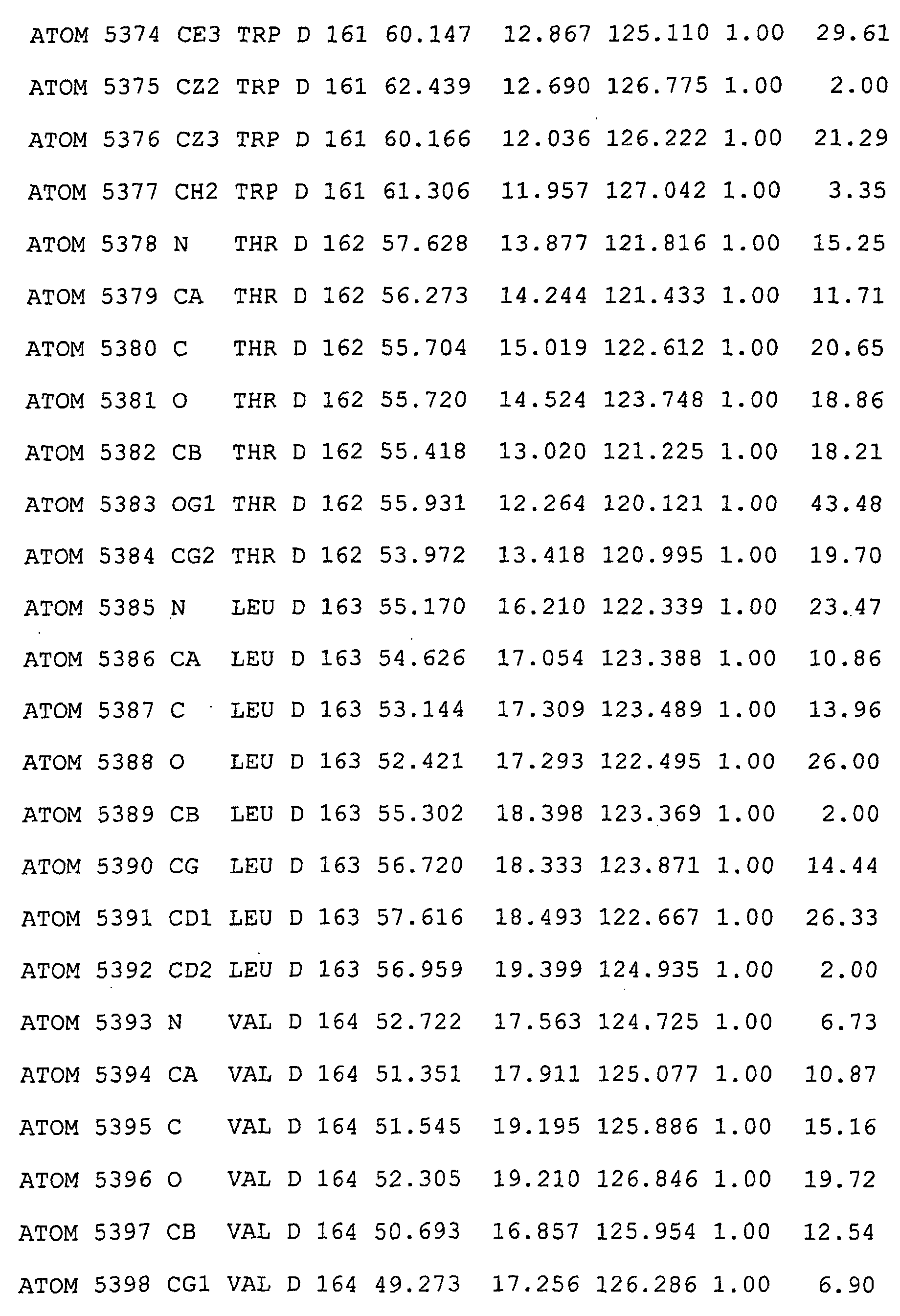

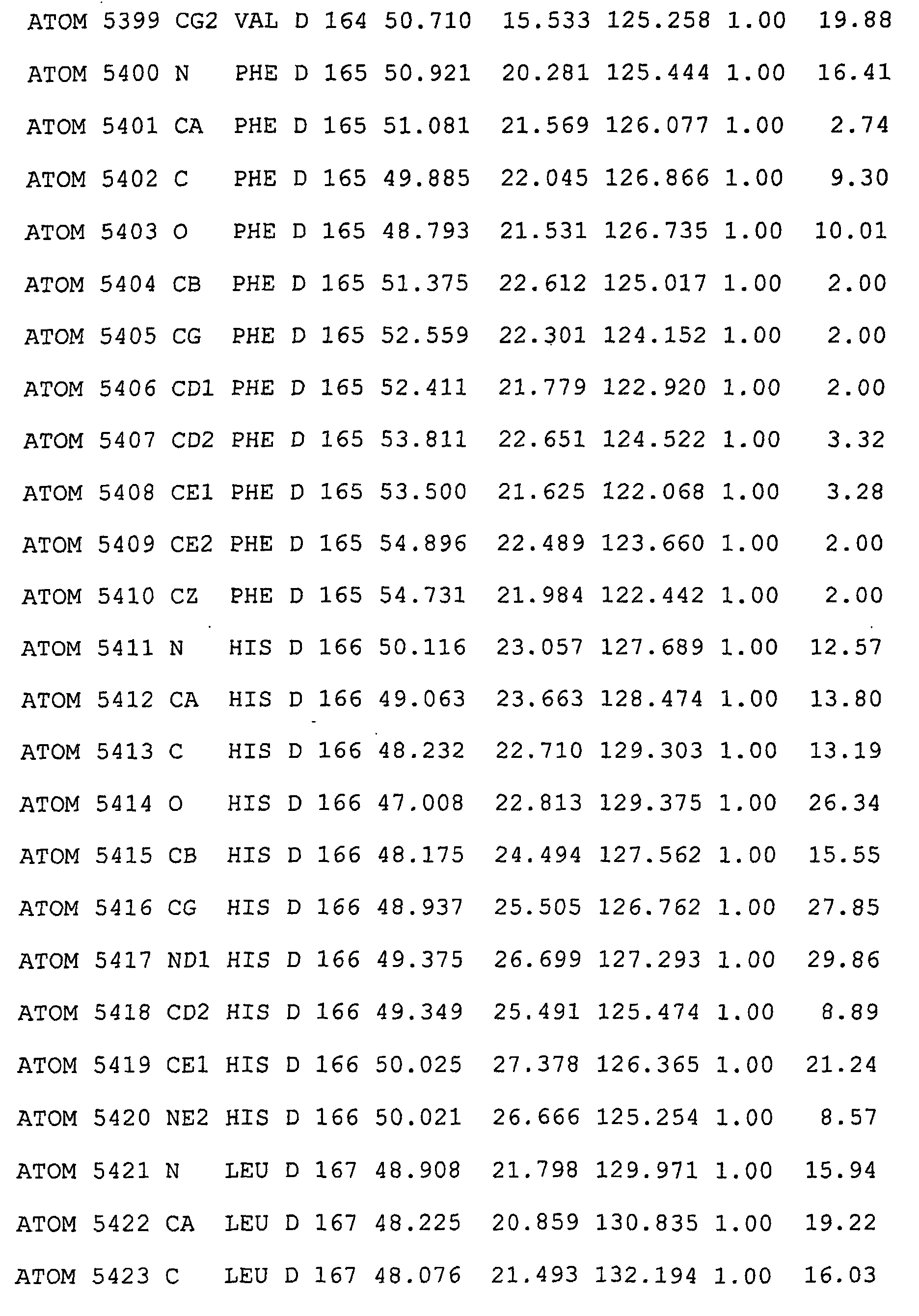

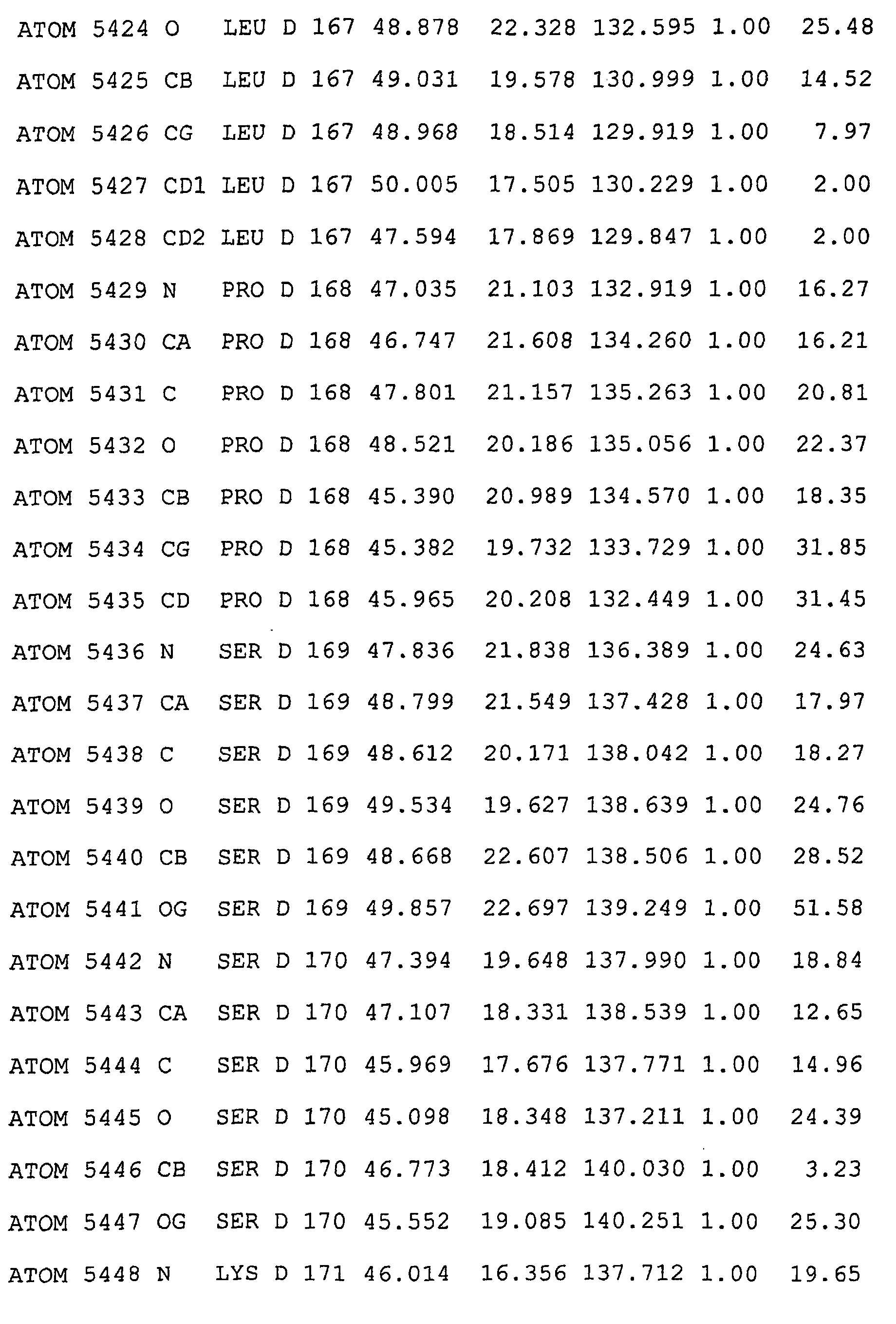

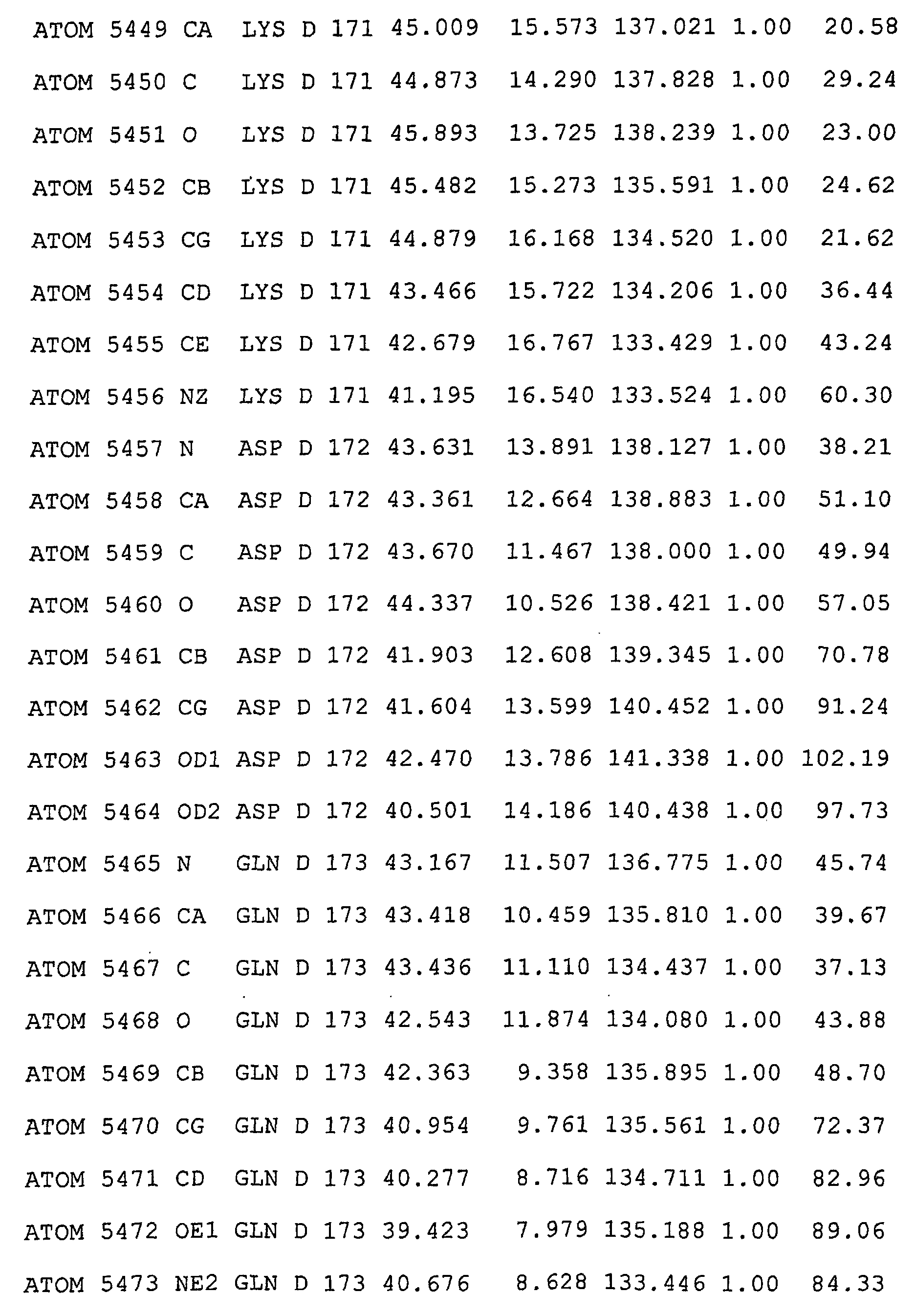

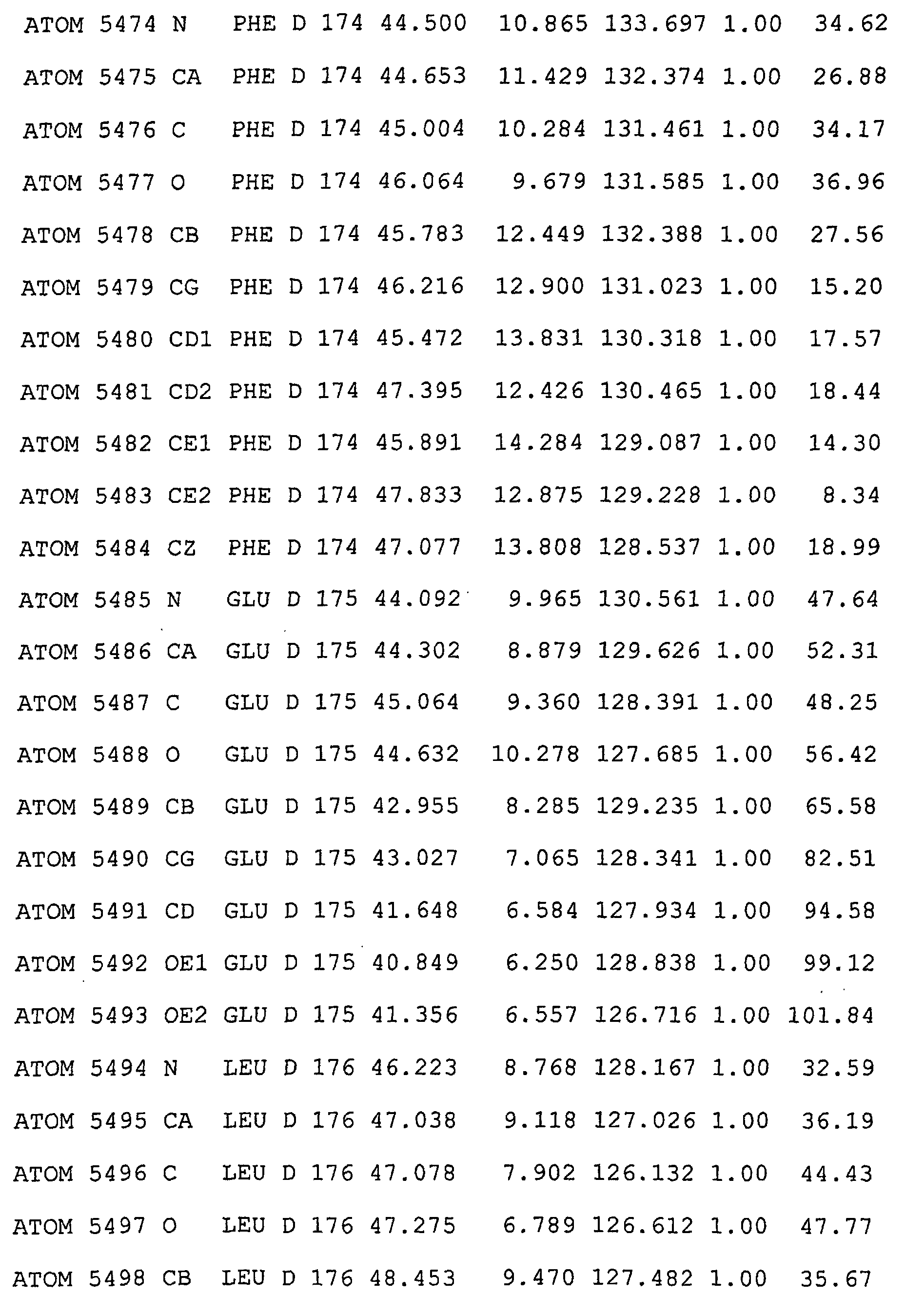

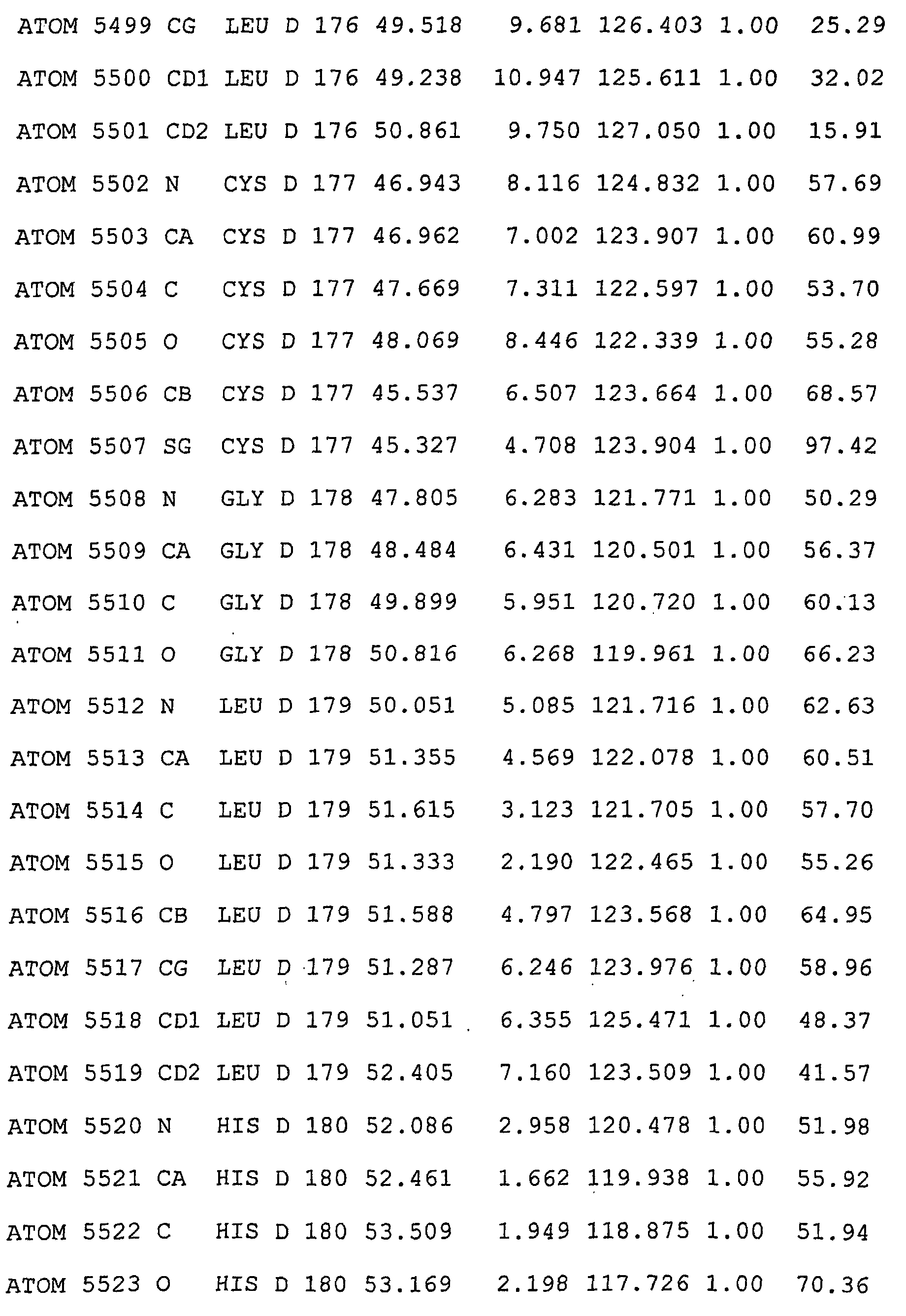

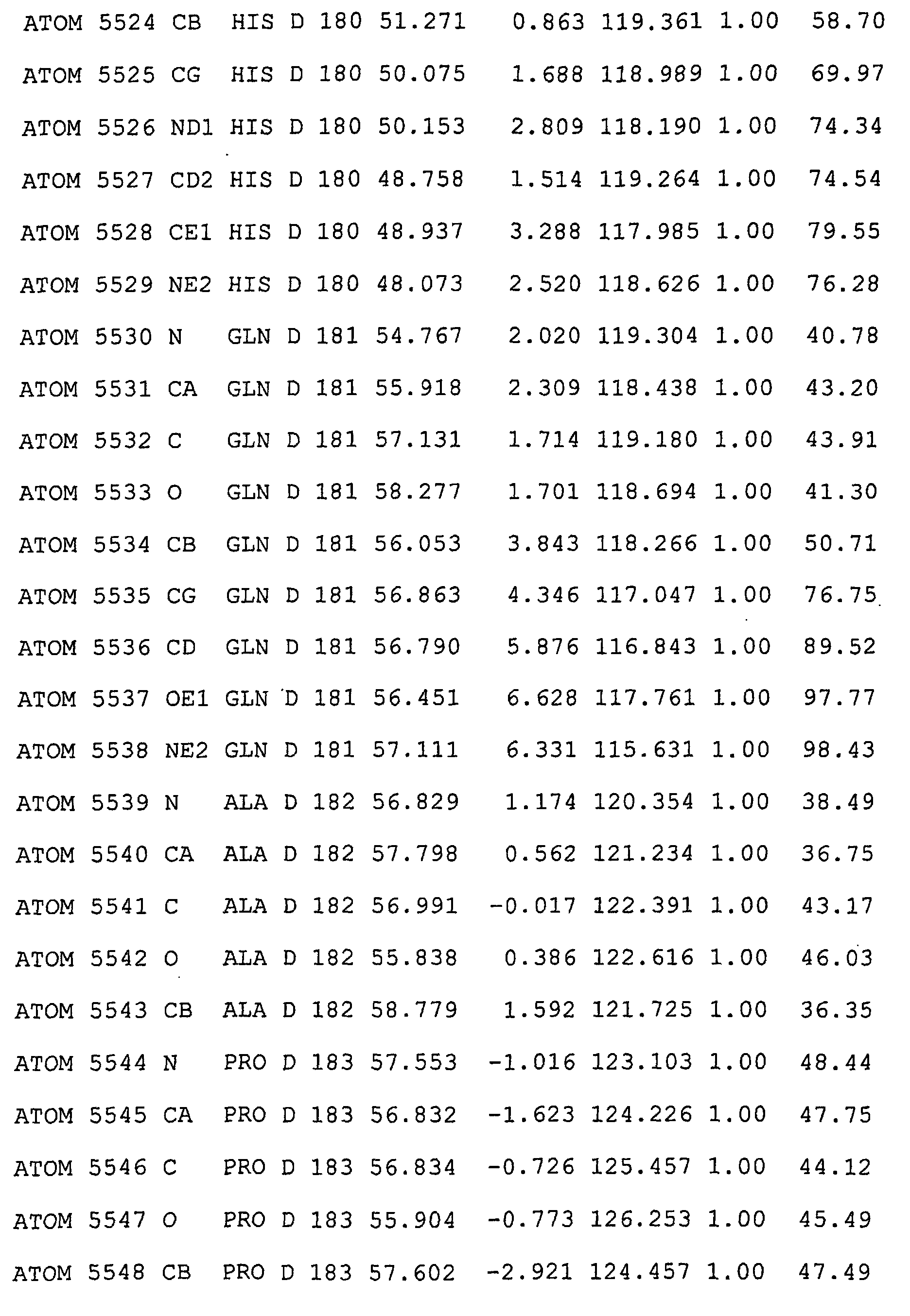

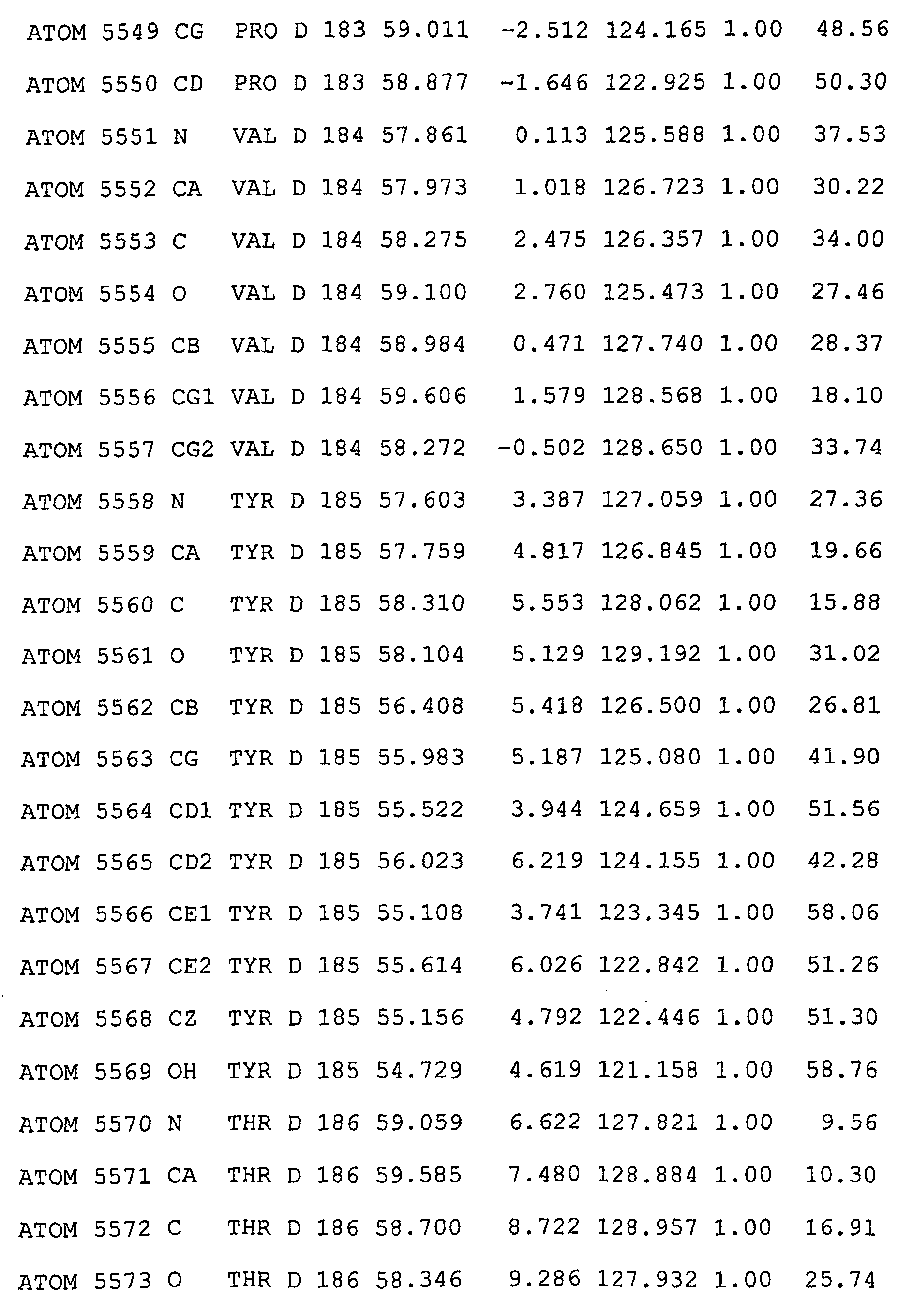

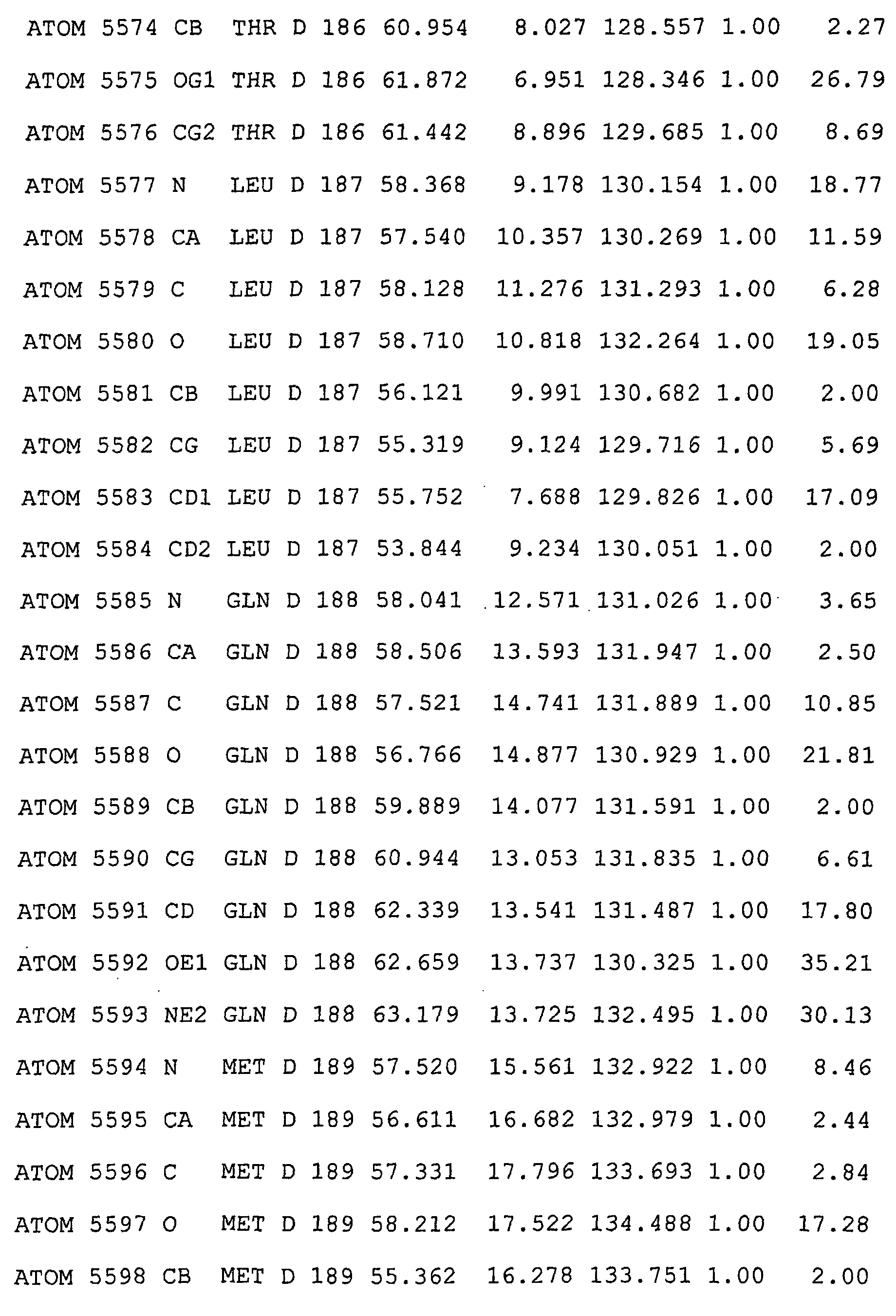

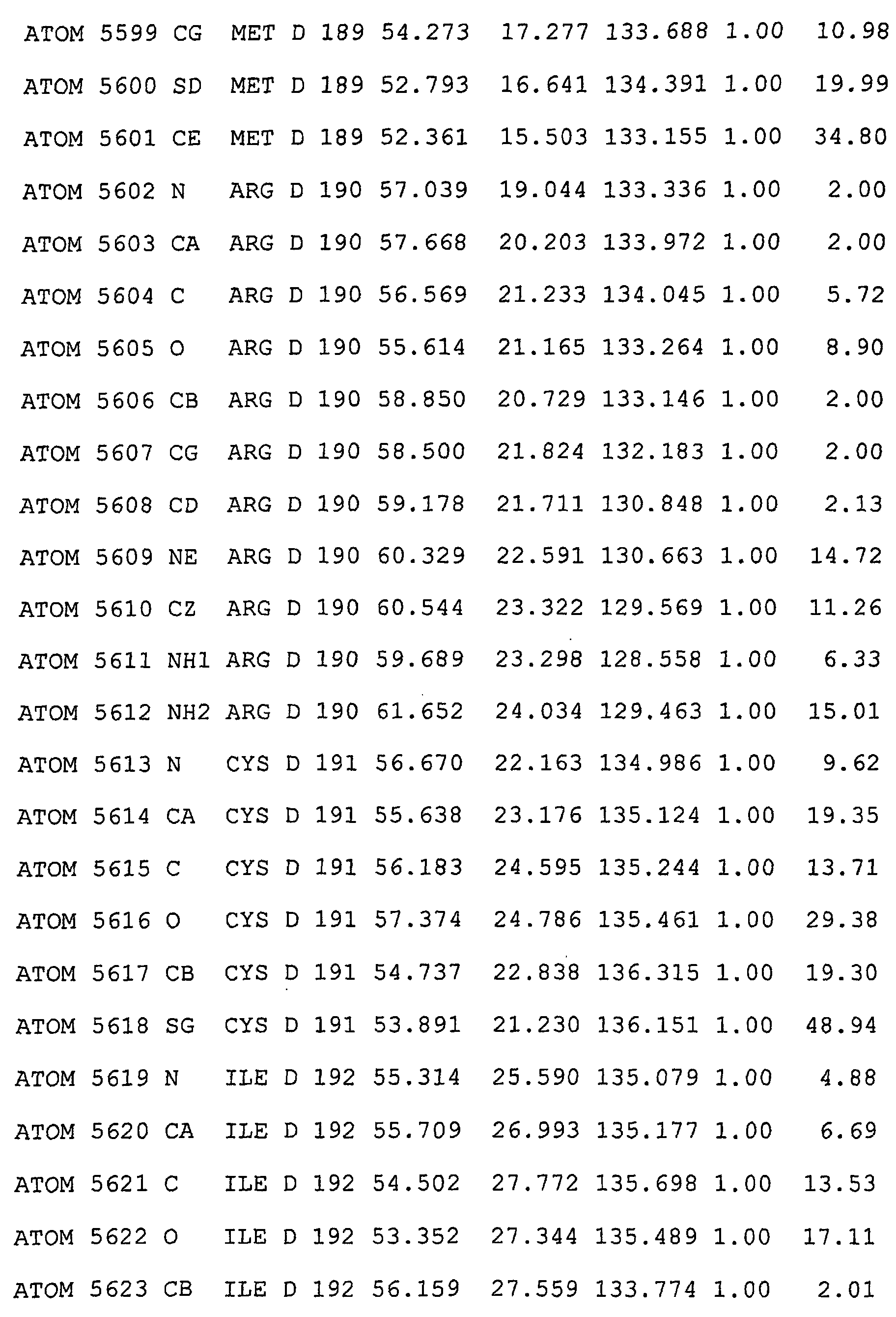

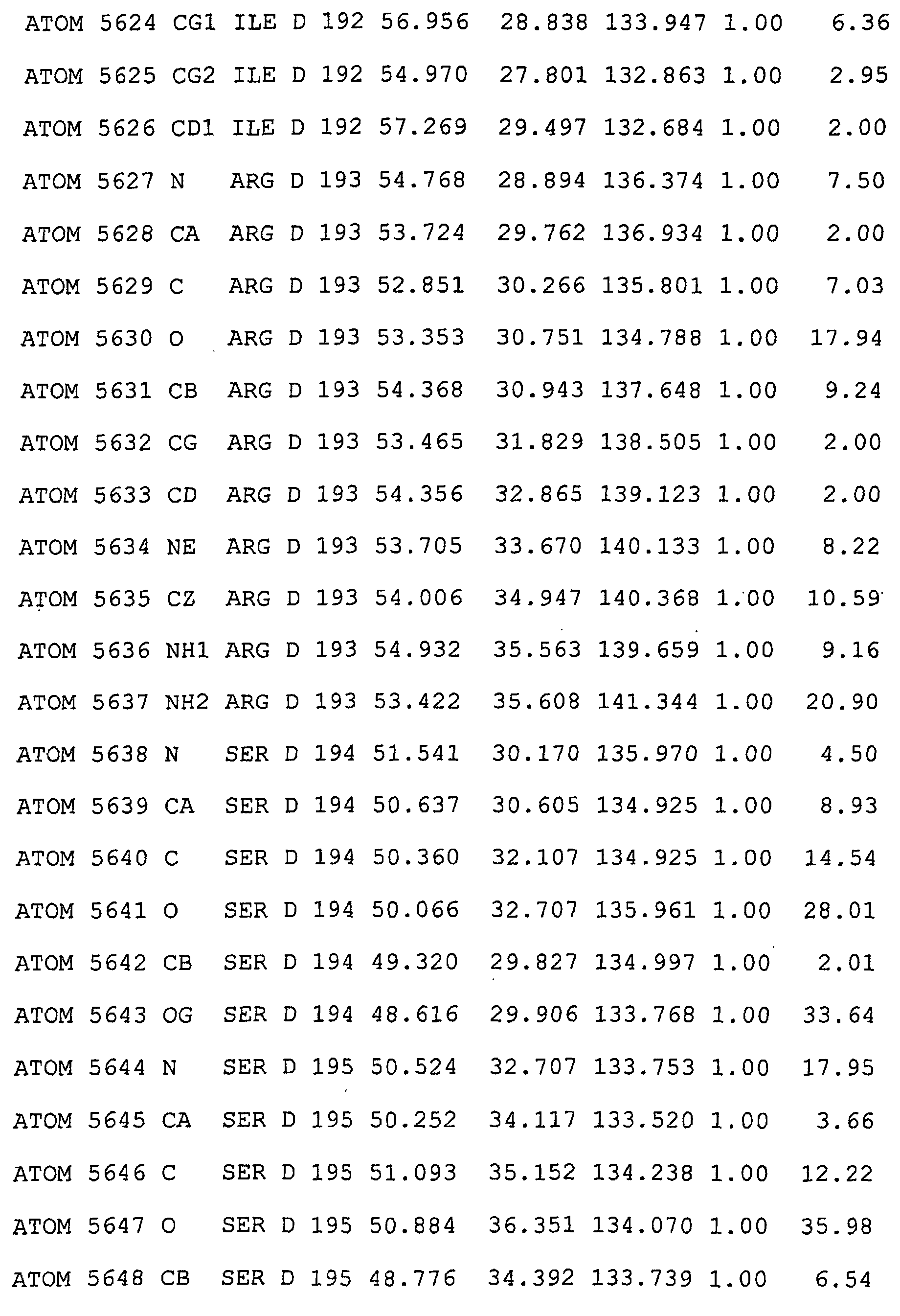

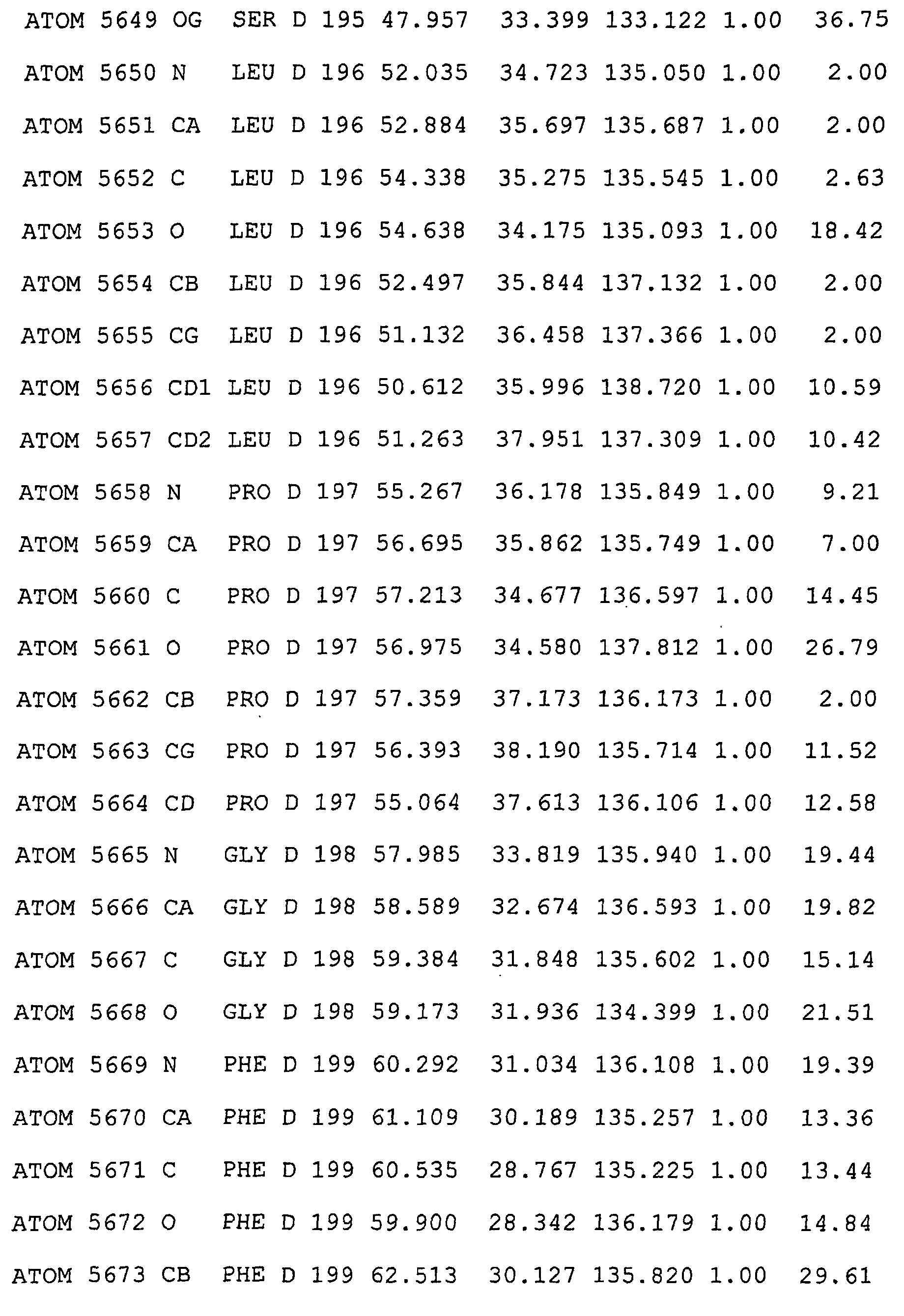

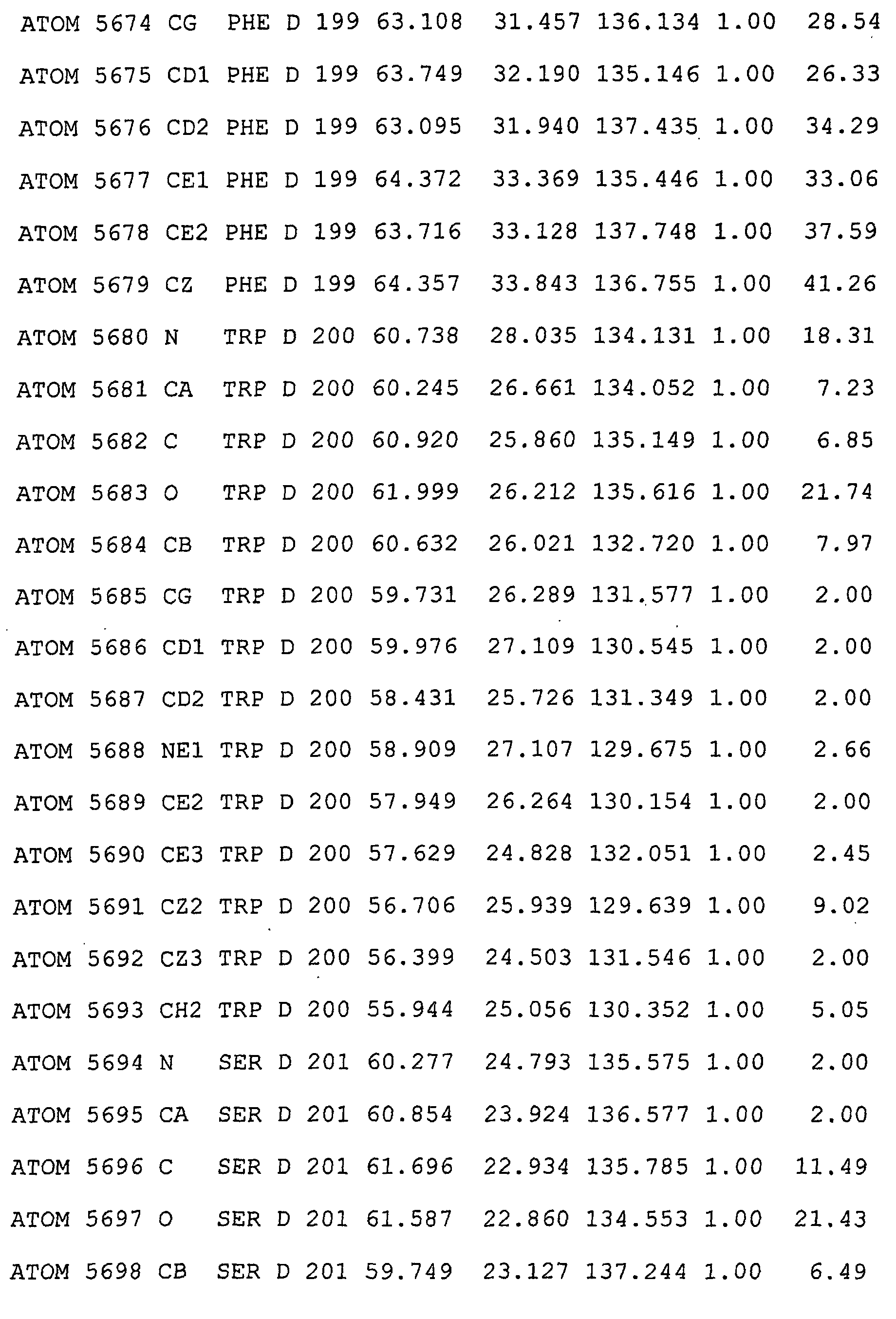

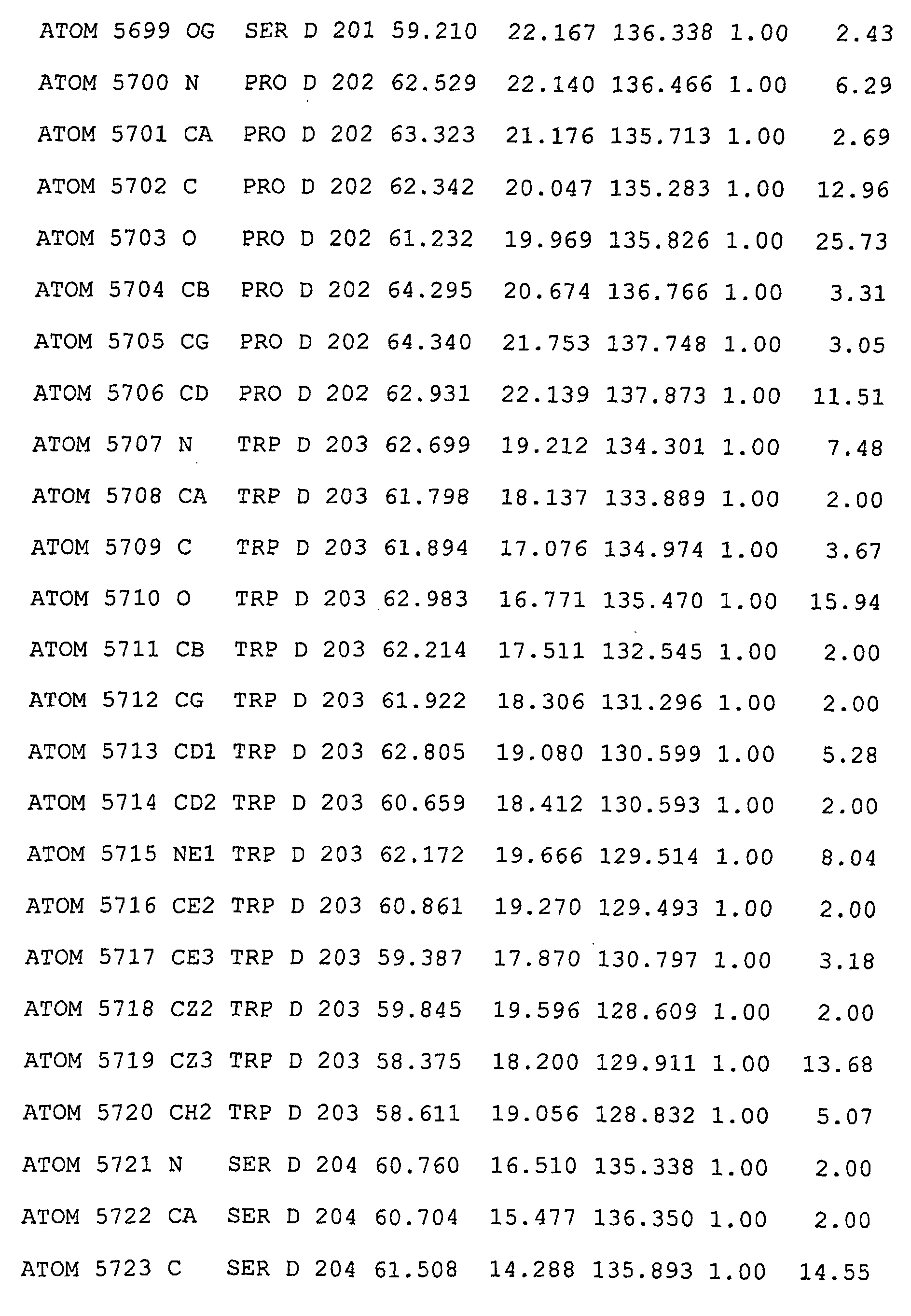

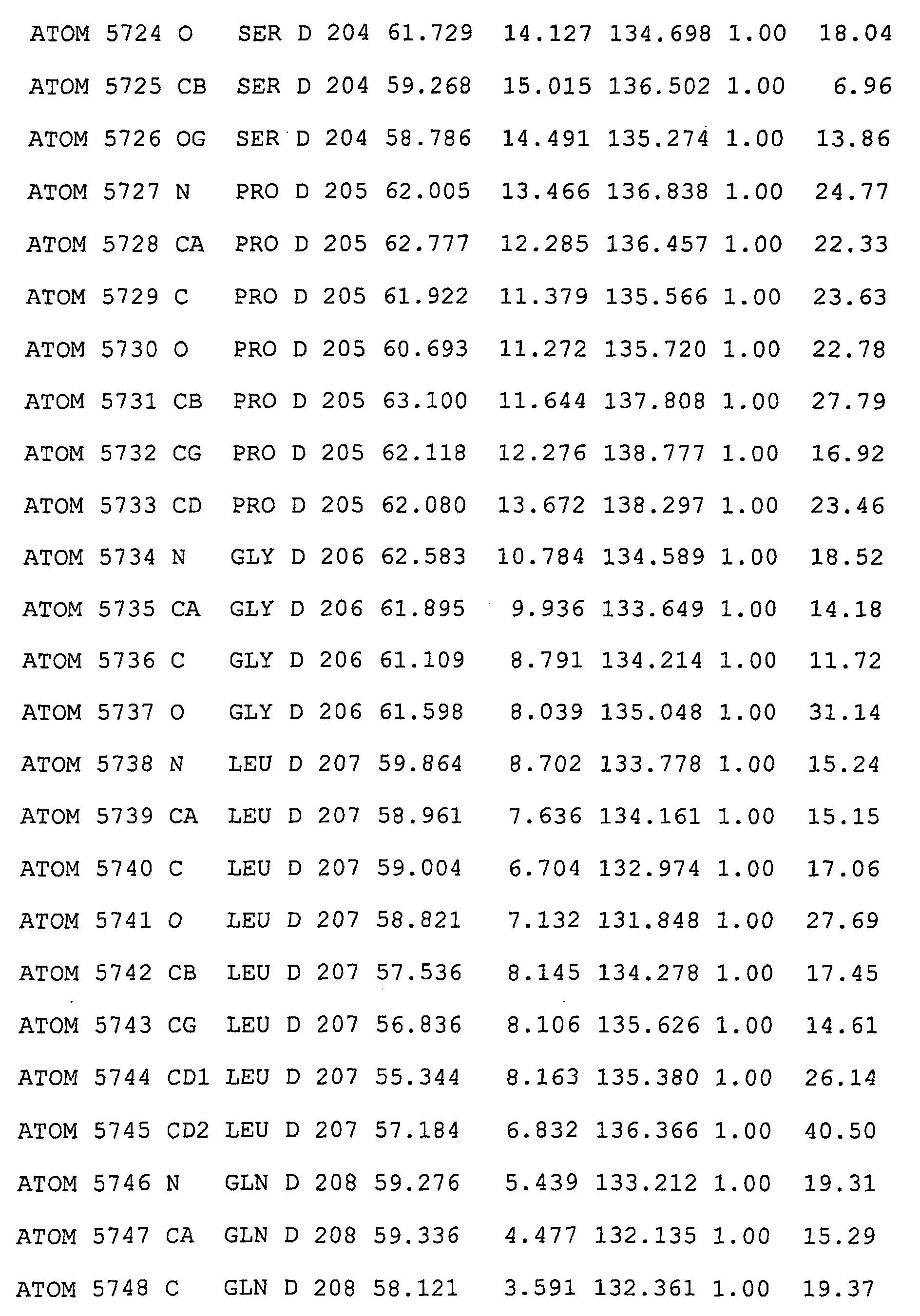

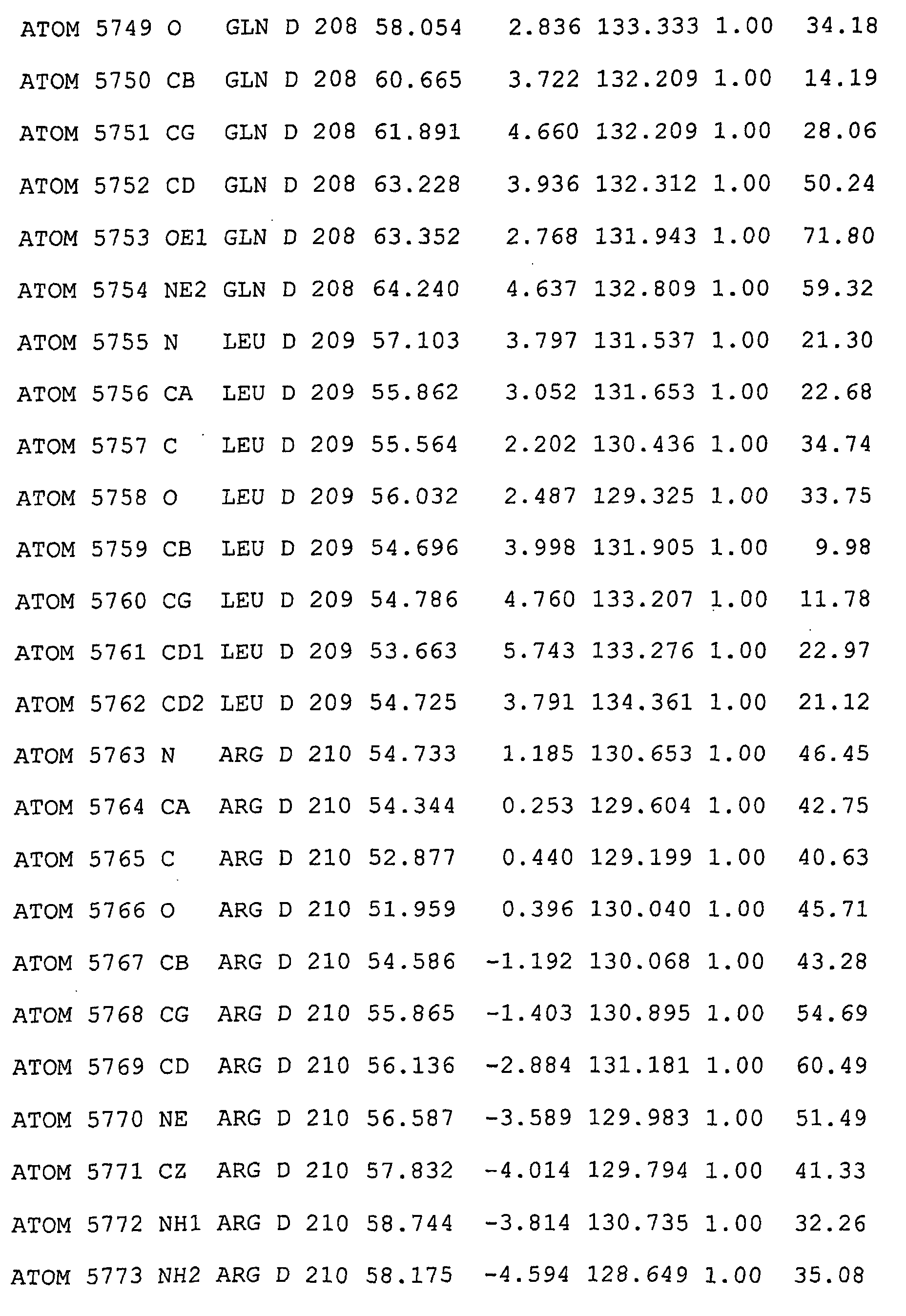

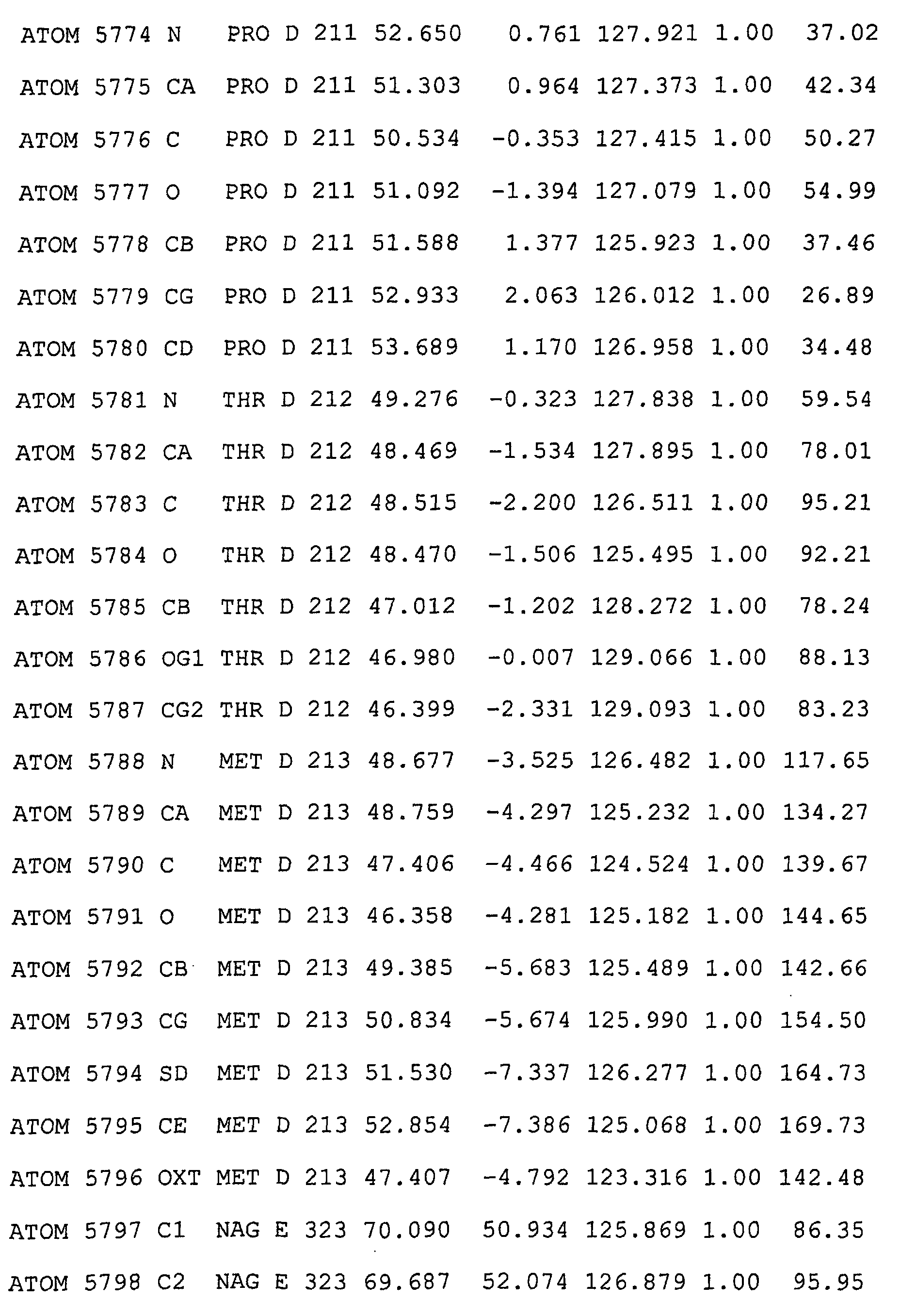

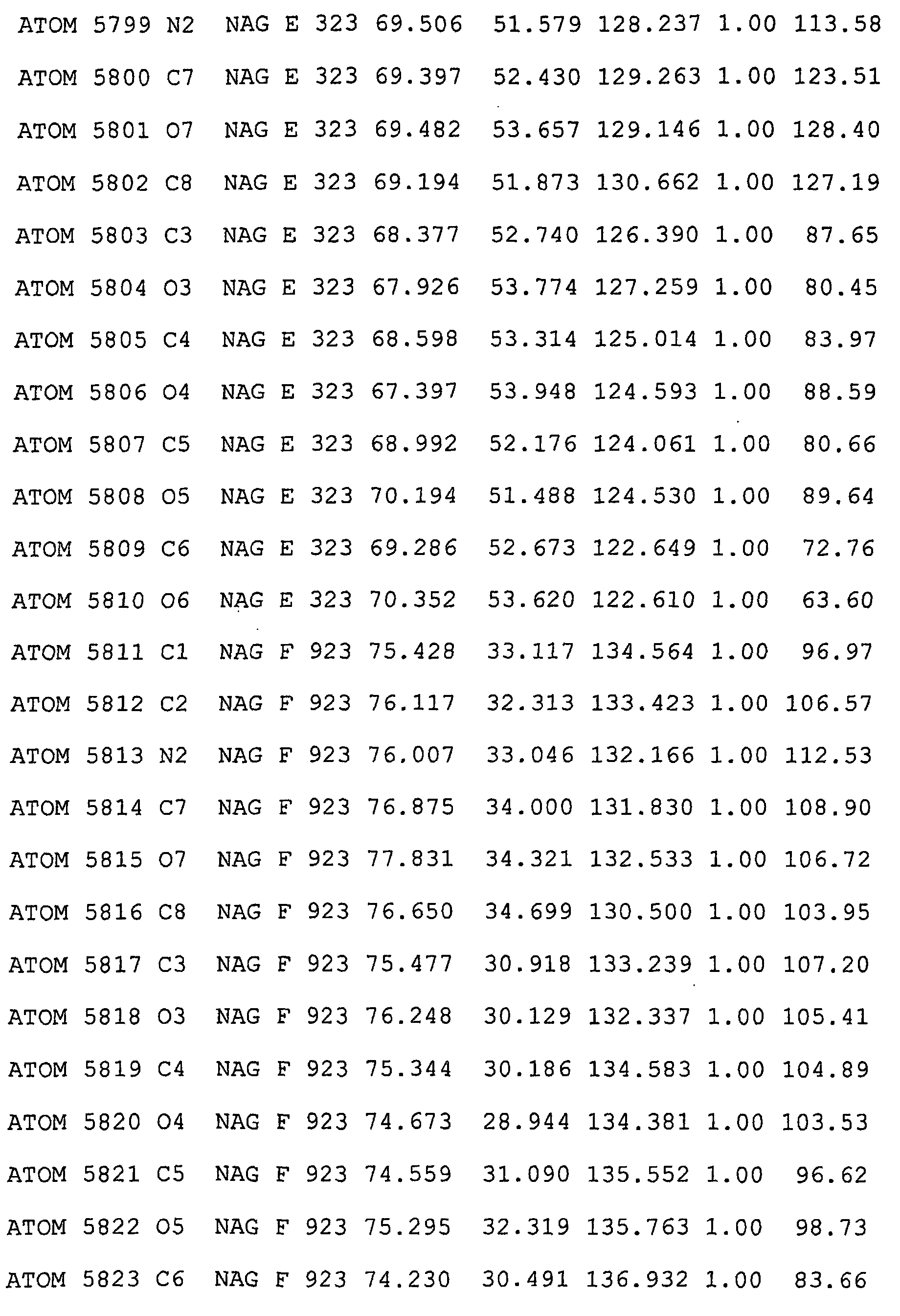

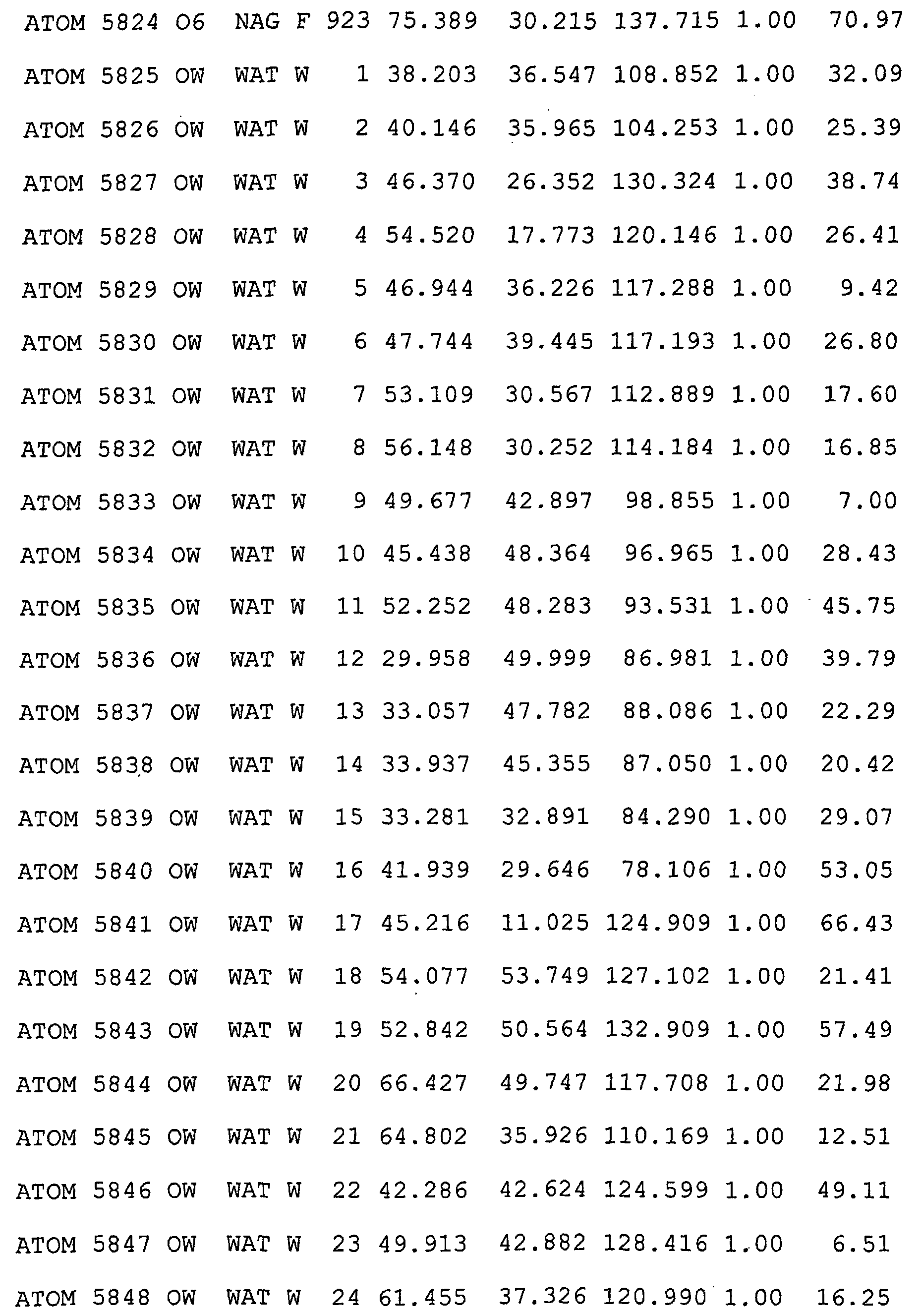

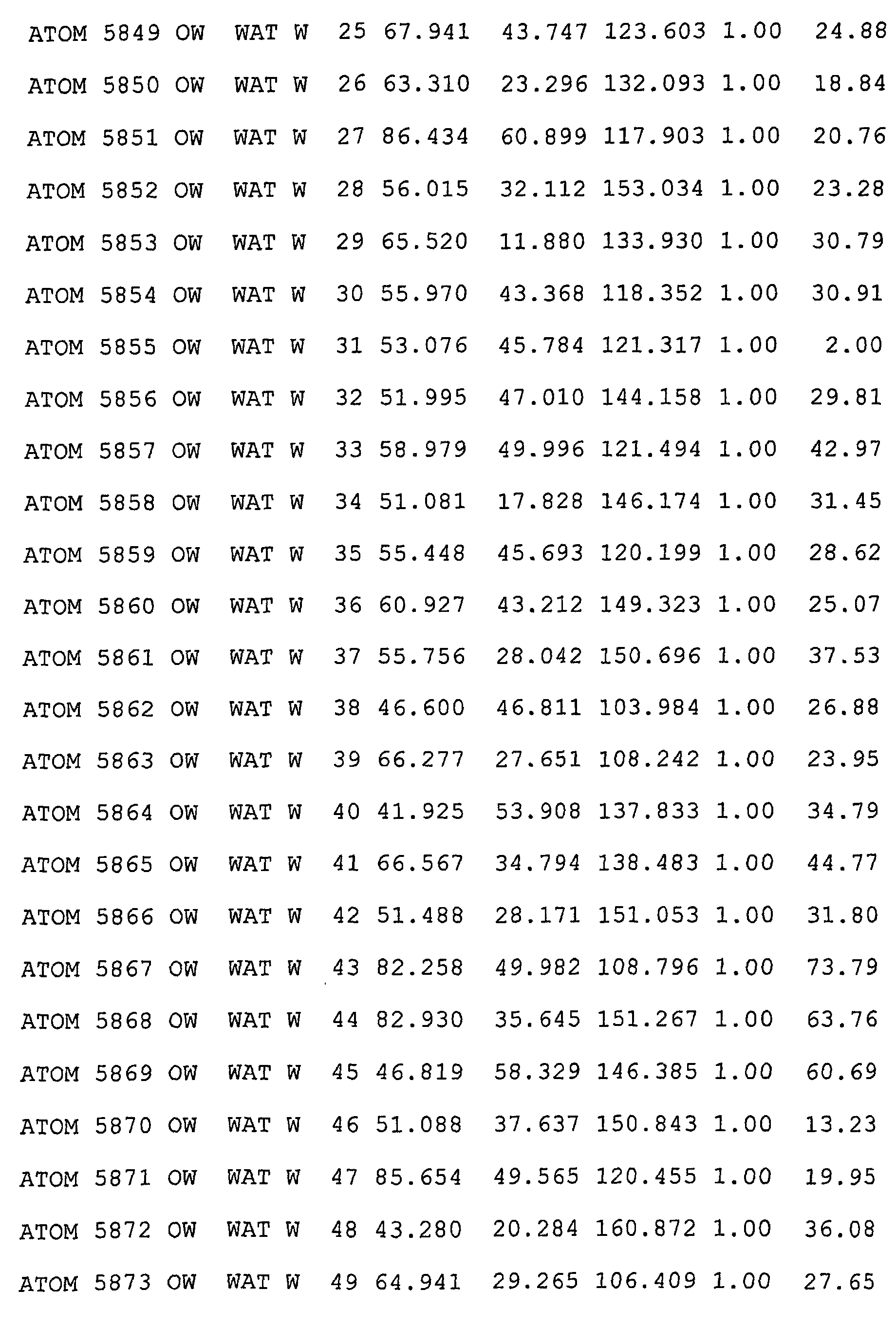

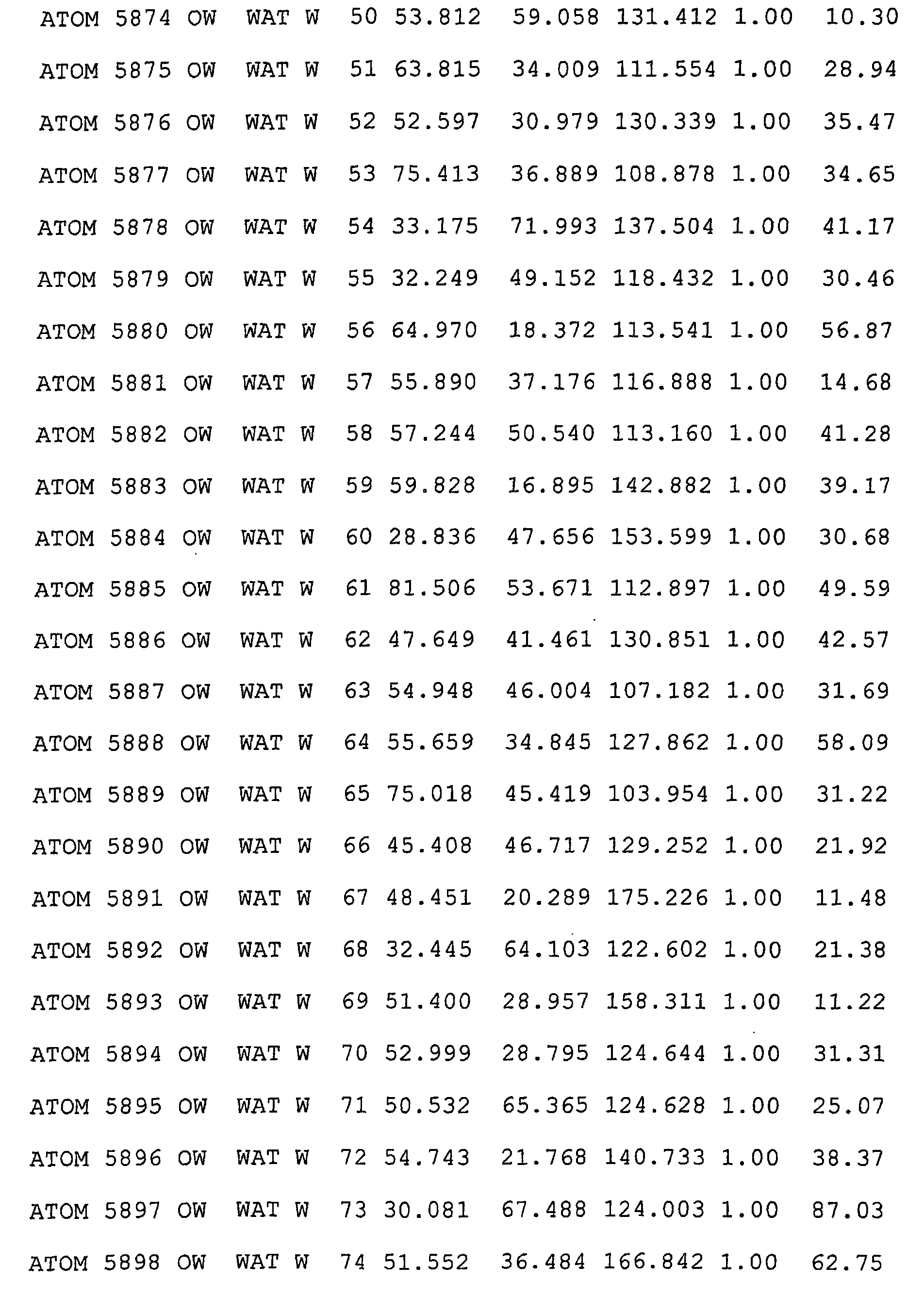

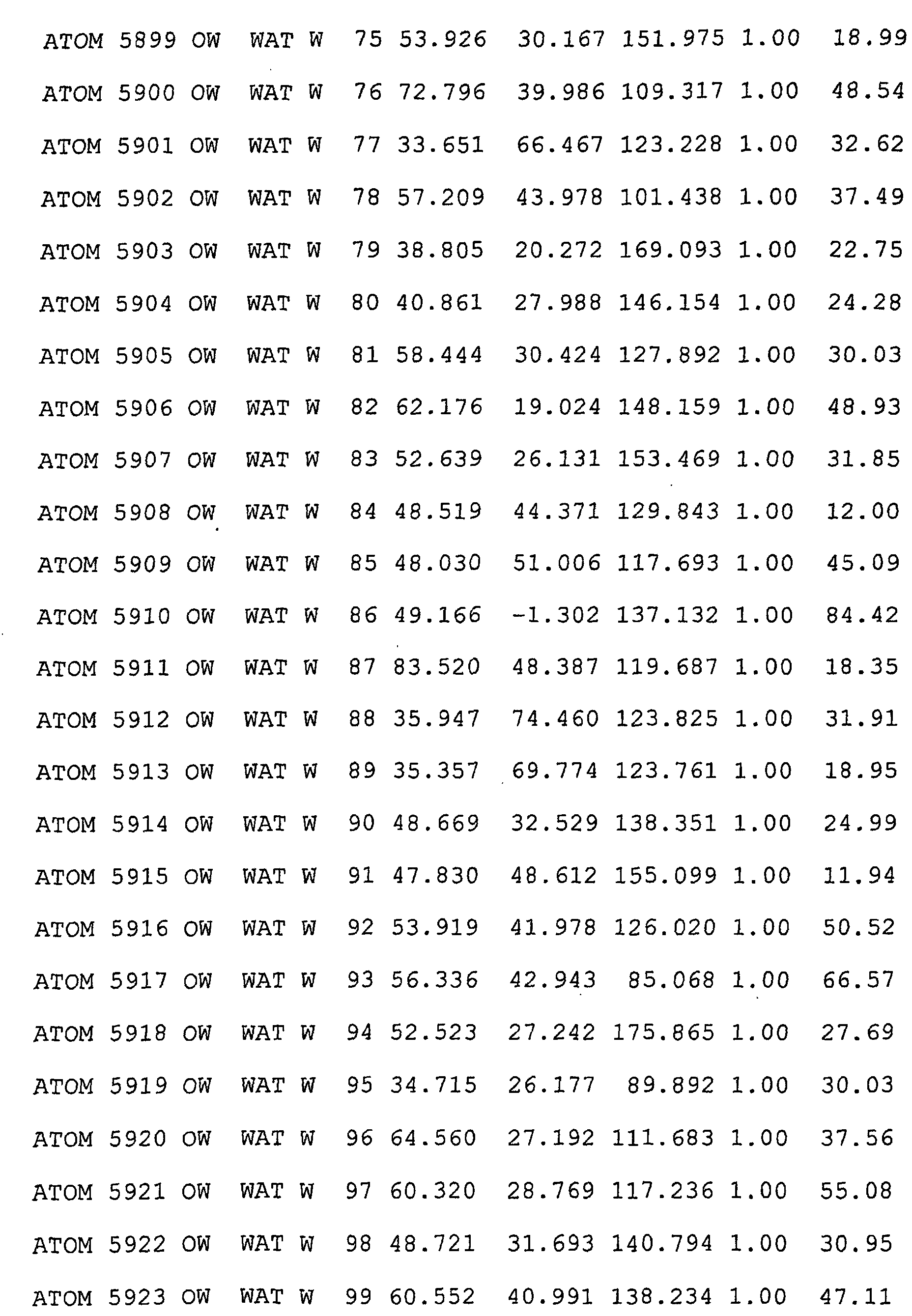

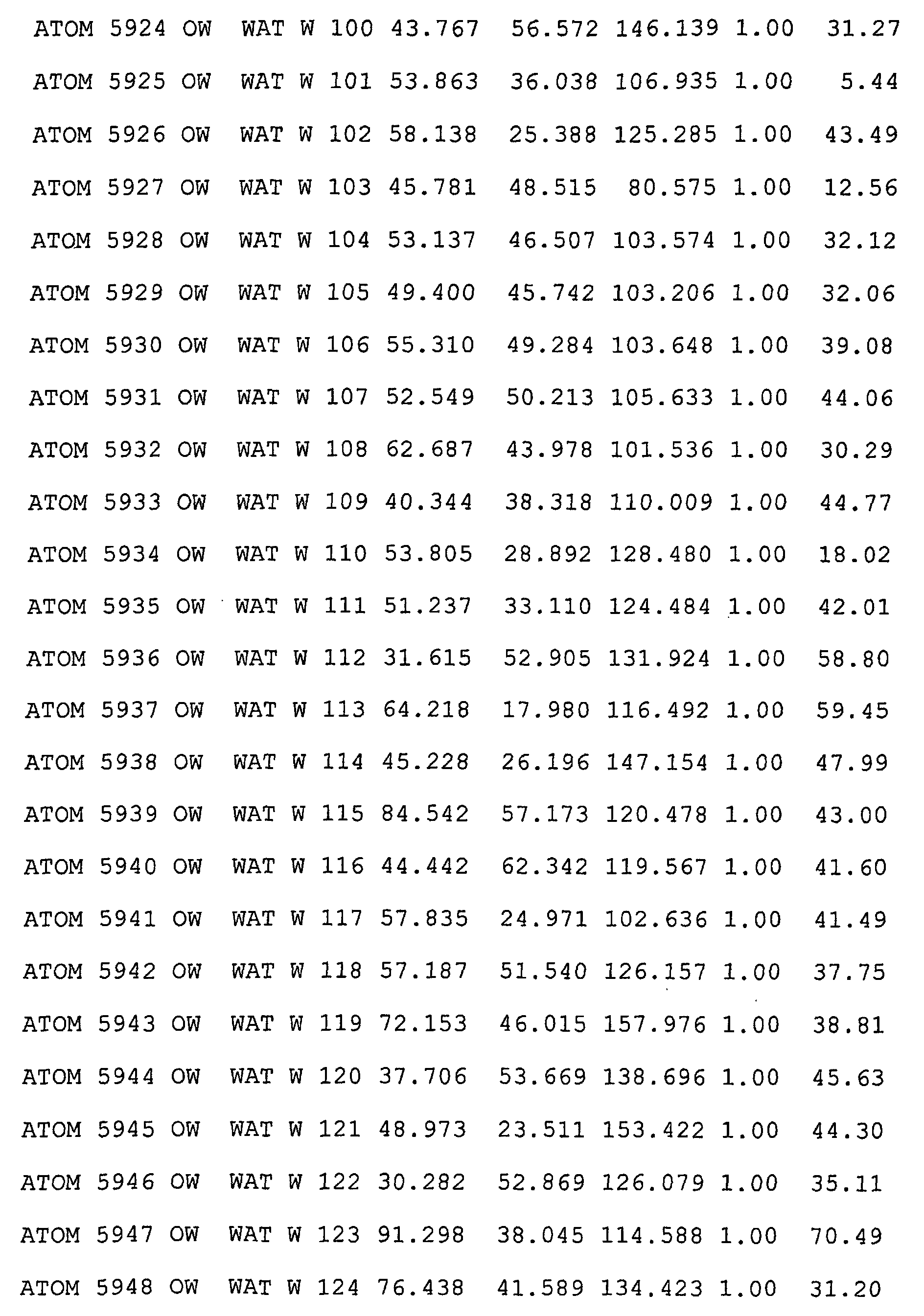

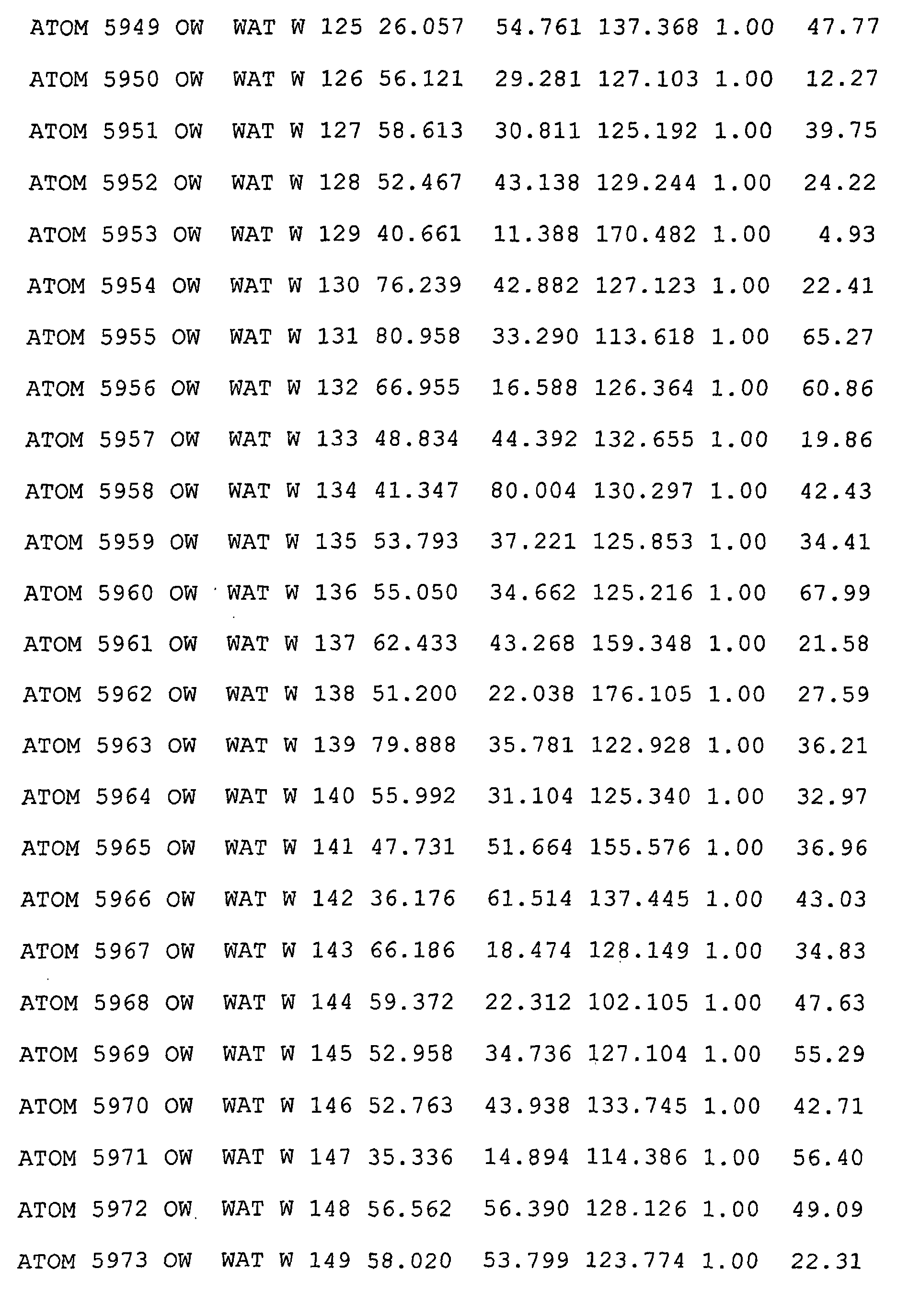

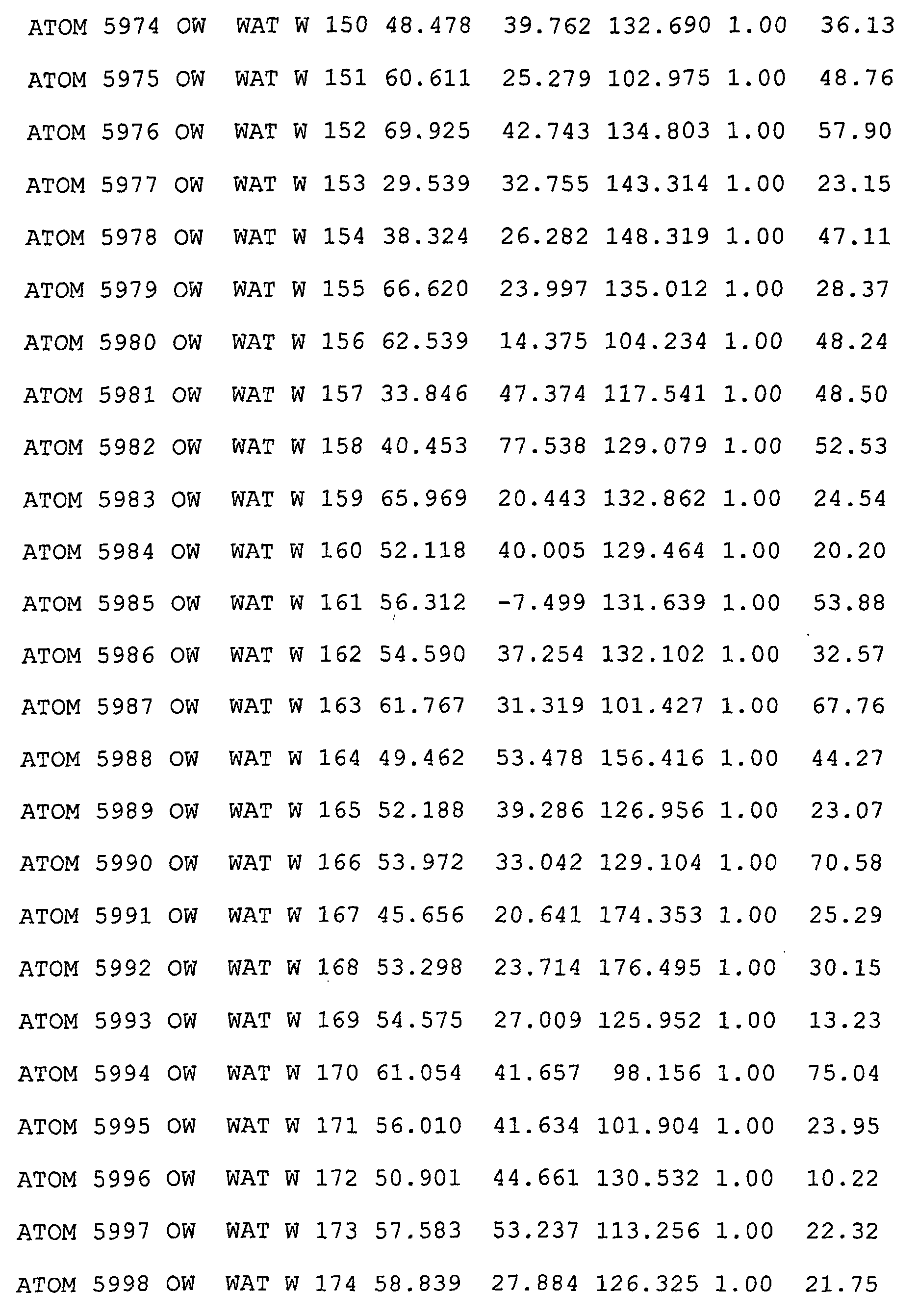

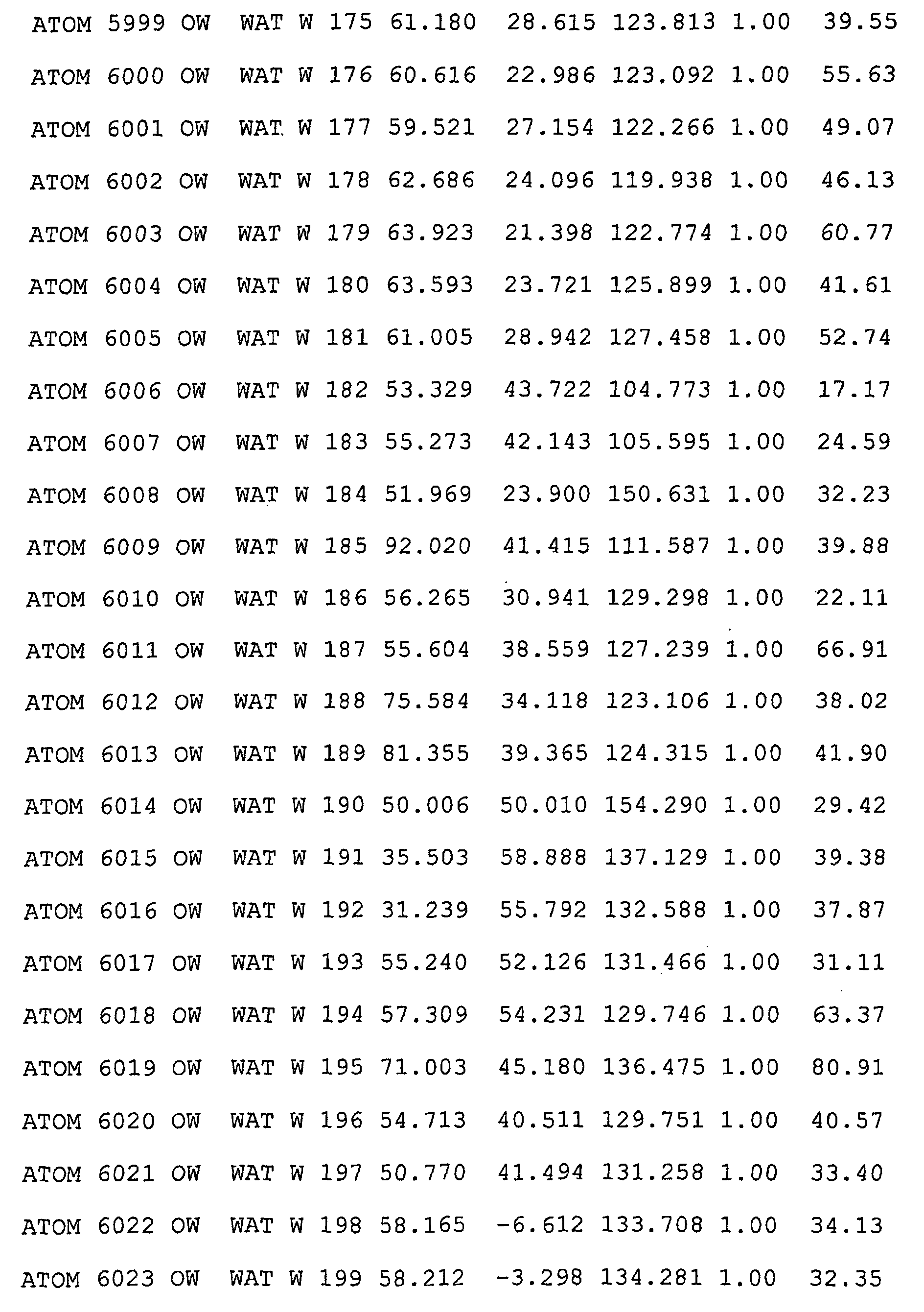

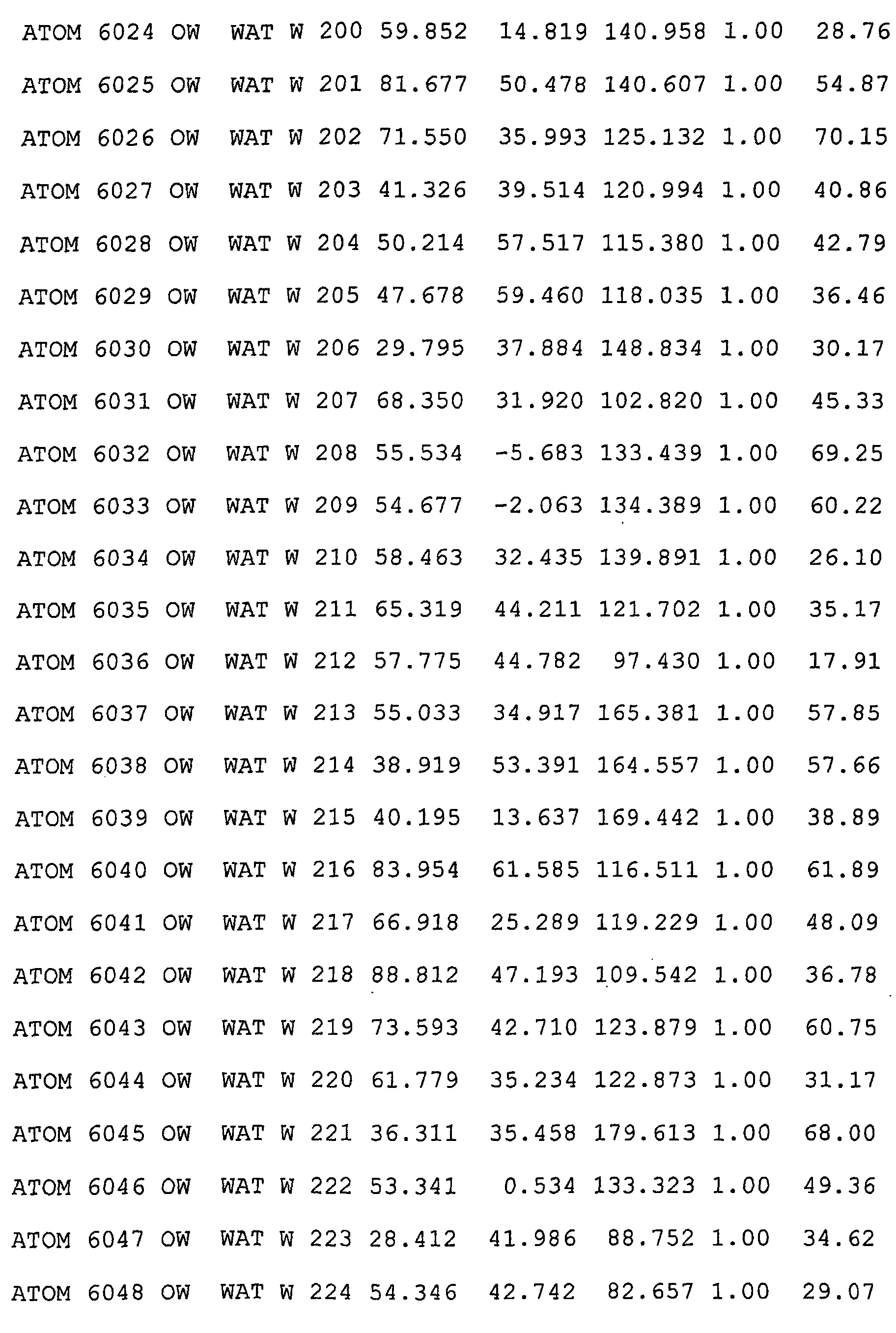

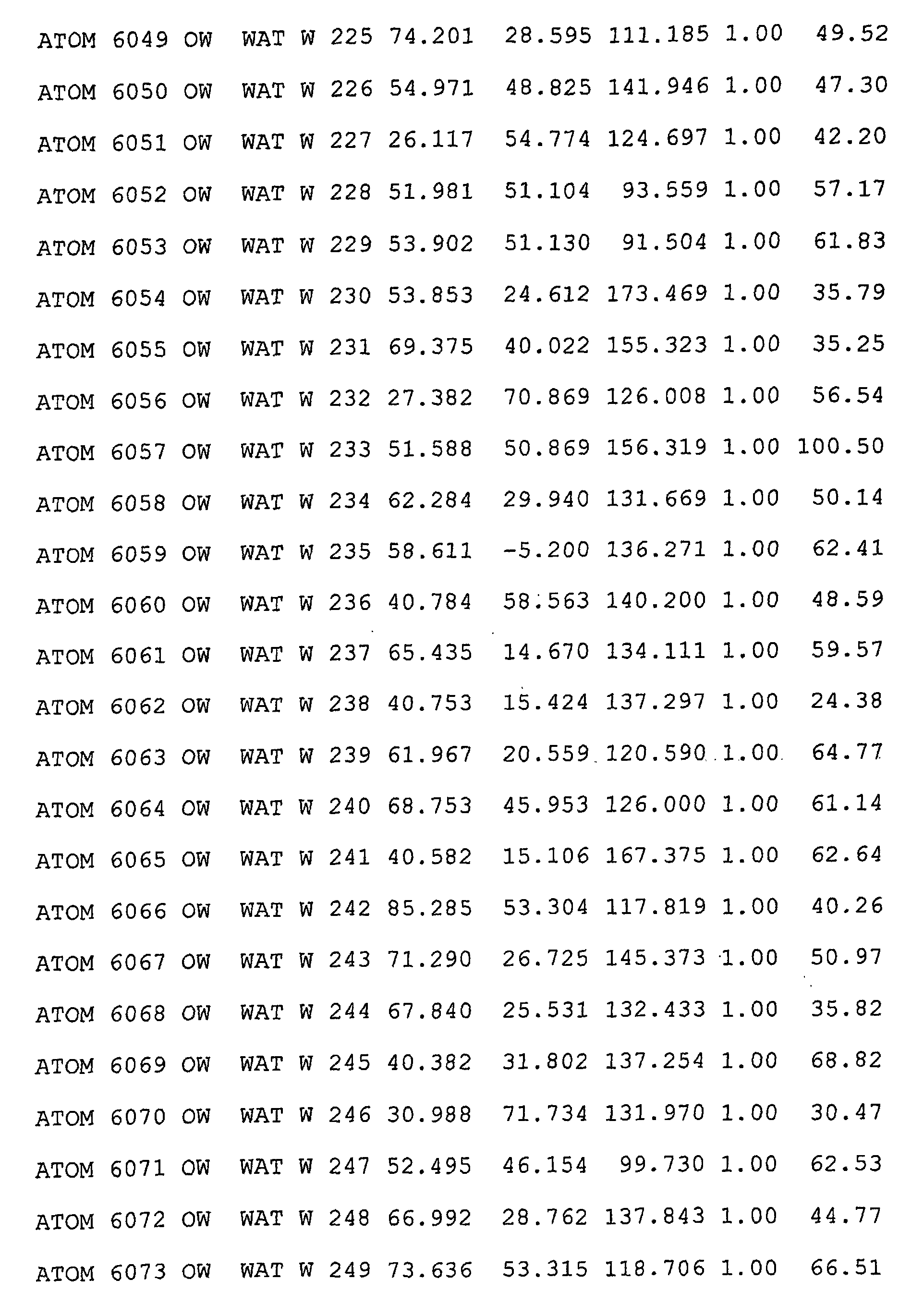

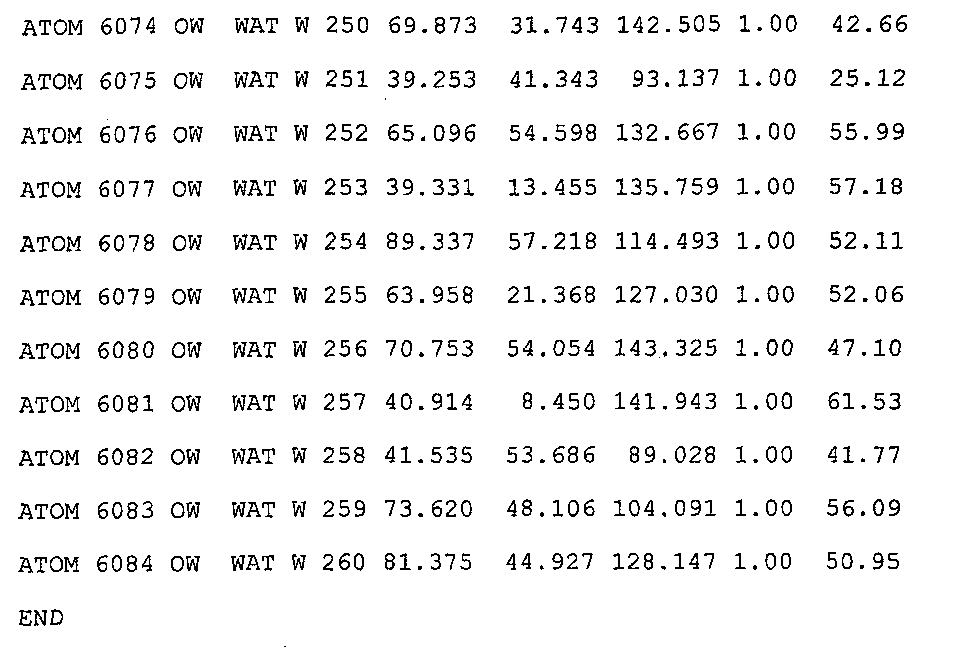

In Table 1, the first row is mathematical descriptions of the crystal and indicates parameters of the unit cell (in Å for a-axis, b-axis, and c-axis directions in the order), the angles between the axes, and the crystal system. The second row through the last row but one describe three-dimensional coordinates for each atom. "ATOM" at the first column indicates that the row describes atom coordinates; the second column indicates the atom number; the third column indicates the atom type in the amino acid residue or the like; the fourth column indicates the amino acid residue or the like; the fifth column indicates the class of molecule (a particular class indicates a single polypeptide); the sixth column indicates the amino acid number or the like according to SEQ ID NOs: 1 and 2; the seventh, eighth, and ninth columns indicate coordinates of the atom (in Å for a-axis, b-axis, and c-axis directions in the order); the tenth column indicates the occupancy of the atom (in the present invention 1.00 for all atoms); and the eleventh column indicates the temperature factor of the atom. The last row indicates that the table ends at this row. In connection with the class of molecule, A and C each indicate one molecule of G-CSF; B and D each indicate one molecule of CRH-G-CSF-R; E and F indicate sugar chains linked to B and D, respectively; and W indicates water molecule found in the crystal. In the fourth column, abbreviations other than those for amino acid residues have the following meanings: NAG, N-acetylglucosamine residue; and WAT, water molecule. This table is described according to the format of Protein Data Bank, a notation system commonly used in the art.

The structure coordinates show that one molecule of G-CSF (A) and one molecule of CRH-G-CSF-R (B) form a complex (A-B), and another molecule of G-CSF (C) and another molecule of CRH-G-CSF-R (D) form a complex (C-D). It is also found that the complex (A-B) and the complex (C-D) are associated to form an associate consisting of two molecules of the complex around a non-crystallographic pseudo-twofold axis. In the present specification, such an associate of the complexes may also sometimes be simply referred to as a "complex".

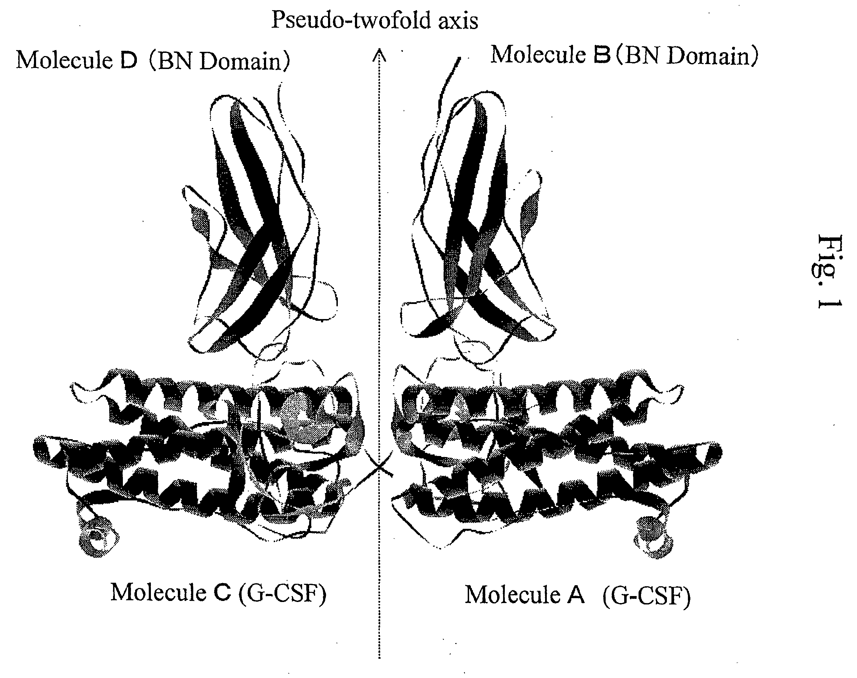

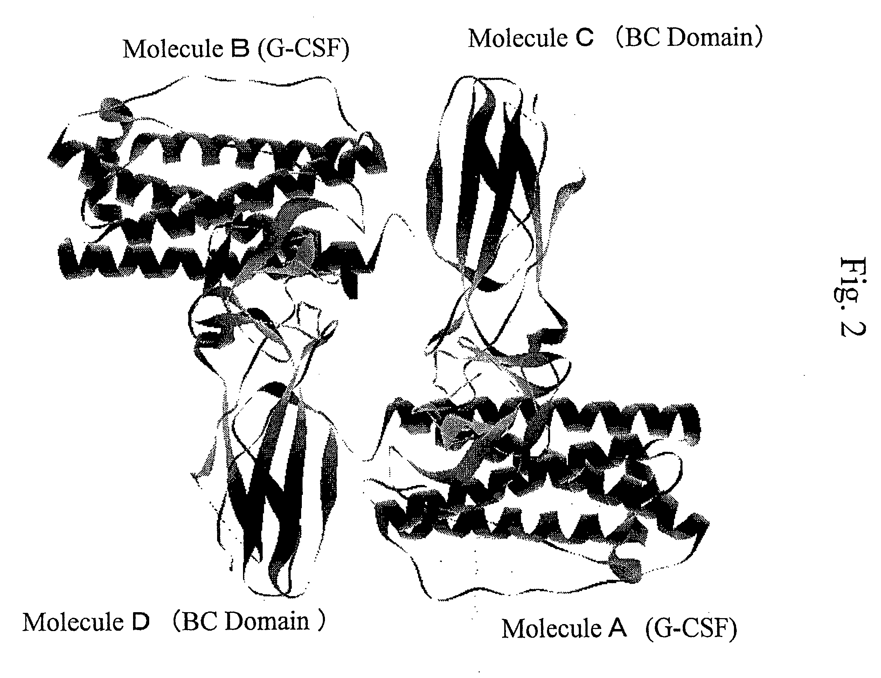

On the basis of the structure coordinates obtained from a crystal of the complex between G-CSF and CRH-G-CSF-R, a ribbon drawing (a representation method commonly used in the art for understanding a structure of a protein) showing the structure viewed from a direction orthogonal to the pseudo-twofold axis and a ribbon drawing showing the structure viewed from a direction parallel to the same axis are shown in Figs. 1 and 2, respectively.

As can be seen from these figures, the crystal of the complex between G-CSF and CRH-G-CSF-R contains two molecules of the complex (A-B and C-D) in the minimal unit in which no crystallographic symmetry exists (i.e., an asymmetric unit). These two molecules of the complex are macroscopically related by a non-crystallographic pseudo-twofold axis. The G-CSF portion consists of 4 long α-helixes, and 1 short α-helix, and loop regions connecting the helixes. The CRH-G-CSF-R portion is broadly divided into two regions, each of which consists of about 7 β-sheets, and also contains loop regions connecting the sheets. In the following descriptions, one of these two regions that is closer to the N-terminus is referred to as BN domain and the other which is closer to the C-terminus is referred to as BC domain. Based on these three-dimensional structures, it can be understood that receiving of G-CSF signal by G-CSF-R represents binding of G-CSF to G-CSF-R, that is, formation of an associate between two molecules of the complex in the relative positions shown in the figures.

It has been shown that G-CSF and the extracellular region of G-CSF-R form a complex consisting of stoichiometrically equivalent quantities of the components, and the complex may exist as a dimer or a tetramer (Horan, T. P. *et al*., *J. Biochem. (Tokyo),* **121:**370-375 (1997); Horan, T. P. *et al*., *Biochemistry,* **35:**4886-4896 (1996); Hiraoka, O. et *al*., *J. Biol. Chem.,* **270:**25928-25934 (1995); and Hiraoka, O. *et al*., *FEBS Lett.,* **356:**255-260 (1994)). It is considered that such facts are reflected in the associate demonstrated in the present invention which is formed by two molecules of G-CSF and two molecules of CRH-G-CSF-R.

Amino acid residues M1 to L4 and side chain portions of Q71 and L131 in G-CSF Molecule A; amino acid residues M1 to P6 and side chain portions of H53, W59, Q68, L70, and Q71 in G-CSF Molecule C; amino acid residues G120 to H126 and K214 to A215 and side chain portions of K63 and K64 in CRH-G-CSF-R Molecule B; and amino acid residues of A1, I119 to H126, and K214 to A215 and side chain portions of K62, K63, R64, Q127 in CRH-G-CSF-R Molecule D were excluded from considerations in X-ray crystallography, because their positions were disordered even in the crystal.

In this context, crystal structures comprising G-CSF variants and/or G-CSF-R variants which are substantially consistent with the crystal structure according to the present invention are within the scope of the present invention. By "substantially consistent", it is meant that in the major part of the structure, in particular, in a part which forms a secondary structure such as α-helix or β-sheet or a part of which temperature factors are lower than other parts, the mean square deviation for the backbone or Cα carbons is about 2 Å or below. Similarly, the beginning and ending sites of each sequence are not necessarily strictly defined for the present invention, and variants such as those in which another protein is attached to the N-terminus and/or the C-terminus or in which one or more amino acid residues are added to the N-terminus and/or the C-terminus are also included by the present invention, provided that they do not result in any substantial changes in molecular recognition between G-CSF and G-CSF-R. Likewise, variants which have additional sugar chains linked thereto or from which a sugar chain moiety has been removed are also within the scope of the present invention so long as they do not result in any substantial changes in molecular recognition between G-CSF and G-CSF-R. In connection with molecular recognition, it is meant by "substantially consistent" that the mean square deviation for the backbone or Cα carbons of amino acid residues involved in the molecular recognition is about 2 Å or below.

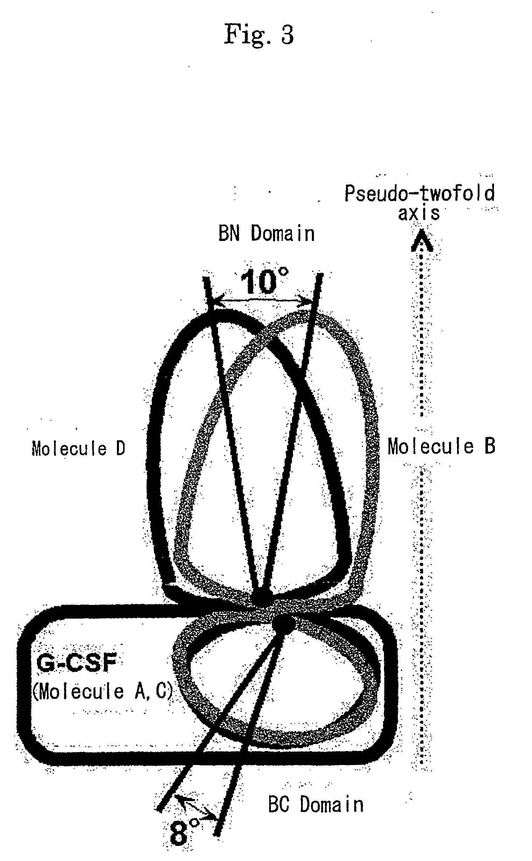

Microscopically, the two molecules of the complex between G-CSF and CRH-G-CSF-R are subtly different from each other. When one of the two molecules of the complex (Molecules A and B; and Molecules C and D) is mathematically subjected to translational and rotational operations in such a way that the respective portions of G-CSFs which form secondary structures, that is, α-helix regions of Molecules A and C, are best superposed, the orientation of Molecules B and D in the BN and BC domains are different from each other. Such difference in orientations of the BN and BC domains are schematically illustrated in Fig. 3. Thus, between the two molecules of the complex contained in a crystallographically independent unit, the relative positions of G-CSF and CRH-G-CSF-R are slightly different by about 10° for BN domain and by about 8° for BC domain. In general, when a substance crystallizes, it proceeds toward the state of least free energy according to a law in natural science. The fact that the two molecules in an asymmetric unit have different structures in the crystal state indicates that the amount of decrease in energy due to formation of the crystal state is larger than the amount of increase in energy due to generation of the difference between the structures.

Furthermore, by the structure coordinates determined by the present invention, it becomes possible for the first time to locate interacting amino acid residues in G-CSF and CRH-G-CSF-R. The residues having an interatomic distance of 4.2 Å or less for van der Waals interacting atoms, of more than 3.4 Å to 5.0 Å for electrostatically interacting atoms, or of 3.4 Å or less for hydrogen-bonding are shown in Tables 2, 3, 4, and 5, respectively. In these tables, hydrogen bonds via water molecules are also indicated.

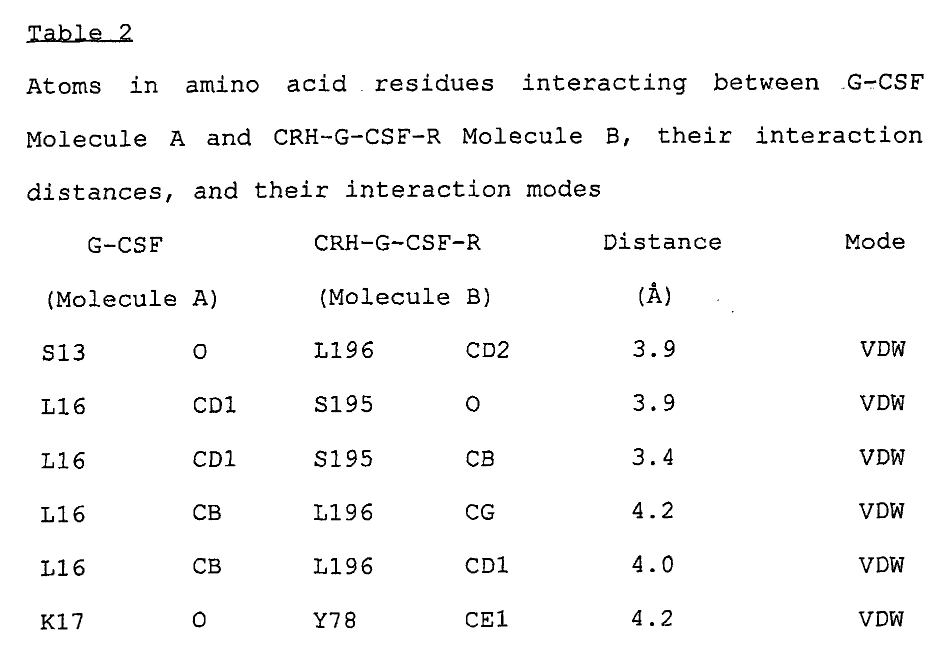

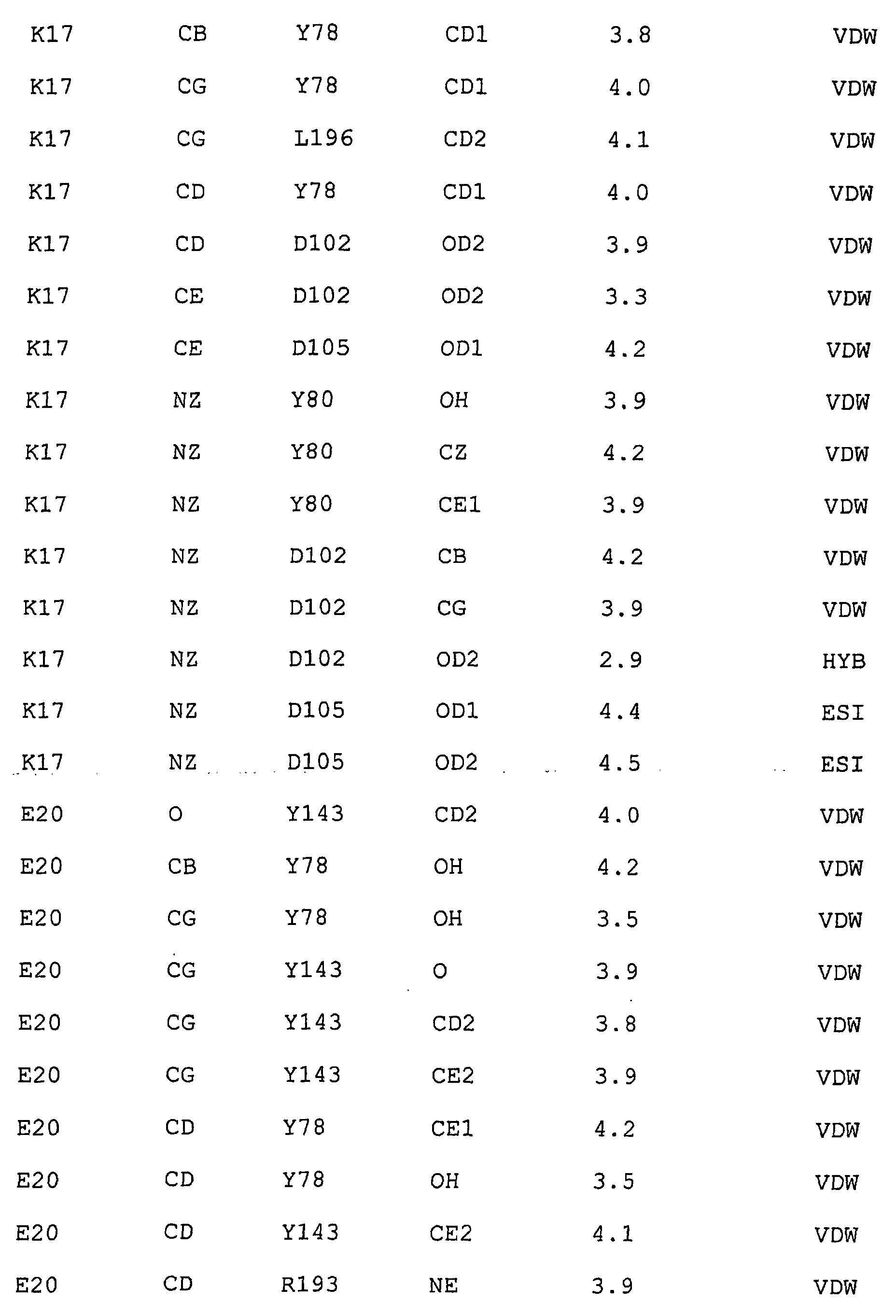

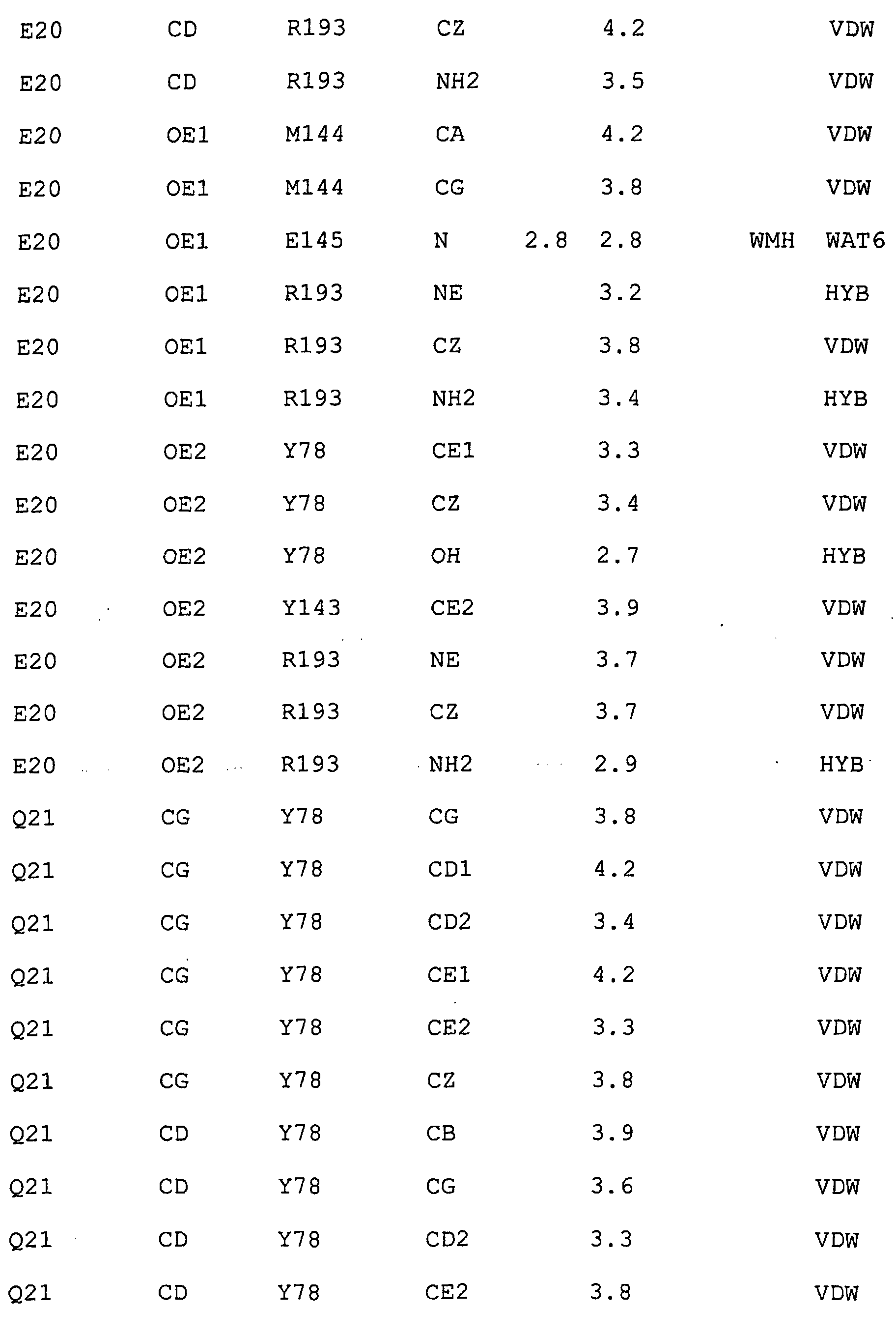

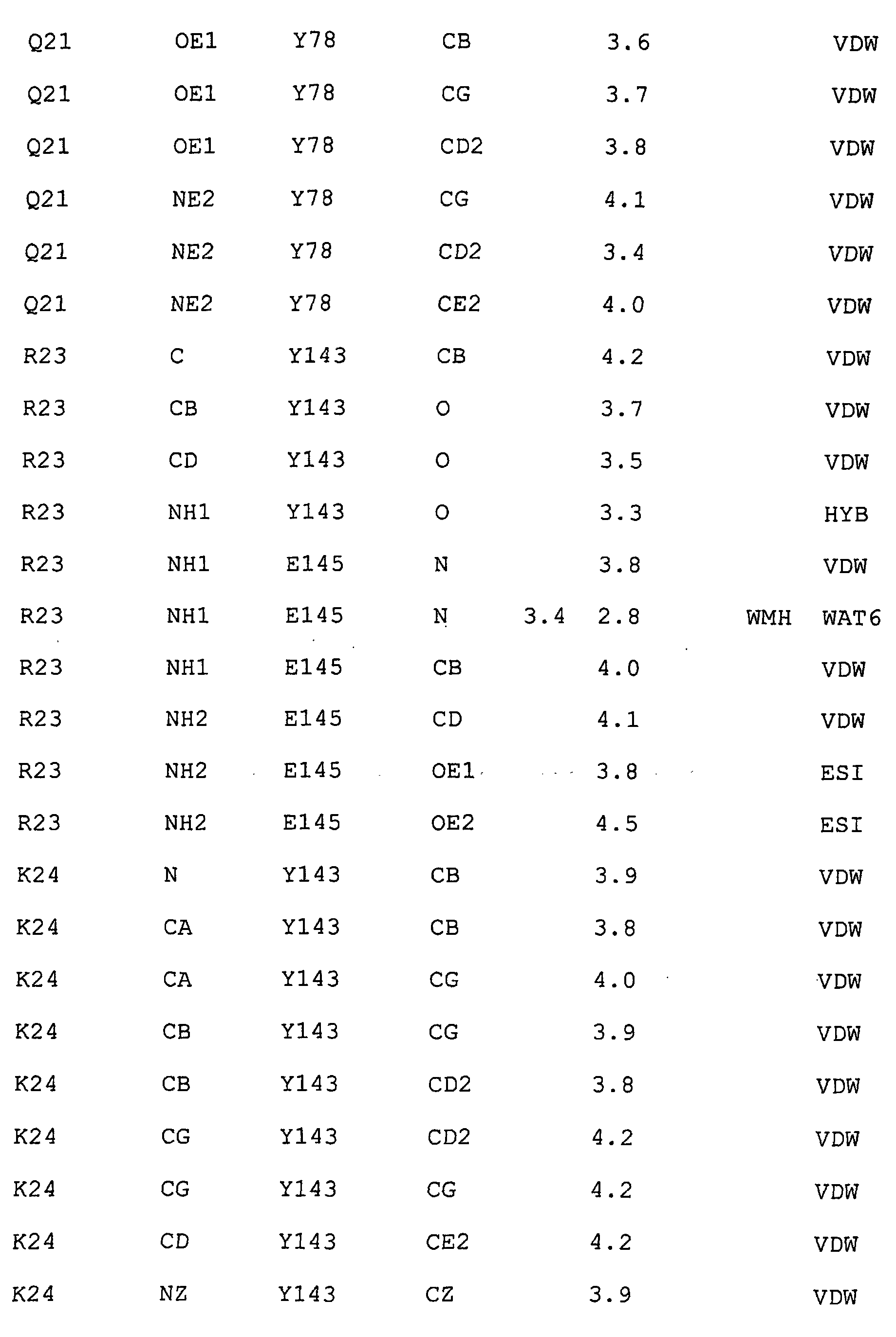

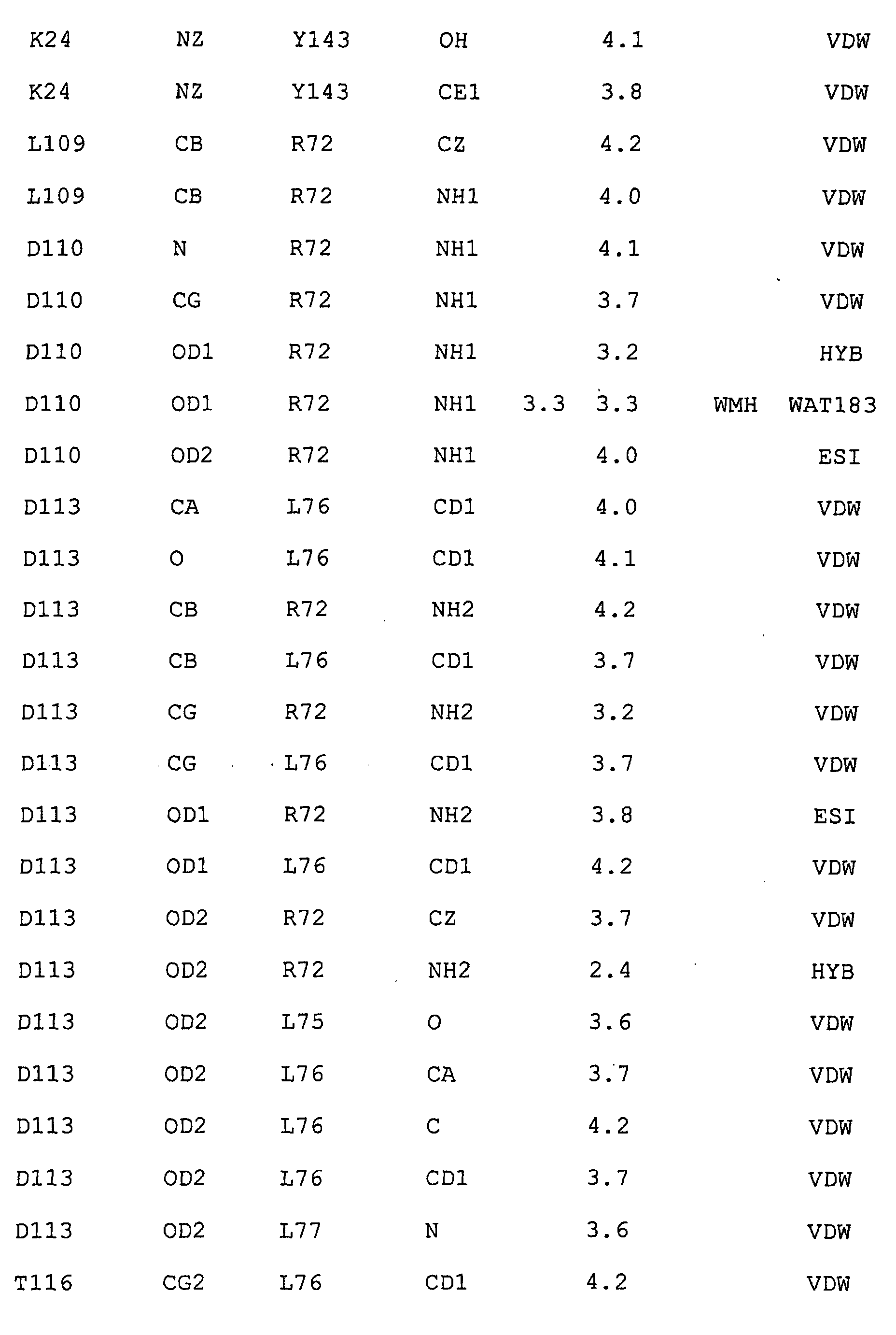

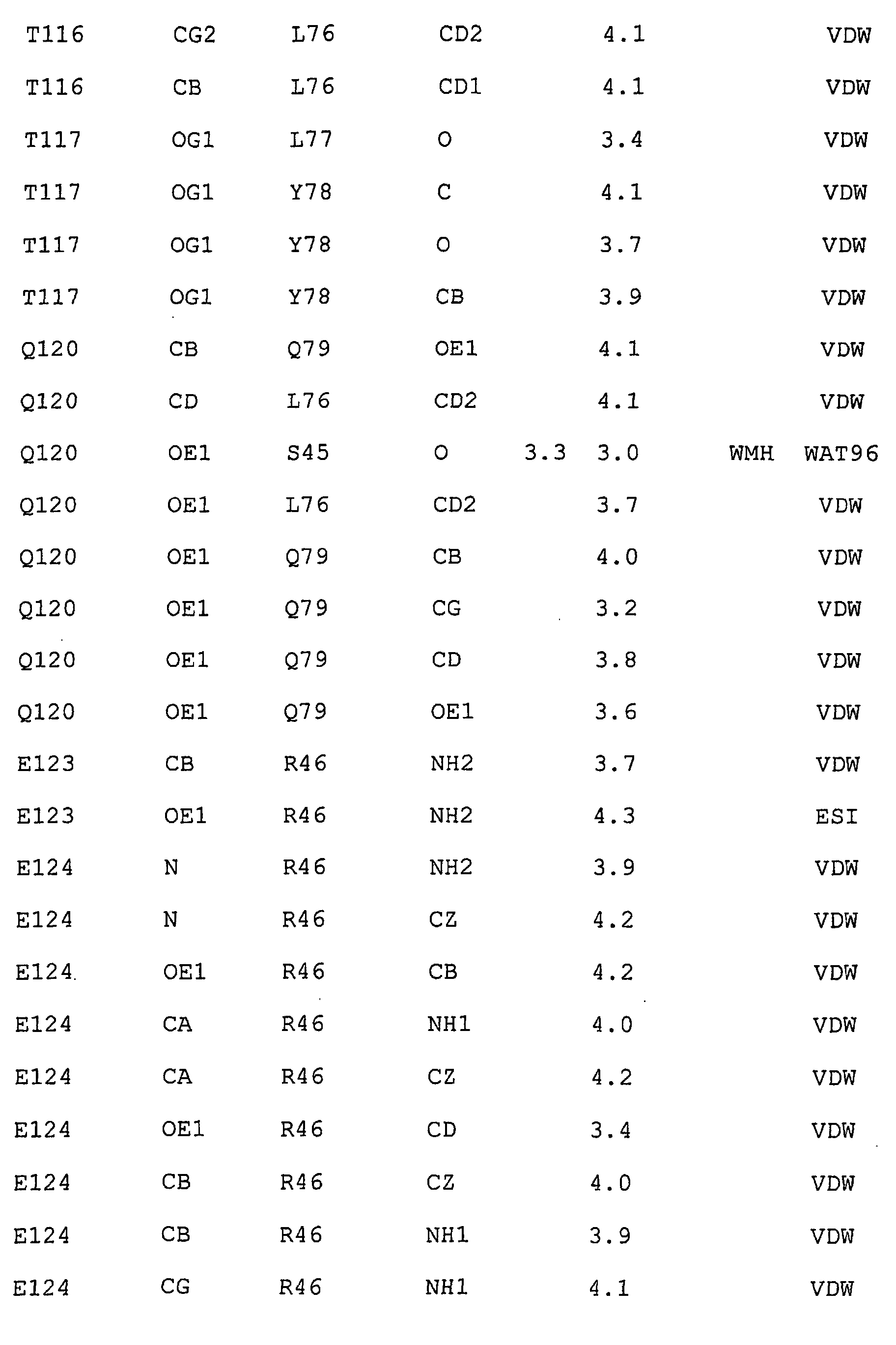

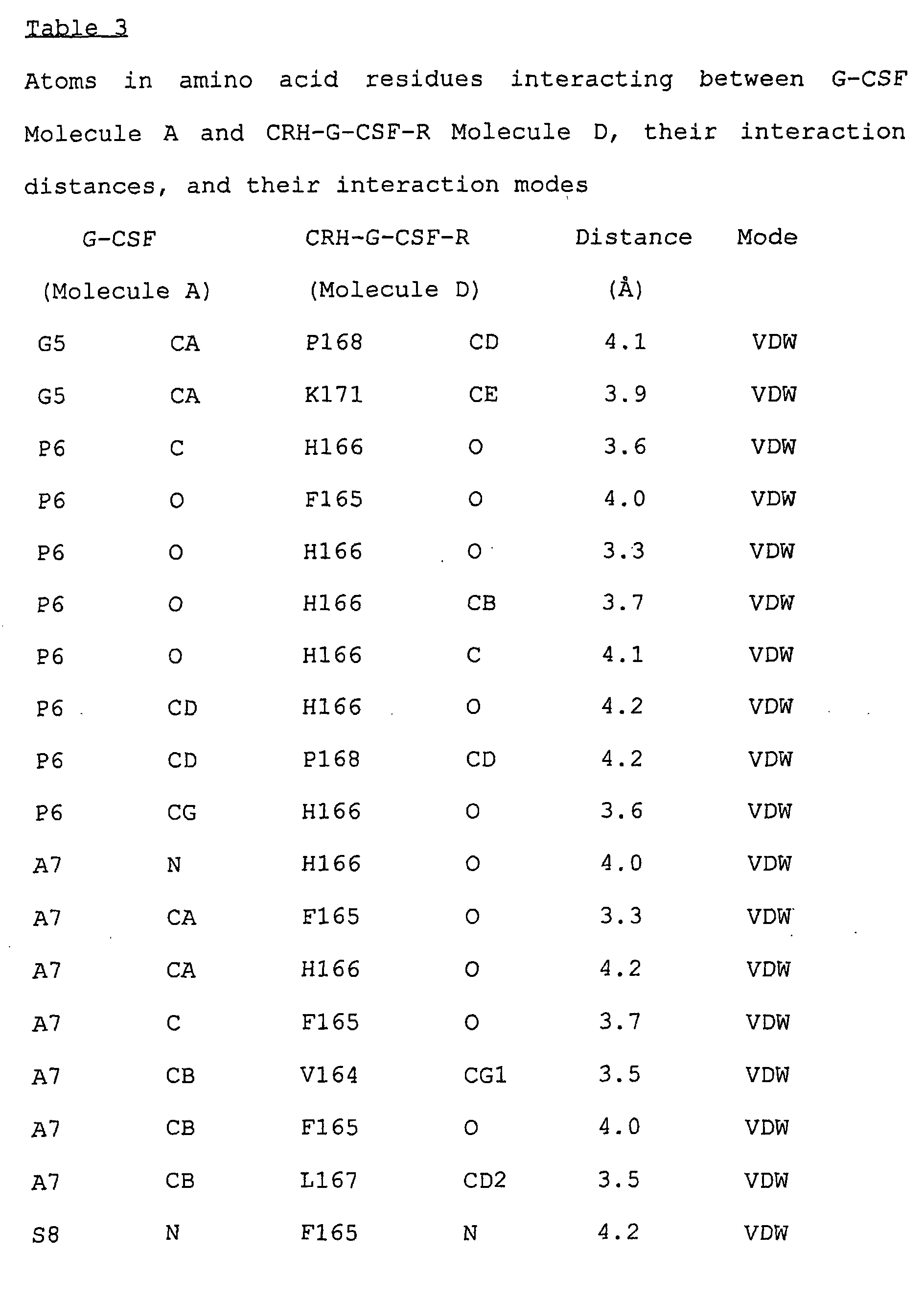

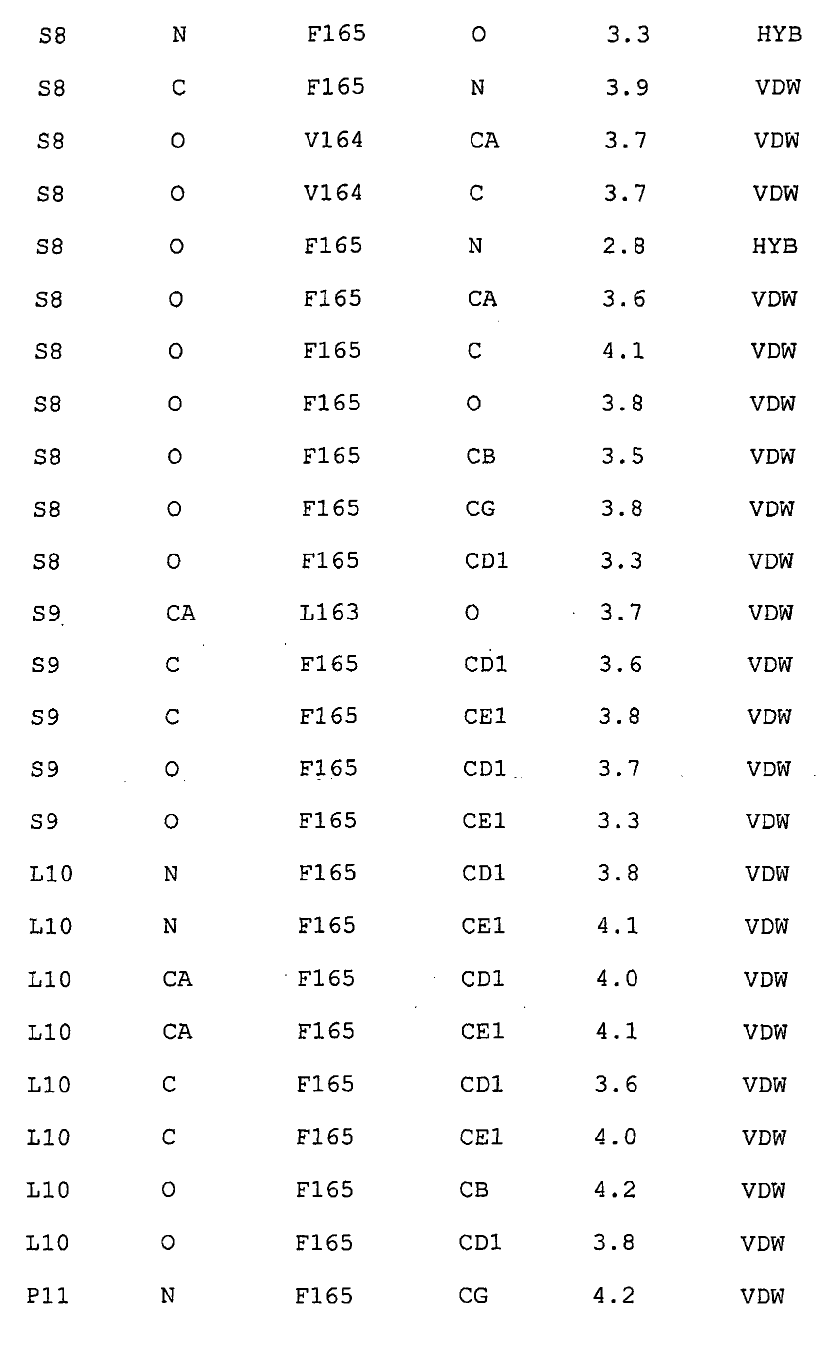

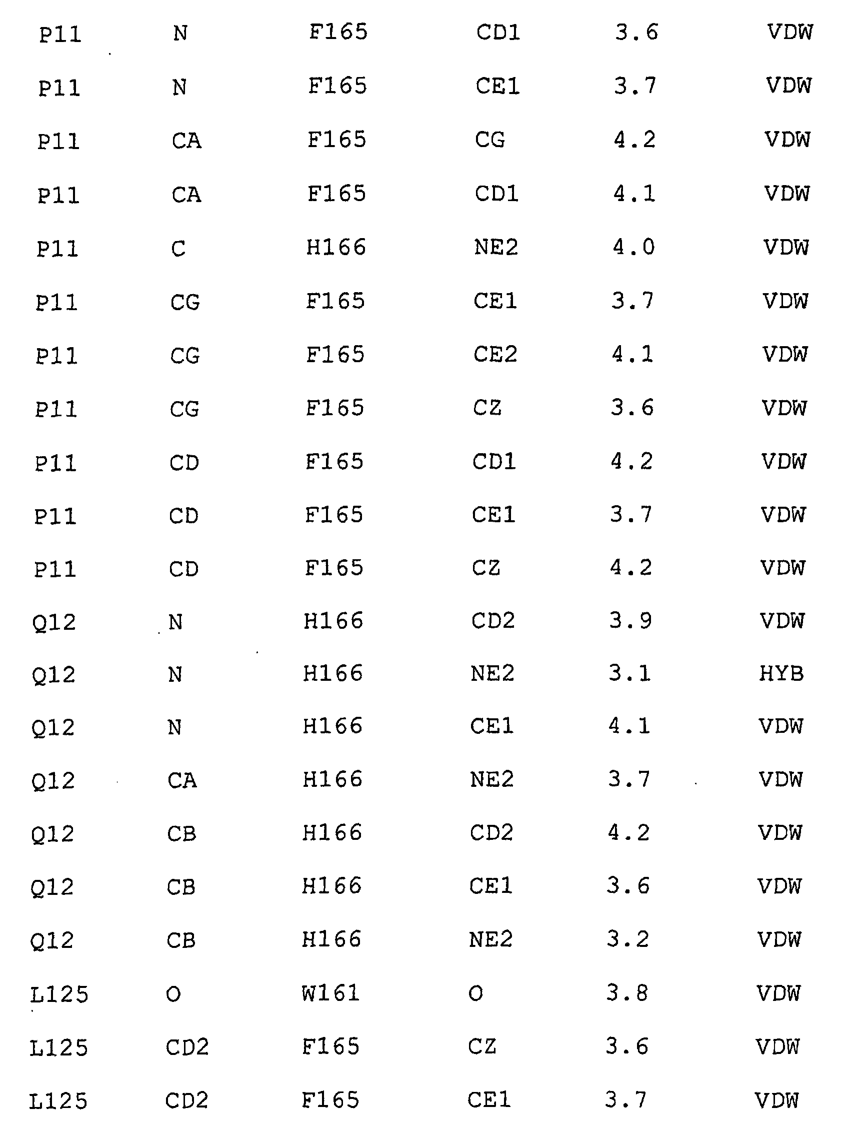

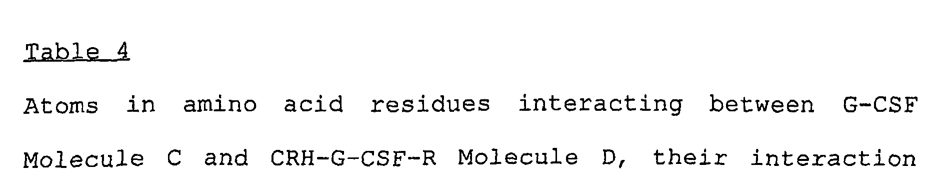

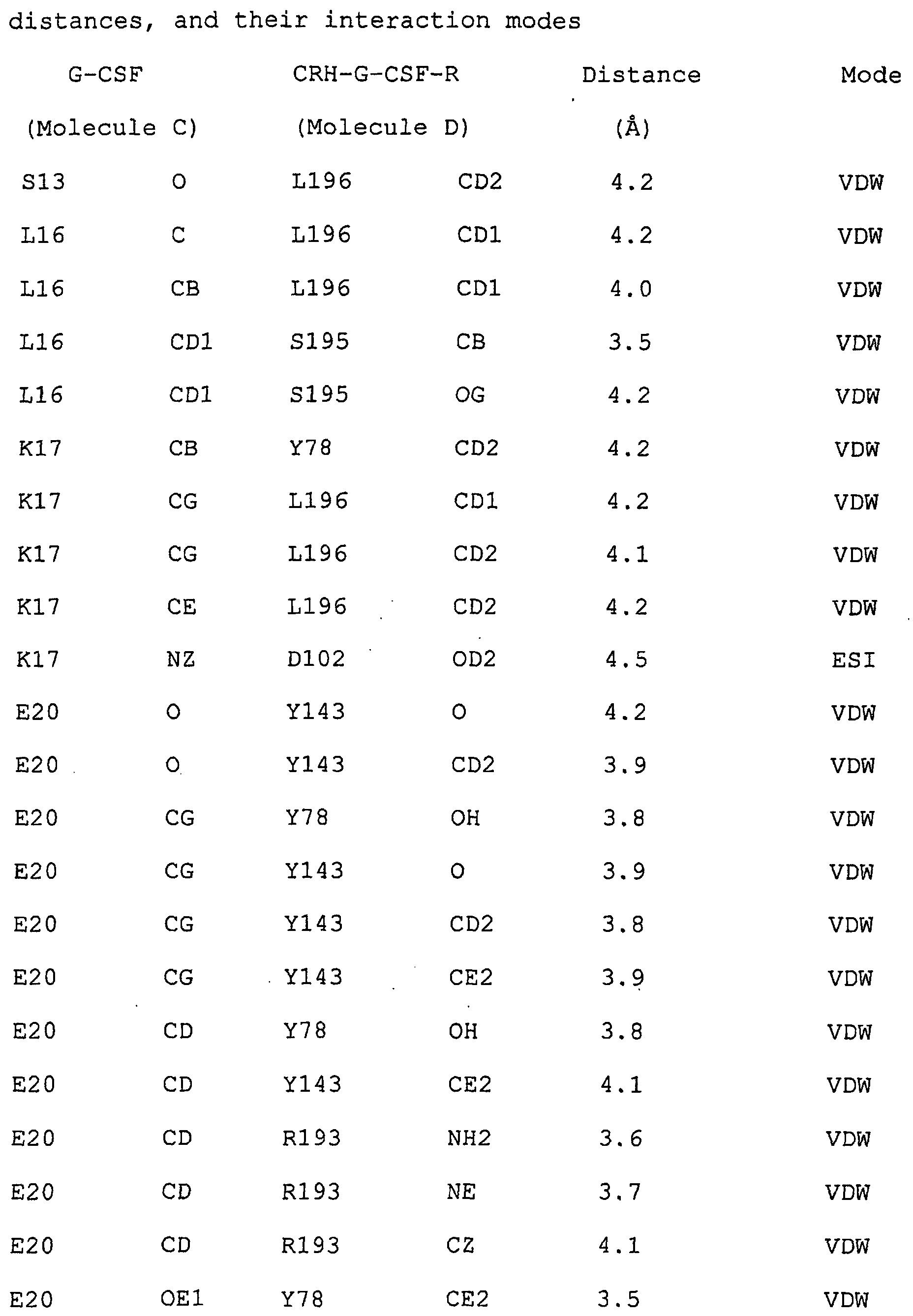

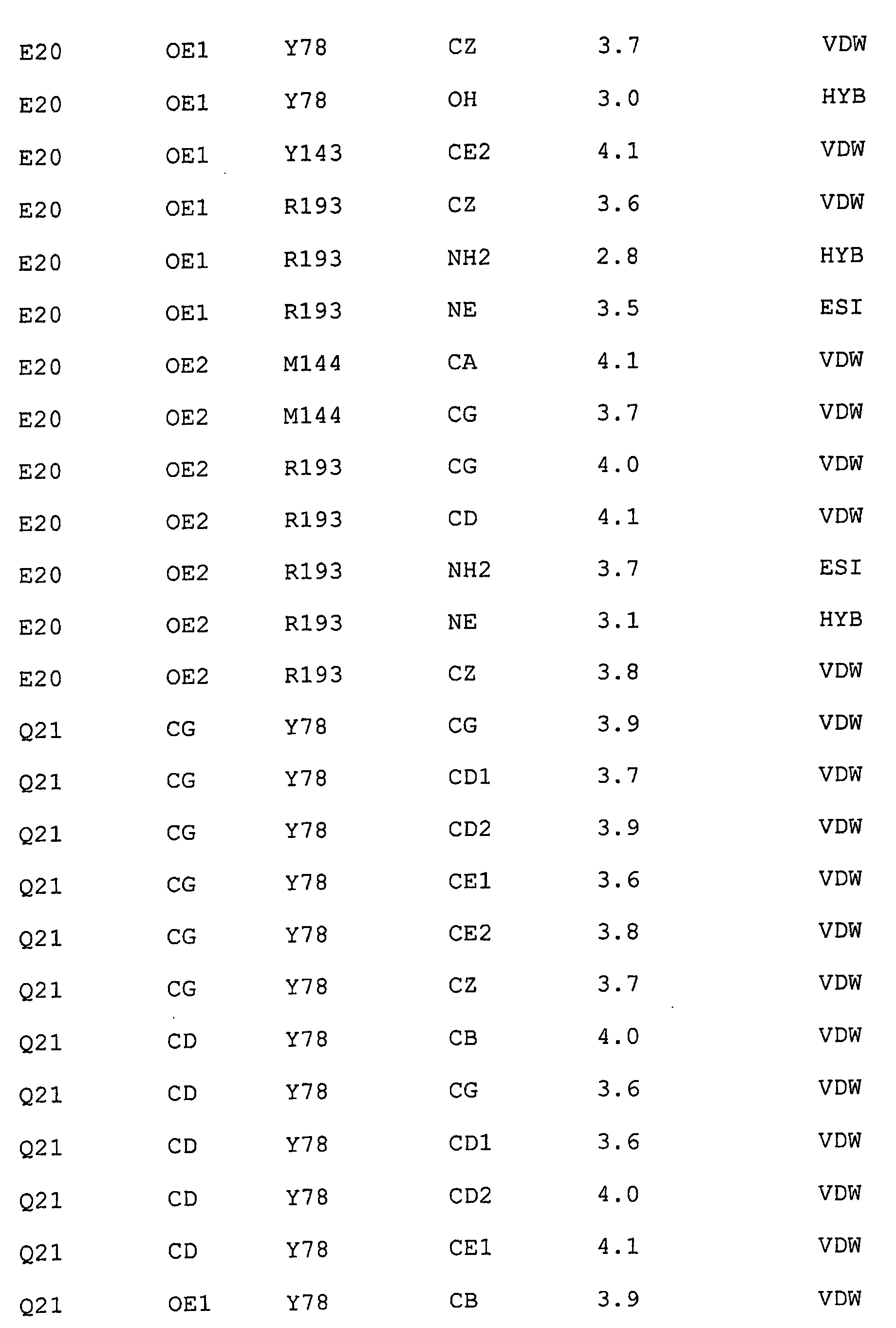

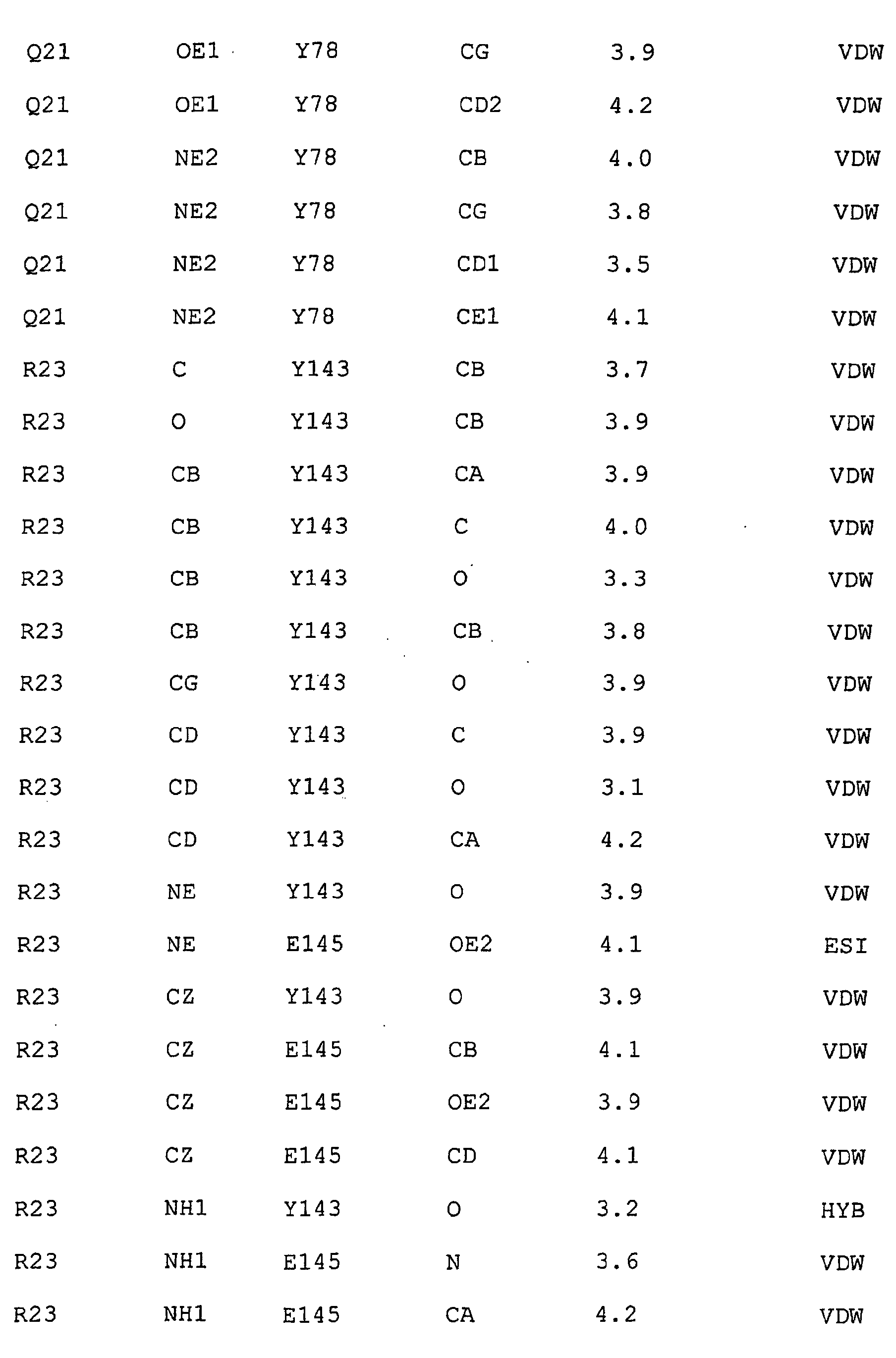

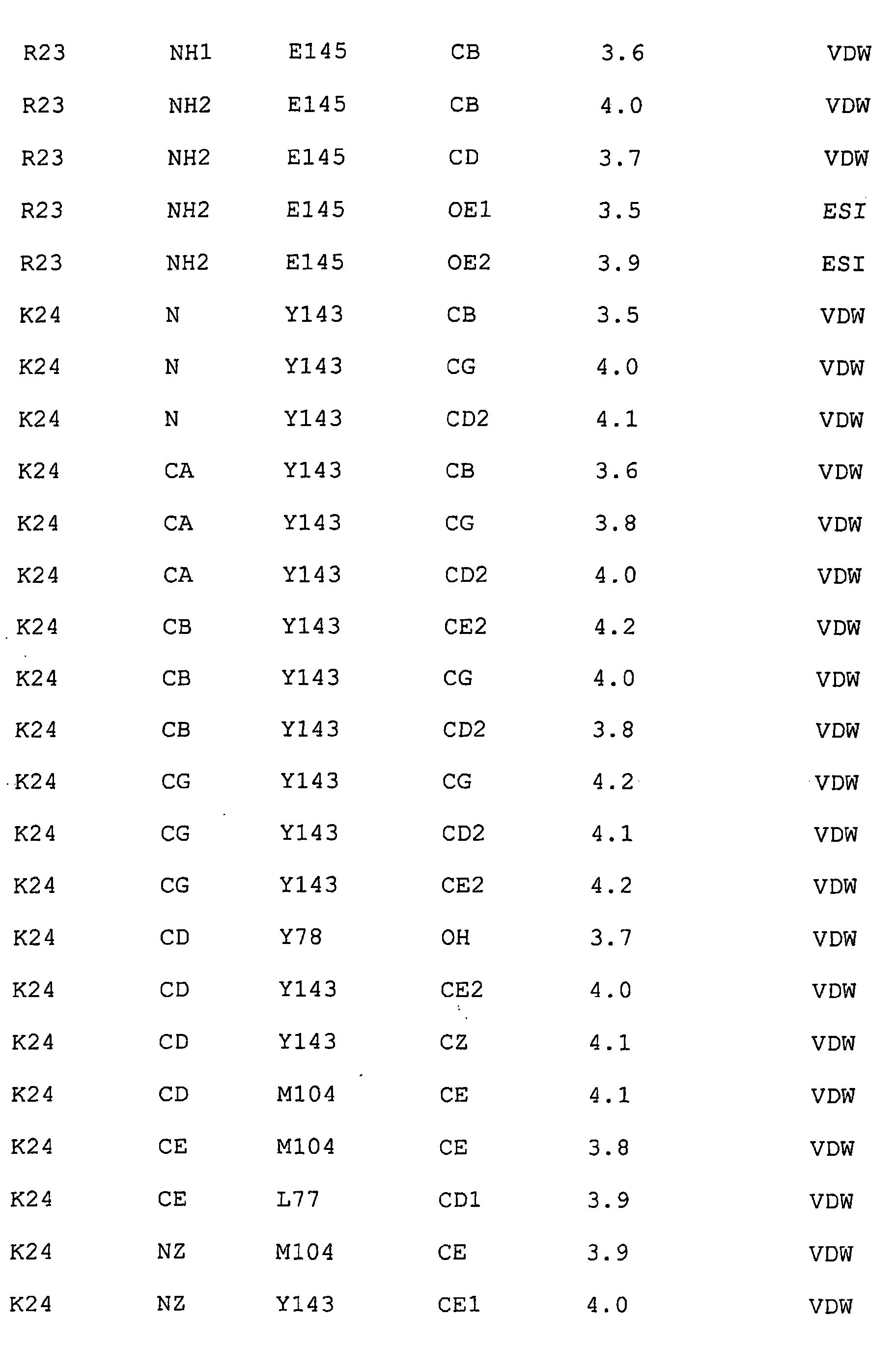

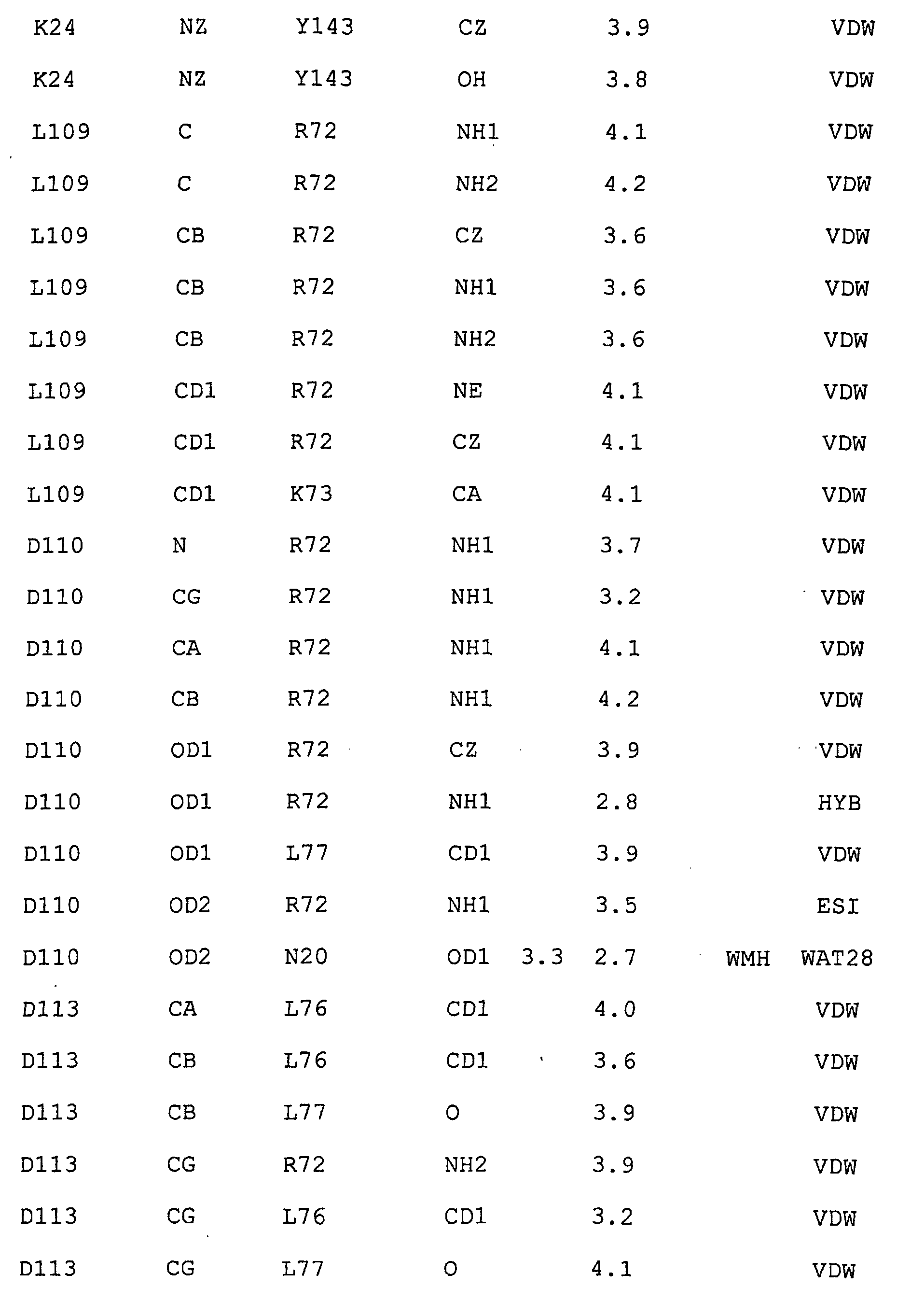

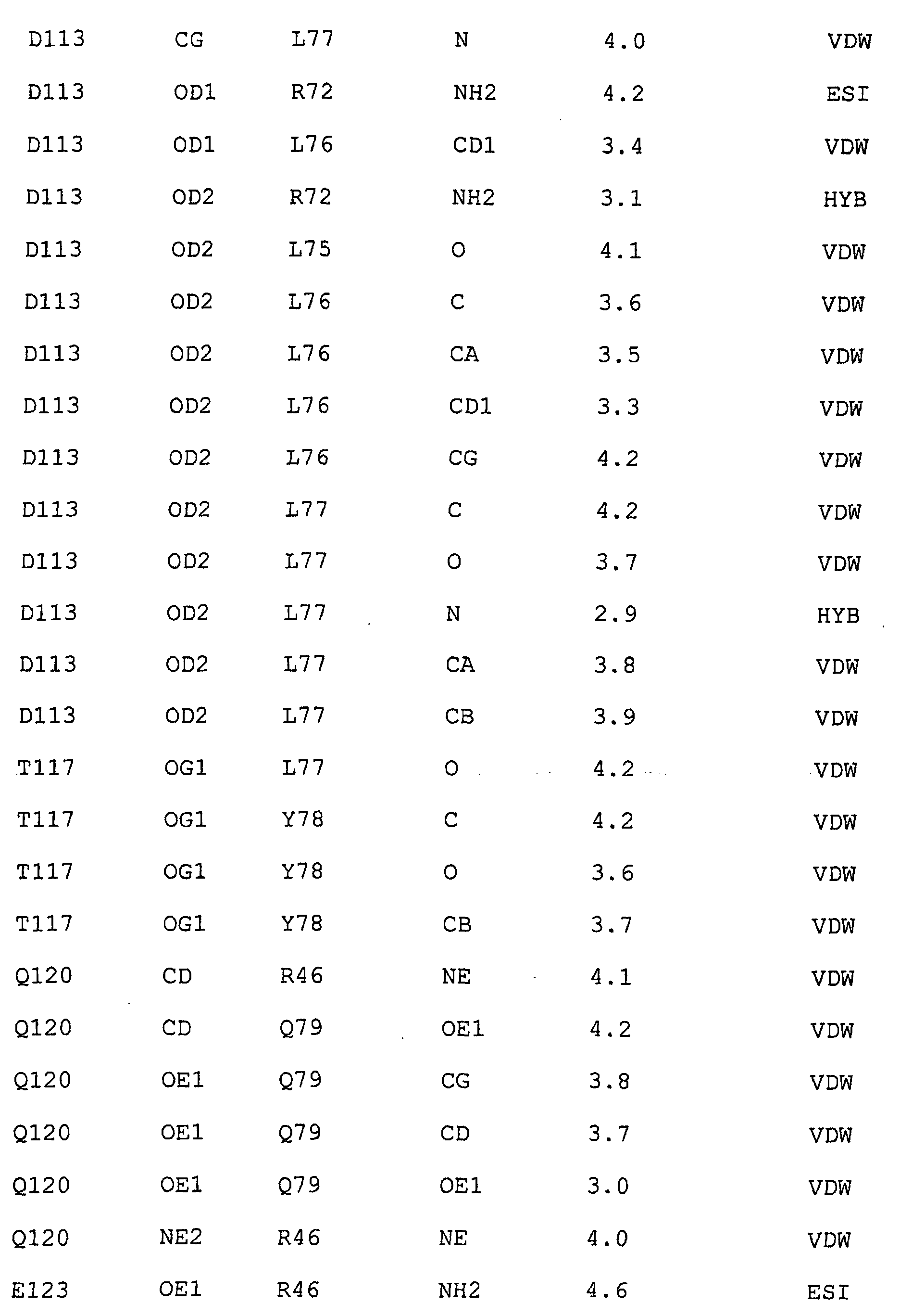

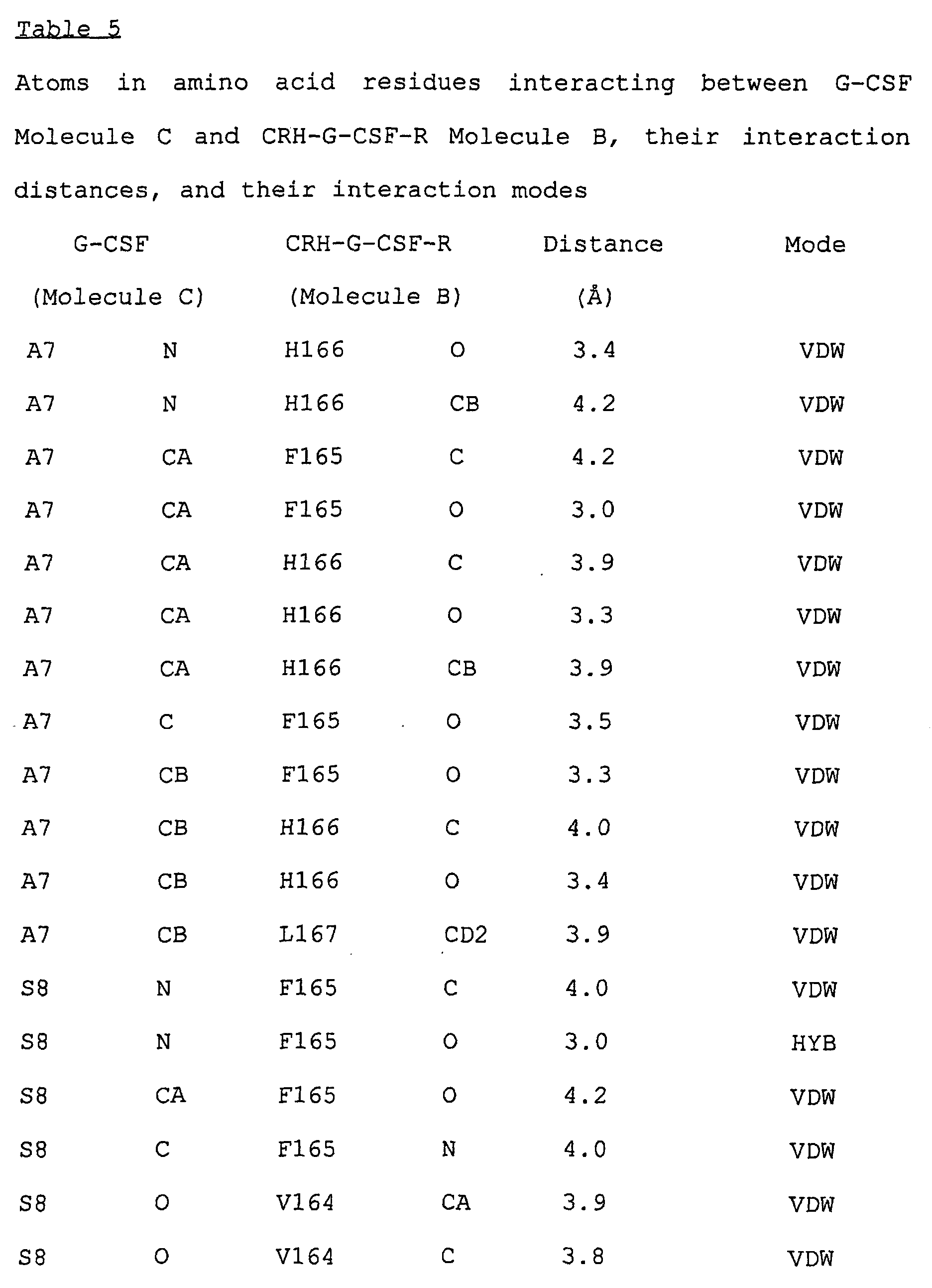

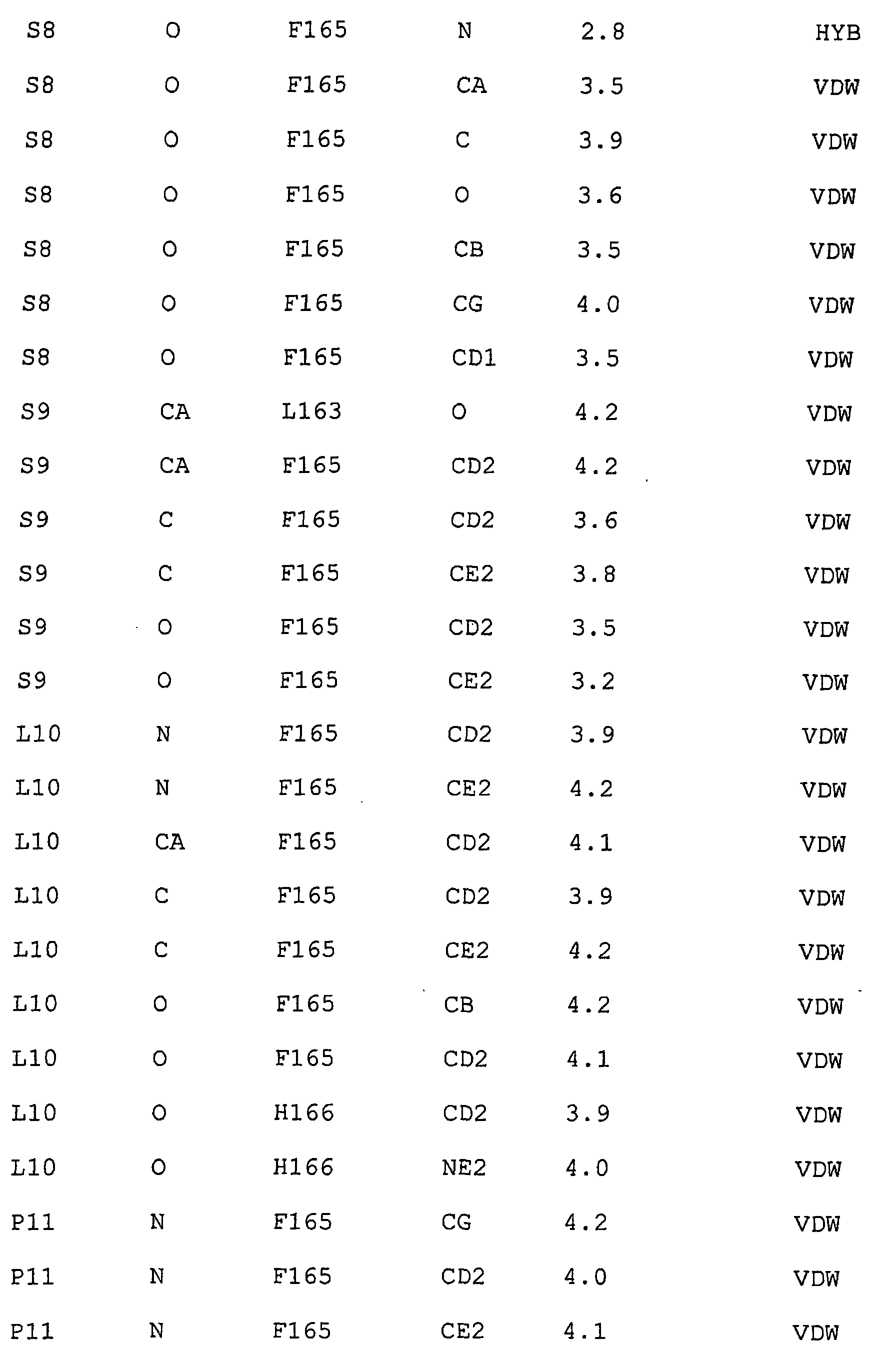

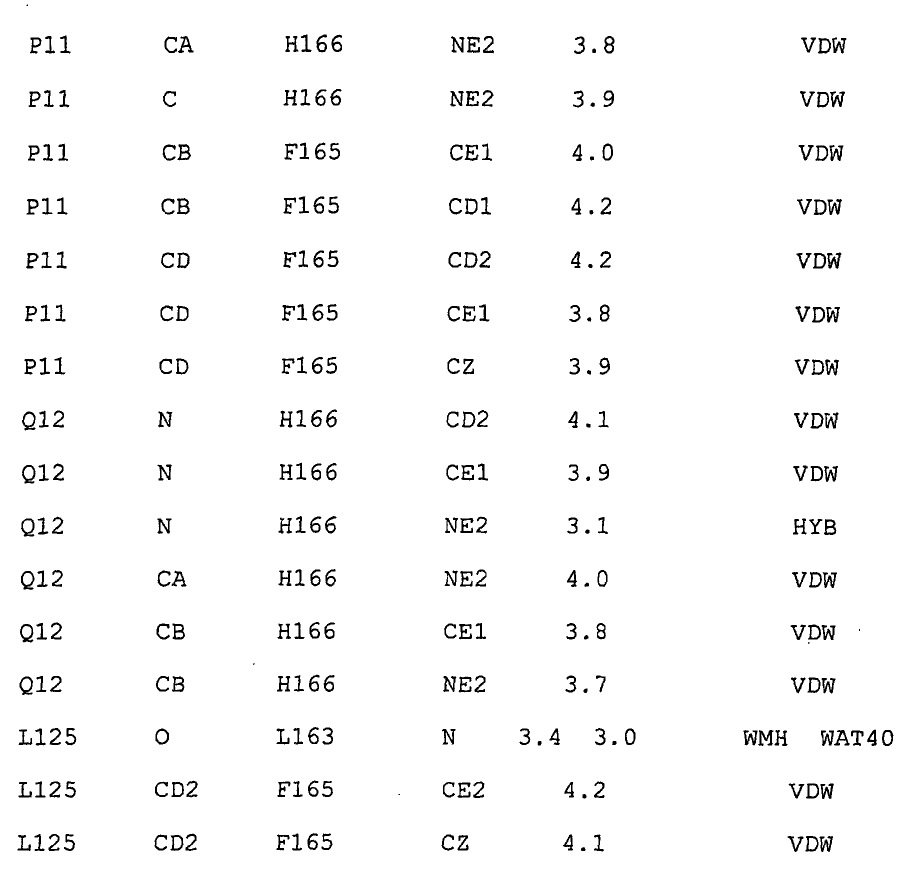

The above tables indicate atoms in amino acid residues interacting between G-CSF Molecule A and CRH-G-CSF-R Molecule B (Table 2), between G-CSF Molecule A and CRH-G-CSF-R Molecule D (Table 3), between G-CSF Molecule C and CRH-G-CSF-R Molecule D (Table 4), or G-CSF Molecule C and CRH-G-CSF-R Molecule B (Table 5), their interaction distances, and their interaction modes. According to the structure coordinates shown in Table 1, the residues having an interatomic distance of 4.2 Å or less for van der Waals interacting atoms, of more than 3.4 Å to 5.0 Å for electrostatically interacting atoms, or of 3.4 Å or less for hydrogen-bonding atoms are shown in each of Tables 2, 3, 4, and 5. In these tables, hydrogen bonds via water molecules are also indicated. The first column describes amino acid residues and their number in Molecule A or C; the second column describes atoms in the amino acid residues; the third column describes amino acid residues and their number in Molecule B or D; the fourth column describes atoms in the amino acid residues; the fifth column describes interatomic distances between them in Å; and the sixth column describes interaction modes. In connection with interaction modes, VDW denotes van der Waals interaction; ESI denotes electrostatic interaction; and HYB denotes hydrogen bonding. In order to describe a hydrogen bond via a water molecule, two values for interatomic distance are necessary. In such cases, the fifth column is further divided into two columns to describe these values; the abbreviation WMH is indicated in the sixth column, and the water molecule number involved is indicated in the seventh column.

First, in one molecule of CRH-G-CSF-R, the loop regions in each of the two domains recognize one molecule of G-CSF at one side. Amino acid residues characterizing this recognition are S13, L16, K17, E20, Q21, R23, K24, L109, D110, D113, T116, T117, Q120, E123, and E124 on the G-CSF side (Tables 2 and 4), and N20, S45, R46, R72, K73, L75, L76, L77, Y78, Q79, Y80, D102, M104, D105, Y143, M144, E145, R193, S195, and L196 on the CRH-G-CSF-R side (Tables 2 and 4), respectively. Thus, recognition between one molecule of G-CSF and one molecule of G-CSF-R (that is, formation of the complex) is characterized by interactions among these and adjacent amino acid residues.

Next, macroscopically, two molecules of the above complex recognize each other (that is, association of the complexes) in such a way related by a non-crystallographic pseudo-twofold axis. Amino acid residues characterizing this recognition are G5, P6, A7, S8, S9, L10, P11, Q12, and L125 on the G-CSF side (Tables 3 and 5), and W161, L163, V164, F165, H166, L167, P168, and K171 on the CRH-G-CSF-R side (Tables 3 and 5), respectively. Thus, the complex consisting of one molecule of G-CSF and one molecule of CRH-G-CSF-R further self-associates to form a homodimer of the complex, and this recognition is characterized by interactions among these and adjacent amino acid residues.

The region surrounded by the dimer of the complex (that is, the associate) forms a space in which water molecules exist. This space is characterized by Y3 to L14, R46 to Y51, G92 to V106, E145 to E147, H166 to S169, S194 to G198, and amino acid residues adjacent thereto in CRH-G-CSF-R.

Furthermore, from the three-dimensional structure coordinates of human-type G-CSF and mouse-type CRH-G-CSF-R in the present invention, three-dimensional structure coordinates of G-CSF and CRH-G-CSF-R derived from other species having homologous amino acid sequences can be derived by homology modelings (Haruki Nakamura and Kenta Nakai, "Biotechnology-no-tameno-Computer-nyumon" (An introduction to computers for biotechnology), pp. 186-204, Corona Publishing Co., 1995). The more homologous between the amino acid sequences is, the more easily the three-dimensional structure coordinates of interest can be derived. Since the amino acid sequence of human-type G-CSF-R has a high homology to that of mouse-type G-CSF-R, three-dimensional structure coordinates of human-type G-CSF-R can easily be derived.

Based on the three-dimensional structure coordinates of human-type G-CSF and mouse-type CRH-G-CSF-R obtained in Examples 3 of the present invention, structure coordinates for human-type CRH-G-CSF-R were prepared by homology modeling. First, in the structure coordinates of mouse CRH-G-CSF-R, the side chain portions of amino acid residues which are not identical between mouse and human CRH-G-CSF-Rs were replaced by corresponding side chains of human amino acid residues. At this stage, conformations of the side chains were selected so that the atoms do not stereochemically overlap one another and the energy became minimal. Furthermore, conformational calculations were conducted for all amino acid residues including their backbone portions to minimize the energy of the whole molecule.

The three-dimensional structure coordinates for human-type CRH-G-CSF-R thus derived are shown in Table 6.

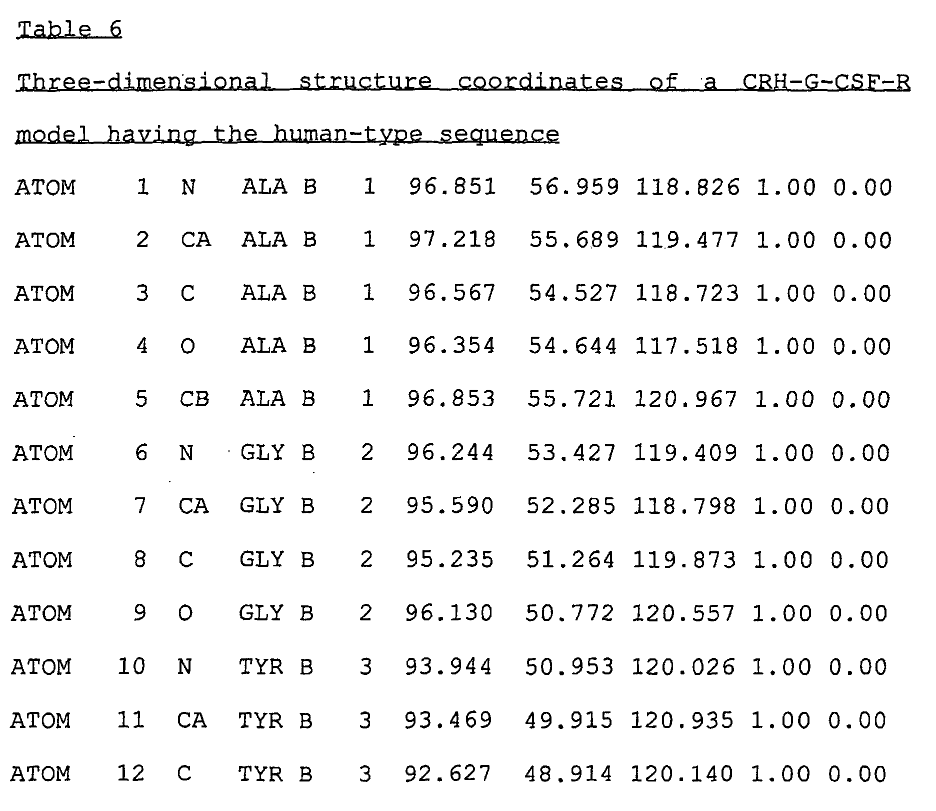

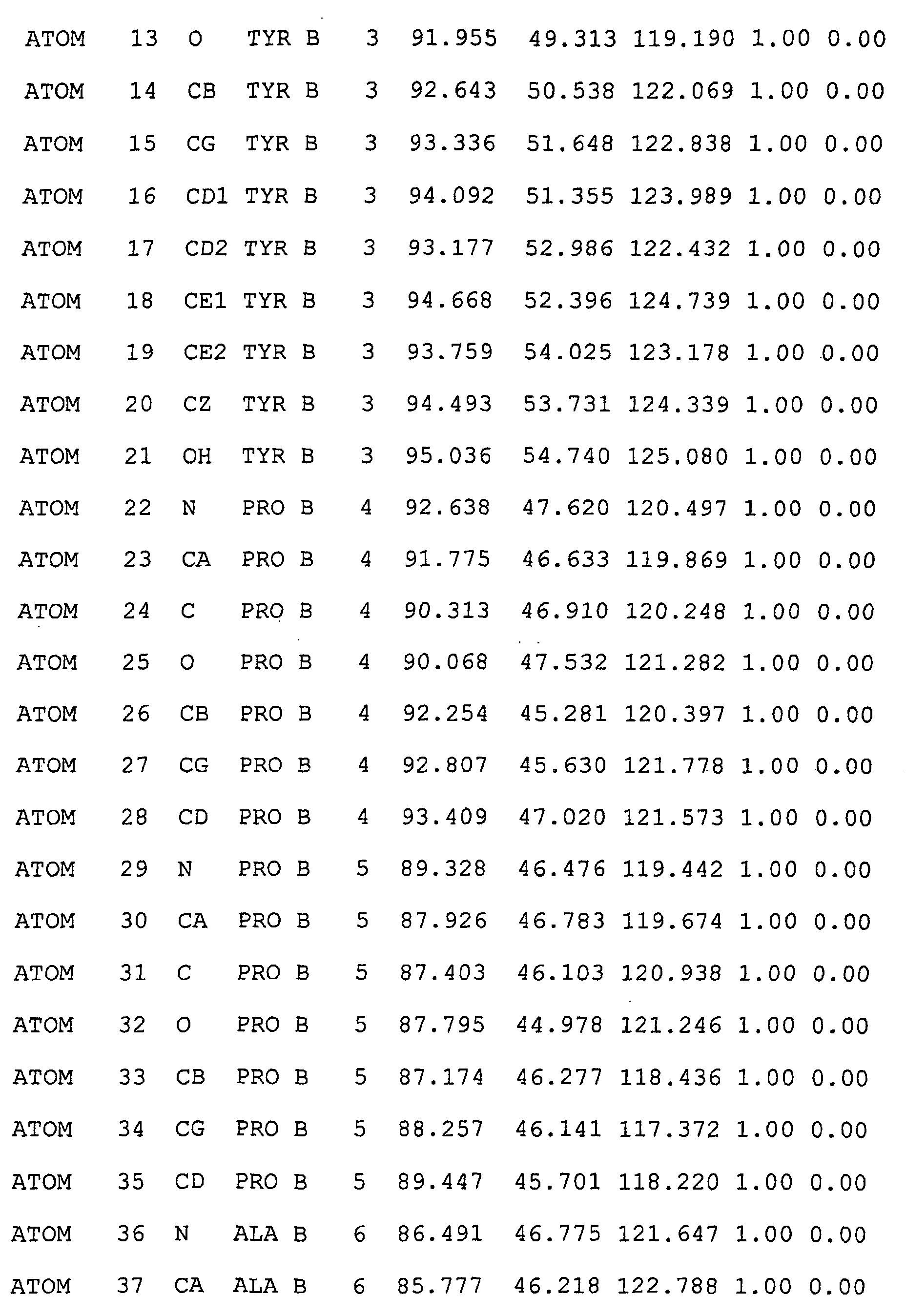

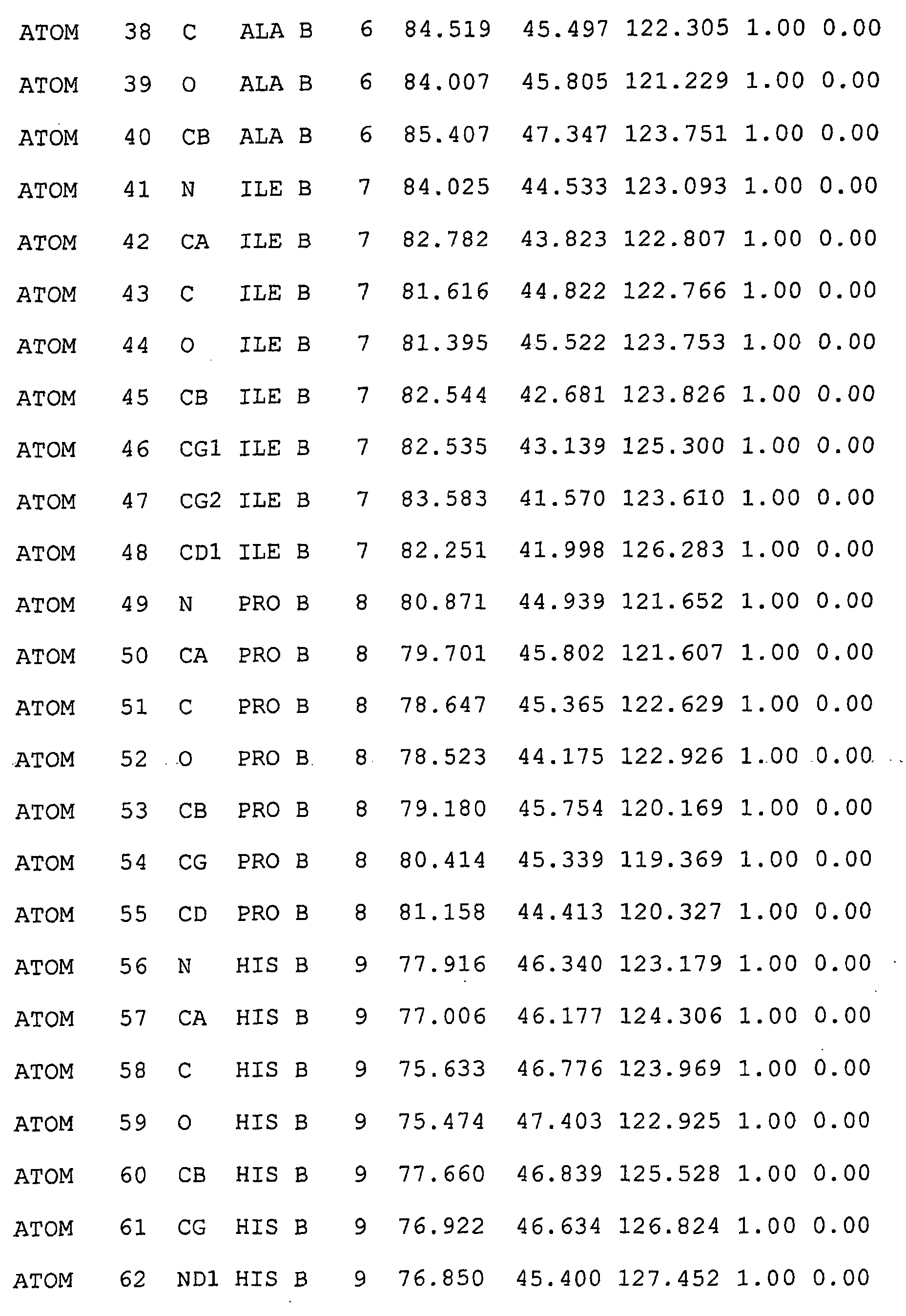

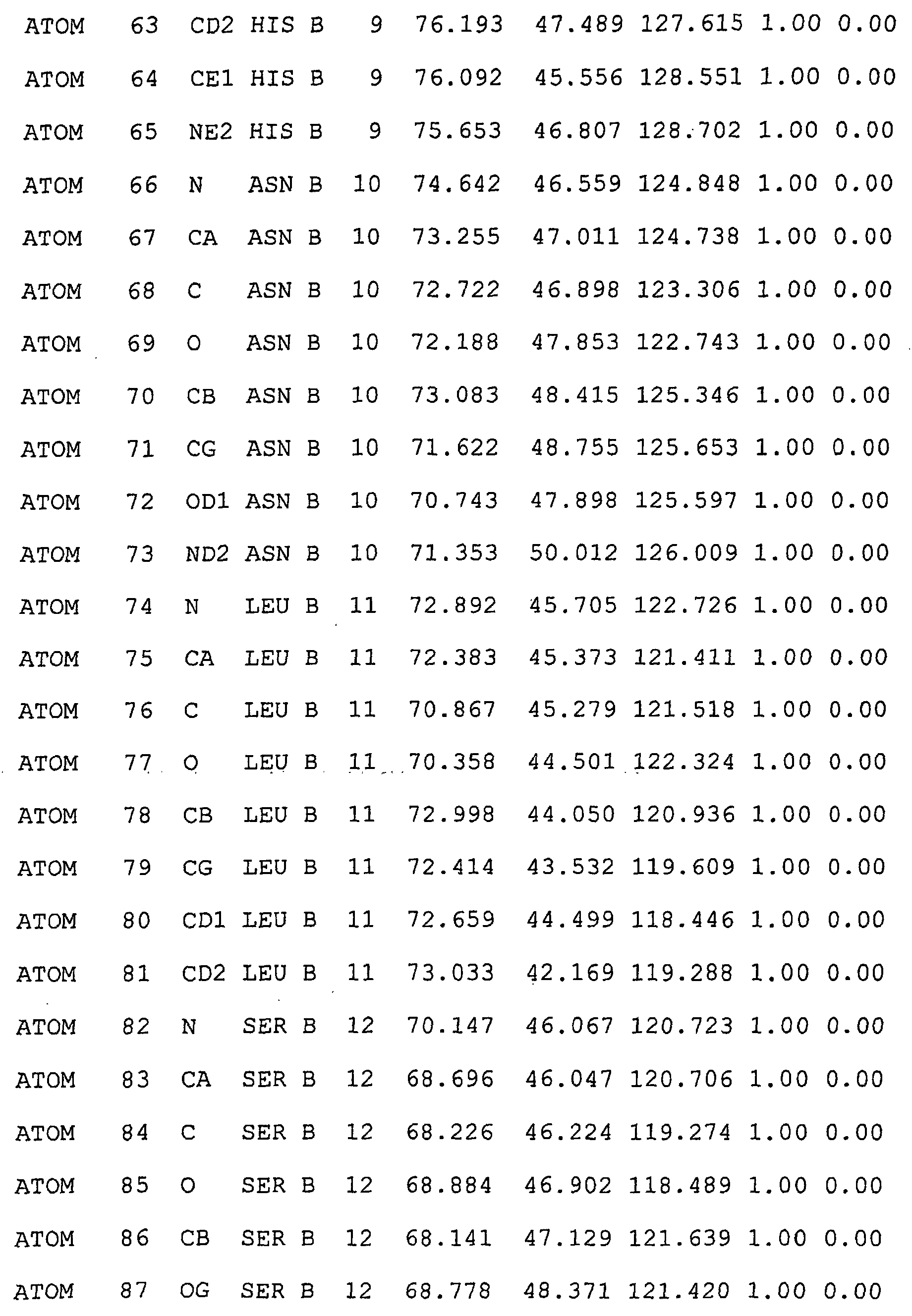

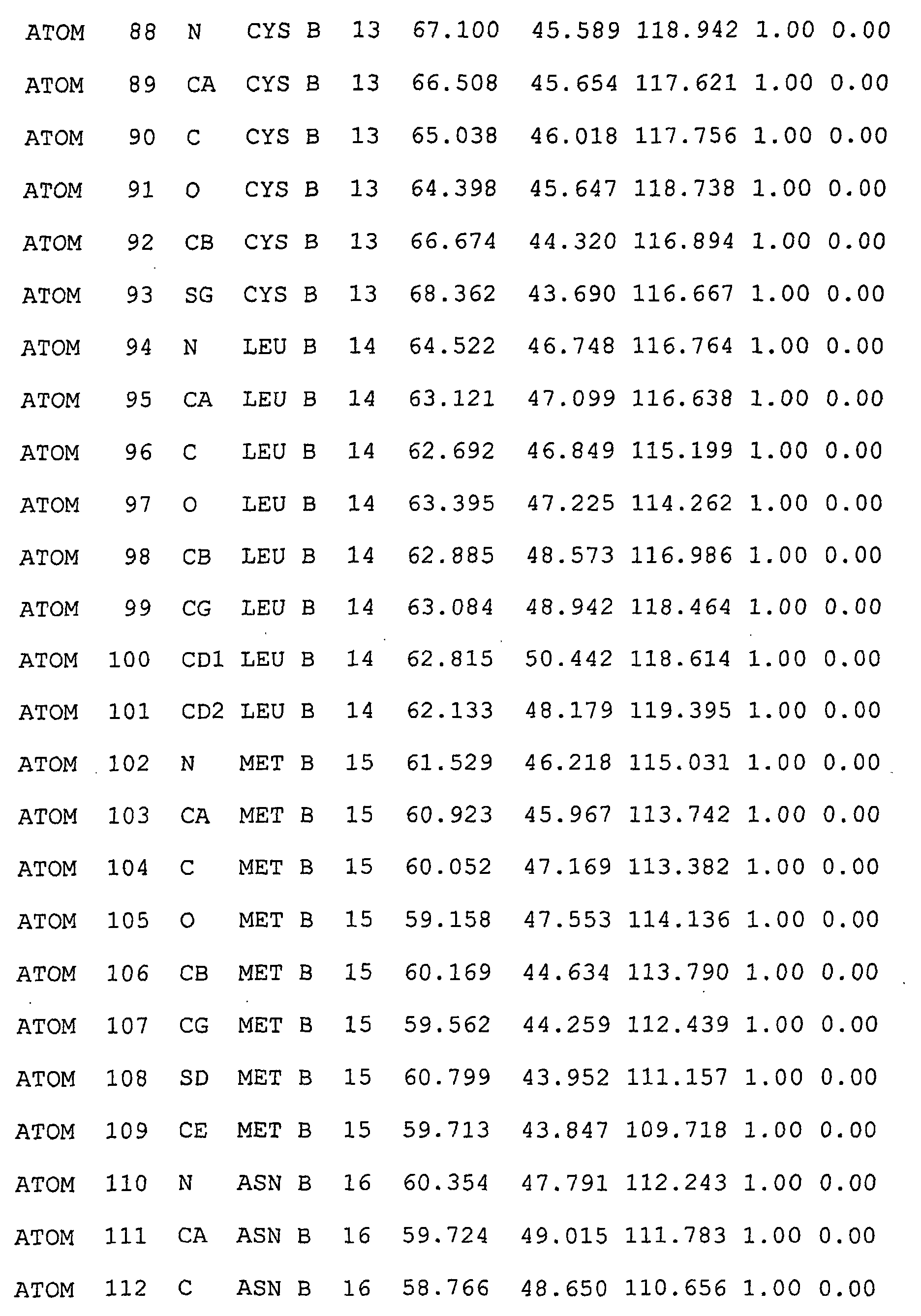

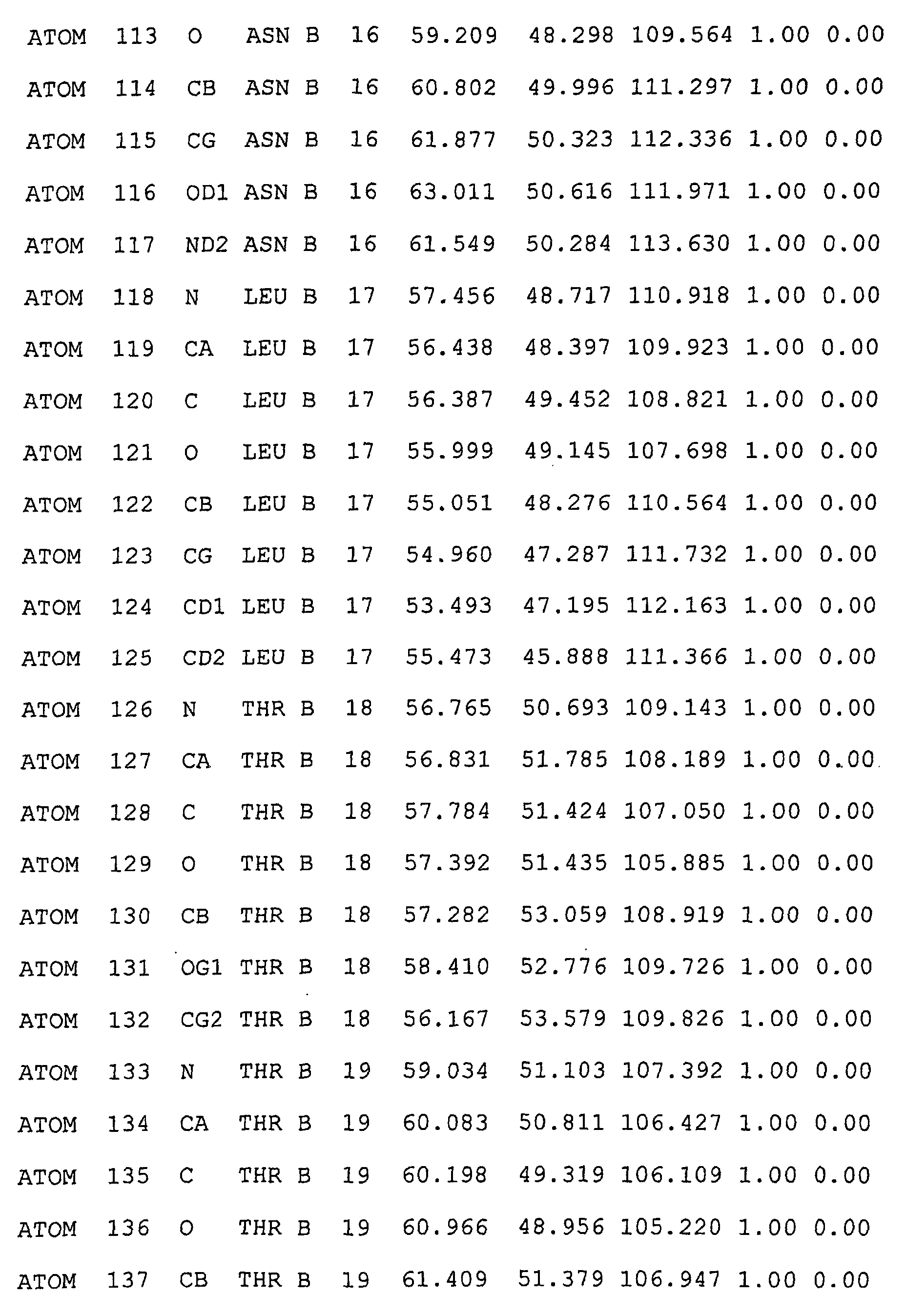

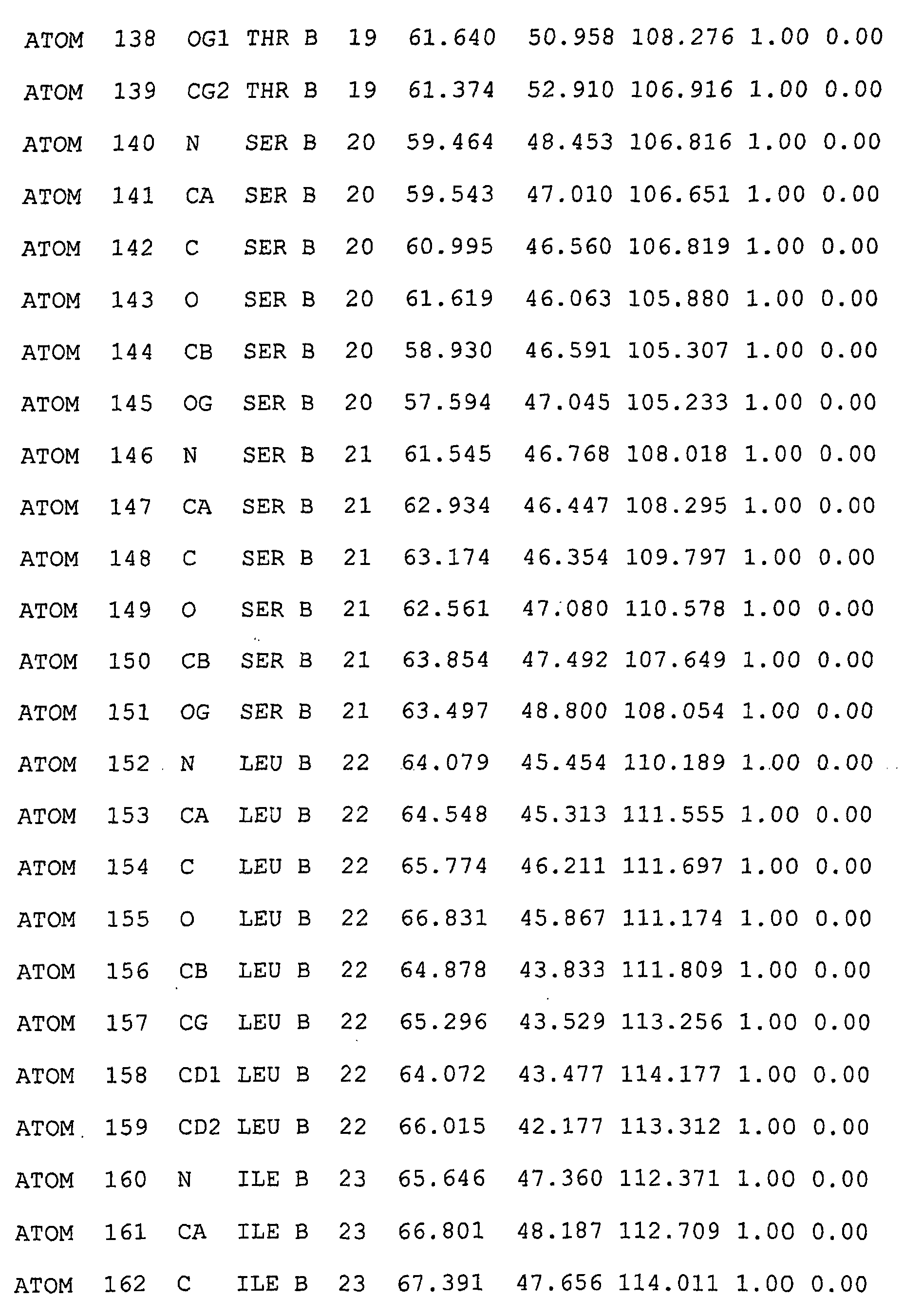

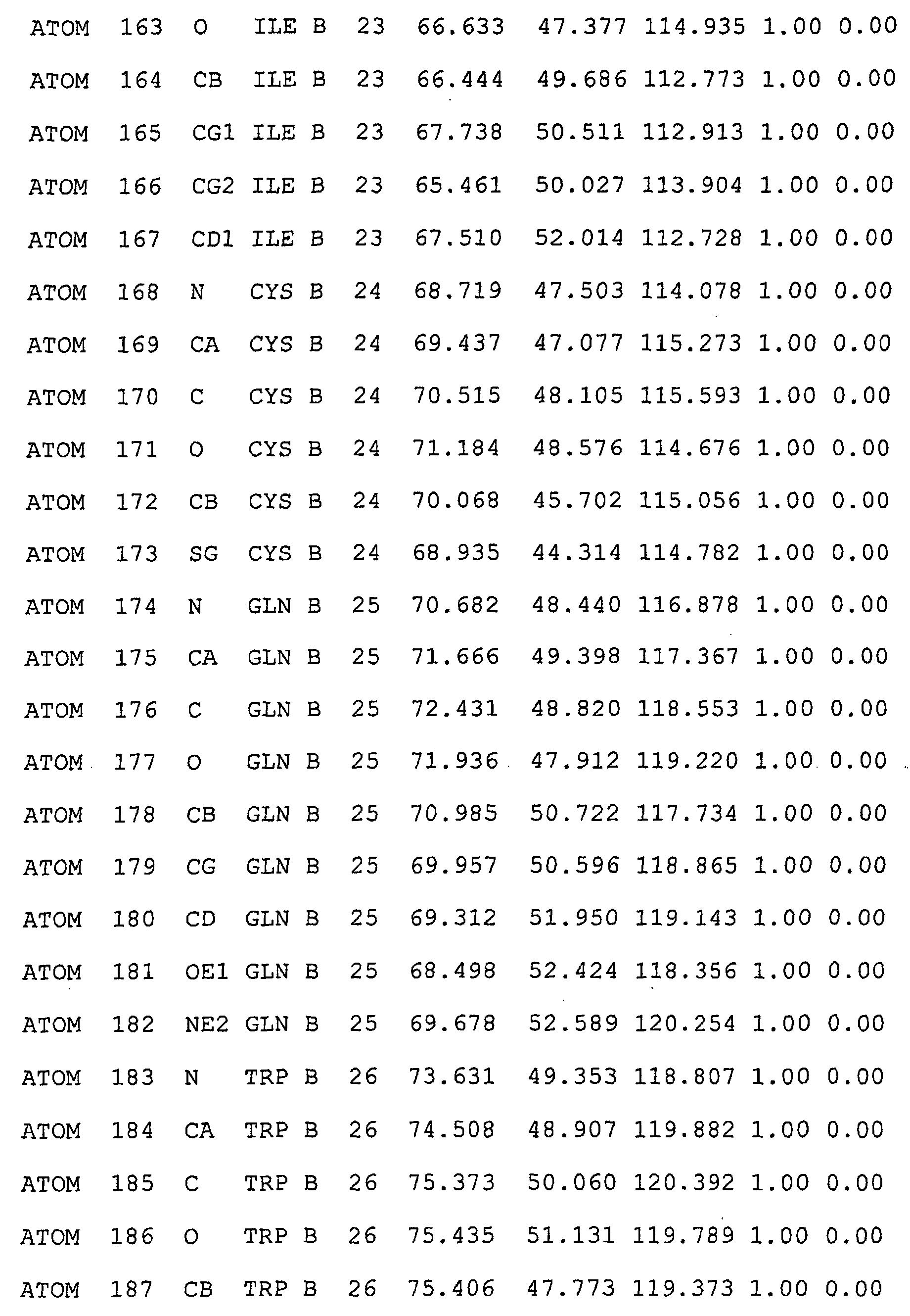

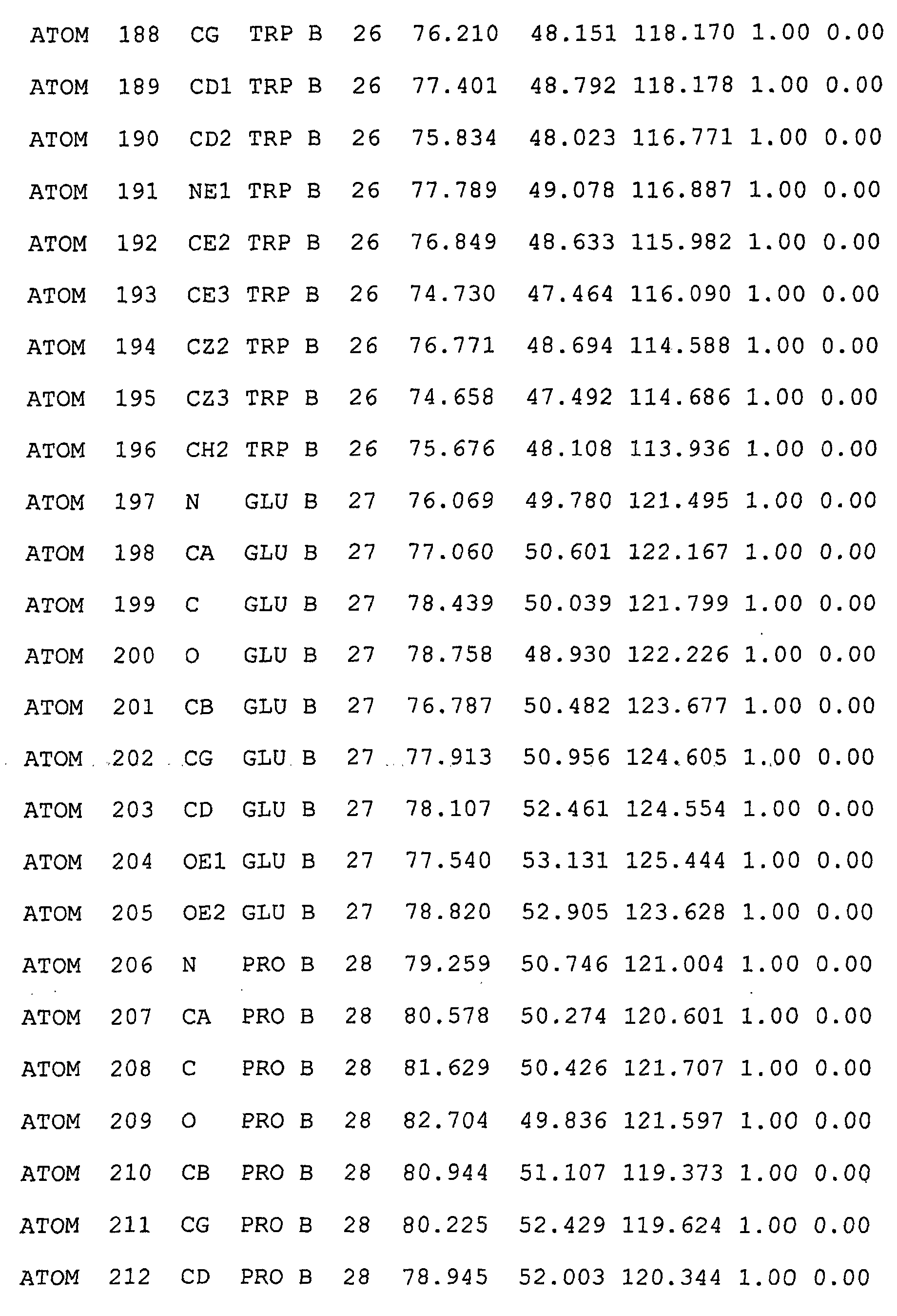

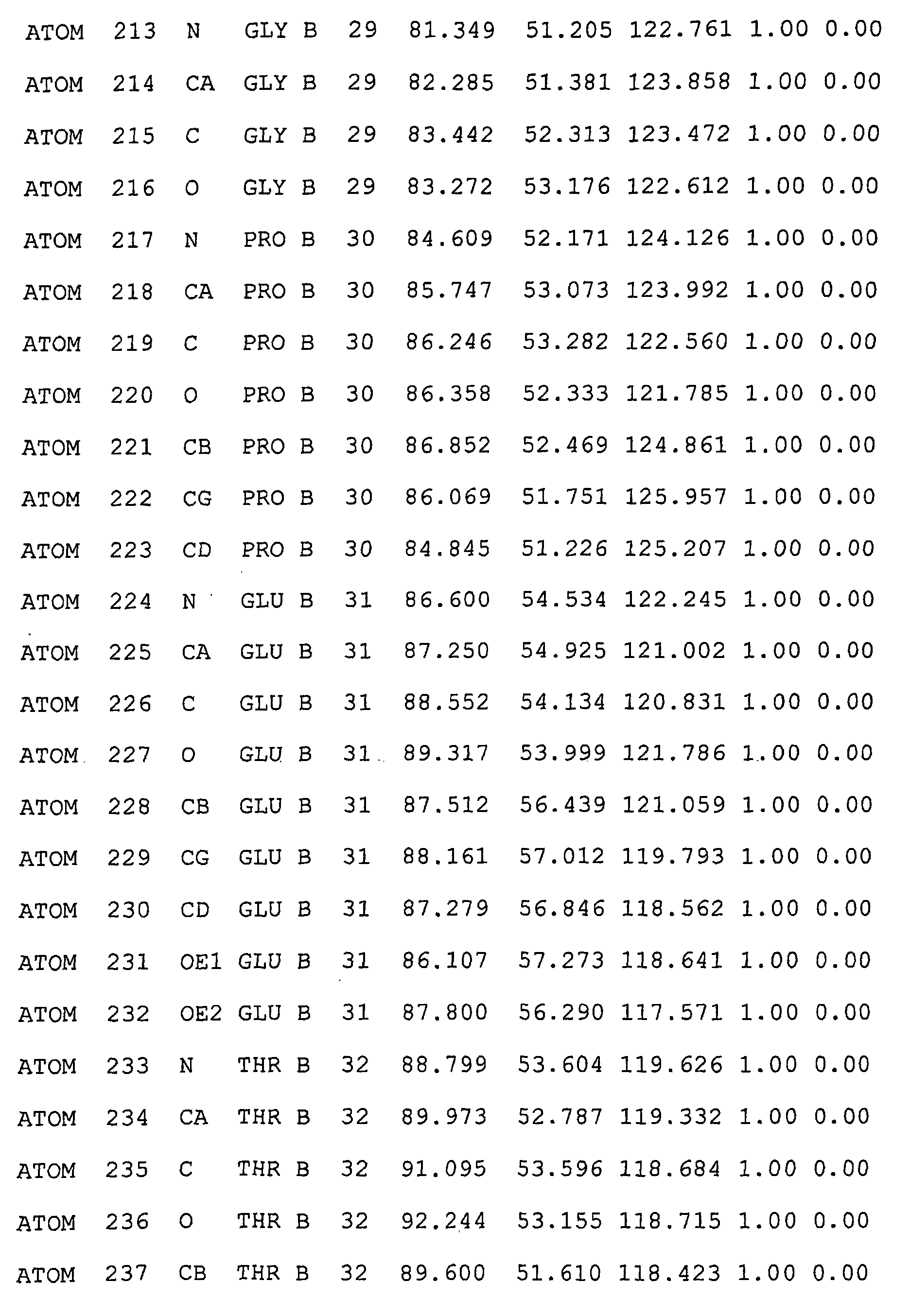

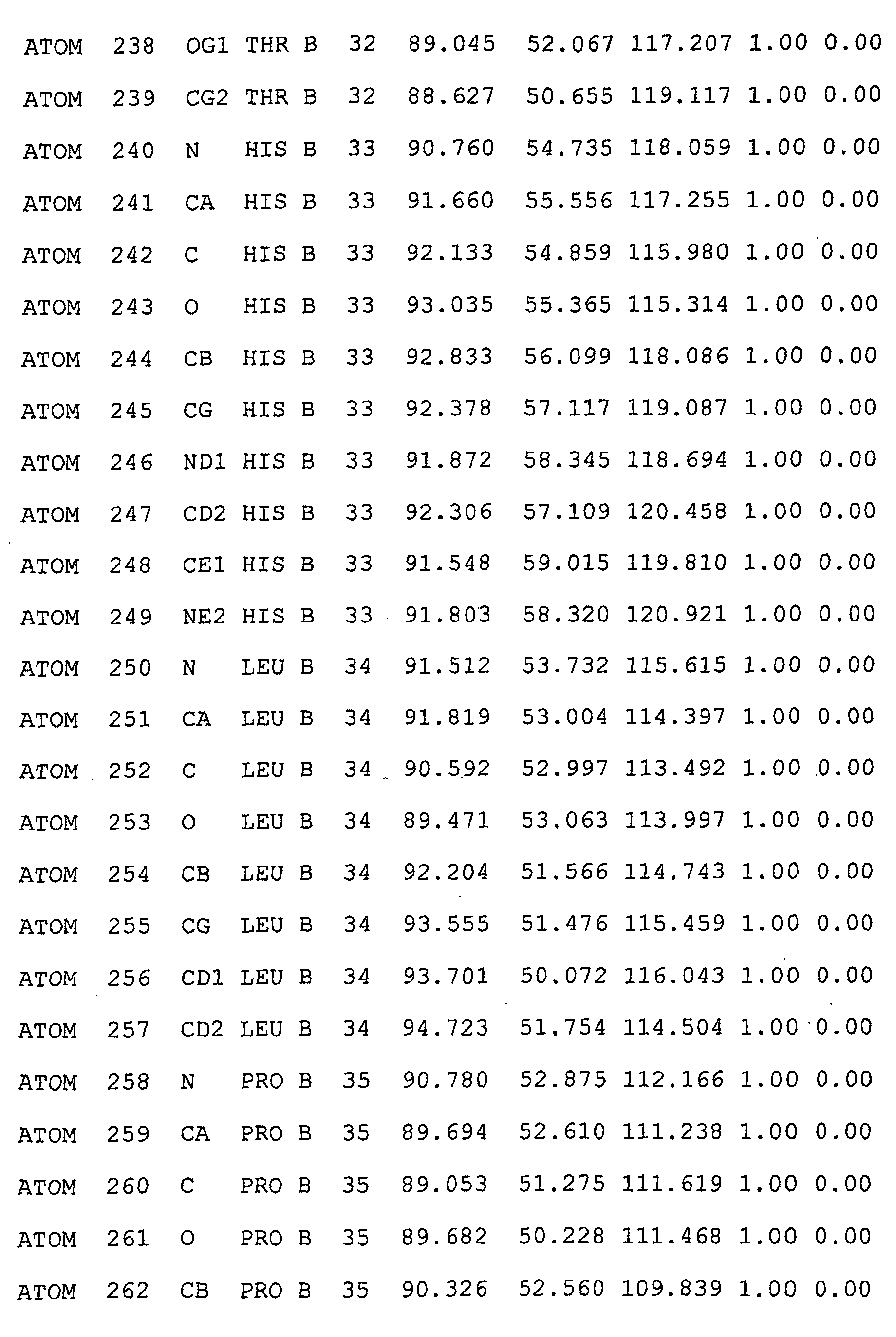

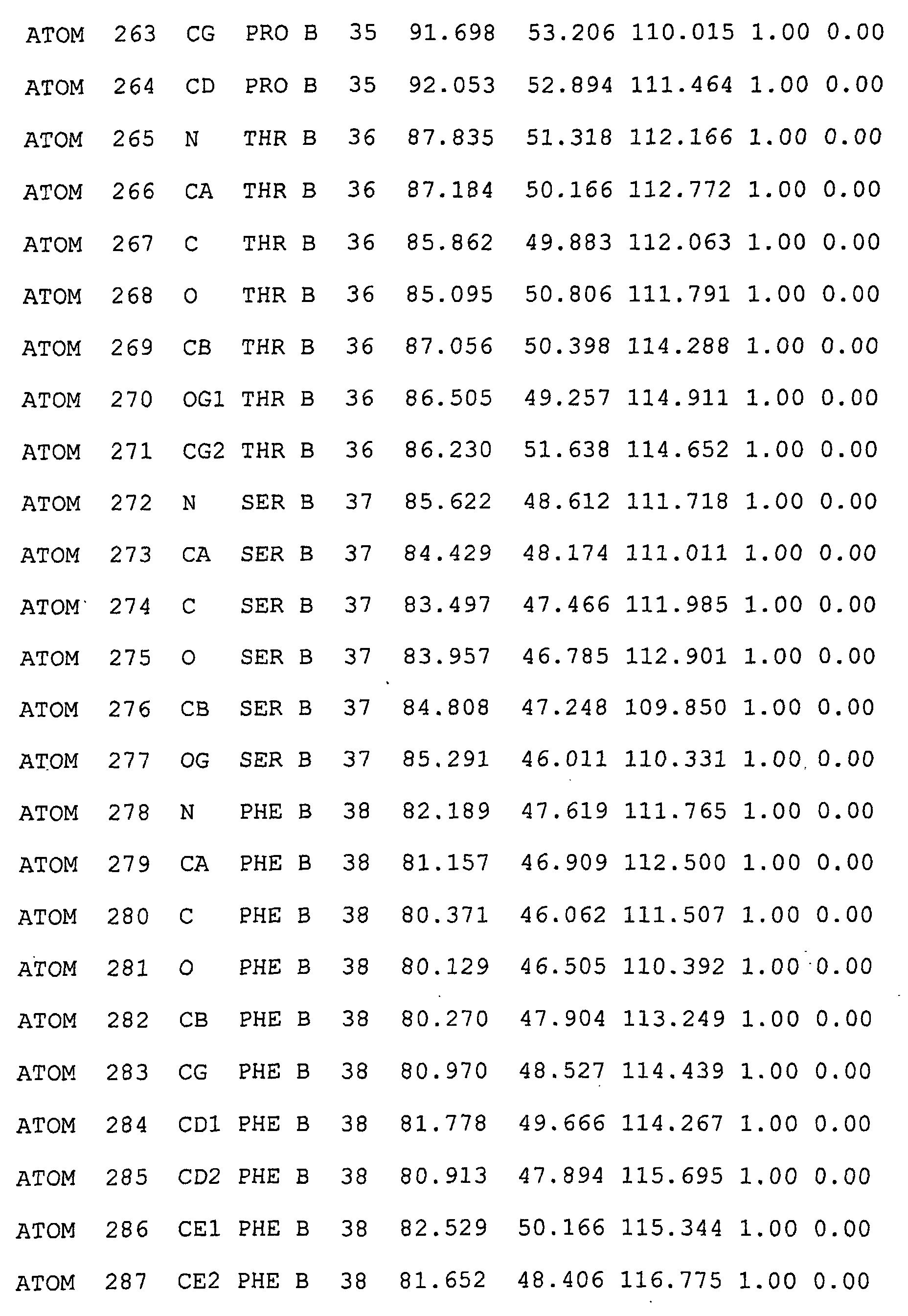

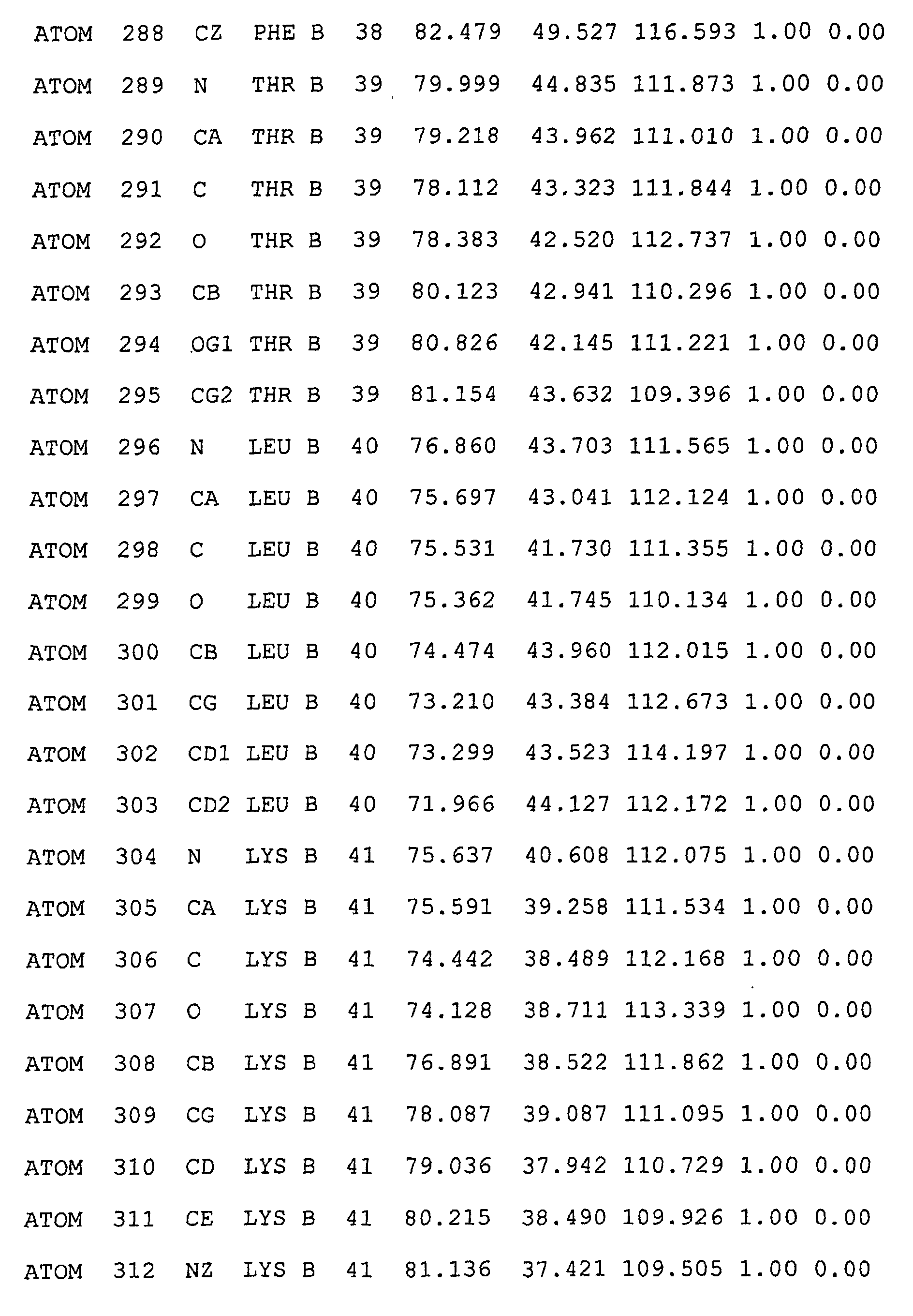

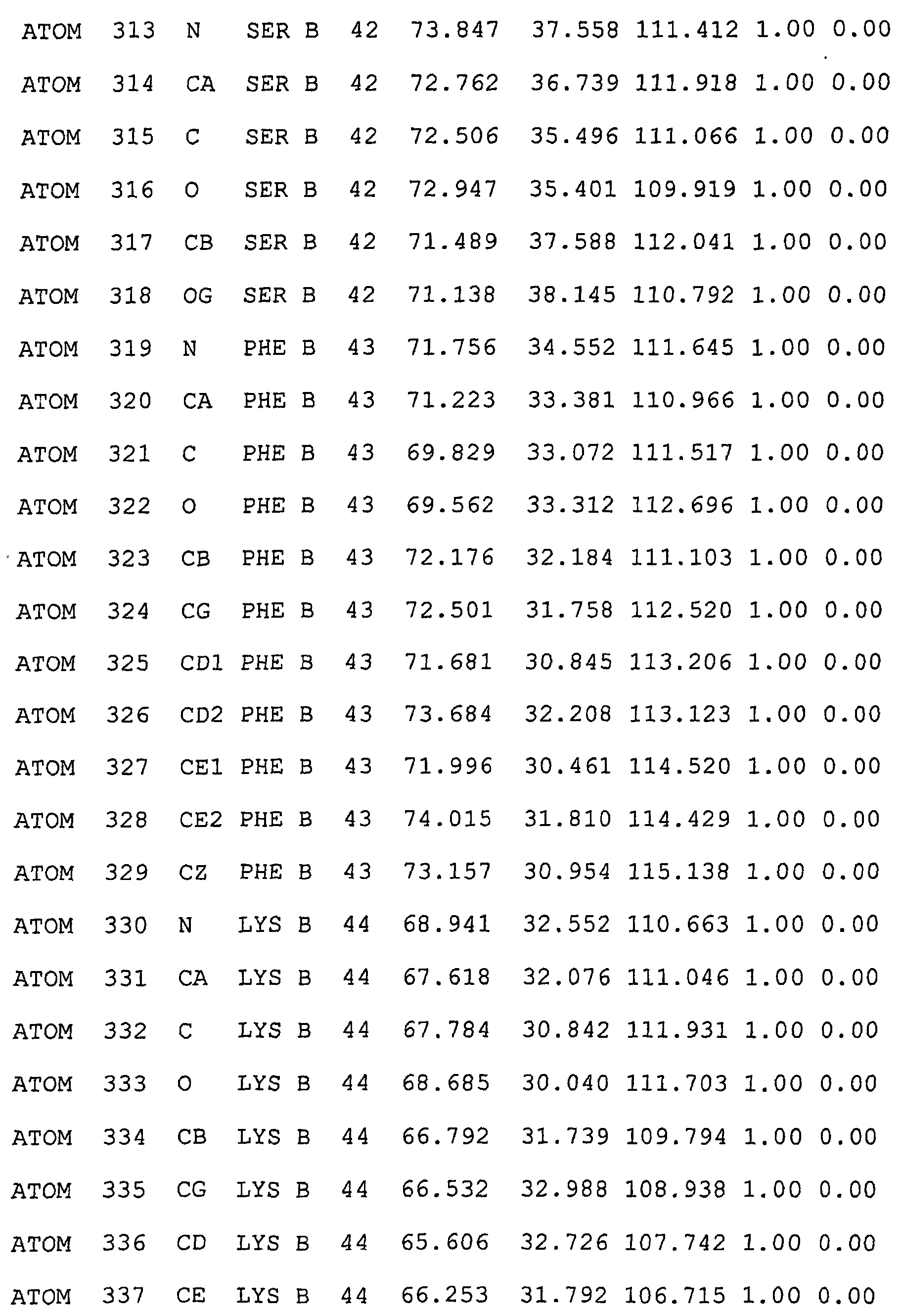

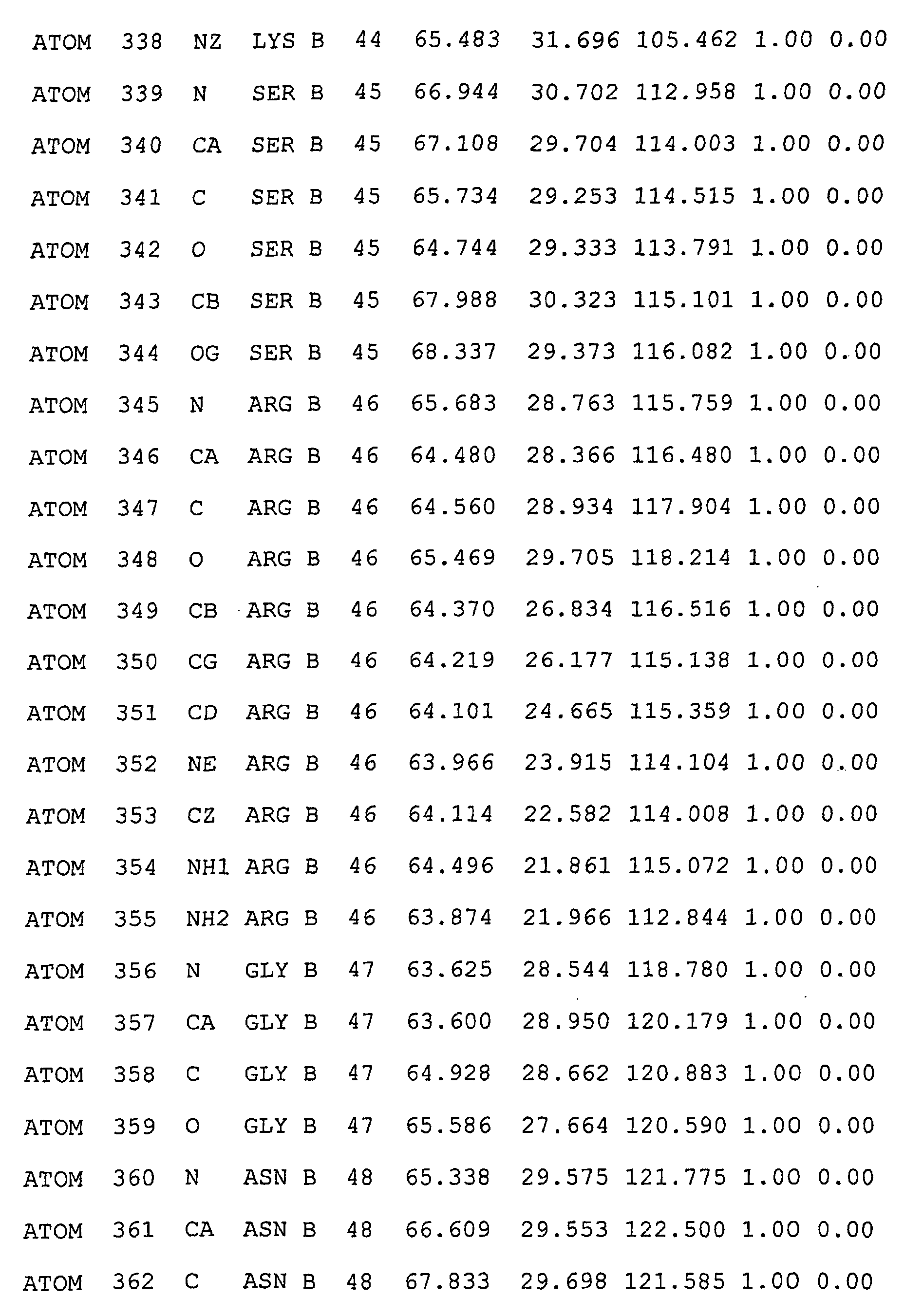

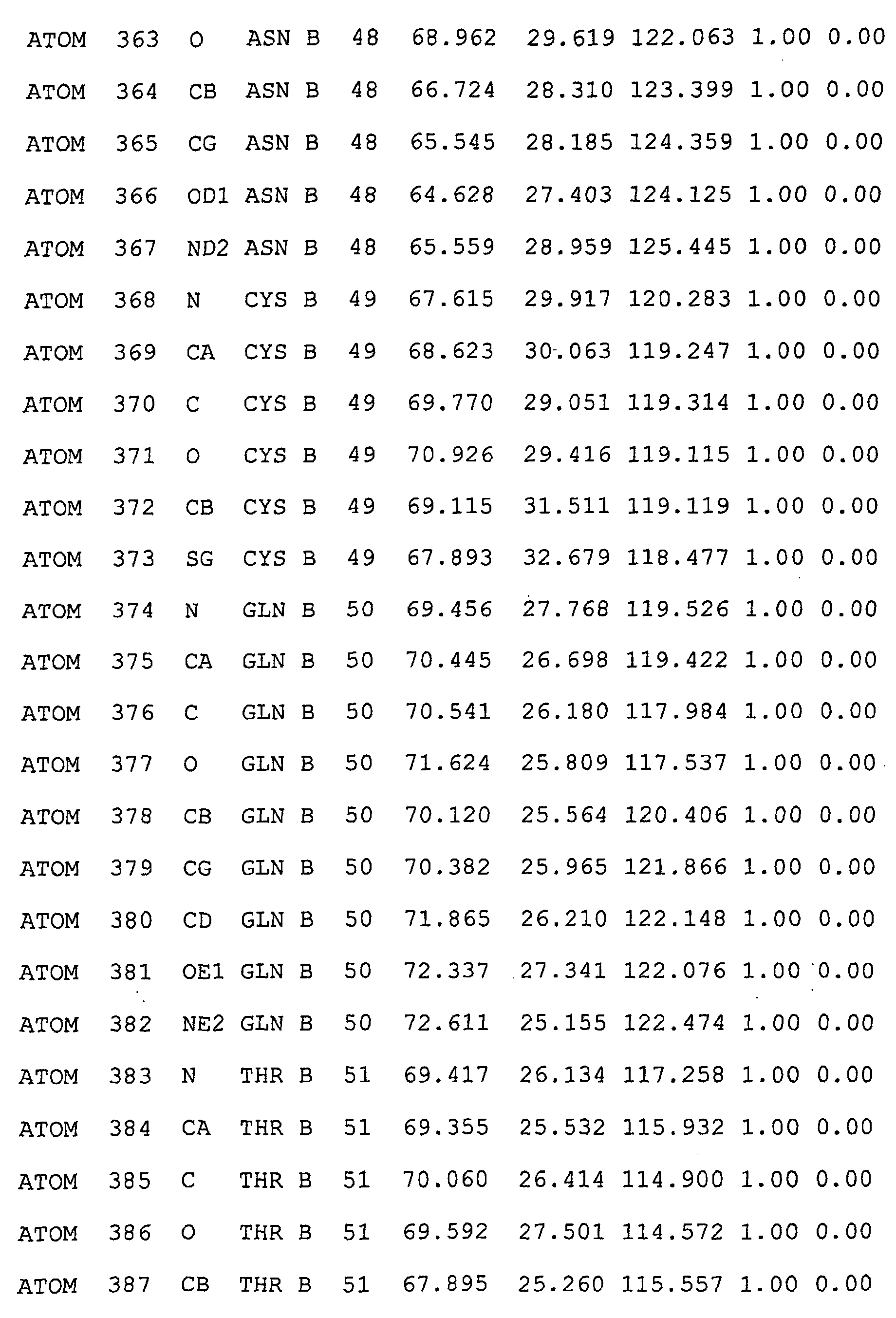

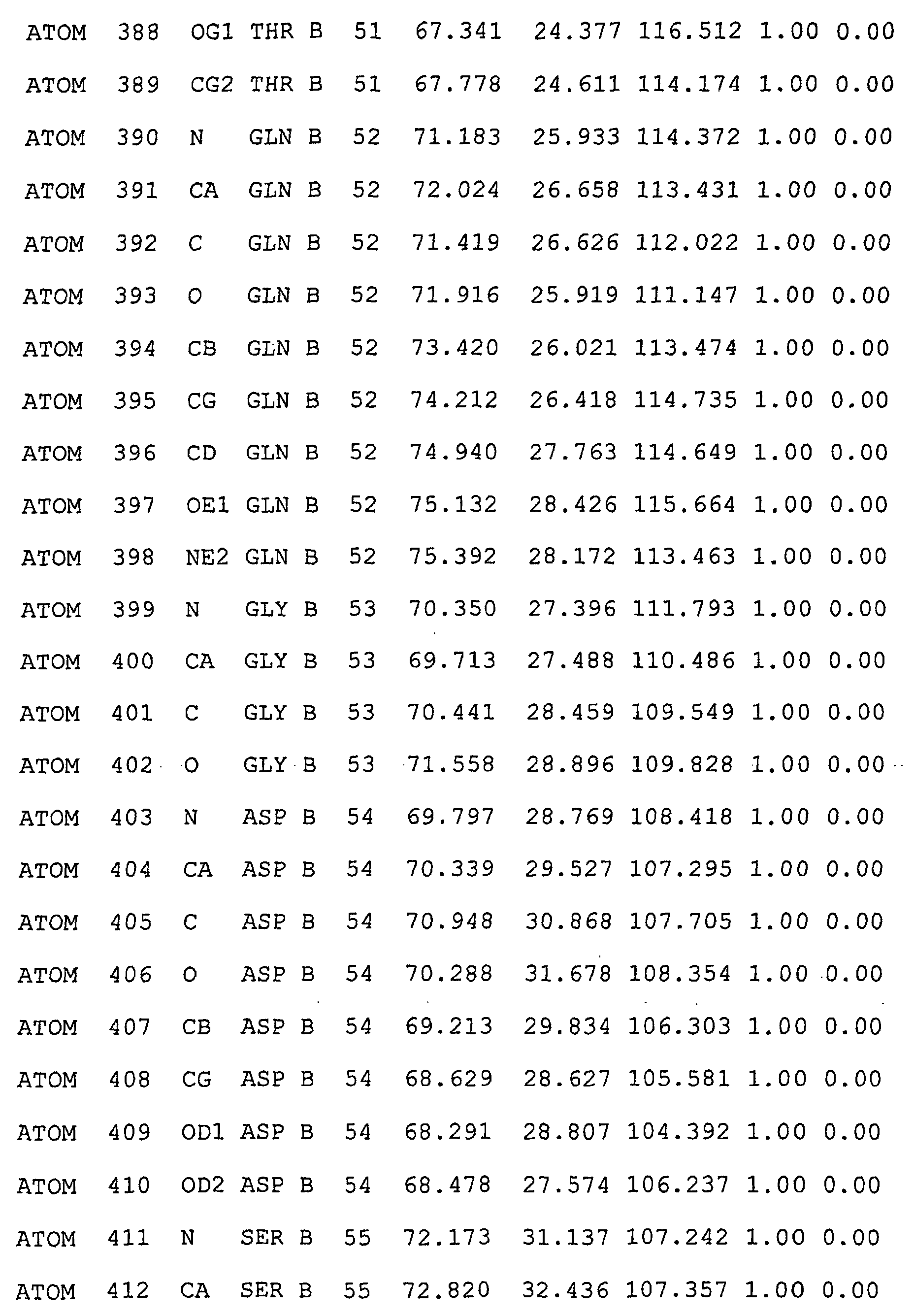

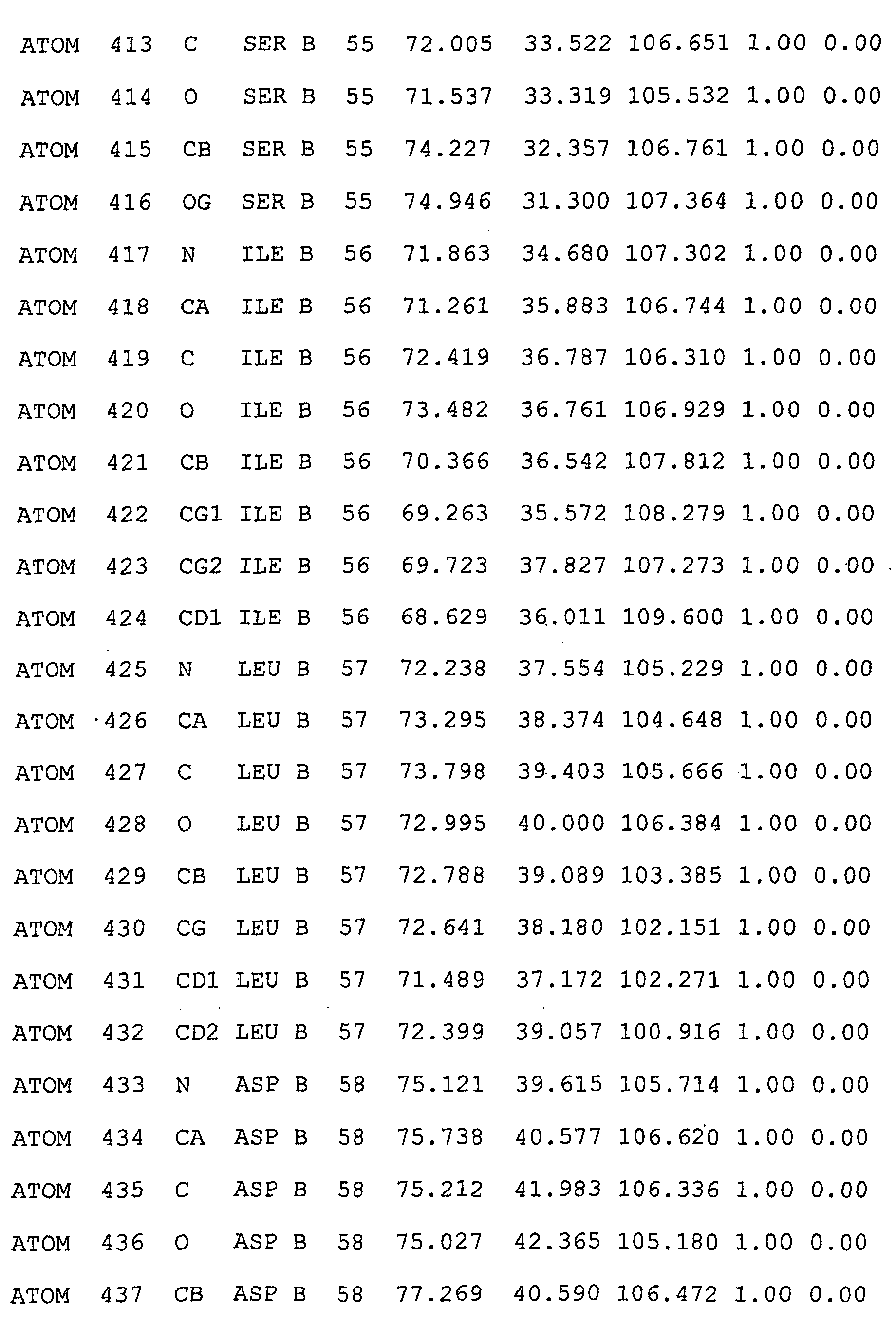

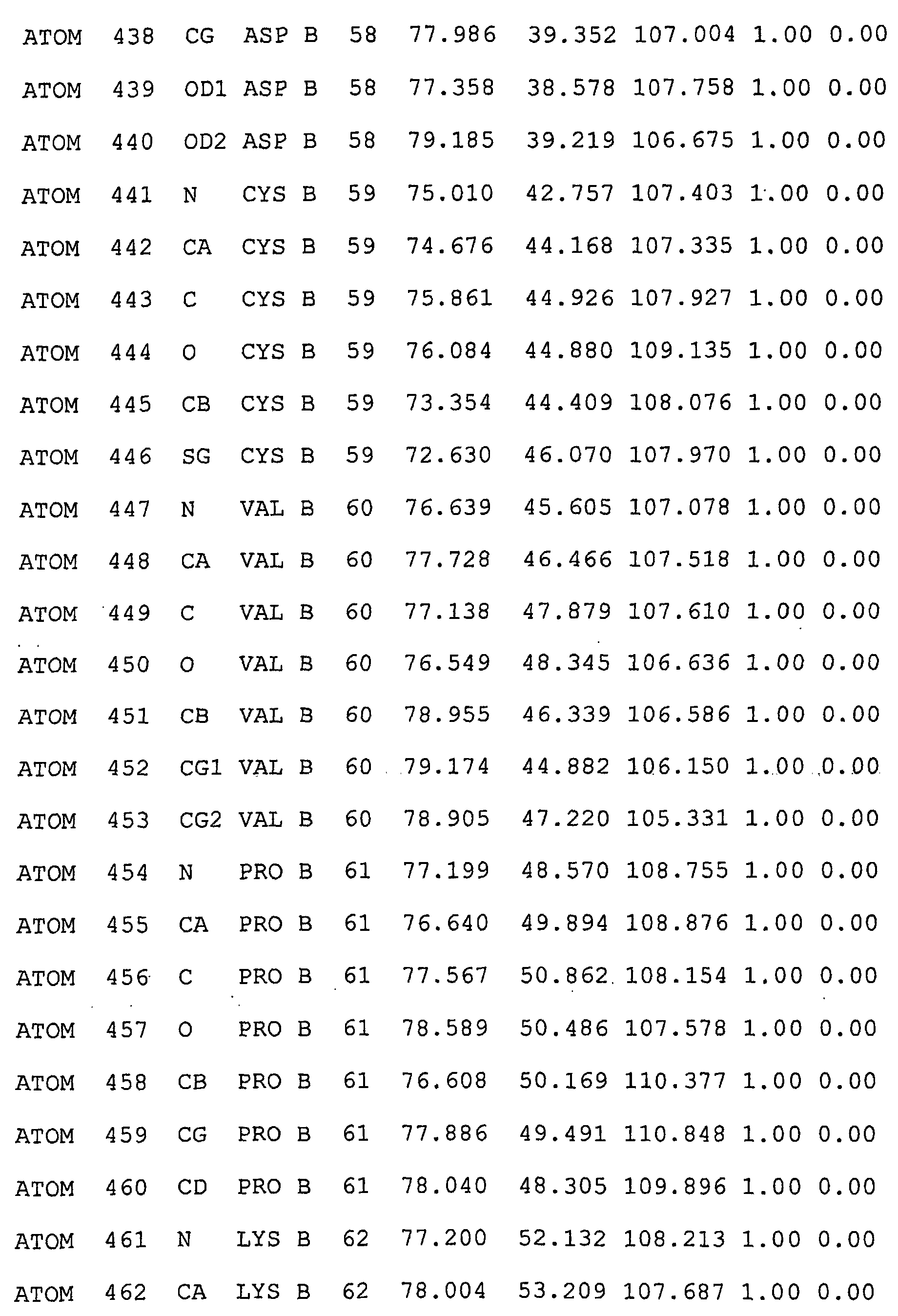

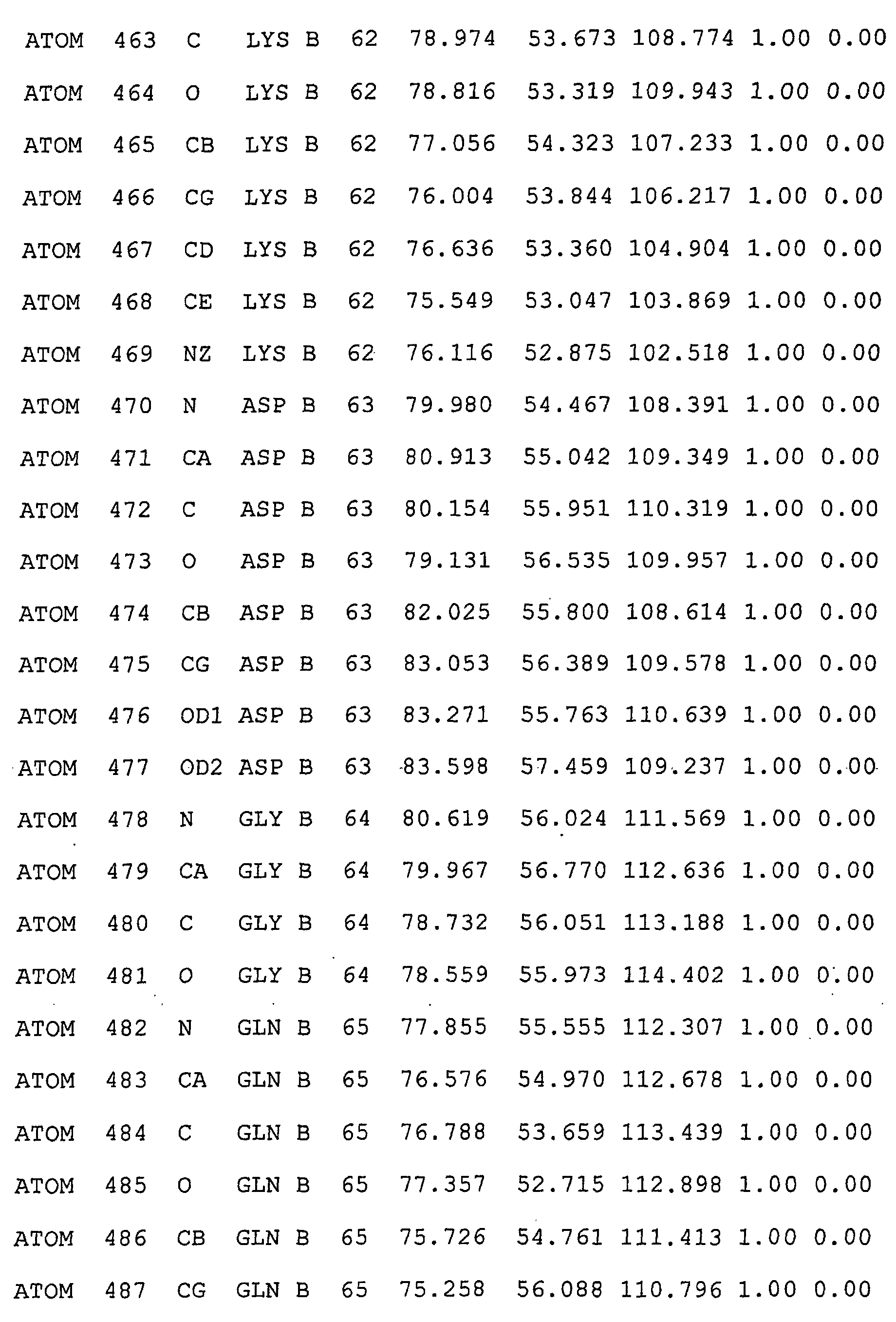

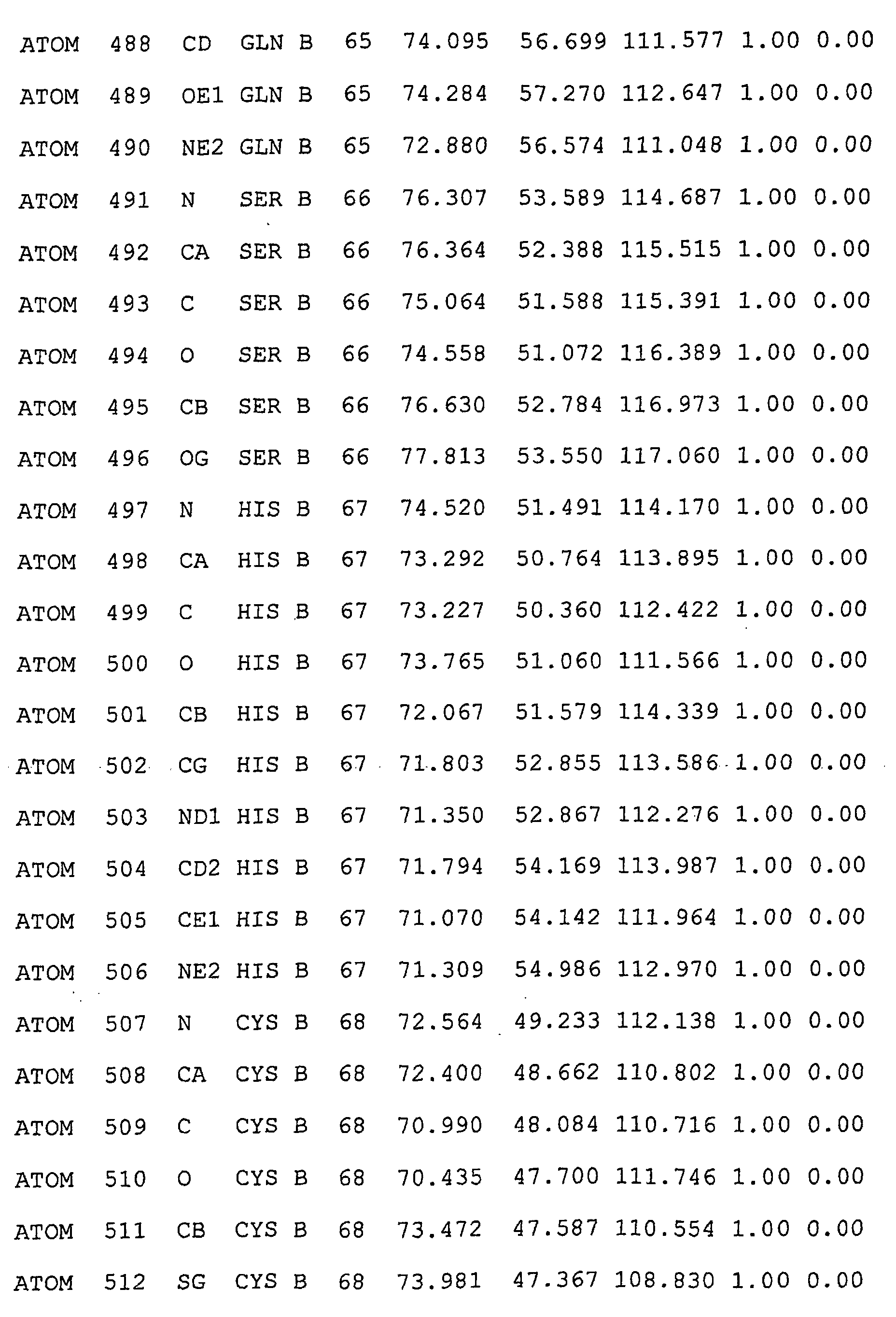

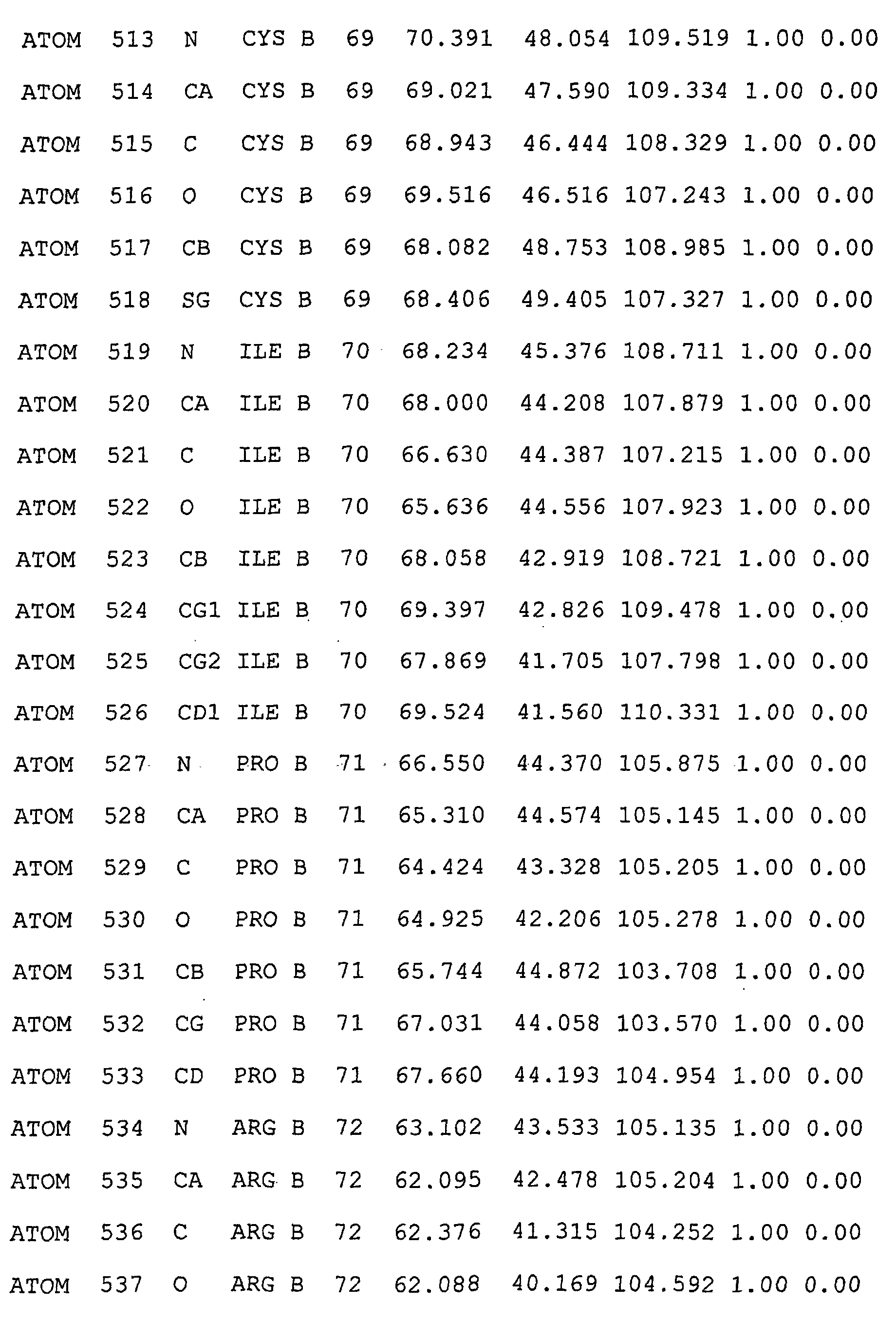

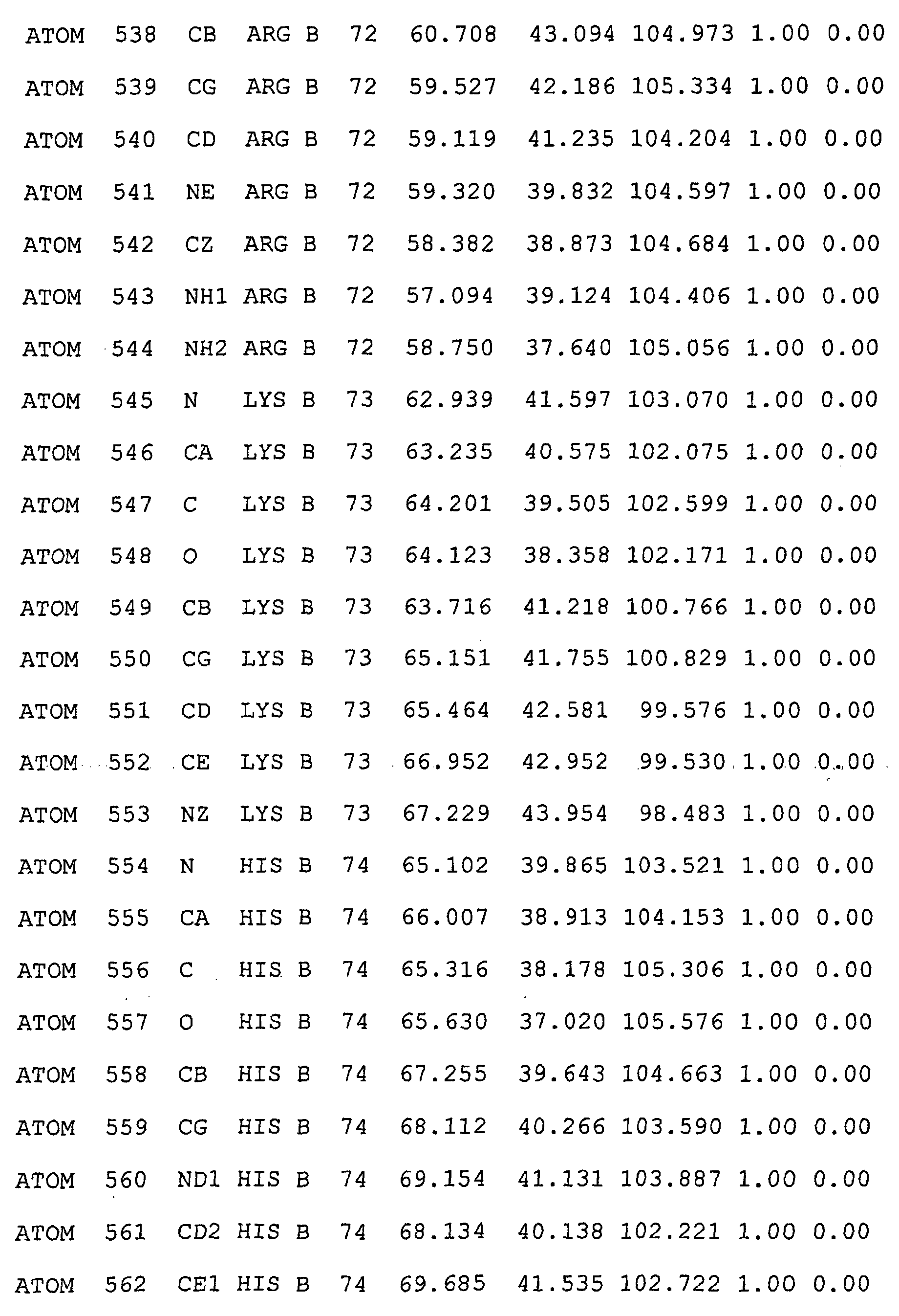

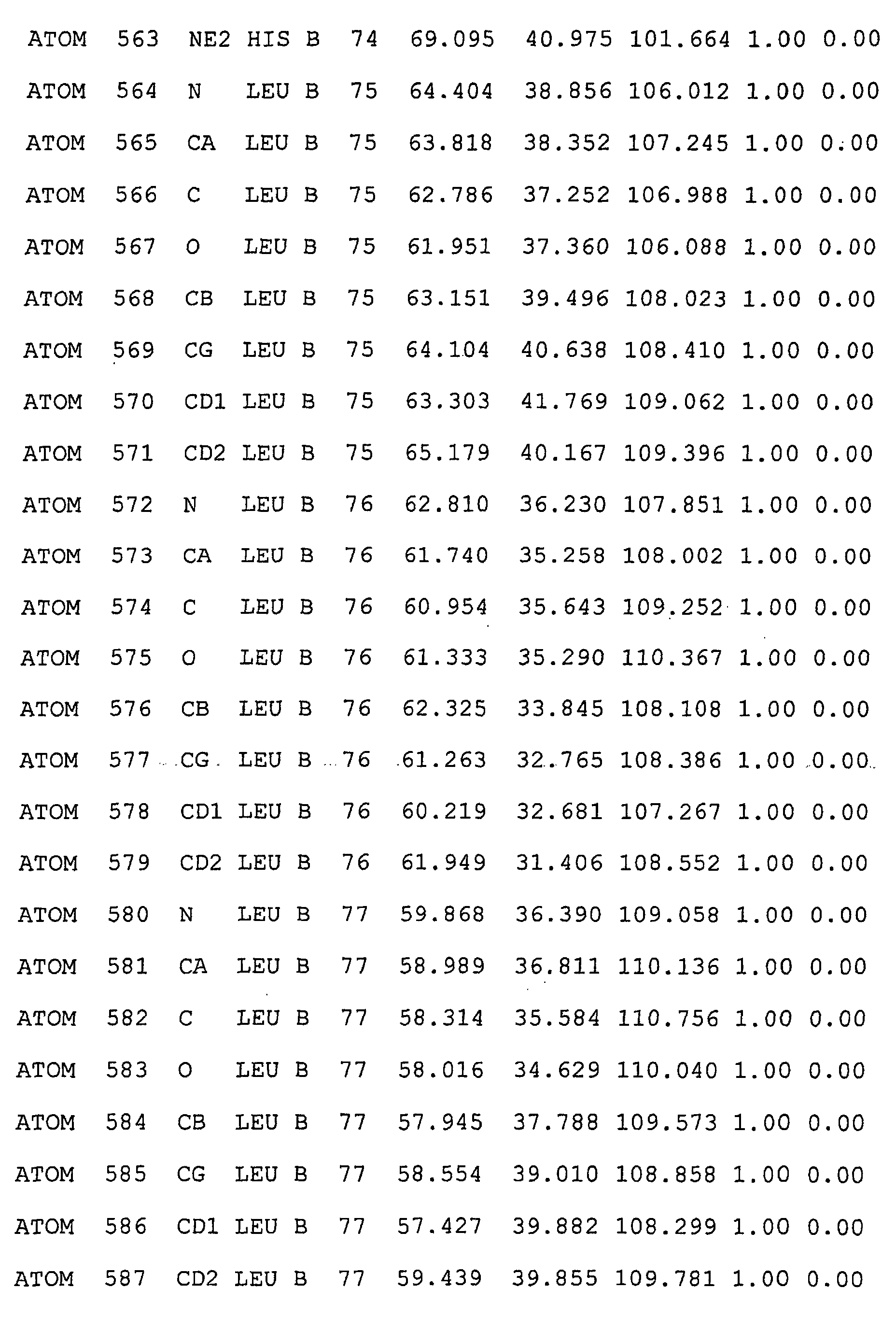

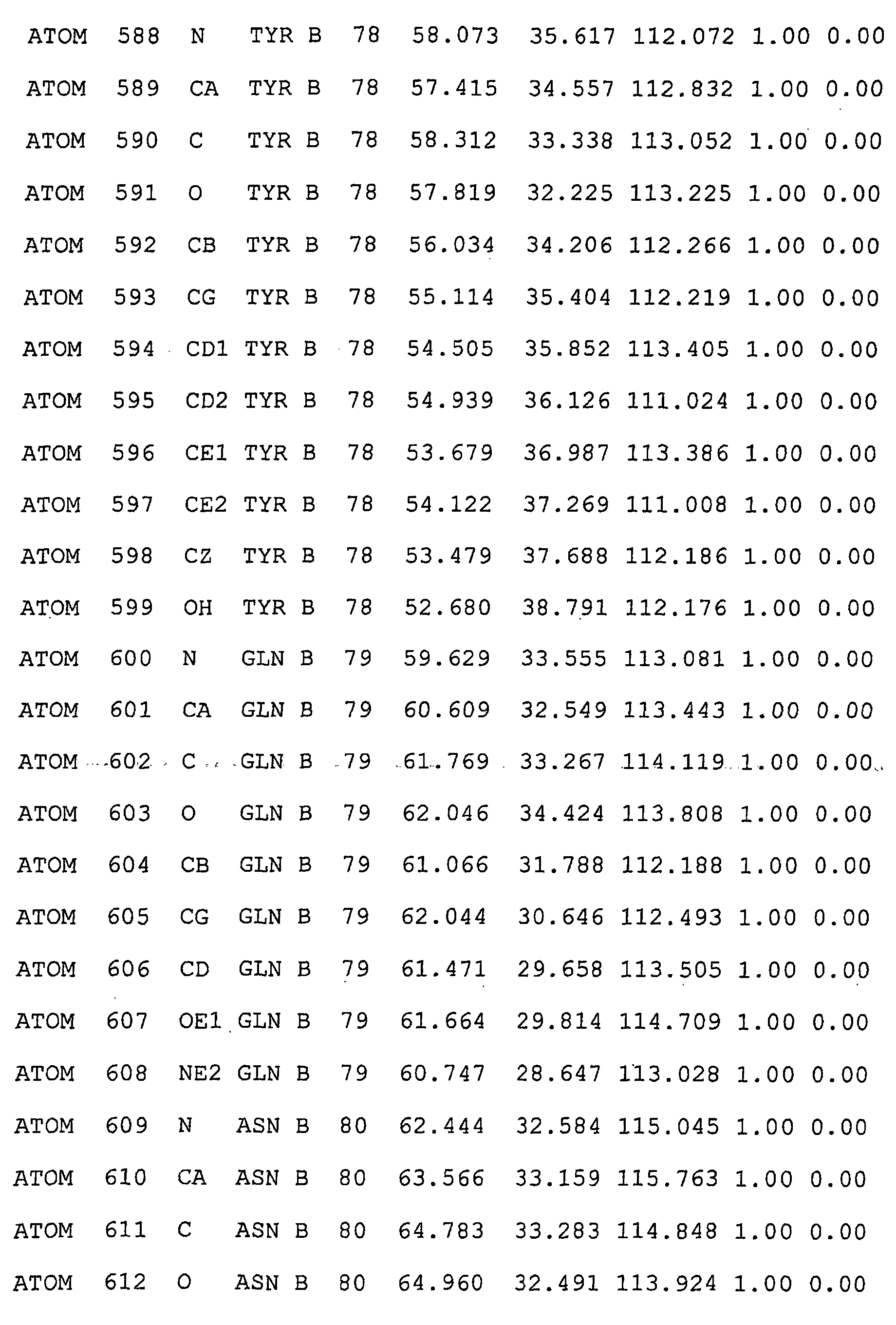

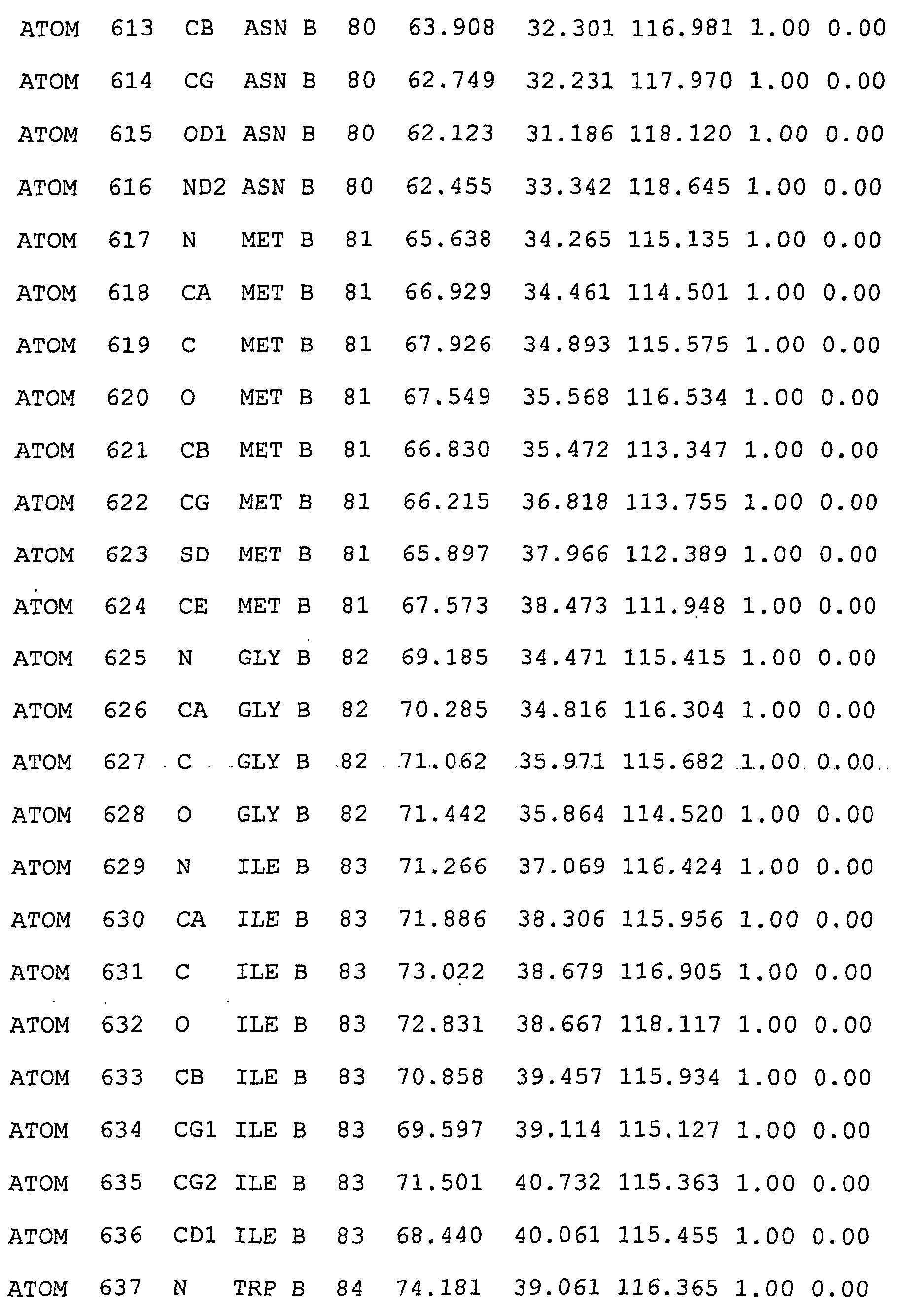

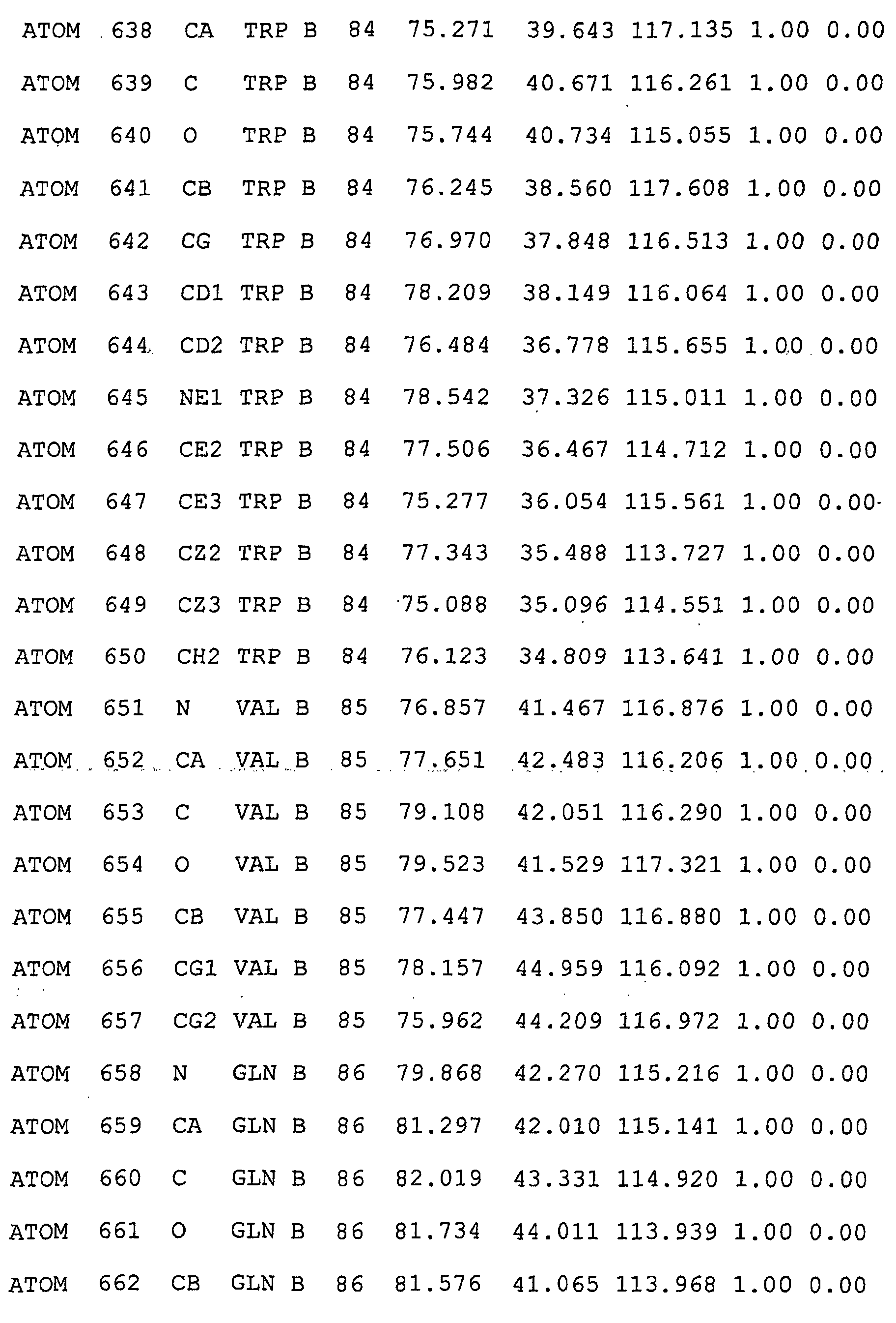

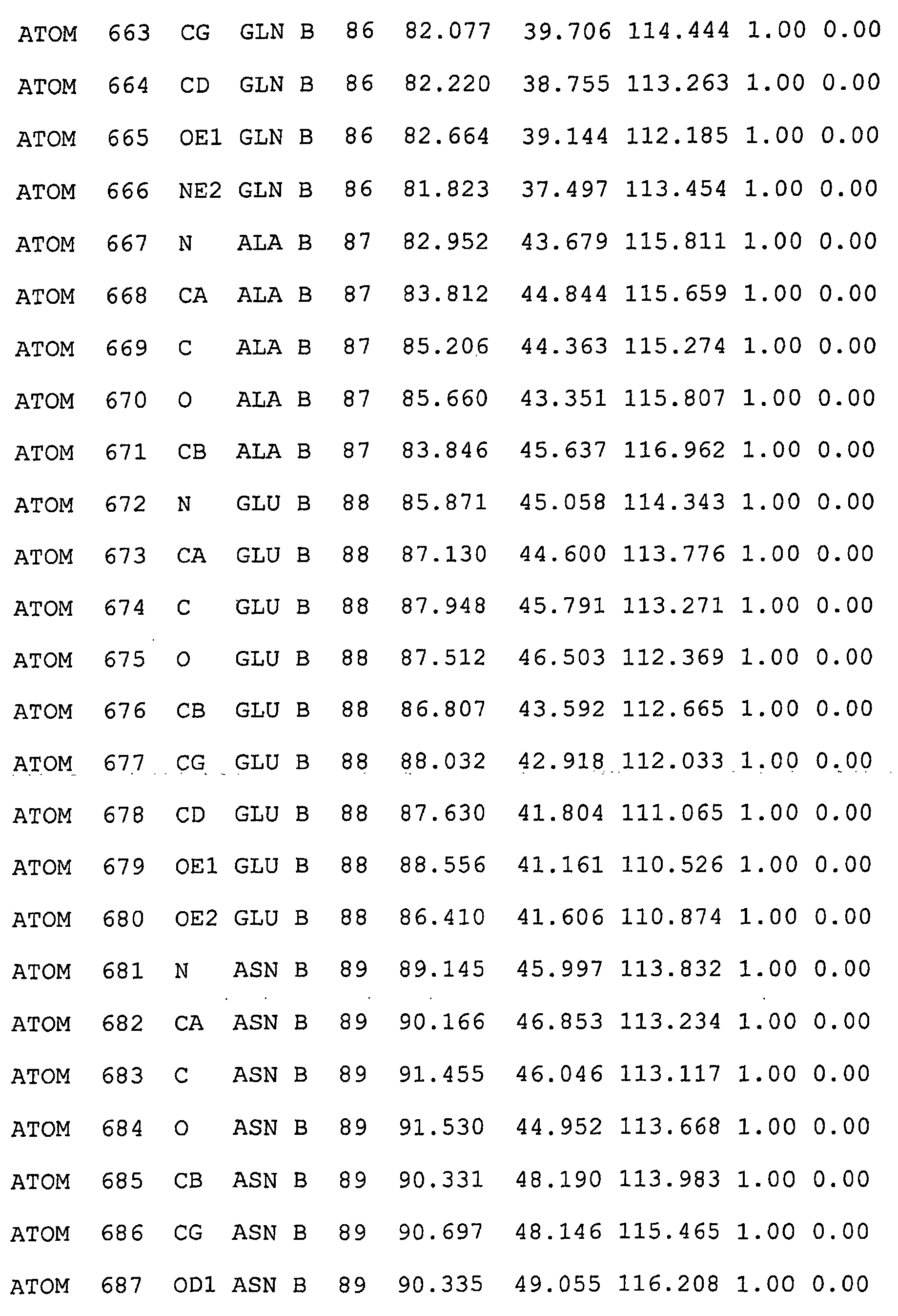

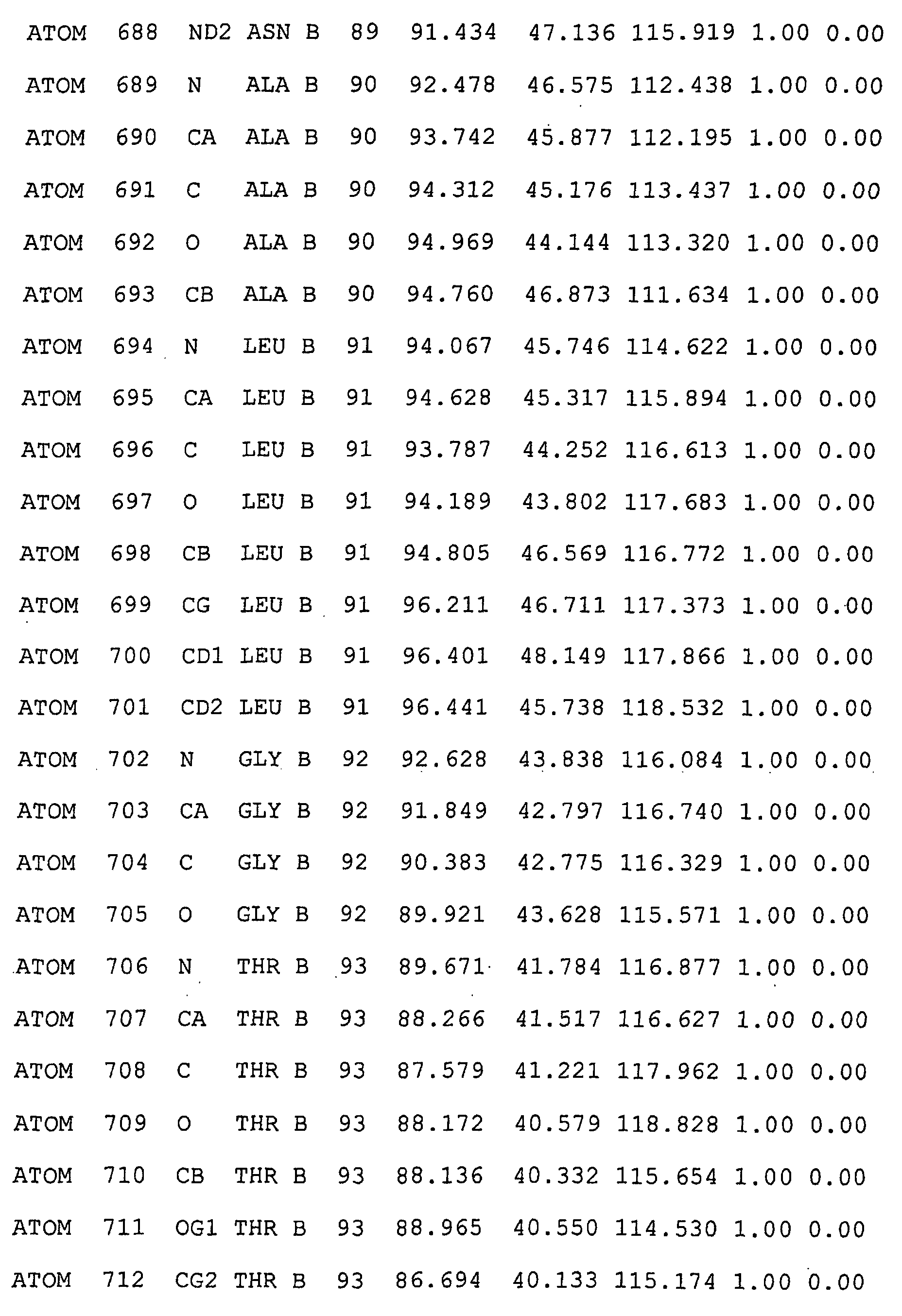

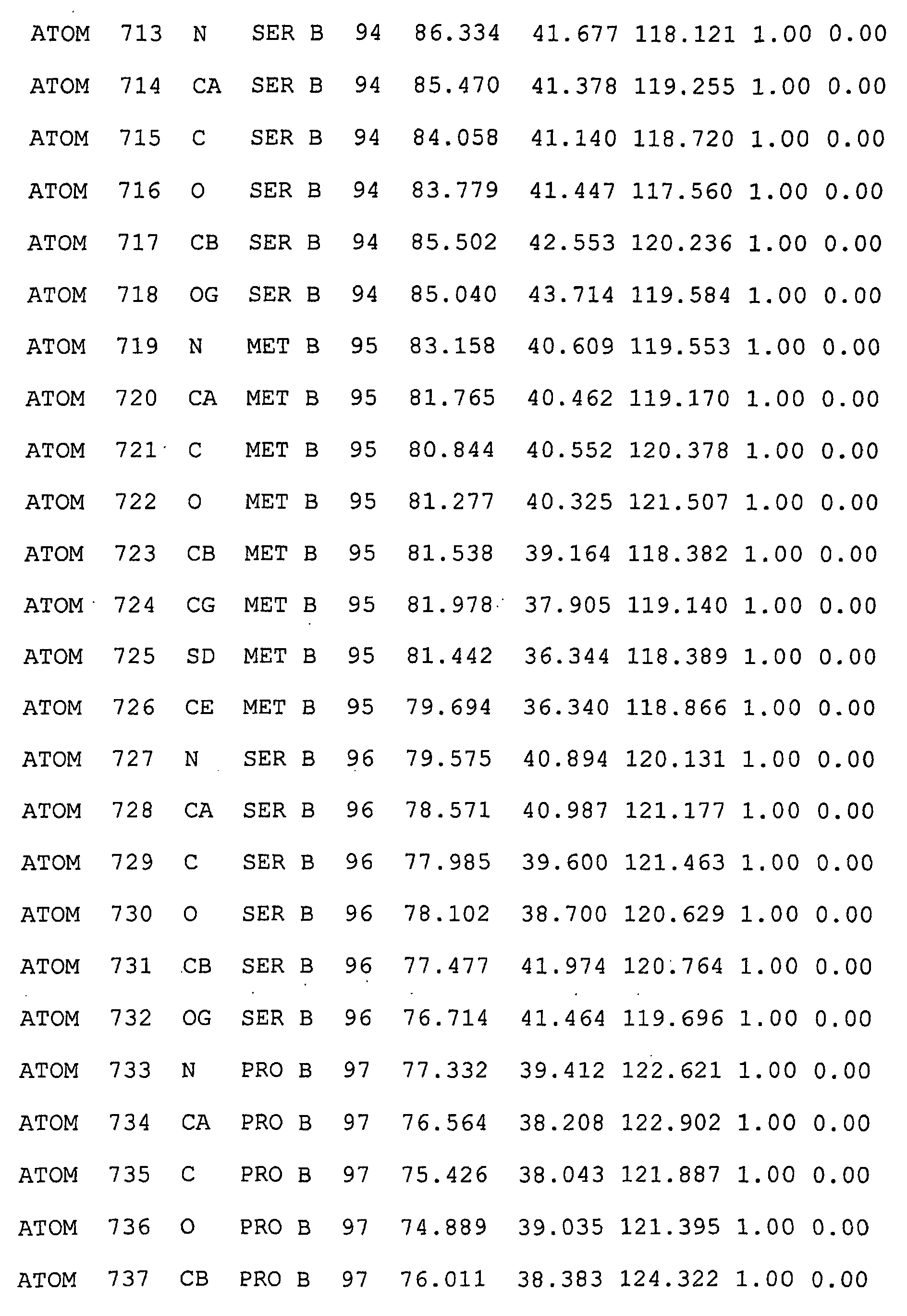

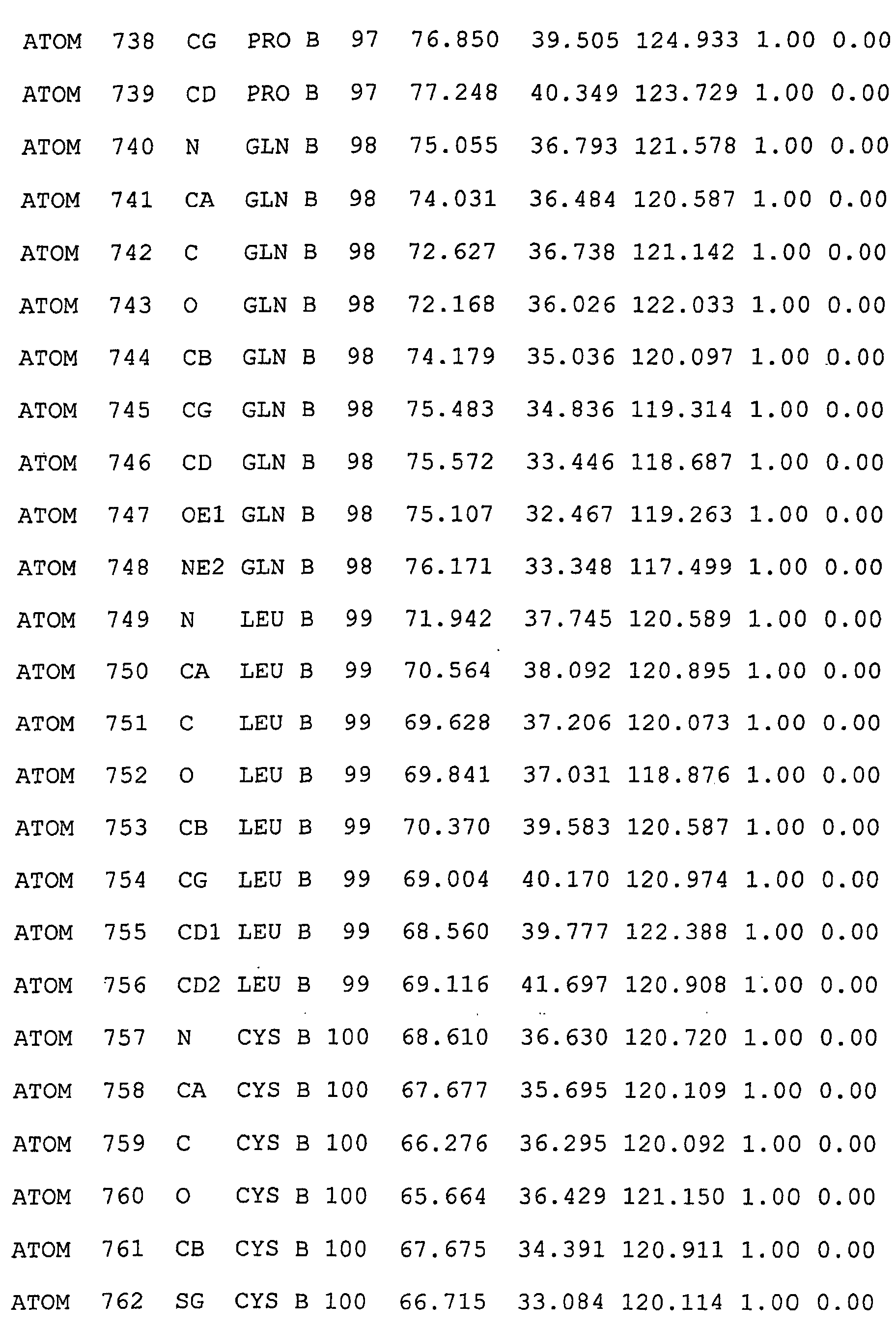

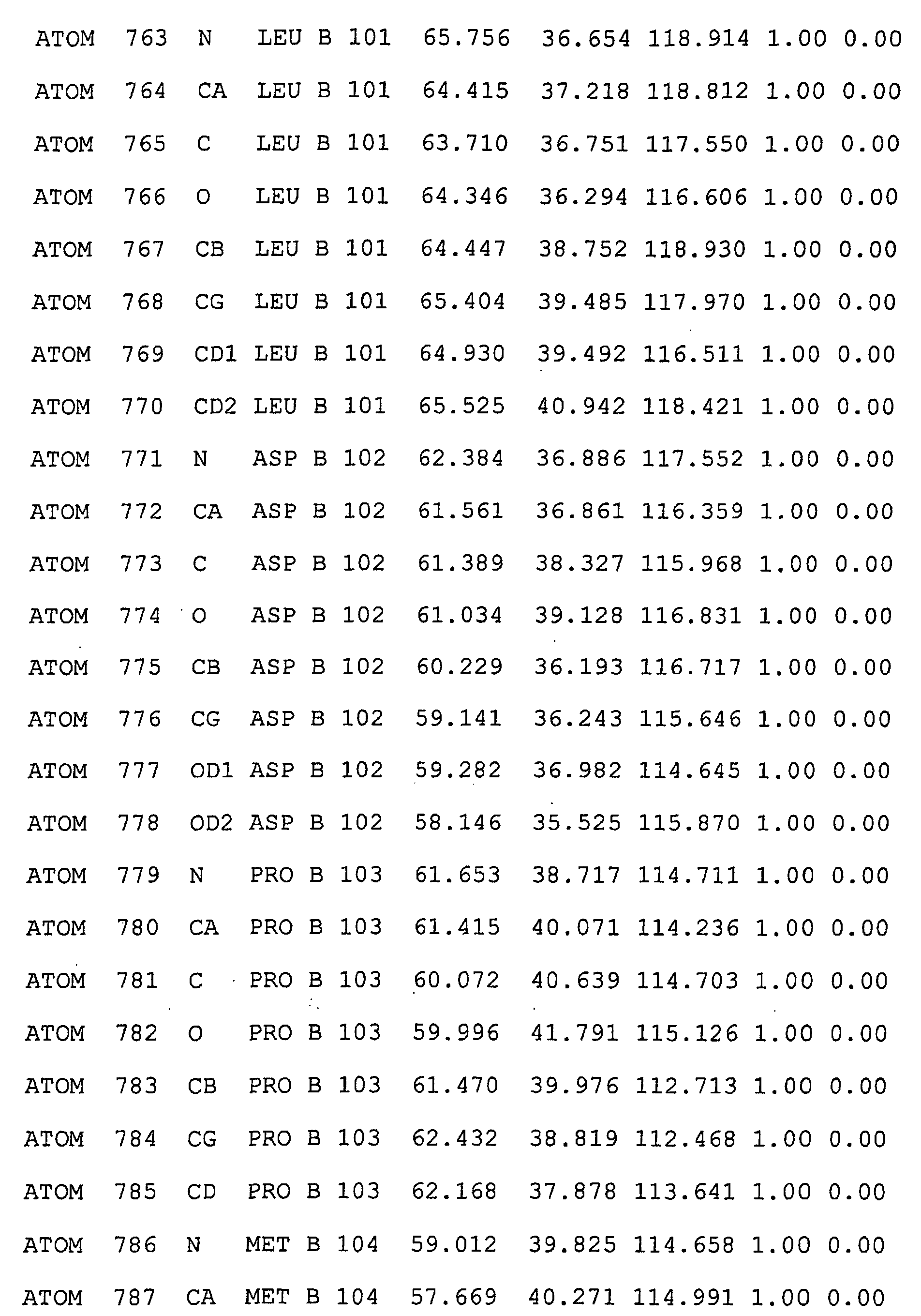

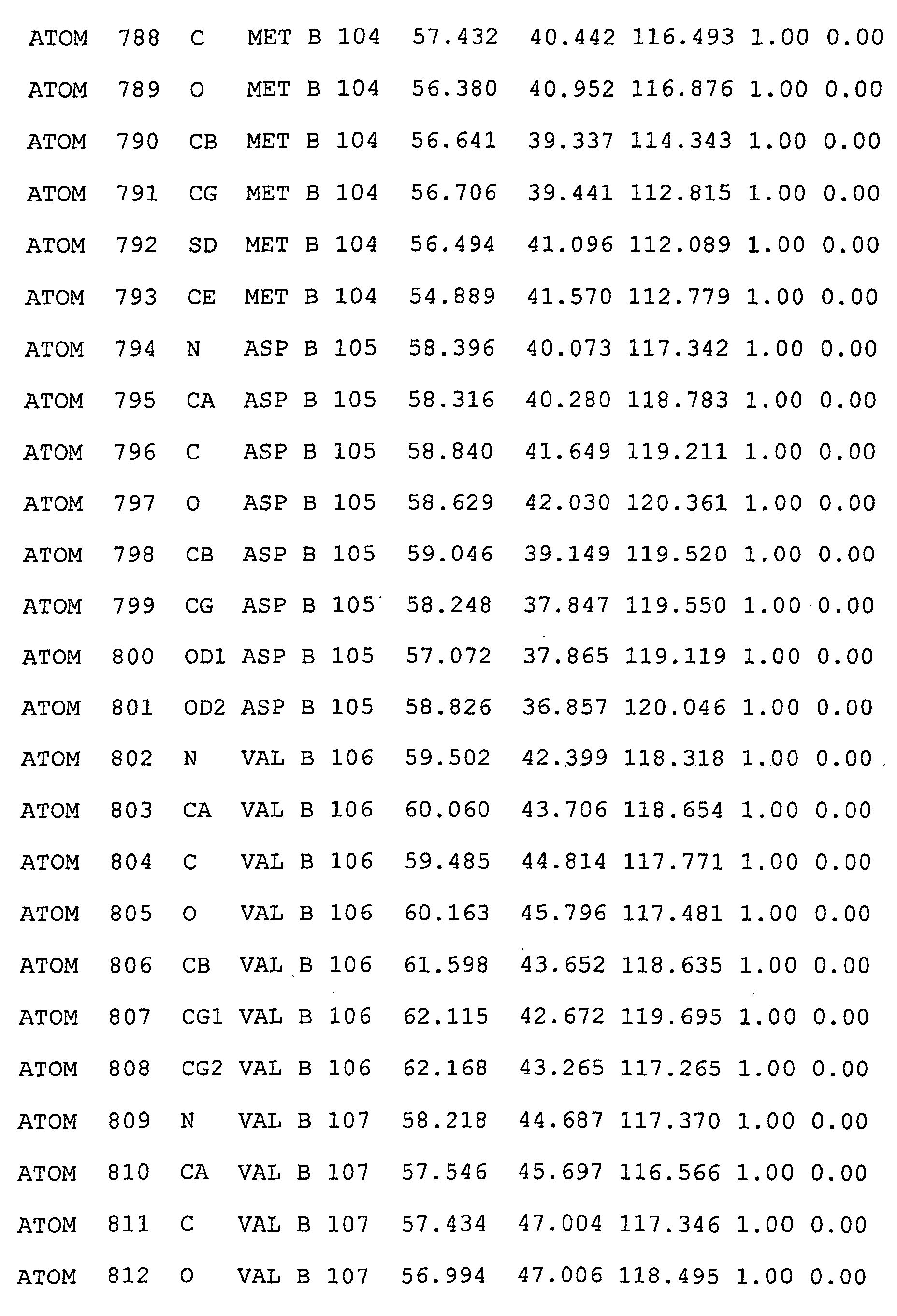

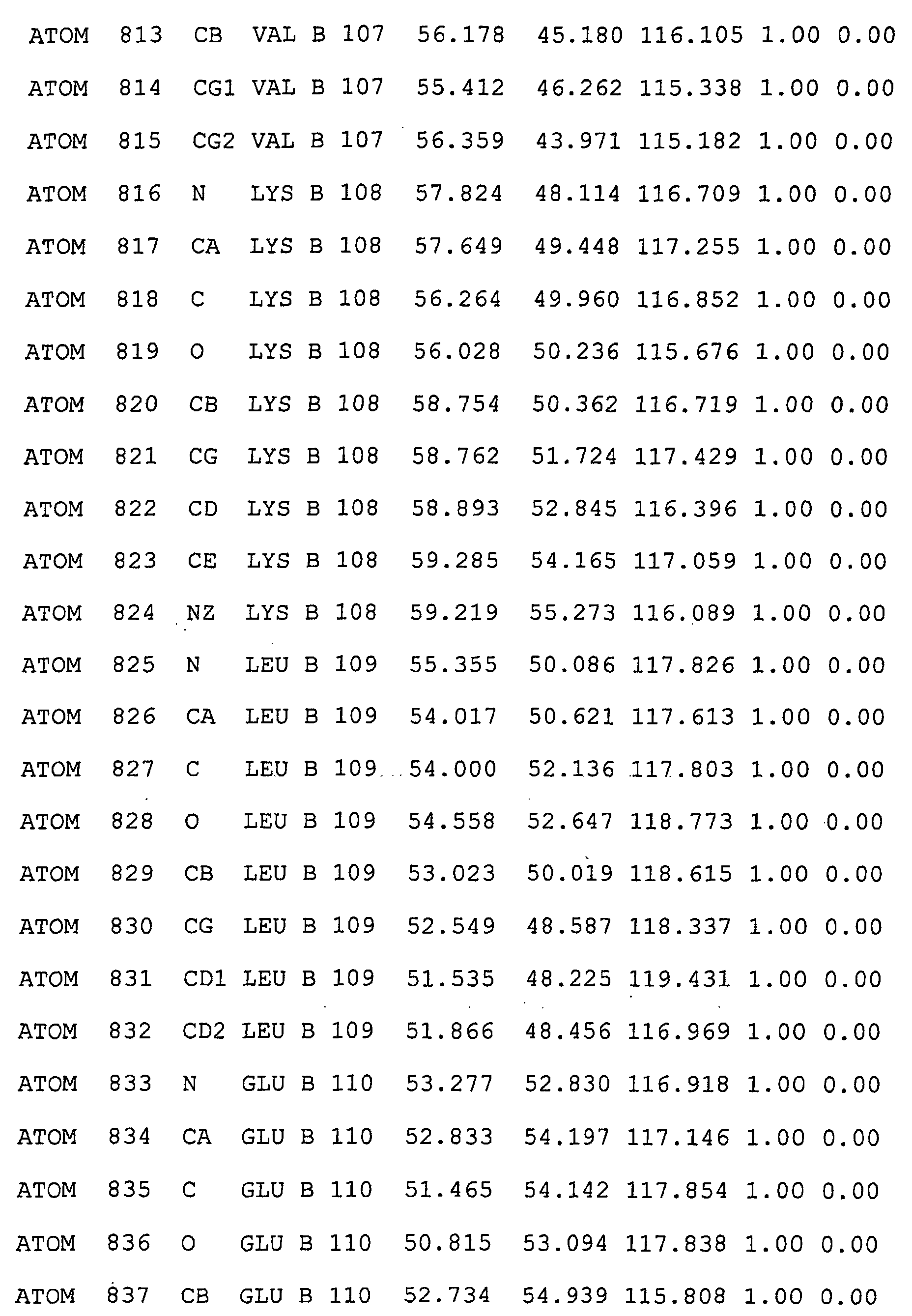

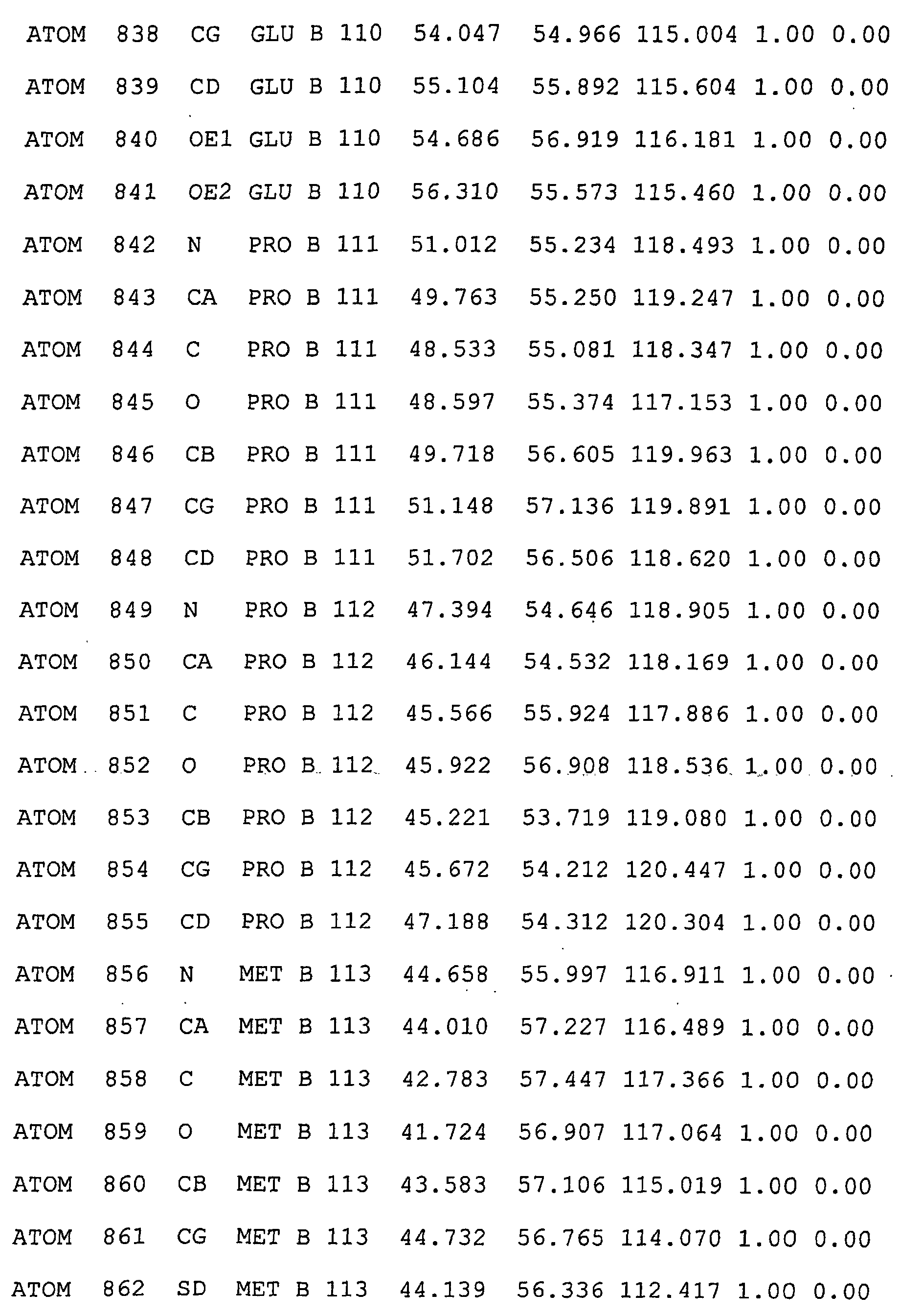

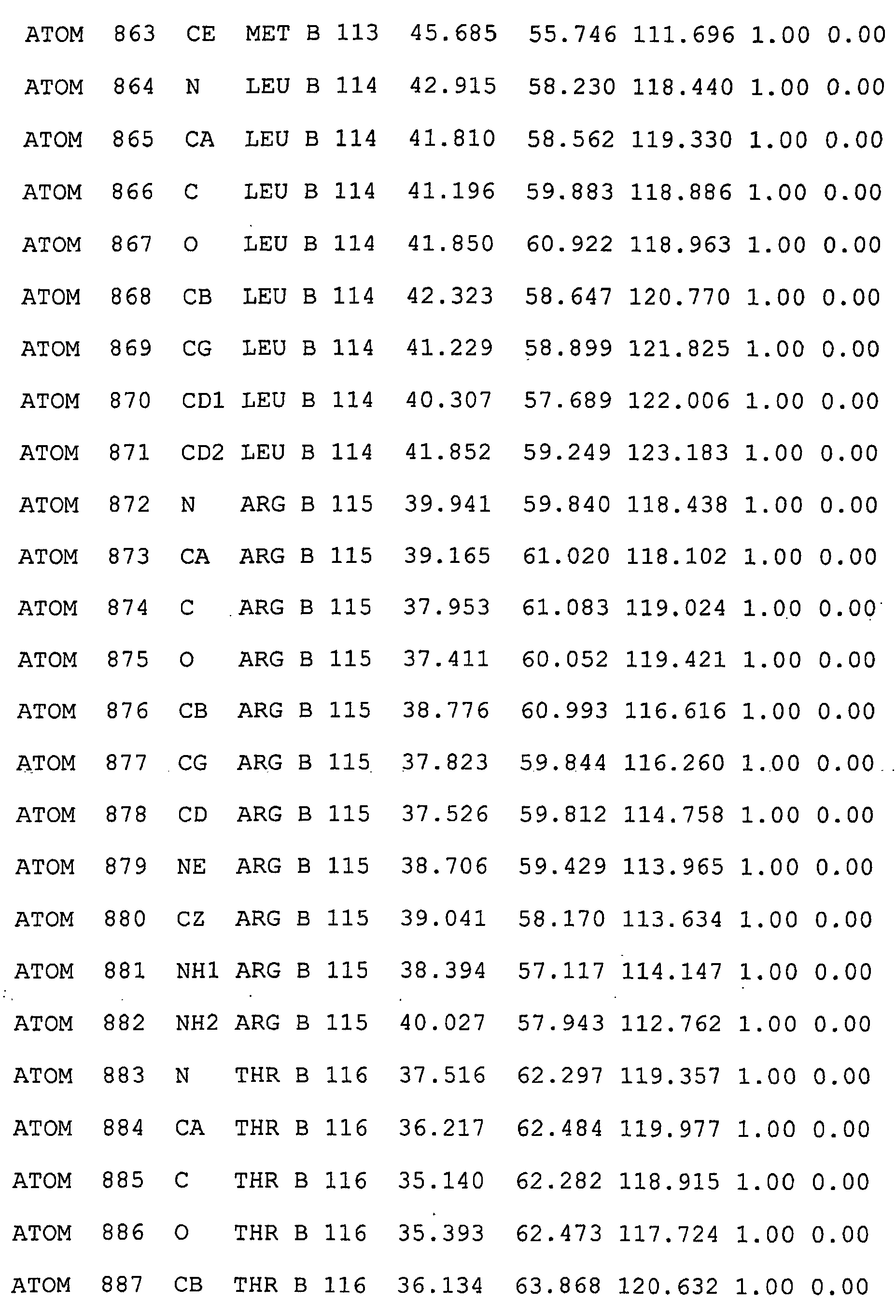

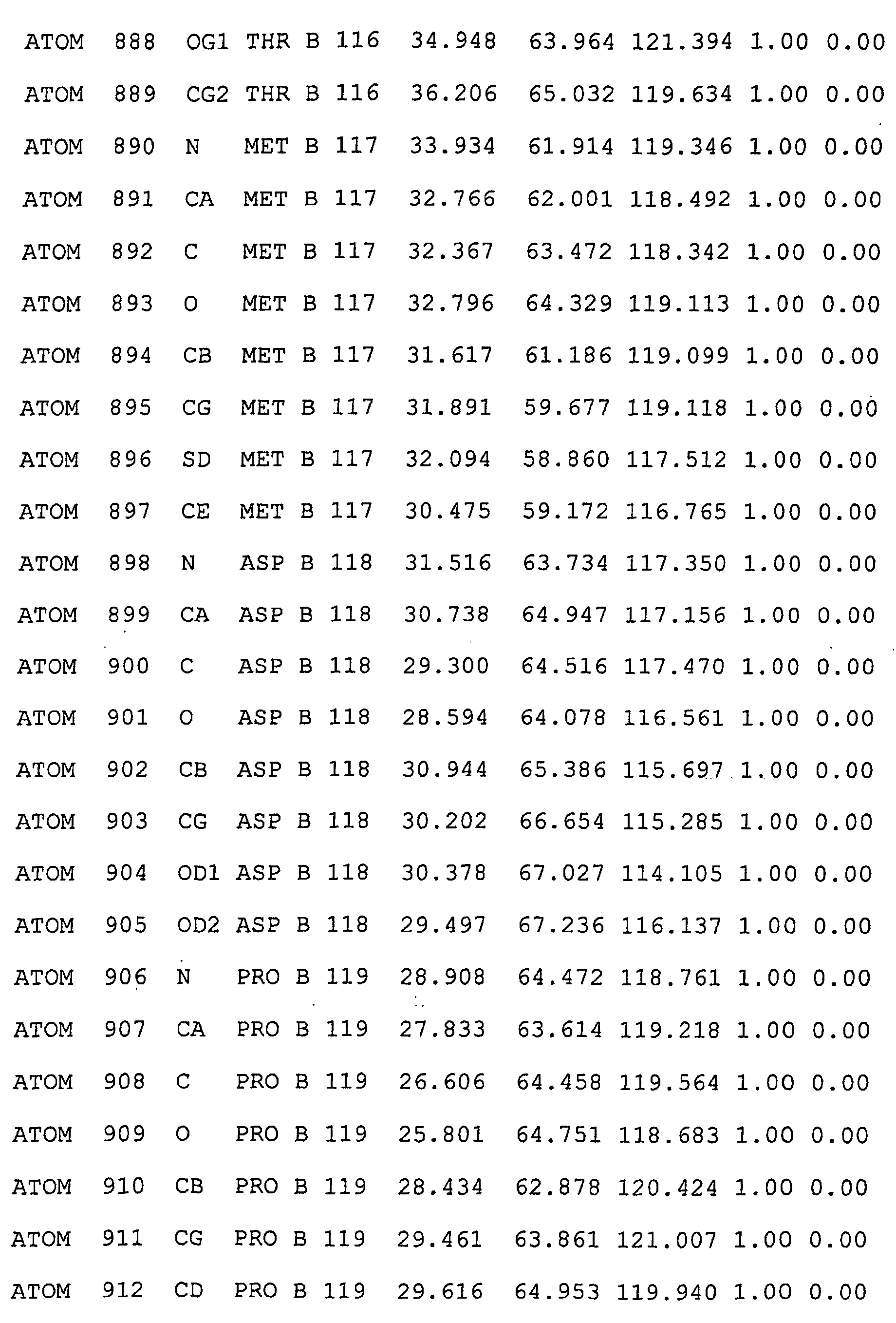

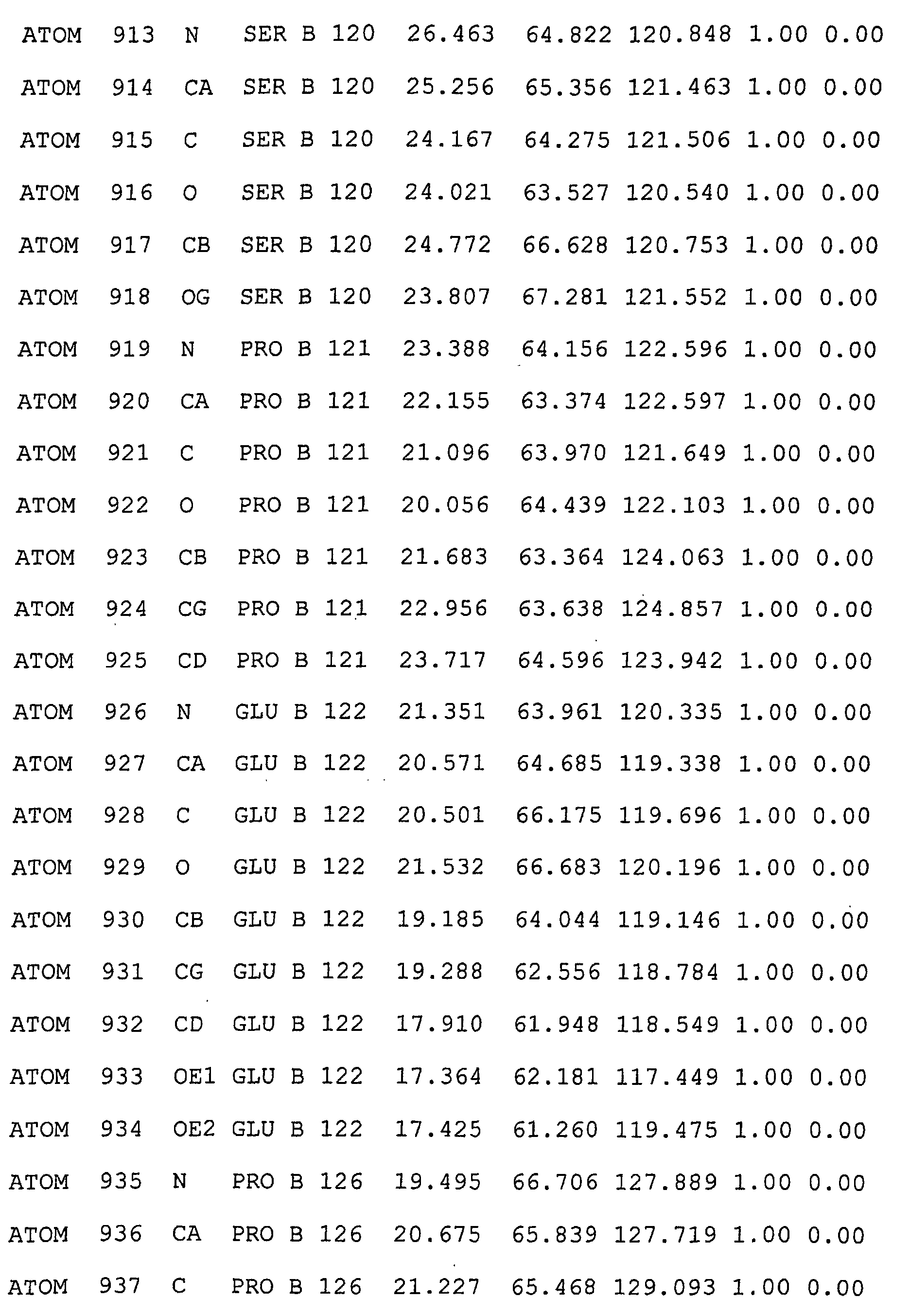

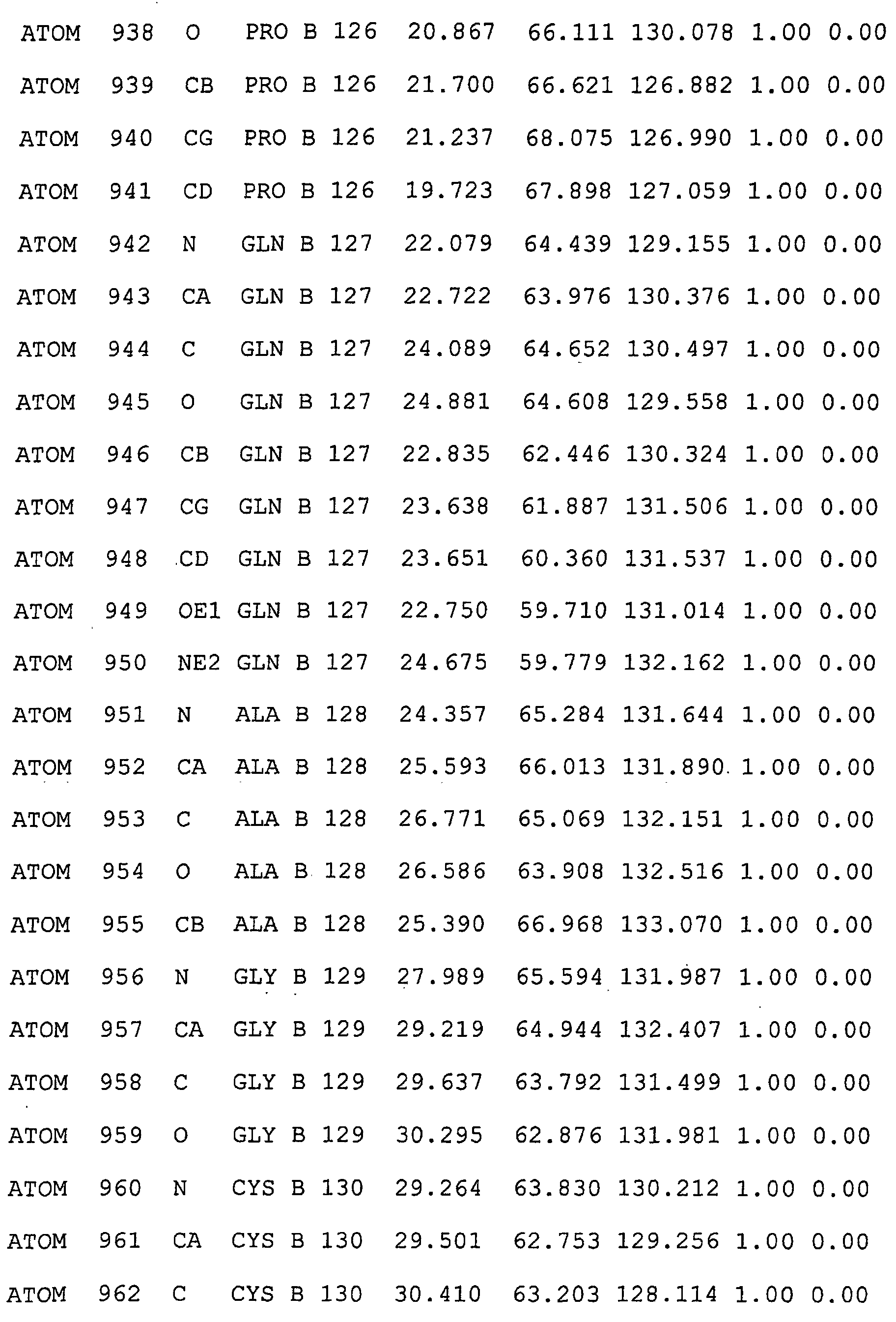

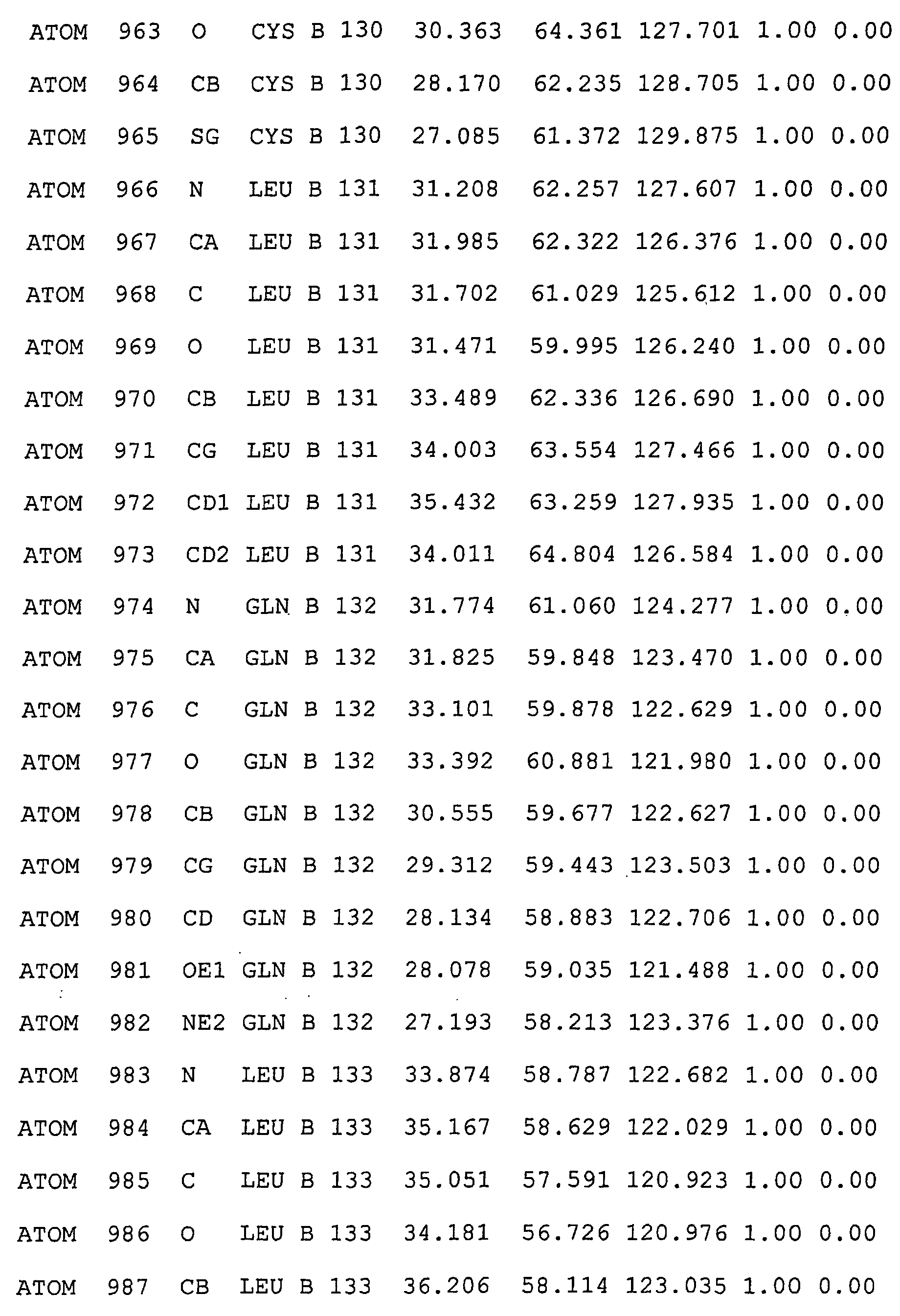

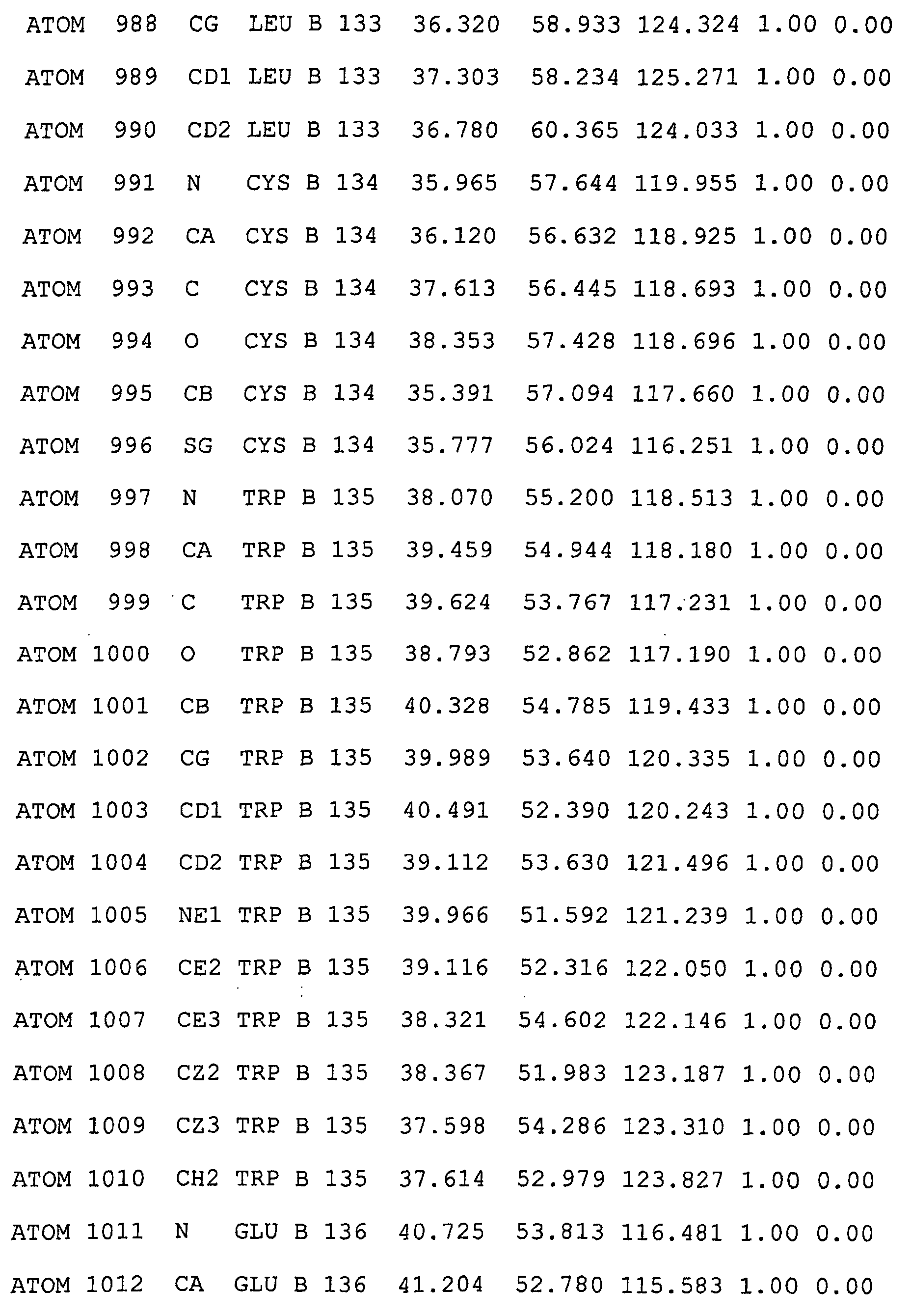

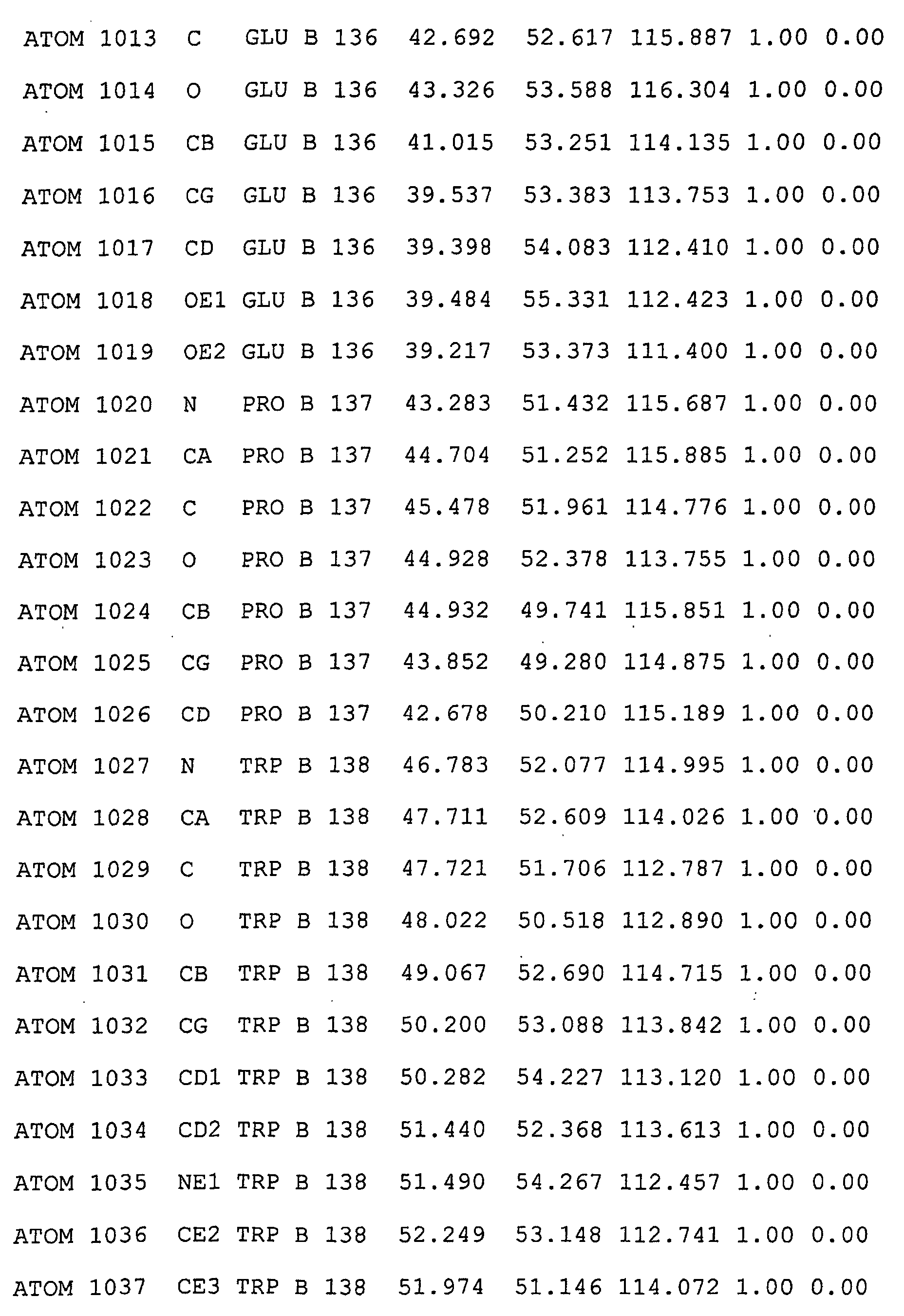

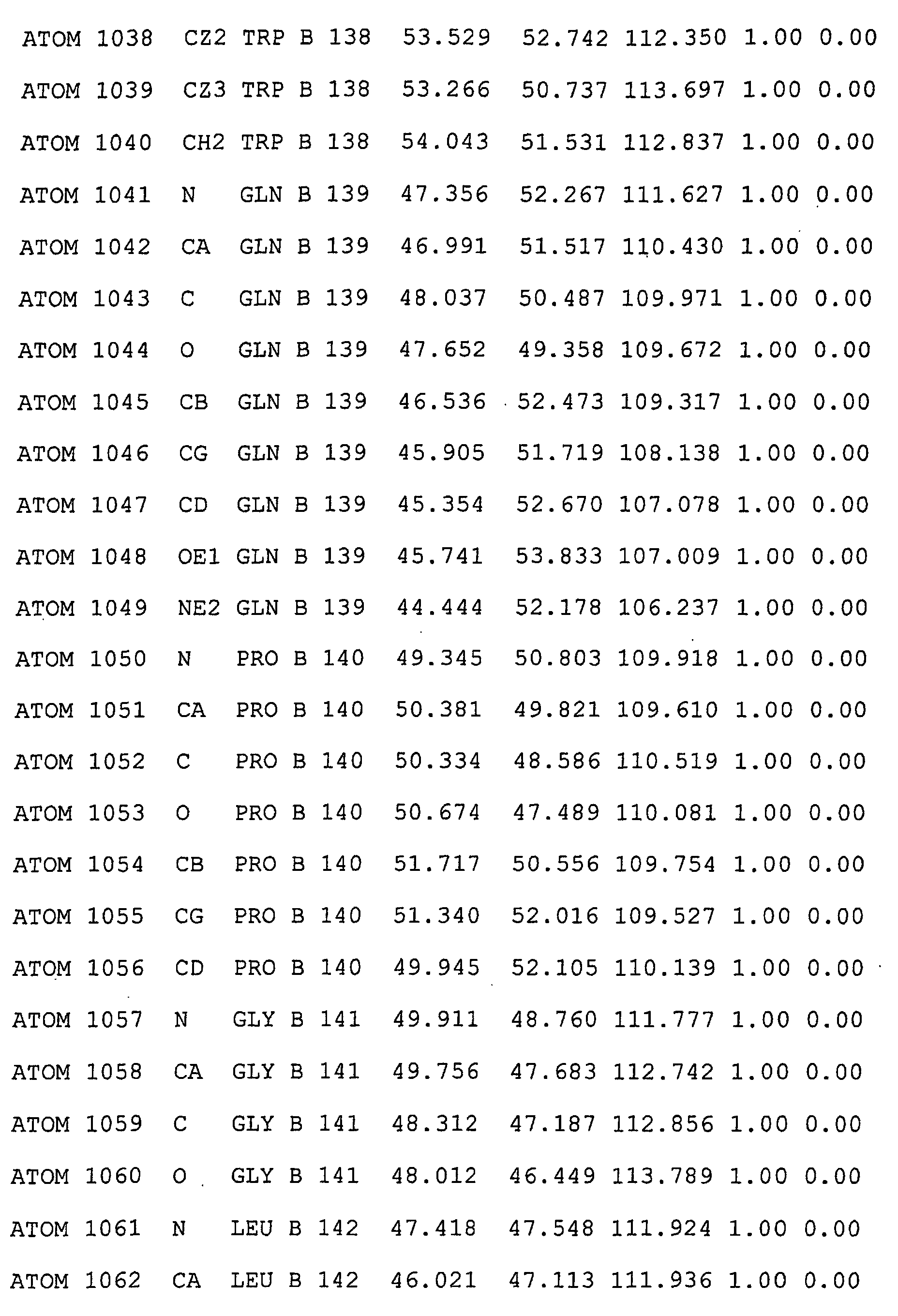

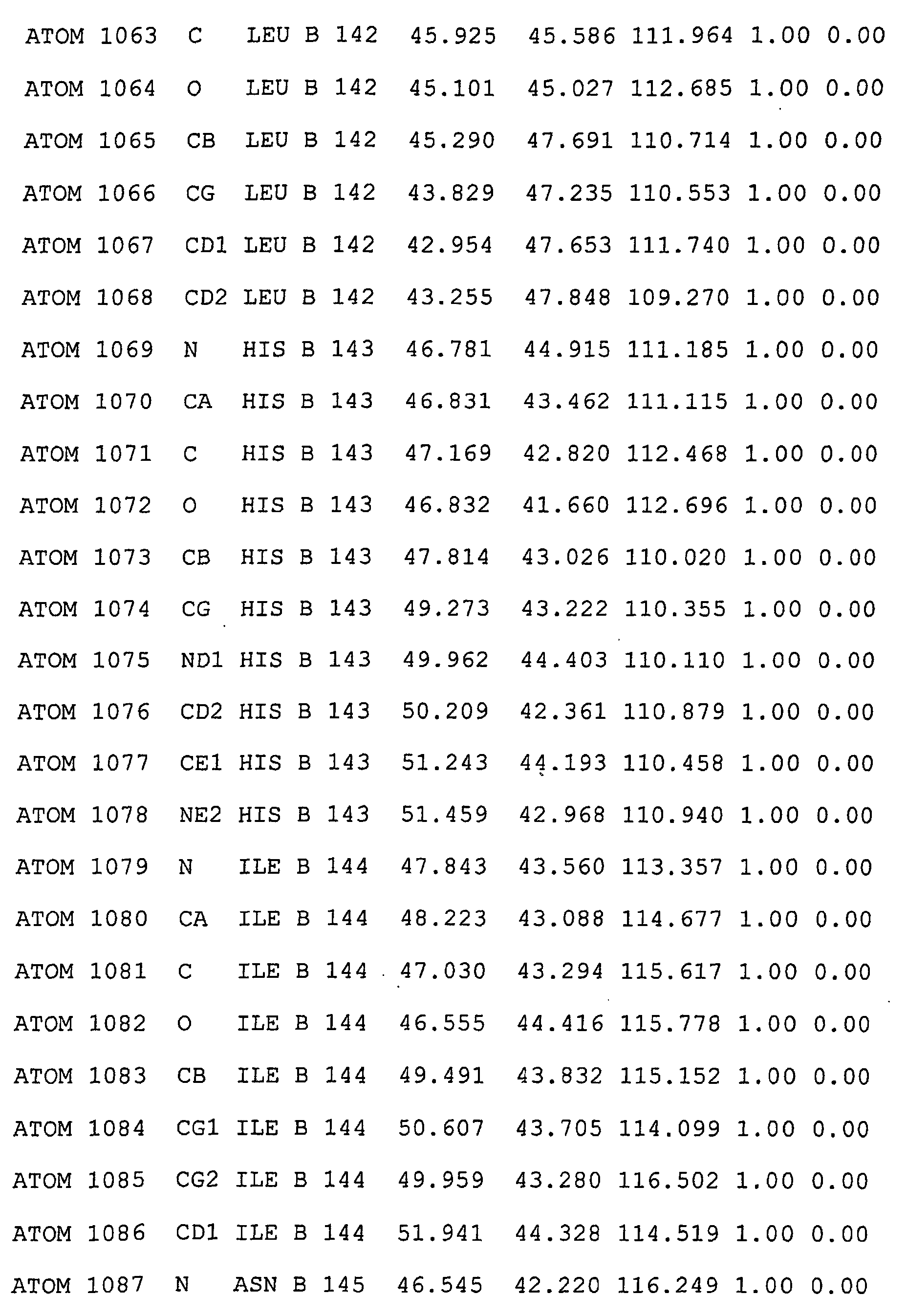

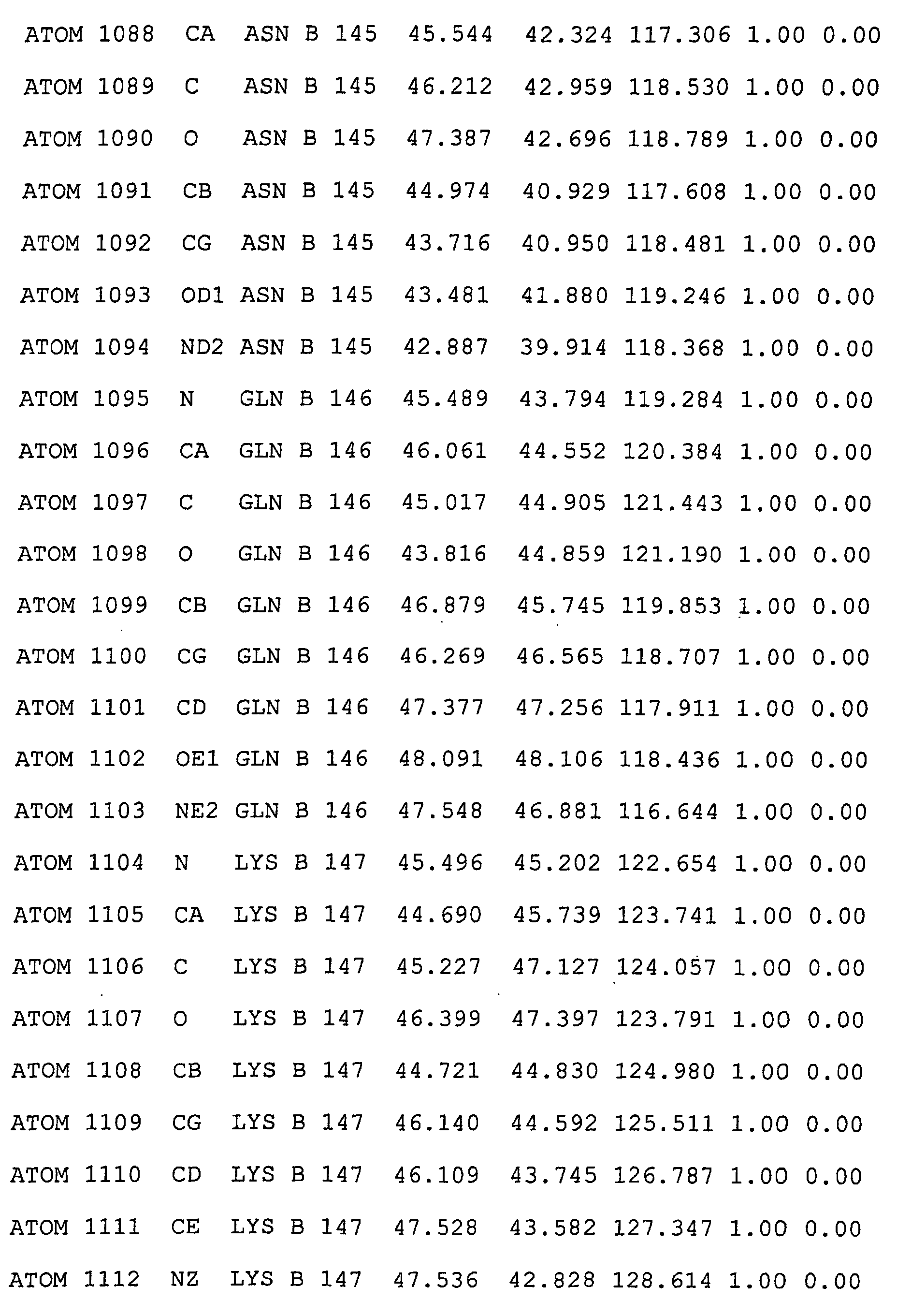

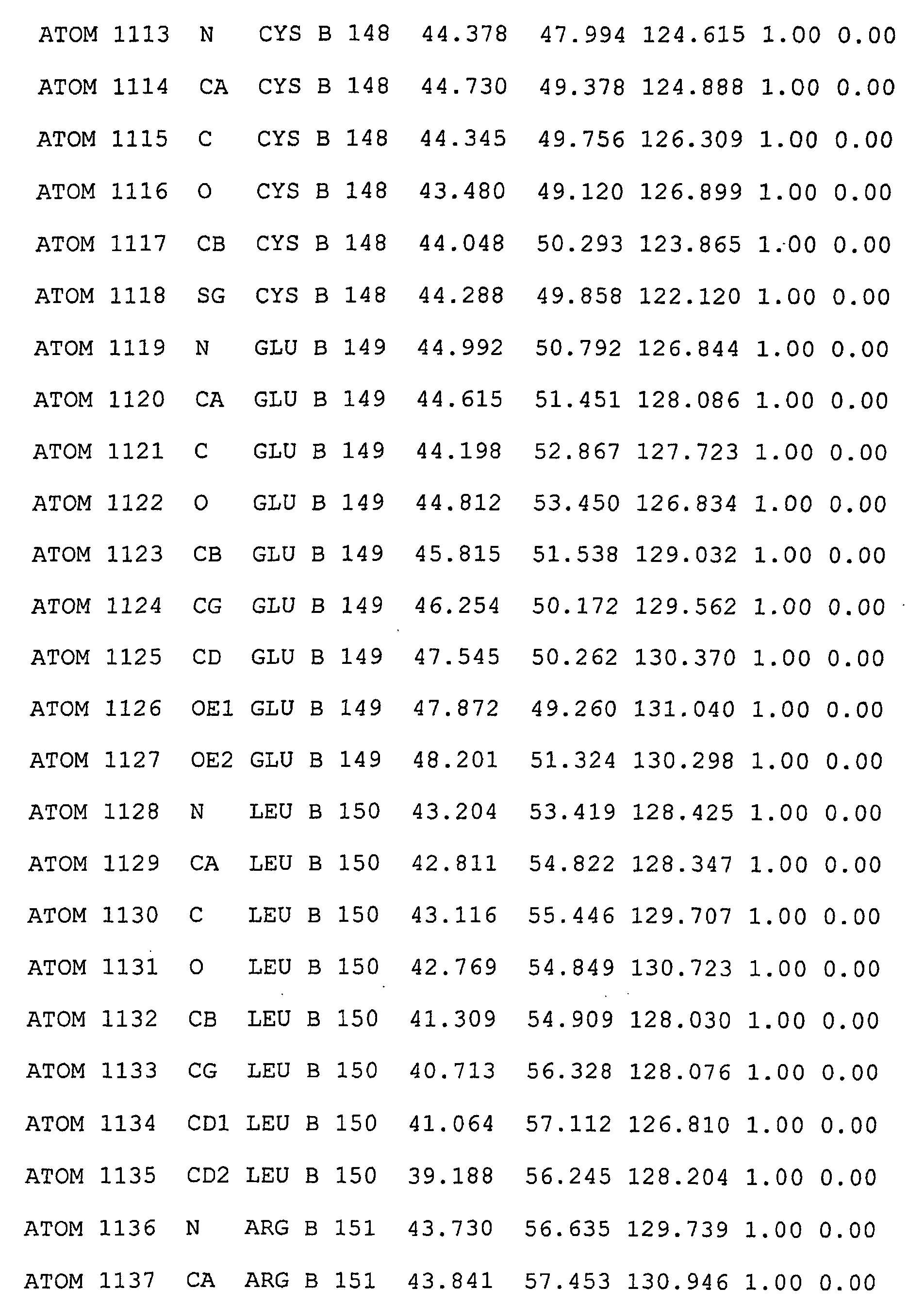

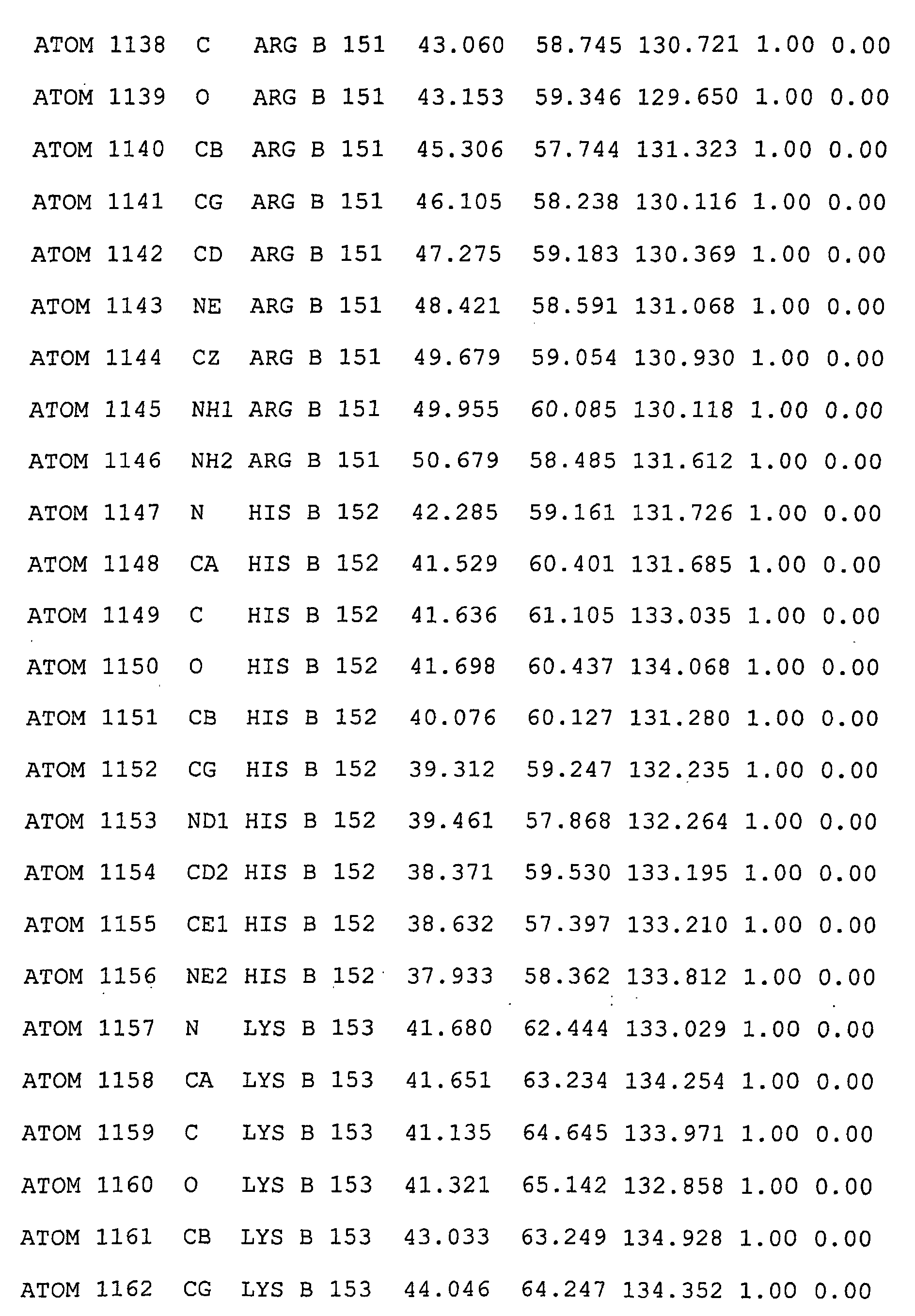

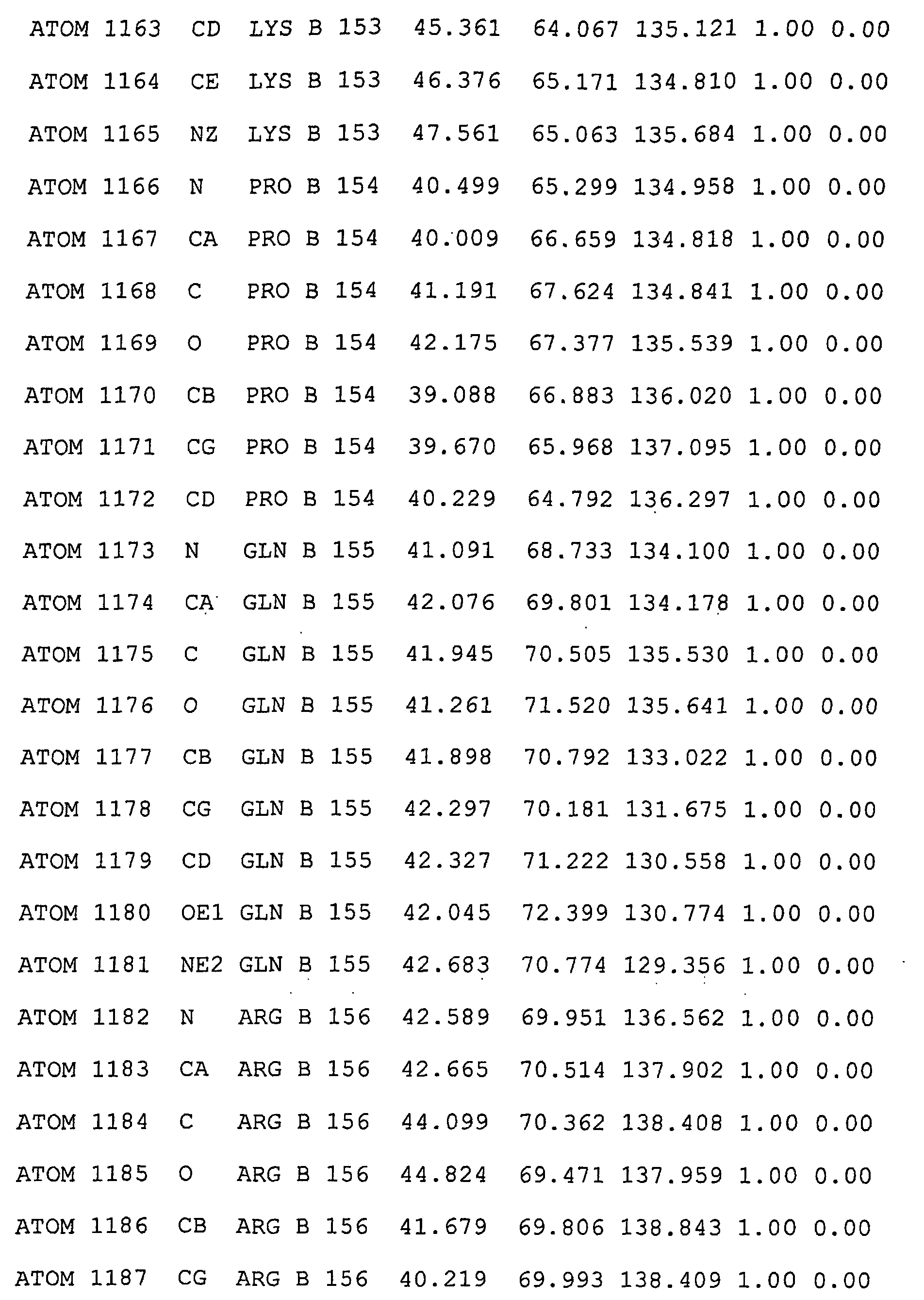

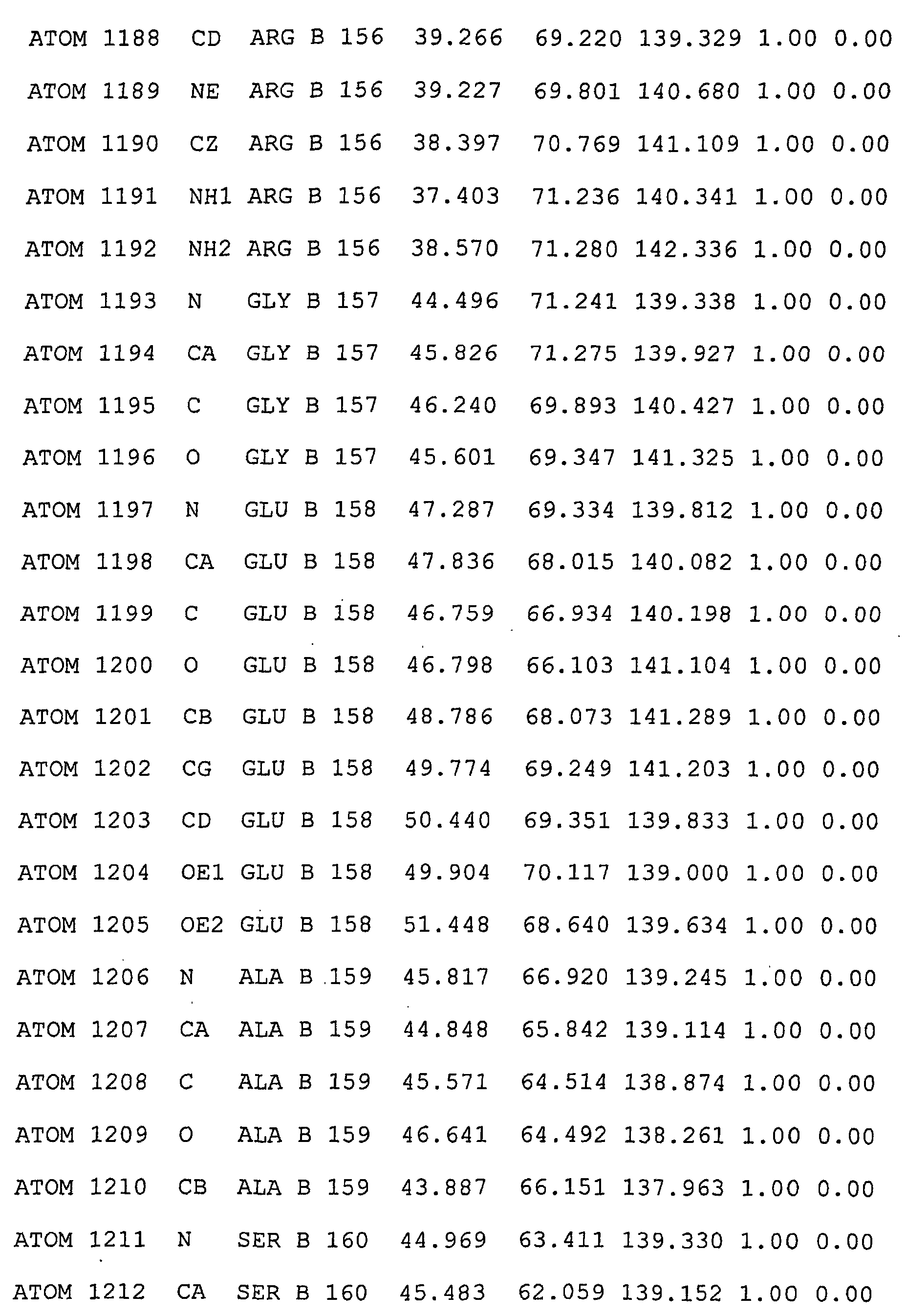

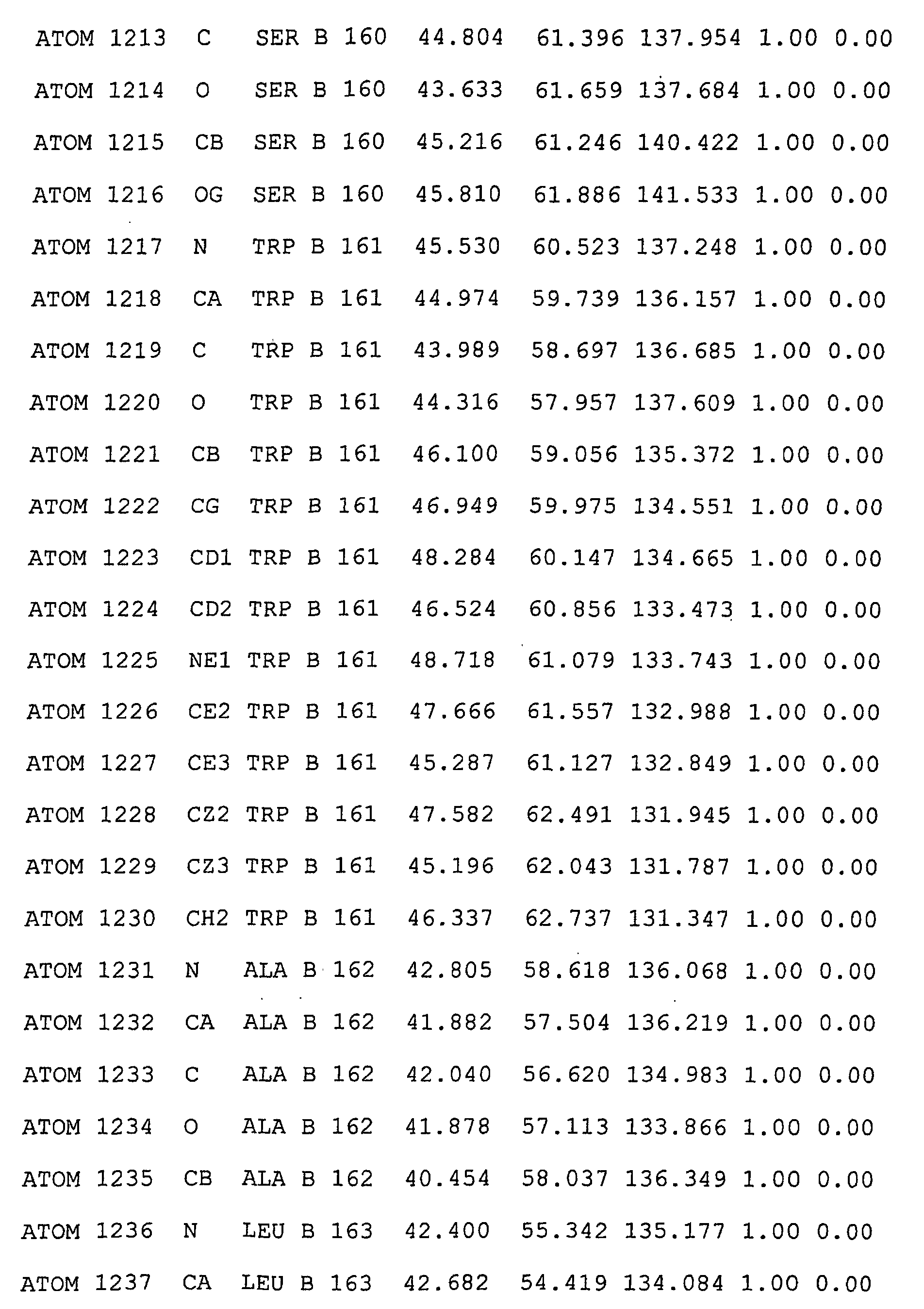

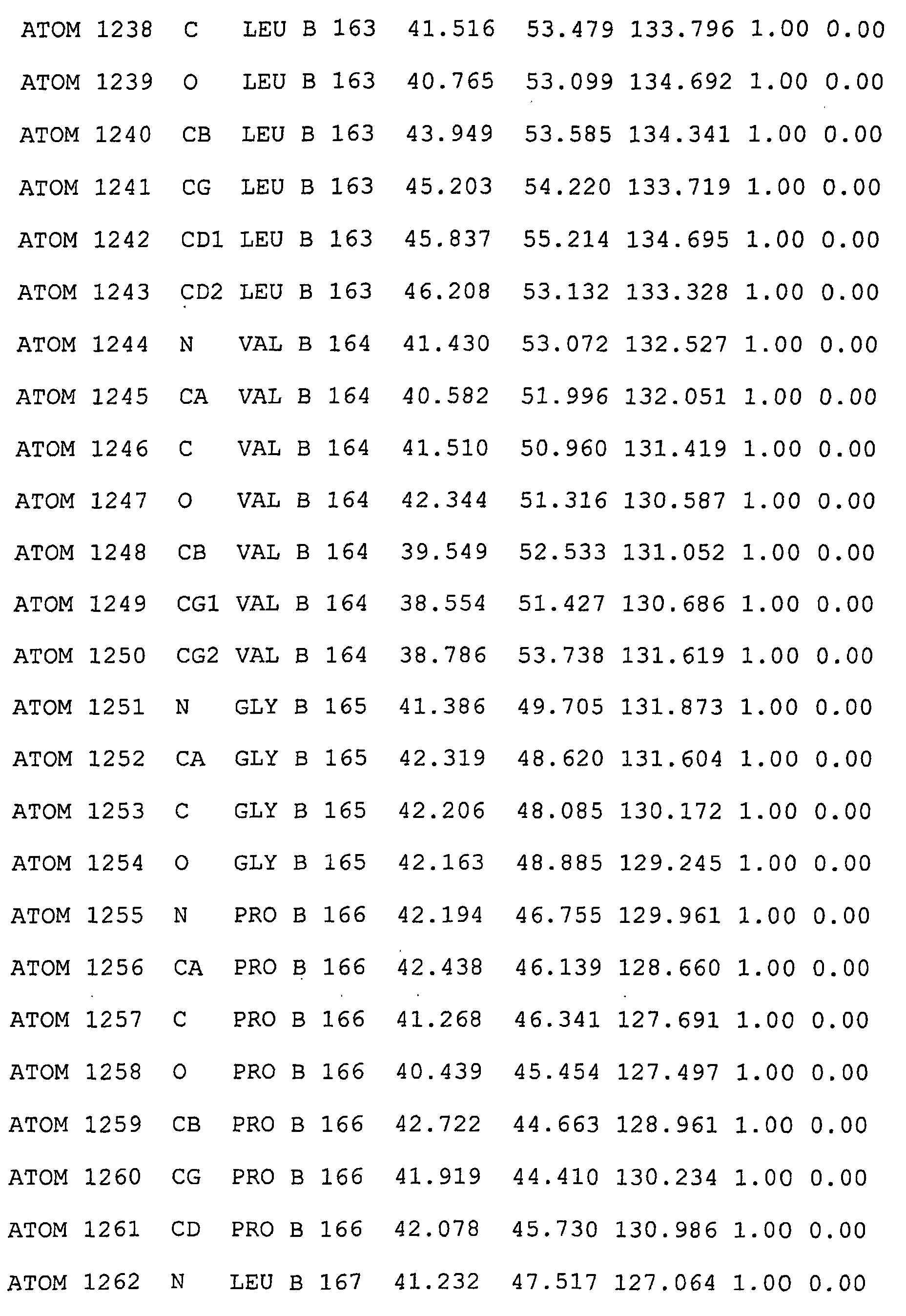

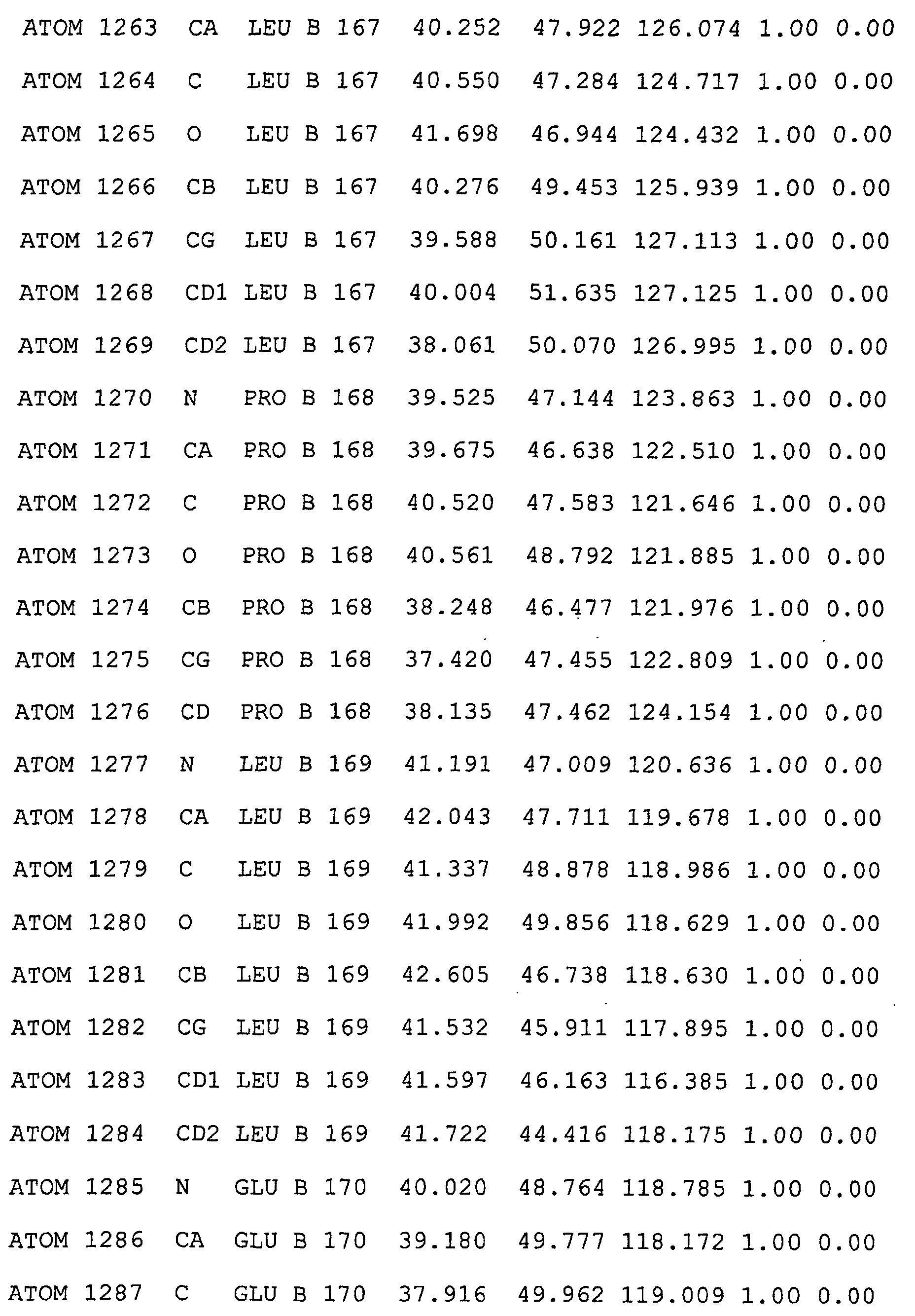

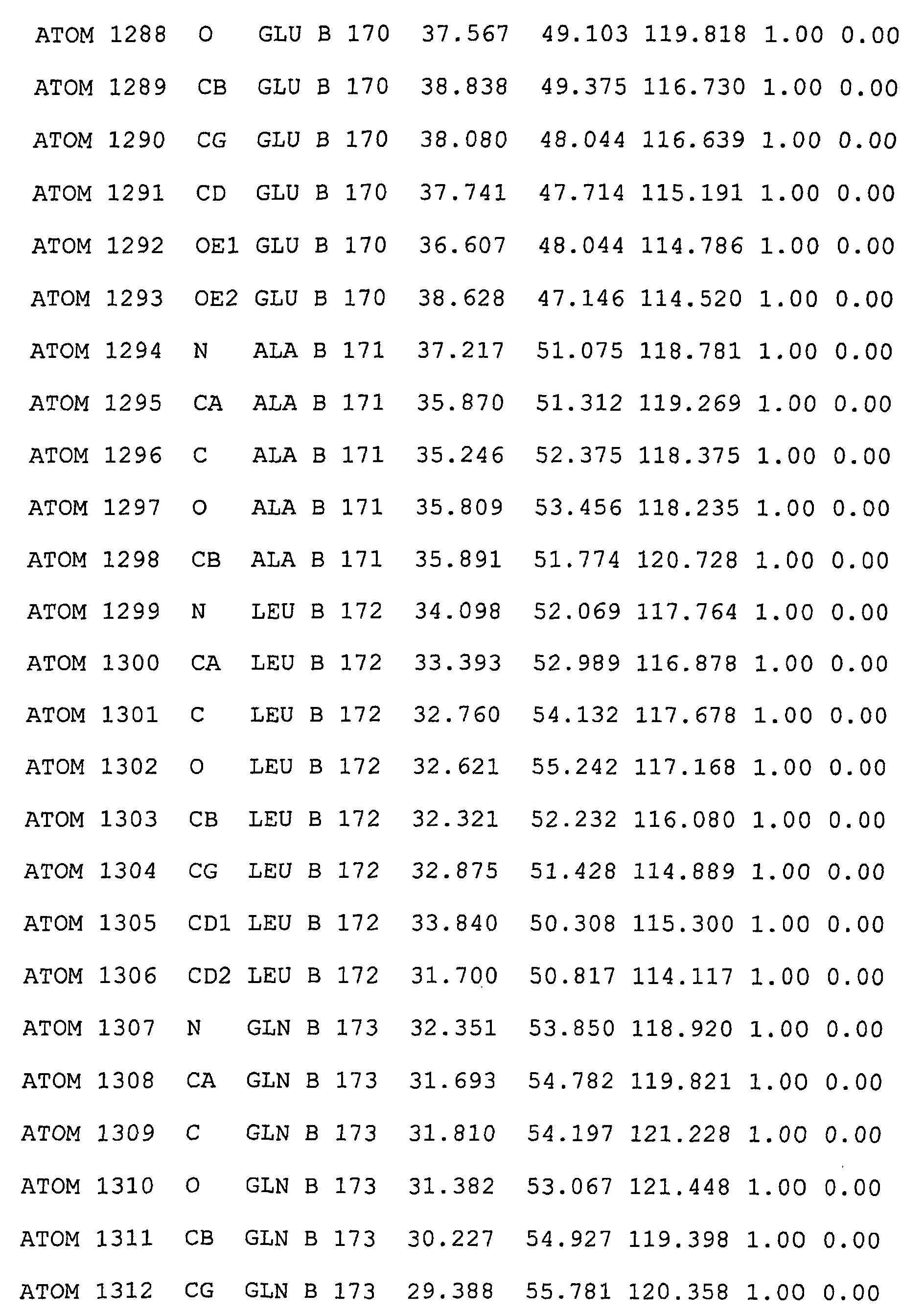

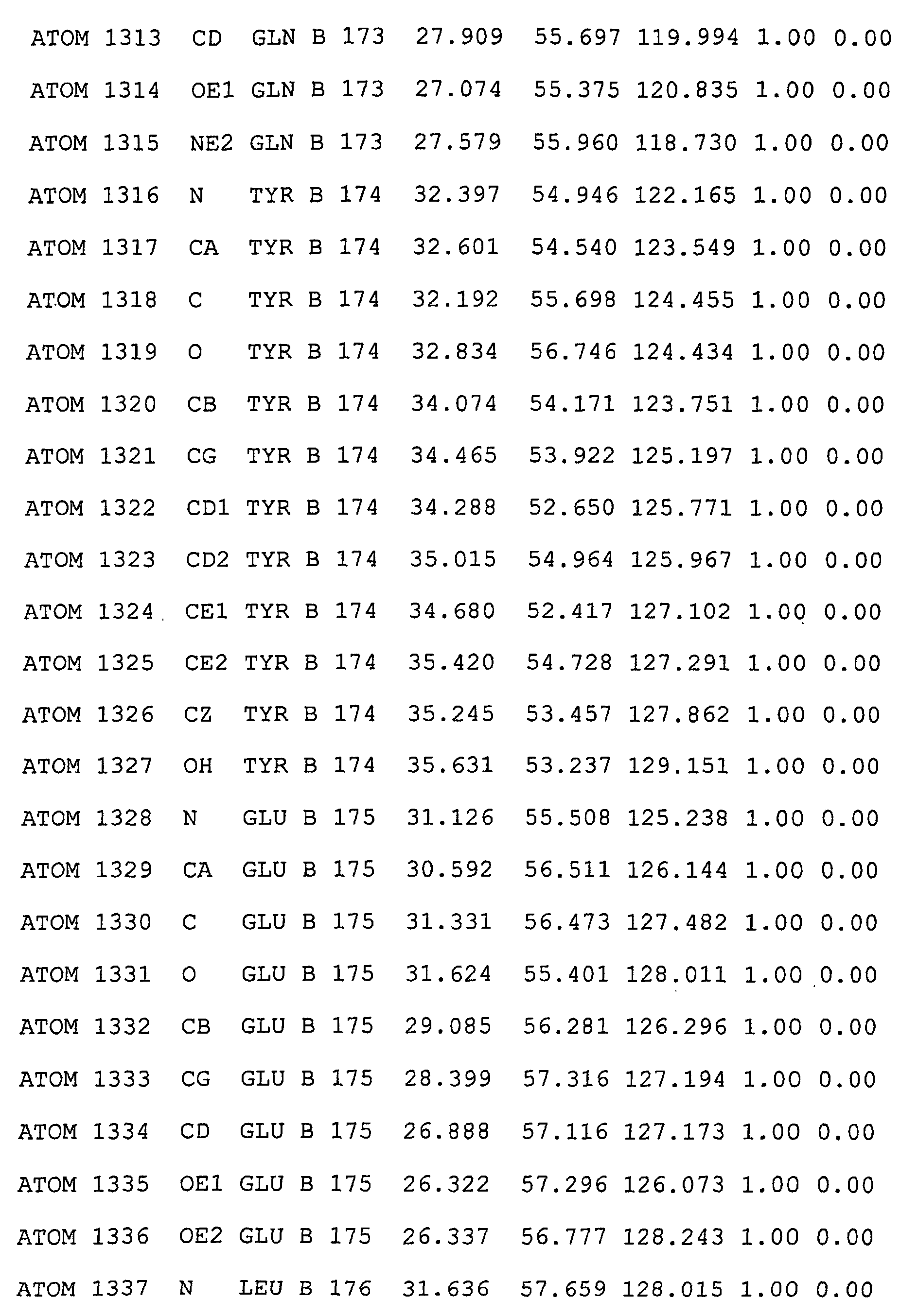

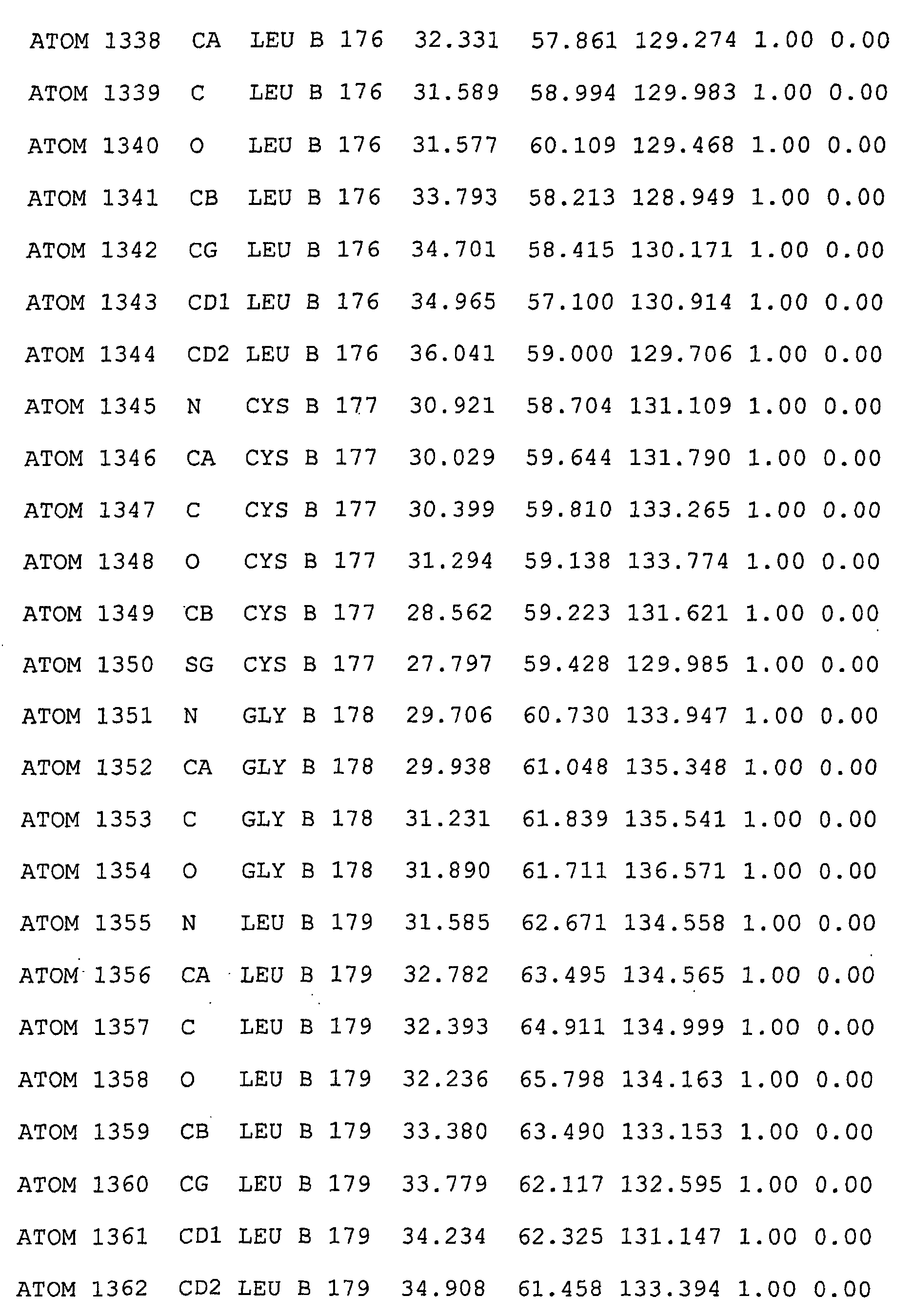

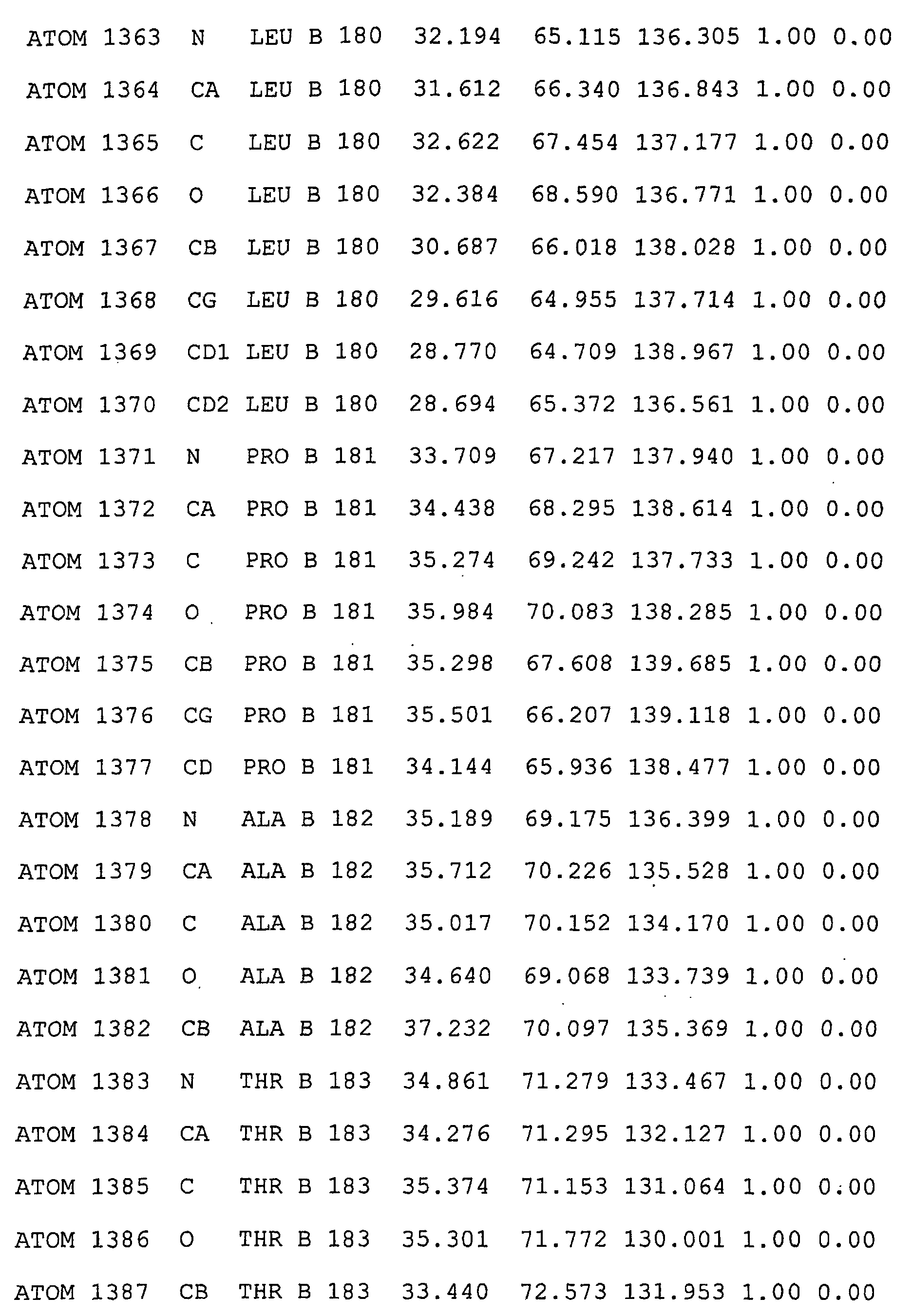

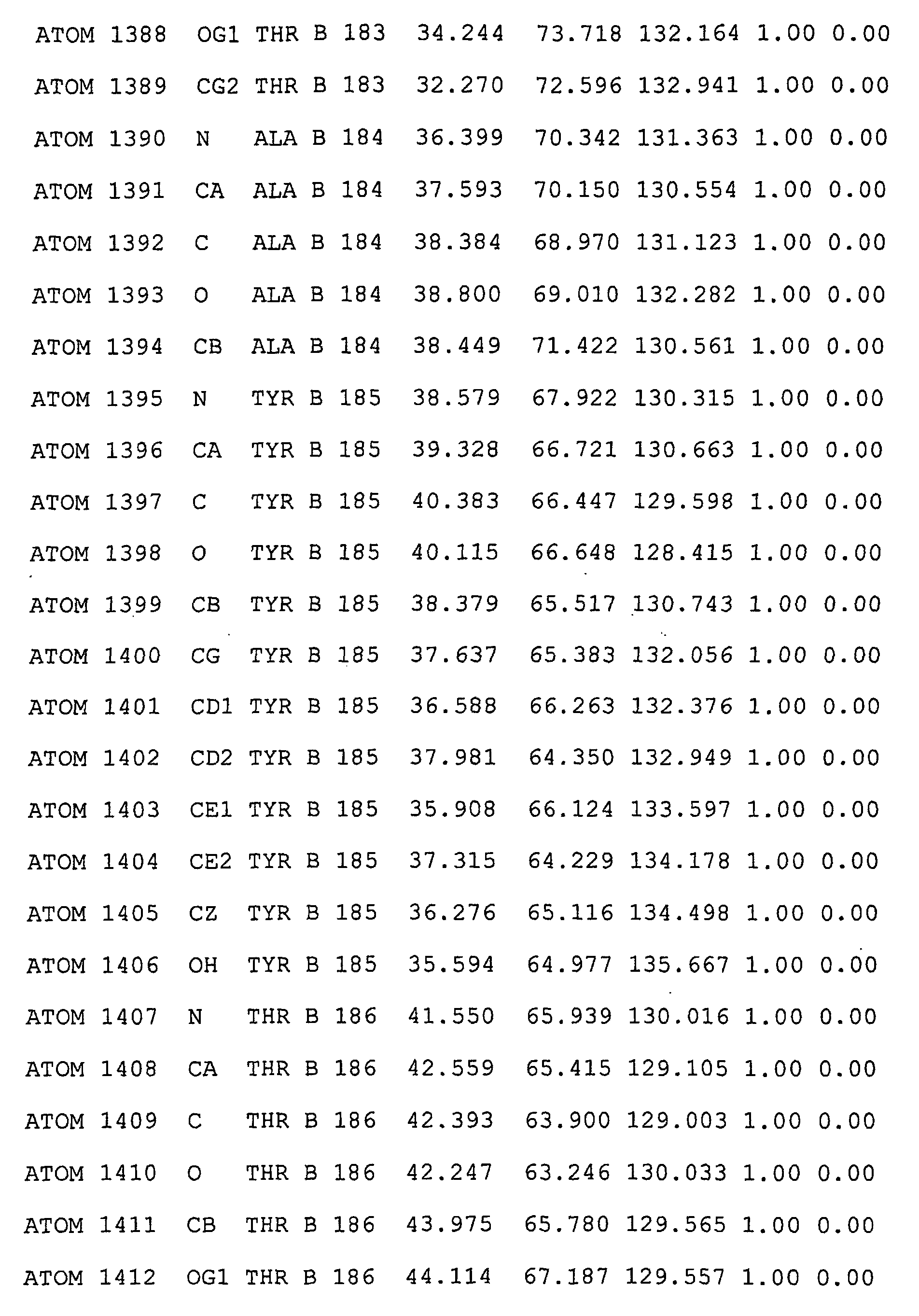

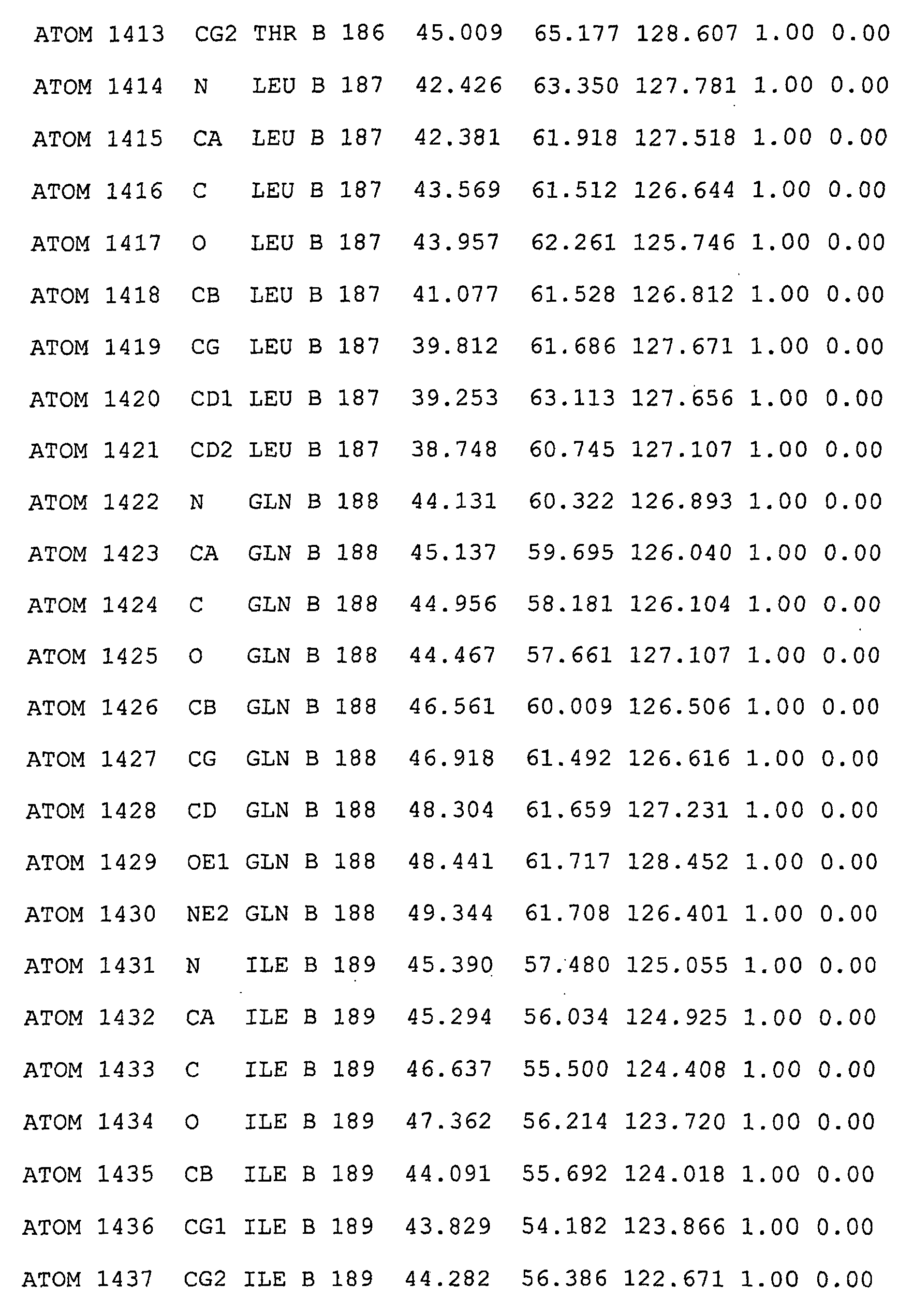

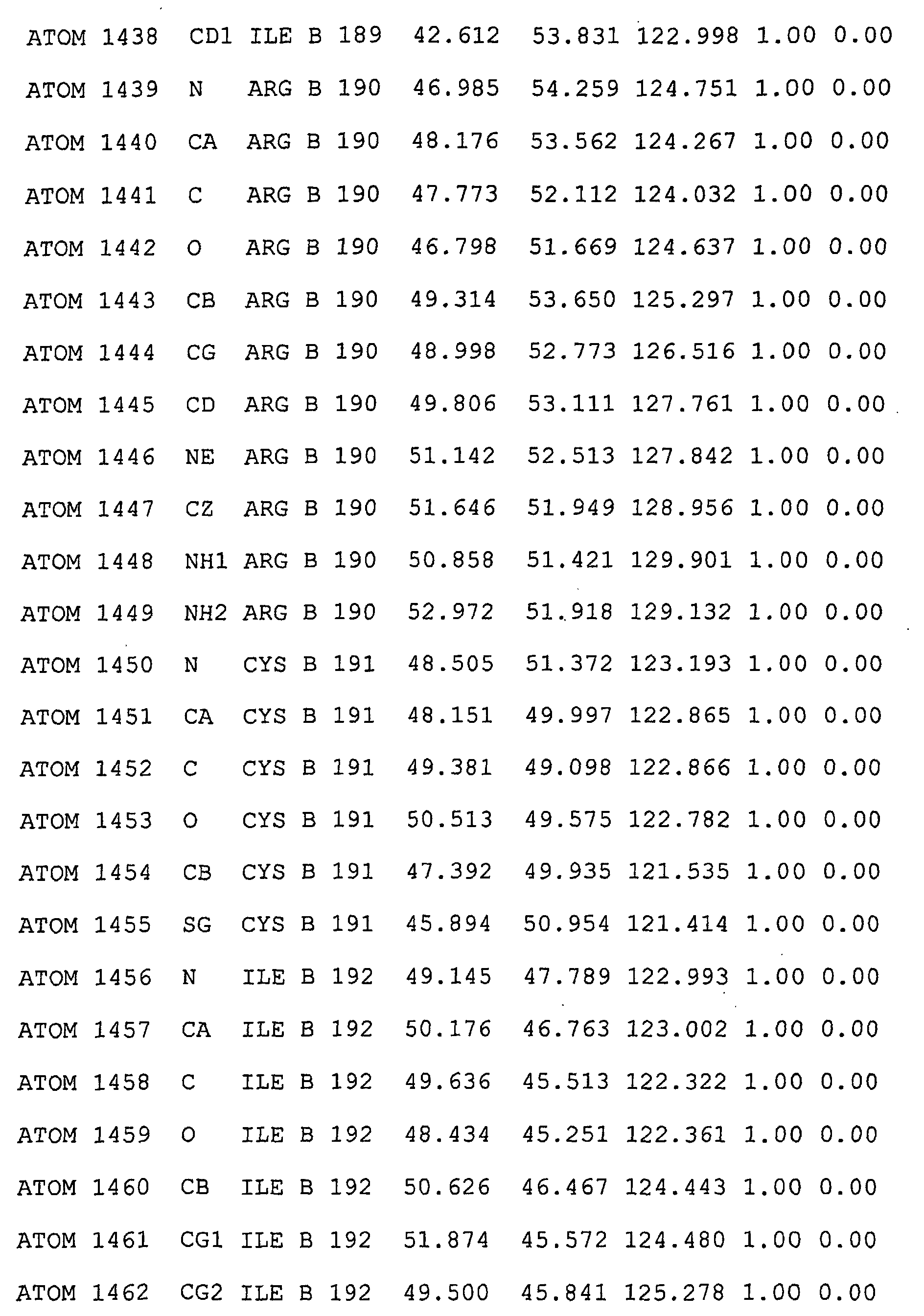

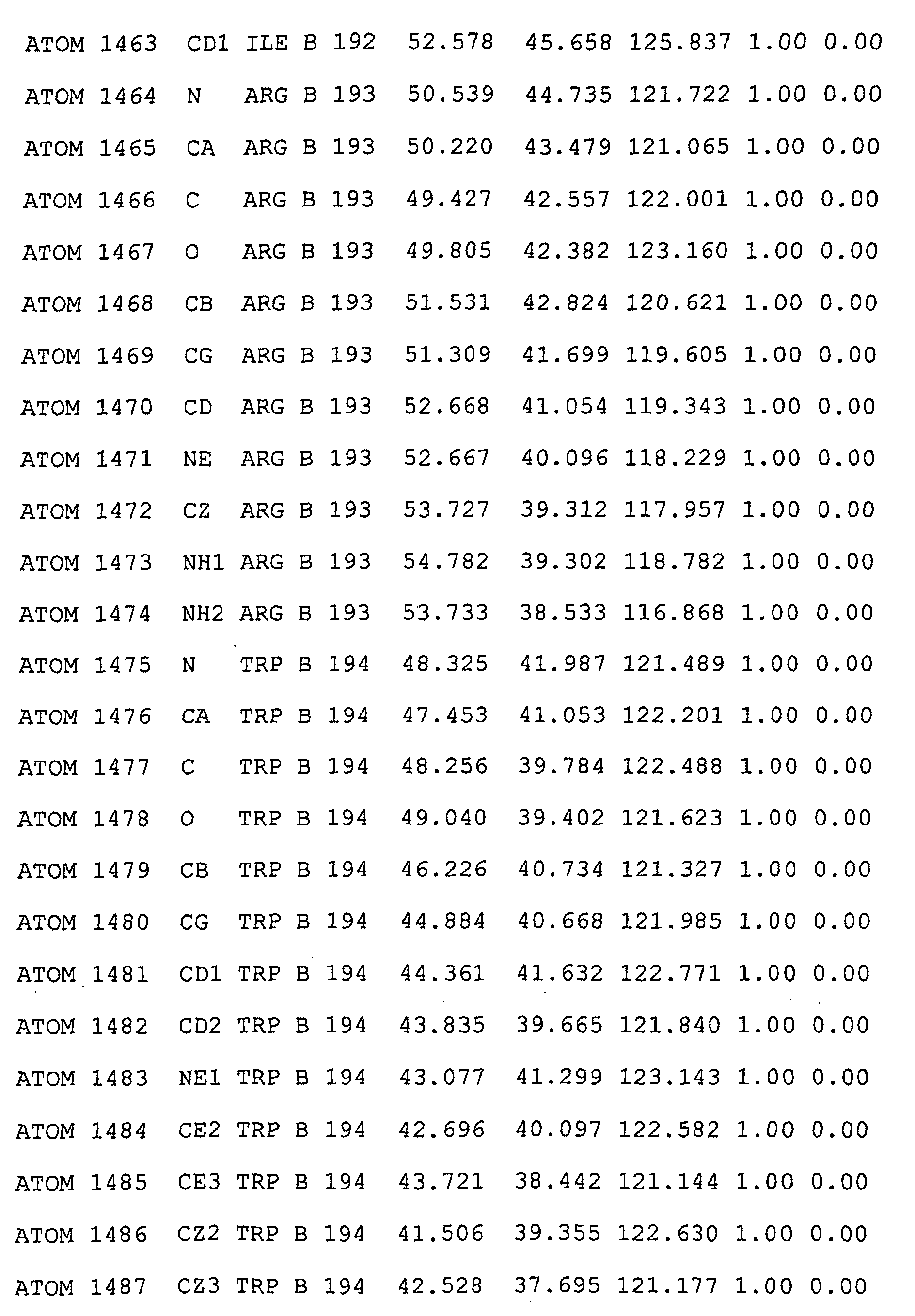

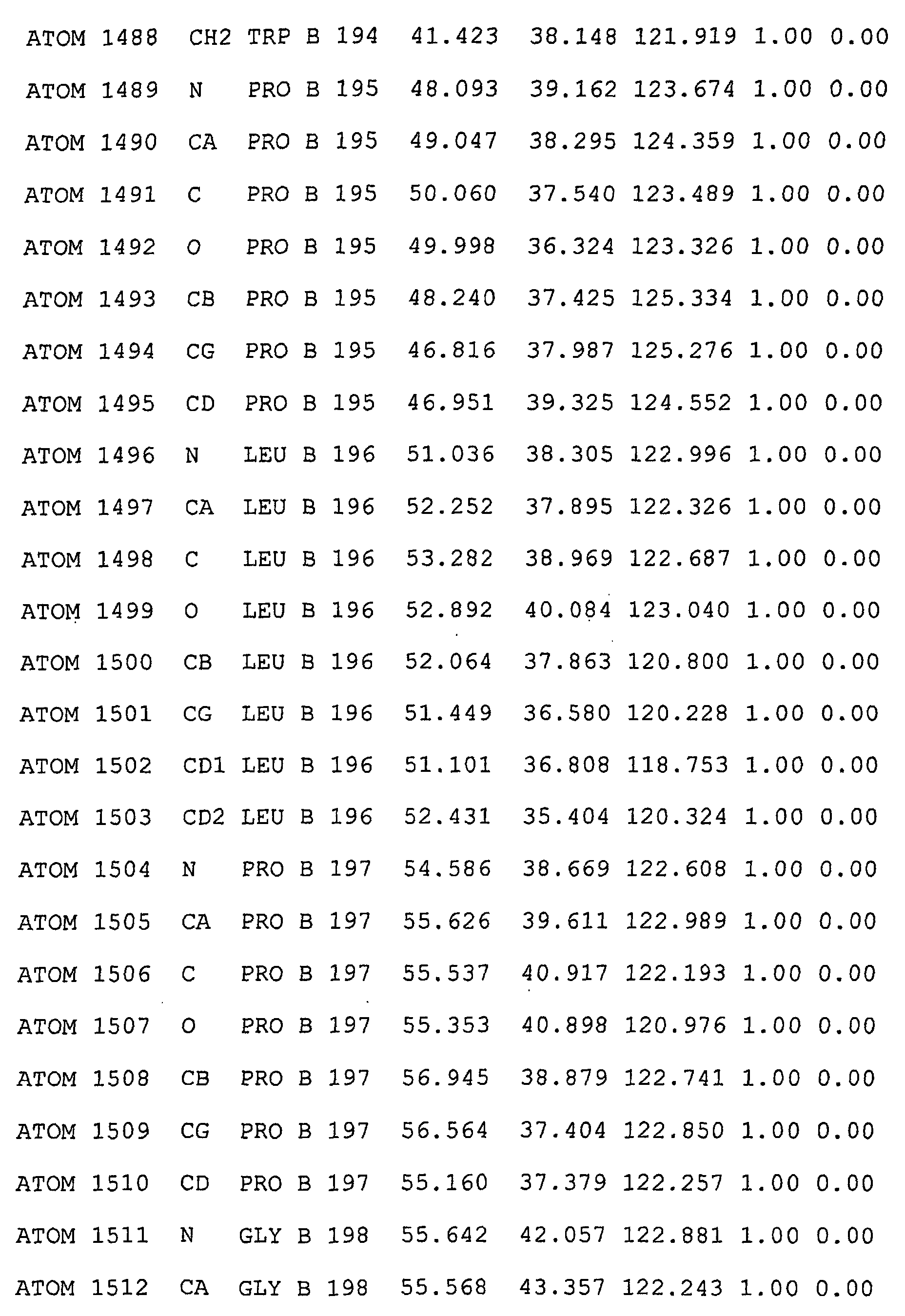

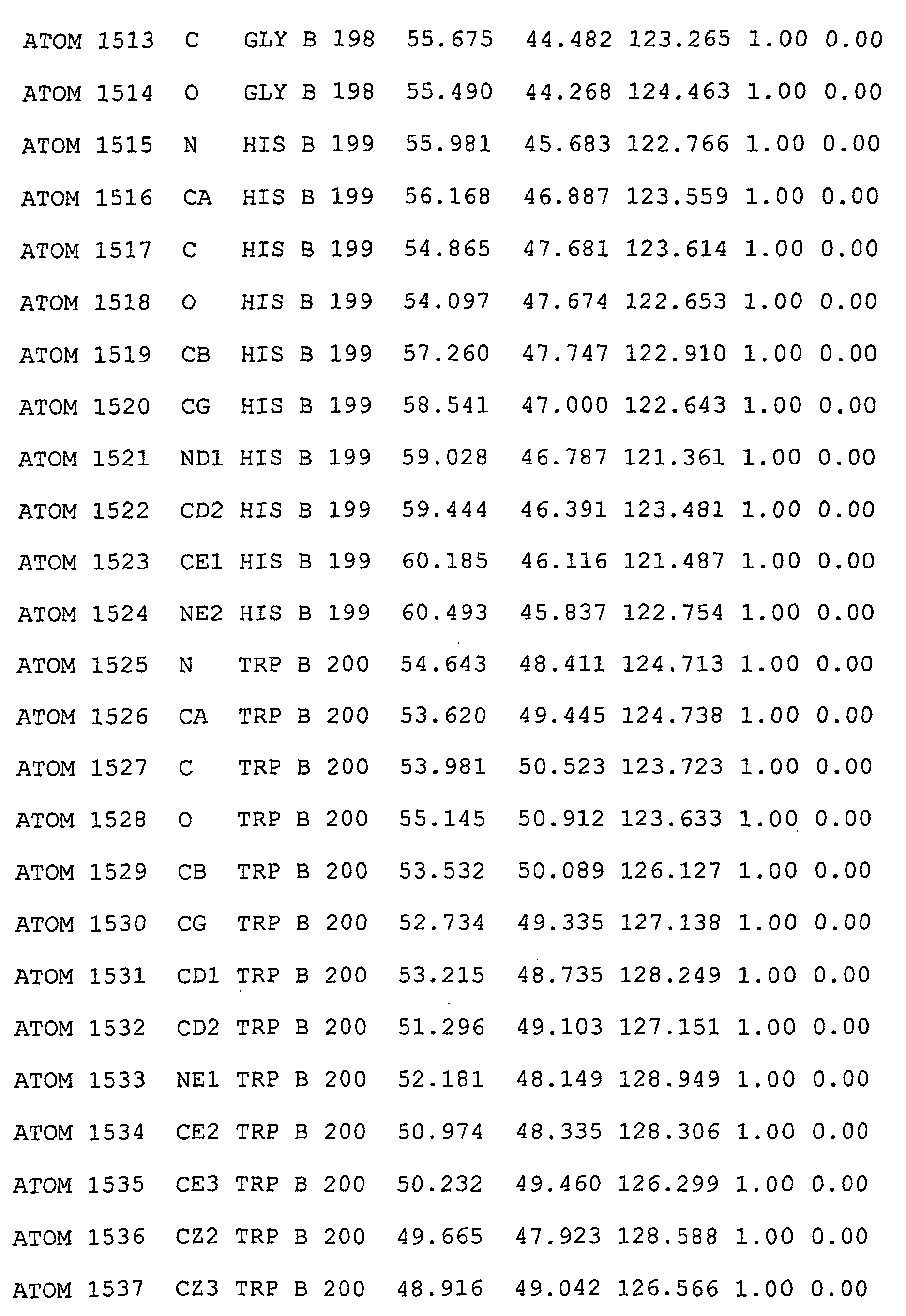

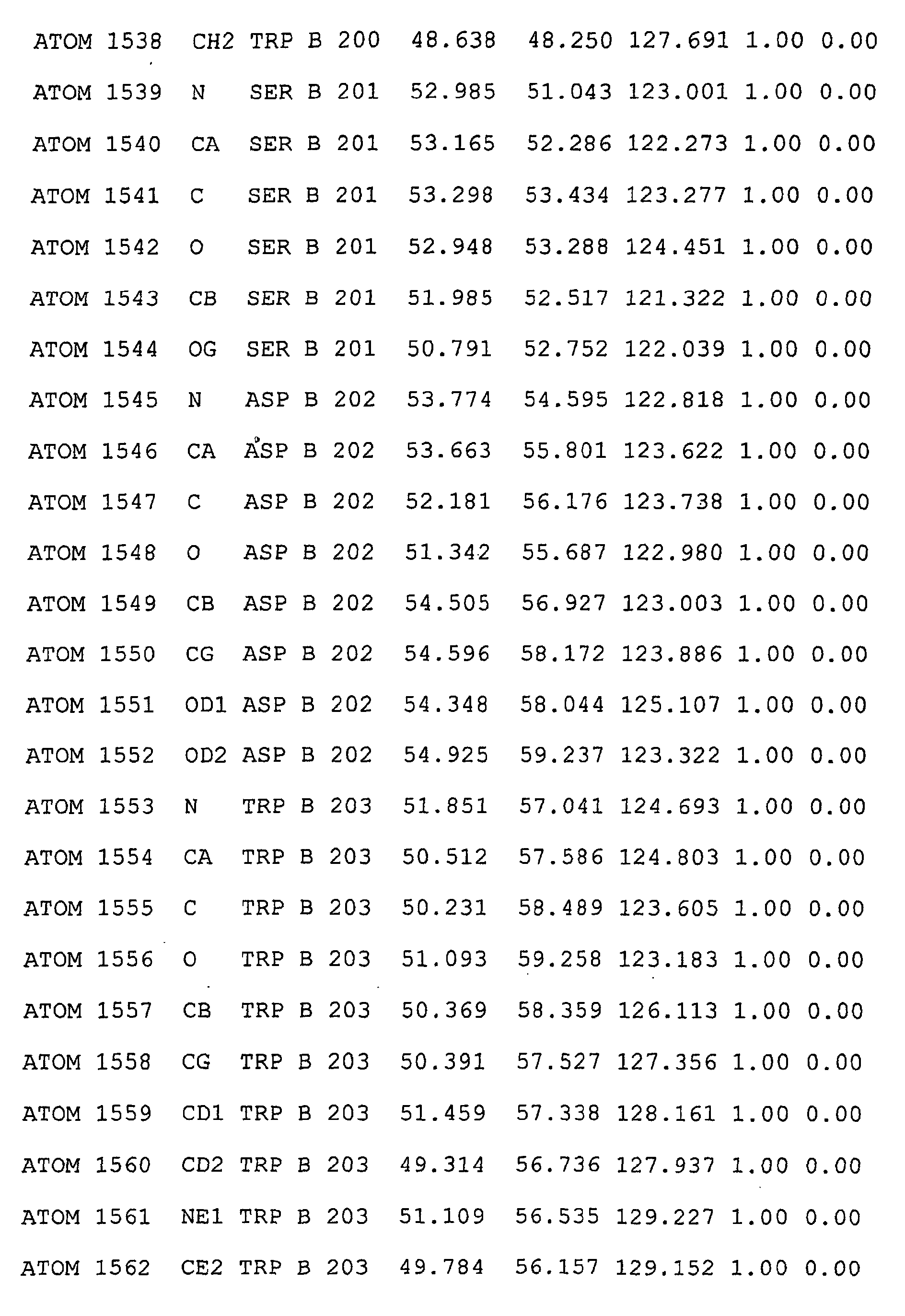

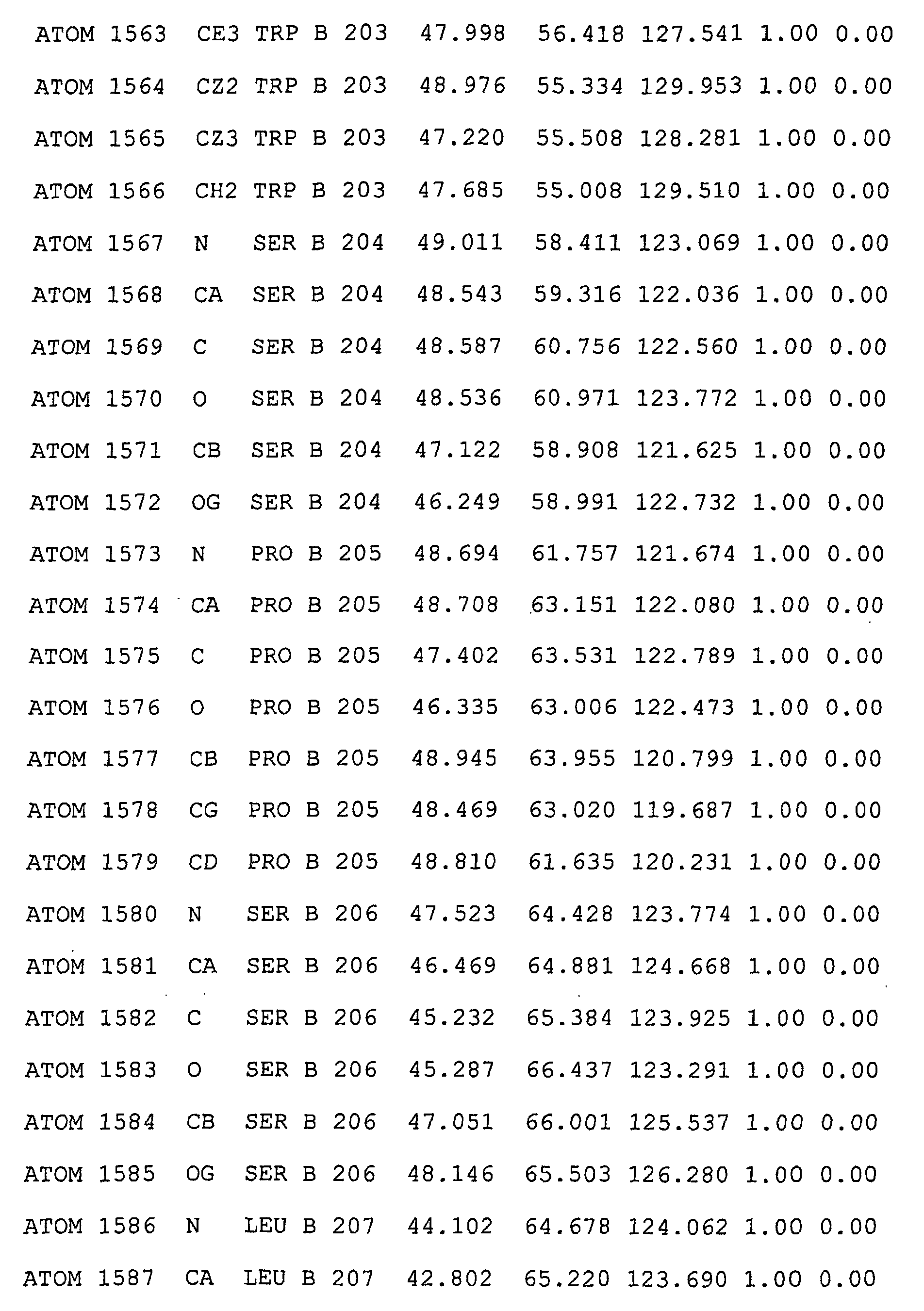

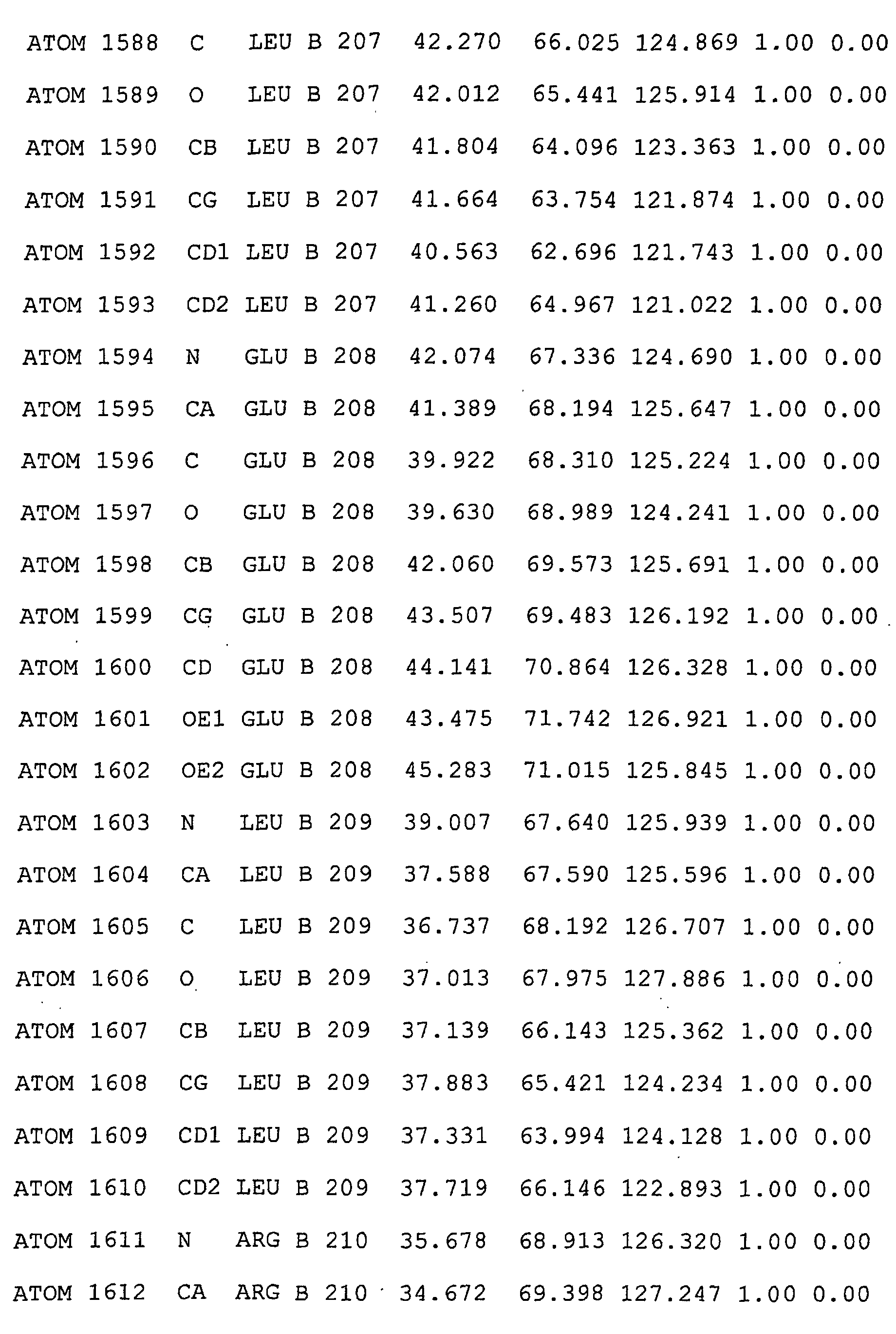

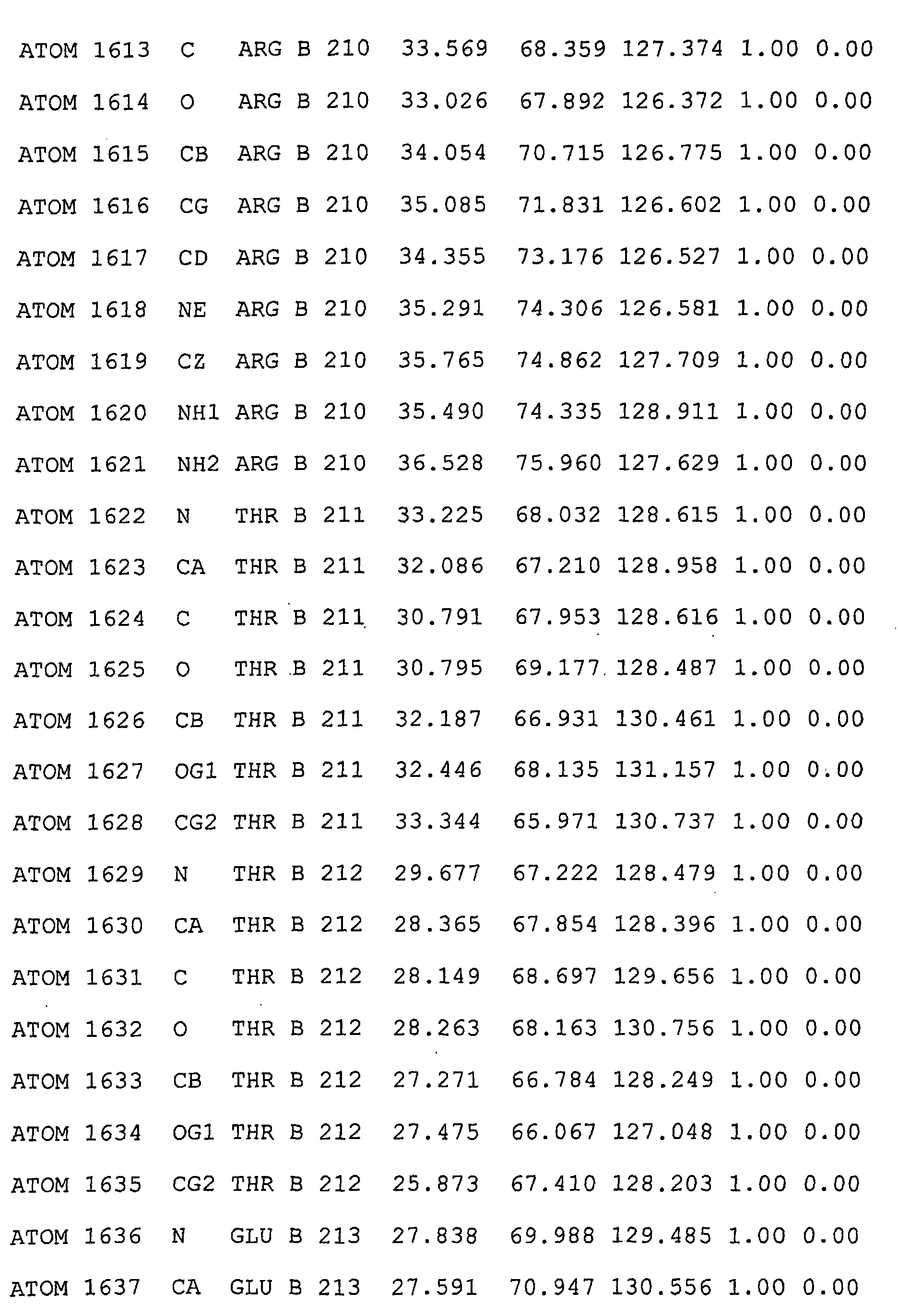

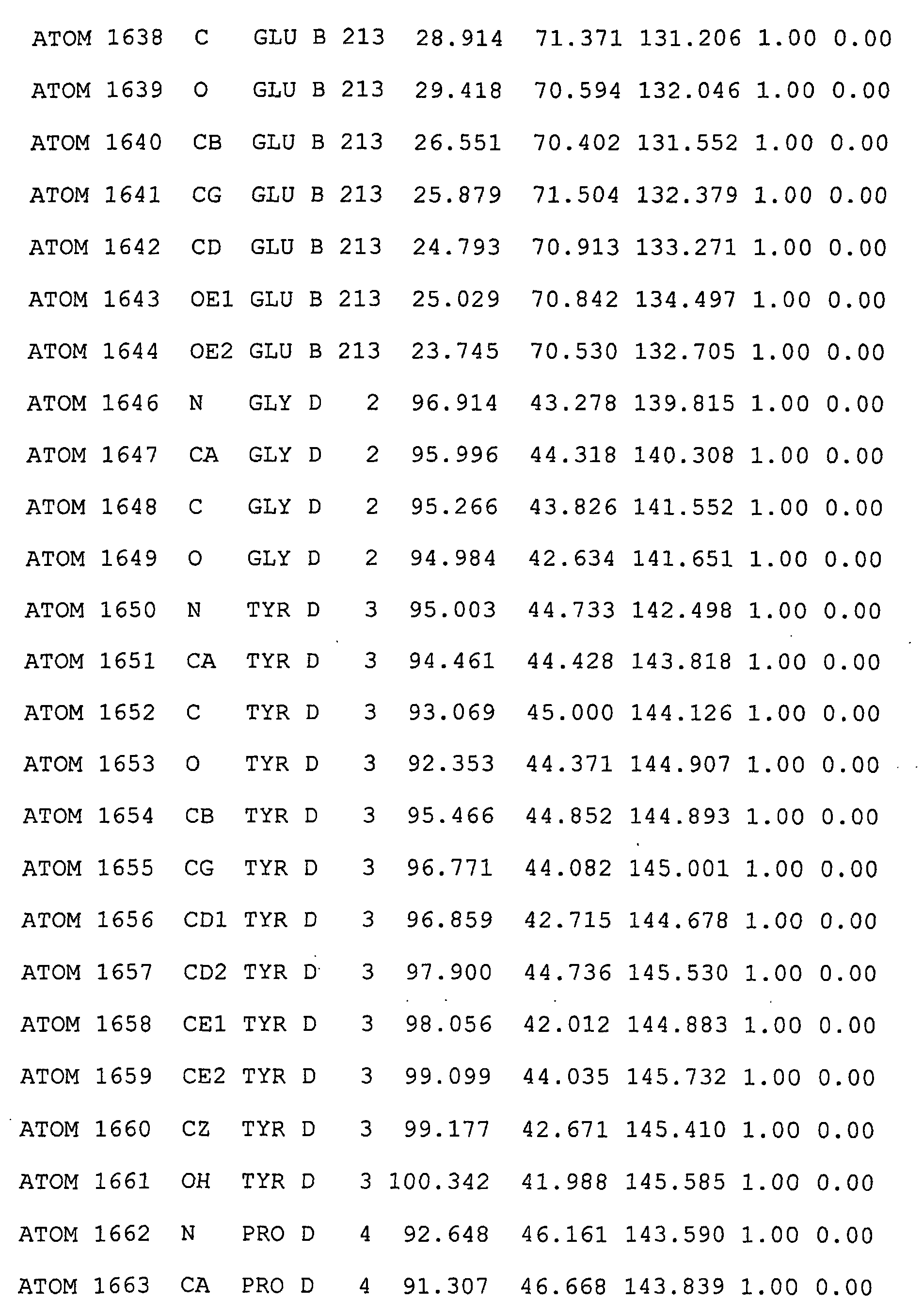

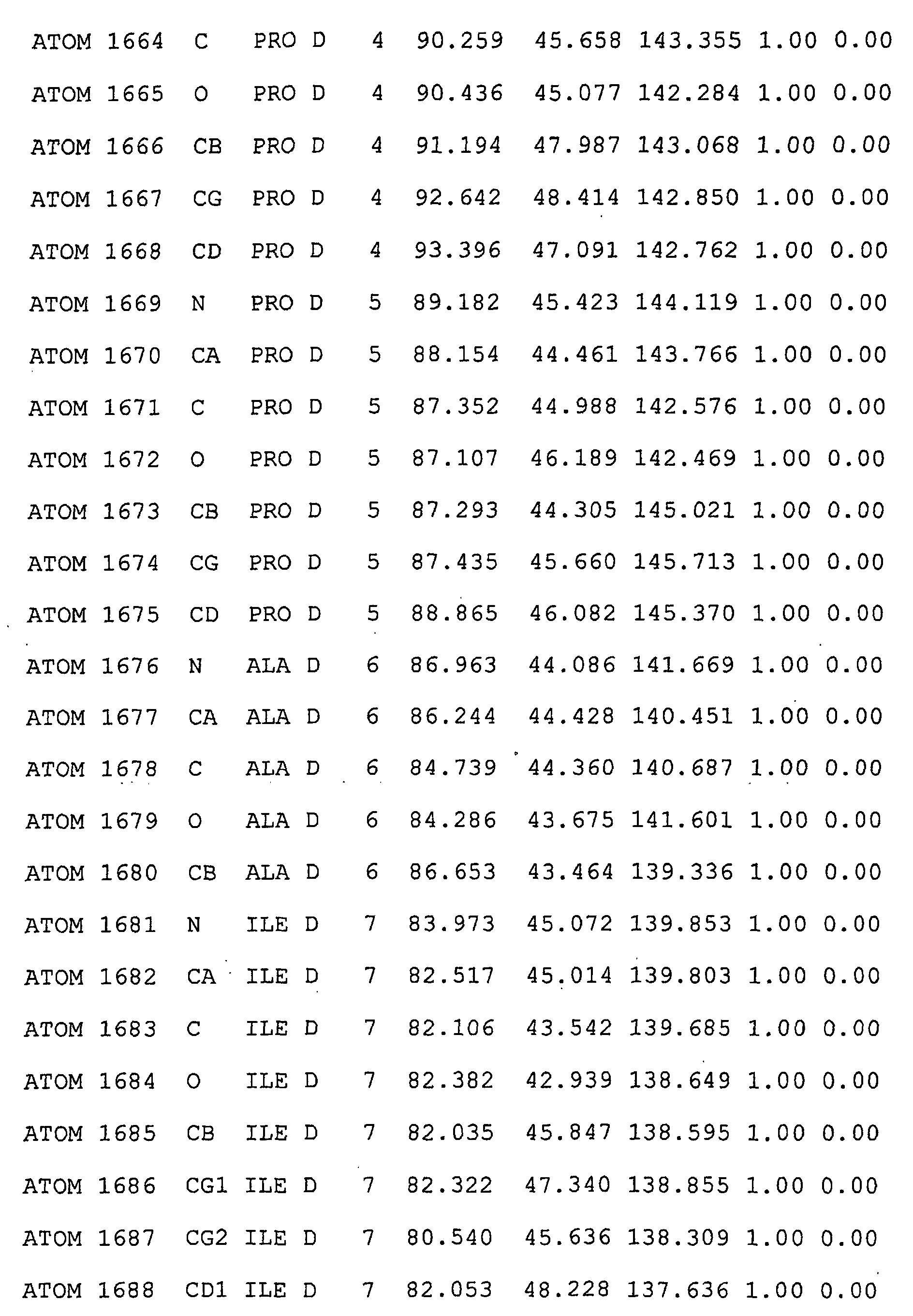

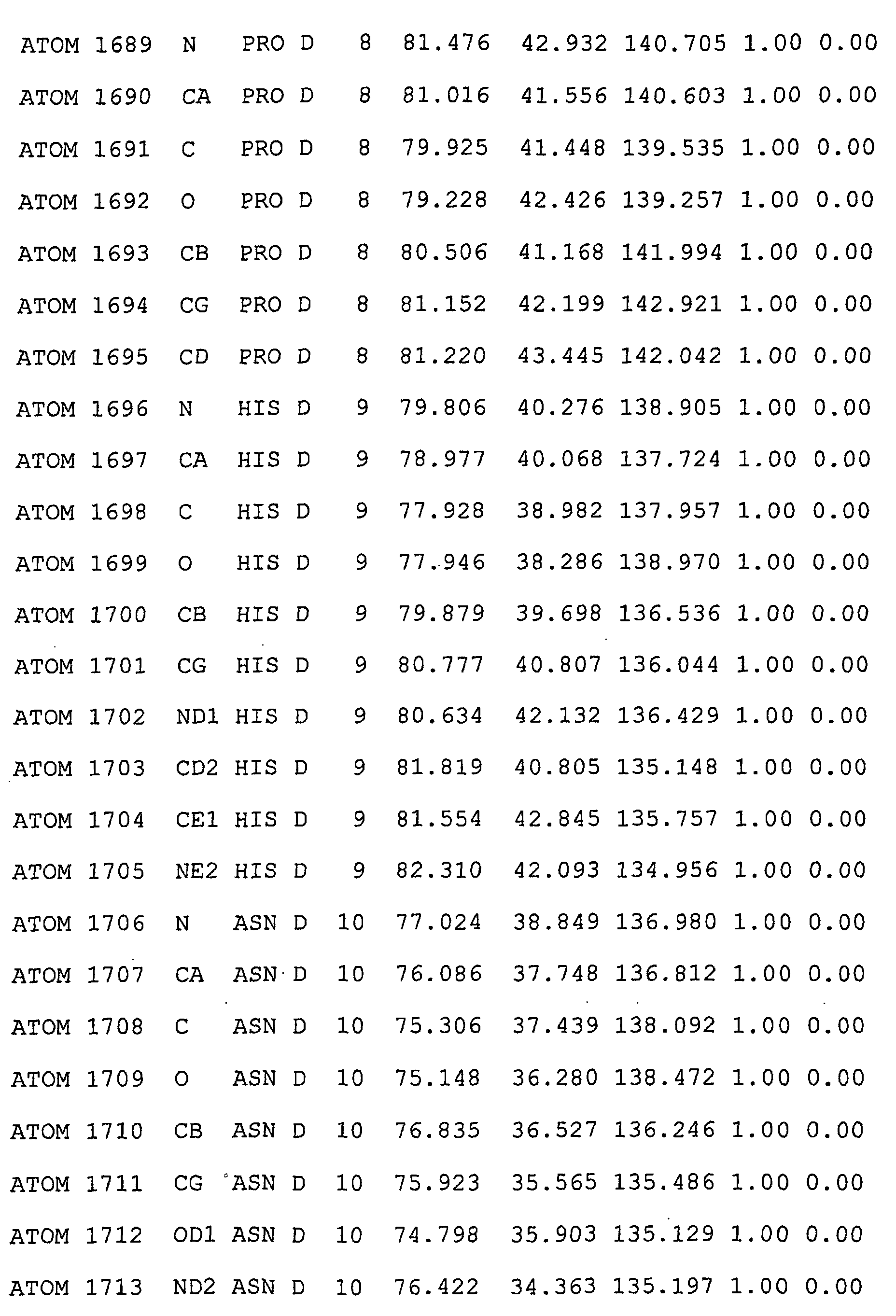

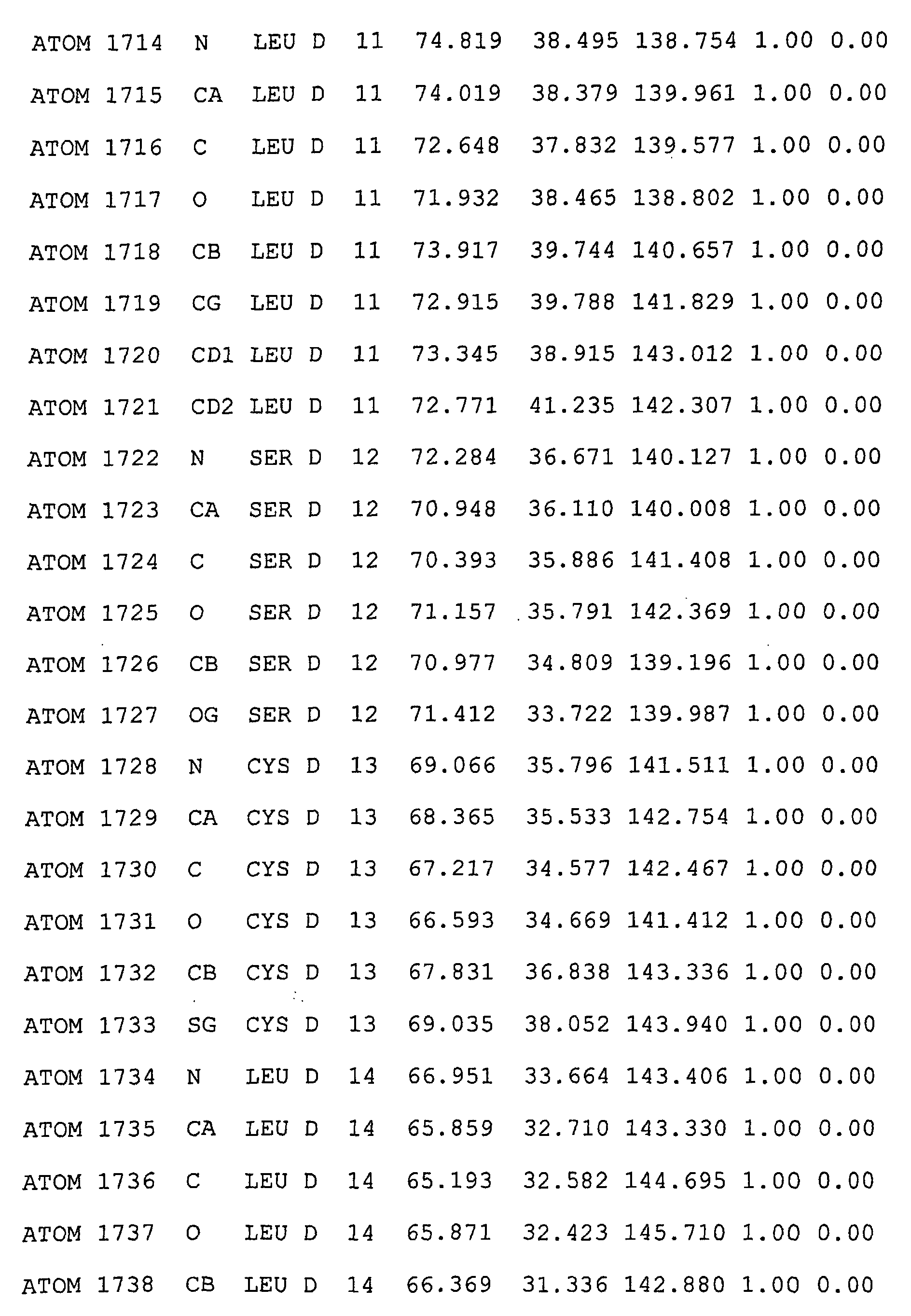

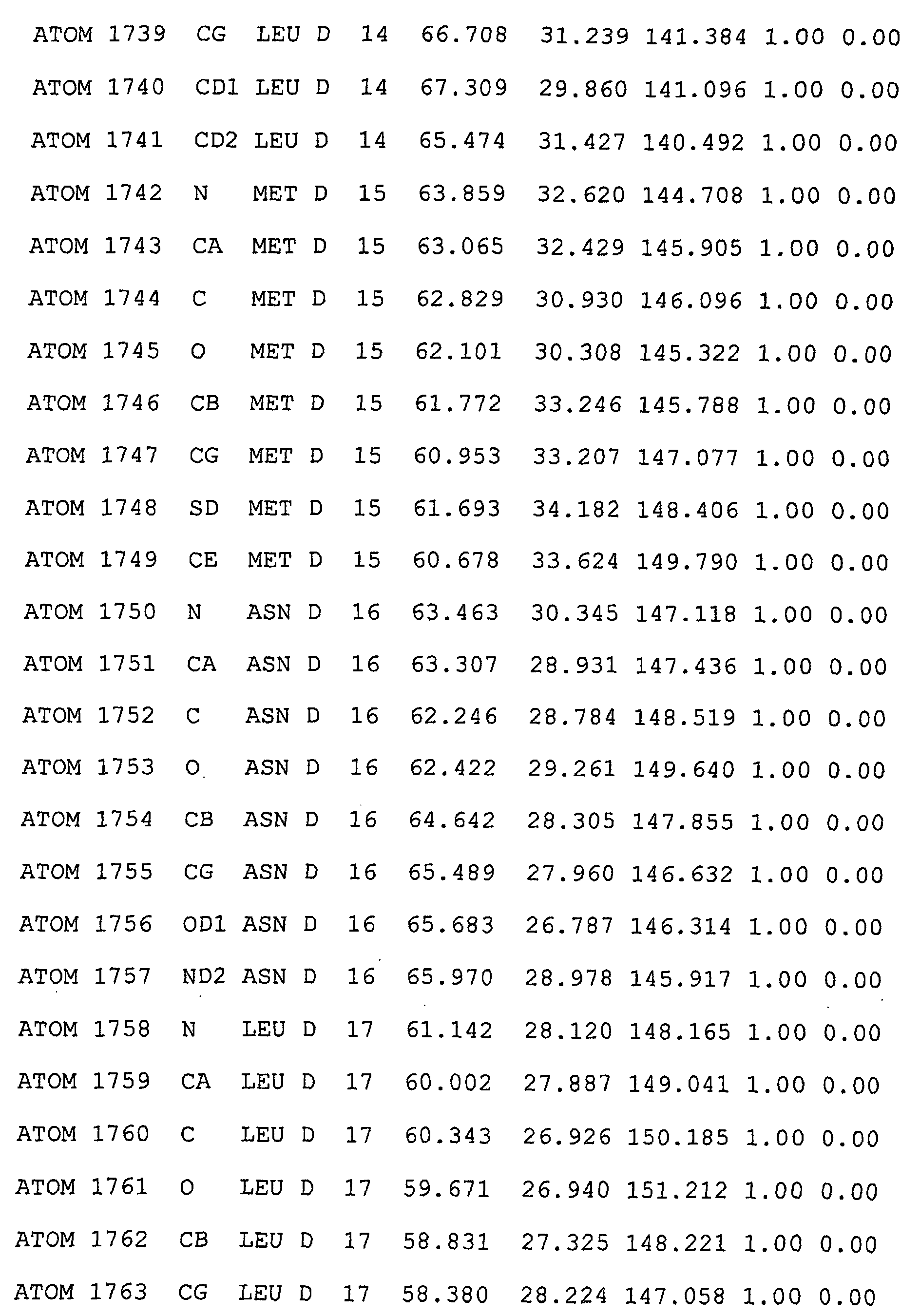

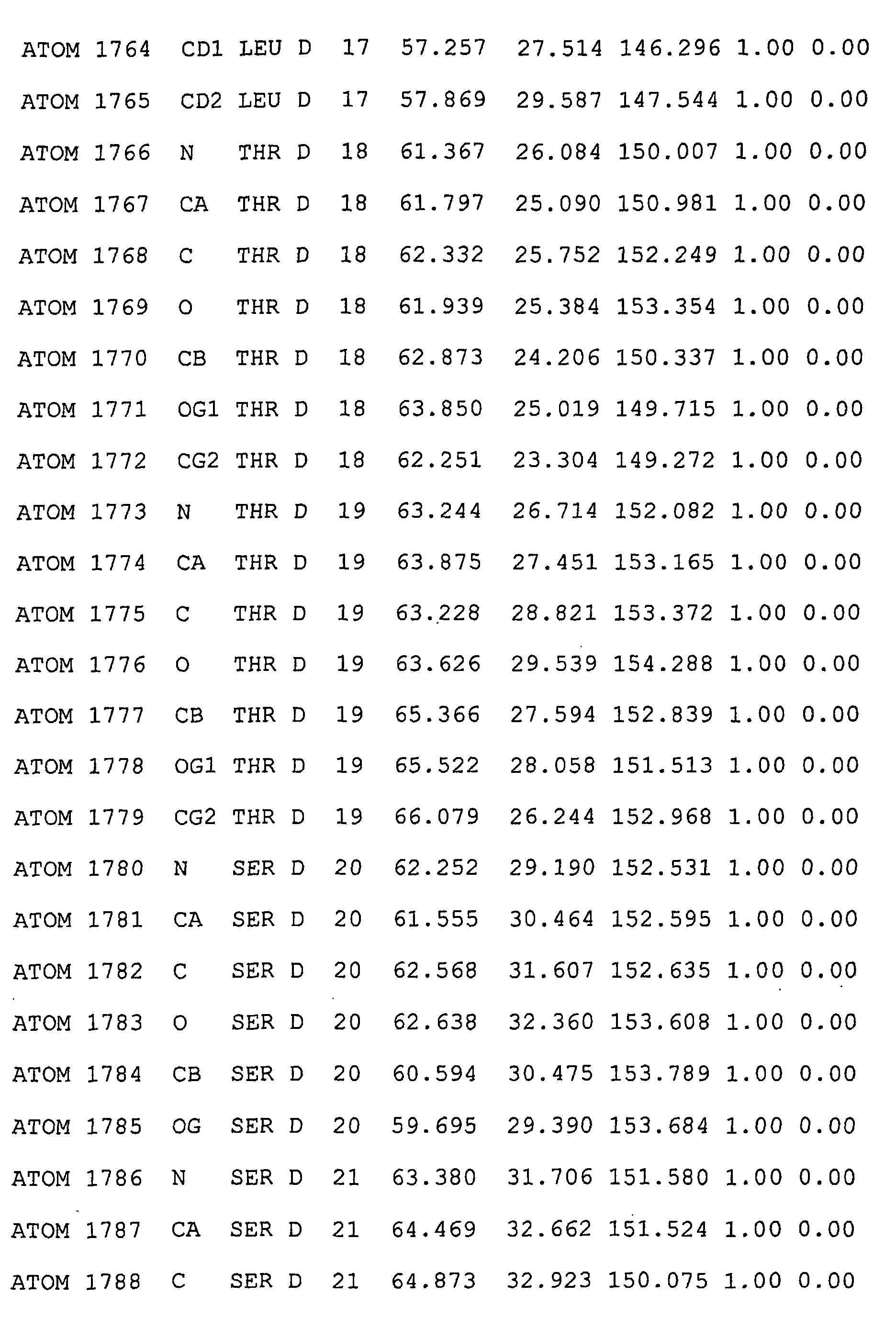

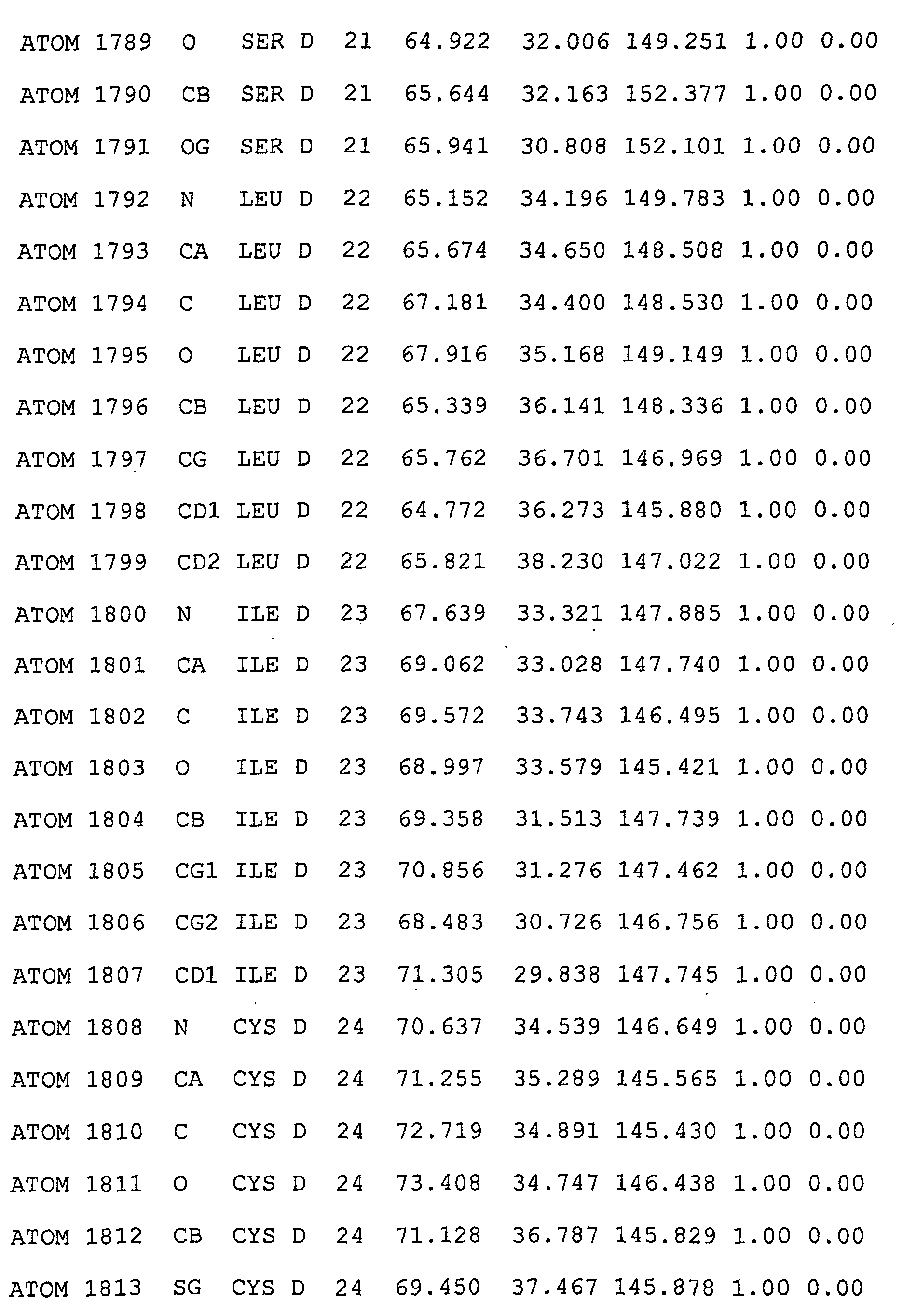

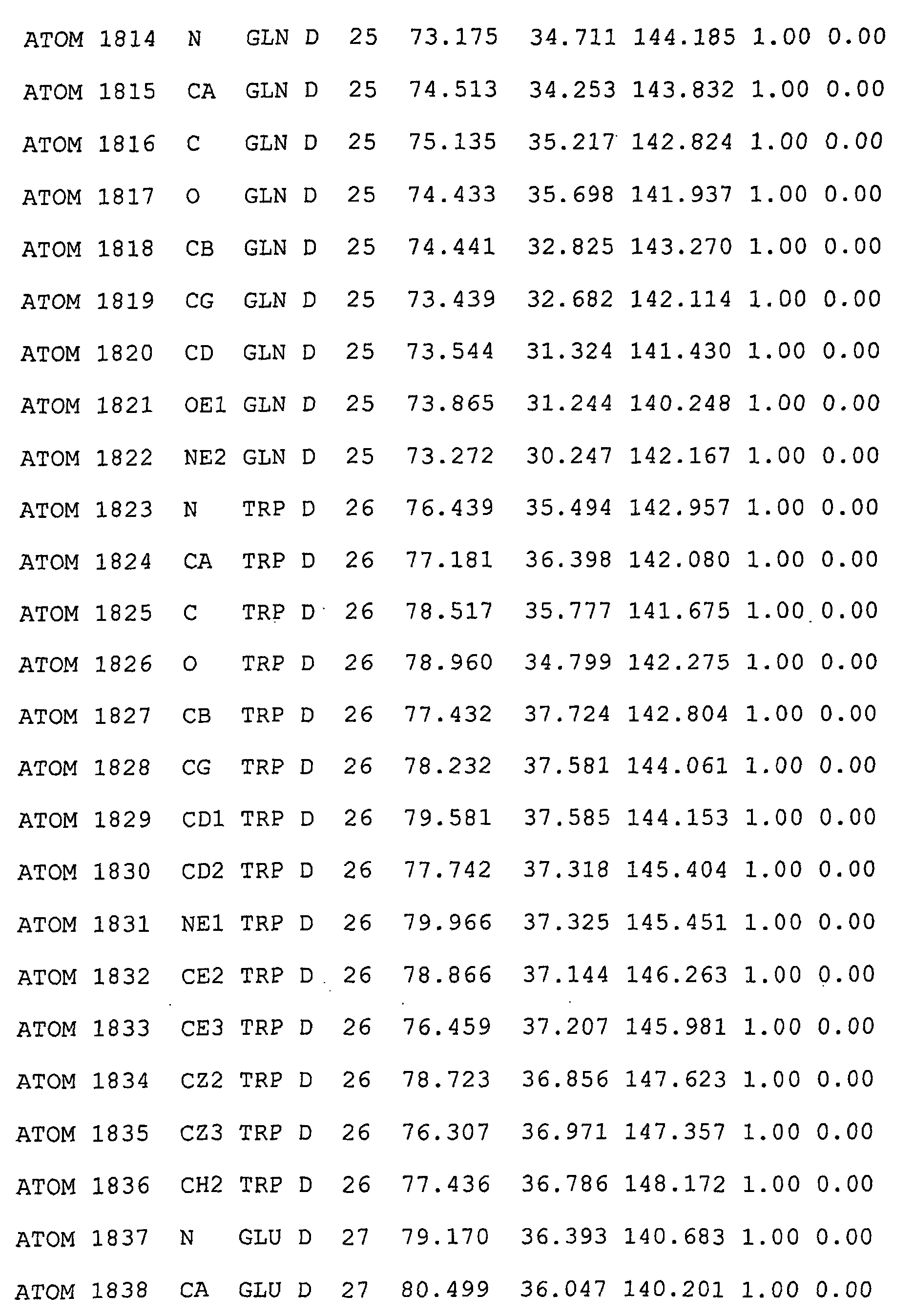

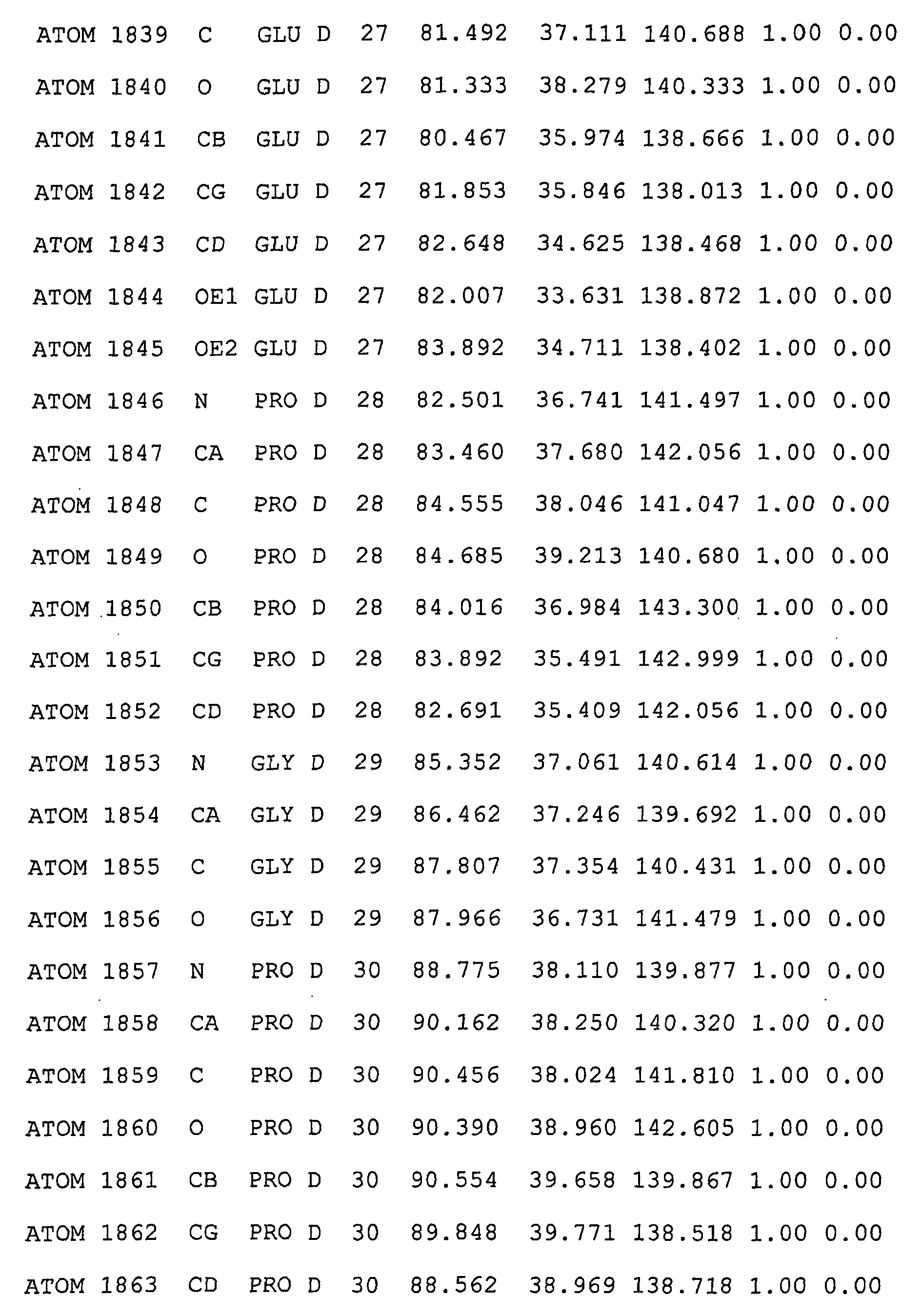

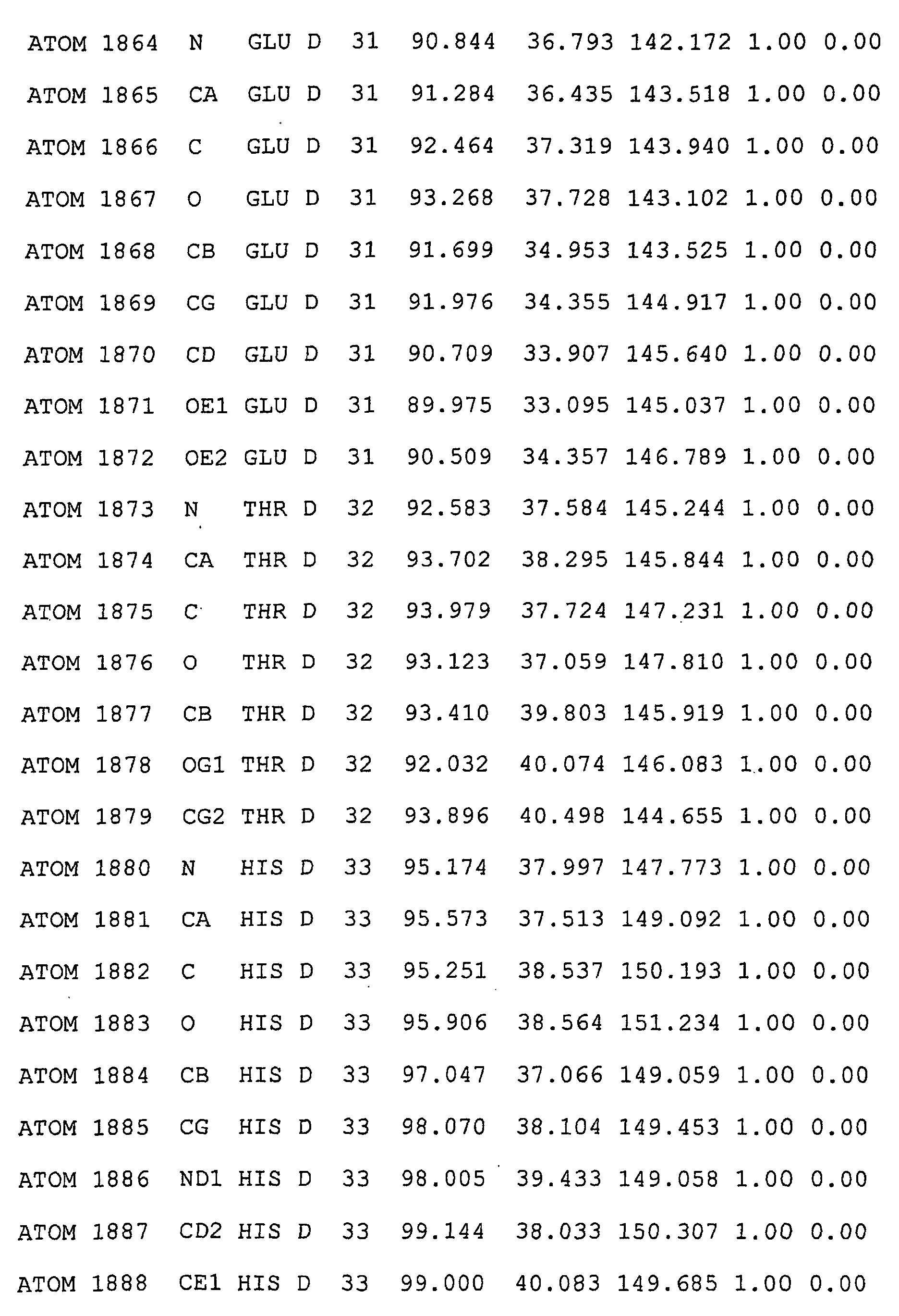

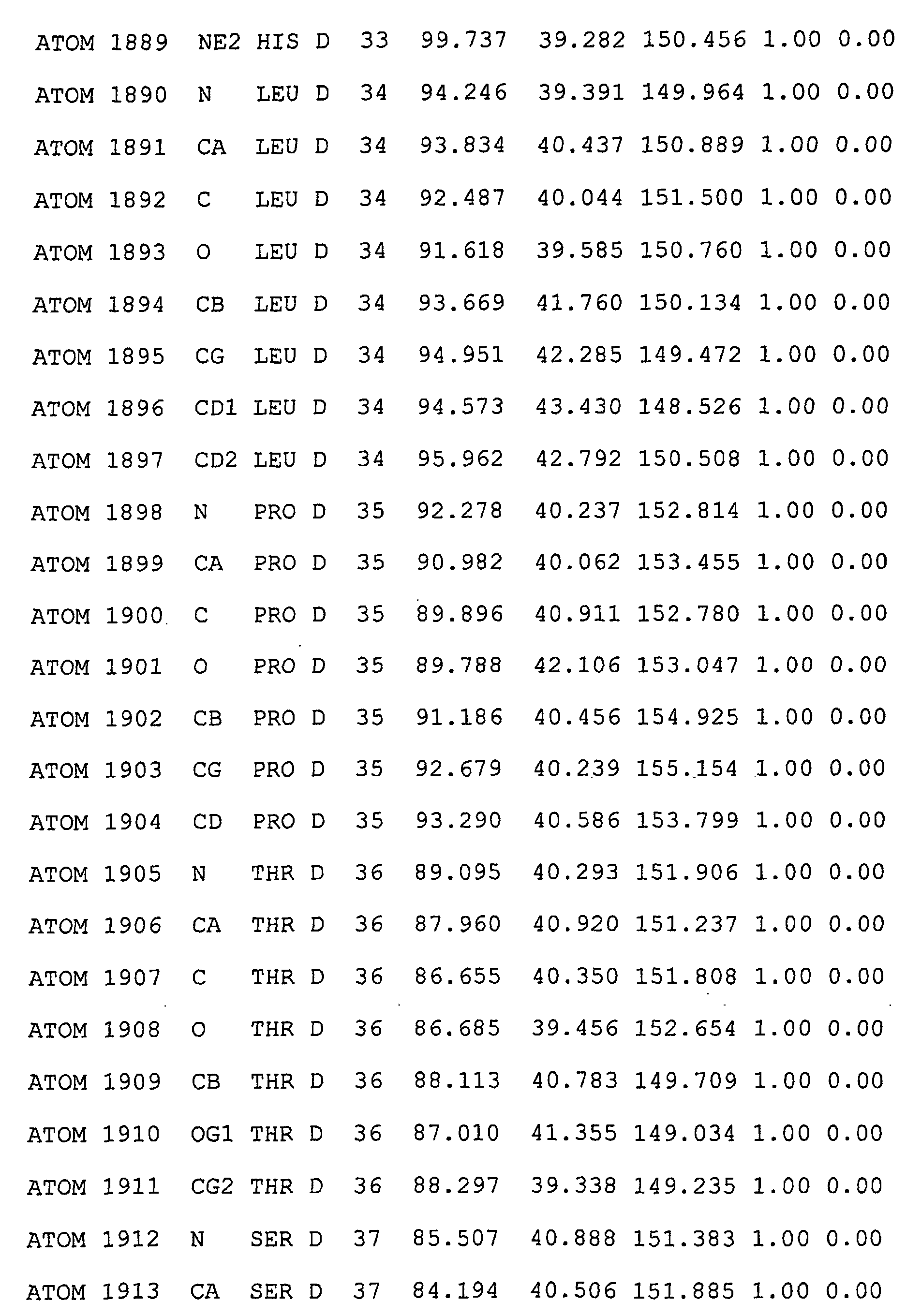

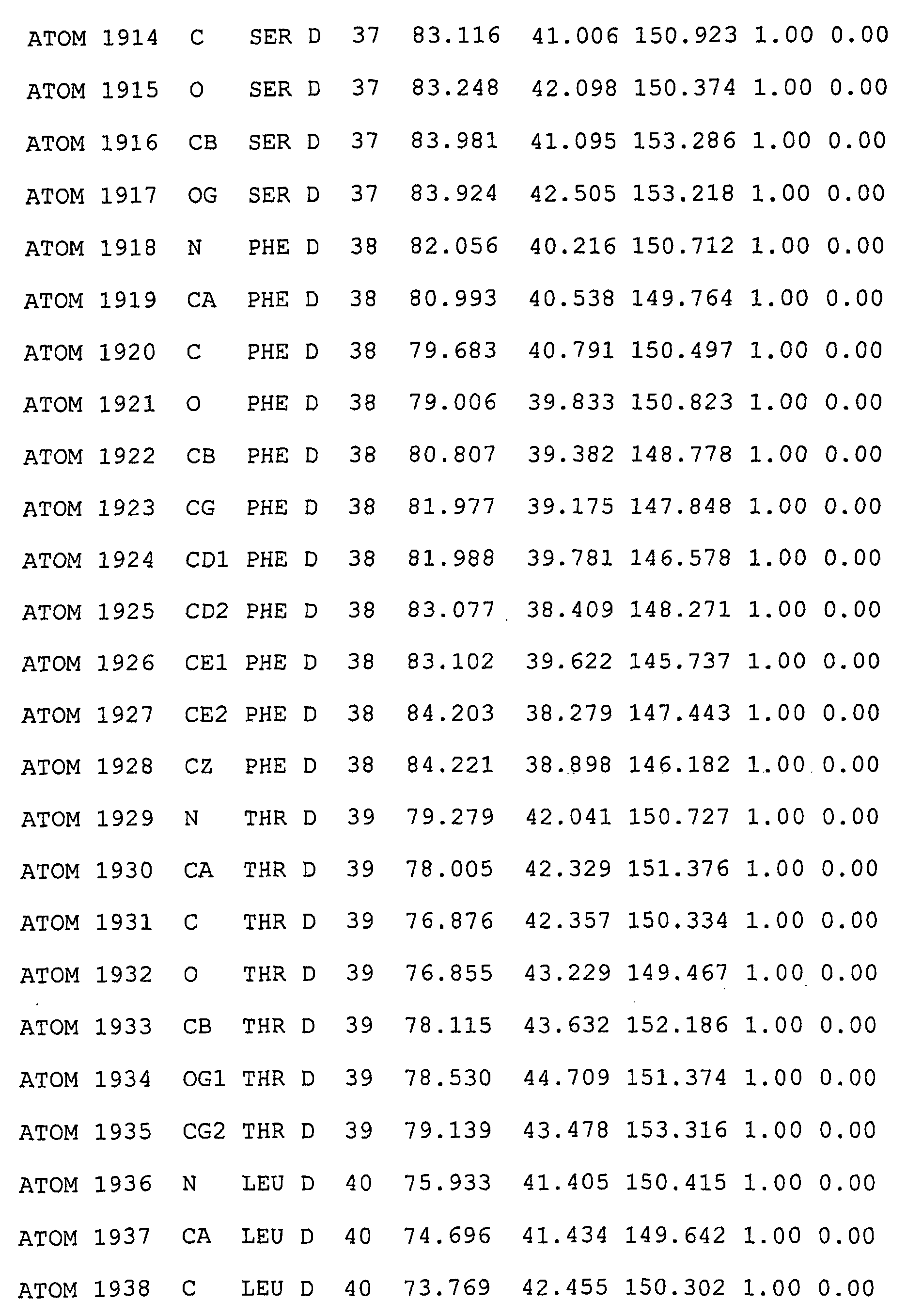

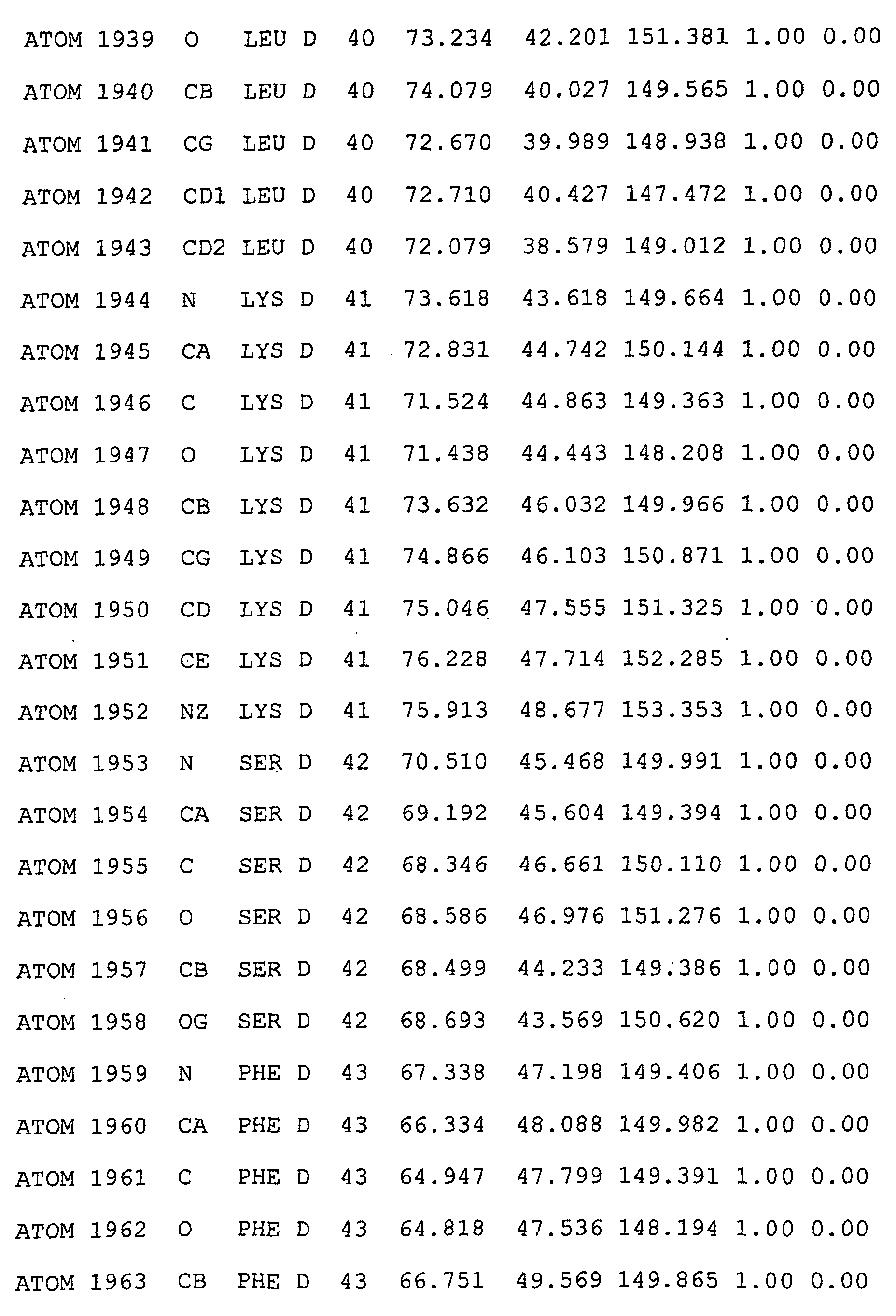

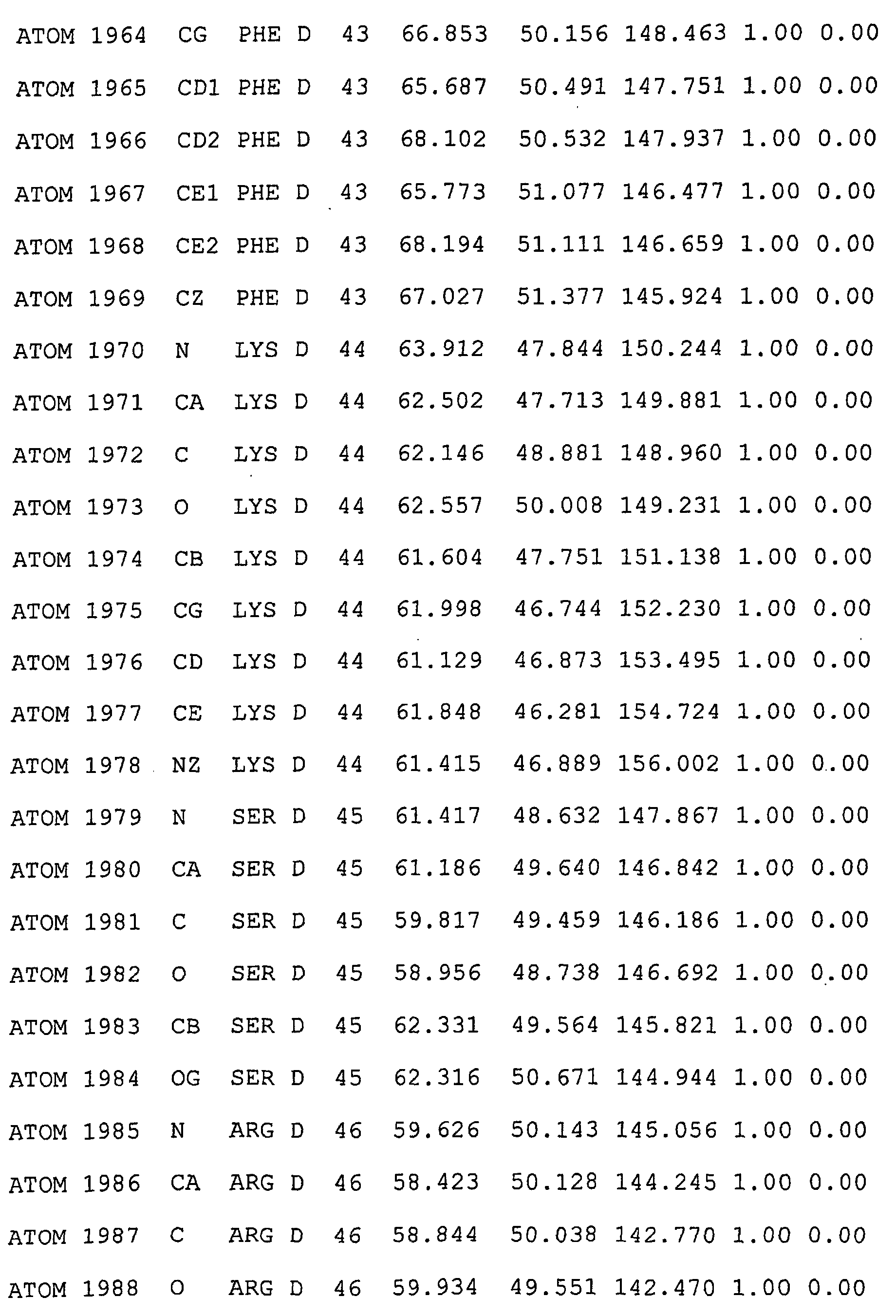

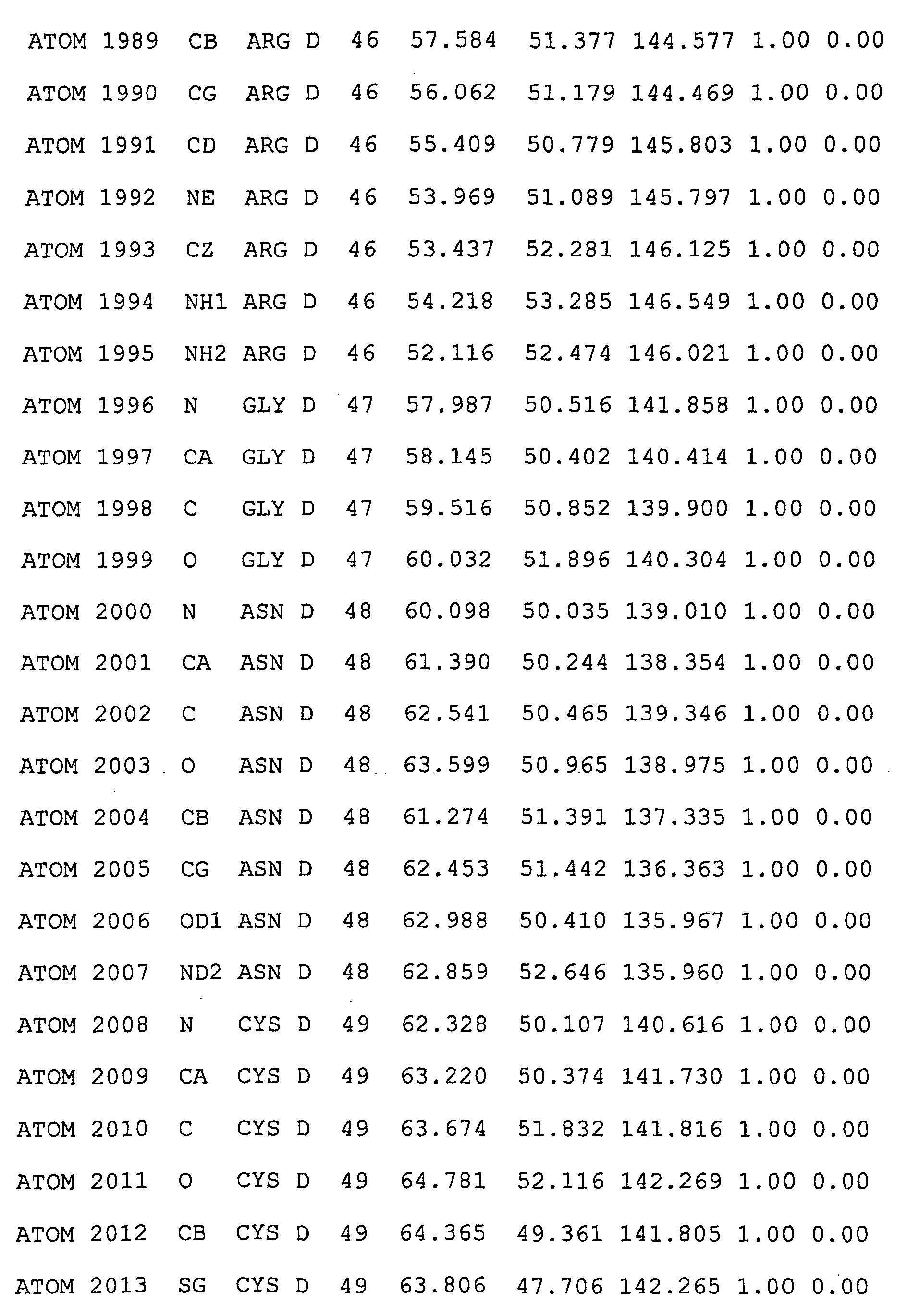

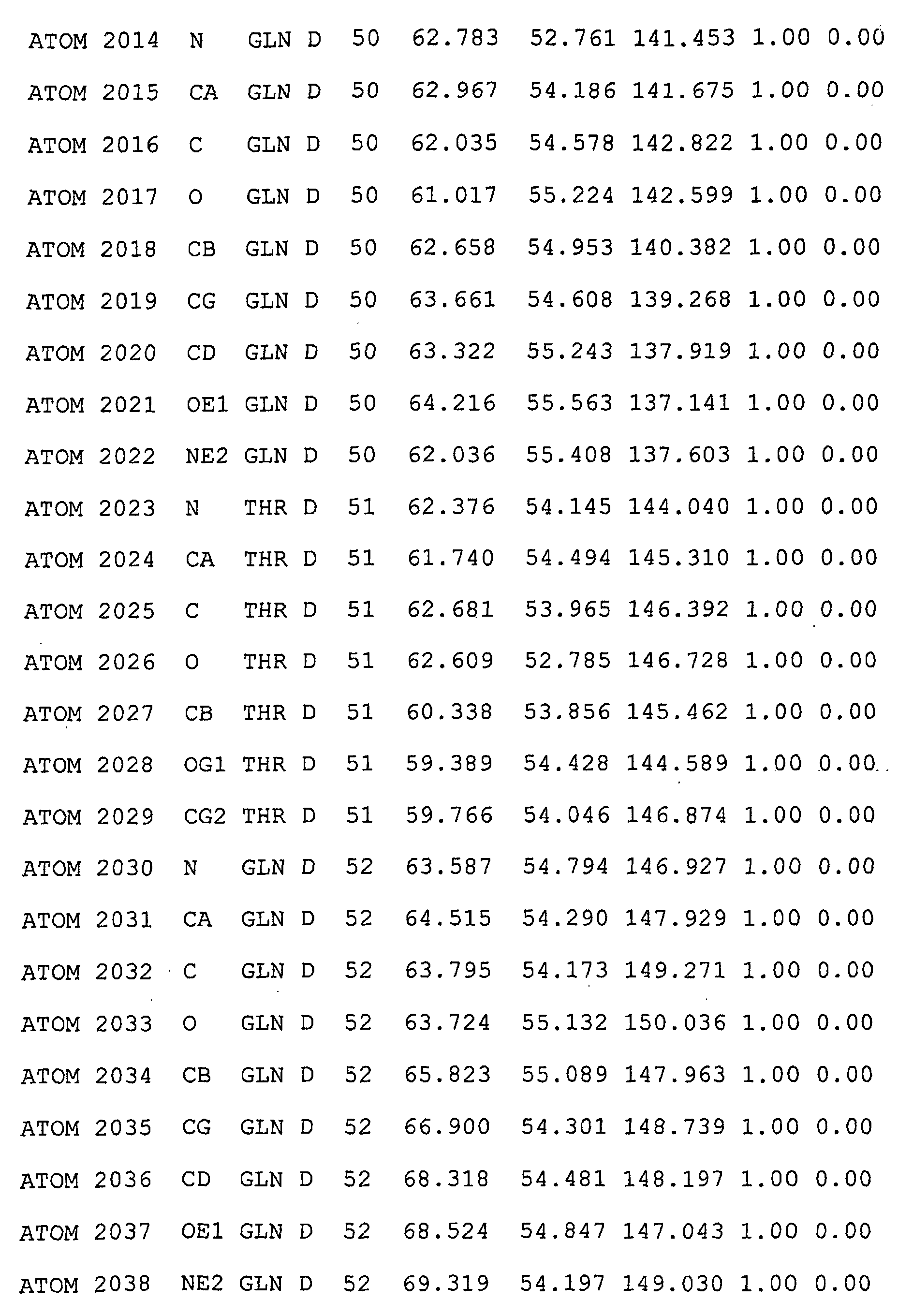

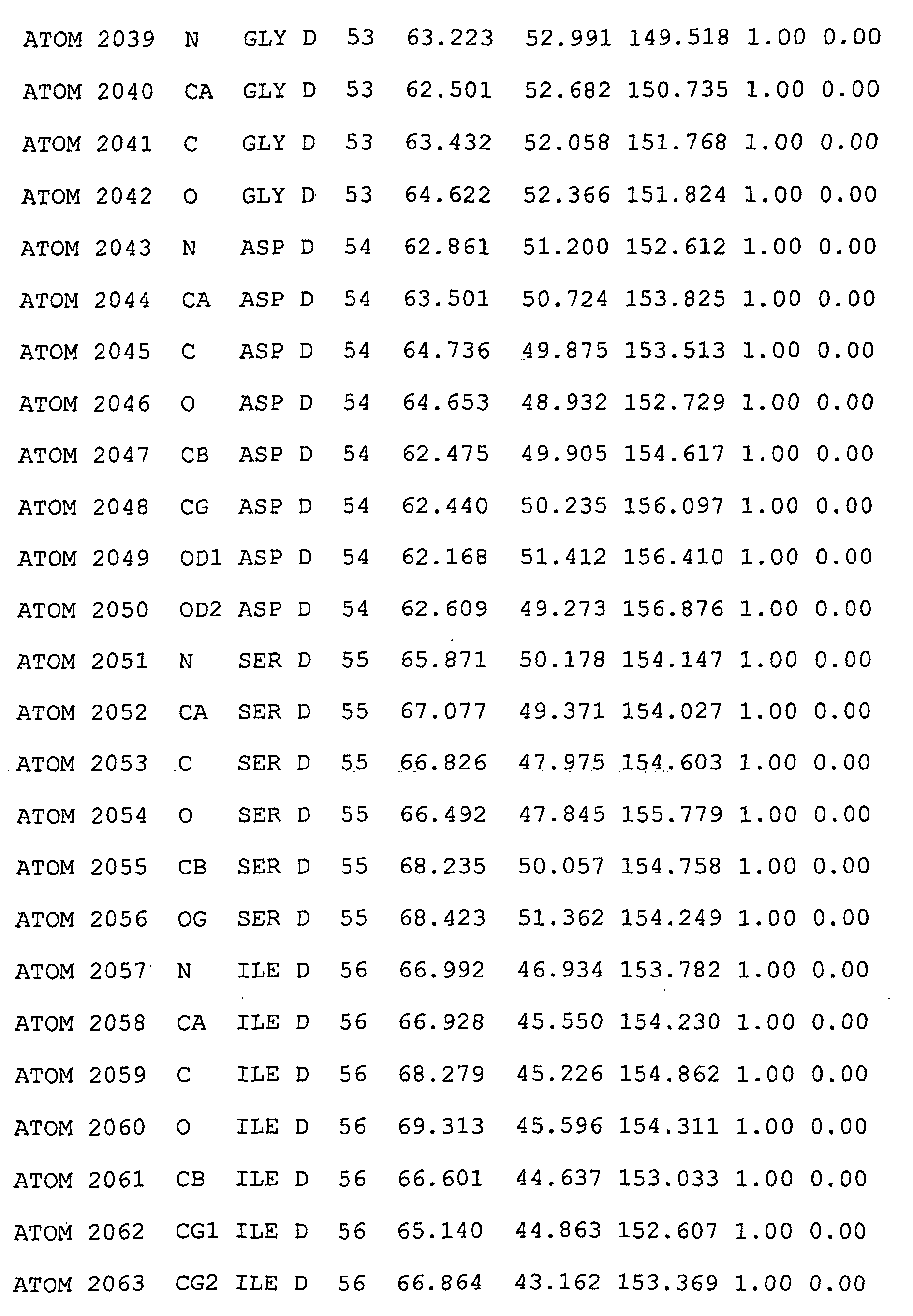

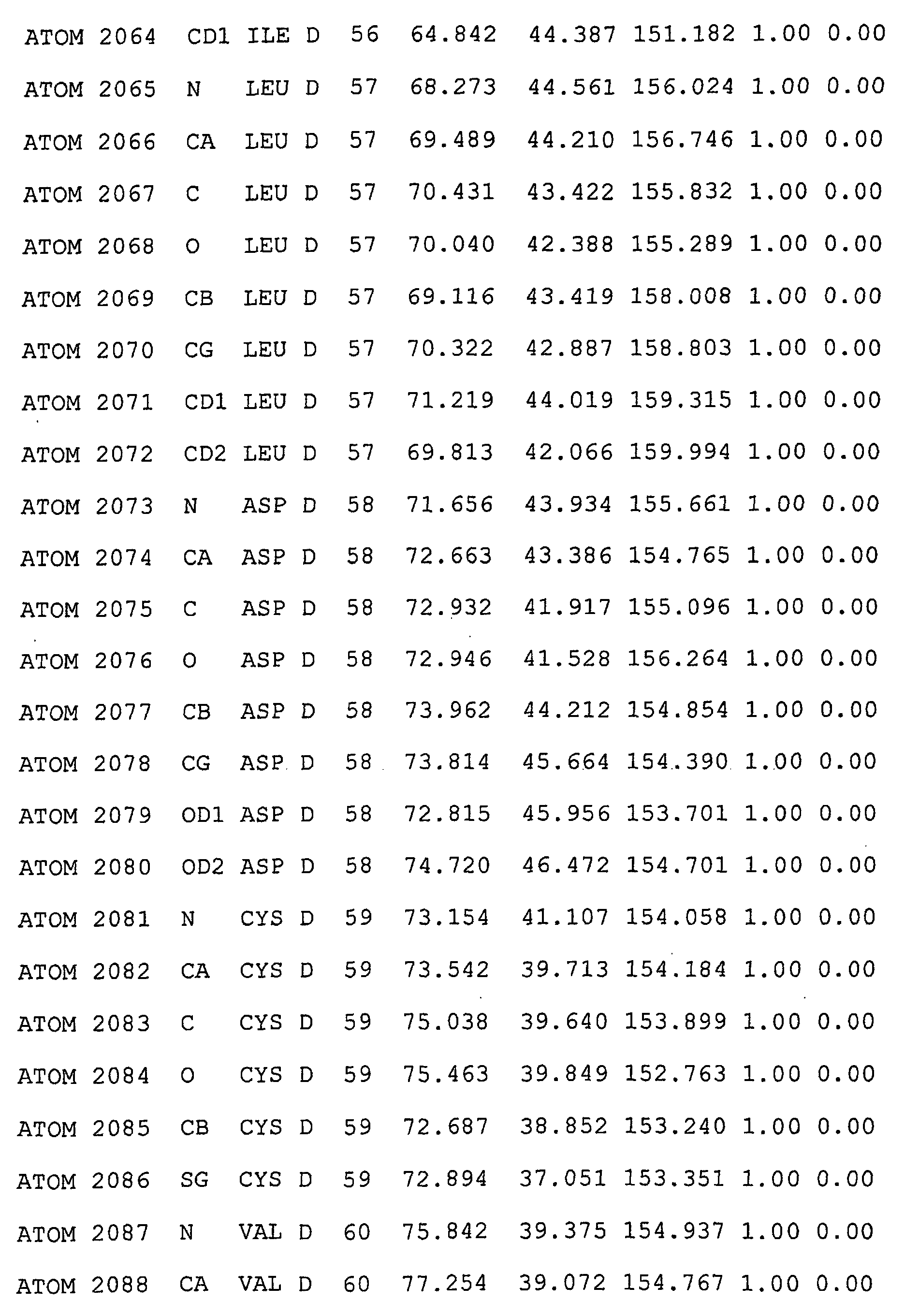

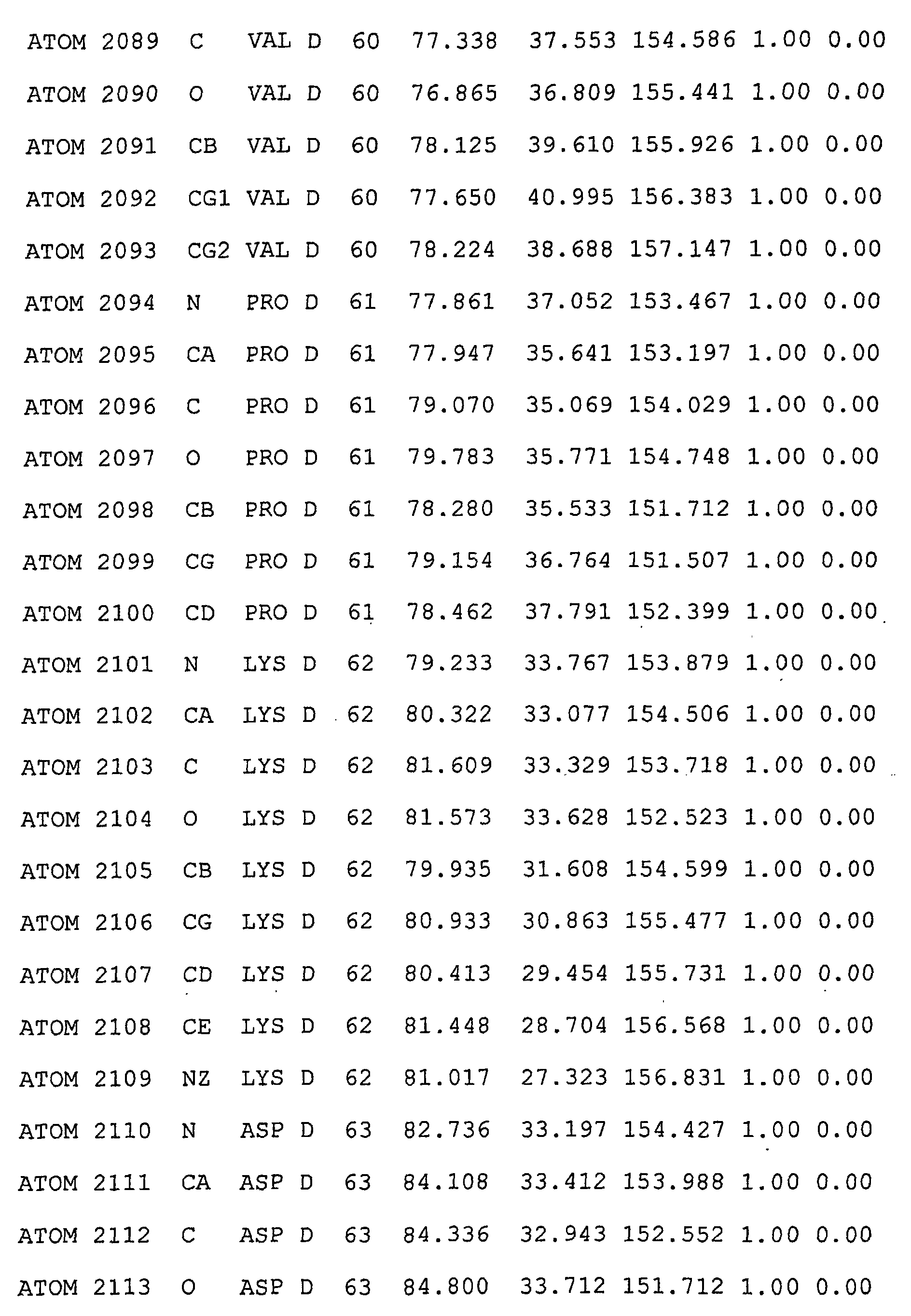

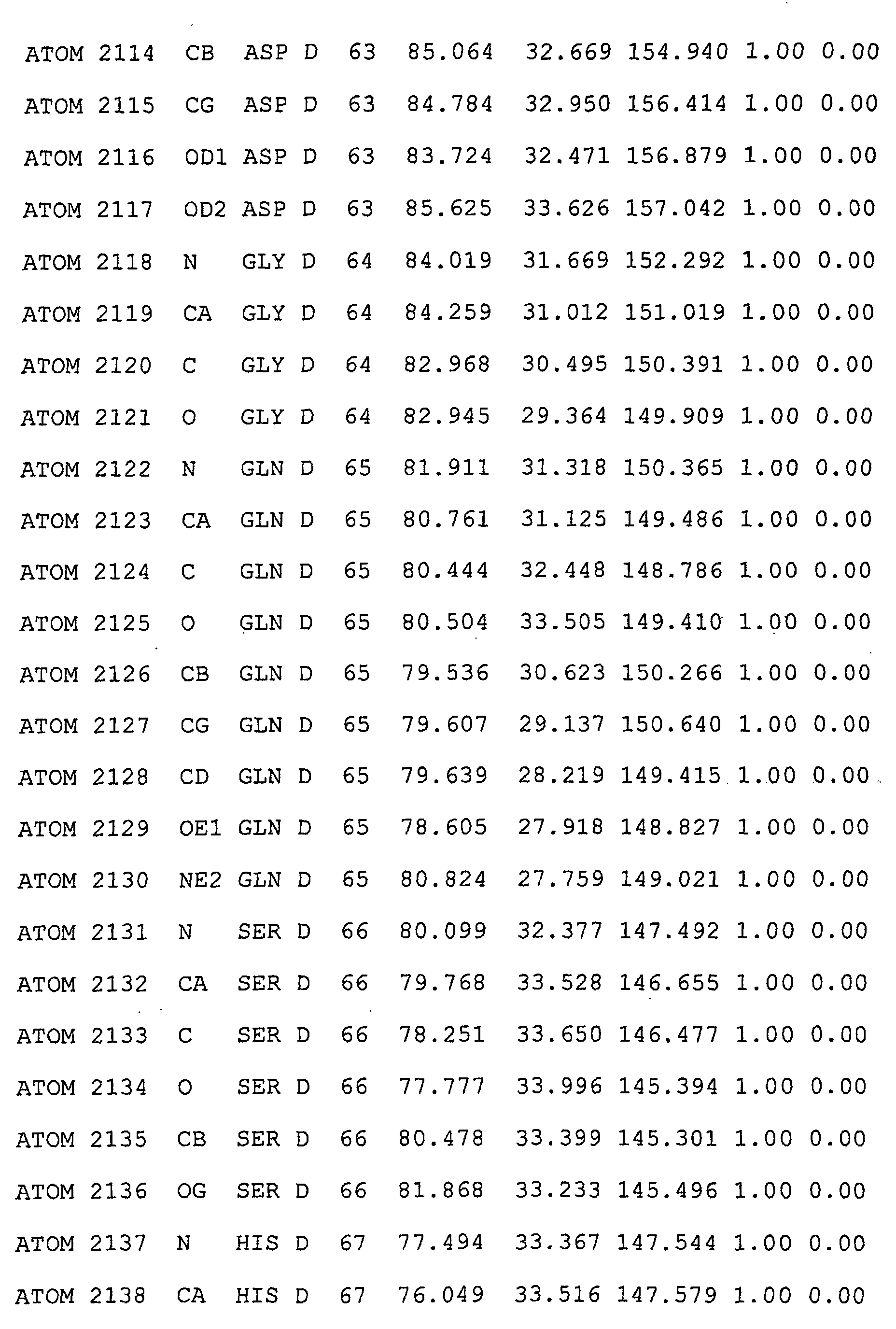

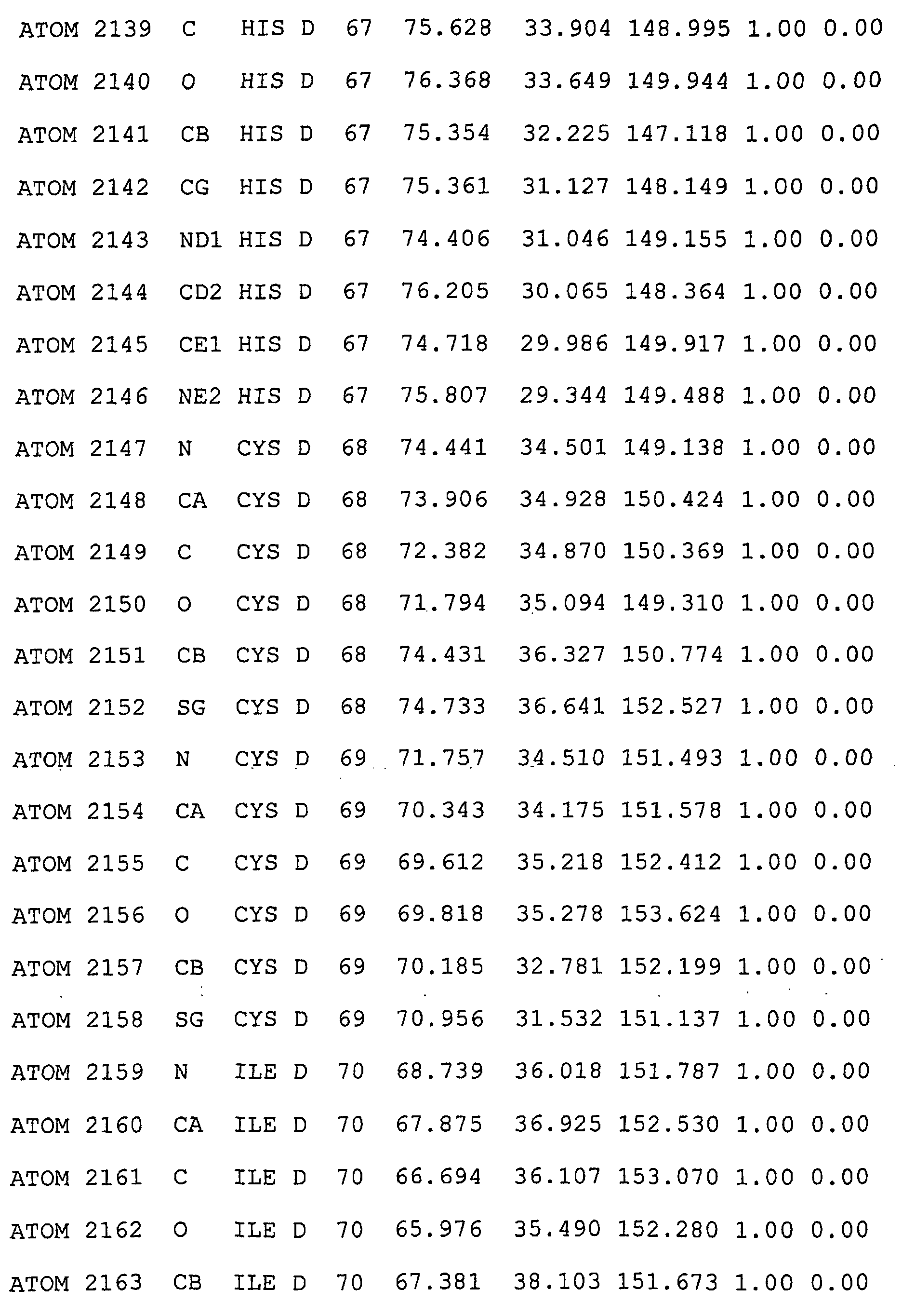

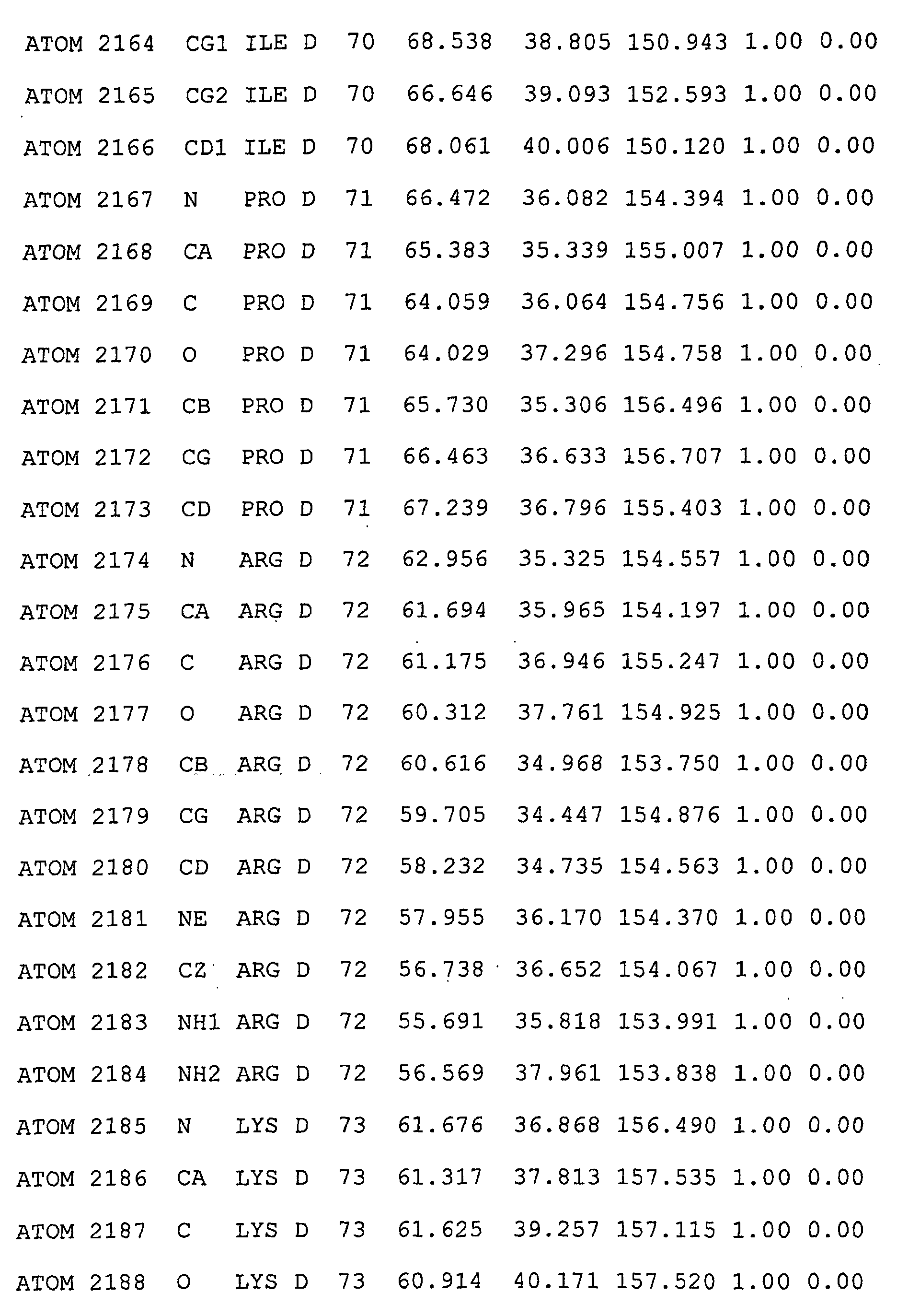

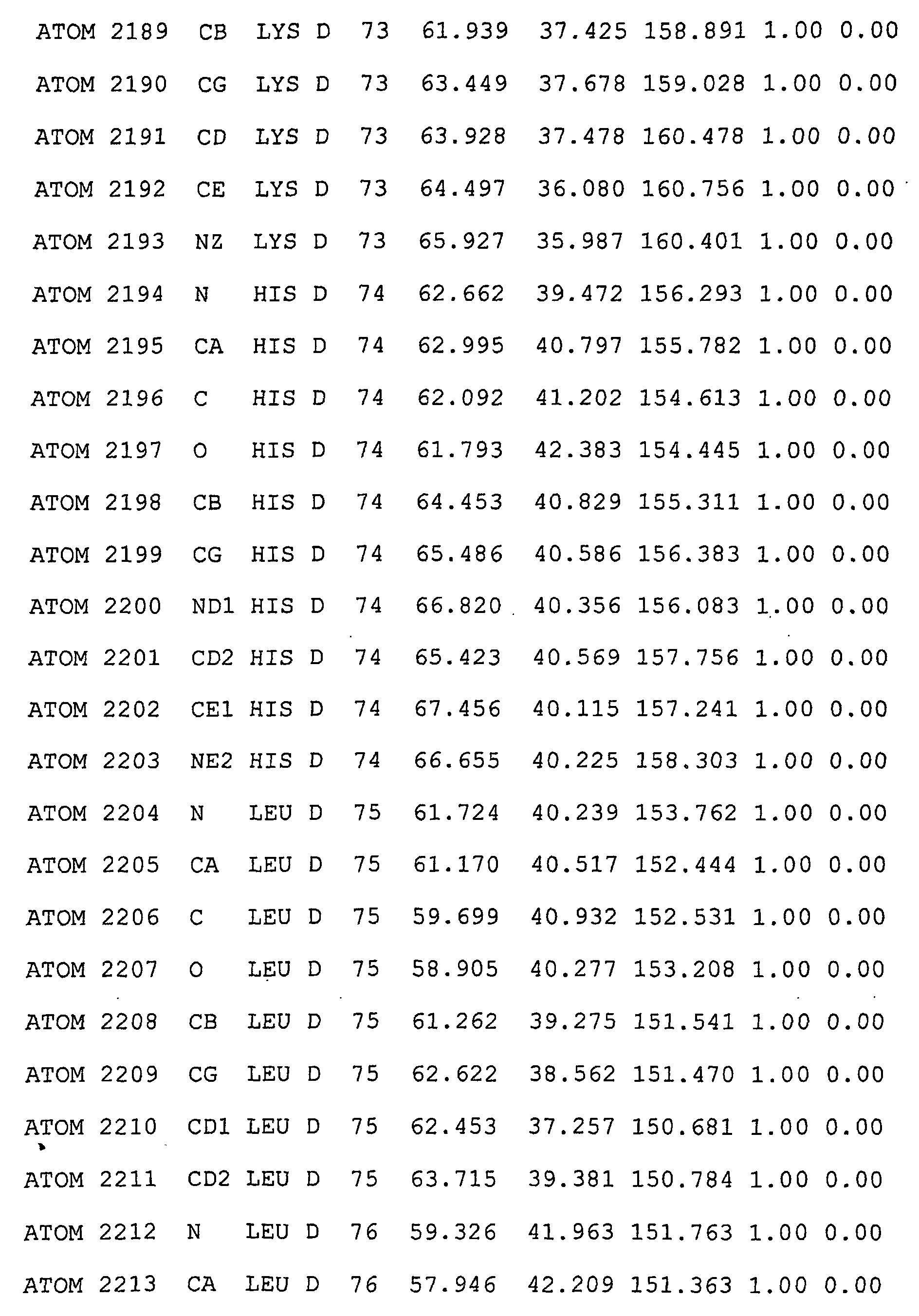

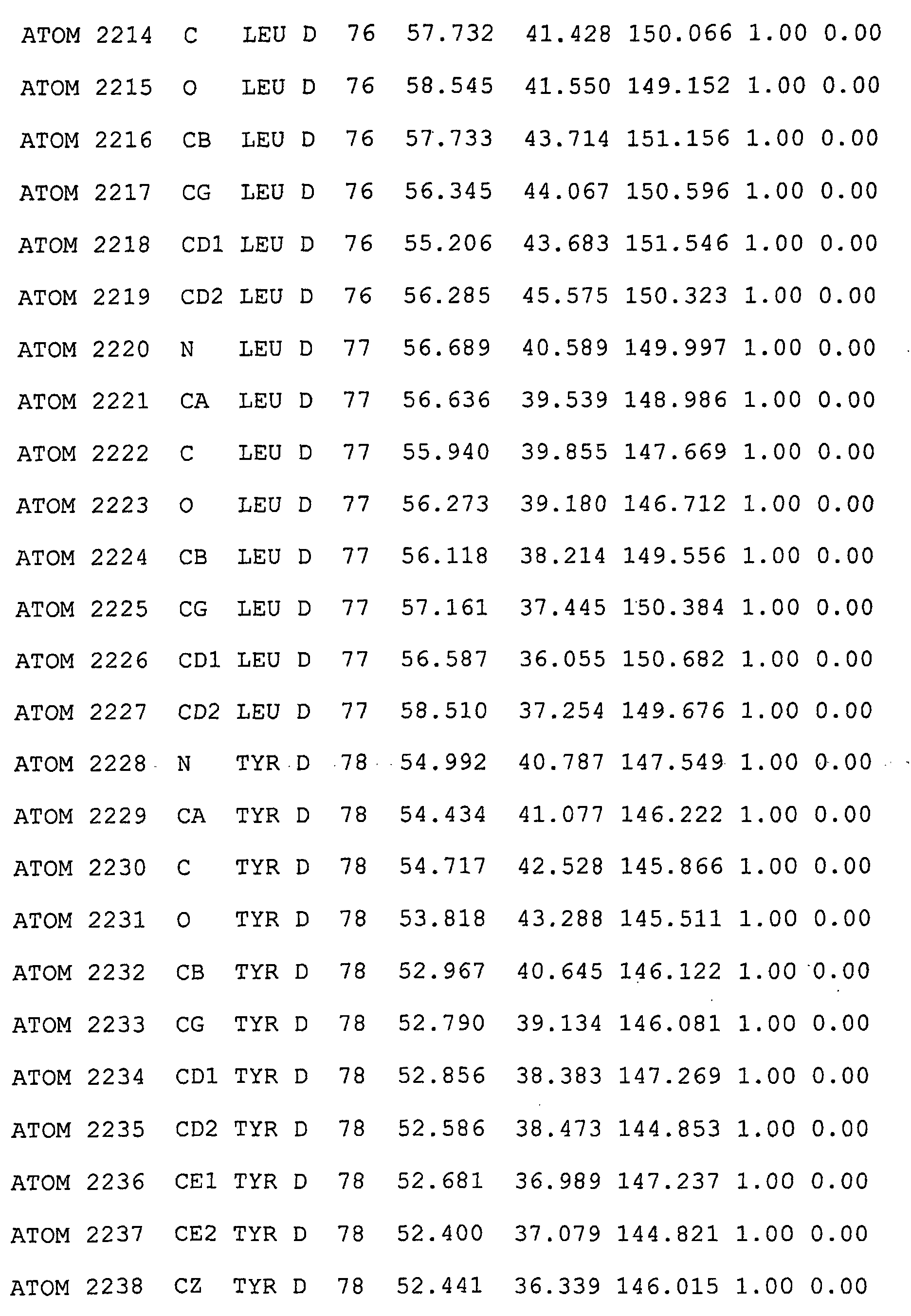

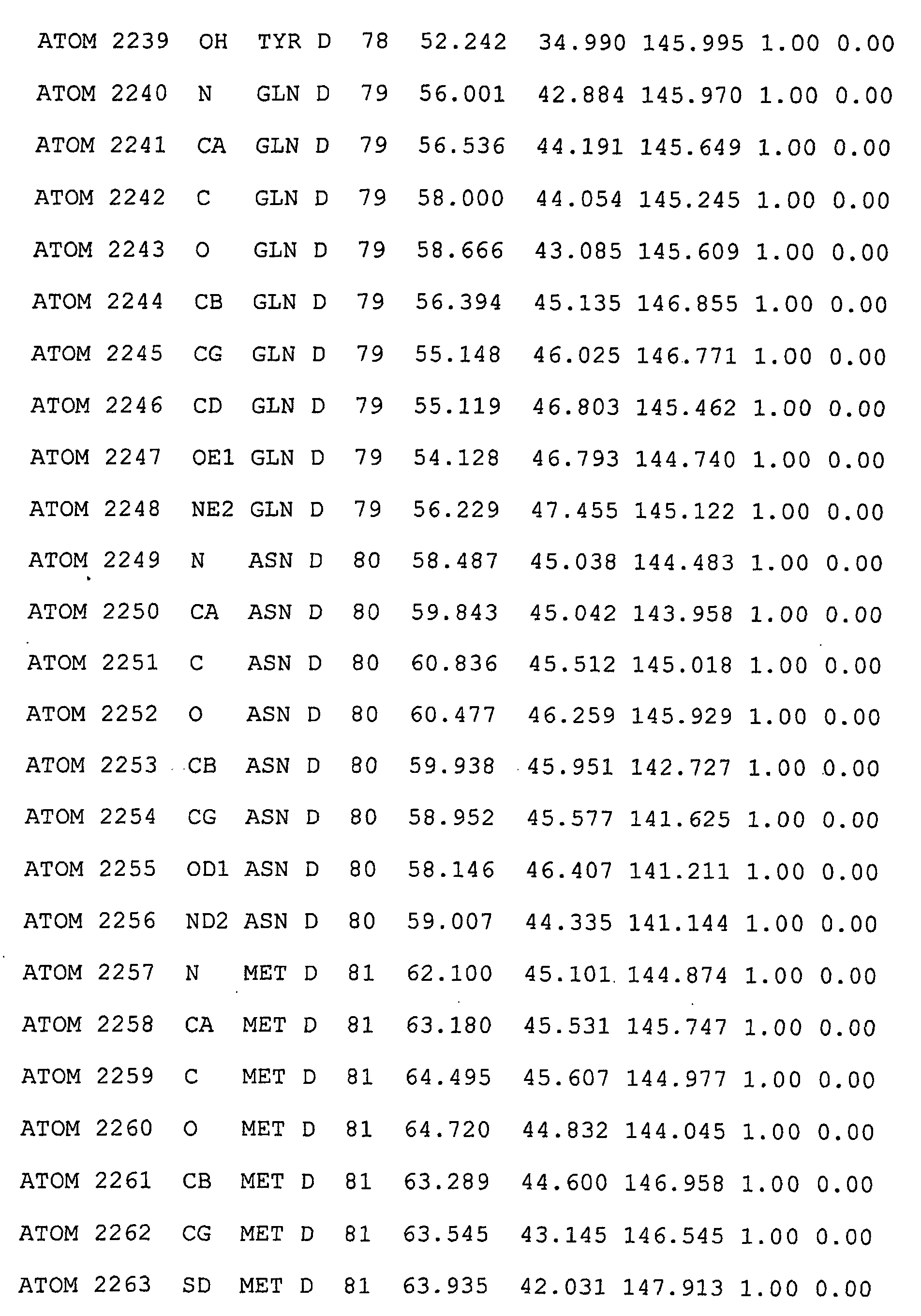

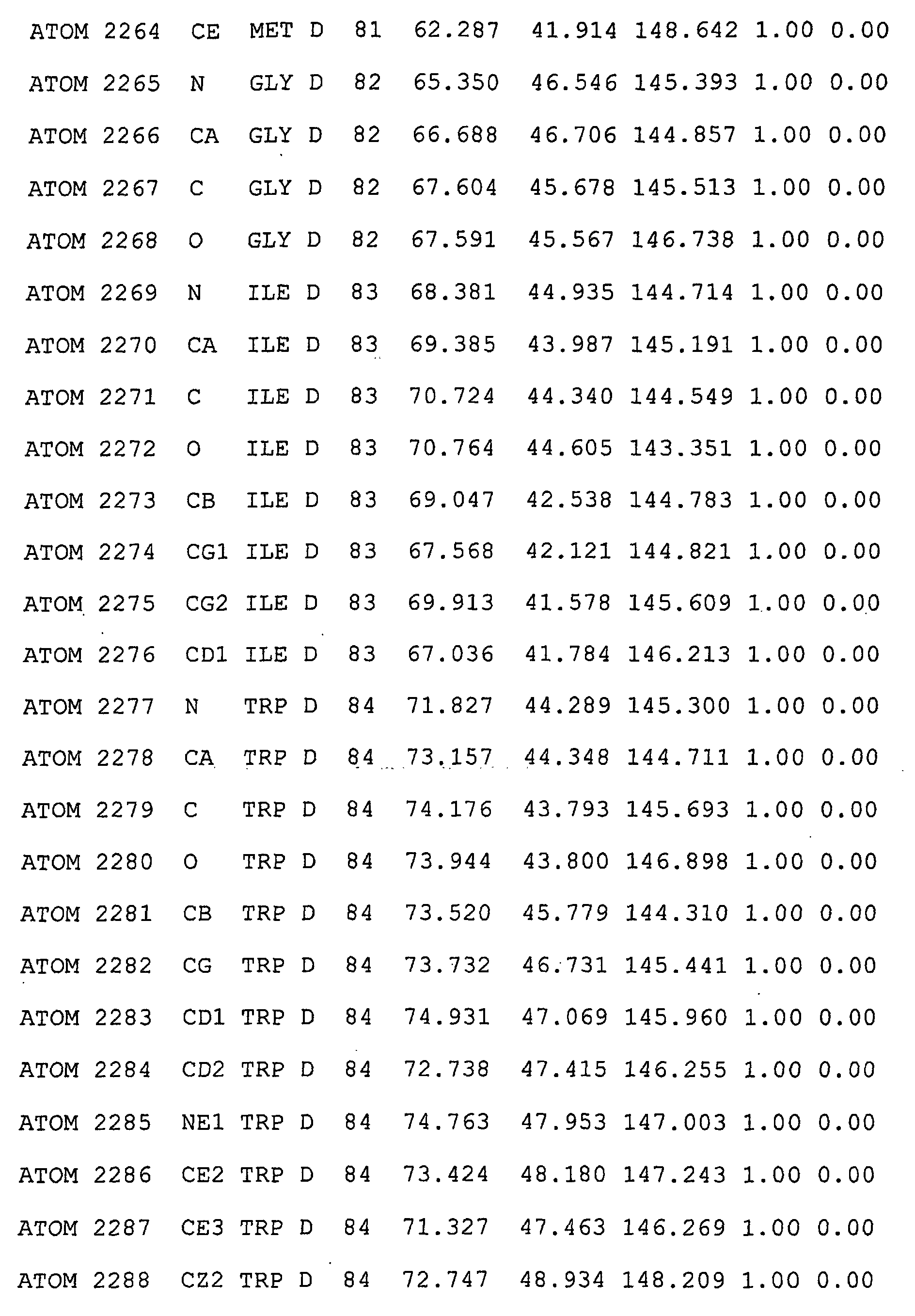

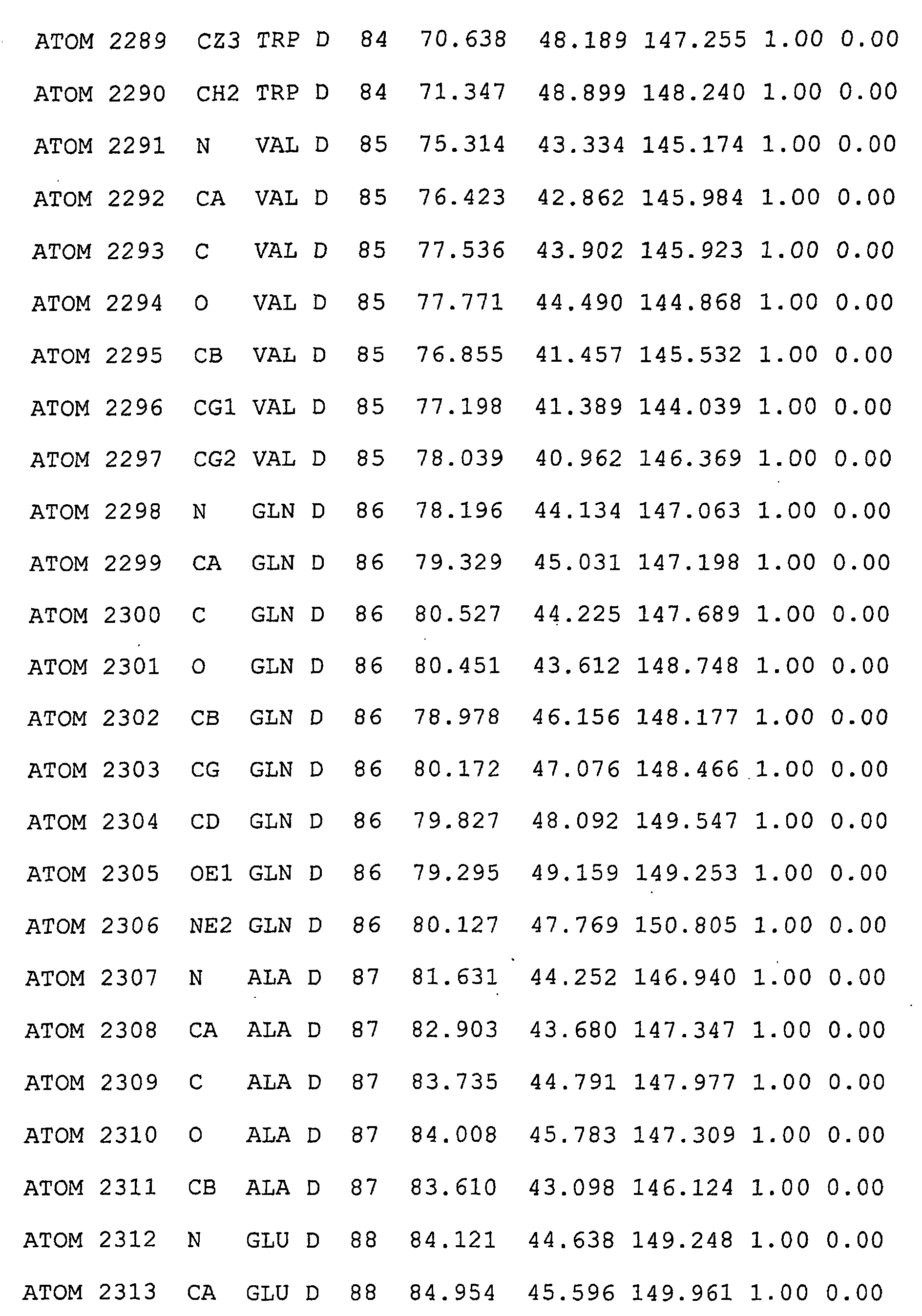

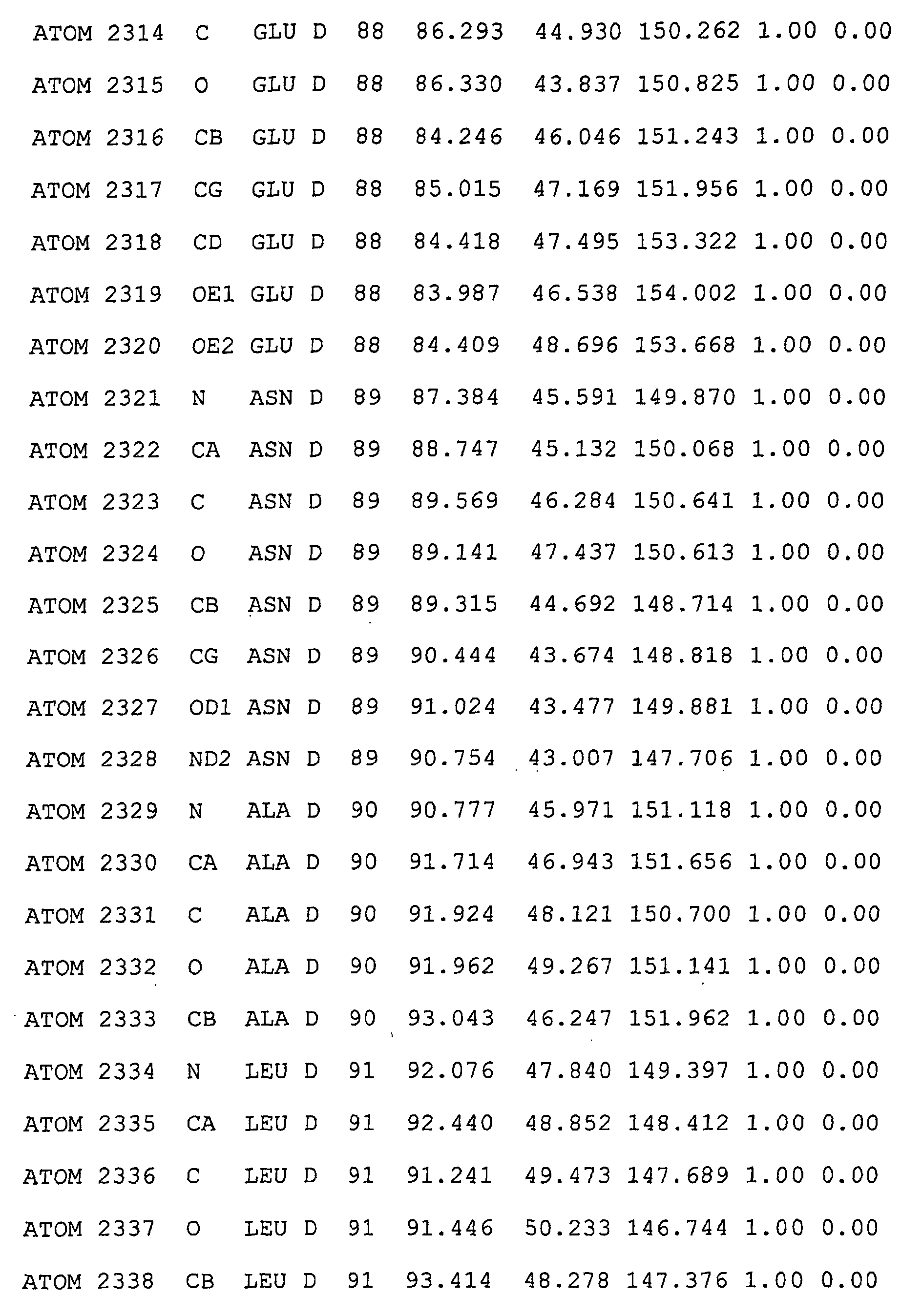

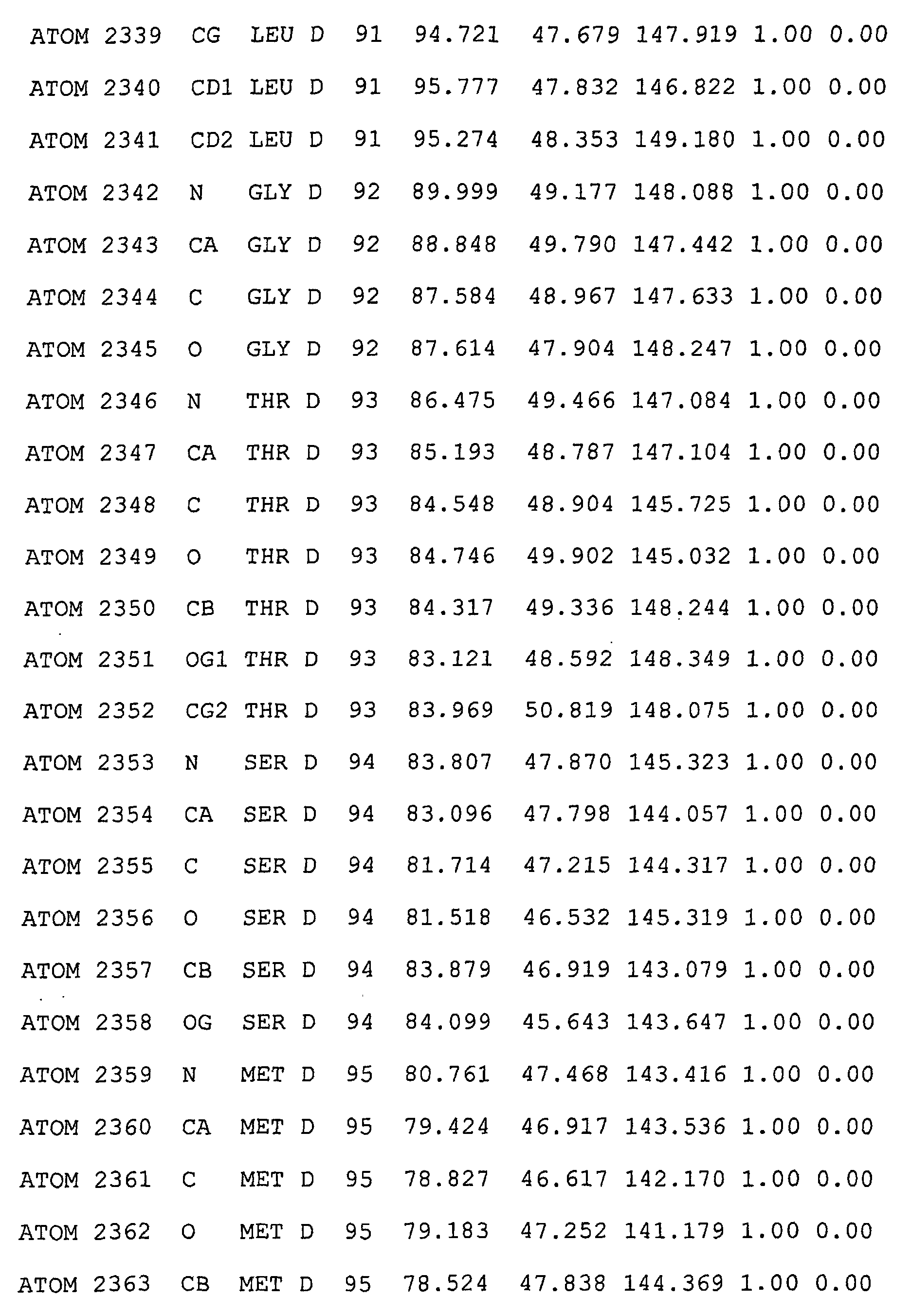

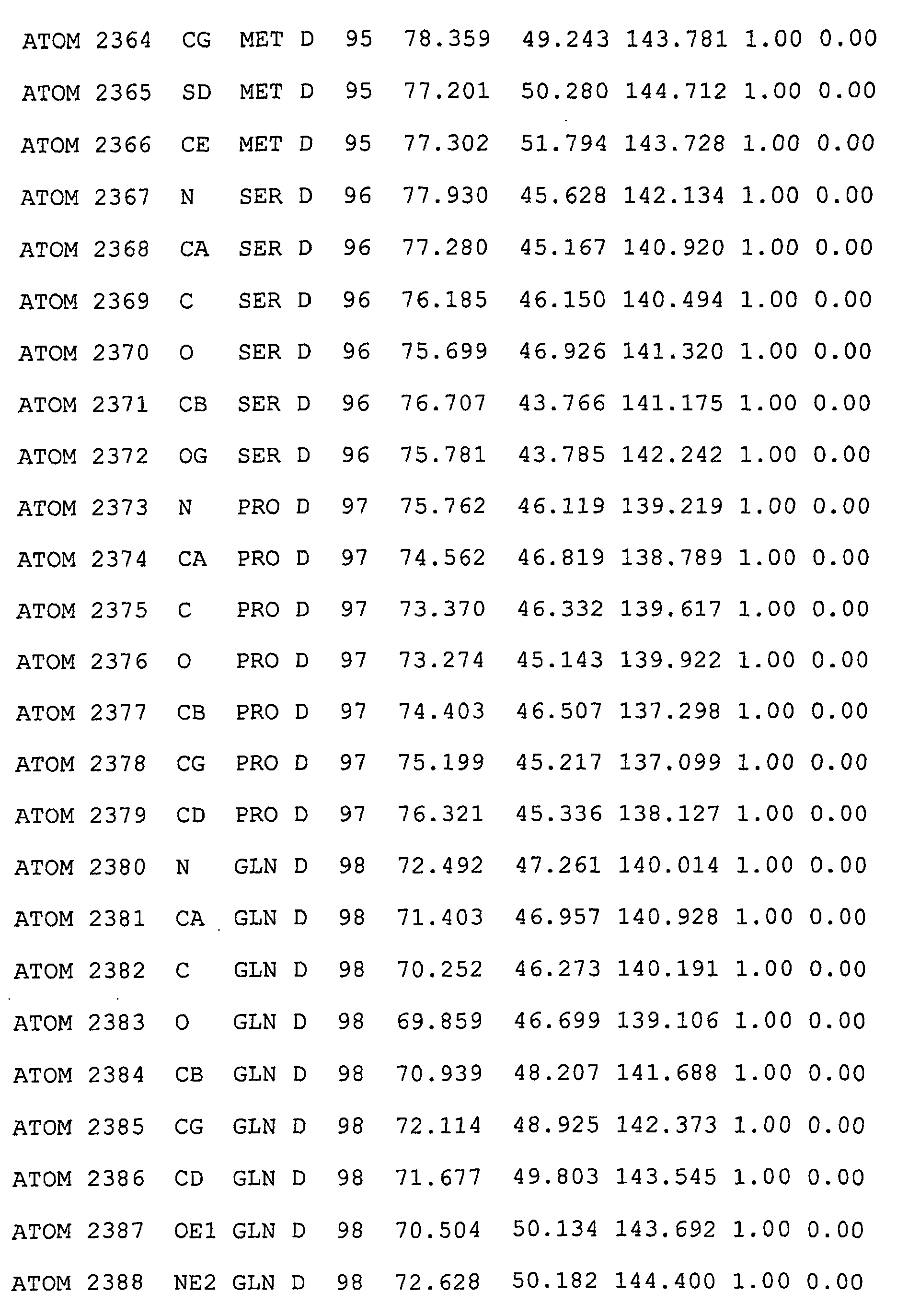

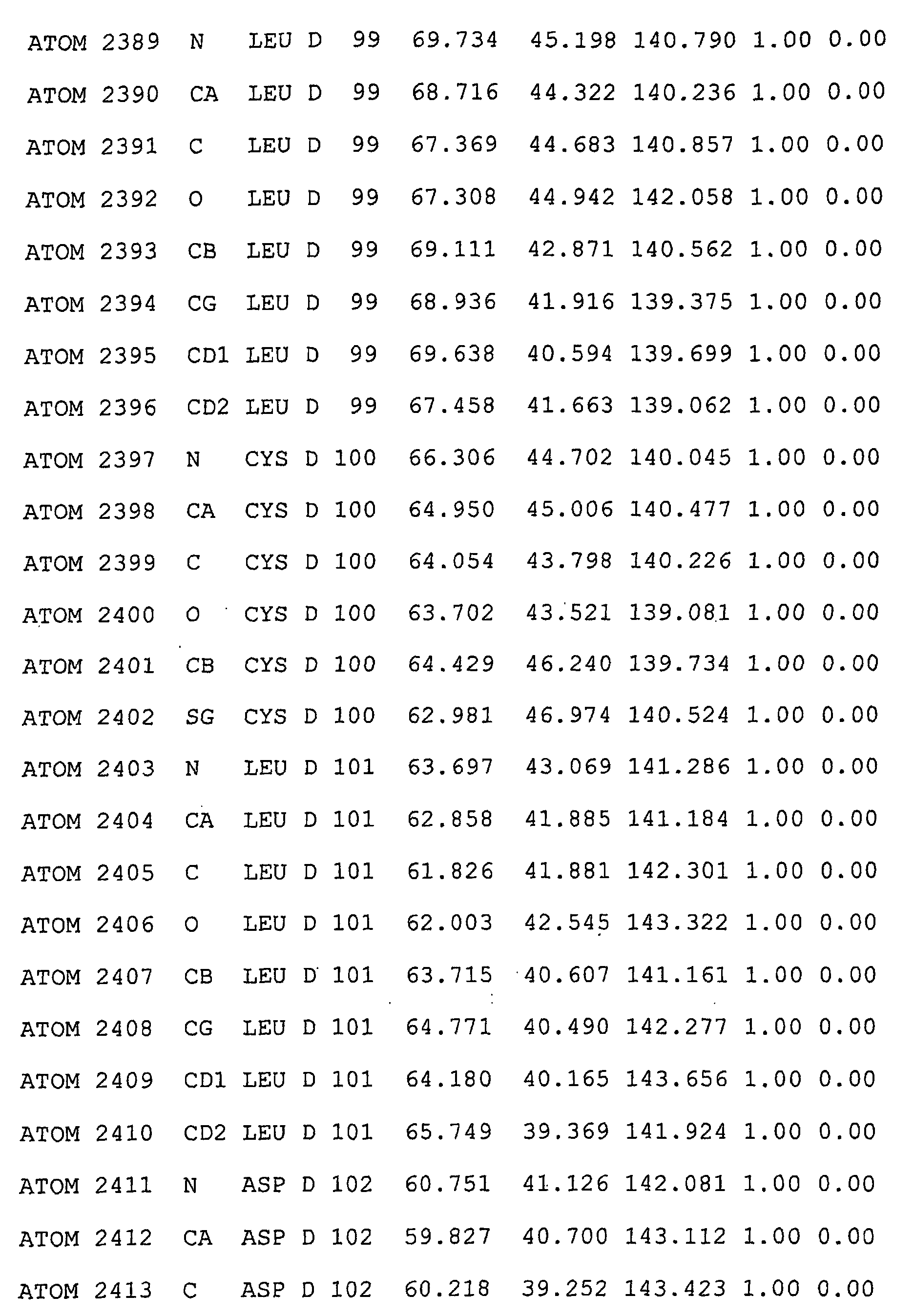

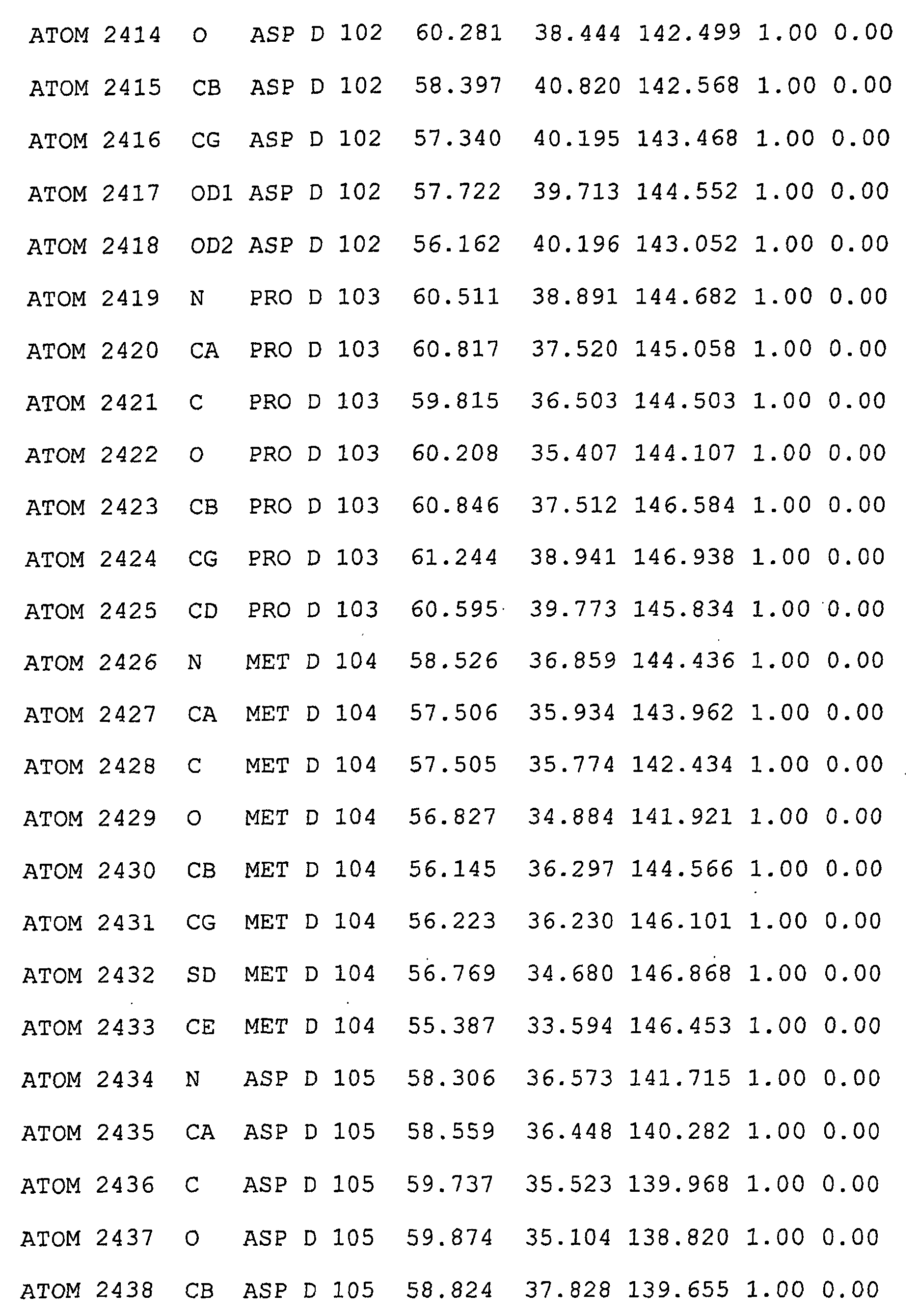

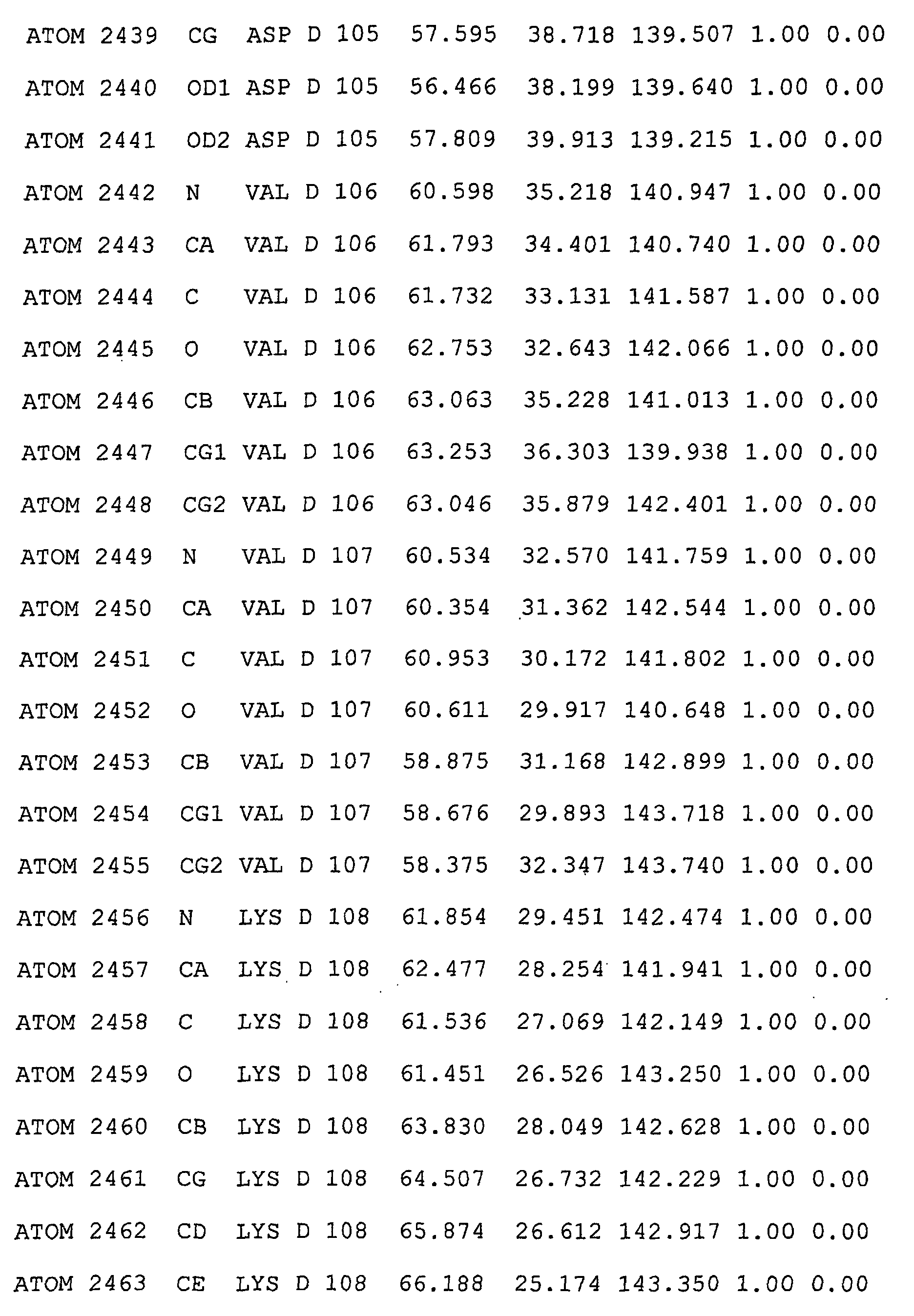

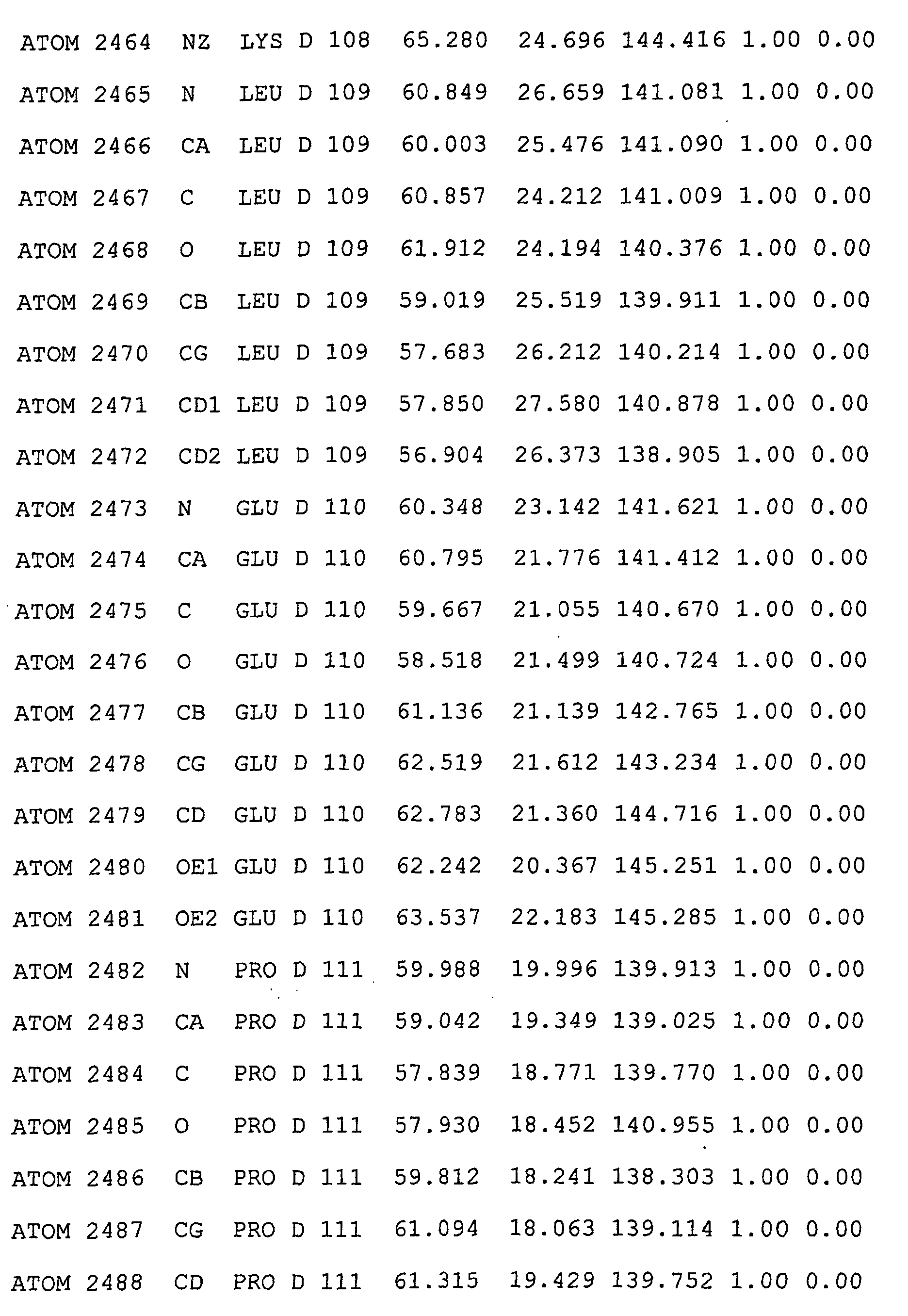

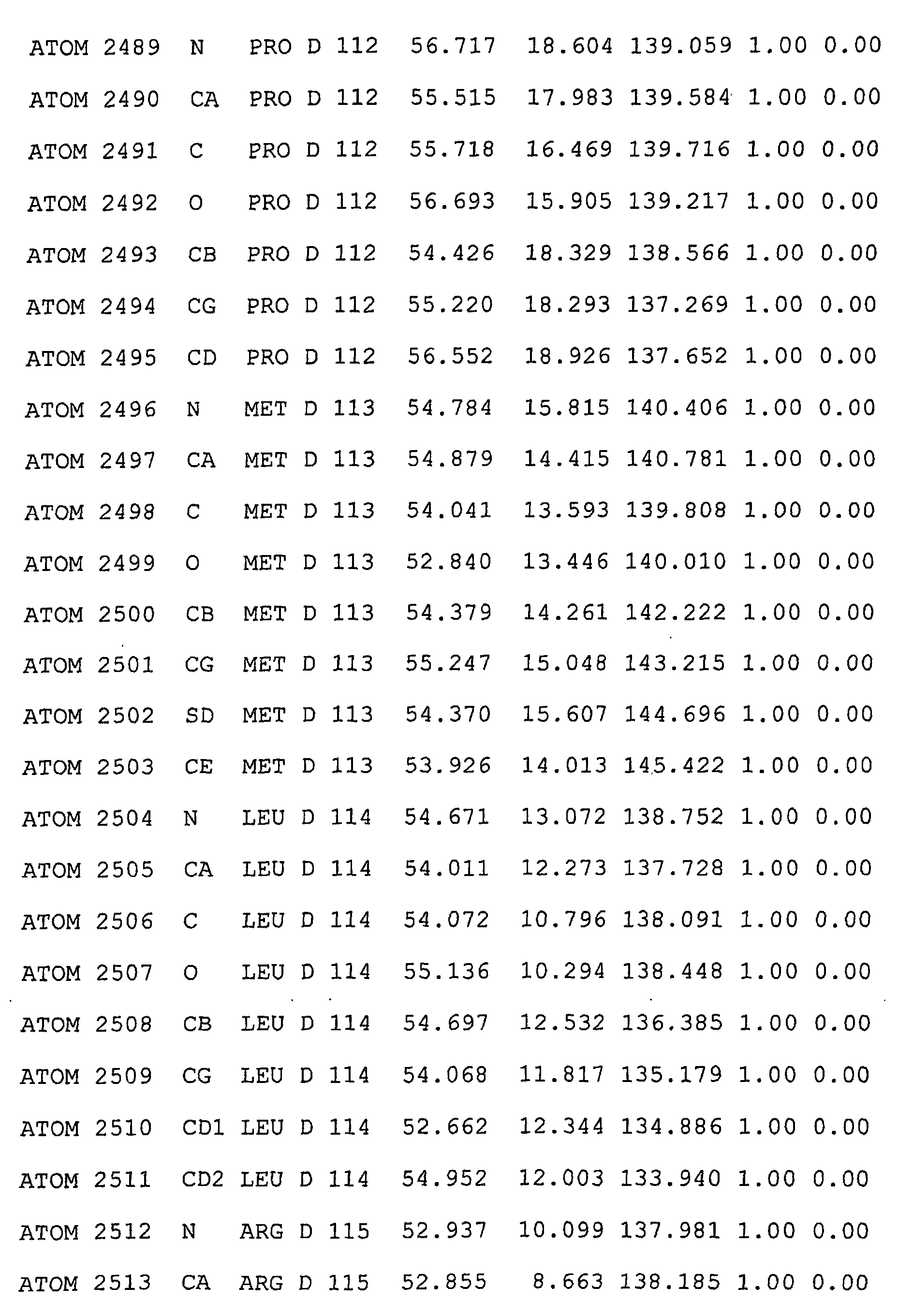

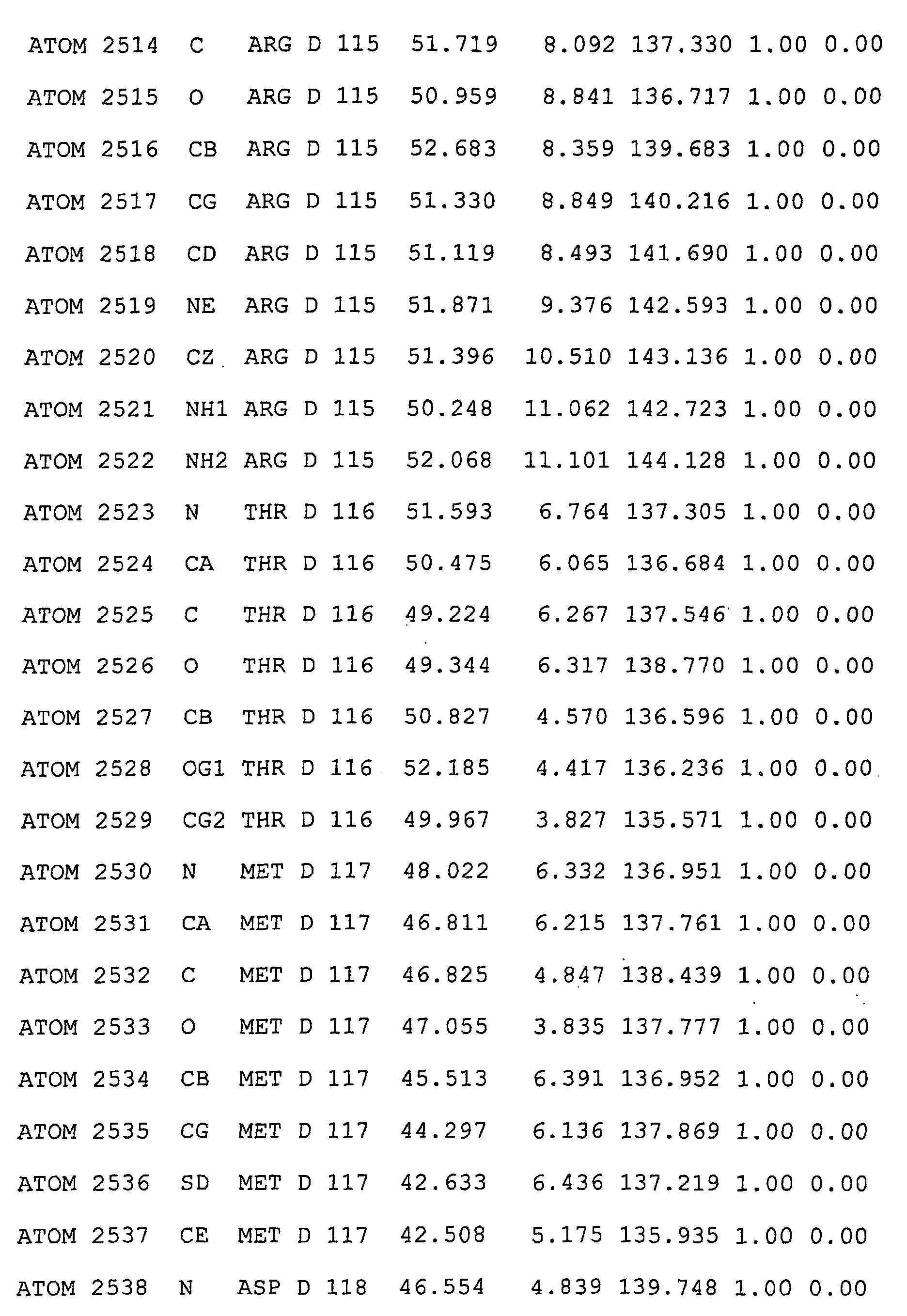

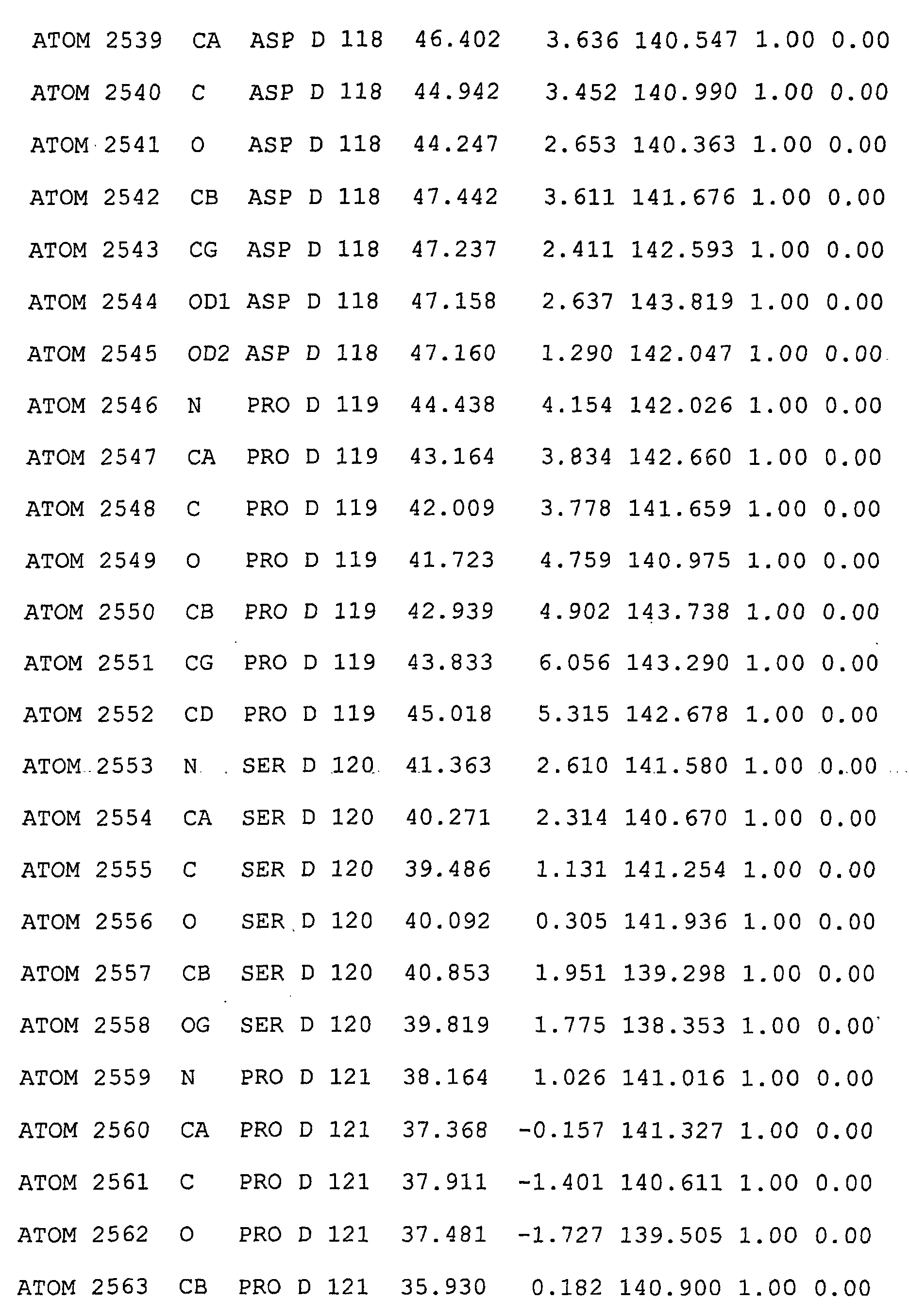

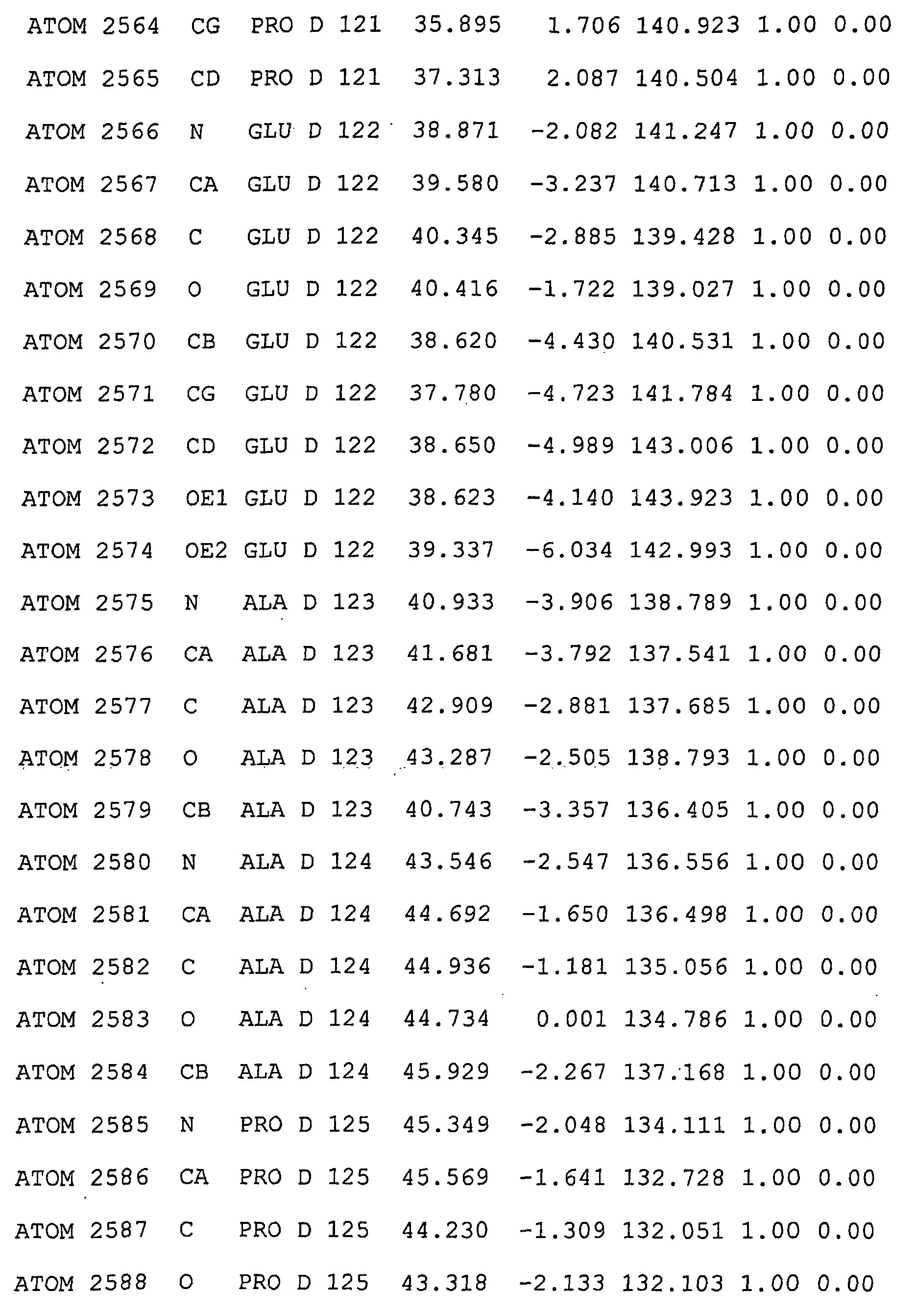

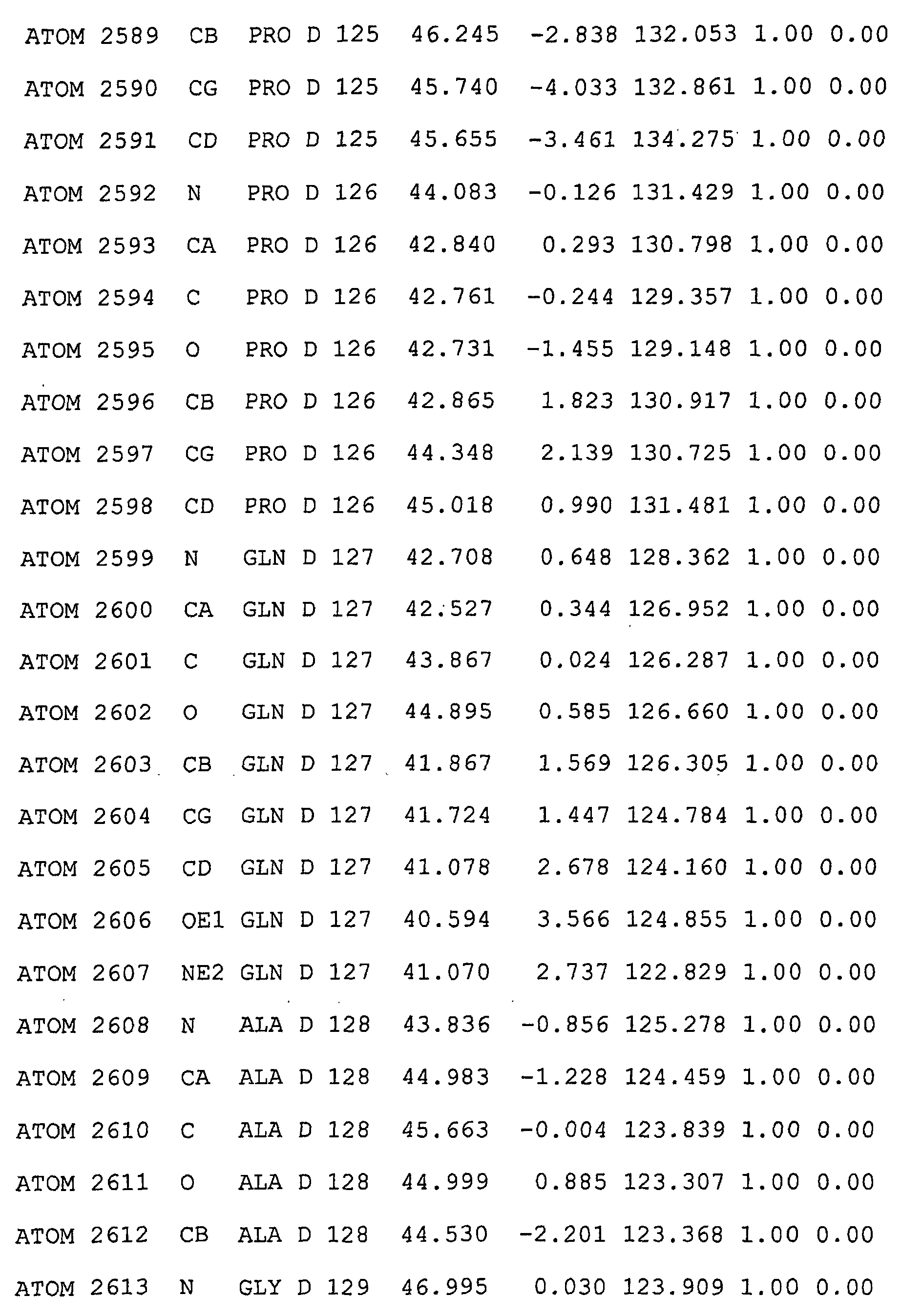

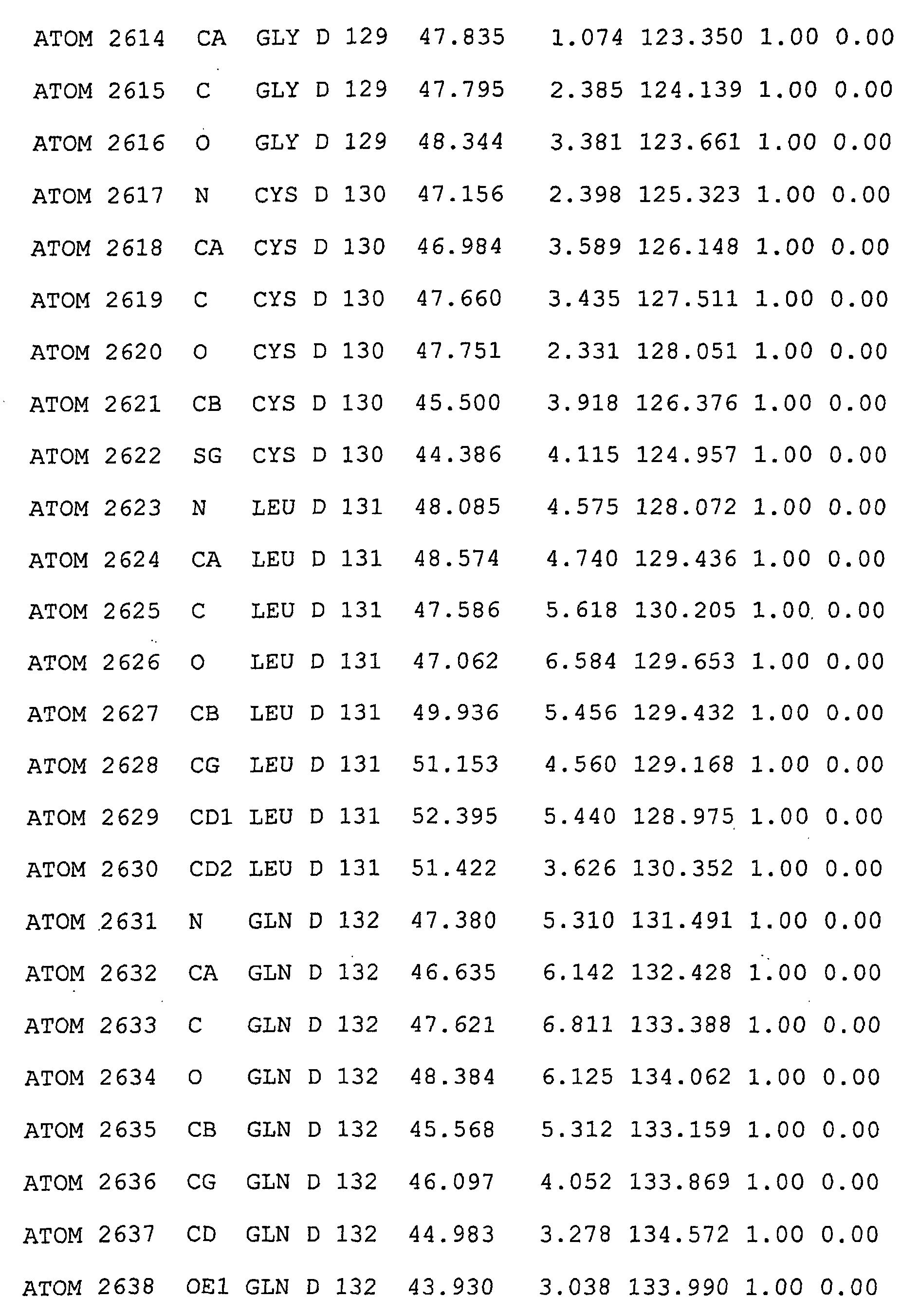

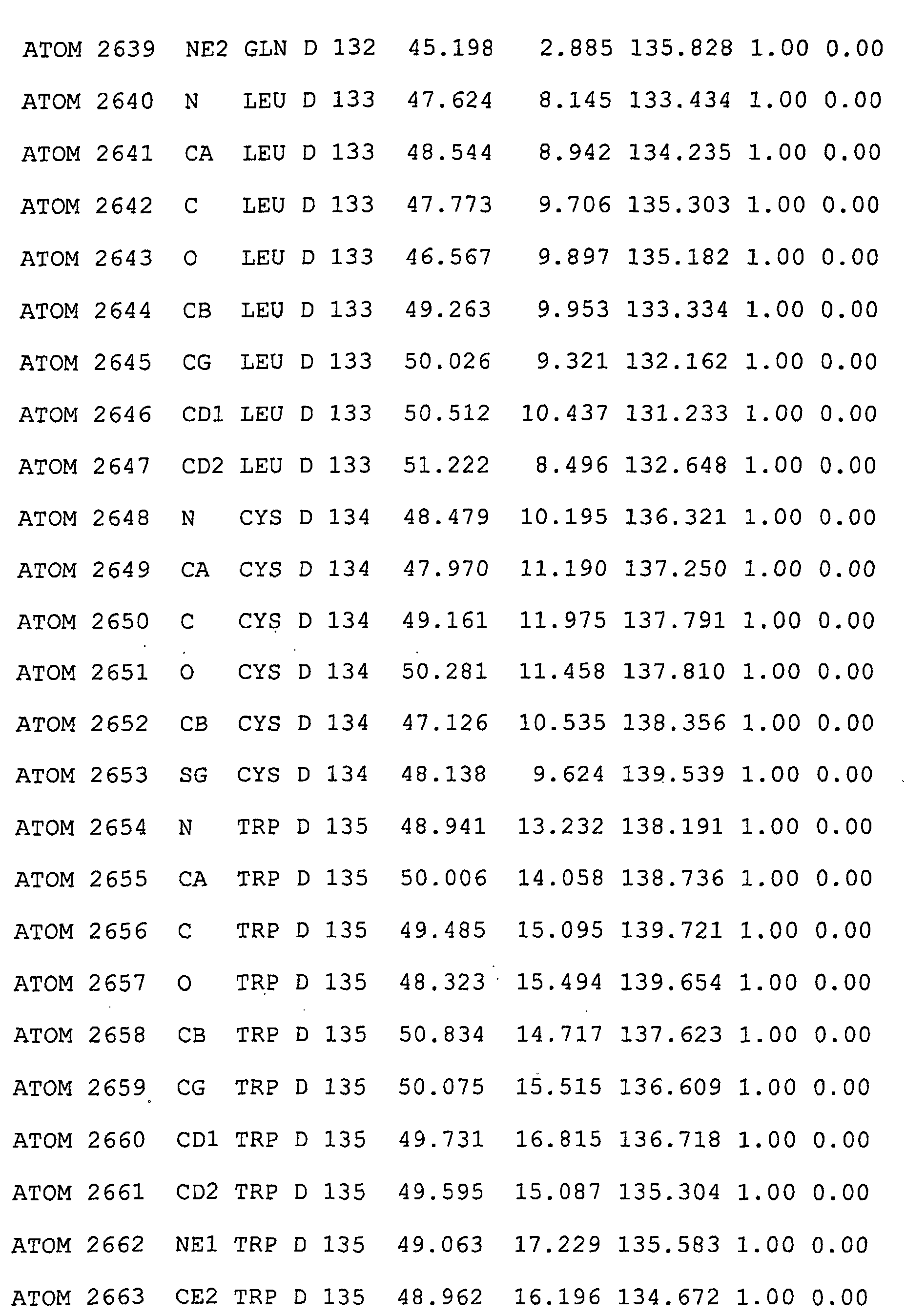

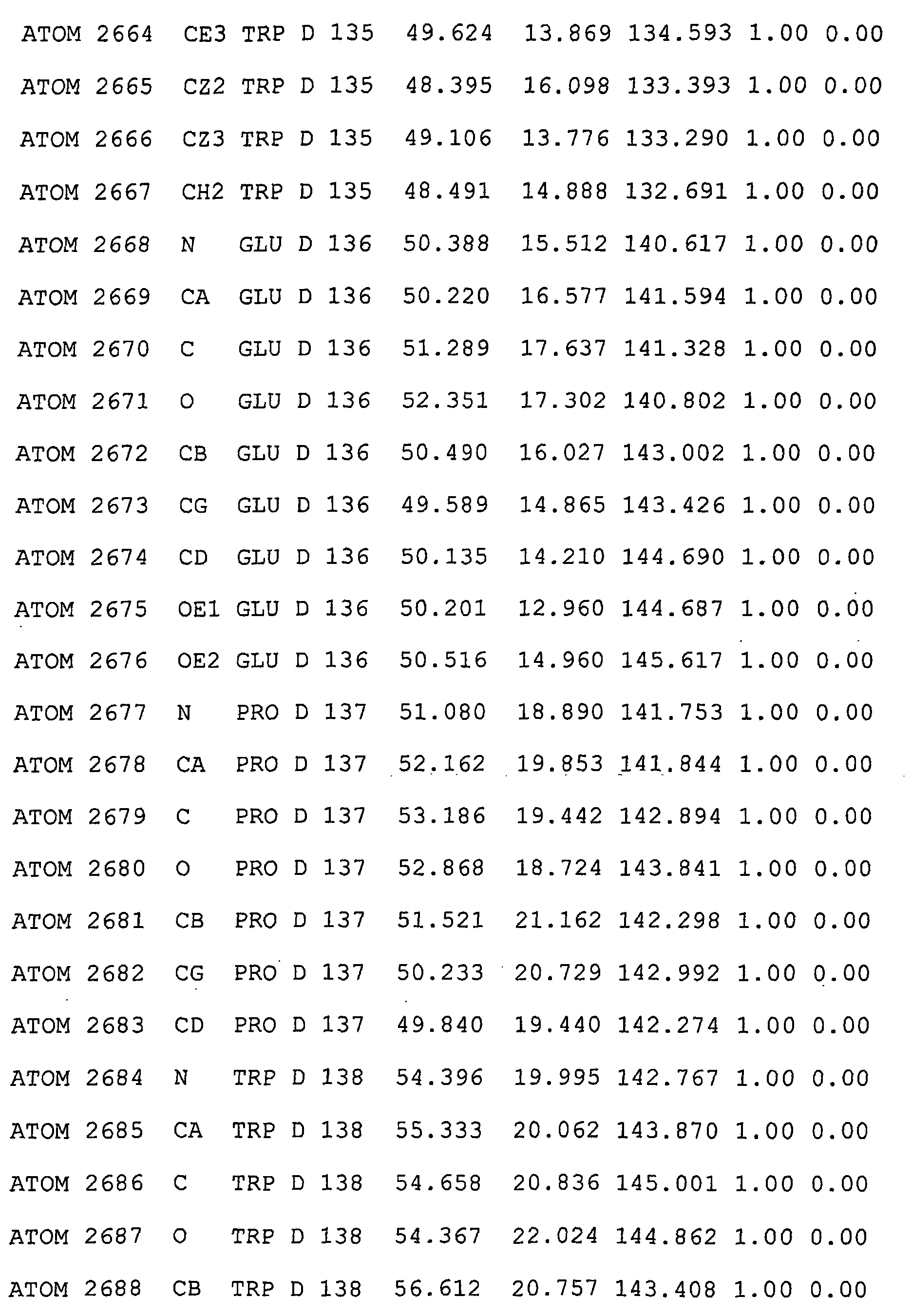

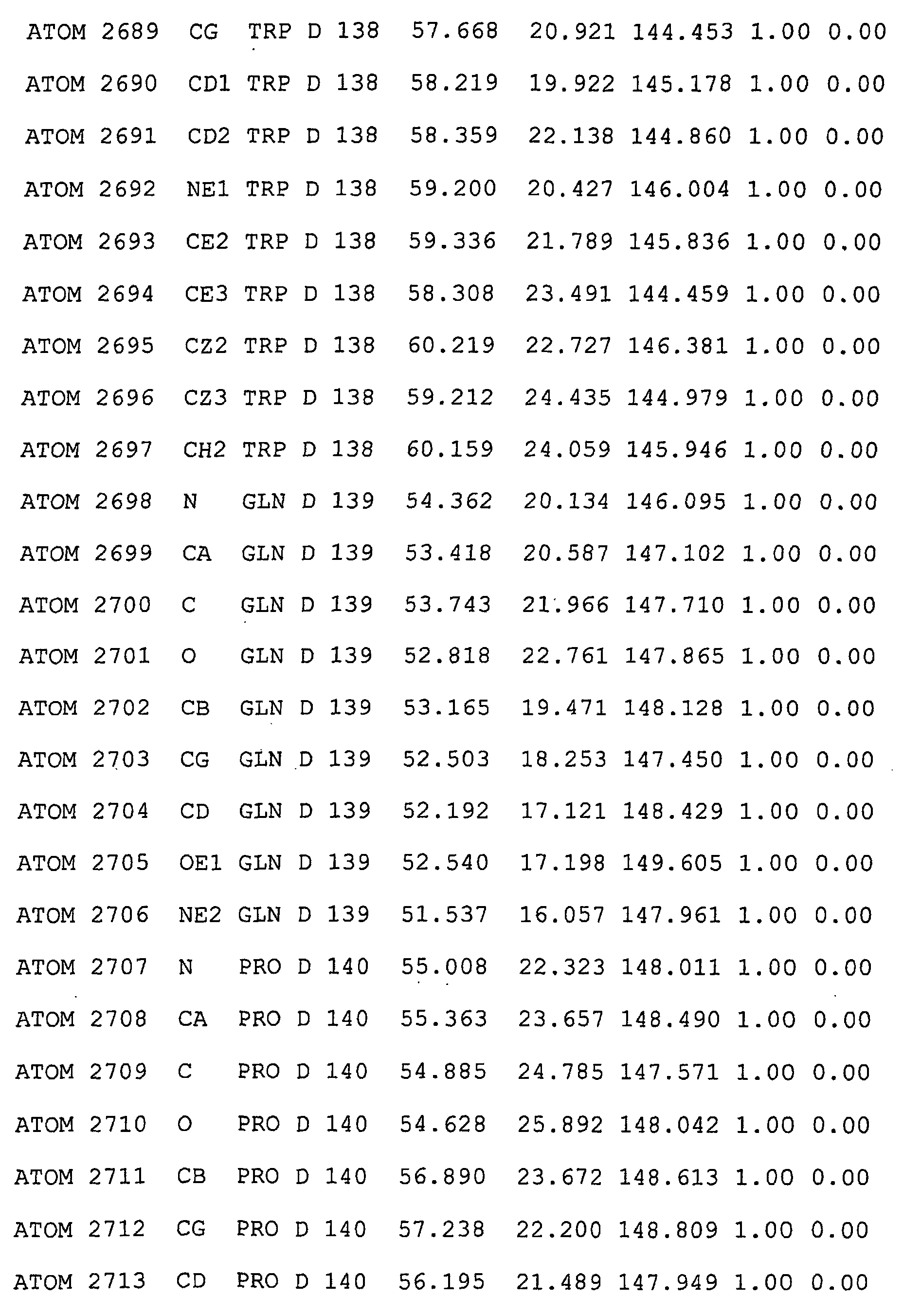

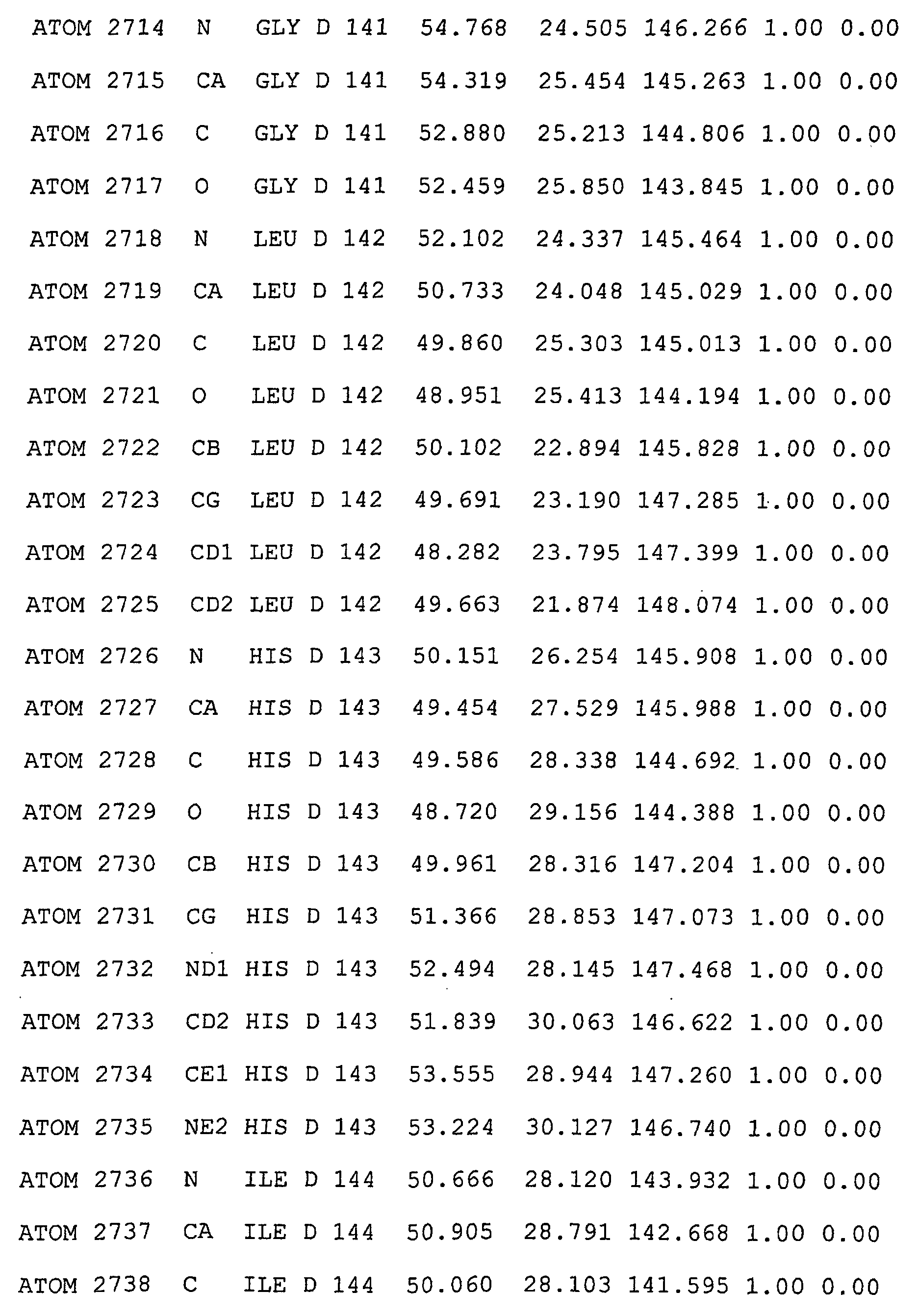

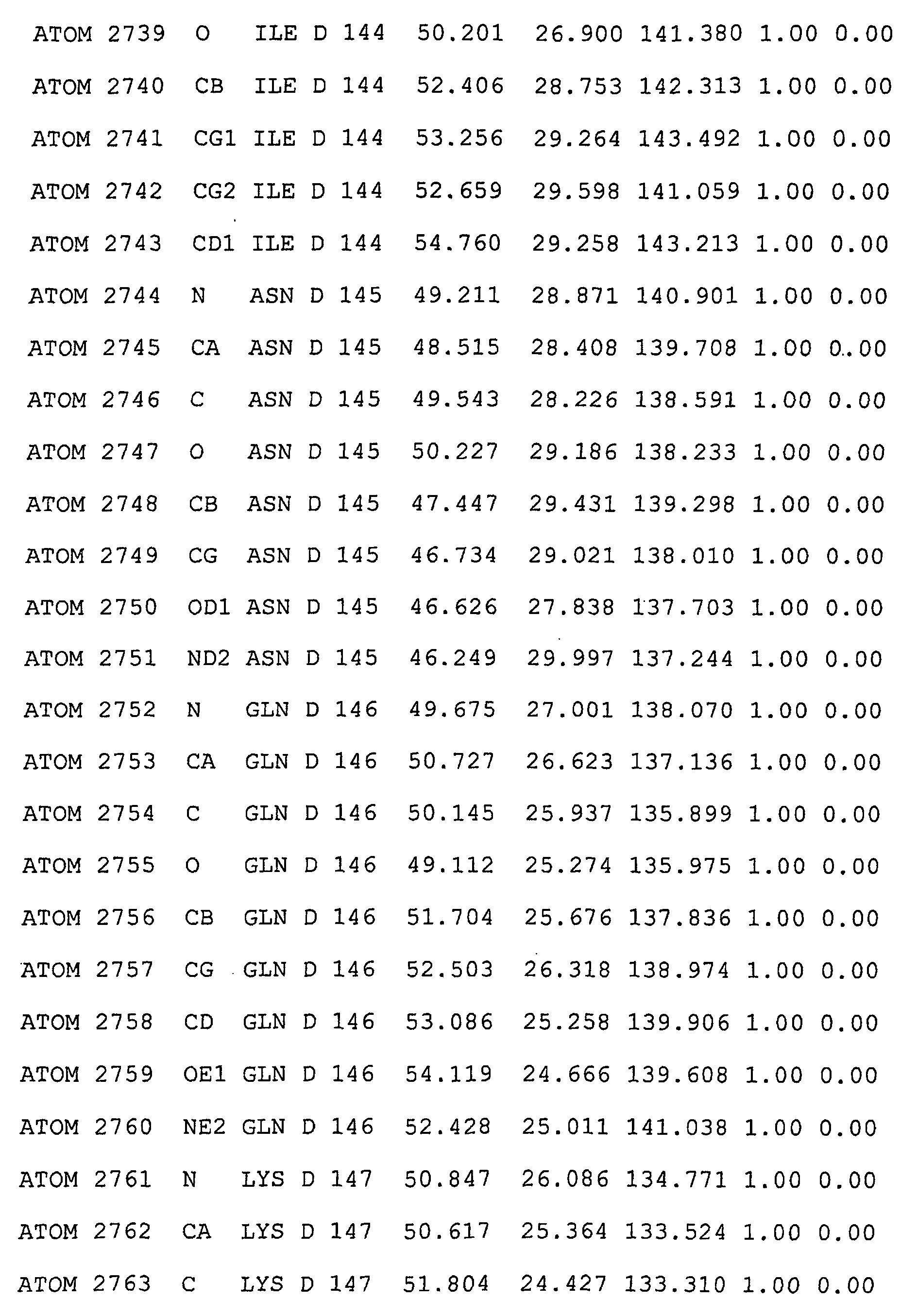

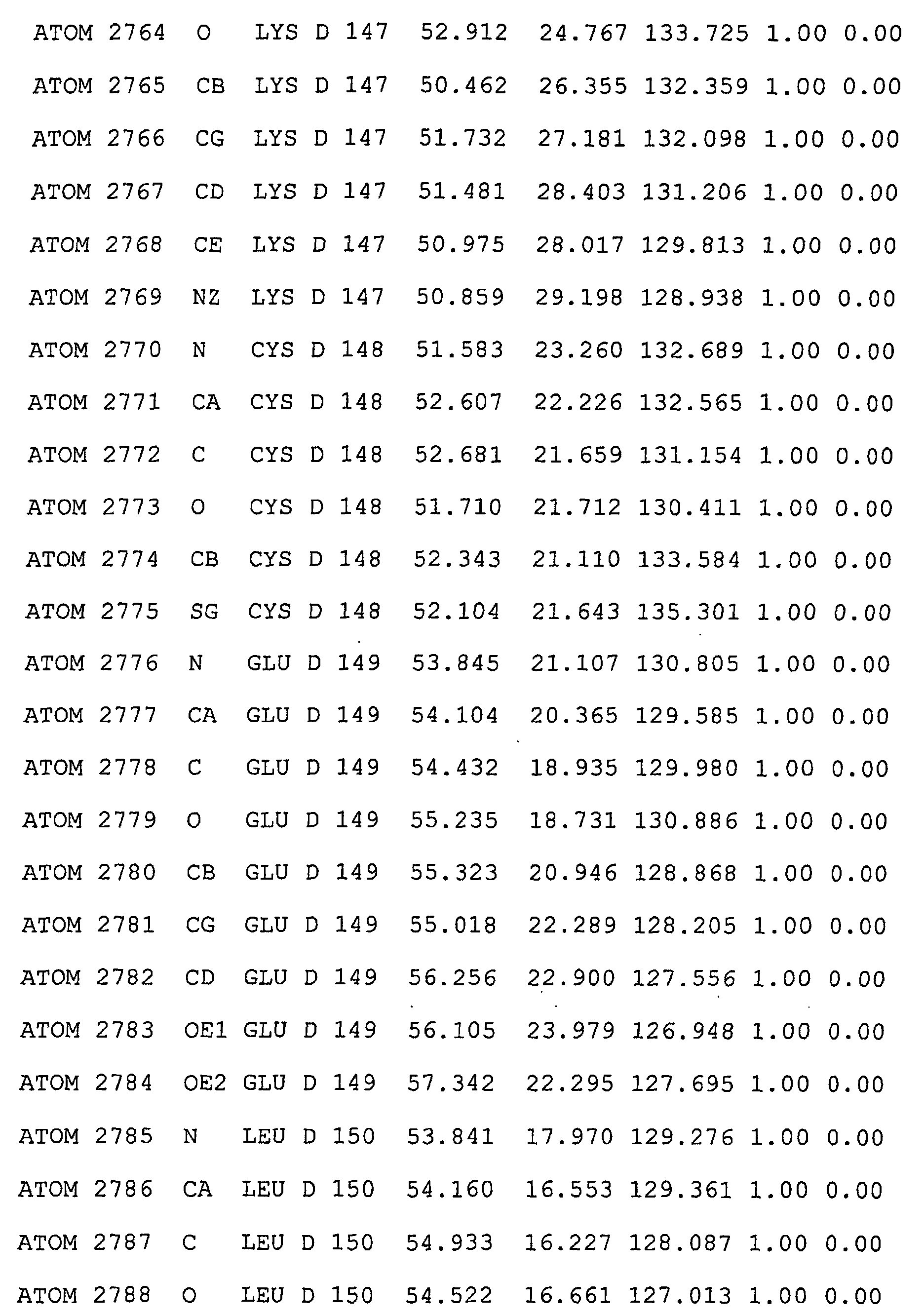

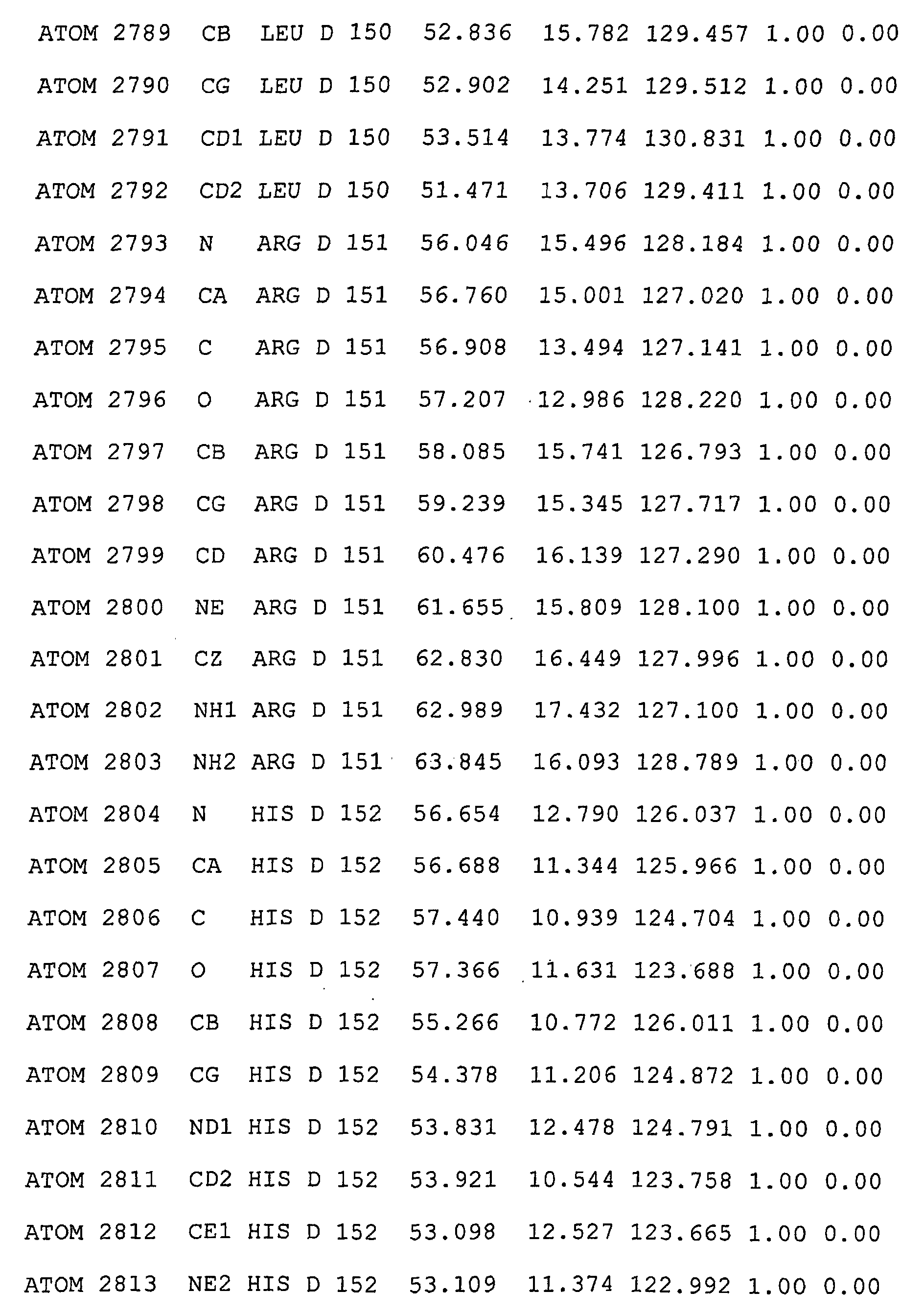

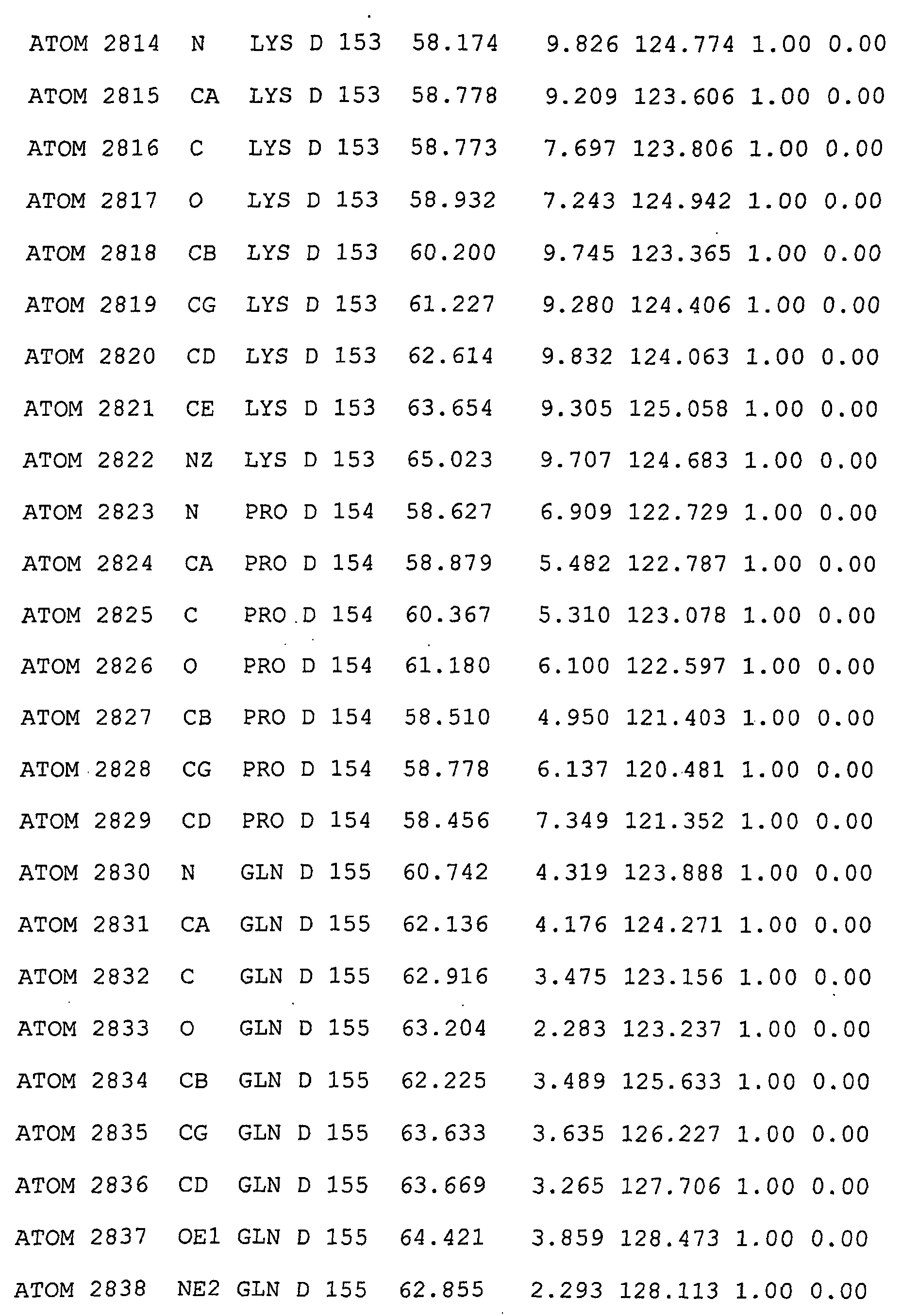

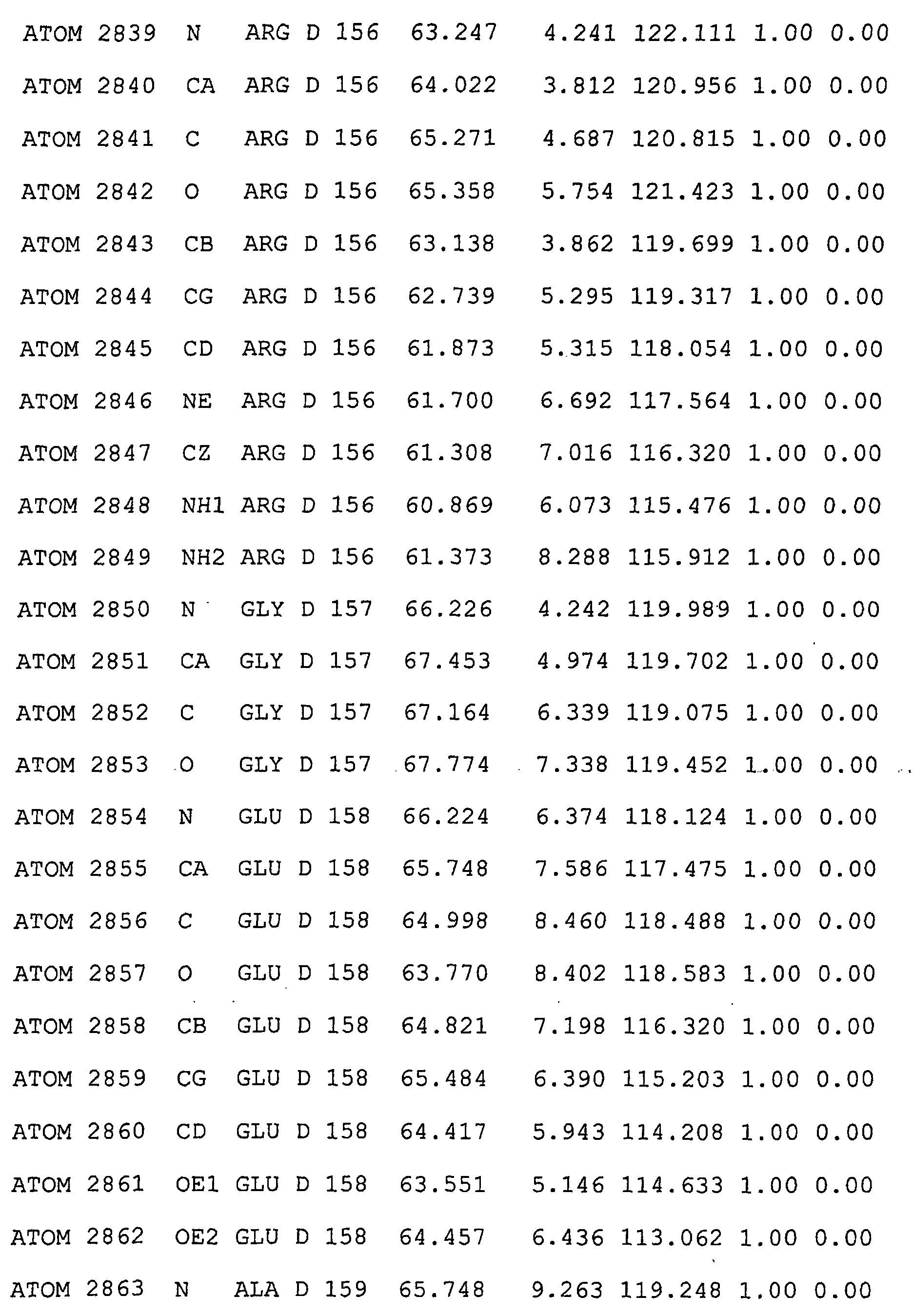

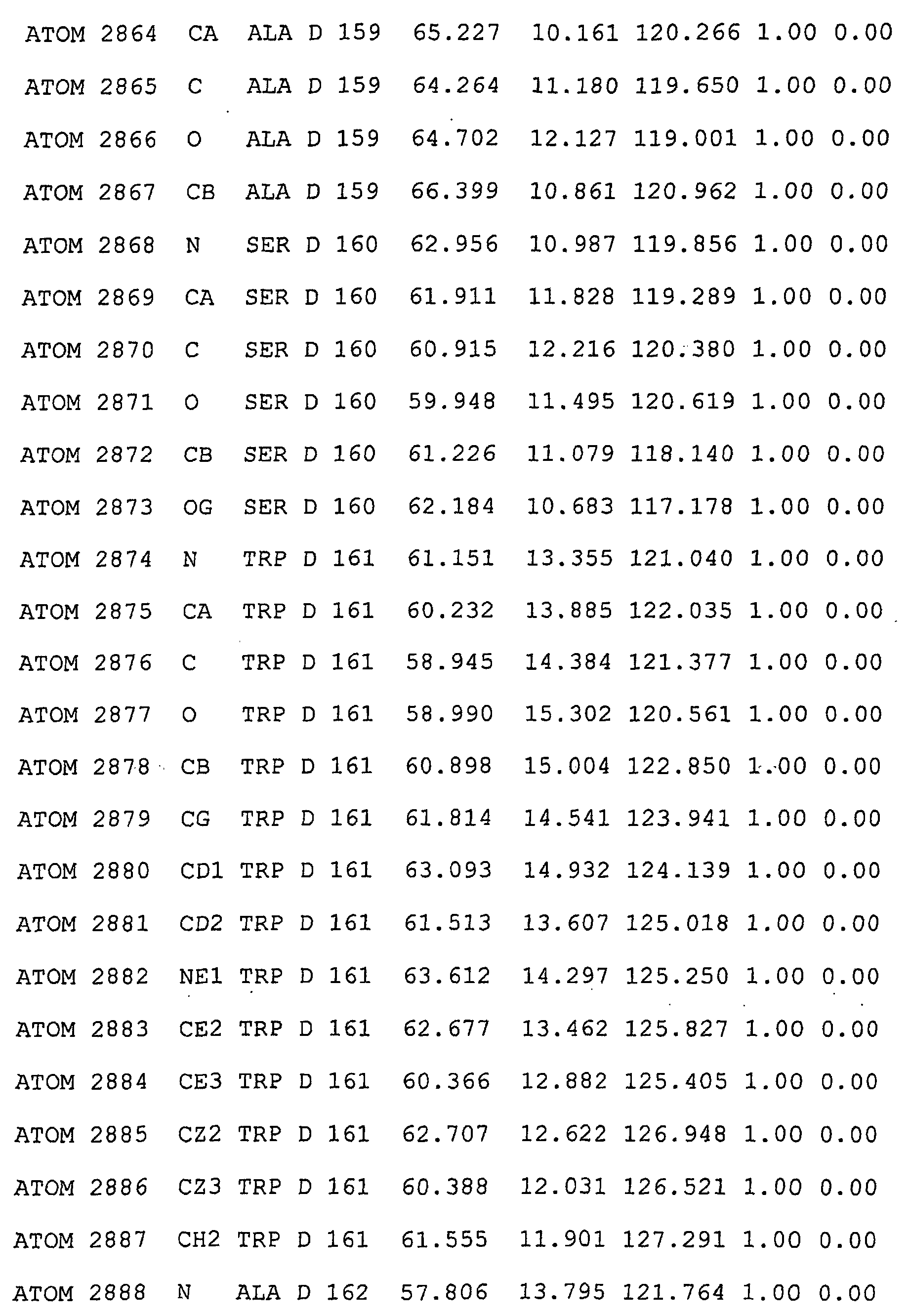

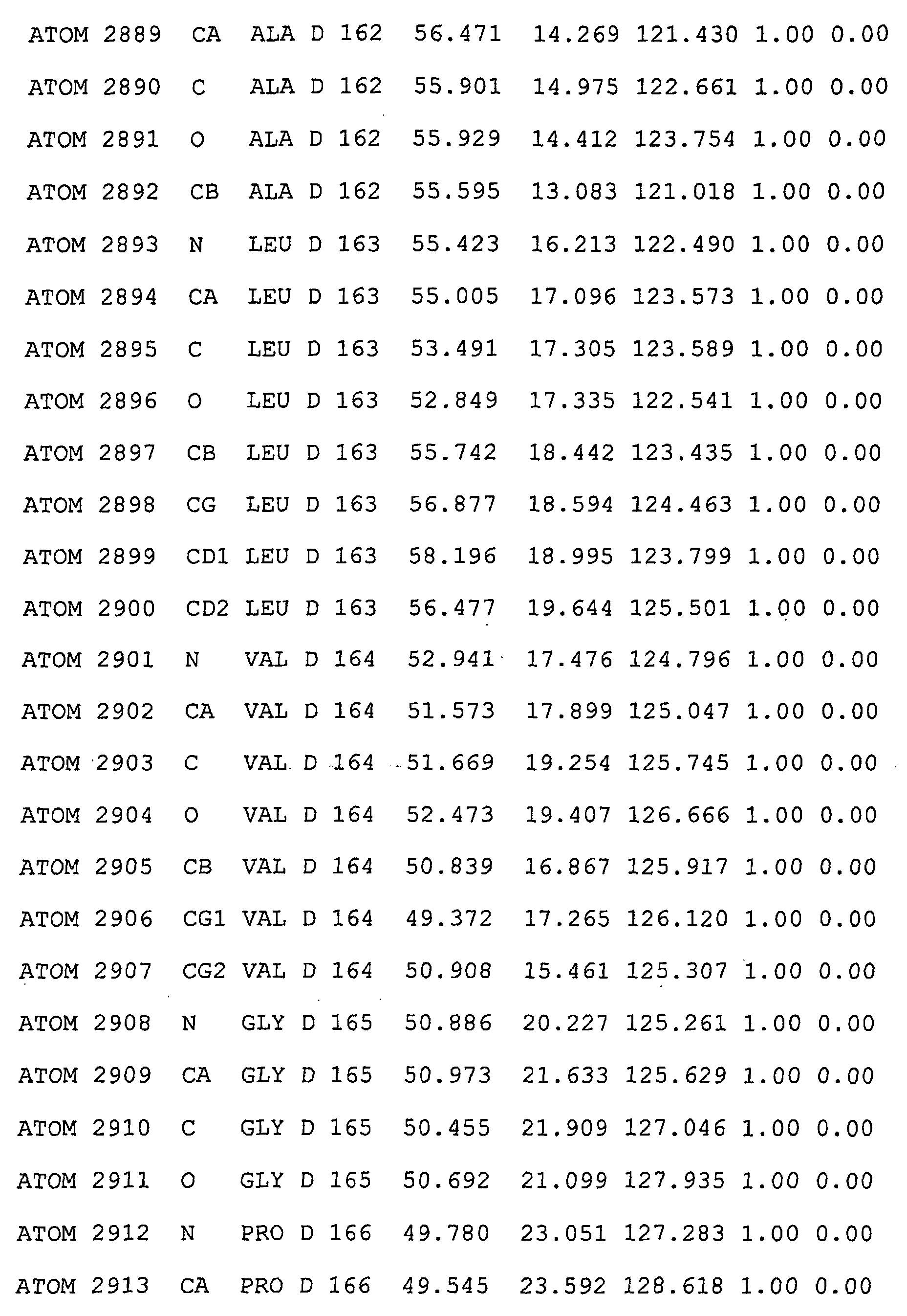

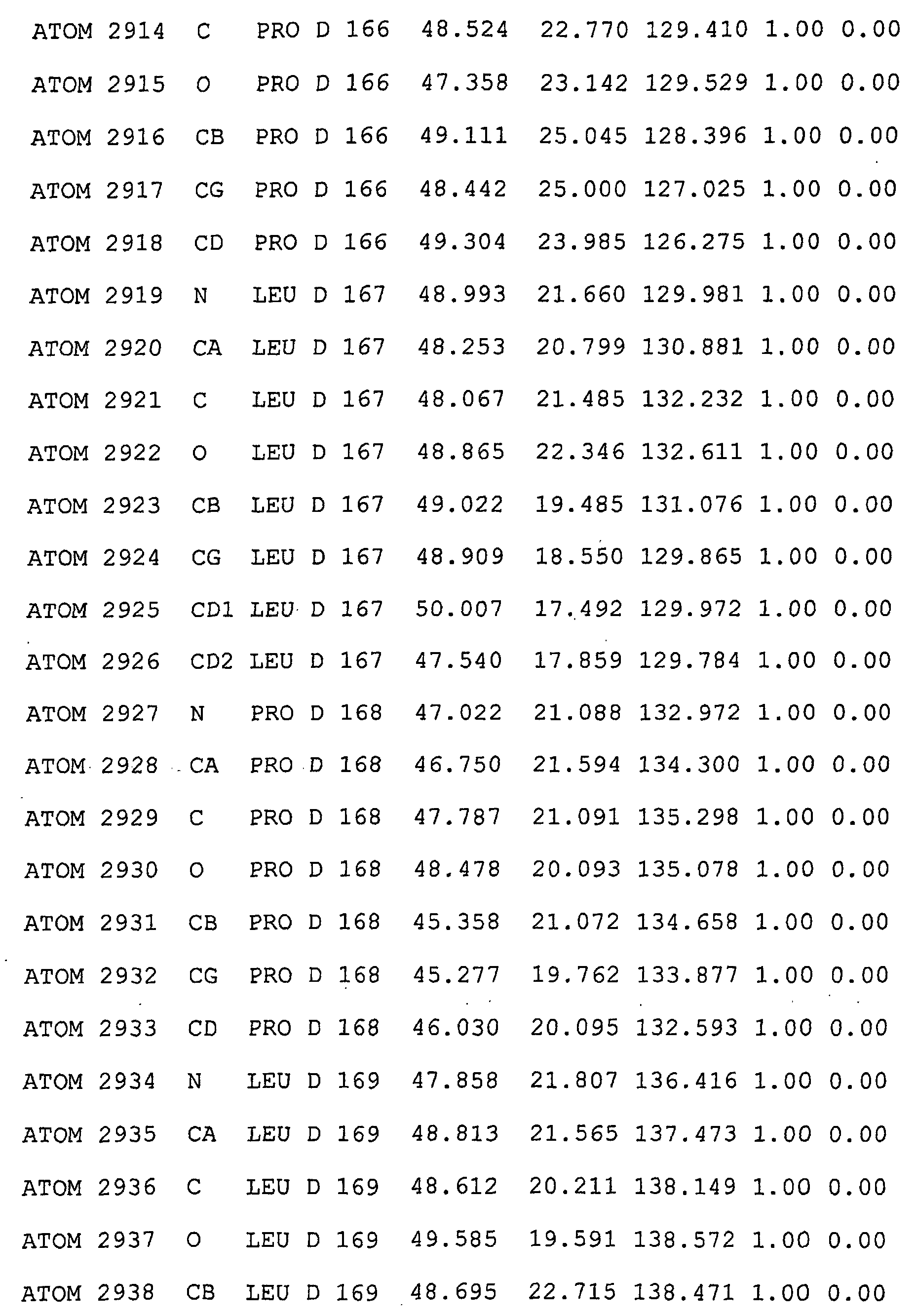

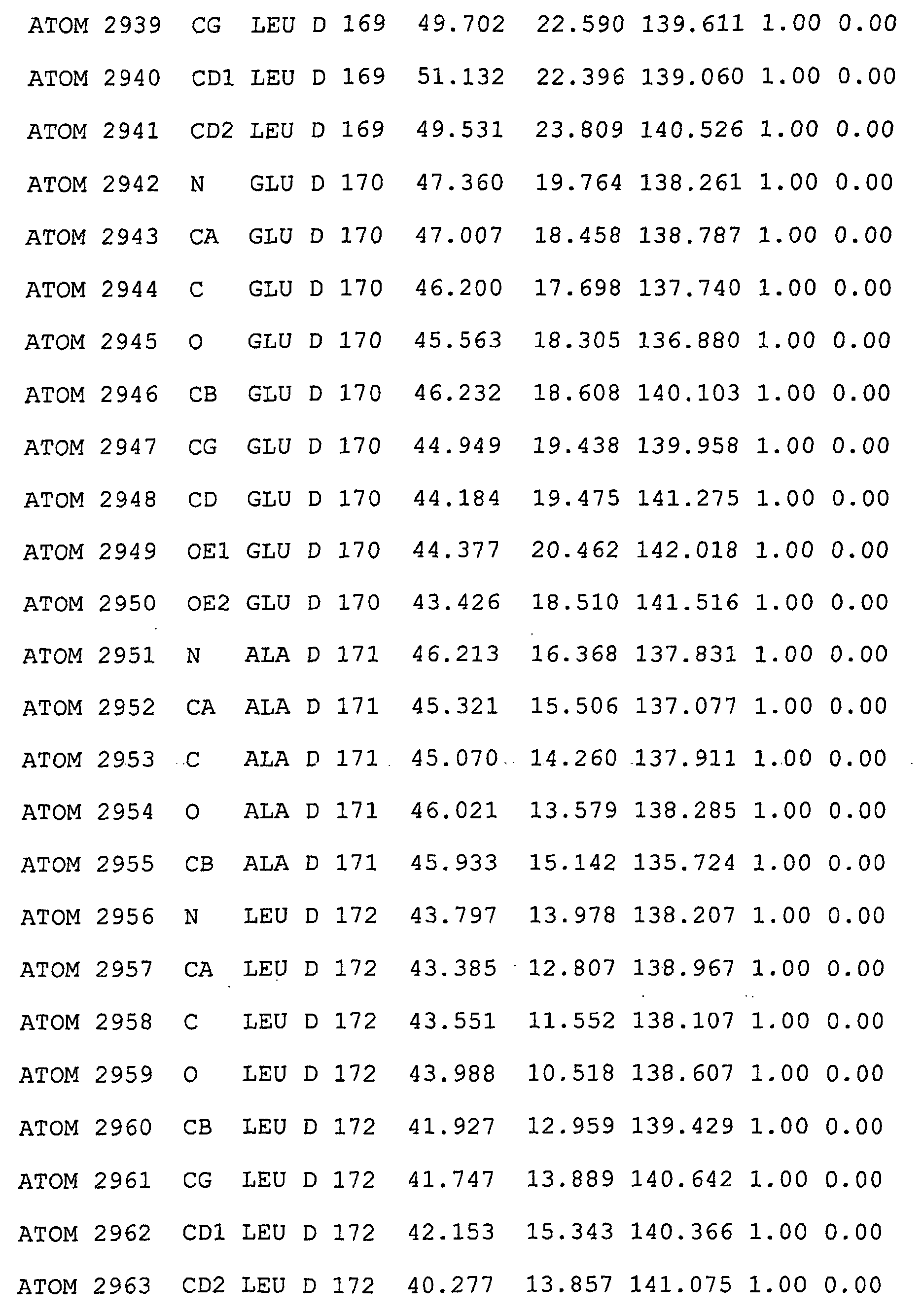

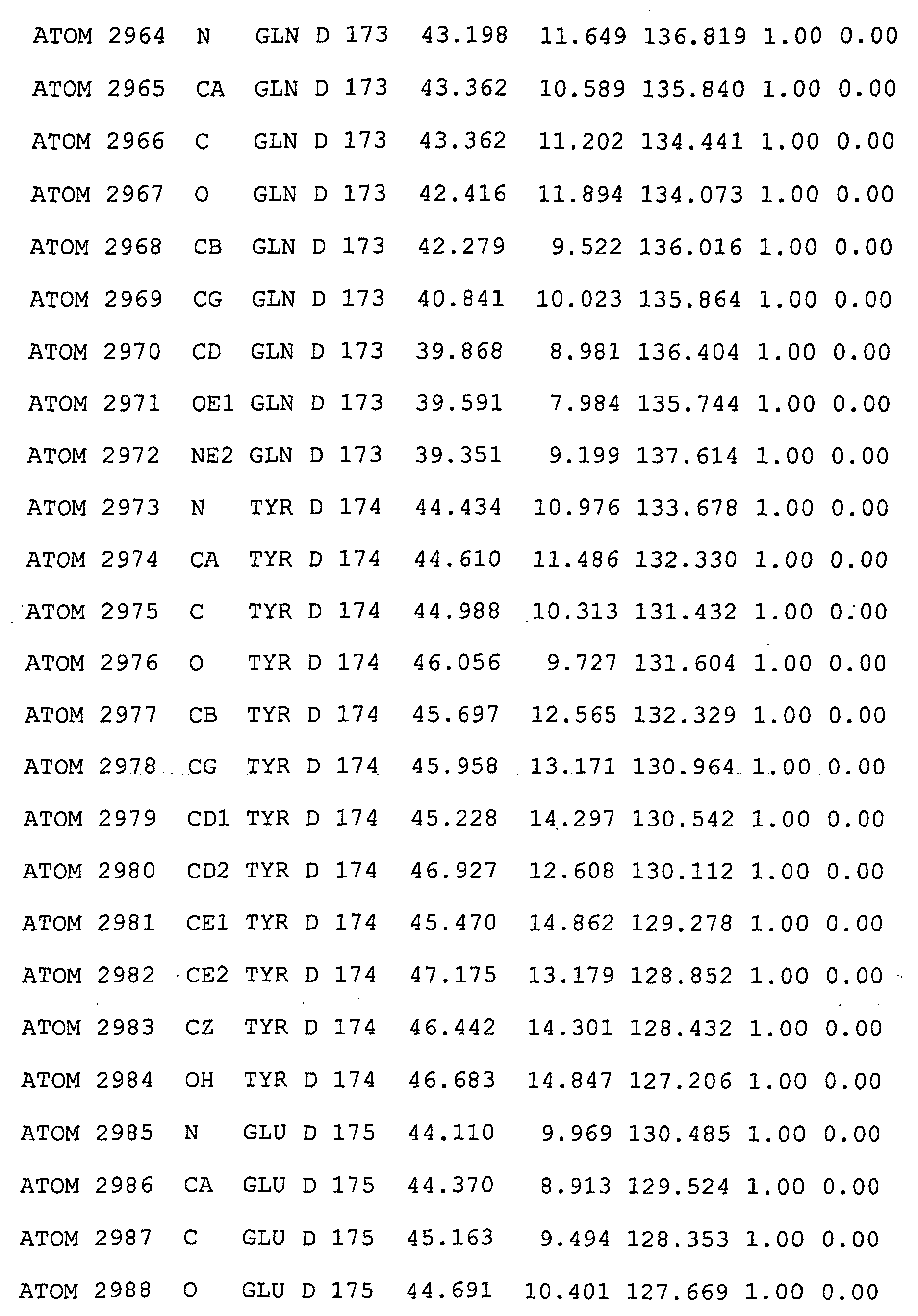

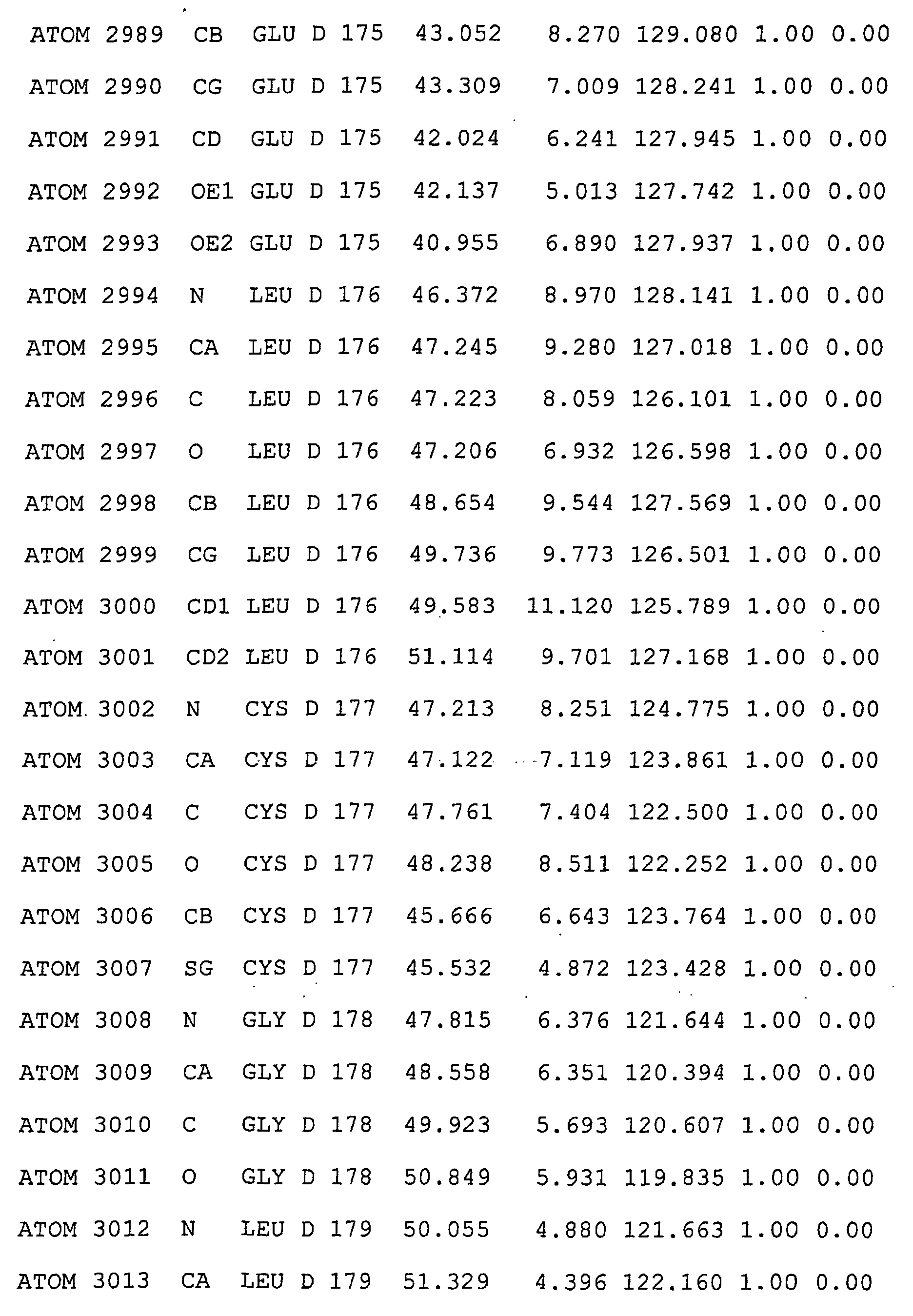

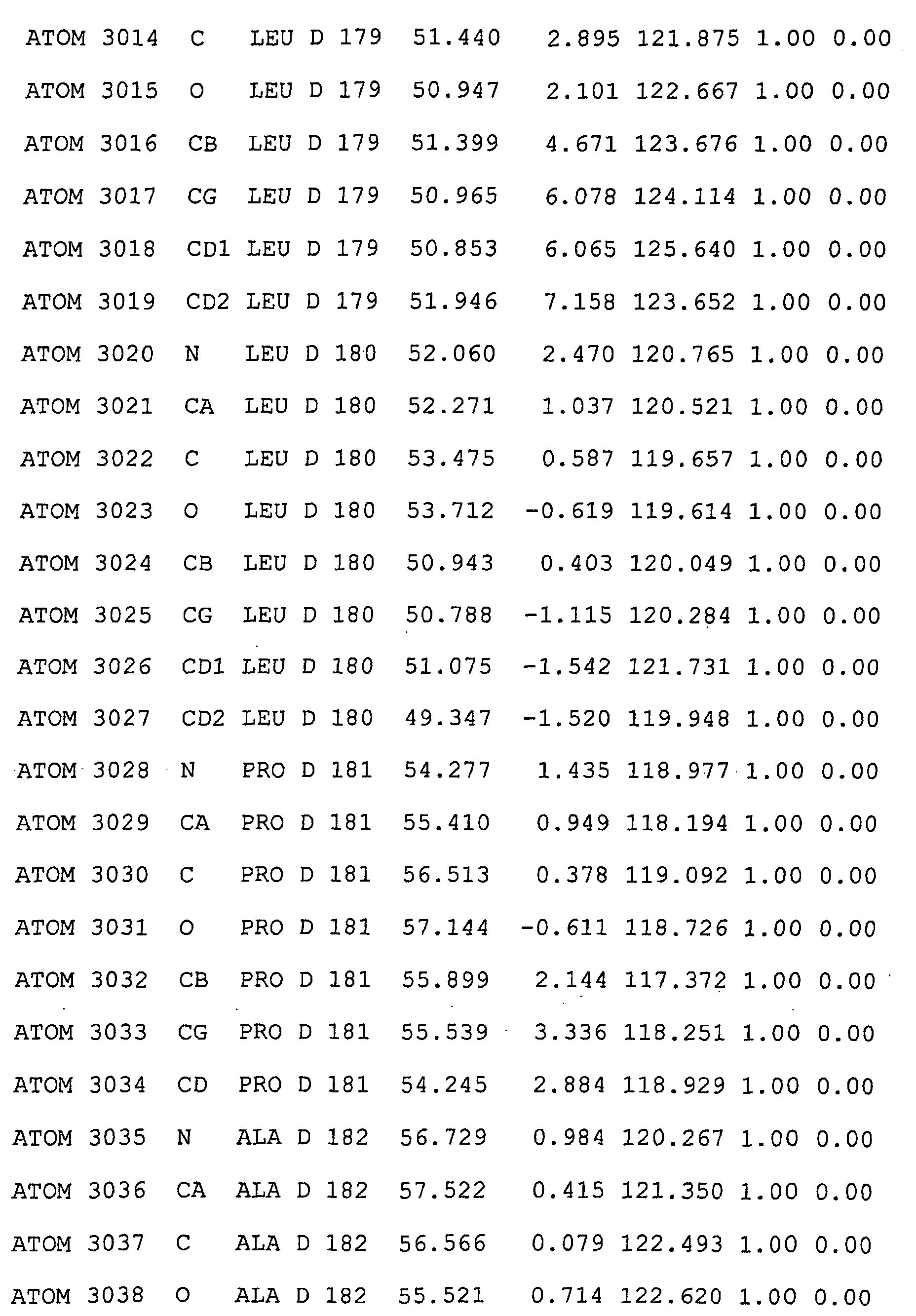

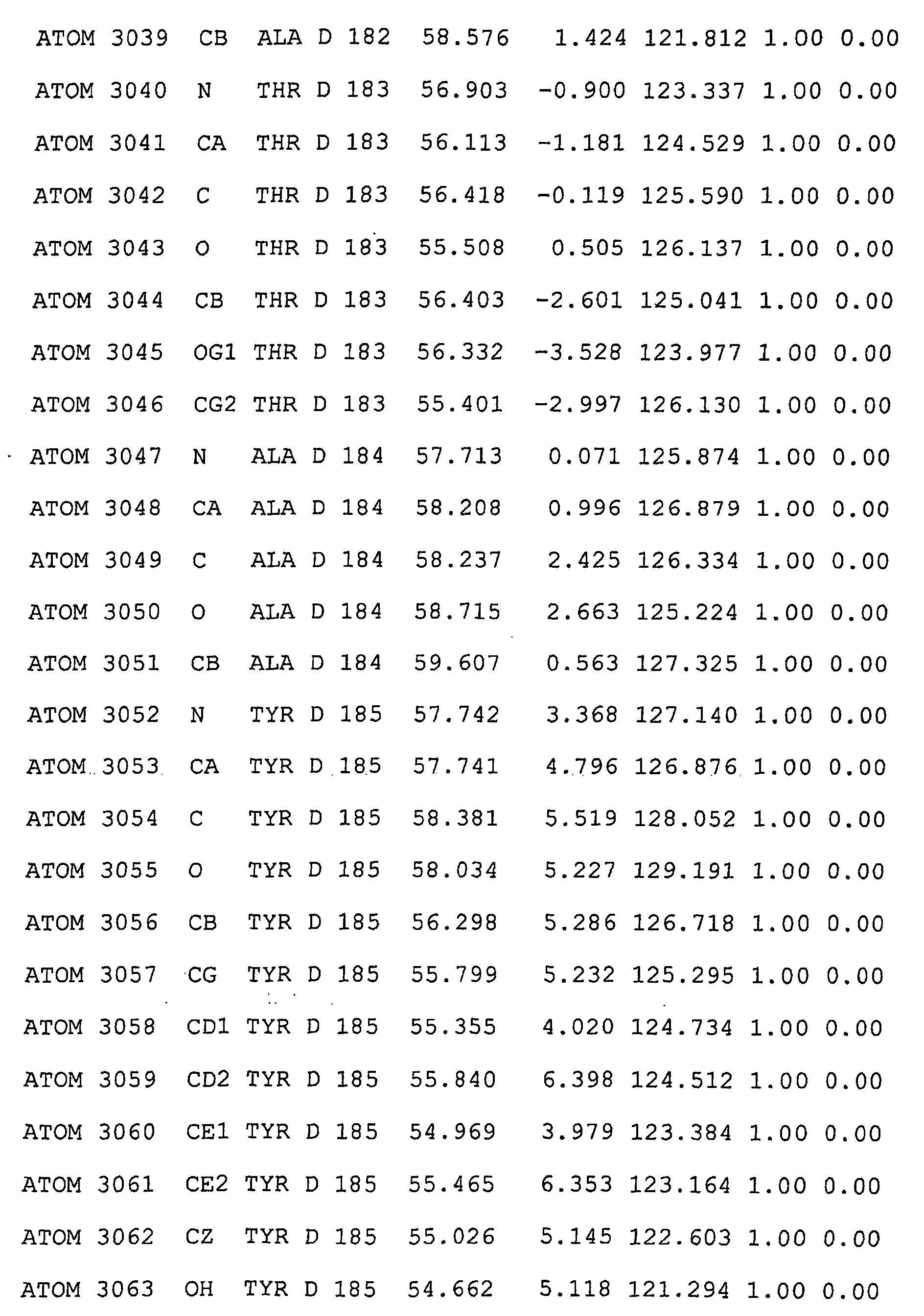

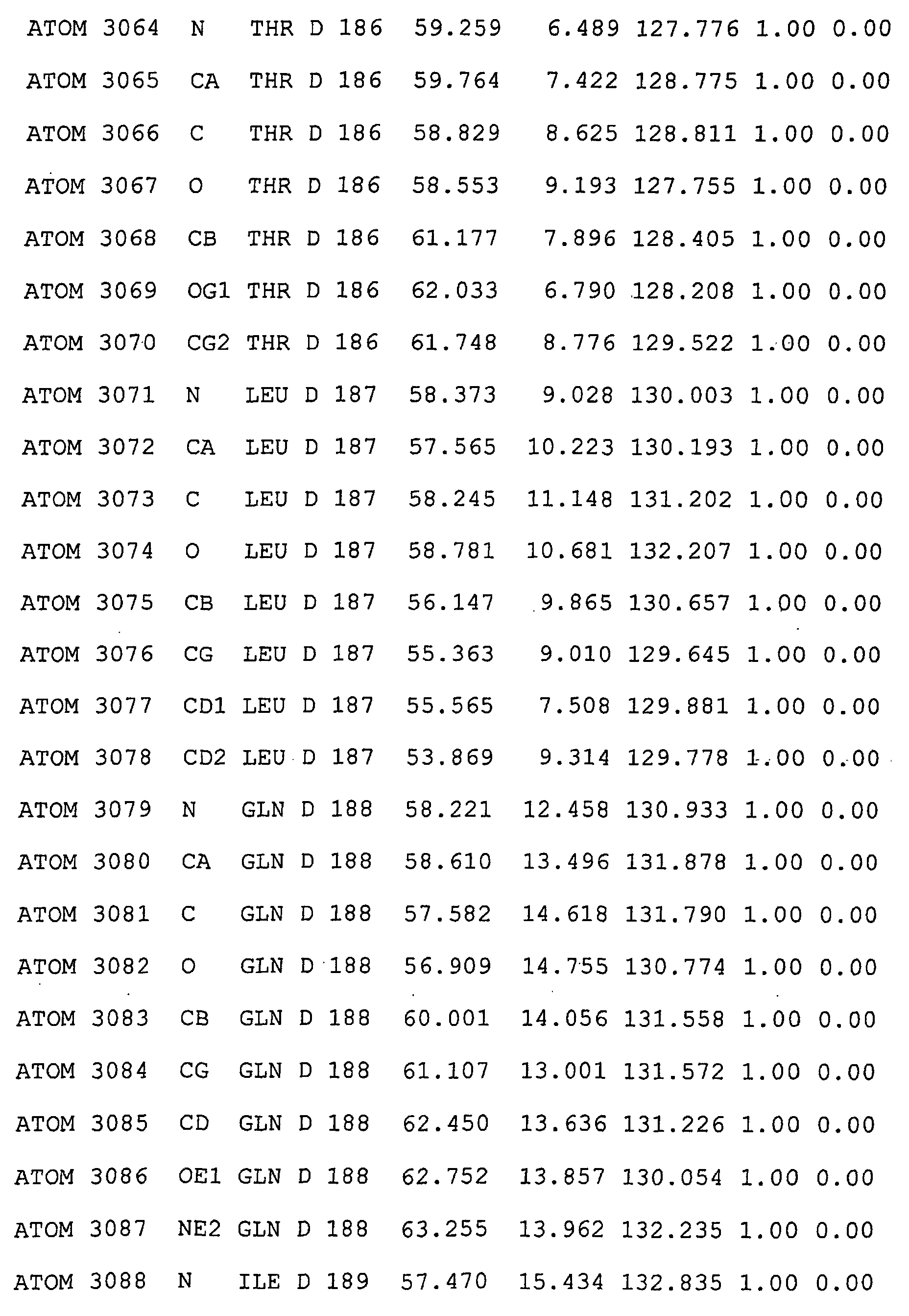

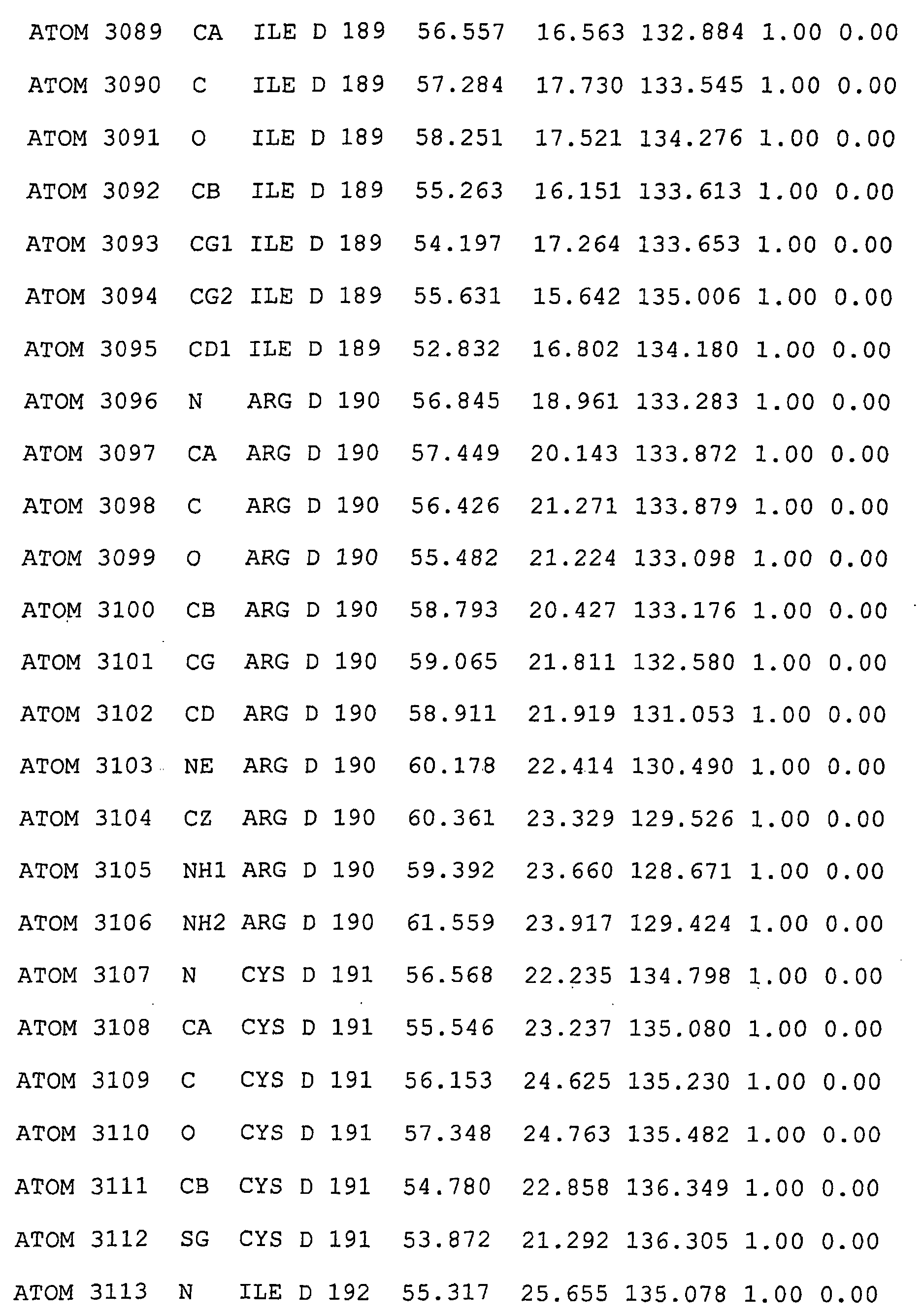

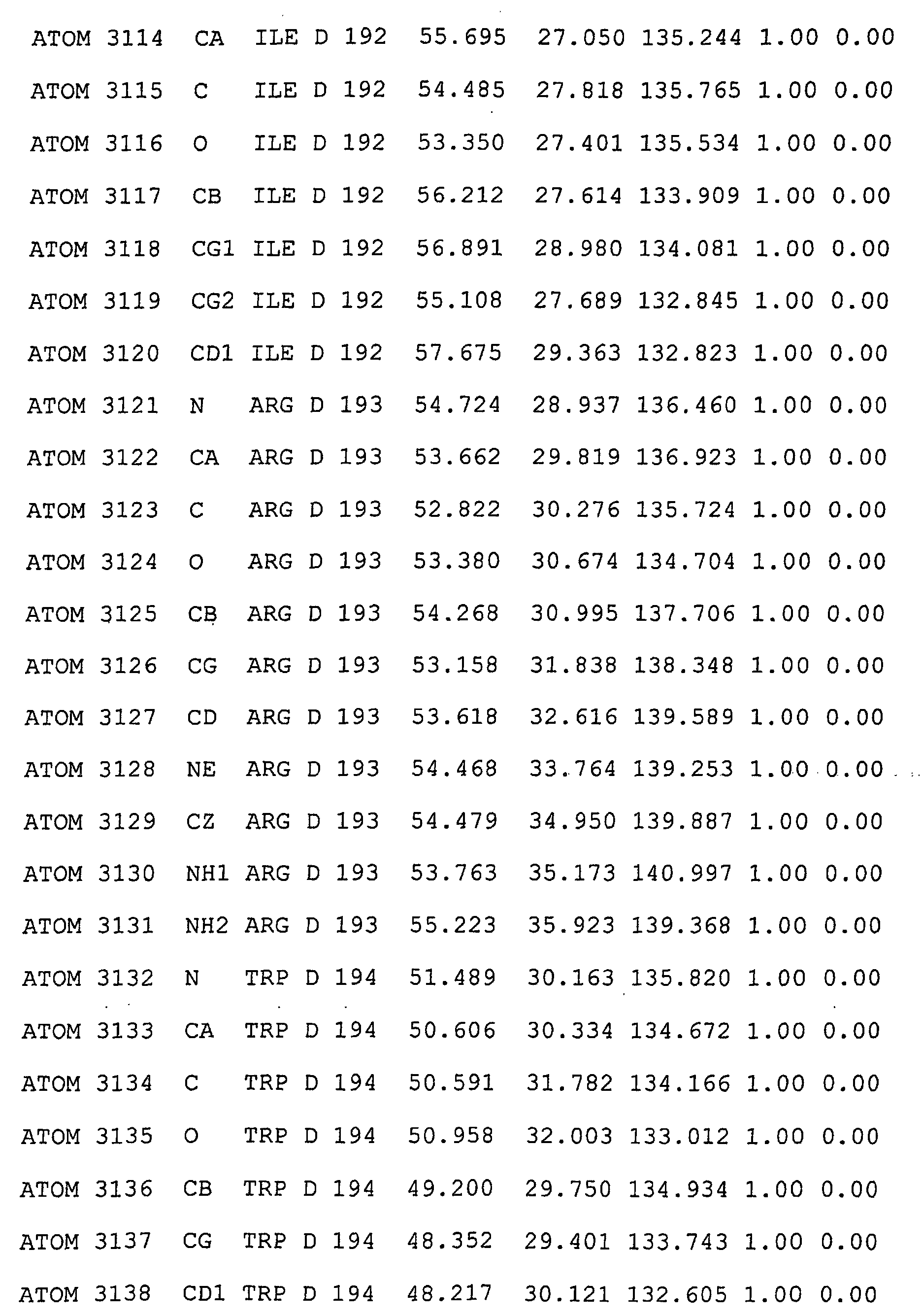

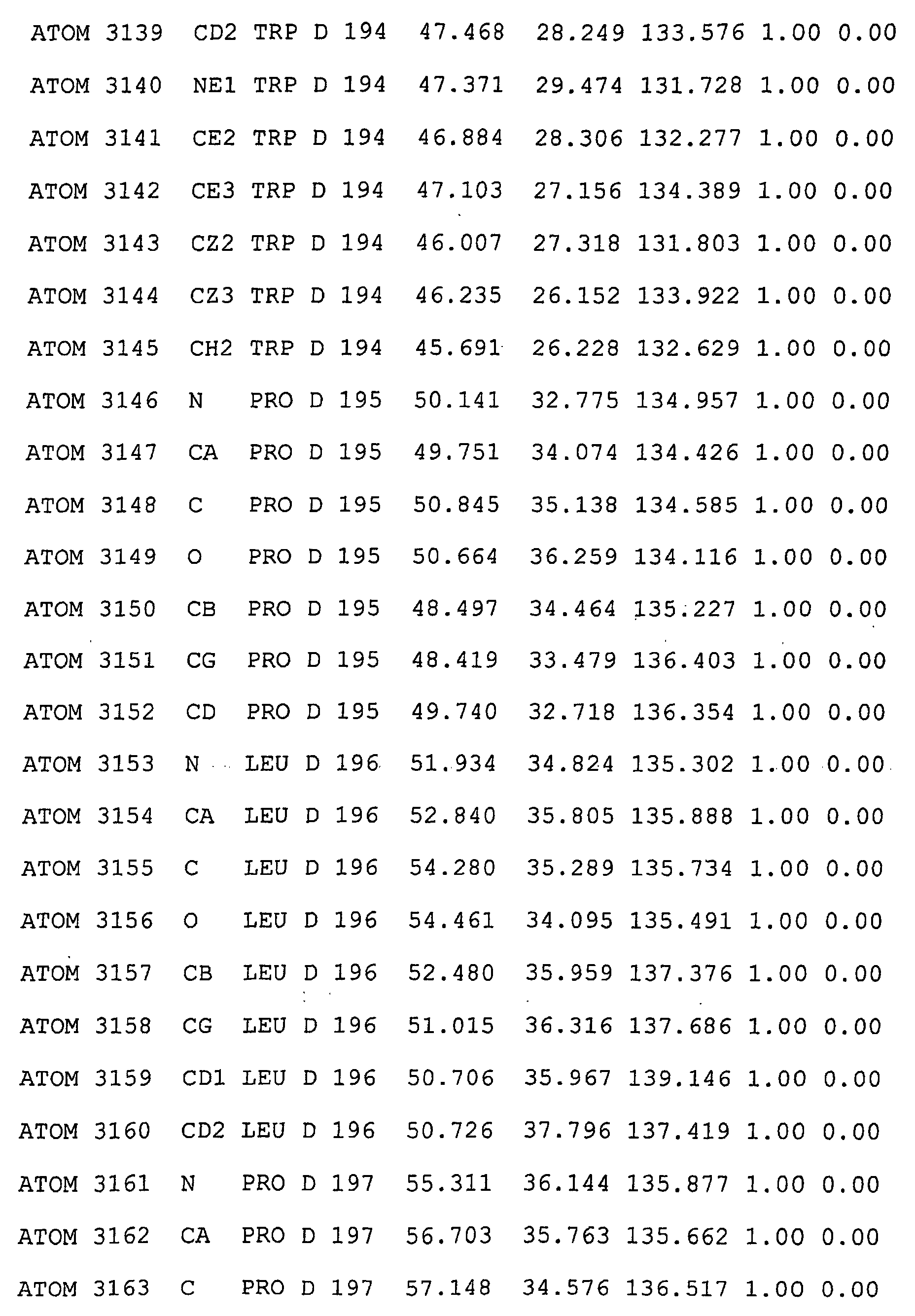

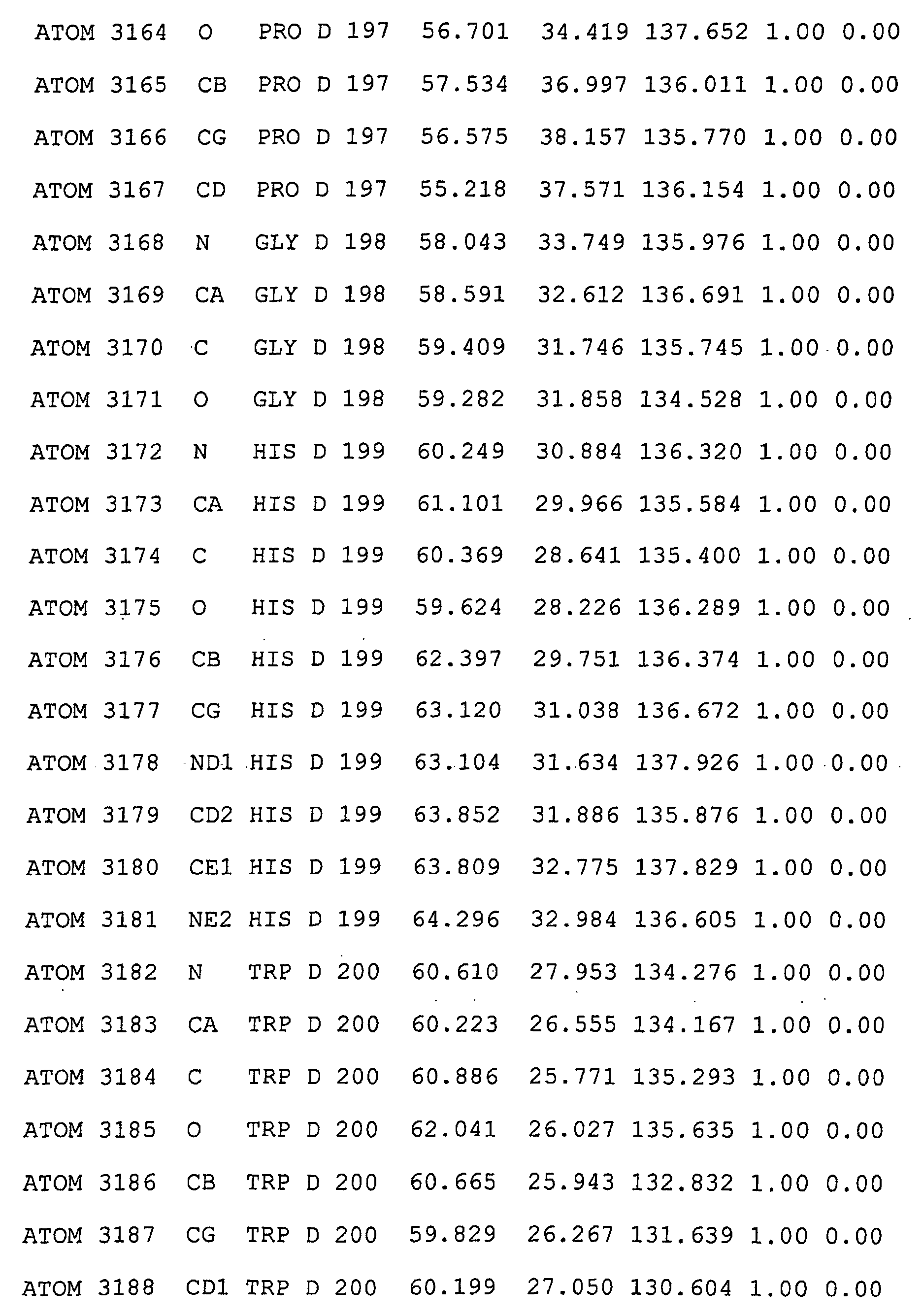

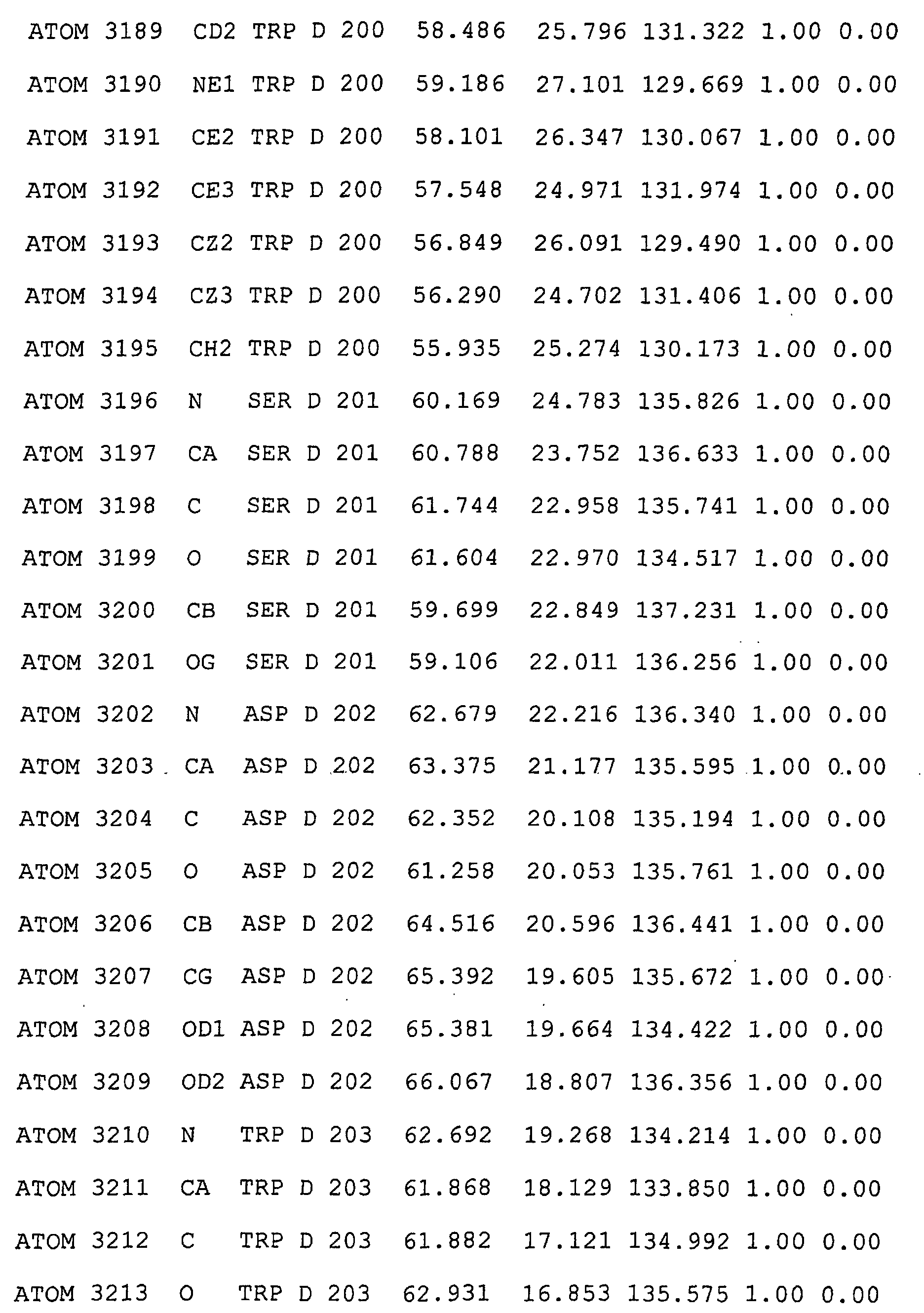

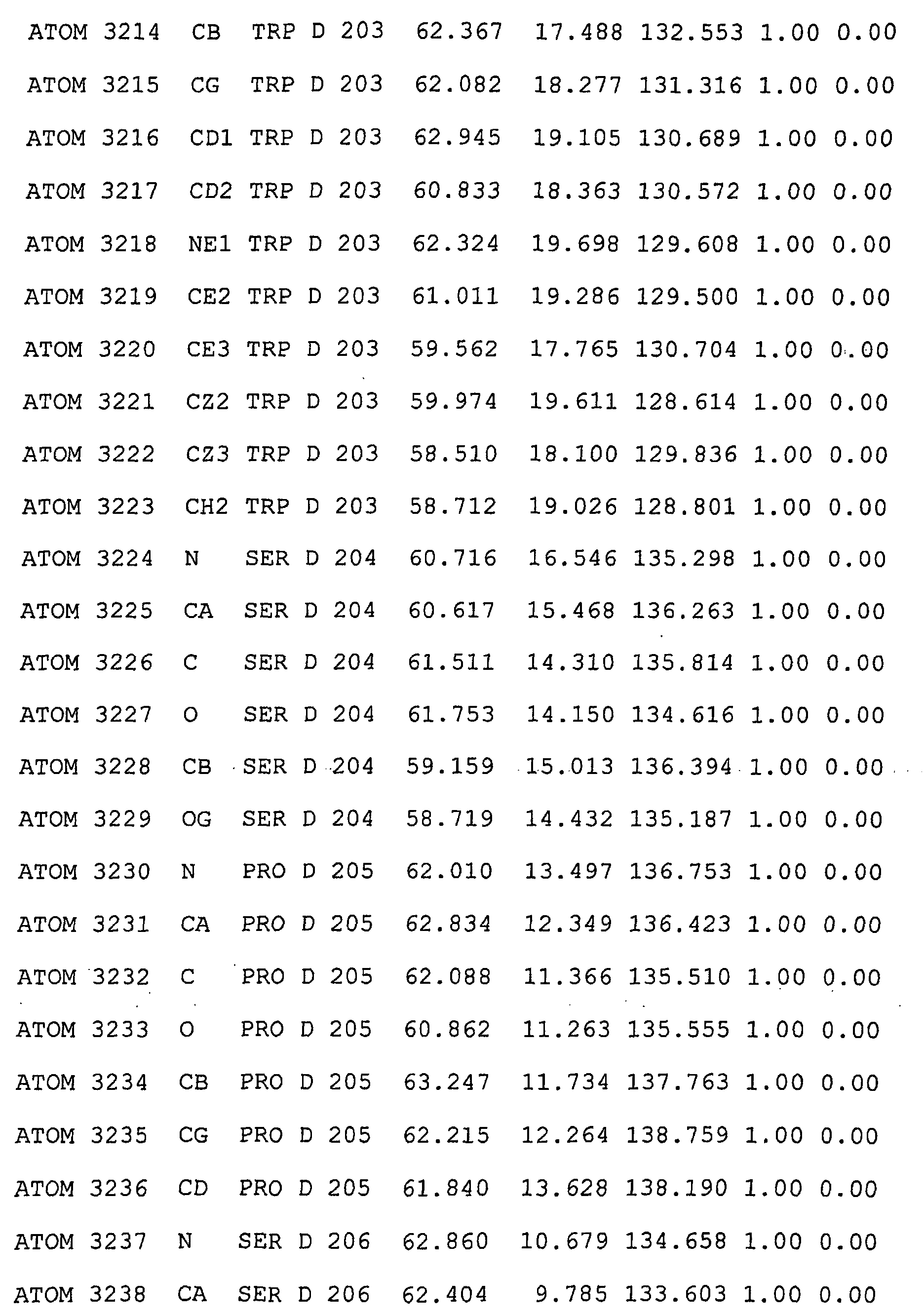

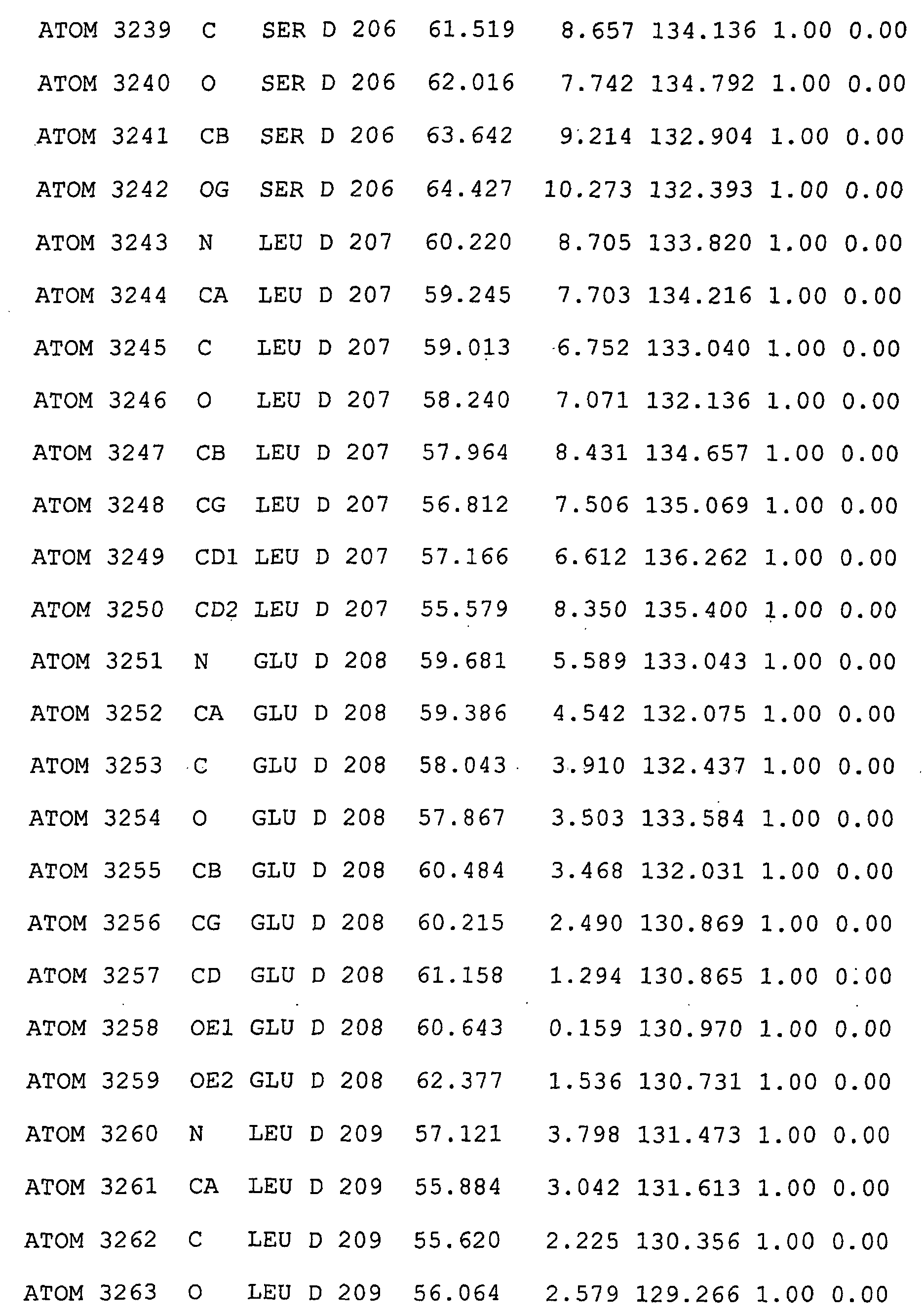

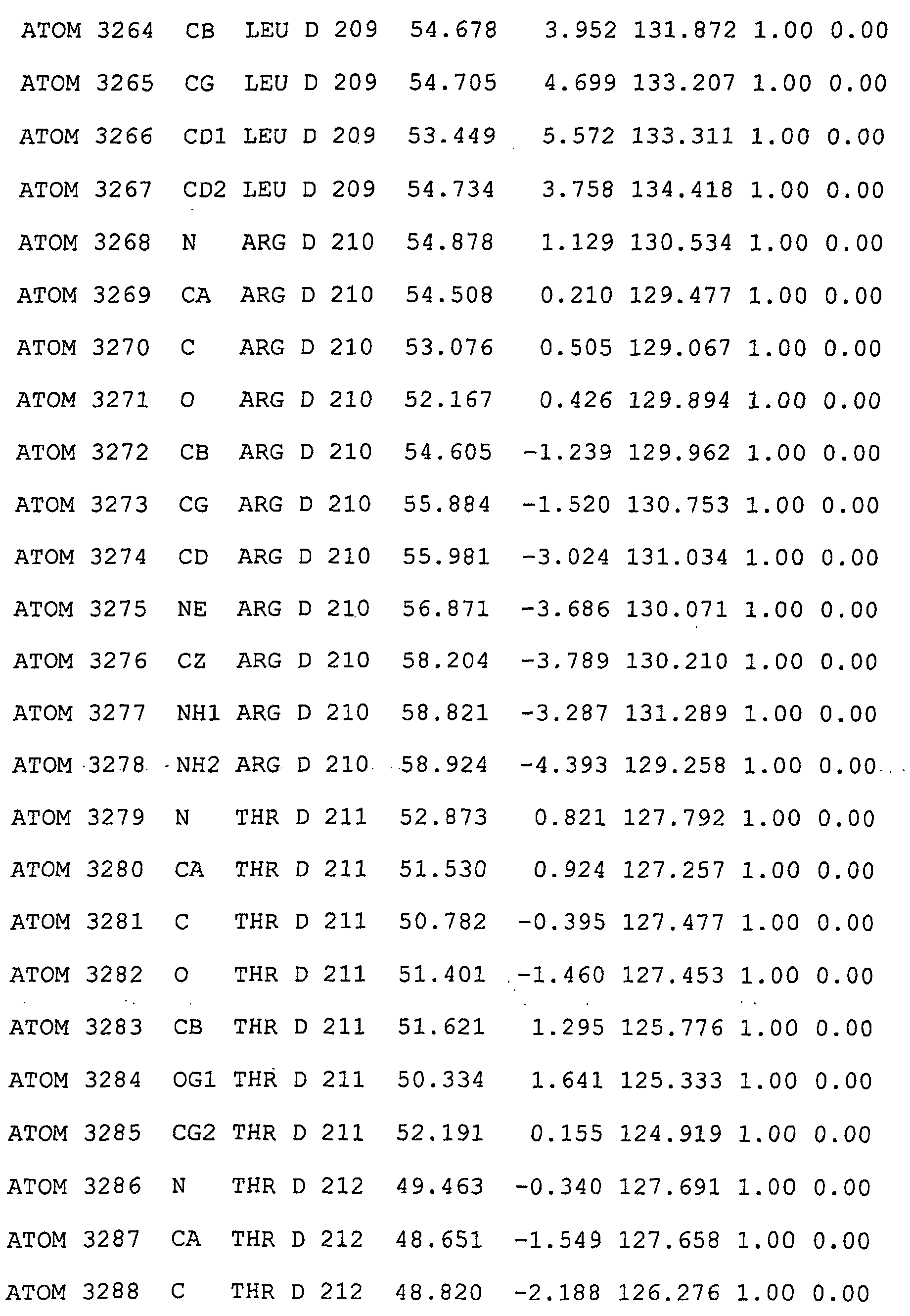

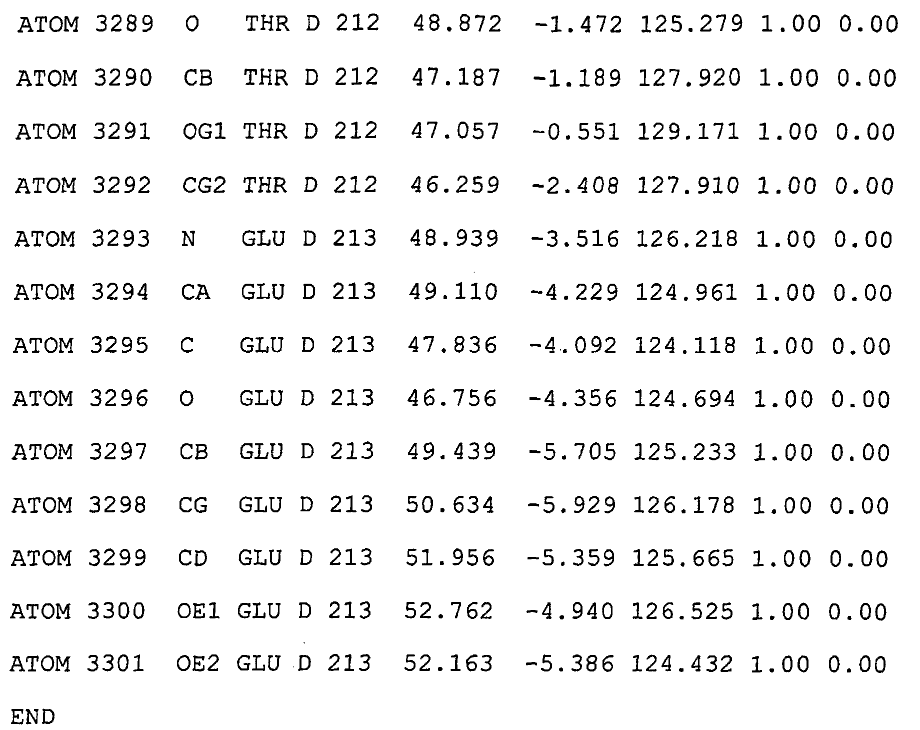

The first row through the last row but one describe three-dimensional coordinates for each atom. "ATOM" at the first column indicates that the row describes atom coordinates; the second column indicates the atom number; the third column indicates the atom type in the amino acid residue; the fourth column indicates the amino acid residue; the fifth column indicates the class of molecule (one particular class indicates a single polypeptide); the sixth column indicates the amino acid number according to SEQ ID NO: 3; the seventh, eighth, and ninth columns indicate coordinates of the atom (in Å for a-axis, b-axis, and c-axis directions in the order); the tenth column indicates the occupancy of the atom (in the present invention 1.00 for all atoms); and the eleventh column indicates the temperature factor of the atom (although in the present invention the value is expedientially 0.00, this value has no special mean). The last row indicates that the table ends at this row. In connection with the class of molecule, B and D each indicate one molecule of CRH-G-CSF-R. This table is described according to the format of Protein Data Bank, a notation system commonly used in the art.

Again, based on the three-dimensional structure coordinates shown in Table 6, amino acid residues characterizing the interactions can be located. When the homology between amino acid sequences becomes less than 20%, the reliability of derived three-dimensional structure coordinates declines. Since both human and mouse belongs to Mammalia, it is expected that amino acid sequences of a mammal-type G-CSF and a mammal-type G-CSF-R are highly homologous to those of human and mouse-type, respectively. Accordingly, if an exact amino acid sequence is determined for such a protein derived from a mammal, three-dimensional structure coordinates can easily be derived using the structure coordinates of the present invention.

The structure coordinates shown in Tables 1 and 6 are described by setting the origin of a unit cell in the crystal as the origin of the three-dimensional space. When, for example, the structure coordinates of the present invention are used in calculations with a computer, new structure coordinates obtained by mathematical operations such as translation, rotation, or symmetry transformation without altering relative positions of the atoms are also within the scope of the present invention.

The above structure coordinates were determined for the complex between G-CSF and CRH-G-CSF-R. However, since CRH-G-CSF-R can be considered as an equivalent of G-CSF-R in respect to binding with G-CSF, it is believed that the structure coordinates obtained above are substantially retained unchanged even in the complex between G-CSF and G-CSF-R. Therefore, the structure coordinates obtained above can be used to elucidate binding between G-CSF and G-CSF-R. Furthermore, they can also be used to identify, search for, evaluate, or design variants, agonists, or antagonists of G-CSF.

### 3. Use of structure coordinates of the complex for preparation of G-CSF variants

It becomes possible to express the mode of three-dimensional chemical interactions between G-CSF and CRH-G-CSF-R in detail by entering the structure coordinates obtained from the crystal of the complex between G-CSF and CRH-G-CSF-R of the present invention into a computer on which a computer program expressing three-dimensional coordinates of molecules runs or into a storage medium of the computer.

Such a computer storage medium is not particularly restricted so long as it can load the structure coordinates obtained from the crystal of the complex between G-CSF and CRH-G-CSF-R into the program. For example, it may be an electrical temporary storage medium known as a memory or a semipermanent storage medium such as a floppy disk, hard disk, optical disk, magneto-optical disk, or magnetic tape.

Likewise, it also becomes possible for the first time to achieve logical design in three-dimensional space for obtaining a variant having activities as an agonist of G-CSF which possesses, for example, higher biological activiies, higher biological stability, or better physical properties such as higher thermodynamic stability when compared with the native G-CSF, or for obtaining a variant having activities as an antagonist, for example, which retains binding activity to G-CSF-R but inhibits inherent biological activities of G-CSF, by entering the structure coordinates obtained from the complex between G-CSF and CRH-G-CSF-R of the present invention into a computer on which a computer program expressing three-dimensional coordinates of molecules runs or into a storage medium of the computer and by conducting visual examinations and/or energy calculations.

Many computer programs which express three-dimensional structure coordinates of protein molecules are commercially available, and they typically provide a means for entering three-dimensional structure coordinates of a molecule, a means for visually expressing the coordinates on the computer screen, a means for measuring interatomic distances or angles in the expressed molecule, a means for editing the coordinates, and the like.

In addition, programs prepared so that it can provide a means for calculating structure energy of a molecule based on coordinates of the molecule and a means for calculating free energy taking into account solvent molecules such as water molecules may also be used. Examples of programs suitable for such purposes include, but not limited to, Insight II or QUANTA commercially available form Molecular Simulation, Inc.

Such programs are usually installed for use on a computer called workstation supplied by Silicon Graphics, Inc., Sun Microsystems, Inc., or the like, although the present invention is not so limited.

Those skilled in the art can understand for the first time the binding mode of the complex between G-CSF and CRH-G-CSF-R in a state in which the atomic positions in three-dimensional space are expressed, using a computer tuned to run a suitable program for the purpose, by entering the structure coordinates of the complex between G-CSF and CRH-G-CSF-R of the present invention into the computer or into a storage medium of the computer, and it becomes thereby possible for the first time to logically and three-dimensionally design a G-CSF variant for obtaining a variant having activity as an agonist or antagonist as described above.

In one of representative methods for designing a G-CSF variant, three-dimensional structure coordinates of the complex between G-CSF and CRH-G-CSF-R of the present invention are entered into a computer or a storage medium: of the computer, and the three-dimensional structure of the protein is displayed on the computer screen using a suitable program to conduct visual examinations.

First, interacting amino acid residues and amino acid residue adjacent thereto in the complex between G-CSF and CRH-G-CSF-R are particularly displayed on the computer screen. One or more amino acid residues on the G-CSF side are then subjected to mutation such as substitution, deletion, or insertion, or to chemical modification in the computer, and changes in interactions produced by such alterations are observed on the computer screen. In this step, to describe three-dimensional structure of a protein on a computer screen, a three-dimensional representation using Crystal Eye glasses supplied by Silicon Graphics, Inc. or a method called Stereo View which displays two kinds of pictures corresponding to the visual fields of right and left eyes at the same time may be used for easier understanding of three-dimensional space, but visual examinations can also be achieved without using such a three-dimensional representation. Local structure coordinates which change due to mutation such as substitution, deletion, or insertion or due to chemical modification can be obtained by determining spatial positions of the atoms so as to retain the validities of the chemical bonds. In this step, one. may let the computer display candidates for appropriate conformations and select therefrom or may also let the computer calculate to determine a structure lowering the energy state. From such candidates, mutations or chemical modifications of G-CSF which result in more preferable binding with CRH-G-CSF-R are found.

Thus, in order to design a G-CSF variant having activity as an agonist, mutations are introduced into amino acid residues shown in Tables 2, 3, 4, and 5 which interacts with CRH-G-CSF-R to form the complex, that is, amino acid residues S13, L16, K17, E20, Q21, R23, K24, L109, D110, D113, T116, T117, Q120, E123, E124 and their adjacent regions and/or amino acid residues which form the associate, that is, amino acid residues G5, P6, A7, S8, S9, L10, P11, Q12, L125 and their adjacent regions so that they will more tightly bind with interacting amino acid residues in the corresponding regions on the CRH-G-CSF-R side. In this context, "their adjacent regions" refers to the regions which participate in interactions such as electrostatic interaction, hydrophobic interaction, van der Waals interaction, and hydrogen bonding with the amino acid residues in question, in particular the regions within about 5Å. The same applies throughout the present specification.

In addition, designing of a variant G-CSF having activity as an agonist, for example, by introducing mutations into positions other than those mentioned above is also within the scope of the present invention so long as the procedure employs the structure coordinates of the present invention.

Noncovalent interactions to be considered in this step include electrostatic interaction, hydrophobic interaction, van der Waals interaction, hydrogen bonding, and the like, and an ultimate design for a variant may be prepared comprehensively taking these interactions into account. For example, mutations are introduced so that in the neighborhood of a side chain of an amino acid residue having negative charge such as glutamic or aspartic acid on the CRH-G-CSF-R side, a side chain of an amino acid residue having positive charge such as lysine, arginine, or histidine will be positioned at an adjacent amino acid residue on the G-CSF, or reversely so that in the neighborhood of a side chain of an amino acid residue having positive charge such as lysine, arginine, or histidine on the CRH-G-CSF-R side, a side chain of an amino acid residue having negative charge such as glutamic or aspartic acid will be positioned at an adjacent amino acid residue on the G-CSF. Likewise, in a region wherein amino acid residues having highly hydrophobic side chains such as alanine, leucine, isoleucine, valine, proline, phenylalanine, tryptophan, and methionine mainly come together to interact, a position at which a hydrophilic amino acid residue such as serine, threonine, tyrosine, asparagine, or glutamine or a changed amino acid residue such as aspartic acid, glutamic acid, lysine, arginine, or histidine exists is sought in G-CSF and the found amino acid residue is replaced by a hydrophobic amino acid residue in order to strengthen the hydrophobic interaction. Furthermore, for backbone portions or side chain portions of amino acid residues like serine or tyrosine, which can form hydrogen bonds, corresponding amino acid residues are mutated so as to produce additional hydrogen bonds. In the above mutation, it is necessary to take care to maximize van der Waals interaction while avoiding steric hindrance among atoms in side chains of amino acid residues and backbone portions. In addition, it is also necessary to consider so as not to produce any new empty space, or in a region wherein an empty space already exists, it is necessary to consider so as to fill the empty space as much as possible. Thus, an ultimate design for a variant can be prepared in visual and comprehensive consideration of electrostatic interaction, hydrophobic interaction, van der Waals interaction, hydrogen bonding, and other factors on a computer screen.

Likewise, in order to design a G-CSF variant having activities as an antagonist, mutations are firstly introduced into amino acid residues shown in Tables 2, 3, 4, and 5 which interacts with CRH-G-CSF-R to form the complex, that is, amino acid residues S13, L16, K17, E20, Q21, R23, K24, L109, D110, D113, T116, T117, Q120, E123, E124 and their adjacent regions and/or amino acid residues which form the associate, that is, amino acid residues G5, P6, A7, S8, S9, L10, P11, Q12, L125 and their adjacent regions. A variant is then selected so that by binding of the variant G-CSF to CRH-G-CSF-R, intrinsic relative positions in three-dimensional space are not retained between G-CSF and two molecules of CRH-G-CSF-R, or such that the variant G-CSF can not interact with CRH-G-CSF-R in the complex or associate forming regions described above and consequently has activity as an antagonist against native G-CSF.

In addition, designing of a variant G-CSF having an antagonist, for example, by introducing mutations into positions other than those mentioned above is also within the scope of the present invention so long as the procedure employs the structure coordinates of the present invention.

Noncovalent interactions to be considered in this step are the same as in the case of G-CSF variants having activity as an agonist and an ultimate design for a variant may be prepared visually and comprehensively taking into account electrostatic interaction, hydrophobic interaction, van der Waals interaction, hydrogen bonding, and other factors on a computer screen.

The second method is to design the above variants by evaluation of binding with CRH-G-CSF-R using energy calculations on a computer. Energy calculations may be achieved by using a computer program which executes molecular force field calculations generally carried out in the art. Programs suitable for such purposes include, but not limited to, CVFF, AMBER force field optimized for proteins, which is contained in DISCOVER module of Insight II.

Furthermore, the first and the second design techniques are not strictly sorted out from each other, and both techniques may be used in combination. Specifically, with candidates expected by visual examinations to be more desirable, energy calculations are actually conducted using the second technique to evaluate their validities, and better variants are designed by repeating such steps.

As described above, preparation of variants which has been hitherto conducted by trial and error under conditions lacking theoretical supports relating to the three-dimensional structure can be achieved on the basis of theoretical analysis in three-dimensional space by using the structure coordinates of the present invention.

The structure coordinates of amino acid residues on the CRH-G-CSF-R side used in the first and second design techniques may be those of mouse-type CRH-G-CSF-R shown in Table 1 of the present specification or those of human-type CRH-G-CSF-R shown in Table 6. Furthermore, they may also be structure coordinates newly prepared, for example, by calculations with a computer based on such structure coordinates using sequences of G-CSF and G-CSF-R derived from other species having a high homology to the amino acid sequences of the above CRH-G-CSF-Rs, or they may even be structure coordinates excerpted in part therefrom. For designing a variant for human medication, it is more desirable to use structure coordinates of human-type CRH-G-CSF-R. As to the structure coordinates of CRH-G-CSF-R used, it is not necessary to use all coordinates of the receptor portion. Since the regions corresponding to the interacting portions of G-CSF and CRH-G-CSF-R shown in Tables 2, 3, 4, and 5 are important for designing a variant, it is also possible that the coordinates of amino acid residues involved in such interactions or, if necessary, of amino acid residues adjacent thereto are selected from Table 1 or 6 and used for designing. In such designing, although structure coordinates of G-CSF and CRH-G-CSF-R are usually used while being fixed in three-dimensional space, it is not necessarily required to fix the coordinates. In particular, the two molecules of the receptor-existing in a crystallographic asymmetric unit may be each subjected to translation or rotation in three-dimensional space as a block, and furthermore, amino acid residues in each block may also be displaced to such an extent that no chemical covalent bonds are cleaved to calculate energy of binding with a G-CSF variant. The structure coordinates changed due to translation, rotation, or displacement during such calculations in three-dimensional space are within the scope of the present invention.

Variants designed according to the present invention may be prepared by many methods. For example, a DNA encoding a variant designed on the basis of the present invention may be obtained as follows: at a position identified as an amino acid residue of which mutation will enhance biological activities on the basis of the present invention, an oligonucleotide portion encoding the corresponding amino acid residue is replaced by chemically synthesizing an oligonucleotide corresponding to the variant and substituting it for the native oligonucleotide using sequence-specific oligonucleotide cleaving enzymes (restriction enzymes). The variant DNA obtained may be then incorporated into an appropriate expression vector, introduced into an appropriate host cell, and expressed as a recombinant protein to obtain the above variant. Such preparation methods are commonly used in the art (see, e.g., "CURRENT PROTOCOLS Compact-ban: Bunshi-seibutsu-gakujikken-Protocol", I, II, III", translators: Kaoru Saigou and Yumiko Sano, Maruzen: the original is Ausubel F. M. et al., "Short Protocols in Molecular Biology", Third Edition, John Wiley & Sons, Inc., New York).

Likewise, chemical modifications of amino acid residues are also generally conducted in the art (see, e.g., Hirs, C. H. W. and Timasheff, S. N. eds., (1977), Methods in Enzymology, Vol. 47, pp. 407-498, Academic Press, New York).

### 4. Use of structure coordinates of the complex for preparation of G-CSF agonists

By entering all or part of the structure coordinates of the complex between G-CSF and CRH-G-CSF-R provided by the present invention into a computer on which a computer program expressing three-dimensional coordinates of molecules runs or into a storage medium of the computer, it becomes possible to identify, search for, evaluate, or design compounds which bind to CRH-G-CSF-R and thereby provide the CRH-G-CSF-R with positions in three-dimensional space substantially identical to those of the two molecules of CRH-G-CSF-R in the associate of G-CSF and CRH-G-CSF-R and which have biological activities equal or superior to those of G-CSF. In the art, such compounds are collectively referred to as agonists. The compounds may be natural or synthetic, and may be high molecular weight or low molecular weight compounds.

As described above, it is believed that the complex formed by one molecule of G-CSF and one molecule of G-CSF-R further self-associate into a dimer and the signal of G-CSF is thereby received by G-CSF-R. Accordingly, agonists should bind to CRH-G-CSF while retaining spatial position of the two molecules of CRH-G-CSF-R in the associate.

For example, Indigo 2, a workstation supplied by Silicon Graphics, Inc., is suitable as a computer used for designing agonists. However, the computer is not limited to this one, and any computer may be used so long as it is tuned to run an appropriate program. Likewise, there is no particular limitation on the computer storage medium. For example, Insight II, a computer program commercially available from Molecular Simulation, Inc. may be used as a program for designing. In particular, a program Ludi or DOCK, a module of Insight II specially prepared for such purposes, may be used alone or in combination to facilitate identification, searching, evaluation, or designing. Furthermore, designing of agonists according to the techniques described in Japanese Patent Kokai Publication Nos. H6-309385 (1994) and H7-133233 (1995) can also be achieved for the first time by using structure coordinates of the complex between G-CSF and CRH-G-CSF-R of the present invention. The present invention is, however, not limited to these programs and techniques.

In designing of agonists, there are conceptually two steps. The first step is to find a compound which serves as a starting point for drug design, known for those skilled in the art as a lead compound. The next step is optimization of the lead compound wherein compounds having better properties as medicines, for example, having better activity, having better pharmacokinetics, or having less toxicities and side effects are sought starting from the lead compound.

The step in which a lead compound is found using the structure coordinates of the complex between G-CSF and CRH-G-CSF-R provided by the present invention is achieved, for example, using a database in a computer into which structures of plural compounds have been entered, by a method in which interactions between three-dimensional structures of a compound in the database and CRH-G-CSF-R are sorted out in a visual manner one after another, or by a method in which amplitudes of binding energy are calculated one after another using a computer and compounds which stably bind to CRH-G-CSF-R are found from the database. Although it is preferred that the database of compound's structures contains determined three-dimensional structure coordinates entered therein, for low molecular weight compounds, it does not have to be a database of three-dimensional structure coordinates, because such low molecular weight compounds may change their conformations relatively freely, and also because three-dimensional structure coordinates for each conformation can be derived by calculations in a relatively short time. In the latter cases, information for chemical covalent bonds of low molecular weight compounds are entered into the database.

Specifically, in the visual method, two molecules of CRH-G-CSF-R in the associate are firstly displayed on a computer screen according to the structure coordinates of the present invention. In this step, although a three-dimensional representation may be made on the computer screen using, for example, Crystal Eye as described above, visual examinations can also be achieved without using such a three-dimensional representation. Then, on the computer, compounds in the database are allowed to bind with two molecules of CRH-G-CSF-R taking chemical interactions into account, and are evaluated one after another whether or not it can strongly bind with CRH-G-CSF-R, and if it can do so, whether or not the relative positions taken by the two molecules of CRH-G-CSF-R interacting with the compound are similar to those of the two molecules of CRH-G-CSF-R in the associate. In this connection, it is preferred that the compound binds with two molecules of CRH-G-CSF-R at a total of two or more sites (at least one site with each CRH-G-CSF-R) so that the relative positions taken by the two molecules of CRH-G-CSF-R in three-dimensional space are maximally conserved. Relative positions taken by the two molecules of CRH-G-CSF-R in three-dimensional space do not have to be strictly conserved, and are permitted to vary to a certain degree so long as activity of the compound as an agonist is retained.

Chemical interactions to be considered include electrostatic interaction, hydrophobic interaction, hydrogen bonding, van der Waals interaction, and the like. Thus, the structure should be comprehensively examined whether it is favorable for interactions, for example, so that functional groups which tend to bear negative charge such as carboxyl group, nitro group, and halogens interact with amino acid residues in CRH-G-CSF-R having positive charge such as lysine, arginine, and histidine, so that functional groups which tend to bear positive charge such as amino, imino, and guanidyl groups interact with amino acid residues in CRH-G-CSF-R having negative charge such as glutamic acid and aspartic acid, so that hydrophobic functional groups such as aliphatic groups and aromatic groups interact with hydrophobic amino acid residues such as alanine, leucine, isoleucine, valine, proline, phenylalanine, tryptophan and methionine, so that functional groups involved in hydrogen bonding such as hydroxyl and amide groups can form hydrogen bonds with backbone or side chain portions of CRH-G-CSF-R, so that binding between the compound and CRH-G-CSF-R causes no steric hindrance, and so that empty spaces are filled to minimize such empty spaces and maximize van der Waals interaction. Thus, electrostatic interaction, hydrophobic interaction, van der Waals interaction, hydrogen bonding, and other factors are visually and comprehensively considered to finally determine whether or not the compound is suitable as a lead compound.

In the method by energy evaluation with a computer, the energy of binding between a compound and the two molecules of CRH-G-CSF-R in the associate is determined by molecular force field calculations. Such calculations are applied to each compound in the database to find a compound which may serve as a lead compound capable of stable binding. As a molecular force field used in the calculations, for example, CVFF, AMBER force field optimized for proteins, which is contained in DISCOVER module of Insight II program may be used. In addition, some computer programs like Ludi in Insight II can automatically output candidates for lead compound when three-dimensional structure coordinates of interacting amino acid residues in a protein molecule are given, and such programs may also be applied to G-CSF or CRH-G-CSF-R.

Furthermore, the visual examinations and the examination considering energy are not strictly sorted out from each other, and both techniques may be used in combination as appropriate.

The next step, in which optimization of the lead compound is conducted using the structure coordinates of the complex between G-CSF and CRH-G-CSF-R provided by the present invention, is used for the purpose of, where a lead compound which binds to CRH-G-CSF-R has already been found by the above method or separately found in an experimental manner, optimizing the lead compound to obtain a better compound, for example, a compound having higher biological activities as an agonist or a compound having a structure favorable for oral administration as a medicine. As a method of experimentally finding a lead compound, a lead compound may be selected, for example, from a series of compounds known in the art as a combinatorial library or from a culture medium of a microbe or the like. Furthermore, it may even be a compound which is found in the course of designing of antagonists described below. In short, it becomes possible only after a precise picture of chemical bonding between the lead compound and CRH-G-CSF-R has been elucidated to directly find a site which is not optimal for interactions between the lead compound and CRH-G-CSF-R and to design a new compound having an optimal functional group at that site, thereby enabling to design a more optimized compound.

Although in order to exactly understand the binding mode between a lead compound and CRH-G-CSF-R in an early stage, it is more desirable to use a method in which a cocrystal of the lead compound and CRH-G-CSF-R is prepared and a precise picture of chemical interactions between the lead compound and CRH-G-CSF-R is experimentally elucidated by X-ray crystallography using a molecular replacement method which is encompassed by the present invention and hereinafter described, chemical interactions between the lead compound and CRH-G-CSF-R may also be understood by visual examinations or energy calculations using a computer.

For visual examinations with a computer, a model of the complex between the lead compound and CRH-G-CSF-R is firstly displayed on a computer screen by entering the three-dimensional structure coordinates of the lead compound and the structure coordinates of CRH-G-CSF-R provided by the present invention into a computer on which a computer program expressing three-dimensional coordinates of molecules runs or into a storage medium of the computer. In this step, although a three-dimensional representation may be made on the computer screen using, for example, Crystal Eye as described above, visual examinations can also be achieved without using such a three-dimensional representation. It is a logical designing of a compound to modify the lead compound so as to yield a compound more favorably interacting with CRH-G-CSF-R or a compound having better pharmacokinetics while retaining the interactions.

Chemical interactions to be considered are the same as in the step to find a lead compound, and a new compound having better properties as an agonist is finally designed starting from the lead compound.

In the method by energy evaluation with a computer, the energy of binding between a new compound designed from the lead compound and CRH-G-CSF-R is determined by molecular force field calculations to judge the validity of the design. In addition, it is also possible to use a method in which other molecules such as solvent molecules are additionally included in the model and the free energy is determined using molecular dynamics to derive a compound capable of stable binding. As a molecular force field used in the calculations, for example, CVFF, AMBER force field optimized for proteins, which is contained in DISCOVER module of Insight II program may be used.

Furthermore, the visual examinations and the method by energy evaluations may be used in combination as appropriate.

It is preferred that the newly designed compound binds with two molecules of CRH-G-CSF-R at a total of two or more sites (at least one site with each CRH-G-CSF-R) so that the relative positions taken by the two molecules of CRH-G-CSF-R in three-dimensional space are maximally conserved. Relative positions taken by the two molecules of CRH-G-CSF-R in three-dimensional space do not have to be strictly conserved, and are permitted to vary to a certain degree so long as activity of the compound as an agonist is retained. The structure coordinates changed due to translation, rotation, or displacement during such calculations in three-dimensional space are within the scope of the present invention.

The structure coordinates of amino acid residues on the CRH-G-CSF-R side used in the above techniques may be those of mouse-type CRH-G-CSF-R shown in Table 1 of the present specification, those of human-type CRH-G-CSF-R shown in Table 6, or even those prepared by calculations based on such structure coordinates. For designing a variant as a medicine for human, it is more desirable to use structure coordinates of human-type CRH-G-CSF-R. As to the structure coordinates of CRH-G-CSF-R used, it is not necessary to use all coordinates of the receptor portion. Since the regions corresponding to the interacting portions of G-CSF and CRH-G-CSF-R shown in Tables 2, 3, 4, and 5 are important for agonists, it is also possible that coordinates of amino acid residues involved in such interactions and, if necessary, of amino acid residues adjacent thereto are selected from Table 1 or 6 and used for designing. Thus, a G-CSF agonist which retains relative positions taken by the two molecules of CRH-G-CSF-R in the associate can be obtained by selecting structure coordinates of the regions corresponding to those amino acid residues and by designing a compound so that one molecule of the compound simultaneously binds to the two regions of CRH-G-CSF-R to which one molecule of G-CSF binds.

For designing an agonist which can bind while retaining the relative positions taken by the two molecules of CRH-G-CSF-R in the associate, it is also useful to employ the structure coordinates of the region spatially surrounded by the two molecules of CRH-GS-CSF-R. These spatially surrounding amino acid residues are characterized by Y3 to L14, R46 to Y51, G92 to V106, E145 to E147, H166 to S169, S194 to G198. Accordingly, structure coordinates corresponding to these amino acid residues and, if necessary, amino acid residues adjacent thereto may be selected from Table 1 or 6 and used for designing. Thus, a G-CSF agonist which retains relative positions taken by the two molecules of CRH-G-CSF-R in the associate can be obtained by designing a compound so that the compound fits into the cavity surrounded by these amino acid residues and simultaneously binds to two or more regions of each CRH-G-CSF-R while minimizing the energy.

Agonists designed by the above techniques may be obtained by commonly used techniques for chemical synthesis depending on the particular compound.

### 5. Use of structure coordinates of the complex for preparation of C-CSF antagonists

By entering all or part of the structure coordinates of the complex between G-CSF and CRH-G-CSF-R provided by the present invention into a computer on which a computer program expressing three-dimensional coordinates of molecules runs or into a storage medium of the computer, it becomes possible for the first time to identify, search for, evaluate, or design compounds which bind with G-CSF and/or CRH-G-CSF-R and inhibit biological activities of G-CSF. In the art, such compounds are collectively referred to as antagonists. The compounds may be natural or synthetic, and may be high molecular weight or low molecular weight compounds.

As described above, it is believed that the complex formed by one molecule of G-CSF and one molecule of G-CSF-R further self-associates into a dimer and the signal of G-CSF is thereby received by G-CSF-R. Accordingly, an antagonist should bind to G-CSF and/or CRH-G-CSF so that it provides positions in three-dimensional space different from those of the two molecules of CRH-G-CSF-R in the associate formed by the natural G-CSF and CRH-G-CSF-R, or so that it inhibits formation of the complex or the associate between G-CSF and CRH-G-CSF.

Thus,. there are several embodiments of antagonists, for example,
(1) antagonists which bind to G-CSF and inhibit formation of the complex,
(2) antagonists which bind to G-CSF-R and inhibit formation of the complex,
(3) antagonists which bind to G-CSF and/or G-CSF-R and allow formation of the complex, but inhibit formation of the associate, and
(4) antagonists which bind to G-CSF and/or G-CSF-R and allow formation of the complex and the associate, but result in an abnormal structure which prevent the signal from entering into the cell.

As in the case of agonists, a computer used for designing antagonists is not particularly limited so long as it is tuned to run an appropriate program. Likewise, there is no particular limitation on the computer storage medium. For example, Insight II, a computer program commercially available from Molecular Simulation, Inc. may be used as a program for designing. In particular, a program Ludi or DOCK, a module of Insight II specially prepared for such purposes, may be used alone or in combination to facilitate identification, searching, evaluation, or designing. Furthermore, designing of antagonists according to the techniques described in Japanese Patent Kokai Publication Nos. H6-309385 (1994) and H7-133233 (1995) can also be achieved for the first time by using structure coordinates of the complex between G-CSF and CRH-G-CSF-R of the present invention. The present invention is, however, not limited to these programs and techniques.

In designing of antagonists, there are conceptually two steps as in the case of agonists. The first step is to find a lead compound and the next step is a process for optimization of the lead compound.

The step in which a lead compound for antagonists is found using the structure coordinates of the complex between G-CSF and CRH-G-CSF-R provided by the present invention is achieved, for example, using a database in a computer into which structures of plural compounds have been entered, by a method in which interactions between three-dimensional structures of a compound in the database and G-CSF and/or CRH-G-CSF-R are sorted out in a visual manner one after another, or by a method in which amplitudes of binding energy are calculated one after another using a computer and compounds which stably bind to G-CSF and/or CRH-G-CSF-R are found from the database. Although it is preferred that the database of compound's structures contains determined three-dimensional structure coordinates entered therein, for low molecular weight compounds, it does not have to be a database of three-dimensional structure coordinates, because such low molecular weight compounds may change their conformations relatively freely, and also because three-dimensional structure coordinates for each conformation can be derived by calculations in a relatively short time. In the latter cases, information for chemical covalent bonds of low molecular weight compounds are entered into the database.

Specifically, in the visual method, G-CSF and/or CRH-G-CSF-R molecule or molecules are firstly displayed on a computer screen according to the structure coordinates of the present invention. In this step, although a three-dimensional representation may be made on the computer screen using, for example, Crystal Eye as described above, visual examinations can also be achieved without using such a three-dimensional representation. Then, on the computer, compounds in the database are allowed to bind with G-CSF and/or CRH-G-CSF-R molecule or molecules taking chemical interactions into account, and are evaluated one after another whether or not the compound can function to provide positions in three-dimensional space different from those taken by the two molecules of CRH-G-CSF-R in the associate formed by G-CSF and CRH-G-CSF-R or to inhibit formation of the complex or the associate between G-CSF and CRH-G-CSF-R. It is desirable that at least one of the sites at which an antagonist binds to G-CSF and/or CRH-G-CSF-R corresponds to the amino acid residue at which G-CSF binds to CRH-G-CSF-R or an amino acid residue adjacent thereto, and a compound of which binding to G-CSF and/or CRH-G-CSF-R sterically hinders the binding between G-CSF and CRH-G-CSF-R with the result that relative positions intrinsically taken by the two molecules of CRH-G-CSF-R in three-dimensional space are not retained or that formation of the complex and/or the associate between G-CSF and CRH-G-CSF-R is inhibited is selected. Furthermore, the compound does not need to simultaneously bind with two molecules of G-CSF, two molecules of CRH-G-CSF-R, or one molecule of G-CSF and one molecule of CRH-G-CSF-R, and it is preferred that a compound which binds to one molecule of G-CSF or CRH-G-CSF-R is selected.

Chemical interactions to be considered are the same as in the case of agonists and final judgement as to whether or not the compound is appropriate as a lead compound is made visually and comprehensively taking into account such interactions.

In the method by energy evaluation with a computer, the energy of binding between a compound selected from the database and G-CSF and/or CRH-G-CSF-R is determined as in the case of agonists to find a compound which may serve as a lead compound for antagonists from the database. As a molecular force field used in the calculations, for example, CVFF, AMBER force field optimized for proteins, which is contained in DISCOVER module of Insight II program may be used. In addition, some computer programs like Ludi in Insight II can automatically output candidates for lead compound when three-dimensional structure coordinates of interacting amino acid residues in a protein molecule are given.

The next step, in which optimization of the lead compound is conducted using the structure coordinates of the complex between G-CSF and CRH-G-CSF-R provided by the present invention, is used for the purpose of, where a lead compound which binds to G-CSF and/or CRH-G-CSF-R has already been found by the above method or separately found in an experimental manner, optimizing the lead compound to obtain a better compound, for example, a compound having higher biological activities as an antagonist or a compound having a structure favorable for oral administration as a medicine. The method for experimentally finding a lead compound is the same as that for agonists, and a compound found in the above-described designing of agonists may also be used as a lead compound. In short, it becomes possible only after a precise picture of chemical bonding between the lead compound and G-CSF and/or CRH-G-CSF-R has been elucidated to directly find a site which is not optimal for interactions between G-CSF and/or CRH-G-CSF-R and the lead compound and to design a new compound having an optimal functional group at that site, thereby enabling to design a more optimized compound.

Although in order to exactly understand the binding mode between a lead compound and G-CSF and/or CRH-G-CSF-R in an early stage, it is more desirable to use a method in which a cocrystal of the lead compound and G-CSF and/or CRH-G-CSF-R is prepared and a precise picture of chemical interactions between the lead compound and G-CSF and/or CRH-G-CSF-R is experimentally elucidated by X-ray crystallography using a molecular replacement method which is encompassed by the present invention and hereinafter described, understanding of chemical interactions by visual examinations or energy calculations using a computer may also suffice.

For visual examinations with a computer, a model of the complex between G-CSF and/or CRH-G-CSF-R and the lead compound is firstly displayed on a computer screen by entering the three-dimensional structure coordinates of the lead compound and the structure coordinates of G-CSF and/or CRH-G-CSF-R provided by the present invention into a computer on which a computer program expressing three-dimensional coordinates of molecules runs or into a storage medium of the computer. In this step, although a three-dimensional representation may be made on the computer screen using, for example, Crystal Eye as described above, visual examinations can also be achieved without using such a three-dimensional representation. It is a logical designing of a compound to modify the lead compound so as to yield a compound which binds to G-CSF and/or CRH-G-CSF-R and inhibits interactions between G-CSF and CRH-G-CSF-R or a compound having better pharmacokinetics while retaining the ability of inhibiting interactions.

Chemical interactions to be considered are the same as in the step to find a lead compound, and a new compound having better properties as an antagonist is finally designed starting from the lead compound.

In the method by energy evaluation with a computer, the energy of binding between a new compound designed from the lead compound and G-CSF and/or CRH-G-CSF-R. is determined by molecular force field calculations to judge the validity of the design. In addition, it is also possible to use a method in which other molecules such as solvent molecules are additionally included in the model and the free energy is determined using molecular dynamics to derive a compound capable of stable binding. As a molecular force field used in the calculations, for example, CVFF, AMBER force field optimized for proteins, which is contained in DISCOVER module of Insight II program may be used.

Furthermore, the visual examinations and the method by energy evaluations may be used in combination as appropriate.

The structure coordinates of amino acid residues on the G-CSF and/or CRH-G-CSF-R side(s) used in the above techniques may be those of human-type G-CSF and mouse-type CRH-G-CSF-R shown in Table 1 of the present specification, those of human-type CRH-G-CSF-R shown in Table 6, or even those prepared by calculations based on such structure coordinates. For designing a variant as a medicine for human, it is more desirable to use structure coordinates of human-type G-CSF and CRH-G-CSF-R. Since designing of an antagonist involves designing of a compound which binds to G-CSF and/or CRH-G-CSF-R, the amino acid residues corresponding to the interacting portions of G-CSF and CRH-G-CSF-R shown in Tables 2, 3, 4, and 5 as well as the regions of amino acid residues adjacent thereto are important among the structure coordinates of G-CSF or CRH-G-CSF-R, and it is also possible that the coordinates of such amino acid residues are selected from Table 1 or 6 and used for designing. Thus, an antagonist may be obtained by selecting the coordinates of the portions corresponding to such amino acid residues and by designing the compound so that it inhibits normal binding between G-CSF and CRH-G-CSF-R to prevent G-CSF from exerting its biological activities.

Antagonists designed by the above techniques may be obtained by commonly used techniques for chemical synthesis depending on the particular compound.

### 6. Use of structure coordinates of the complex for conducting X-ray crystallography by molecular replacement method

The three-dimensional structure coordinates of G-CSF and CRH-G-CSF-R according to the present invention which are shown in Table 1 or 6 may be used in X-ray crystallography, for example, of crystals containing the whole or part of G-CSF and G-CSF-R or crystals obtained from other proteins which comprise an amino acid sequence having a significant homology to G-CSF or G-CSF-R. Specifically, in a molecular replacement method commonly used in the art as one of techniques for X-ray crystallography (see, e.g., Blundell, T. L. and Johnson, L. N., (1976), PROTEIN CRYSTALLOGRAPHY, pp. 443-464, Academic Press, New York), all or part of the three-dimensional structure coordinates of the complex between G-CSF and CRH-G-CSF-R of the present invention may be used to determine structure coordinates from a structure factor obtained from X-ray diffraction patterns of a crystal without using heavy atom isomorphous replacement methods and much rapidly even for a crystal of a protein having unknown structure coordinates as mentioned above.

For conducting a molecular replacement method, a computer tuned to run a program for use in the molecular replacement method is used. Examples of such programs include Insight II (commercially available from MSI), X-PLOR (commercially available from Molecular Simulation, Inc.,), and AMORE (one of the programs in CCP4 (Collaborative Computational Project, Number 4., *Acta Crystallogr.* D**50,** 670-673 (1994) ), but other programs may also be used.

In addition to crystals containing the whole or part of G-CSF and G-CSF-R and crystals obtained from other proteins which comprise an amino acid sequence having a significant homology to G-CSF or G-CSF-R, crystals to which a molecular replacement method should be applied using the three-dimensional coordinates of the complex between G-CSF and CRH-G-CSF-R of the present invention include, for example, crystals obtained from a complex between a compound binding with G-CSF-R (e.g., an agonist or antagonist) and G-CSF-R, a complex between a compound binding with G-CSF (e.g., an antagonist) and G-CSF, G-CSF-R containing amino acid residues other than those described in Table 6, a substance containing a protein having a significant homology to G-CSF at the amino acid level, a substance containing a protein having a significant homology to G-CSF-R at the amino acid level, a variant of G-CSF and a substance containing thereof, a variant of G-CSF-R and a substance containing thereof, and also include crystals obtained from complexes thereof. The term "significant homology" usually refers to a case wherein the identity of the amino acid sequences is 20% or above, and preferably 30% or above. The validity of application of the molecular replacement method may be determined by actually applying the molecular replacement method to the structure factor calculated from X-ray diffraction pattern of a crystal in question and thereby obtaining a significant solution.

Accordingly, to analyze structure of a crystal of any unknown substance other than those described above by a molecular replacement method using all or part of the three-dimensional structure coordinates of G-CSF and CRH-G-CSF-R is also within the scope of the present invention so long as it yields a significant solution.

### EXAMPLES

The present invention is described in more detail with reference to the following working examples. The present invention is, however, not to limited to these examples in any way. The scope of the present invention to be claimed should be defined by the teachings in the sections in detailed description of the invention rather than the specific embodiments shown in the examples.

### Example 1

### Preparation of complex between G-CSF and CRH-G-CSF-R

Mouse-type CRH-G-CSF-R was prepared by the method described in Japanese Patent Application Ser. No. H6-280655 (1994) (Japanese Patent Kokai Publication No. H8-140678 (1996)). In order to obtain a material suitable for crystallization, the CRH-G-CSF-R fraction was further purified by its electrical properties using ion-exchange column chromatography (Mono-S HR10/10, Pharmacia). Human-type G-CSF produced as a recombinant protein in *E. coli* was provided by Kirin Brewery Co., Ltd.

In order to prepare the complex between G-CSF and CRH-G-CSF-R, protein concentrations of G-CSF and CRH-G-CSF-R obtained were each determined by measuring their ultraviolet absorbances, and the proteins were combined using stoichiometrically equivalent quantities calculated from these concentrations or using an excess of G-CSF. The sample was fractionated by gel filtration chromatography (HiLoad Superdex 200 HR26/60, Pharmacia) using a buffer solution consisting of 0.01M 2-(N-morpholino)ethanesulfonic acid (pH 6) containing 0.1M sodium chloride as the mobile phase to yield a highly purified complex between G-CSF and CRH-G-CSF-R.

### Example 2

### Preparation of crystal of the complex between G-CSF and CRH-G-CSF-R

The crystal of the complex between G-CSF and CRH-G-CSF-R was prepared using a vapor diffusion technique describe below. The highly purified complex between G-CSF and CRH-G-CSF-R in Example 1 was adjusted to a protein concentration of 0.5 to 2 mg/ml using a device with an ultrafiltration membrane (Centricon-10, Grace Japan). To 1 to 10 µl of this protein solution, an equal volume of 0.1M N-2-hydroxyethylpiperazine-N'-2-ethanesulfonic acid buffer solution (pH 7 to 8) containing 1.0 to 1.2 M ammonium sulfate was added as a crystallization solution, mixed, and placed on a glass plate of which surface has been treated with an agent for silicone coating (Sigmacote, Sigma) so as to become hydrophobic. The glass plate surmounted by the sample was put into an air-tight container containing 1 to 5 ml of the crystallization solution, and allowed to stand at 20°C. After standing for 3 days to 1 month, a prismatic crystal having approximate dimensions of 20 µm x 20 µm x 150 µm was obtained. Furthermore, by adding 2 to 10% of 1,4-dioxane to the crystallization solution, a larger crystal having approximate dimensions of 60 µm x 60 µm x 600 µm at the maximum was obtained.

### Example 3

### Crystal structure analysis of the complex between G-CSF and CRS-G-CSF-R

The crystal obtained in Example 2 was soaked in the crystallization solution containing 50% sucrose and then put under a nitrogen gas stream at 100 K to be flash frozen. Under a 100 K nitrogen gas stream, X-ray diffraction data were collected by the oscillation method. In addition, the crystal obtained in Example 2 was also soaked in the crystallization solution containing 1 mM ethylmercurithiosalicylate for 6 hours to obtain an isomorphous derivative crystal containing a mercury atom in the crystal. The crystal was soaked in the crystallization solution containing 50% sucrose and then put under a nitrogen gas stream at 100 K to be flash frozen. Under a 100 K nitrogen gas stream, X-ray diffraction data were collected by the oscillation method. Furthermore, G-CSF in which methionine residues have been substituted by selenomethionine residues was obtained by cultivating, in a medium containing selenomethionine, an *E*. *coli* strain prepared so as to express G-CSF. Using this G-CSF which contained substituting selenomethionine residues, a crystal of the complex between G-CSF which contained selenomethionine residues substituting for methionine residues and CRH-G-CSF-R was prepared according to the method described in Example 2. The crystal was soaked in the crystallization solution containing 50% sucrose and then put under a nitrogen gas stream at 100 K to be flash frozen. Under a 100 K nitrogen gas stream, X-ray diffraction data were collected by the oscillation method. From each diffraction data obtained, diffraction amplitudes were converted into numerical forms using DENZO/SCALEPACK (MAC Science Co.) to determine crystal structure factors. At this stage, the crystal belonged to the tetragonal space group I4₁22 with the unit cell parameters of a=b=125+10 Å and c=373±10 Å.

Then, the following analysis was conducted using a series of programs called CCP4. Calculation of the Fourier transform was conducted using difference in diffraction amplitudes obtained by the isomorphous mercury derivative crystal and the native crystal, and from the difference Patterson map obtained, the position of the mercury atom in the unit cell in real space satisfying the map was determined. Using the mercury coordinates obtained, the phase of the native crystal structure factor was determined. Fourier synthesis was made using the phase and absolute values of the crystal structure factors of the selenomethionine-substituted crystal and the native crystal to prepare a difference electron density map and thereby determine the atom coordinates of selenium atoms. In order to more precisely determine the positions of mercury and selenium atoms, calculation of refinement was conducted using the three crystal structure factors of the native crystal, the mercury derivative and the selenium derivative. Using the phase of native crystal structure factor calculated from the obtained positions of mercury and selenium atom, an electronic density map in real space of the crystal of the complex between G-CSF and CRH-G-CSF-R was obtained. Furthermore, smoothing of the electron density in the solvent region and electron density averaging using non-crystallographic symmetry were conducted, and the sites corresponding to amino acid residues of the complex between G-CSF and CRH-G-CSF-R were identified on the electron density map using QUANTA (Molecular Simulation, Inc.).

Refinement of the positions of the sites corresponding to amino acid residues was then conducted using X-PLOR (Molecular Simulation, Inc.), and amino acid residues were identified using QUANTA. This procedure was repeated to identify the following: as to two molecules of G-CSF each consisting of 175 amino acid residues, structure coordinates of amino acid residues except for M1 to L4, P129 to G136 and the side chain of Q71 in one molecule (Molecule A) and structure coordinates of amino acid residues except for M1 to A7, Q68 to L70 and the side chain of Q71 in the other molecule (Molecule C); as to two molecules of CRH-G-CSF-R, structure coordinates of amino acid residues except for V123 to S125, K214 to A215 and the side chains of K63, R64, and H126 in one molecule (Molecule B) and structure coordinates of amino acid residues except for A1 to G2, H33 to P35, G120 to S125, K214 to A215 and the side chains of K62, R64, 1119, Q127, and M213 in the other molecule (Molecule D); and further one N-acetylglucosamine residue and 182 water molecules.

At this stage, the R factor recognized in the art as an indicator of accuracy of the structure coordinates was 19.5% when a structure factor obtained from a diffraction pattern having Bragg reflection angle of 6 to 2.8 Å was used. An R-factor calculated from a structure factor independently excluded from the calculation for refinement at the refinement stage (known in the art as free-R-factor) was 28.3%. Furthermore, the root mean square errors from the ideal states of bond distances and bond angles among atoms were 0.012 Å and 2.0 degree, respectively.

### Example 4

### Re-refinement of structure coordinates of the complex between G-CSF and CRH-G-CSF-R

The structure coordinates of the complex between G-CSF and CRH-G-CSF-R obtained in Example 3 were further refined. Using the crystal structure factor and structure coordinates obtained in Example 3, calculations with REFMAC (in CCP4 programs) and X-PLOR and model modification with QUANTA were repeated to identify the following: structure coordinates of amino acid residues except for M1 to L4 and the side chain portions of Q71 and L131 in one molecule (Molecule A) of two molecules of G-CSF each consisting of 175 amino acid residues; structure coordinates of amino acid residues except for M1 to P6, and the side chain portions of H53, W59, Q68, L70, Q71 in Molecule C of G-CSF; structure coordinates of amino acid residues except for G120 to H126, K214 to A215 and the side chain portions of K63 and R64 in Molecule B of CRH-G-CSF-R; structure coordinates of amino acid residues except for A1, 1119 to H126, K214 to A215 and the side chain portions of K62, K63, R64, Q127 in Molecule D of CRH-G-CSF-R; and further two N-acetylglucosamine residues and 260 water molecules. At this stage, the R factor recognized in the art as an indicator of accuracy of the structure coordinates was 22.2% when a structure factor obtained from a diffraction pattern having Bragg reflection angle of 25 to 2.8 Å was used. An R-factor calculated from a structure factor independently excluded from the calculation for refinement at the refinement stage (known in the art as free-R-factor) was 29.8%. Furthermore, the root mean square errors from the ideal states of bond distances and bond angles among atoms were 0.014 Å and 1.9 degree, respectively.

Structure coordinates obtained were shown in Table 1 according to the format of Protein Data Bank, a notation system commonly used in the art.

### Example 5

### Molecular replacement method using structure coordinates of G-CSF and CRH-G-CSF-R

The crystal of the complex between G-CSF and CRH-G-CSF-R obtained in Example 2 was soaked in 0.1 M N-2-hydroxyethylpiperazine-N'-2-ethanesulfonic acid buffer solution containing 20% glycerol and 2.4 M ammonium sulfate for one day and then put under a nitrogen gas stream at 100 K to be flash frozen. Under cooling with a 100 K nitrogen gas stream, X-ray diffraction data were collected by the oscillation method. Each diffraction pattern was converted into a structure factor using DENZO/SCALEPACK. At this stage, the crystal belonged to the tetragonal space group P4₁2₁2 or P4₃2₁2 with the unit cell parameters of a=b=126+10 Å and c=373±10 Å. It was thus demonstrated that the crystal obtained in Example 2 changed its space group from I4₁22 to P4₁2₁2 or P4₃2₁2 by being soaked in a solution containing a high concentration of ammonium sulfate.

Using a structure factor obtained and three-dimensional structure coordinates of the complex between G-CSF and CRH-G-CSF-R shown in Table 1, a molecular replacement method was conducted (see, e.g., Blundell, T. L. and Johnson, L. N., (1976), PROTEIN CRYSTALLOGRAPHY, pp. 443-464, Academic Press, New York). For this molecular replacement method, rigid-body refinement of X-PLOR was used. A structure factor obtained from a diffraction pattern having Bragg reflection angles of 8 to 3.5 Å was used for calculations. The R factor was 31.7%. Three-dimensional structure coordinates of the complex between G-CSF and CRH-G-CSF-R having a new structure were thereby obtained. It was also shown that the space group was P4₃2₁2. The obtained structure coordinates indicated that the complex between G-CSF and CRH-G-CSF-R contains four molecules of the complex in the asymmetric unit. It was also shown that when compared with the structure obtained from the I4₁22 crystal, the structure obtained was different in the angle between G-CSF molecule and CRH-G-CSF-R molecule and in the angle between domains of CRH-G-CSF-R by several degrees, indicating that these molecular recognitions have variability within several degrees.

### Example 6

### Structure coordinates of human-type G-CSF-R

Based on the structure coordinates obtained in Example 4, structure coordinates of human-type CRH-G-CSF-R shown in SEQ ID NO: 3 were prepared using a homology model (Haruki Nakamura and Kenta Nakai, "Biotechnology-no-tameno-Computer-nyumon" (An introduction to computers for biotechnology), pp. 186-204, Corona Publishing Co., 1995). First, in the structure coordinates of mouse CRH-G-CSF-R shown in Table 1, side chain portions of the positions at which amino acid residues are not identical between mouse and human CRH-G-CSF-Rs were replace by corresponding side chains of the human amino acid residues. This replacement of amino acid residues was conducted using a biopolymer energy calculation program PRESTO 2.0 on the above described workstation Indy XZ. At this stage, conformations of the side chains were selected so that the atoms do not stereochemically overlap one another and the energy became minimal. This calculation was conducted using programs Side-Chain Modeller and PRESTO 2.0 on the above workstation Indy XZ. Furthermore, conformational calculations were conducted for all amino acid residues including their backbone portions to minimize the energy of the whole molecule. This calculation was conducted using the program PRESTO 2.0 on the above workstation Indy XZ. The coordinates thus obtained are shown in Table 6.

## Claims

1. A crystal of a protein complex between granulocyte colony-stimulating factor (G-CSF) and the G-CSF binding region (CRH-G-CSF-R) of the granulocyte colony-stimulating factor receptor (G-CSF-R).

2. A crystal of a protein complex according to claim 1 wherein G-CSF and CRH-G-CSF-R are derived from a mammal or mammals.

3. A crystal of a protein complex according to claim 2 wherein G-CSF comprises a human-type sequence shown in SEQ ID NO: 1, and CRH-G-CSF-R comprises a mouse-type sequence shown in SEQ ID NO: 2.

4. A crystal according to any one of claims 1 to 3 wherein the crystal has the tetragonal space group symmetry I4₁22.

5. A crystal according to claim 4 wherein the unit cell parameters of the crystal are a=b=125±10 Å and c=373±10 Å.

6. A crystal according to any one of claims 1 to 3 wherein the crystal has the tetragonal space group symmetry P4₃2₁2.

7. A crystal according to claim 6 wherein the unit cell parameters of the crystal are a=b=126±10 Å and c=373±10 Å.

8. A crystal according to any one of claims 1 to 7 wherein stoichiometrically equivalent quantities of G-CSF and CRH-G-CSF-R bind together to form the complex.

9. A crystal according to any one of claims 1 to 5 wherein stoichiometrically equivalent quantities of G-CSF and CRH-G-CSF-R bind together to form the complex and two molecules of the complex exist in a crystallographic asymmetric unit.

10. A crystal according to any one of claims 1 to 3, 6, and 7 wherein stoichiometrically equivalent quantities of G-CSF and CRH-G-CSF-R bind together to form the complex and four molecules of the complex exist in a crystallographic asymmetric unit.

11. A crystal according to any one of claims 1 to 10 wherein the regions forming the complex between G-CSF wand CRH-G-CSF-R are **characterized by** all or part of amino acid residues S13, L16, K17, E20, Q21, R23, K24, L109, D110, D113, T116, T117, Q120, E123, E124 and amino acid residues adjacent thereto in the amino acid sequence of human-type G-CSF shown in SEQ ID NO: 1.

12. A crystal according to any one of claims 1 to 10 wherein the regions forming the complex between G-CSF and CRH-G-CSF-R are **characterized by** all or part of amino acid residues N20, 545, R46, R72, K73, L75, L76, L77, Y78, Q79, Y80, D102, M104, D105, Y143, M144, E145, R193, S195, L196 and amino acid residues adjacent thereto in the amino acid sequence of mouse-type CRH-G-CSF-R shown in SEQ ID NO: 2.

13. A crystal according to any one of claims 1 to 10 wherein the regions forming the associate of the complex between G-CSF and CRH-G-CSF-R are **characterized by** all or part of amino acid residues G5, P6, A7, S8, S9, L10, P11, Q12, L125 and amino acid residues adjacent thereto in the amino acid sequence of human-type G-CSF shown in SEQ ID NO: 1.

14. A crystal according to any one of claims 1 to 10 wherein the regions forming the associate of the complex between G-CSF and CRH-G-CSF-R are **characterized by** all or part of amino acid residues W161, L163, V164, F165, H166, L167, P168, and K171 and amino acid residues adjacent thereto in the amino acid sequence of mouse-type CRH-G-CSF-R shown in SEQ ID NO: 2.

15. A crystal according to any one of claims 1 to 10 wherein amino acid residues exposed to the solvent region on the side of the binding surface formed by the associate of the complex between G-CSF and CRH-G-CSF-R were **characterized by** all or part of amino acid residues Y3 to L14, R46 to Y51, G92 to V106, E145 to E147, H166 to S169, S194 to G198 and amino acid residues adjacent thereto in the amino acid sequence of mouse-type CRH-G-CSF-R shown in SEQ ID NO: 2.

16. Three-dimensional structure coordinates of a complex formed by G-CSF and CRH-G-CSF-R for use in identifying, searching for, evaluating, or designing variants, agonists, or antagonists of G-CSF.

17. Three-dimensional structure coordinates according to claim 16 wherein the three-dimensional structure coordinates are those shown in Table 1.

18. Three-dimensional structure coordinates according to claim 16 wherein the three-dimensional structure coordinates are those of a complex between G-CSF derived from a species other than human comprising a sequence having 20% or more homology to the amino acid sequence of human-type G-CSF and CRH-G-CSF-R derived from a species other than mouse comprising a sequence having 20% or more homology to the amino acid sequence of mouse-type CRH-G-CSF-R determined by a molecular replacement method or using a homology model.

19. Three-dimensional structure coordinates according to claim 18 wherein the three-dimensional structure coordinates are those shown in Table 6.

20. A computer storage medium storing all or part of the three-dimensional structure coordinates according to any one of claims 16 to 19 for use in identifying, searching for, evaluating, or designing a variant, agonist, or antagonist of G-CSF.

21. Use of all or part of the three-dimensional structure coordinates according to any one of claims 16 to 19 or the computer storage medium according to claim 20 for identifying, searching for, evaluating, or designing a variant, agonist, or antagonist of G-CSF.

22. Use of claim 21 **characterized in that** the three-dimensional structure coordinates are those of amino acid residues shown in Tables 2 to 5 or amino acid residues adjacent thereto.

23. A method of identifying, searching for, evaluating, or designing a G-CSF variant which has biological activities equal or superior to those of native G-CSF and in which one or more amino acid residues have been substituted, deleted, inserted, or chemically modified, the method being **characterized in that** it uses all or part of the three-dimensional coordinates according to any one of claims 16 to 19 or the computer storage medium according to claim 20.

24. A method of identifying, searching for, evaluating, or designing a G-CSF variant which has activity as an antagonist and in which one or more amino acid residues have been substituted, deleted, inserted, or chemically modified, the method being **characterized in that** it uses all or part of the three-dimensional coordinates according to any one of claims 16 to 19 or the computer storage medium according to claim 20.

25. A method according to claim 23 or 24 wherein the variant is substitution, deletion, insertion, or chemical modification of one or more of amino acid residues G5, P6, A7, S8, S9, L10, P11, Q12, S13, L16, K17, E20, Q21, R23, K24, L109, D110, D113, T116, T117, Q120, E123, E124, L125 and amino acid residues adjacent thereto in human-type G-CSF shown in SEQ ID NO: 1.

26. A G-CSF variant in which one or more of amino acid residues G5, P6, A7, S8, S9, L10, P11, Q12, S13, L16, K17, E20, Q21, R23, K24, L109, D110, D113, T116, T117, Q120, E123, E124, L125 and amino acid residues adjacent thereto have been substituted, deleted, inserted, or chemically modified in human-type G-CSF shown in SEQ ID NO: 1.

27. A method of identifying, searching for, evaluating, or designing a G-CSF agonist, the method being **characterized in that** it uses all or part of the three-dimensional structure coordinates according to claims 16 to 19 or the computer storage medium according to claim 20.

28. A method according to claim 27 wherein it particularly uses three-dimensional coordinates of the positions corresponding to amino acid residues Y3 to L14, R46 to Y51, G92 to V106, E145 to E147, H166 to S169, S194 to G198 and amino acid residues adjacent thereto among those shown in Table 1 or 6.

29. A method according to claim 27 or 28 wherein the agonist binds to CRH-G-CSF-R and thereby provides spatial positions of CRH-G-CSF-R substantially identical to those of the CRH-G-CSF-R in the associate of the complex between CHR-G-CSF-R and G-CSF and wherein the agonist binds to CRH-G-CSF-R at two or more sites.

30. A compound which is an agonist of G-CSF and is obtained using a method of drug design according to any one of claims 27 to 29.

31. A compound according to claim 30 wherein the agonist of G-CSF is a natural or synthetic compound.

32. A method of identifying, searching for, evaluating, or designing an antagonist of G-CSF, the method being **characterized in that** it uses all or part of the three-dimensional coordinates according to any one of claims 16 to 19 or the computer storage medium according to claim 20.

33. A method according to claim 32 wherein the antagonist is a compound which binds to G-CSF and inhibits binding of G-CSF to G-CSF-R.

34. A method according to claim 32 wherein the antagonist is a compound which binds to CRH-G-CSF-R and inhibits binding of G-CSF to G-CSF-R.

35. A method according to claim 32 wherein the antagonist is a compound which binds to the complex between G-CSF and G-CSF-R and inhibits normal binding between G-CSF and G-CSF-R.

36. A compound which is an antagonist of G-CSF and is obtained using a method according to any one of claims 32 to 35.

37. A compound according to claim 36 wherein the antagonist of G-CSF is a natural or synthetic compound.

38. Use of all or part of the three-dimensional structure coordinates according to any one of claims 16 to 19 or the computer storage medium according to claim 20 in crystallography using the technique of molecular replacement.
